# EUROPEAN PATENT SPECIFICATION

(11) **EP 3 443 096 B1**
(45) Date of publication and mention of the grant of the patent: **01.03.2023**
(21) Application number: 17737079.8
(22) Date of filing: 14.04.2017
(51) Int. Cl.: C12N 15/62, C07K 14/725

(54) **COMPOSITIONS AND METHODS FOR SELECTIVE EXPRESSION OF CHIMERIC ANTIGEN RECEPTORS**
ZUSAMMENSETZUNGEN UND VERFAHREN ZUR SELEKTIVEN EXPRESSION VON CHIMÄREN ANTIGENREZEPTOREN
COMPOSITIONS ET MÉTHODES POUR L'EXPRESSION SÉLECTIVE DES RÉCEPTEURS ANTIGÉNIQUES CHIMÉRIQUES

(30) Priority: 15.04.2016 US 201662322931 P; 03.04.2017 US 201762481094 P
(43) Date of publication of application: 20.02.2019
(60) Divisional application: 23151319.3
(73) Proprietor: Novartis AG, 4056 Basel (CH); The Trustees of The University of Pennsylvania, Philadelphia, PA 19104 (US)
(72) Inventor: GOLOSOV, Andrei, Cambridge, MA 02139 (US); GUIMARAES, Carla, Cambridge, MA 02139 (US); MOTZ, Gregory, Quincy, MA 02170 (US); MILONE, Michael, Cherry Hill, NJ 08002 (US); ELLEBRECHT, Christoph, T., Philadelphia, PA 19147 (US); PAYNE, Aimee, S., Merion Station, PA 19066 (US)
(74) Representative: Wilding, James Roger
(86) International application number: PCT/US2017/027778
(87) International publication number: WO 2017/181119

(56) References cited:
- WO-A1-2015/134877
- WO-A1-2017/024318
- WO-A1-2017/032777
- WO-A2-00/23602
- WO-A2-01/57242
- WO-A2-2014/012001
- WO-A2-2015/168613
- AJAMETE KAYKAS ET AL: "Mutant Frizzled 4 associated with vitreoretinopathy traps wild-type Frizzled in the endoplasmic reticulum by oligomerization", NATURE CELL BIOLOGY, vol. 6, no. 1, 14 December 2003 (2003-12-14), pages 52-58, XP055407961, GB ISSN: 1465-7392, DOI: 10.1038/ncb1081
- RIVERA V M ET AL: "REGULATION OF PROTEIN SECRETION THROUGH CONTROLLED AGGREGATION IN THE ENDOPLASMIC RETICULUM", SCIENCE, AMERICAN ASSOCIATION FOR THE ADVANCEMENT OF SCIENCE, vol. 287, 1 January 2000 (2000-01-01), pages 826-830, XP000887329, ISSN: 0036-8075, DOI: 10.1126/SCIENCE.287.5454.826 cited in the application
- LUIS QUINTINO ET AL: "Functional Neuroprotection and Efficient Regulation of GDNF Using Destabilizing Domains in a Rodent Model of Parkinson's Disease", MOLECULAR THERAPY, vol. 21, no. 12, 1 December 2013 (2013-12-01), pages 2169-2180, XP055408532, US ISSN: 1525-0016, DOI: 10.1038/mt.2013.169
- HOKYUNG K CHUNG ET AL: "Tunable and reversible drug control of protein production via a self-excising degron", NATURE CHEMICAL BIOLOGY, vol. 11, no. 9, 27 July 2015 (2015-07-27), pages 713-720, XP055408268, Basingstoke ISSN: 1552-4450, DOI: 10.1038/nchembio.1869
- M. R. PRATT ET AL: "Small-molecule-mediated rescue of protein function by an inducible proteolytic shunt", PROCEEDINGS NATIONAL ACADEMY OF SCIENCES PNAS, vol. 104, no. 27, 3 July 2007 (2007-07-03) , pages 11209-11214, XP055408262, US ISSN: 0027-8424, DOI: 10.1073/pnas.0700816104
- YUSUKE MIYAZAKI ET AL: "Destabilizing Domains Derived from the Human Estrogen Receptor", JOURNAL OF THE AMERICAN CHEMICAL SOCIETY, vol. 134, no. 9, 7 March 2012 (2012-03-07) , pages 3942-3945, XP55387238, US ISSN: 0002-7863, DOI: 10.1021/ja209933r
- MICHAEL C JENSEN ET AL: "Design and implementation of adoptive therapy with chimeric antigen receptor-modified T cells", IMMUNOLOGICAL REVIEWS., vol. 257, no. 1, Special Issue, 13 December 2013 (2013-12-13), pages 127-144, XP055156740, US ISSN: 0105-2896
- GHORASHIAN SARA ET AL: "CD19 chimeric antigen receptor T cell therapy for haematological malignancies", BRITISH JOURNAL OF HAEMATOLOGY, BLACKWELL PUBLISHING LTD, vol. 169, no. 4, 1 May 2015 (2015-05-01), pages 463-478, XP002762697, ISSN: 1365-2141
- JIE SUN ET AL: "The quest for spatio-temporal control of CAR T cells", CELL RESEARCH - XIBAO YANJIU, vol. 25, no. 12, 17 December 2015 (2015-12-17), pages 1281-1282, XP055361929, GB, CN ISSN: 1001-0602, DOI: 10.1038/cr.2015.131
- ANTONIO DI STASI ET AL: "Inducible Apoptosis as a Safety Switch for Adoptive Cell Therapy", NEW ENGLAND JOURNAL OF MEDICINE, vol. 365, no. 18, 3 November 2011 (2011-11-03), pages 1673-1683, XP055181696, ISSN: 0028-4793, DOI: 10.1056/NEJMoa1106152
- LIHUA E. BUDDE ET AL: "Combining a CD20 Chimeric Antigen Receptor and an Inducible Caspase 9 Suicide Switch to Improve the Efficacy and Safety of T Cell Adoptive Immunotherapy for Lymphoma", PLOS ONE, vol. 8, no. 12, 17 December 2013 (2013-12-17), page e82742, XP055213511, DOI: 10.1371/journal.pone.0082742
- GRADA ZAKARIA ET AL: "TanCAR: A Novel Bispecific Chimeric Antigen Receptor for Cancer Immunotherapy", MOLECULAR THERAPY-NUCLEIC ACIDS, vol. 2, July 2013 (2013-07), page Article No.: e105, ISSN: 2162-2531(print)
- ZELENETZ ANDREW D ET AL: "Chronic lymphocytic leukemia/small lymphocytic lymphoma, version 1.2015.", JOURNAL OF THE NATIONAL COMPREHENSIVE CANCER NETWORK : JNCCN MAR 2015, vol. 13, no. 3, March 2015 (2015-03), pages 326-362, ISSN: 1540-1413

## Description

### BACKGROUND OF THE INVENTION

A variety of constructs have been developed to enable selective expression of a desired protein of interest. Recent constructs have been tested which incorporate a domain engineered to degrade in the absence of a stabilizing ligand (known in the art as "degrons"). Other constructs incorporate a domain engineered to aggregate in the absence of a deaggregating ligand (e.g., an aggregation domain). Such domains can be fused to proteins of interest to permit selective expression of such proteins only in the presence of the stabilizing or deaggregating ligand.

Degrons and aggregation domains known in the art can, in certain circumstances, have certain disadvantages associated with disrupting the desired function of the protein of interest due to the size and/or conformation of the fusion protein. Such disadvantages can be particularly evident when the protein of interest is a transmembrane protein. Thus, there is a need for improving the degron/aggregation domain technology. Furthermore, a need exists for methods of modifying T cells to treat various diseases and conditions such as but not limited to cancer, autoimmunity and alloimmunity without permanently modifying or suppressing the immune system. WO2017/024318 relates to attenuating adoptive T cell therapy associated adverse inflammatory responses using targeted protein degradation.

### SUMMARY OF THE INVENTION

The invention is set out in the appended set of claims. The present disclosure provides further instances which are not part of the claimed invention.

The present invention features fusion proteins including two protein domains separated by a heterologous protease cleavage site (e.g., a protease cleave site that is cleaved by a mammalian intracellular or extracellular protease), wherein a first of the protein domains is a conditional expression domain which is a degradation domain, and the second protein is a protein of interest which is a CAR.

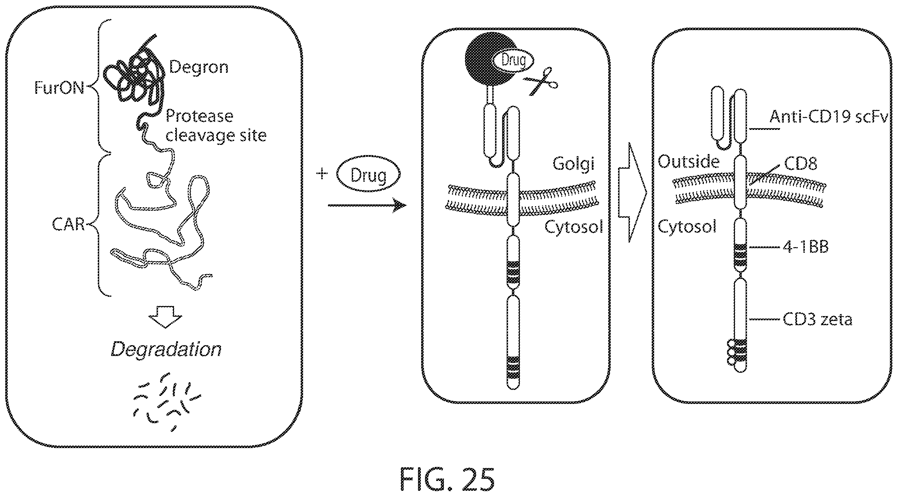

In the present invention, the conditional expression domain is a degradation domain, e.g., a degradation domain as described herein. Without wishing to be bound by theory, in some embodiments, the degradation domain is unstable and/or unable to fold into a stable conformation in the absence of an expression compound, e.g., a stabilization compound. The misfolded/unfolded degradation domain can be degraded by intracellular degradation pathways along with the other domain(s) of the fusion protein (see, for example, Figure 25). In the presence of the expression compound, the degradation domain is able to fold into a stable conformation and is less susceptible to intracellular degradation pathways, e.g., relative to the degradation domain in the absence of the expression compound. Thus, the level and/or rate of cell surface expression or extracellular expression of the fusion protein is enhanced, e.g., by at least 2-, 3-, 4-, 5-, 6-, 7-, 8-, 9-, 10-, 20-, or 30-fold, in the presence of the expression compound relative to the absence of the expression compound. In some embodiments, once the degradation domain of the fusion protein assumes a proper conformation, the heterologous cleavage site is exposed, leading to removal of the degradation domain, and thus, freeing the second protein domain.

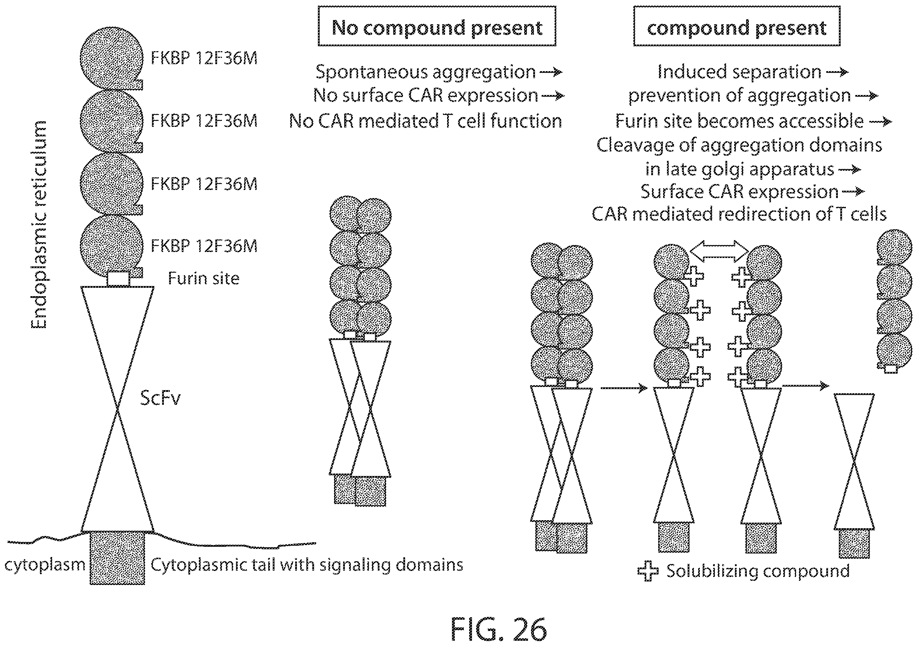

In other instances disclosed herein which do not form part of the claimed invention, the conditional expression domain is an aggregation domain. Without wishing to be bound by theory, in some instances in the absence of expression compound, e.g., deaggregation compound, the aggregation domain of the fusion protein associates with one or more other aggregation domains into oligomers and aggregates (see, for example, Figure 26). The aggregated fusion protein can be sequestered in the cellular compartment in which it is aggregated. In the presence of expression compound, e.g., deaggregation compound, the aggregation domains dissociate from one another, and the fusion proteins are solubilized (e.g., assumes a monomeric configuration). Thus, the level and/or rate of cell surface expression or extracellular expression of the fusion protein is enhanced, e.g., by at least 2-, 3-, 4-, 5-, 6-, 7-, 8-, 9-, 10-, 20-, or 30-fold, in the presence of the expression compound relative to the absence of the expression compound. In some instances, once the aggregation domain of the fusion protein is solubilized, the heterologous cleavage site is exposed, leading to removal of the aggregation domain, and thus, freeing the second protein domain.

In any of the foregoing instances and embodiments, the fusion protein includes a first protein domain that is or comprises a conditional expression domain, e.g., a degradation domain or an aggregation domain, and a second protein domain that is or comprises a protein of interest, e.g., a transmembrane protein (e.g., a CAR), wherein the first and the second domains of the fusion protein are separated by a heterologous protease cleavage site (e.g., a protease cleave site that is cleaved by a mammalian intracellular or extracellular protease). In some embodiments and instances, the conditional expression domain, e.g., the degradation or the aggregation domain, is located N-terminal to the second protein domain. In certain embodiments, the fusion protein further includes a signal peptide N-terminal to the degradation domain.

Disclosed herein is a fusion protein, comprising two protein domains separated by a heterologous protease cleavage site, wherein a first of said protein domains is a conditional expression domain, and a second of said protein domains is a transmembrane protein, wherein the conditional expression domain has a first state associated with a first level of surface expression of the fusion protein and a second state associated with a second level of surface expression and/or extracellular expression of the fusion protein, wherein the second level is increased, e.g., by at least 2, 3, 4, 5, 10, 20 or 30 fold over the first level in the presence of an expression compound.

In another aspect, the disclosure pertains to a fusion protein, comprising two protein domains separated by a heterologous protease cleavage site, wherein a first of said protein domains is a conditional expression domain, and a second of said protein domains is a transmembrane protein, wherein the heterologous protease cleavage site is a furin cleavage site, provided that the furin cleavage site does not comprise the amino acid sequence SARNRQKR (SEQ ID NO: 981).

In the present invention, the conditional expression domain is a degradation domain.

In the present invention, the fusion protein comprises two protein domains separated by a heterologous protease cleavage site, wherein the first of said protein domains (also referred to herein as the first protein domain) is or comprises a degradation domain, e.g., a degradation domain as described herein, and the second of said protein domains (also referred to herein as the second protein domain) is a protein of interest. In the present invention, the protein of interest is a transmembrane protein, which is a CAR.

The degradation domain is chosen from an estrogen receptor (ER) domain, an FKB protein (FKBP) domain or an dihydrofolate reductase (DHFR).

In the present invention, the degradation domain has a first state associated with a first level of surface expression and/or extracellular expression of the fusion protein and a second state associated with a second level of surface expression and/or extracellular expression of the fusion protein, wherein the second level is increased, e.g., by at least 2, 3, 4, 5, 10, 20 or 30 fold over the first level in the presence of a stabilization compound.

In certain embodiments, the degradation domain is derived from an estrogen receptor. For example, the degradation domain can comprise an amino acid sequence selected from SEQ ID NO: 58 or a sequence having at least 90%, 95%, 97%, 98%, or 99% identity thereto, or SEQ ID NO: 121 or a sequence having at least 90%, 95%, 97%, 98%, or 99% identity thereto. In some embodiments, the degradation domain comprises an amino acid sequence selected from SEQ ID NO: 58 or SEQ ID NO: 121. When the degradation domain is derived from an estrogen receptor, the stabilization compound can be selected from Bazedoxifene or 4-hydroxy tamoxifen (4-OHT). In some embodiments, the stabilization compound is Bazedoxifene. Tamoxifen and Bazedoxifene are FDA approved drugs, and thus are safe to use in a human.

In certain embodiments, the degradation domain is derived from an FKB protein (FKBP). For example, the degradation domain can comprise an amino acid sequence of SEQ ID NO: 56 or a sequence having at least 90%, 95%, 97%, 98%, or 99% identity thereto. In some embodiments, the degradation domain comprises SEQ ID NO: 56. When the degradation domain is derived from a FKBP, the stabilization compound can be Shield-1.

In some embodiments, the degradation domain is derived from dihydrofolate reductase (DHFR). In some embodiments, the degradation domain comprises an amino acid sequence selected from SEQ ID NO: 57 or a sequence having at least 90%, 95%, 97%, 98%, or 99% identity thereto. In some embodiments, the degradation domain comprises SEQ ID NO: 57. When the degradation domain is derived from a DHFR, the stabilization compound can be Trimethoprim.

In other embodiments, the degradation domain is not derived from an FKB protein or estrogen receptor.

In some instances disclosed herein which do not form part of the claimed invention, the fusion protein comprises two protein domains separated by a heterologous protease cleavage site, wherein the first of said protein domains (also referred to herein as the first protein domain) is or comprises a aggregation domain, e.g., an aggregationdomain as described herein, and the second of said protein domains (also referred to herein as the second protein domain) is a protein of interest. In one instance, the protein of interest is a transmembrane protein, e.g., a CAR.

In some instances, the fusion protein comprises two protein domains separated by a heterologous protease cleavage site, wherein a first of said protein domains is an aggregation domain, and a second of said protein domains is a transmembrane protein, wherein the aggregation domain has a first state associated with a first level of surface expression and/or extracellular expression of the fusion protein and a second state associated with a second level of surface expression and/or extracellular expression of the fusion protein, and wherein the second level is increased, e.g., by at least 2, 3, 4, 5, 10, 20 or 30 fold over the first level in the presence of a deaggregation compound.

In some instances, the fusion protein, comprises two protein domains separated by a heterologous protease cleavage site, wherein a first of said protein domains is an aggregation domain, and a second of said protein domains is a transmembrane protein, wherein the heterologous protease cleavage site is a furin cleavage site, provided that the furin cleavage site does not comprise the amino acid sequence SARNRQKR (SEQ ID NO: 981).

In some instances, the fusion protein, comprises two protein domains separated by a heterologous protease cleavage site, wherein a first of said protein domains is an aggregation domain, and a second of said protein domains is a chimeric antigen receptor (CAR).

In some instances, the aggregation domain comprises 1, 2, 3, 4 5, 6, 7, 8, or more repeats of a dimerization domain, e.g., a homodimerization or a heterodimerization domain.

In some instances, the aggregation domain is from an FKB protein (FKBP).

In some instances, the aggregation domain is an FKBP F36M domain.

In some instances, the aggregation domain is from an FKB protein (FKBP) and comprises an amino acid sequence that is at least 90, 95, 97, 98, 99, or 100% identical to either of SEQ ID NOs: 975 or 976.

In some instances, the fusion protein comprises a further 2nd, 3^{rd}, 4^{th}, 5^{th}, 6^{th}, 7^{th}, 8^{th}, 9^{th}, or 10^{th} aggregation domain.

In some instances, the 2nd, 3^{rd}, 4^{th}, 5^{th}, 6^{th}, 7^{th}, 8^{th}, 9^{th}, or 10^{th} aggregation domain is the same type of aggregation domain as the first aggregation domain.

In some instances, the aggregation domain forms homodimers with the same aggregation domain.

In some instances, the fusion protein comprises a plurality of aggregation domains, wherein the plurality comprises more than one, e.g., two, types of aggregation domains, and a first type of aggregation domain forms heterodimers with a second type of aggregation domain.

In some instances, the fusion protein comprises 2, 4, 6, 8, or 10 aggregation domains, wherein the fusion protein comprises equal numbers of the first type of aggregation domain and the second type of aggregation domain.

In some instances, the aggregation domains are disposed in the fusion protein in an alternating order of first type and second type, e.g., first, second, first, second, or second, first, second, first.

In some instances, said deaggregation compound is selected from FK506, rapamycin, AP22542, AP21998, and Shield-1 when the fusion protein comprises an aggregation domain derived from FKB protein (FKBP), e.g., FKBP F36M.

In some embodiments, the heterologous cleavage site is cleaved by a mammalian intracellular protease.

In some embodiments, said cleavage site is cleaved by a protease selected from the group consisting of furin, PCSK1 , PCSK5, PCSK6, PCSK7, cathepsin B, Granzyme B, Factor XA, Enterokinase, genenase, sortase, precission protease, thrombin, TEV protease, and elastase 1.

In some embodiments, said cleavage site comprises a polypeptide having an cleavage motif selected from the group consisting of RX(K/R)R consensus motif, RXXX[KR]R consensus motif, RRX consensus motif, I-E-P-D-X consensus motif (SEQ ID NO: 35), Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A consensus motif, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41), and [AGSV]-x (SEQ ID NO: 42).

In some embodiments, said cleavage site is cleaved by furin.

In some embodiments, the fusion protein comprises a furin cleavage site selected from RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); or CKINGYPKRGRKRR (SEQ ID NO: 137).

In some embodiments, the fusion protein comprises a furin cleavage site selected from GTGAEDPRPSRKRRSLGDVG (SEQ ID NO: 125); or GTGAEDPRPSRKRR (SEQ ID NO: 127).

In some embodiments, the fusion protein comprises the furin cleavage site of GTGAEDPRPSRKRRSLGDVG (SEQ ID NO: 125).

In some embodiments, said heterologous protease cleavage site is cleaved by a mammalian extracellular protease.

In some embodiments, said mammalian extracellular protease is selected from the group consisting of Factor XA, Enterokinase, genenase, sortase, precission protease, thrombin, TEV protease, and elastase 1.

In some embodiments, said cleavage site comprises a polypeptide having an amino acid sequence selected from the group consisting of Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A consensus motif, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41), and [AGSV]-x (SEQ ID NO: 42).

In any of the foregoing aspects and embodiments, the heterologous cleavage site is cleaved by furin, PCSK1, PCSK5, PCSK6, PCSK7, cathepsin B, Granzyme B, Factor XA, Enterokinase, genenase, sortase, precission protease, thrombin, TEV protease, or elastase 1. For example, the protease cleave site can include a polypeptide having an cleavage motif selected from RX(K/R)R consensus motif, RXXX[KR]R consensus motif, RRX consensus motif, I-E-P-D-X consensus motif (SEQ ID NO: 35), Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A consensus motif, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41), or [AGSV]-x (SEQ ID NO: 42). In certain embodiments, the mammalian extracellular protease is selected from Factor XA, Enterokinase, genenase, sortase, precission protease, thrombin, TEV protease, or elastase 1 (e.g., the cleavage site can include a polypeptide having an amino acid sequence selected from Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A consensus motif, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41), or [AGSV]-x (SEQ ID NO: 42)).

In some embodiments, the fusion protein of the invention includes a furin cleavage site. In some embodiments, the fusion proteins of the invention include any one of furin cleavage sites listed in Table 20. In some embodiments, the fusion proteins of the invention include a furin cleavage site selected from RTKR (SEQ ID NO: 123) or a sequence having at least 90%, 95%, 97%, 98%, or 99% identity thereto; GTGAEDPRPSRKRRSLGDVG (SEQ ID NO: 125) or a sequence having at least 90%, 95%, 97%, 98%, or 99% identity thereto; GTGAEDPRPSRKRR (SEQ ID NO: 127) or a sequence having at least 90%, 95%, 97%, 98%, or 99% identity thereto; LQWLEQQVAKRRTKR (SEQ ID NO: 129) or a sequence having at least 90%, 95%, 97%, 98%, or 99% identity thereto; GTGAEDPRPSRKRRSLGG (SEQ ID NO: 131) or a sequence having at least 90%, 95%, 97%, 98%, or 99% identity thereto; GTGAEDPRPSRKRRSLG (SEQ ID NO: 133) or a sequence having at least 90%, 95%, 97%, 98%, or 99% identity thereto; SLNLTESHNSRKKR (SEQ ID NO: 135) or a sequence having at least 90%, 95%, 97%, 98%, or 99% identity thereto; or CKINGYPKRGRKRR (SEQ ID NO: 137) or a sequence having at least 90%, 95%, 97%, 98%, or 99% identity thereto. In some embodiments, the fusion proteins of the invention include a furin cleavage site selected from GTGAEDPRPSRKRRSLGDVG (SEQ ID NO: 125) or a sequence having at least 90%, 95%, 97%, 98%, or 99% identity thereto , or GTGAEDPRPSRKRR (SEQ ID NO: 127) or a sequence having at least 90%, 95%, 97%, 98%, or 99% identity thereto. In some embodiments, the fusion proteins of the invention include the furin cleavage site of GTGAEDPRPSRKRRSLGDVG (SEQ ID NO: 125) or a sequence having at least 90%, 95%, 97%, 98%, or 99% identity thereto.

In some embodiments, the fusion proteins of the invention include a furin cleavage site selected from RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); or CKINGYPKRGRKRR (SEQ ID NO: 137). In some embodiments, the fusion proteins of the invention include a furin cleavage site selected from GTGAEDPRPSRKRRSLGDVG (SEQ ID NO: 125) or GTGAEDPRPSRKRR (SEQ ID NO: 127). In some embodiments, the fusion proteins of the invention include the furin cleavage site of GTGAEDPRPSRKRRSLGDVG (SEQ ID NO: 125).

In some embodiments, the conditional expression domain is located N-terminal to said second protein domain or C-terminal to said second protein domain. In the present invention the conditional expression domain is a degradation domain.

In some embodiments, said fusion protein further comprises a signal peptide N-terminal to said conditional expression domain. In some embodiments, the fusion protein further comprises a linker positioned between the signal peptide and said conditional expression domain. In some embodiments, the linker is a linker in any fusion protein listed in Tables 23 and 24.

In some embodiments, the fusion protein comprises an amino acid sequence of any fusion protein listed in Tables 22, 23, or 24.

In any of the foregoing aspects and embodiments, a second of the protein domains is a transmembrane protein which is a chimeric antigen receptor. Chimeric antigen receptors can include, e.g., in a N-terminal to C-terminal direction, an antigen binding domain, a transmembrane domain, and one or more intracellular signaling domains. The signaling domain may include one or more primary signaling domains (e.g., a CD3-zeta stimulatory domain) and, optionally, one or more costimulatory signaling domains (e.g., an intracellular domain from a costimulatory protein selected from CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, or a ligand that specifically binds CD83).

In certain of the above embodiments, the antigen binding domain is an scFv. Also, the antigen binding domain may binds an antigen selected from CD19; CD123; CD22; CD30; CD171; CS-1; C-type lectin-like molecule-1, CD33; epidermal growth factor receptor variant III (EGFRvIII); ganglioside G2 (GD2); ganglioside GD3; TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2; Mesothelin; Interleukin 11 receptor alpha (IL-1 1 Ra); prostate stem cell antigen (PSCA); Protease Serine 21; vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CA!X); Proteasome (Prosome, Macropain) Subunit, Beta Type, 9 (LMP2); glycoprotein 100 (gp100); oncogene fusion protein including breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3; transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1, melanoma antigen recognized by T cells 1; Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like, Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); or immunoglobulin lambda-like polypeptide 1 (IGLL1).

In some embodiments, the fusion protein comprises an antigen binding domain that binds CD19. In some embodiments, the fusion protein comprises an antigen binding domain comprising an amino acid sequence selected from any one of SEQ ID NOs: 356-368 or 381. In some embodiments, the fusion protein comprises a chimeric antigen receptor comprising an amino acid sequence selected from any one of SEQ ID NOs: 897, 902, 907, 912, 917, 922, 927, 932, 937, 942, 947, 952, 956.

In some embodiments, the fusion protein comprises an antigen binding domain that binds CD123. In some embodiments, the fusion protein comprises an antigen binding domain comprising an amino acid sequence selected from any one of SEQ ID NOs: 751, 756, 761, or 766. In some embodiments, the fusion protein comprises a chimeric antigen receptor comprising an amino acid sequence selected from any one of SEQ ID NOs: 750, 755, 760, or 765.

In some embodiments, the fusion protein comprises an antigen binding domain that binds BCMA. In some embodiments, the fusion protein comprises an antigen binding domain comprising an amino acid sequence selected from any one of SEQ ID NOs: 382, 386, 390, 394, 398, 402, 406, 410, 414, 418, 422, 426, 430, 434, 438, 442, 446, 450, 454, 458, 462, 466, 470, 474, 478, 482, 486, 490, 494, 498, 502, 506, 510, 514, 518, 522, 528, 531, 534, or 537. In some embodiments, the fusion protein comprises a chimeric antigen receptor comprising an amino acid sequence selected from any one of SEQ ID NOs: 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, or 859.

In some embodiments, the fusion protein comprises an antigen binding domain that binds CD20. In some embodiments, the fusion protein comprises an antigen binding domain comprising an amino acid sequence located at positions 470-712 or 470-939 of SEQ ID NO: 3033. In some embodiments, the fusion protein comprises a chimeric antigen receptor comprising an amino acid sequence of SEQ ID NO: 3033.

### Cells, nucleic acids and method of making the fusion proteins

In another aspect, the invention features a cell, e.g., a host cell including any one of the foregoing fusion proteins of the invention. In some embodiments, the cell, e.g., the host cell, is an immune cell, e.g., an immune effector cell. In some embodiments, the cell is a T cell or an NK cell.

In yet another aspect, the invention features a nucleic acid (e.g., an mRNA or DNA molecule) encoding any one of the foregoing fusion proteins of the invention. In another aspect, the invention features a vector (e.g., a viral vector (such as a lentiviral vector)) containing such a nucleic acid. The invention also features a viral particle including such a viral vector.

In yet another aspect, the invention features a cell, e.g., host cell (e.g., a human T cell) containing any of the foregoing vectors, nucleic acids, or fusion proteins of the invention.

In certain embodiments, the cell further includes a protease capable of cleaving the heterologous protease cleavage site. In certain embodiments, the host cell can further include a stabilization compound (e.g., Bazedoxifene, Shield1 or 1 µM 4-OHT (4-hydroxy tamoxifen)) wherein said degradation domain assumes a conformation permissive to cellular degradation in the absence of said stabilization compound.

In some embodiments, in the absence of an expression compound, e.g., a stabilization compound, the fusion protein is degraded by cellular degradation pathways, e.g., at least 50%, 60%, 70%, 80%, 90% or greater of the fusion protein is degraded.

In some instances disclosed herein which do not form part of the claimed invention, in the absence of an expression compound, e.g., deaggregation compound, the fusion protein is in an aggregated state in the cell, e.g., in the endoplasmic reticulum or the cytosol, e.g., at least 50%, 60%, 70%, 80%, 90% or greater is in the aggregated state.

In some embodiments, said cell further comprises an expression compound, e.g., a stabilization compound.

In some embodiments, the conditional expression domain, which is a degradation domain, assumes a conformation more resistant to cellular degradation in the presence of the expression compound, e.g., stabilization compound, relative to a conformation in the absence of the expression compound.

In some embodiments, the conformation of the fusion protein is more permissive to cleavage at the heterologous protease cleavage site in the presence of the expression compound, e.g., stabilization compound, relative to a conformation in the absence of the expression compound.

In some embodiments, the level of cell surface expression or extracellular expression of the fusion protein is greater, e.g., 2, 3, 4, 5, 6, 7, 8, 9, 10, 20, or 30 fold greater, than the level of cell surface expression or extracellular expression of the fusion protein in a cell not comprising an expression compound, e.g., a stabilization compound.

In yet another aspect, a method of making a fusion protein as described herein is disclosed. The method includes providing a cell, e.g., a host cell as described herein, e.g., a host cell comprising any of the foregoing vectors, nucleic acids, or fusion proteins, under conditions suitable for expression.

In another aspect, the invention also features a method of conditionally expressing a protein of interest.

In the present invention, the protein of interest is a transmembrane protein which is a CAR.

In some embodiments, the invention also features a method for conditionally expressing a CAR on the surface of a cell (e.g., an immune cell, e.g., a host cell). The method includes:
providing a cell, e.g., an immune cell (e.g., a host cell) comprising a fusion protein or a nucleic acid encoding the fusion protein of the invention;
contacting the fusion protein or the cell comprising said fusion protein with an expression compound, wherein:
   (a) in the presence of said expression compound, the surface expression of said protein of interest, transmembrane protein, or CAR is increased, e.g., 2, 3, 4, 5, 6, 7, 8, 9, 10, 20, or 30 fold greater, relative a reference value, e.g., relative to the level of surface expression of said protein of interest, transmembrane protein, or CAR in the absence of said expression compound; and
   (b) in the absence of said expression compound, the surface expression of said protein of interest, transmembrane protein, or CAR is substantially decreased, e.g., e.g., 2, 3, 4, 5, 6, 7, 8, 9, 10, 20, or 30 fold less, relative a reference value, e.g., relative relative to the level of surface expression of said protein of interest, transmembrane protein, or CAR in the presence of the expression compound.

In some embodiments, the presence of said expression compound is associated with, e.g., causes, a change in conformation of the conditional expression domain from a first folding state to second folding state, wherein the first folding state is more susceptible to degradation, e.g., cellular degradation, or aggregation relative to the second folding state.

In some embodiments, the presence of said expression compound exposes the heterologous protease cleavage site, e.g., to a greater extent, e.g., 2, 3, 4, 5, 6, 7, 8, 9, 10, 20, or 30 fold greater, relative the exposure of the protease cleavage site in the absence of said expression compound.

In some embodiments, the invention also features a method of conditionally expressing a CAR, said method comprising contacting a cell, e.g., a host cell and/or a cell described herein, with a stabilization compound, wherein:
(a) in the presence of said stabilization compound,
   (i) said degradation domain assumes a conformation more resistant to cellular degradation relative to a conformation in the absence of the stabilization compound,
   thereby resulting in cleavage of said degradation domain from said protein of interest, transmembrane protein, or CAR and the expression of said protein of interest, transmembrane protein, or CAR; and
(b) in the absence of said stabilization compound, said degradation domain assumes a conformation more permissive to cellular degradation relative to a conformation in the presence of stabilization compound, thereby resulting in degradation of said protein of interest, transmembrane protein, or CAR.

The invention provides a method of conditionally expressing a CAR on the surface of a cell, said method comprising: contacting a fusion protein of the invention, or a cell of the invention, with a stabilization compound, wherein:
(a) in the presence of said stabilization compound, the surface expression of said CAR is increased, e.g., 2, 3, 4, 5, 6, 7, 8, 9, 10, 20, or 30 fold greater, relative to the level of surface expression of said CAR in the absence of said stabilization compound; and
(b) in the absence of said stabilization compound, the surface expression of said CAR is substantially decreased, e.g., 2, 3, 4, 5, 6, 7, 8, 9, 10, 20, or 30 fold less, relative to the level of surface expression of said CAR in the presence of the stabilization compound.

In some embodiments, said cell is contacted with said stabilization compound ex vivo.

In some instances, said cell is contacted with said stabilization compound in vivo.

In some instances disclosed herein which do not form part of the claimed invention, the disclosure also features a method of conditionally expressing a protein of interest, transmembrane protein, or CAR, said method comprising contacting a cell, e.g., a host cell and/or a cell described herein, with a deaggregation compound, wherein:
(a) in the presence of said deaggregation compound,
   (i) said aggregation domain assumes a conformation more resistant to aggregation or oligomerization relative to a conformation in the absence of the deaggregation compound,
   thereby resulting in cleavage of said aggregation domain from said protein of interest, transmembrane protein, or CAR and the expression of said protein of interest, transmembrane protein, or CAR; and
(b) in the absence of said deaggregation compound, said aggregation domain assumes a conformation more permissive to aggregation or oligomerization relative to a conformation in the presence of the deaggregation compound, thereby resulting in aggregation of said protein of interest, transmembrane protein, or CAR.

In some instances, said cell is contacted with said deaggregation compound ex vivo.

In some instances, said cell is contacted with said deaggregation compound in vivo.

In yet another aspect, the invention features cells for use in a method of treating a subject having a disease associated with expression of a tumor antigen, including administering to the subject an effective amount of any of the host cells of the invention, wherein the second protein is a chimeric antigen receptor and includes, in a N-terminal to C-terminal direction, an antigen binding domain, a transmembrane domain, and one or more intracellular signaling domains and the antigen binding domain specifically binds the tumor antigen. In yet another aspect, the disclosure features a method of treating an autoantibody or alloantibody disease or condition, the method comprising administering to the subject an effective amount of the foregoing host cell, wherein said second protein is a chimeric antigen receptor and comprises, in a N-terminal to C-terminal direction, an antigen binding domain, a transmembrane domain, and one or more intracellular signaling domains and said antigen binding domain specifically binds an antigen specific of said autoantibody or alloantibody disease. In such methods, the host cell can be either autologous or non-autologous, e.g., allogeneic, to the subject. Such methods can further include the step of contacting the host cell, in vivo or ex vivo, with the foregoing stabilization compounds.

In some embodiments, the cell is contacted with an expression compound, and:
(a) in the presence of said expression compound,
   (i) said conditional expression domain assumes a conformation more resistant to cellular degradation relative to a conformation in the absence of said expression compound, thereby resulting in cleavage of said conditional expression domain from said chimeric antigen receptor (CAR) and the expression of said CAR; and
(b) in the absence of said expression compound, said conditional expression domain assumes a conformation more permissive to cellular degradation relative to a conformation in the presence of said expression compound, thereby resulting in degradation or aggregation of said fusion protein.

In some embodiments, the cell, e.g., host cell, is contacted with a stabilization compound, and:
(a) in the presence of said stabilization compound,
   (i) said degradation domain assumes a conformation more resistant to cellular degradation relative to a conformation in the absence of said stabilization compound,
   thereby resulting in cleavage of said degradation domain from said chimeric antigen receptor (CAR)and the expression of said CAR; and
(b) in the absence of said stabilization compound, said degradation domain assumes a conformation more permissive to cellular degradation relative to a conformation in the presence of said stabilization compound, thereby resulting in degradation of said fusion protein.

In some embodiments, said stabilization compound is selected from Bazedoxifene or 4-hydroxy tamoxifen (4-OHT) when the fusion protein comprises a degradation domain derived from estrogen receptor.

In some embodiments, said stabilization compound is Shield-1 when the fusion protein comprises a degradation domain derived from an FKB protein.

In some instances disclosed herein which do not form part of the claimed invention, the cell is contacted with a deaggregation compound, and:
(a) in the presence of said deaggregation compound,
   (i) said aggregation domain assumes a conformation more resistant to aggregation or oligomerization relative to a conformation in the absence of said deaggregation compound,
   thereby resulting in cleavage of said aggregation domain from said chimeric antigen receptor (CAR) and the expression of said CAR; and
(b) in the absence of said deaggregation compound, said aggregation domain assumes a conformation more permissive to aggregation or oligomerization relative to a conformation in the presence of said deaggregation compound, thereby resulting in aggregation of said fusion protein.

In some instances, said deaggregation compound is selected from FK506, rapamycin, AP22542, and AP21998 when the fusion protein comprises an aggregation domain derived from FKB protein (FKBP), e.g., FKBP F36M.

In some instances, the autoantibody disease or condition is selected from the group consisting of bullous pemphigoid, epidermolysis bullosa acquisita, p200 pemphigoid, linear IgA bullous dermatosis, other pemphigoid group diseases, dermatitis herpetiformis, celiac disease, myasthenia gravis, Goodpasture's syndrome, granulomatosis with polyangiitis and other ANCA+ vasculitidies, autoimmune limbic encephalitis, anti-N-methyl-D-aspartate receptor encephalitis, neuromyelitis optica, autoimmune hemolytic anemia, autoantibody-associated end-organ damage in lupus and other connective tissue diseases (due to anti-dsDNA, anti-Ro, and other autoantibodies), Graves` and Hashimoto's thyroiditis, anti-insulin antibodies in diabetes, anti-insulin receptor antibodies in autoimmune hypoglycemia, cryoglobulinemia, rheumatoid arthritis, multiple sclerosis, Sjogren's syndrome, dermatomyositis, anti-Fc-epsilon receptor antibodies in chronic idiopathic urticaria, anti-folate receptor antibodies, anti-endothelial receptor or anti-adrenergic receptor antibodies in pulmonary arterial hypertension, refractory hypertension, dilated cadiomyopathy, an autoinflammatory syndrome, neuromyelitis optica, Goodpasture's syndrome, anti-NMDAR encephalitis, AIHA, ITP, TTP, Graves'/Hashimoto's disease, Primary biliary cirrhosis, Neonatal lupus, Maternal autoabs causing T cell destruction, Pulmonary alveolar proteinosis, Anti-folate receptor, Chronic inflammatory demyelinating polyneuropathy, and Idiopathic membranous nephropathy. In some instances, the alloantibody disease or condition is an immune reaction in response to an organ transplant, blood transfusion, pregnancy, or protein replacement therapy.

In some embodiments, the cancer is mesothelioma (e.g., malignant pleural mesothelioma), e.g., in a subject who has progressed on at least one prior standard therapy; lung cancer (e.g., non-small cell lung cancer, small cell lung cancer, squamous cell lung cancer, or large cell lung cancer); pancreatic cancer (e.g., pancreatic ductal adenocarcinoma, or metastatic pancreatic ductal adenocarcinoma (PDA), e.g., in a subject who has progressed on at least one prior standard therapy); esophageal adenocarcinoma, ovarian cancer (e.g., serous epithelial ovarian cancer, e.g., in a subject who has progressed after at least one prior regimen of standard therapy), breast cancer, colorectal cancer, bladder cancer or any combination thereof.

In some embodiments, the disease associated with expression of a tumor antigen is a cancer.

In some embodiments, the disease associated with expression of the tumor antigen is a hematological cancer, e.g., a hematological cancer chosen from a leukemia or lymphoma.

In some embodiments, the cancer is chosen from: chronic lymphocytic leukemia (CLL), mantle cell lymphoma (MCL), multiple myeloma, acute lymphoid leukemia (ALL), Hodgkin lymphoma, B-cell acute lymphoid leukemia (BALL), T-cell acute lymphoid leukemia (TALL), small lymphocytic leukemia (SLL), B cell prolymphocytic leukemia, blastic plasmacytoid dendritic cell neoplasm, Burkitt's lymphoma, diffuse large B cell lymphoma (DLBCL), DLBCL associated with chronic inflammation, chronic myeloid leukemia, myeloproliferative neoplasms, follicular lymphoma, pediatric follicular lymphoma, hairy cell leukemia, small cell- or a large cell-follicular lymphoma, malignant lymphoproliferative conditions, MALT lymphoma (extranodal marginal zone lymphoma of mucosa-associated lymphoid tissue), Marginal zone lymphoma, myelodysplasia and myelodysplastic syndrome, non-Hodgkin lymphoma, plasmablastic lymphoma, plasmacytoid dendritic cell neoplasm, Waldenstrom macroglobulinemia, splenic marginal zone lymphoma, splenic lymphoma/leukemia, splenic diffuse red pulp small B-cell lymphoma, hairy cell leukemia-variant, lymphoplasmacytic lymphoma, a heavy chain disease, plasma cell myeloma, solitary plasmocytoma of bone, extraosseous plasmocytoma, nodal marginal zone lymphoma, pediatric nodal marginal zone lymphoma, primary cutaneous follicle center lymphoma, lymphomatoid granulomatosis, primary mediastinal (thymic) large B-cell lymphoma, intravascular large B-cell lymphoma, ALK+ large B-cell lymphoma, large B-cell lymphoma arising in HHV8-associated multicentric Castleman disease, primary effusion lymphoma, B-cell lymphoma, acute myeloid leukemia (AML), or unclassifiable lymphoma.

In some embodiments, the cancer is chosen from MCL, CLL, ALL, Hodgkin lymphoma, AML, or multiple myeloma.

In yet another aspect, the invention features a fusion protein, cell, nucleic acid, viral particle, or vector of the invention for use as a medicament.

In yet another aspect, the invention features a fusion protein, cell, nucleic acid, or vector of the invention for use in the treatment of a disease expressing a tumor antigen.

### Methods and compositions for use in treating autoantibody or alloantibody disease

Also disclosed herein but not forming part of the invention as claimed are methods of treating an autoantibody or alloantibody disease or condition in a subject in need thereof comprising administering an effective amount of a pharmaceutical composition comprising a modified T cell to the subject, wherein the modified T cell comprises a nucleic acid comprising a suicide gene and a nucleic acid encoding a chimeric antigen receptor (CAR) comprising an anti-B cell binding domain, a transmembrane domain, a costimulatory domain and an intracellular signaling domain.In another aspect, the disclosure features methods of treating an autoantibody or alloantibody disease or condition in a subject in need thereof comprising administering an effective amount of a pharmaceutical composition comprising a modified T cell to the subject, wherein the modified T cell comprises a nucleic acid encoding a dimerization domain and a chimeric antigen receptor (CAR) comprising an anti-B cell binding domain, a transmembrane domain, a costimulatory domain and an intracellular signaling domain.

In some instances disclosed herein which do not form part of the claimed invention, the suicide gene encodes the amino acid sequence selected from the group consisting of SEQ ID NOs: 3005-3007.

In some instances, the suicide gene further comprises a dimerization domain comprising the amino acid sequence selected from the group consisting of SEQ ID NO: 3013 and 3014.

In some instances, the dimerization domain comprises the amino acid sequence of SEQ ID NO: 980.

In some instances, the dimerization domain further comprises a furin cleavage site comprising the amino acid sequence of SEQ ID NO: 980.

In some instances, the CAR further comprises a signal peptide.

In some instances, the signal peptide comprises the amino acid sequence of SEQ ID NO: 3035.

In some instances, administering the effective amount comprises activating the modified T cell to effect cytotoxic function against B cells.

In some instances, the method further comprises activating a suicide gene product of the suicide gene to induce cell death of the modified T cell.

In some instances, activating the suicide gene product further comprises administering a dimerization agent to promote dimerization of the suicide gene product.

In some instances, activating the suicide gene product occurs after the modified T cell exerts cytotoxic function against B cells.

In some instances, activating the suicide gene product occurs after an onset of an adverse reaction in the subject to the modified T cell.

In some instances, the method further comprises repressing activation of a suicide gene product of the suicide gene to repress cell death of the modified T cell.

In some instances, repressing activation of the suicide gene product further comprises administering a solubilizing agent to prevent dimerization of the suicide gene product.

In some instances, administering the solubilizing agent occurs concurrently with administration of the modified T cell and continues as the modified T cell exerts cytotoxic function against B cells.

In some instances, administering the solubilizing agent is ceased after an onset of an adverse reaction in the subject to the modified T cell.

In some instances, the anti-B cell binding domain of the CAR comprises an antibody selected from the group consisting of a monoclonal antibody, a polyclonal antibody, a synthetic antibody, human antibody, humanized antibody, single domain antibody, single chain variable fragment, and antigen-binding fragments thereof.

In some instances, the anti-B cell binding domain of the CAR specifically binds a B cell marker selected from the group consisting of CD19, BCMA, CD20, CD21, CD27, CD38, CD138 and any combination thereof.

In some instances, the anti-B cell binding domain of the CAR specifically binds a B cell marker selected from the group consisting of CD20, CD21, CD27, CD38, CD138, any combination thereof, and at least one surface marker selectively found on a pro-B cell, pre-B cell, immature B cell, mature B cell, memory B cell, and plasma cell.

In some instances, the intracellular domain of the CAR comprises dual signaling domains.

In some instances, the costimulatory domain is selected from the group consisting of CD3, CD27, CD28, CD83, CD86, CD127, 4-1BB, 4-1BBL, PD1, PD1L, T cell receptor (TCR), any derivative or variant thereof, any synthetic sequence thereof that has the same functional capability, and any combination thereof.

In some instances, the method further comprises administering a solubilizing agent to prevent dimerization of the CAR.

In some instances, administering the solubilizing agent occurs concurrently with administration of the modified T cell and continues as the modified T cell exerts cytotoxic function against B cells.

In some instances, administering the solubilizing agent is ceased after an onset of an adverse reaction in the subject to the modified T cell.

In some instances, the autoantibody disease or condition is selected from the group consisting of bullous pemphigoid, epidermolysis bullosa acquisita, p200 pemphigoid, linear IgA bullous dermatosis, other pemphigoid group diseases, dermatitis herpetiformis, celiac disease, myasthenia gravis, Goodpasture's syndrome, granulomatosis with polyangiitis and other ANCA+ vasculitidies, autoimmune limbic encephalitis, anti-N-methyl-D-aspartate receptor encephalitis, neuromyelitis optica, autoimmune hemolytic anemia, autoantibody-associated end-organ damage in lupus and other connective tissue diseases (due to anti-dsDNA, anti-Ro, and other autoantibodies), Graves` and Hashimoto's thyroiditis, anti-insulin antibodies in diabetes, anti-insulin receptor antibodies in autoimmune hypoglycemia, cryoglobulinemia, rheumatoid arthritis, multiple sclerosis, Sjogren's syndrome, dermatomyositis, anti-Fc-epsilon receptor antibodies in chronic idiopathic urticaria, anti-folate receptor antibodies, anti-endothelial receptor or anti-adrenergic receptor antibodies in pulmonary arterial hypertension, refractory hypertension, dilated cadiomyopathy, and an autoinflammatory syndrome.

In some instances, the alloantibody disease or condition is an immune reaction in response to an organ transplant, blood transfusion, pregnancy, and protein replacement therapy.

In some instances, the modified T cell is further modified by deleting a gene selected from the group consisting of a T cell receptor (TCR) chain, a major histocompatibility complex protein, and any combination thereof.

In some instances, the modified T cell is further modified before administration to the subject in need thereof.

In some instances, the modified T cell is further modified by inducing a CRISPR/Cas system.

Also disclosed herein but not forming part of the invention as claimed is a pharmaceutical composition formulated for use in a method described herein, the composition comprising a modified T cell comprising a nucleic acid encoding a suicide gene and a nucleic acid encoding a chimeric antigen receptor (CAR) comprising an anti-B cell binding domain, a transmembrane domain, a costimulatory domain and an intracellular signaling domain.

Also disclosed herein but not forming part of the invention as claimed is a pharmaceutical composition formulated for use in a method described herein, the composition comprising a modified T cell comprising a nucleic acid encoding a dimerization domain and a chimeric antigen receptor (CAR) comprising an anti-B cell binding domain, a transmembrane domain, a costimulatory domain and an intracellular signaling domain.

In some instances, the suicide gene encodes the amino acid sequence selected from the group consisting of SEQ ID NOs: 3005-3007.

In some instances, the suicide gene further comprises a dimerization domain comprising the amino acid sequence selected from the group consisting of SEQ ID NOs: 3013 and 3014.

In some instances, the dimerization domain comprises the amino acid sequence of SEQ ID NO: 980.

In some instances, the dimerization domain further comprises a furin cleavage site comprising the amino acid sequence of SEQ ID NO: 980.

In some instances, the CAR further comprises a signal peptide.

In some instances, the signal peptide comprises the amino acid sequence of SEQ ID NO: 3035.

In some instances, the composition further comprises an inducing agent to induce activation of the suicide gene.

In some instances, the modified T cell lacks at least one gene encoding a T cell receptor (TCR) chain, and a major histocompatibility complex protein.

In one aspect, the disclosure features an isolated nucleic acid sequence comprising a nucleic acid sequence comprising (i) a suicide gene comprising a nucleic acid sequence selected from the group consisting of SEQ ID NOs: 3001-3004; and (ii) a nucleic acid sequence encoding a chimeric antigen receptor (CAR) comprising an anti-B cell binding domain, a transmembrane domain, a costimulatory domain and an intracellular signaling domain.

In some instances, the isolated nucleic acid sequence comprises SEQ ID NO: 3018, 3020, 3024, 3026, 3028 or 3030.

Also disclosed herein but not forming part of the invention as claimed is an isolated polypeptide comprising (i) an amino acid sequence encoded by a suicide gene wherein the amino acid sequence is selected from the group consisting of SEQ ID NOs: 3005-3007; and (ii) a chimeric antigen receptor (CAR) comprising an anti-B cell binding domain, a transmembrane domain, a costimulatory domain and an intracellular signaling domain.

In some instances, the isolated polypeptide comprises an amino acid sequence of SEQ ID NO: 3019, 3021, 3026, 3028, 3030 or 3034.

Also disclosed herein but not forming part of the invention as claimed is an isolated nucleic acid sequence comprising (i) a nucleic acid encoding a dimerization domain; and (ii) a chimeric antigen receptor (CAR) comprising an anti-B cell binding domain, a transmembrane domain, a costimulatory domain and an intracellular signaling domain.

In some instances, the dimerization domain comprises the amino acid sequence of SEQ ID NO: 980.

In some instances, the isolated nucleic acid sequence comprises SEQ ID NO: 977 or 3032.

Also disclosed herein but not forming part of the invention as claimed is an isolated polypeptide comprising (i) a dimerization domain; and (ii) a chimeric antigen receptor (CAR) comprising an anti-B cell binding domain, a transmembrane domain, a costimulatory domain and an intracellular signaling domain. In some instances, the isolated polypeptide comprises an amino acid sequence of SEQ ID NO: 978 or 3033.

### BRIEF DESCRIPTION OF THE DRAWINGS

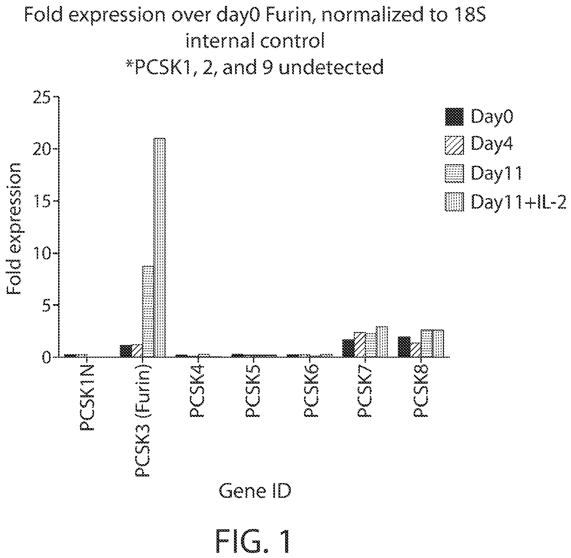

**FIG. 1** is a graph showing expression of PCSK (proprotein convertase) family members in primary human T cells. The expression of the PCSK family members was measured by qRT-PCR. RNA was harvested from normal donor T cells on days 0, 4, and 11 following stimulation with anti-CD3/anti-CD28 activation beads. An additional group was supplemented with 100 U/mL IL-2 during culture and RNA was harvested on day 11.

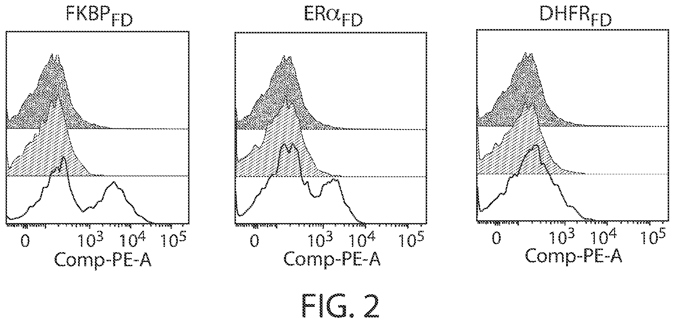

**FIG. 2** is a series of graphs showing compound-dependent CAR expression in Jurkat T cells transduced with an anti-CD19 scFv CAR construct fused to the indicated furin degradation domain (FKBP_{FD}, ERα_{FD}, or DHFR_{FD}) followed by treatment with the corresponding stabilizing compound. FKBP_{FD} transduced cells treated with 1µM Shield1; ERα_{FD} transduced cells treated with 1 µM Bazedoxifene; DHFR_{FD} transduced cells treated with 1mM Trimethoprim (TMP). Expression of the anti-CD19 scFv is inducible by the stabilizing compound. Black=UTD (untransduced cells); Gray=construct, no compound; White=construct, with compound.

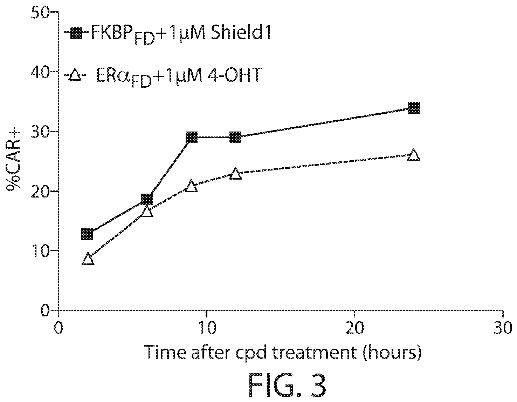

**FIG. 3** is a graph showing kinetics of CAR expression in Jurkat T cells transduced with an anti-CD19 scFv CAR construct fused to the indicated furin degradation domain (FKBP_{FD} or ERα_{FD}) following addition of a stabilization compound. The FKBP_{FD} transduced cells were treated with 1 µM Shield1 and the ERα_{FD} transduced cells were treated with 1 µM 4-OHT (4-hydroxy tamoxifen) for the time indicated; and CAR expression was determined by FACS.

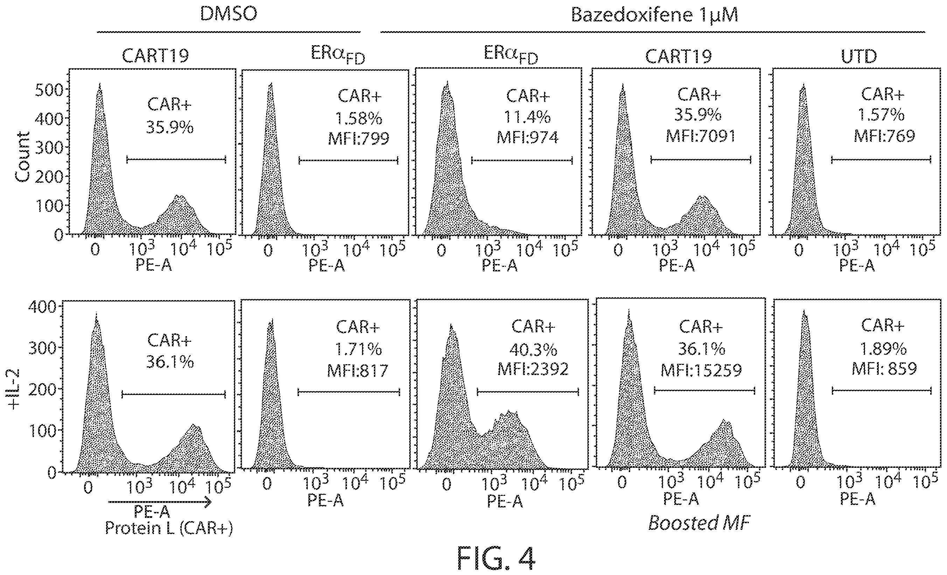

**FIG. 4** is a series of histograms showing a furin degron domain ERα_{FD} can regulate CAR19 expression in a Bazedoxifene -dependent manner in primary human T cells, and that stabilization is enhanced in the presence of IL-2 in vitro. Primary human T cells were transduced with ERα_{FD} domain fused to an anti-CD19 scFv CAR construct. 100 U/mL IL-2 was added on day 9 following activation with anti-CD3/CD28 stimulation beads. Bazedoxifene was added on day 10, and CAR expression was determined by FACS on day 11.

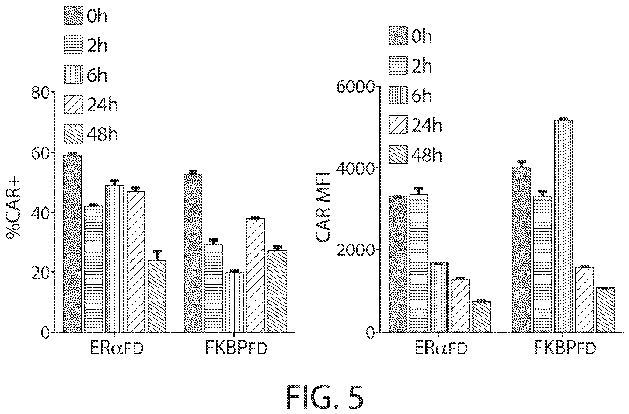

**FIG. 5** is a pair of graphs showing kinetics of CAR expression following compound washout in primary T cells transduced with CAR construct fused to a furin degradation domain. Primary human T cells were transduced with the indicated furin degradation domain (FKBP_{FD} or ERα_{FD}) fused to an anti-CD19 scFv CAR construct. 100 U/mL IL-2 was added on day 9 following activation with anti-CD3/CD28 stimulation beads. Bazedoxifene was added on day 10, and T cells were frozen on day 11. T cells were thawed, extensively washed, and placed in culture for the times indicated followed by determination of CAR expression by FACS.

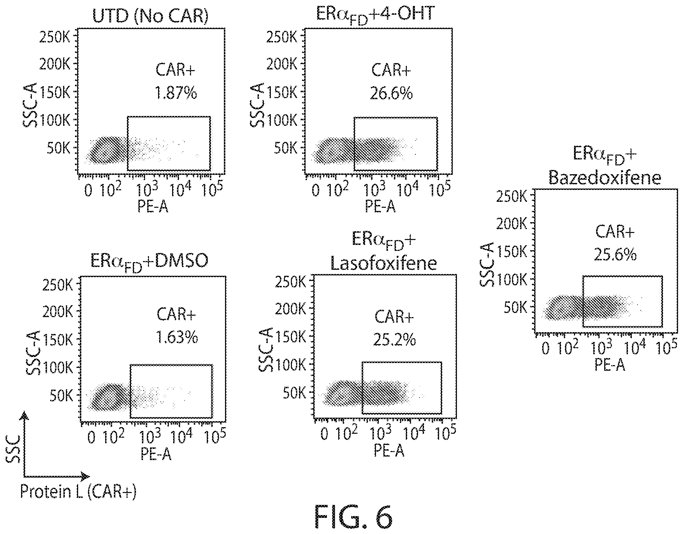

**FIG. 6** is a series of plots showing multiple ERα targeting drugs stabilize FurOn CARTs. Jurkat T cells were transduced with ERα_{FD} degradation domain fused to an anti-CD19 scFv CAR construct followed by treatment with the indicated compounds for 24hours. ERα targeting drugs used were: 10 µM 4-OHT, 1 µM Bazedoxifene, or 1 µM Lasofoxifene.

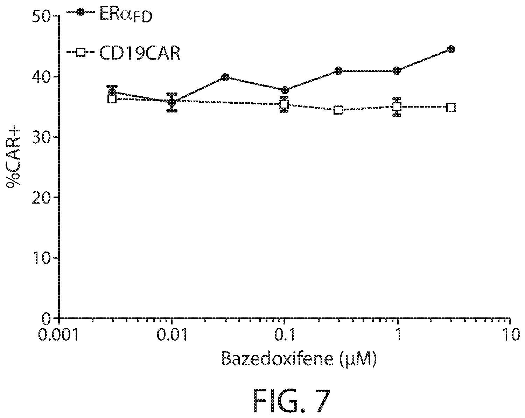

**FIG. 7** is a graph showing dose response of ERα_{FD} fused CAR expression to Bazedoxifene. Primary human T cells were transduced with ERα_{FD} degradation domain fused to an anti-CD19 scFv CAR construct or the parental CD19 CAR construct. 100 U/mL IL-2 was added on day 9 following activation with anti-CD3/CD28 stimulation beads, and T cells were frozen on day 11. T cells were thawed and placed in culture with Bazedoxifene for 48 hours at the concentrations indicated. CAR expression was determined by FACS.

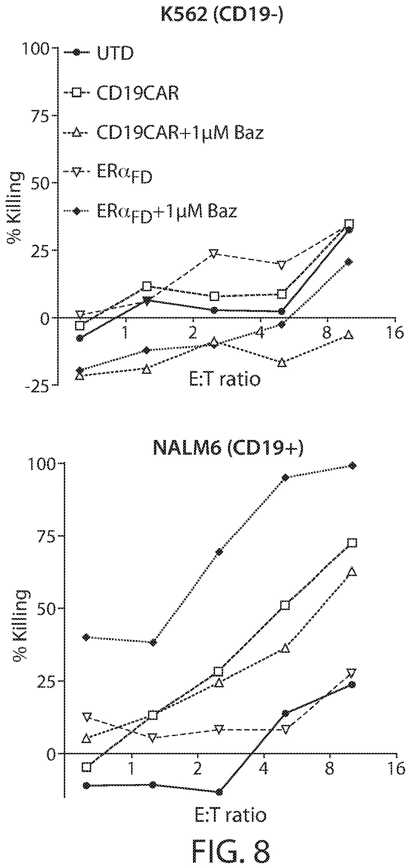

**FIG. 8** is a pair of graphs showing compound-dependent target specific cell killing by ER-alpha based FurON CART. Primary human T cells were transduced with ERα_{FD} degradation domain fused to an anti-CD19 scFv CAR construct or the parental CD19 CAR construct. 100 U/mL IL-2 and Bazedoxifene were added on day 9 following activation with anti-CD3/CD28 stimulation beads, and T cells were frozen on day 11. T cells were thawed and incubated for 20 hours with the indicated luciferized cell line targets, K562 (CD19-) or NALM6 (CD19+). Percent killing was determined by analysis of remaining luciferase activity.

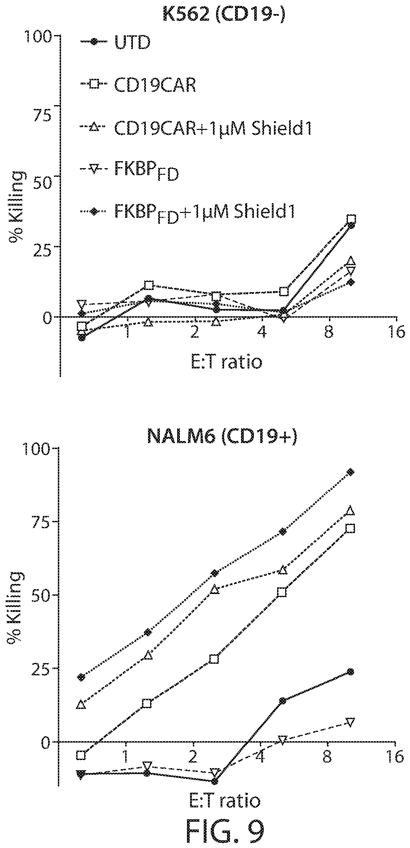

**FIG. 9** is a pair of graphs showing compound-dependent target specific cell killing by FKBP based FurON CART. Primary human T cells were transduced with FKBP_{FD} furin degradation domain fused to an anti-CD19 scFv CAR construct or the parental CD19 CAR construct. 100 U/mL IL-2 and Shield1 were added on day 9 following activation with anti-CD3/CD28 stimulation beads, and T cells were frozen on day 11. T cells were thawed and incubated for 20 hours with the indicated luciferized cell line targets, K562 (CD19-) or NALM6 (CD19+). Percent killing was determined by analysis of remaining luciferase activity.

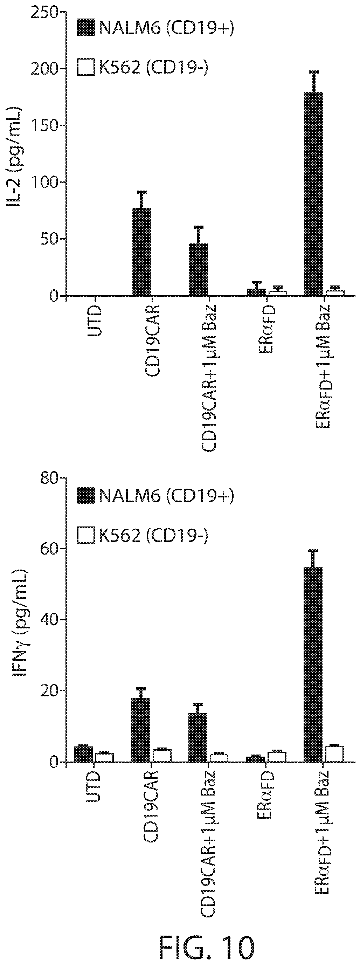

**FIG. 10** is a pair of graphs showing compound-dependent cytokine production of ER-alpha FurOn CART. Primary human T cells were transduced with ERα_{FD} furin degradation domain fused to an anti-CD19 scFv CAR construct or the parental CD19 CAR construct. 100 U/mL IL-2 and Bazedoxifene were added on day 9 following activation with anti-CD3/CD28 stimulation beads, and T cells were frozen on day 11. T cells were thawed and incubated with the indicated cell line targets for 20 hrs. Supernatants were harvested and analyzed by cytokine bead array.

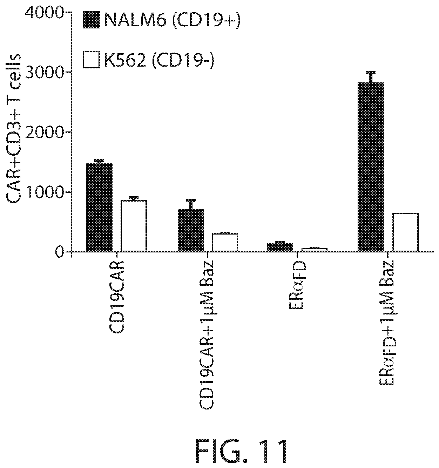

**FIG. 11** is a graph showing compound dependent proliferation of ER-alpha FurOn CART. Primary human T cells were transduced with ERα_{FD} degradation domain fused to an anti-CD19 scFv CAR construct or the parental CD19 CAR construct. 100 U/mL IL-2 and Bazedoxifene were added on day 9 following activation with anti-CD3/CD28 stimulation beads, and T cells were frozen on day 11. T cells were thawed and incubated with the indicated cell line targets for 4 days. T cell FurON-CAR numbers were analyzed by FACS.

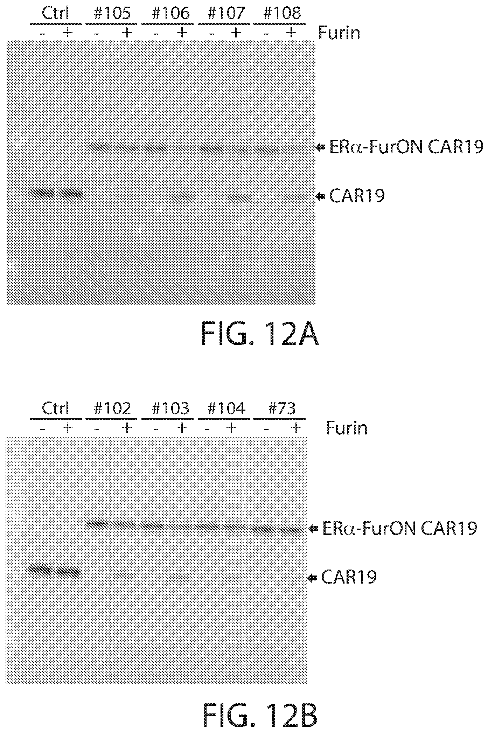

**FIGs. 12A-12B** are representative immunoblots showing the degree of furin cleavage across the various tested furin cleavage sites in ERα-FurON CAR19 constructs. The tested furin cleavage sites are: #105 - LQWLEQQVAKRRTKR (SEQ ID NO: 129); #106 - GTGAEDPRPSRKRRSLGDVG (SEQ ID NO: 125); #107 - GTGAEDPRPSRKRRSLGG (SEQ ID NO: 131); #108 - GTGAEDPRPSRKRRSLG (SEQ ID NO: 133); #102 - SLNLTESHNSRKKR (SEQ ID NO: 135); #103 - GTGAEDPRPSRKRR (SEQ ID NO: 127); #104 - CKINGYPKRGRKRR (SEQ ID NO: 137); #73 - RTKR (SEQ ID NO: 123).

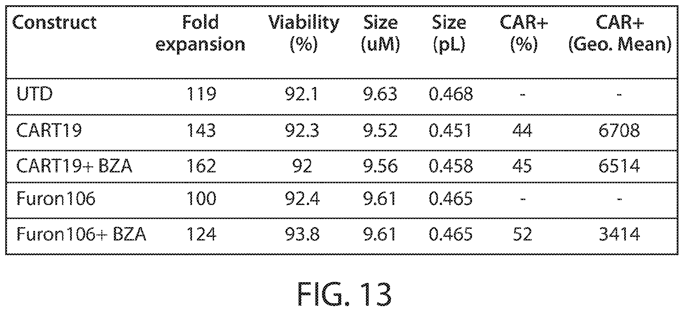

**FIG. 13** is a table showing the furon (furin degron) domain regulates CAR19 expression in a stabilizing compound-dependent manner in human primary T cells, but had no impact on cell viability and cell proliferation.

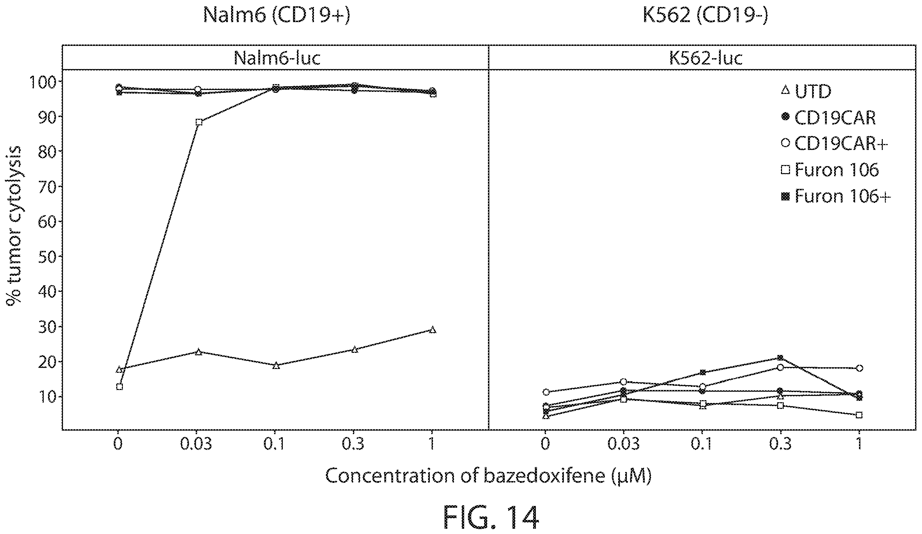

**FIG. 14** is a series of line graphs showing that CAR19 with the furin degron domain kills CD19+ tumor cells in a stabilizing compound dose-dependent manner and is noninferior to the parental CAR construct.

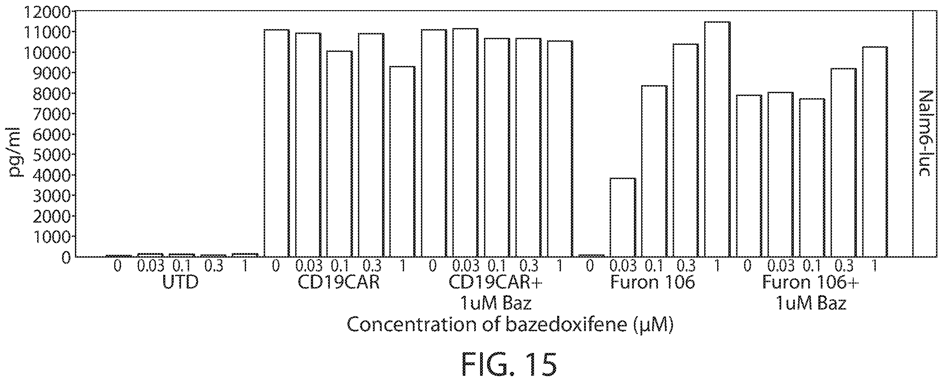

**FIG. 15** is a bar graph showing primary human T cells expressing ERαFurON CAR19 secrete IFNγ in the presence of CD19+ tumor cells, in a manner that is stabilizing compound dose-dependent and is noninferior to the parental CAR construct.

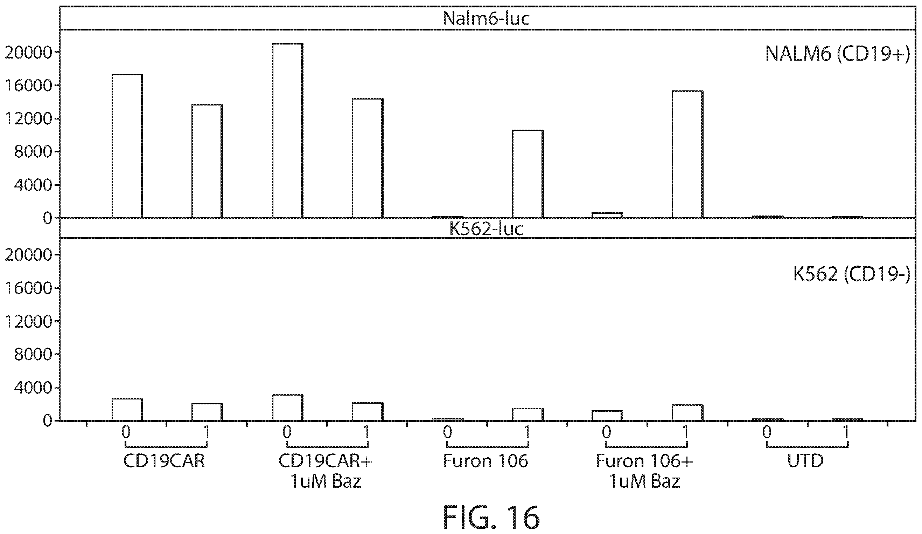

**FIG. 16** is a series of bar graphs showing primary human T cells expressing ERαFurON CAR19 proliferate in the presence of CD19+ tumor cells, in a manner that is stabilizing compound-dependent and is noninferior to the parental CAR19 construct.

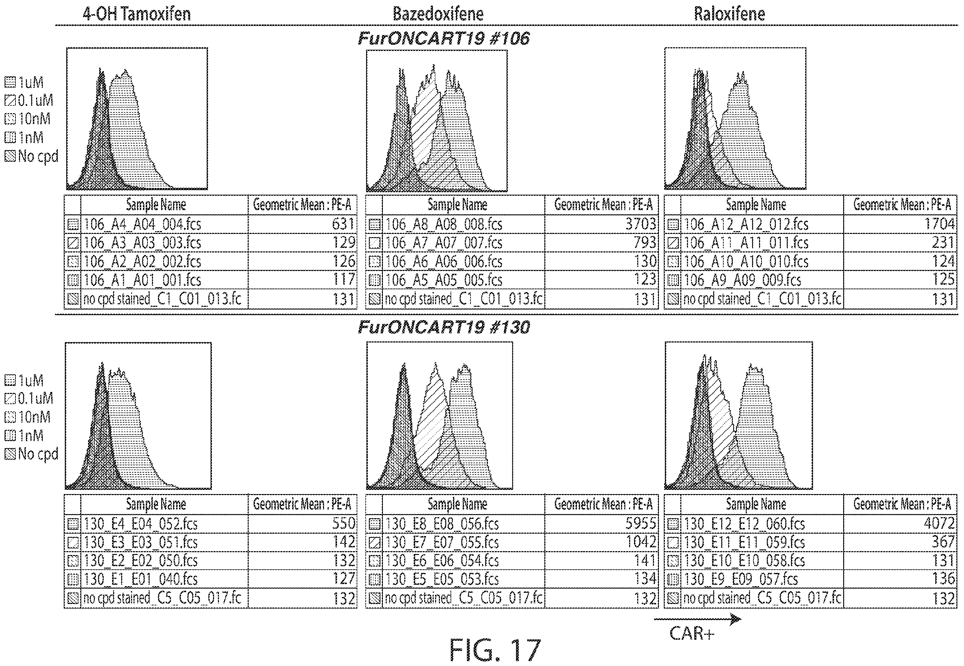

**FIG. 17** is a series of graphs showing that the tested FurON domains regulate CAR19 expression in Jurkat cells in a compound-dependent manner, regardless of the number of mutations; and no CAR surface expression is detected in the absence of stabilizing compound.

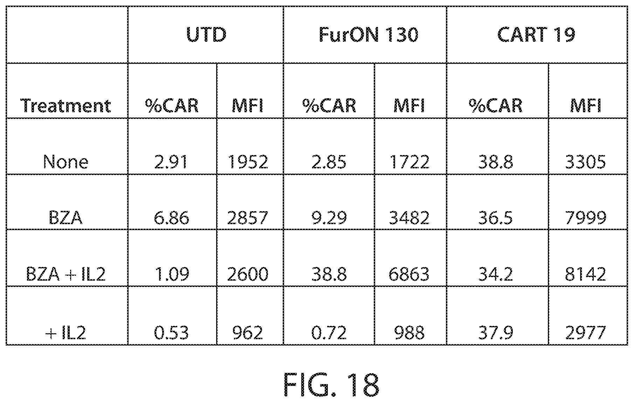

**FIG. 18** is a table showing that the FurON domain regulates CAR19 expression in a stabilizing compound- and IL-2-dependent manner in human primary T cells. No surface CAR expression is detected in the absence of the stabilizing compound bazedoxifene, when the FurON moiety is fused to CAR19.

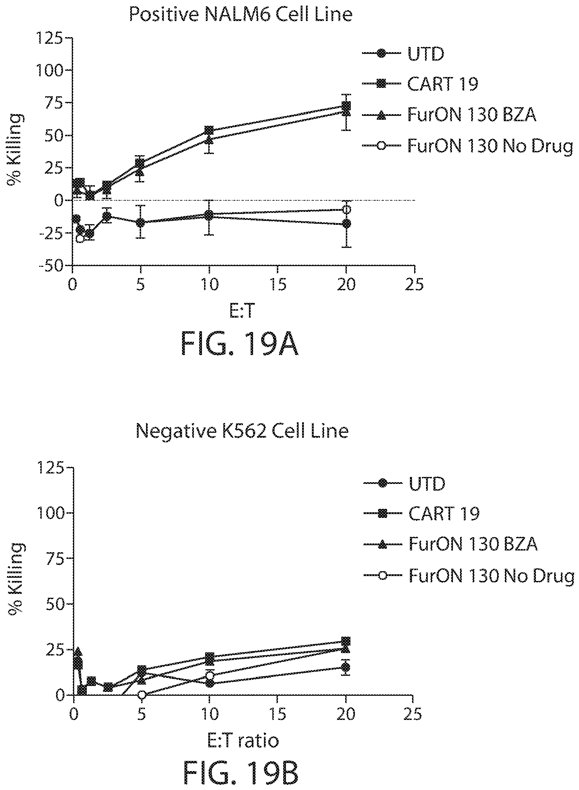

**FIGs. 19A-19B** are line graphs showing that CART cells expressing FurON CAR19, comprising a limited number of mutations in the degron domain, kill CD19+ tumor cells, in a stabilizing compound- and target-dependent manner and is noninferior to the parental CAR construct.

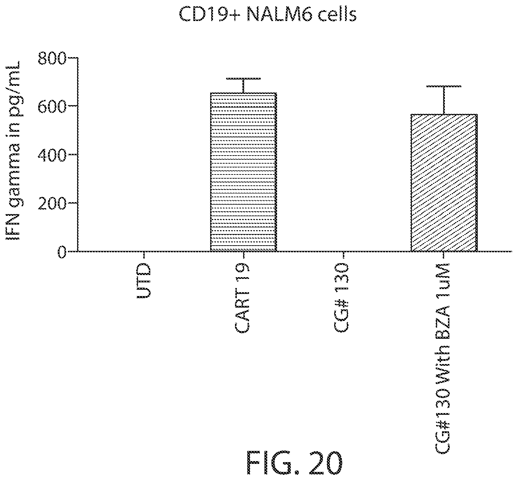

**FIG. 20** is a bar graph showing that CART cells expressing FurON CAR19, comprising a limited number of mutations in the degron domain, secrete cytokines in the presence of CD19+ tumor cells, in a manner that is stabilizing compound-dependent and is noninferior to the parental CAR construct.

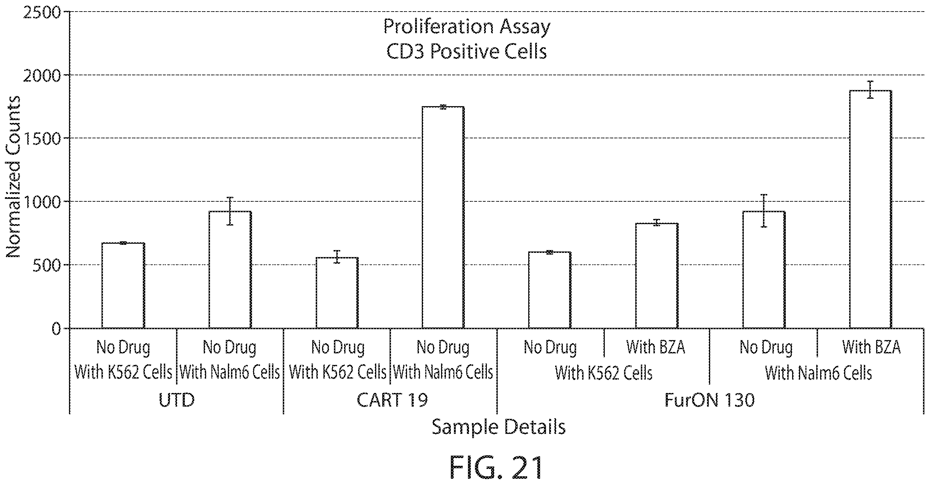

**FIG. 21** is a bar graph showing that CD3+ T cells comprising FurON CAR19 proliferate in the presence of CD19+ tumor cells, in a manner that is stabilizing compound-dependent and is noninferior to the parental CAR construct.

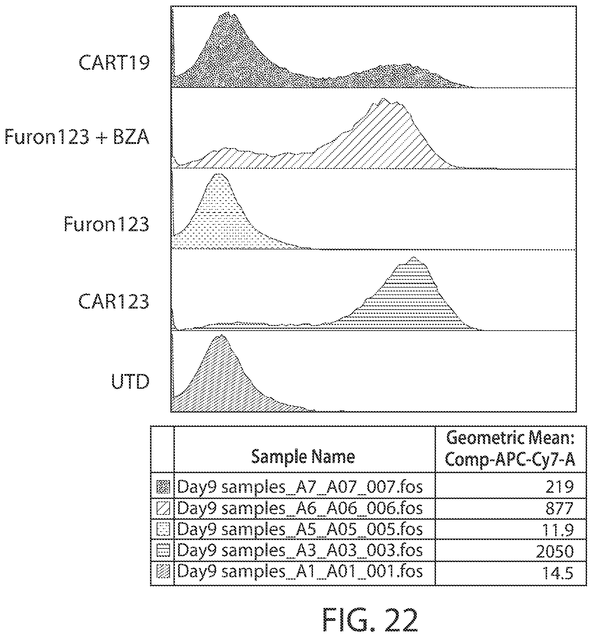

**FIG. 22** is a series of graphs showing that the FurON domain regulates CAR123 expression in primary human T cells, in a stabilizing compound-dependent manner.

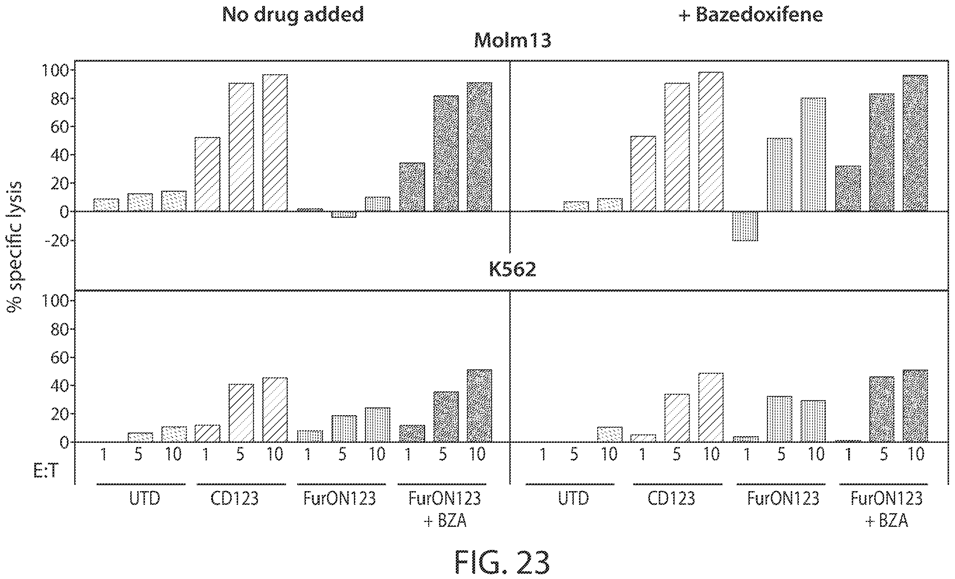

**FIG. 23** is a series of bar graphs showing that CART cells expressing FurON CAR123 kill CD123+ tumor cells, in a stabilizing compound- and target-dependent manner and is noninferior to the parental CAR construct.

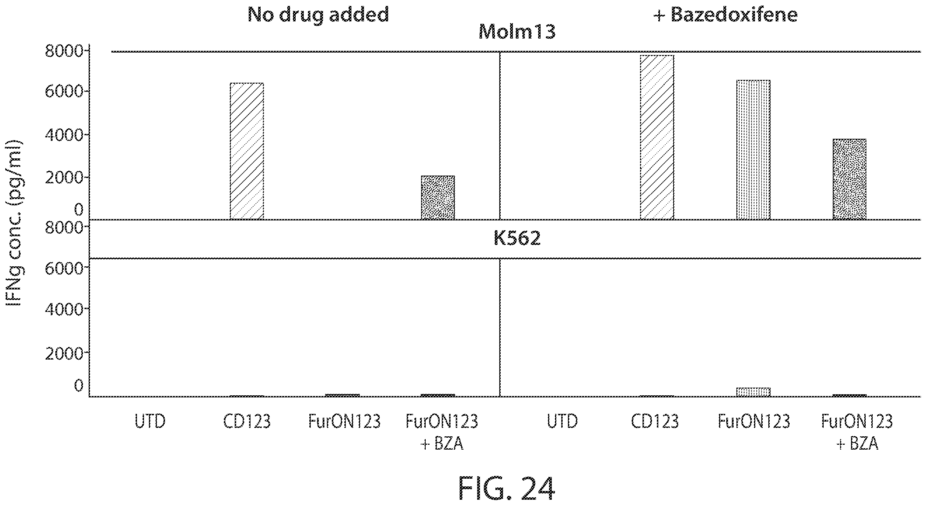

**FIG. 24** is a series of bar graphs showing that CART cells expressing FurON CAR123 secrete cytokines in the presence of CD123+ tumor cells, in a manner that is stabilizing compound-dependent and is noninferior to the parental CAR construct.
**FIG. 25** is a schematic showing an exemplary fusion protein comprising a degradation domain (degron), protease cleavage site, and a second protein domain (a CAR), and the change in degradation of the fusion protein in the presence of a drug, e.g., stabilization compound.
**FIG. 26** is a schematic showing an exemplary fusion protein comprising four copies of an aggregation domain (FKBP12F36M), a protease cleavage site (Furin site), and a second protein domain (ScFv with cytoplasmic tail), and the change in aggregation of the fusion protein in the presence of a compound, e.g., deaggregation compound. This figure is an exemplary illustration of a regulatory on-CAR system, which is yet another instance to control the cell surface expression and hence function of the CAR. The CAR is expressed downstream of modified FKBP12 domains, separated by a furin site. Without a solubilizing compound present, the CAR molecules spontaneously aggregate in the endoplasmic reticulum and a reduced number of CAR molecules, e.g., no CAR molecule, can egress to the surface of the T cell. This results in a reduced CAR mediated T cell function, e.g., no CAR mediated T cell function. When the solubilizing compound is present, the CAR aggregation domains are separated and aggregation is prevented. A furin site which is located N-terminal of the scFv becomes accessible for cleavage. After removal of the aggregation domains by furin in the late Golgi apparatus, CAR molecules egress to the surface of the T cells and CAR mediated T cell function can occur.

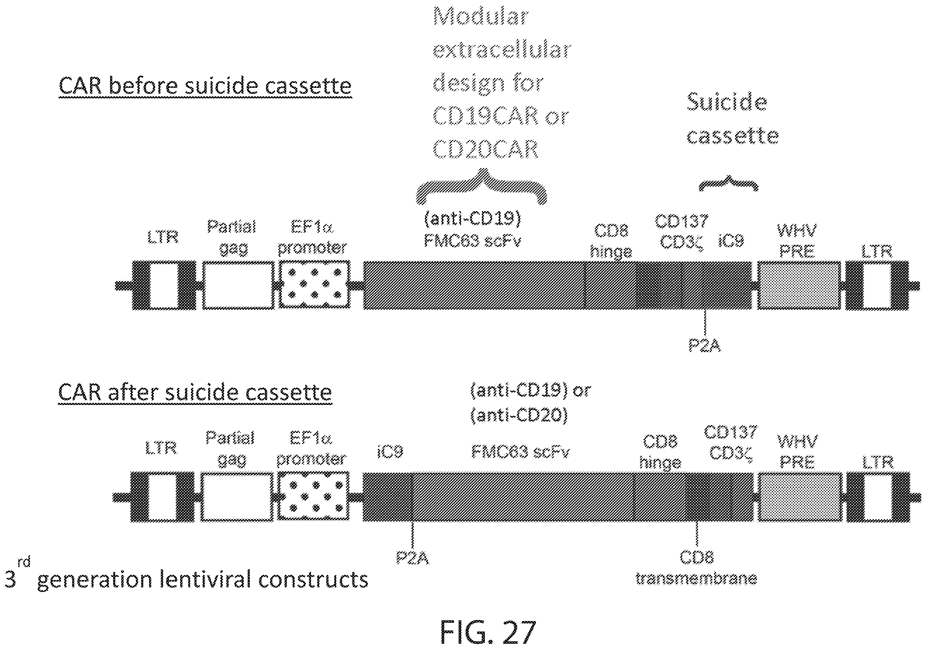

**FIG. 27** is an illustration of an anti-CD19 or anti-CD20 chimeric antigen receptor construct with a suicide switch (inducible caspase-9 or iCasp9 or iC9), collectively referred to as CD19 sCAR or CD20 sCAR, designed to cause complete but transient B cell depletion. CD19/CD20 sCAR T cells exert their therapeutic effect by depleting B cells upon infusion. Subsequent treatment with small molecule compounds allow caspase-9 to dimerize and activate caspase activity to induce suicide of CD19/CD20 sCAR T cells, thereby allowing B cell repopulation to occur.

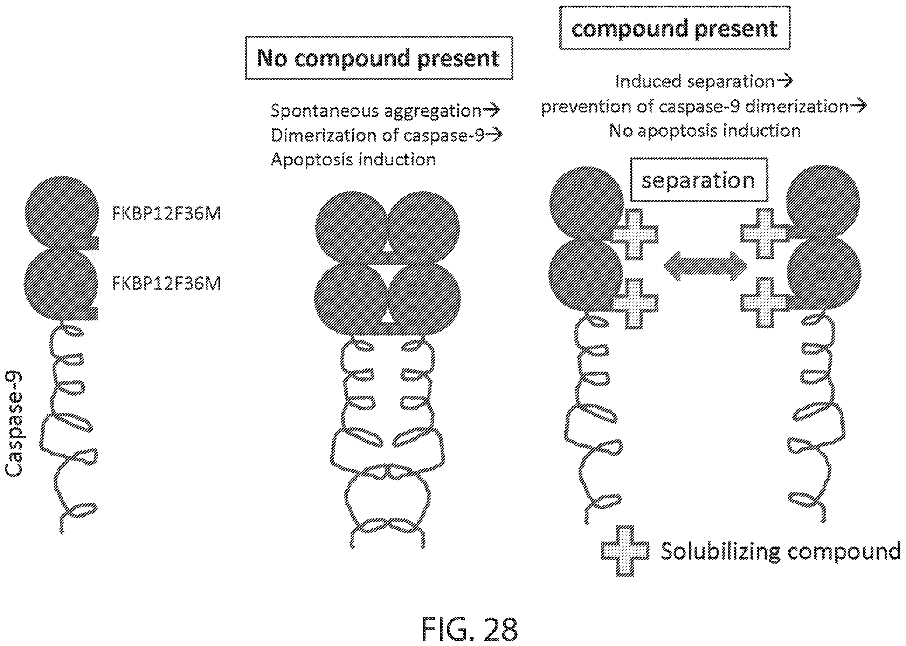

**FIG. 28** is an illustration of a reversible caspase-9 suicide cassette, which is another instance of a caspase-9 suicide cassette. In the CD19/CD20 revCAR system, caspase-9 is constitutively expressed and spontaneously dimerizes, thus inducing apoptosis as the default in CD19 revCAR T cells. In the presence of a small molecule compound that solubilizes the caspase-9 molecules (i.e., inhibits dimerization), caspase activity is inhibited. Thus, small molecule solubilizer treatment inhibits caspase-9 activity during expansion and infusion of revCAR T cells, and withdrawal of the small molecule solubilizer results in activation of caspase-9 activity and suicide of revCAR T cells, thereby allowing B cell repopulation to occur.

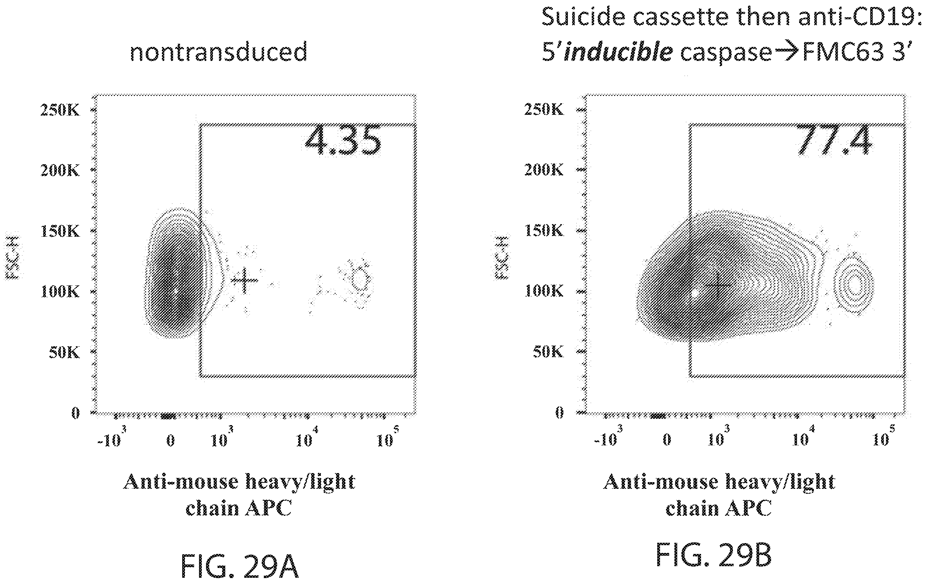

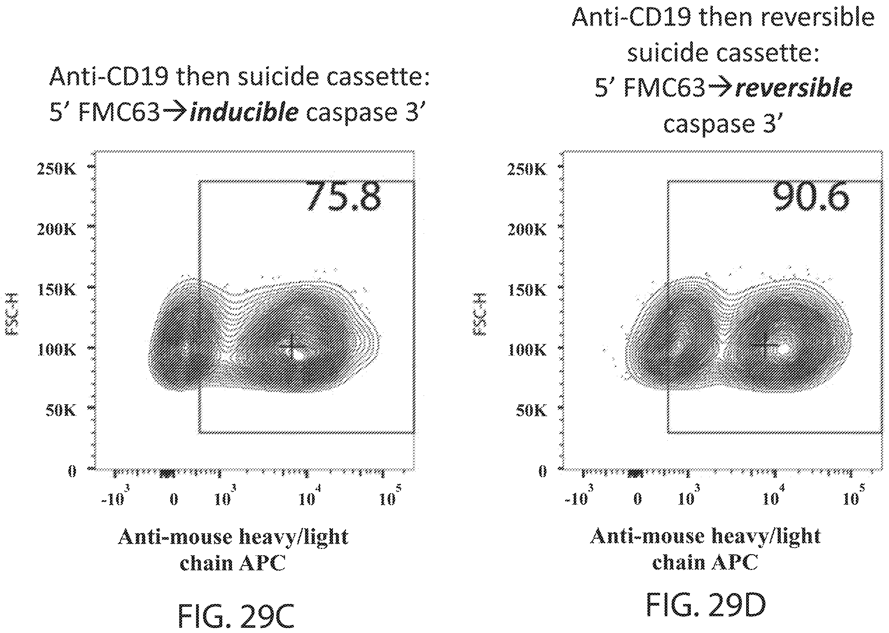

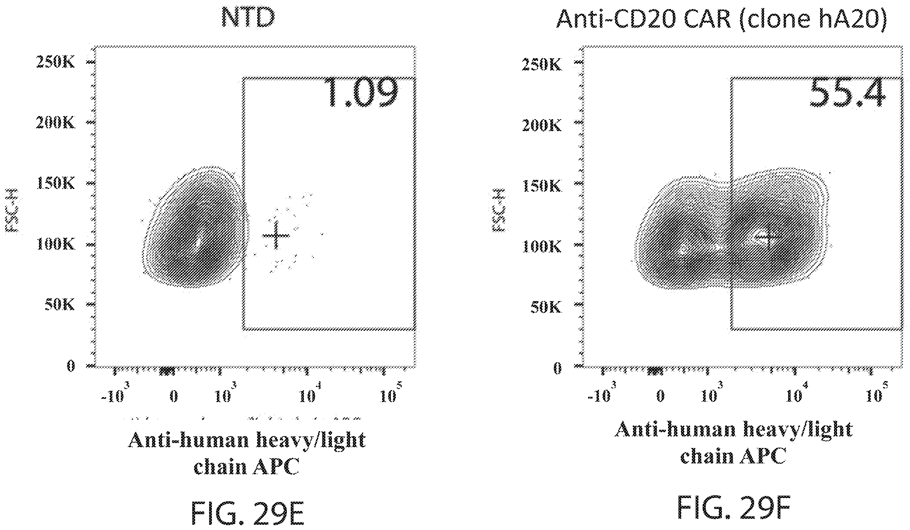

**FIGS. 29A-29F** are a series of graphs showing efficient expression of CD19 sCAR, CD19revCAR, and CD20 CAR constructs in primary human T cells.

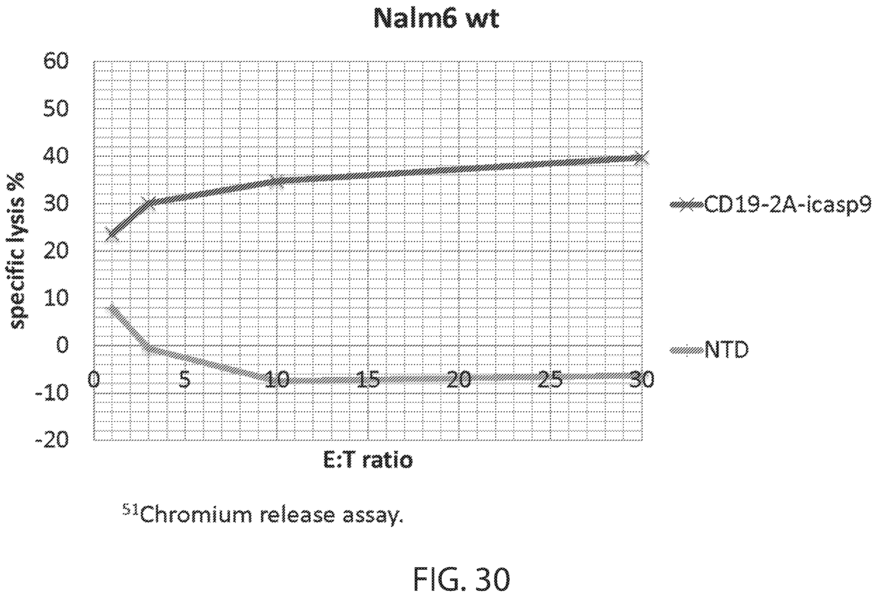

**FIG. 30** is a graph showing specific killing in vitro by CD19 sCARs. Nalm6 is a B cell line that expresses CD19 (wt, wildtype). CD19 sCAR T cells killed CD19+ Nalm6 cells, whereas nontransduced (NTD) T cells or T cells expressing a control sCAR (negative control) did not recognize Nalm6 wt cells .

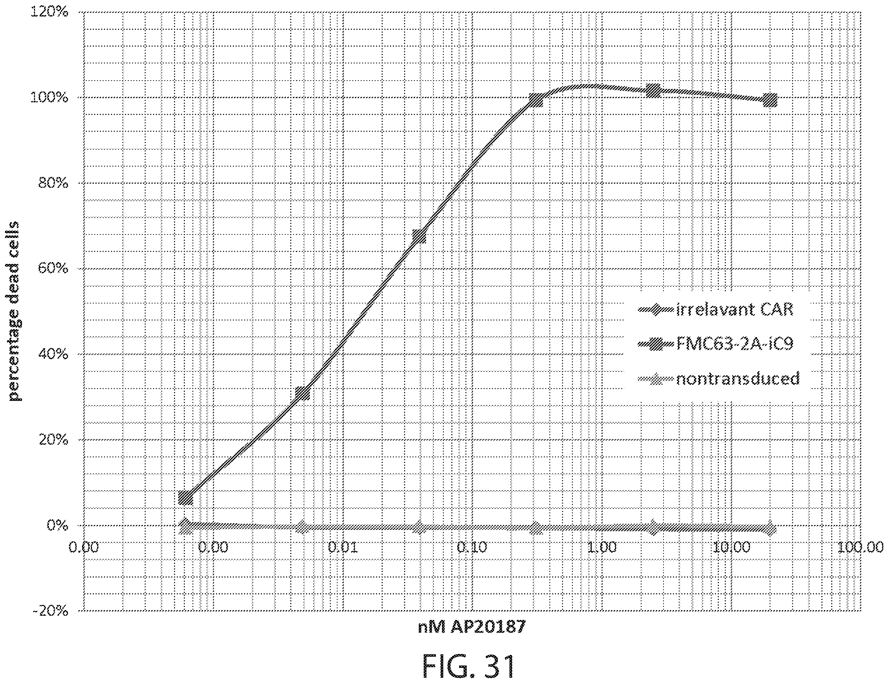

**FIG. 31** is a graph showing specific and robust elimination of CD19 sCAR T cells after activation of the caspase-9 suicide switch with AP20187. CAR T cells were incubated in the presence of the indicated concentrations of AP20187 for 16 hours at 37°C. Dead cells were detected with live/dead-violet dye and quantified by flow cytometry. Only T cells that expressed the inducible caspase were eliminated (FMC63 iC9 or CD19 sCAR, as well as iC9 control CAR). T cells that did not express control CAR were not affected (nontransduced, NTD, or a control CAR not expressing iC9).

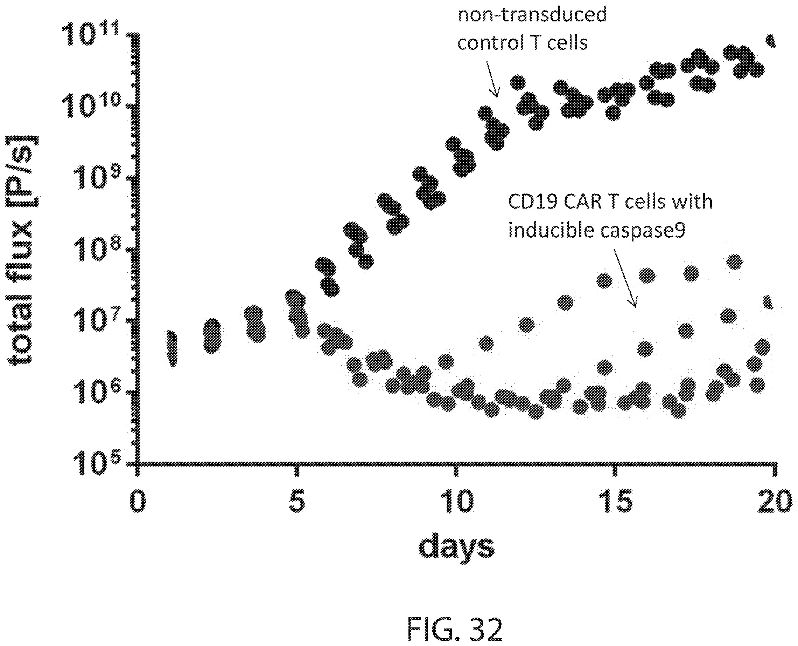

**FIG. 32** is a graph showing that CD19 sCAR T cells expressing the inducible caspase-9 are effective in vivo. NSG mice were injected with 1×10⁶ CD19+ Nalm6 cells. Five days later, mice were treated either with CD19 CAR T cells that express an inducible caspase-9 or with non-transduced control T cells.

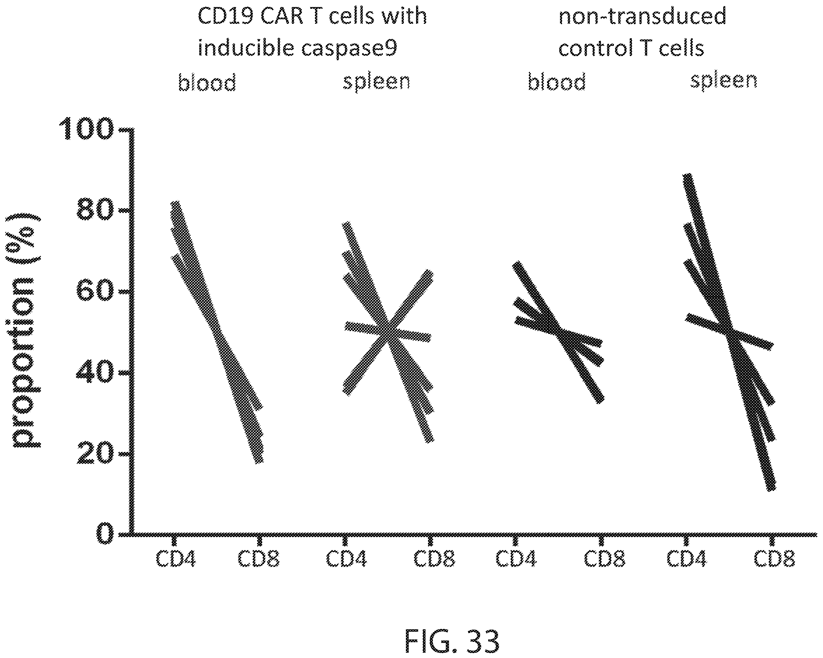

**FIG. 33** is a graph showing detection of CD19 sCAR T cells expressing an inducible caspase-9 in the blood and spleen by flow cytometry at the conclusion of the in vivo experiment, indicating engraftment.

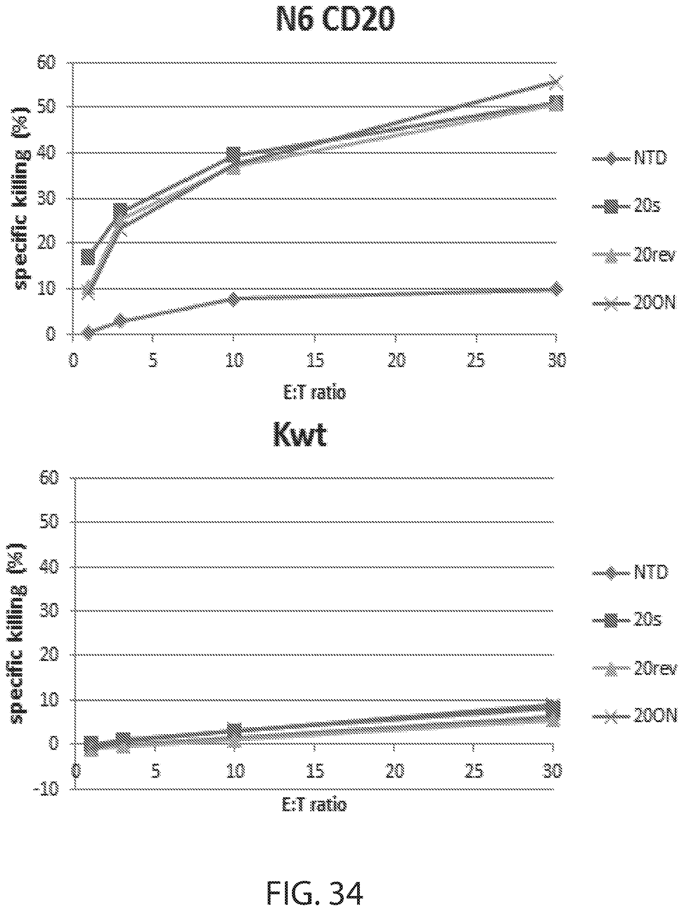

**FIG. 34** is a series of graph depicting Killing assay: CD20sCAR, 20revCAR and CD20 onCAR. 4h chromium release assay to assess killing of Nalm6 cell that were previously engineered to express CD20. Kwt = K562 wild-type cells that are CD20 negative and serve as irrelevant target to demonstrate specific killing. CD20sCAR, 20revCAR and CD20 onCAR show equivalent and specific killing of the CD20+ target cells. On-CAR was tested in presence of 500nM Shield-1.

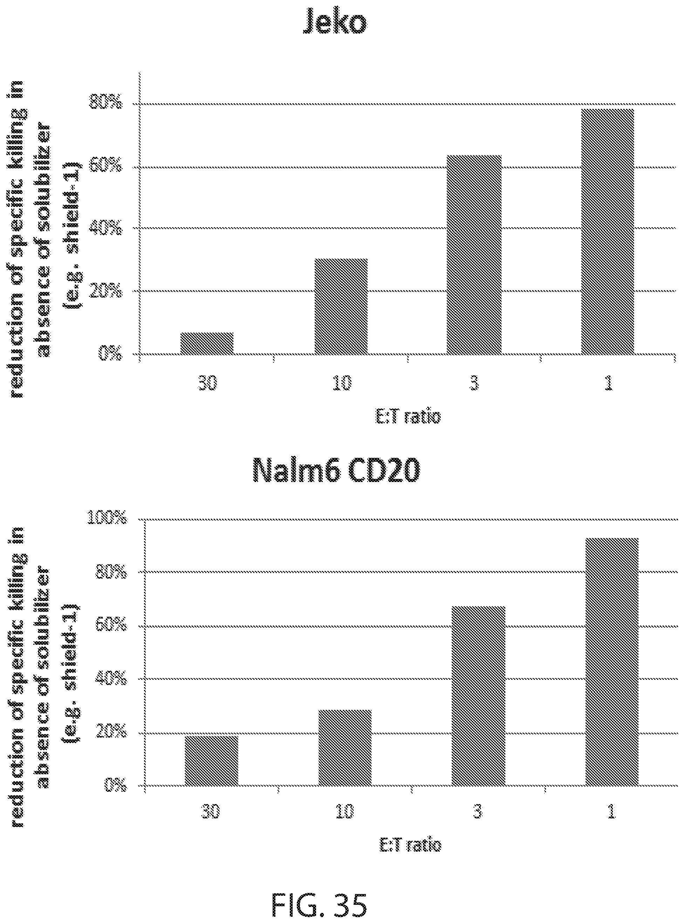

**FIG. 35** is a series of histograms demonstrating that the absence of solubilizing FKBP ligand (e.g. shield-1) inhibits CAR function. Shown is the percent reduction of killing in absence of shield-1 compared to killing in presence of 500nM shield-1. Jeko cells express CD20. At lower E:T ratio's the On-CAR function is strongly inhibited if no FKBP ligand is present.

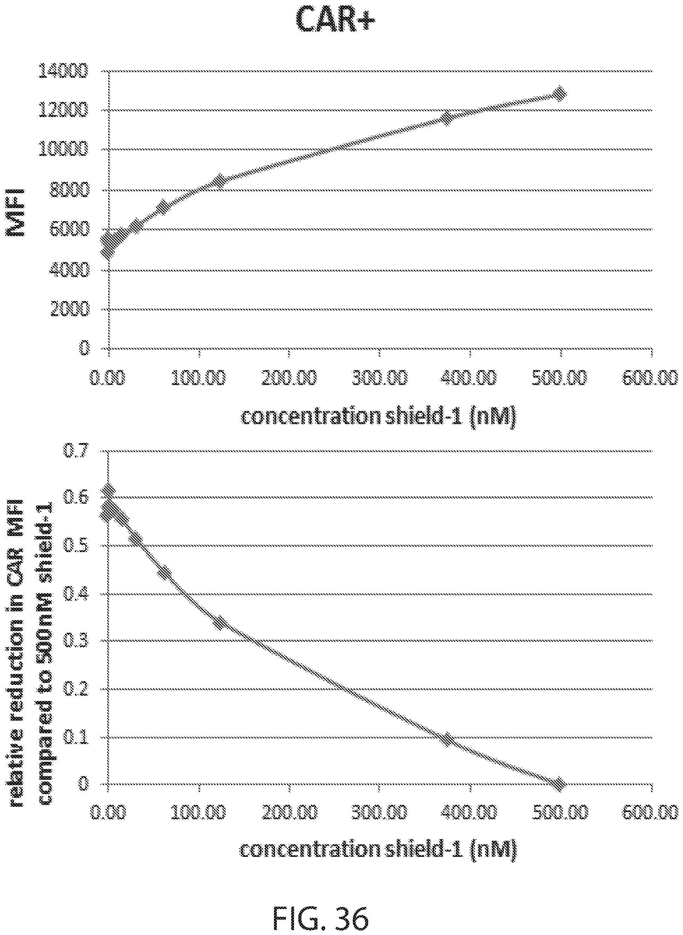

**FIG. 36** is series of graphs illustrating the modulation of CAR surface expression with FKBP ligand Shield-1. CD20 on-CAR T cells were stained for CAR expression and the mean fluorescence intensity (MFI, assessed by flow cytometry) of the CAR signal was plotted against the Shield-1 concentration. Shield-1 results in a dose-dependent increase in CAR expression. Absence of Shield-1 results in -60% reduction of CD20 on-CAR expression (bottom graph).

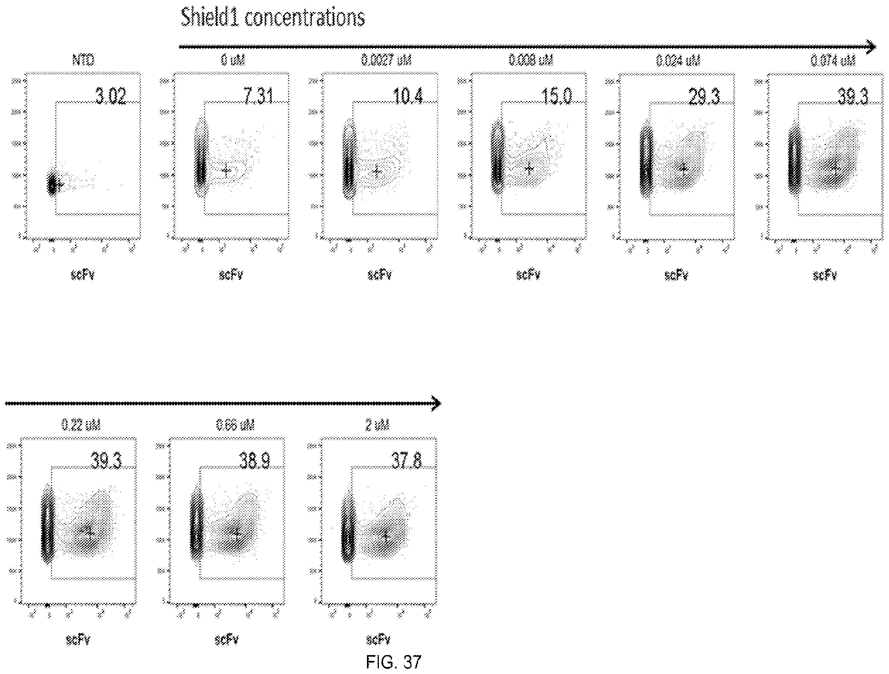

**FIG. 37** is a series of flow cytometry graphs depicting the titration of Shield-1 in CD19rev CAR. CD19rev CAR T cells (-38% transduced) were incubated in different doses of Shield-1 for 24h to assess dose-dependent caspase-9 acitvation. The remaining CAR+ cells were quantified by flow cytometry. In the CD19revCAR system, lower doses of Shield-1 result in higher caspase-9 activation and increased apoptosis (lower proportion of CAR+ cells).

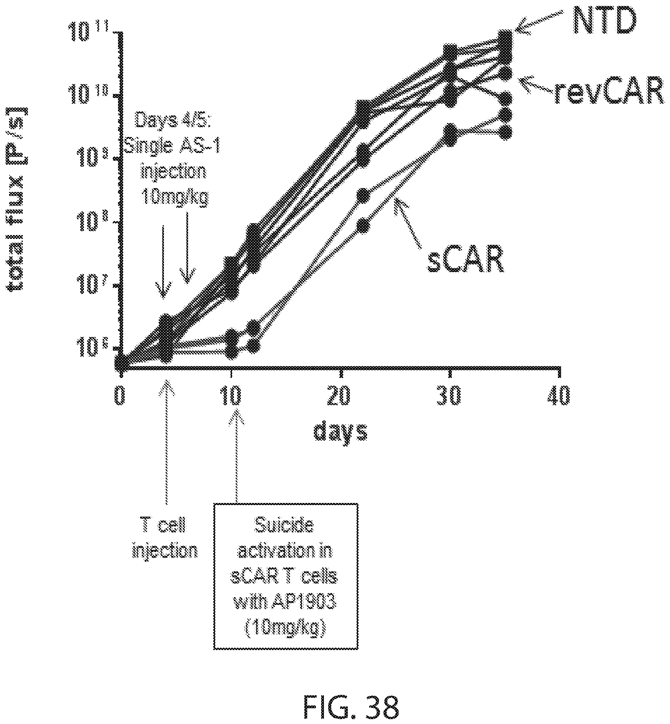

**FIG. 38** is a graph depicting the *in vivo* assessment of apoptosis efficiency in anti CD19 revCAR T cells. Nalm6 cells (expressing click-beetle luciferase) that are CD19 positive were injected (i.v.) into NSG mice on day 0. On day 4, the mice were injected with CD19 targeting revCAR, 19sCAR, or irrelevant (nontransduced) T cells. Bioluminescence signals of Nalm6 cells were quantified at indicated time points. revCAR treated mice were injected twice with 10mg/kg aquashield-1 at indicated time points, these injections were anticipated to be insufficient to keep the revCAR T cells alive. Therefore the bioluminescence curve for revCAR and NTD mice is vastly overlapping, indicating sufficient in vivo apoptosis of revCAR T cells.

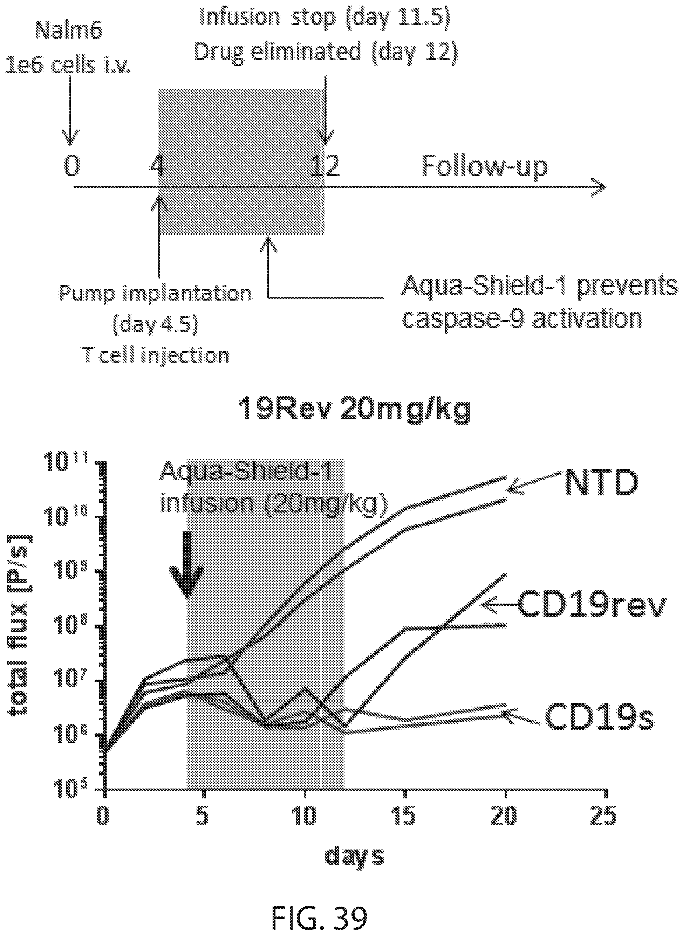

**FIG. 39** is a series of a schematic timeline and a graph showing the *in vivo* efficacy of CD19 revCAR T cells. Nalm6 cells (expressing click-beetle luciferase) that are CD19 positive were injected (i.v.) into NSG mice on day 0. On day 4, the osmotic infusion pumps were implanted into the mice to secrete aquashield-1 at a dose of 20mg/kg/day. CD19rev CAR T cells were injected 4 hours after successful pump implantation (Bottom graph, black arrow). Bioluminescence signals of Nalm6 cells was quantified at indicated time points. The infusion pumps secreted aquashield-1 for 7 days, after which the bioluminescence of Nalm6 cells started to rise (CD19 revCAR curves).

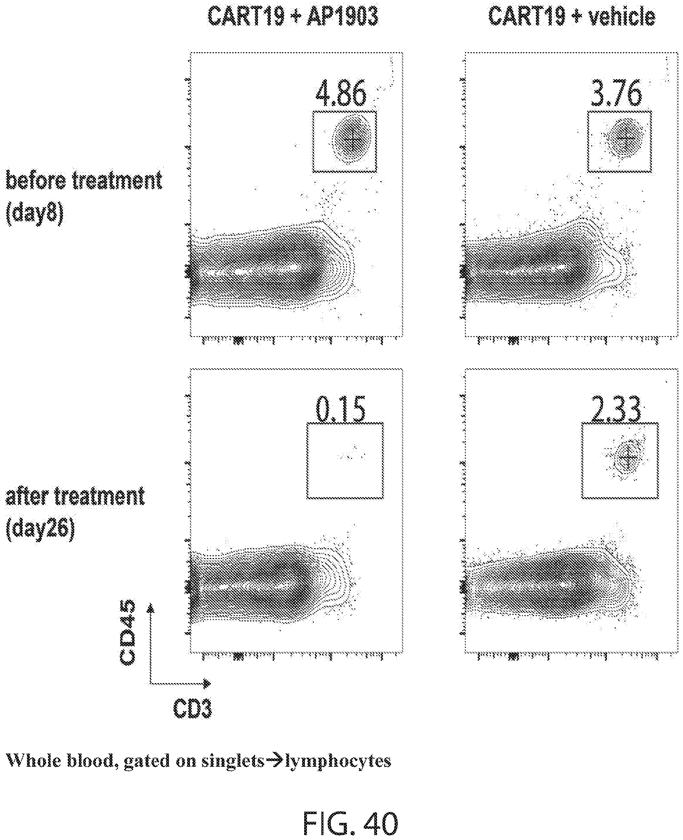

**FIG. 40** is a series of flow cytometry graphs depicting that the suicide activation results in pheripheral depletion of the suicide CAR T cells. Mice were injected with Nalm6 cells and after 5 days were treated with 19s CAR T cells. CD19s CAR T cells were sorted before injection based on scFV expression. Flow plots show peripheral T cells (CD3+, CD45+) on day 8 and day26. CD19 sCAR T cells were depleted on day 10 by injection of AP1903 (10mg/kg). In the AP1903 treated mice, the 2 plots on the left show that T cells are hardly detectable after suicide activation and In the vehicle treated mice, the 2 plots on the right showed stable T cell percentages.

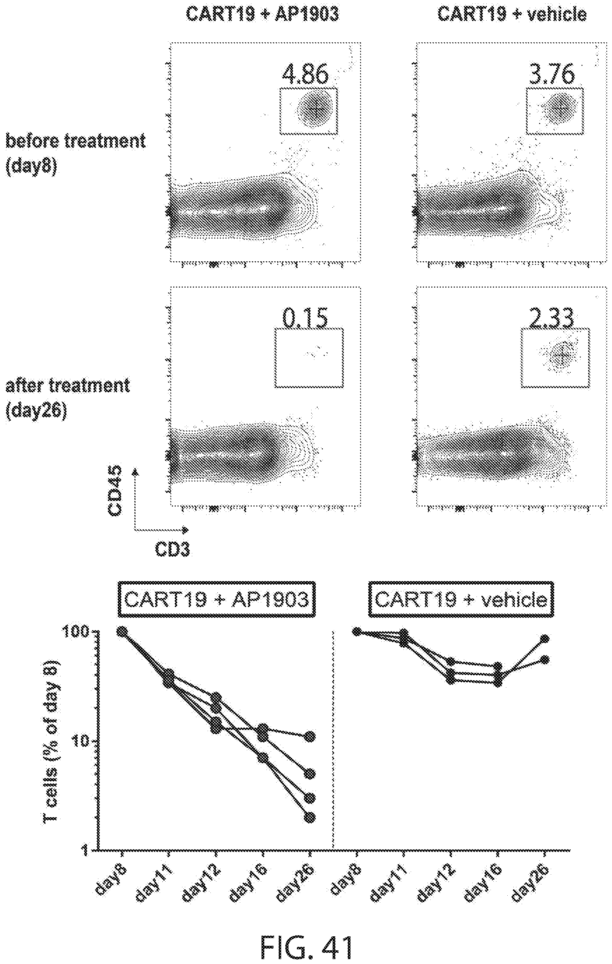

**FIG. 41** is a series of graphs demonstrating that suicide activation results in the peripheral depletion of sCAR T cells. The quantification of T cells was done from several mice. Mice were treated with AP1903 on day 10 to deplete sCAR T cells. After treatment the T cell percentages drop in the AP1903 group as opposed to the vehicle treated group.

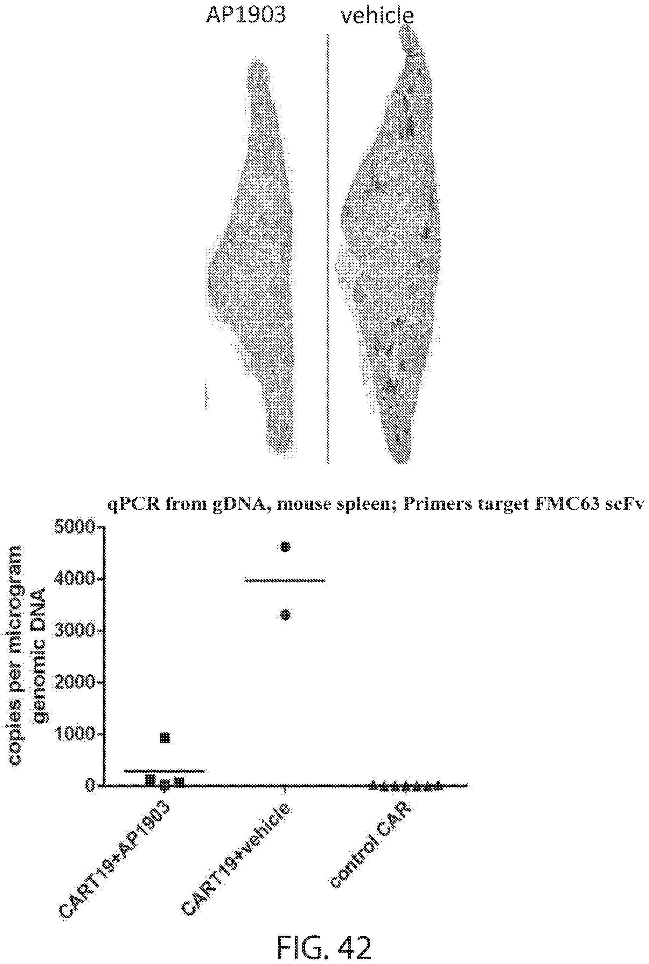

**FIG. 42** is a series of images and graphs demonstrating that suicide activation results in the depletion of sCAR T cells from lymphoid organs. Top image sections: spleens of mice were harvested >2 weeks after AP1903 treatment and stained for human CD3; in AP1903 treated mice, T cells are not detectable as opposed to vehicle treated mice. Bottom graph: quantitative PCR was performed to detect sCAR T cells in spleens of AP1903 and vehicle treated mice, demonstrating near-total sCAR absence in most mice.

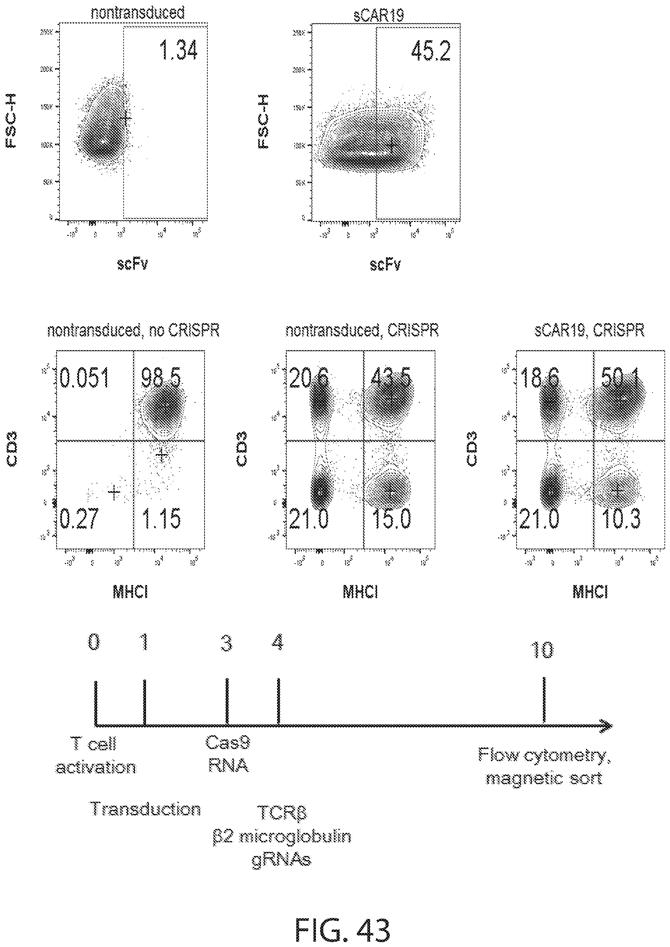

**FIG. 43** is a series of a schematic timeline and graphs showing that using BT (bone marrow, liver, thymus) mice as host requires 'universal' T cells lacking TCR and MHCI expression. T cells were transduced on day 1 after activation with CD19 sCAR encoding lentivirus, the cells were electroporated with Cas9 guide RNA on day 3 (10ug/10^6 cells) and again electroporated on day 4 with guide RNAs targeting the TCRbeta chain and beta-2 microglobulin. Flow plots show sCAR19 expression in 45.2% of the cells 10 days after activation and double knock-out for TCRbeta and beta-2 microglobulin in -20% of the cells. The schematic outlines the timeline for T cell production.

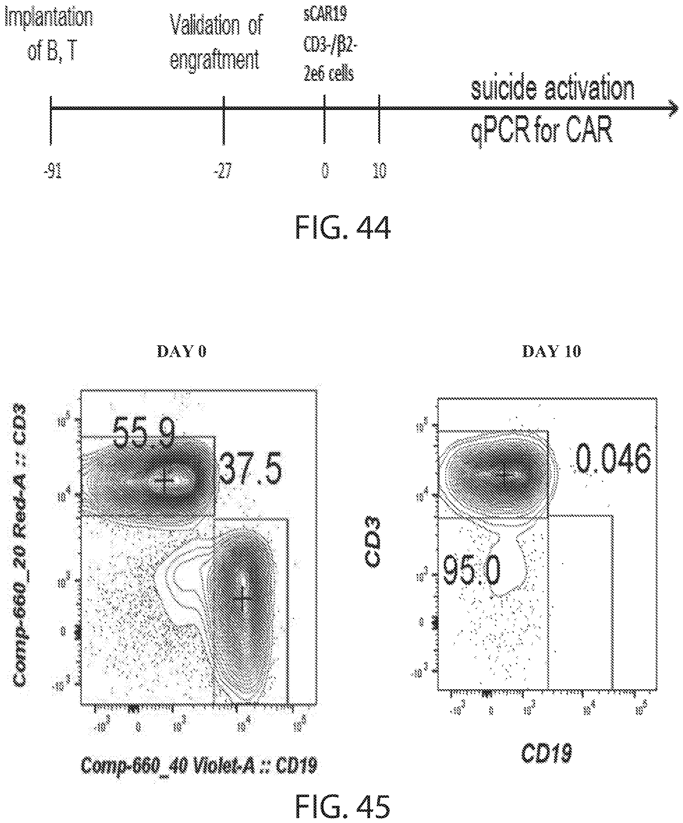

**FIG. 44** is a schematic timeline of the experimental design of the in vivo experiment of the present disclosure. Using BT (bone marrow, liver, and thymus) mice for assessment of universal sCAR function in a non-oncology mouse model. Human fetal bone marrow and thymus were implanted into NSG mice on day -91. Validation of engraftment was done by flow cytometry on day -27. Universal T cells expressing CD19 sCAR T cells were injected on day 0, B cell depletion was assessed on day 10 and mice were treated with AP1903 (3 daily injections with 10mg/kg). sCAR survival was assessed with qPCR.
**FIG. 45** is a series of flow cytometry graphs depicting that `Universal' sCAR T cells deplete peripheral B cells in non-autologous BT (bone marrow, liver, and thymus) mice. Universal CAR T cells were able to deplete human B cells in a non-cancer humanized mouse model. Flow plots on day 0 and day 10 demonstrate complete absence of CD19 positive cells in the peripheral circulation.

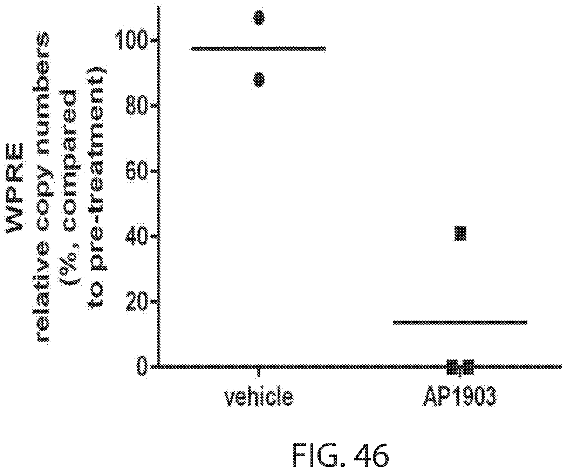

**FIG. 46** is a graph showing that the universal sCAR T cells can be depleted with AP1903 treatment. A quantitative PCR was performed to detect sCAR T cells in peripheral blood of AP1903 and vehicle treated mice, demonstrating near-total sCAR absence in 2/3 mice. A qPCR from peripheral blood WPRE copy number was performed with a gDNA, 4 weeks after AP1903 treatment.

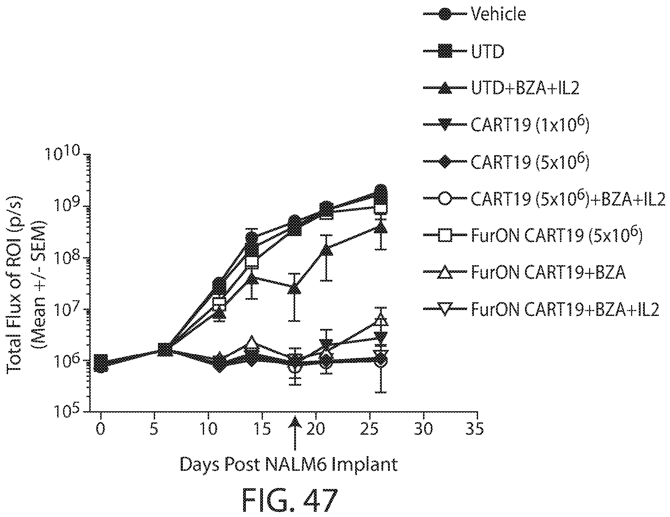

**FIG. 47** is a graph showing that CD19+ NALM6 tumor growth was inhibited in mice infused with CART19, or infused with FurON CART19 and treated with BZA, with or without cotreatment with IL2.

### DETAILED DESCRIPTION

In general, the disclosure features the regulated expression of recombinant fusion proteins. This regulation features expression of fusion proteins containing a protein of interest fused to a conditional expression domain, e.g., degradation domain (frequently termed a "degron" in the art) or an aggregation domain. Typically, such degradation domains fold into a stable conformation only in the presence of a specific ligand (e.g., a soluble ligand or a ligand tethered to the fusion protein). In the absence of such a ligand, the degradation domain assumes a disorganized structure, leading to degradation of the entire fusion protein by native intracellular mechanisms. Typically, such aggregation domains associate into oligomers and/or aggregates in the absence of a specific ligand (e.g., a soluble ligand or a ligand tethered to the fusion protein), leading to the aggregation and/or sequestration of the entire fusion protein. The disclosure is based on the insight that the degradation domain or aggregation domain can be separated from the protein of interest by a cleavage site (e.g., a protease cleavage site) when expressed under stabilizing conditions (e.g., in the presence of a stabilization compound) or deaggregating conditions (e.g., in the presence of a deaggregation compound). Thus, in the absence of a cognate protease, the fusion protein comprising a degradation domain will escape degradation only in the presence of the stabilizing compound and the fusion protein comprising an aggregation domain will escape aggregation only in the presence of the deaggregation compound. However, once the cleavage site is cleaved by a cognate protease, the presence of a stabilizing compound or deaggregating compound is no longer necessary to allow the protein of interest to escape degradation or aggregation because it is no longer associated with the degradation domain or aggregation domain. Thus, at the time of initial expression, the fusion protein comprising a degradation domain is susceptible to degradation and the fusion protein comprising an aggregation domain is susceptible to aggregation, however, once the cleavage has occurred, the resulting protein of interest can be otherwise indistinguishable from its non-fusion protein counterpart.

The fusion proteins of the disclosure thus have three essential elements: a conditional expression domain (e.g., degradation domain or aggregation domain), a domain containing the protein of interest, and a cleavage domain separating the two. Within each of these elements, the specific domains are interchangeable and discussed below. Notably, the fusion protein can be arranged such that conditional expression domain is located either N-terminal or C-terminal to the protein of interest. However, in certain instances of the disclosure, the conditional expression domain is N-terminal to the protein of interest. In such instances, where the conditional expression domain is a degradation domain, the degradation domain, where disorganized, targets the entire fusion protein for degradation prior to the cleavage of the cleavage domain. In case of a C-terminal fusion, the protease cleavage site has to be oriented towards the compartment where the protease resides. In the particular case of furin, the C-terminal degron needs to face the lumen of the endoplasmic reticulum and Golgi. In the present invention, the conditional expression domain is a degradation domain.

### DEFINITIONS

As used herein, the term "conditional expression domain" refers to a domain of a fusion protein that has a first state and a second state, e.g., states of aggregation or conformational states, e.g., states of stabilization/destabilization, or states of folding/misfolding. The first state is associated with, causes, or mediates cell surface expression or extracellular expression of one or more (e.g., all) portions of the fusion protein at a first rate or level and the second state is associated with, causes, or mediates cell surface expression or extracellular expression of one or more (e.g., all) portions of the fusion protein at a second rate or level. Absent an expression compound, when expressed in a cell of interest, a large fraction of the fusion protein comprising a conditional expression domain is not detectable either on the cell surface or extracellularly. Conversely, in the presence of an expression compound, surface expression and/or extracellular expression of one or more (e.g., all) domains of the fusion protein is substantially increased. Thus, a conditional expression domain is identifiable by the following characteristics: (1) it is not naturally occurring in the context of the fusion protein; (2) surface expression and/or extracellular expression is regulated co-translationally or post-translationally; (3) the rate of surface expression and/or extracellular expression is substantially increased in the presence of an expression compound. In the present invention, a conditional expression domain is a degradation domain, for example, as described herein. In other instances of the disclosure which do not form part of the claimed invention, a conditional expression domain is an aggregation domain, for example as described herein.

In the present invention, the fusion protein comprising the conditional expression domain comprises a chimeric antigen receptor (CAR), for example, as described herein. In the present invention, the fusion protein comprises a heterologous protease cleavage site disposed between the conditional expression domain and a second domain. In such embodiments, in the absence of an expression compound, the protease cleavage site of a large fraction of the fusion proteins is not accessible by the congnate protease, and the cellular fate of the fusion protein is directed by the conditional expression domain. In the presence of an expression compound, the protease cleavage site of a large fraction of the fusion proteins become accessible to the cognate protease, the conditional expression domain is cleaved from the fusion protein, and the cellular fate of the remainder of the fusion protein proceeds as is uninterrupted by the conditional expression domain.

As used herein, the term "aggregation domain" refers to a domain of a fusion protein that causes intracellular aggregation of the fusion protein. Absent a deaggregation compound, when expressed in a cell of interest, a large fraction of the fusion protein comprising an aggregation domain is present in aggregates which, for example, are sequestered within the cell before reaching mature expression, for example, before being expressed on the surface of the cell or before being secreted extracellulary. Conversely, in the presence of a deaggregation compound, surface expression and/or extracellular expression of one or more (e.g., all) domains of the fusion protein is substantially increased. Thus, an aggregation domain is identifiable by the following characteristics: (1) it is not naturally occurring in the context of the fusion protein; (2) surface expression and/or extracellular expression is regulated co-translationally or post-translationally; (3) the rate of surface expression and/or extracellular expression is substantially increased in the presence of a deaggregation compound. In instances of the present disclosure, the aggregation domain comprises one or more, e.g., 1, 2, 3, 4, 5, 6, 7, or more repeats of a dimerization domain. In instances, the dimerization domain is a homodimerization domain. In other instances, the dimerization domain is a heterodimerization domain.

As used herein, the term "expression compound" refers to a compound that, when added to a cell expressing a fusion protein comprising a conditional expression domain, binds to a conditional expression domain and results in increased surface expression and/or extracellular expression of one or more (e.g., all) domains of the fusion protein. Expression compounds can be naturally occurring or synthetic.

As used herein, the term "deaggregation compound" refers to a compound that, when added to a cell expressing a fusion protein comprising an aggregation domain, binds to an aggregation domain and results in increased surface expression and/or extracellular expression of one or more (e.g., all) domains of the fusion protein. Deaggregation compounds can be naturally occurring or synthetic.

By the term "degradation domain" is meant a domain of a fusion protein that assumes a stable conformation when expressed in the presence of a stabilizing compound. Absent the stable conformation when expressed in a cell of interest, a large fraction of degradation domains (and, typically, any protein to which they are fused) will be degraded by endogenous cellular machinery. Notably, a degradation domain is not a naturally occurring domain of a protein but is rather engineered to be unstable absent contact with the stabilizing compound. Thus, a degradation domain is identifiable by the following characteristics: (1) it is not naturally occurring; (2) its expression is regulated co-translationally or post-translationally through increased or decreased degradation rates; (3) the rate of degradation is substantially decreased in the presence of a stabilizing compound. In some embodiments, the degradation domain does not lead to aggregation of the fusion proteins. In some embodiments, absent a stabilizing compound, the degradation domain or other domain of the fusion protein is not substantially detectable in or on the cell.

As used herein, the terms "deaggregation" and "disaggregation" are used interchangeably.

As used herein, a "fusion protein" or "chimeric protein" refers to a protein created through the joining of two or more heterologous protein domains into a single, continuous protein.

By the term "heterologous" is meant a domain (e.g., a protein domain) that has a different origin (i.e., does not naturally occur fused to at least one of the other referenced domains) than one or more protein domains to which it is fused. Furthermore, by "heterologous protease cleavage site" is meant a protease cleavage site that is not cleaved by any domain of the fusion protein in which it is present.

By "protease" is meant a protein that cleaves another protein based on the presence of a cleavage site in the to-be-cleaved protein.

By "intracellular protease" is meant a protease that is natively expressed inside a cell of interest.

By "extracellular protease" is meant a protease that is natively expressed in an organism (e.g., a mammal) and secreted or exposed to the outside of cells (e.g., in the blood or the surface of the skin).

By "stabilization compound" or "stabilizing compound" is meant a compound that, when added to a cell expressing a degradation domain, stabilizes the degradation domain and decreases the rate at which it is subsequently degraded. Stabilization compounds or stabilizing compounds can be naturally occurring or synthetic.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule as defined below. In some embodiments, the domains in the CAR polypeptide construct are in the same polypeptide chain, e.g., comprise a chimeric fusion protein. In some embodiments, the domains in the CAR polypeptide construct are not contiguous with each other, e.g., are in different polypeptide chains, e.g., in regulatable chimeric antigen receptor (RCAR).

In one aspect, the stimulatory molecule is the zeta chain associated with the T cell receptor complex. In one aspect, the cytoplasmic signaling domain comprises a primary signaling domain (e.g., a primary signaling domain of CD3-zeta). In one aspect, the cytoplasmic signaling domain further comprises one or more functional signaling domains derived from at least one costimulatory molecule as defined below. In one aspect, the costimulatory molecule is chosen from 4-1BB (i.e., CD137), CD27, ICOS, and/or CD28. In one aspect, the CAR comprises a chimeric fusion protein comprising an extracellular antigen binding domain, a transmembrane domain and an intracellular signaling domain comprising a functional signaling domain derived from a stimulatory molecule. In one aspect, the CAR comprises a chimeric fusion protein comprising an extracellular antigen binding domain, a transmembrane domain and an intracellular signaling domain comprising a functional signaling domain derived from a co-stimulatory molecule and a functional signaling domain derived from a stimulatory molecule. In one aspect, the CAR comprises a chimeric fusion protein comprising an extracellular antigen binding domain, a transmembrane domain and an intracellular signaling domain comprising two functional signaling domains derived from one or more co-stimulatory molecule(s) and a functional signaling domain derived from a stimulatory molecule. In one aspect, the CAR comprises a chimeric fusion protein comprising an extracellular antigen binding domain, a transmembrane domain and an intracellular signaling domain comprising at least two functional signaling domains derived from one or more co-stimulatory molecule(s) and a functional signaling domain derived from a stimulatory molecule. In one aspect the CAR comprises an optional leader sequence at the amino-terminus (N-ter) of the CAR fusion protein. In one aspect, the CAR further comprises a leader sequence at the N-terminus of the extracellular antigen binding domain, wherein the leader sequence is optionally cleaved from the antigen recognition domain (e.g., a scFv) during cellular processing and localization of the CAR to the cellular membrane.

A CAR that comprises an antigen binding domain (e.g., a scFv, or TCR) that targets, e.g., binds to, a specific antigen X, such as those described herein, is also referred to as XCAR, X-CAR or X-targeing CAR. For example, a CAR that comprises an antigen binding domain that targets CD19 is referred to as CD19CAR.

The term "signaling domain" refers to the functional portion of a protein which acts by transmitting information within the cell to regulate cellular activity via defined signaling pathways by generating second messengers or functioning as effectors by responding to such messengers. In some aspects, the signaling domain of the CAR described herein is derived from a stimulatory molecule or co-stimulatory molecule described herein, or is a synthesized or engineered signaling domain.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an intact antibody, or recombinant variants thereof, and refers to the antigen binding domain, e.g., an antigenic determining variable region of an intact antibody, that is sufficient to confer recognition and specific binding of the antibody fragment to a target, such as an antigen. Examples of antibody fragments include, but are not limited to, Fab, Fab', F(ab')₂, and Fv fragments, scFv antibody fragments, linear antibodies, single domain antibodies such as sdAb (either VL or VH), camelid VHH domains, and multi-specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked via a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The term "complementarity determining region" or "CDR," as used herein, refers to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a VH, e.g., a mammalian VH, e.g., a human VH; and amino acid residues 24-34 (LCDR1), 50-56 (LCDR2), and 89-97 (LCDR3) in a VL, e.g., a mammalian VL, e.g., a human VL.

The portion of the CAR of the invention comprising an antibody or antibody fragment thereof may exist in a variety of forms where the antigen binding domain is expressed as part of a contiguous polypeptide chain including, for example, scFv antibody fragments, linear antibodies, single domain antibodies such as sdAb (either VL or VH), camelid VHH domains ,a humanized antibody, a bispecific antibody, an antibody conjugate (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR of the invention comprises an antibody fragment. In a further aspect, the CAR comprises an antibody fragment that comprises a scFv.

As used herein, the term "binding domain" or "antibody molecule" (also referred to herein as "anti-target (e.g., CD19) binding domain") refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "immunoglobulin" or "Ig," as used herein is defined as a class of proteins, which function as antibodies. Antibodies expressed by B cells are sometimes referred to as the BCR (B cell receptor) or antigen receptor. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function, but may serve as an antigen receptor. IgE is the immunoglobulin that mediates immediate hypersensitivity by causing release of mediators from mast cells and basophils upon exposure to allergen.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present disclosure includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated or can be derived from a biological sample, or might be macromolecule besides a polypeptide. Such a biological sample can include, but is not limited to a tissue sample, a tumor sample, a cell or a fluid with other biological components.

The term "autoantigen" means, in accordance with the present disclosure, any self-antigen which is recognized by the immune system as being foreign. Autoantigens comprise, but are not limited to, cellular proteins, phosphoproteins, cellular surface proteins, cellular lipids, nucleic acids, glycoproteins, including cell surface receptors.

"Autoantibody" refers to an antibody that is produced by a B cell specific for an autoantigen.

The term "autoantibody disease" as used herein is defined as a disorder that results from an autoantibody immune response. An autoantibody disease is the result of an inappropriate and excessive response production of autoantibodies. Examples of autoantibody diseases include but are not limited to, bullous pemphigoid, epidermolysis bullosa acquisita, p200 pemphigoid, linear IgA bullous dermatosis, other pemphigoid group diseases, dermatitis herpetiformis, celiac disease, myasthenia gravis, Goodpasture's syndrome, granulomatosis with polyangiitis and other ANCA+ vasculitidies, autoimmune limbic encephalitis, anti-N-methyl-D-aspartate receptor encephalitis, neuromyelitis optica, autoimmune hemolytic anemia, autoantibody-associated end-organ damage in lupus and other connective tissue diseases (due to anti-dsDNA, anti-Ro, and other autoantibodies), Graves` and Hashimoto's thyroiditis, anti-insulin antibodies in diabetes, anti-insulin receptor antibodies in autoimmune hypoglycemia, cryoglobulinemia, rheumatoid arthritis, multiple sclerosis, Sjogren's syndrome, dermatomyositis, anti-Fc-epsilon receptor antibodies in chronic idiopathic urticaria, anti-folate receptor antibodies, anti-endothelial receptor or anti-adrenergic receptor antibodies in pulmonary arterial hypertension, refractory hypertension, dilated cadiomyopathy, and autoinflammatory syndromes such as IgG4-related disease, among others.

The term "autoimmune disease" as used herein is defined as a disorder or condition that results from an antibody mediated autoimmune response against autoantigens. An autoimmune disease results in the production of autoantibodies that are inappropriately produced and/or excessively produced to a self-antigen or autoantigen.

The term "autologous" refers to any material derived from the same individual to whom it is later to be reintroduced into the individual.

The term "anti-tumor effect" or "anti-tumor activity" refers to a biological effect which can be manifested by various means, including but not limited to, e.g., a decrease in tumor volume, a decrease in the number of tumor cells, a decrease in the number of metastases, an increase in life expectancy, decrease in tumor cell proliferation, decrease in tumor cell survival, or amelioration of various physiological symptoms associated with the cancerous condition. An "anti-tumor effect" can also be manifested by the ability of the peptides, polynucleotides, cells and antibodies of the disclosure in prevention of the occurrence of tumor in the first place.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically The term "xenogeneic" refers to a graft derived from an animal of a different species.

The term "apheresis" as used herein refers to an extracorporeal process by which the blood of a donor or patient is removed from the donor or patient and passed through an apparatus that separates out selected particular constituent(s) and returns the remainder to the circulation of the donor or patient, e.g., by retransfusion. Thus, in the context of "an apheresis sample" refers to a sample obtained using apheresis.

The term "cleavage" refers to the breakage of covalent bonds, such as in the backbone of a nucleic acid molecule or the hydrolysis of peptide bonds. Cleavage can be initiated by a variety of methods, including, but not limited to, enzymatic or chemical hydrolysis of a phosphodiester bond. Both single-stranded cleavage and double-stranded cleavage are possible. Double-stranded cleavage can occur as a result of two distinct single-stranded cleavage events. DNA cleavage can result in the production of either blunt ends or staggered ends. In certain embodiments, fusion polypeptides may be used for targeting cleaved double-stranded DNA.

The term "cancer" or "tumor" refers to a disease characterized by the uncontrolled growth of aberrant cells. Cancer includes all types of cancerous growths or oncogenic processes, metastatic tissues or malignantly transformed cells, tissues or organs irrespective of the histopathologic type or stage of invasiveness. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

The term "CRISPR/CAS," "clustered regularly interspaced short palindromic repeats system," or "CRISPR" refers to DNA loci containing short repetitions of base sequences. Each repetition is followed by short segments of spacer DNA from previous exposures to a virus. Bacteria and archaea have evolved adaptive immune defenses termed CRISPR-CRISPR-associated (Cas) systems that use short RNA to direct degradation of foreign nucleic acids. In bacteria, the CRISPR system provides acquired immunity against invading foreign DNA via RNA-guided DNA cleavage.

In the type II CRISPR/Cas system, short segments of foreign DNA, termed "spacers" are integrated within the CRISPR genomic loci and transcribed and processed into short CRISPR RNA (crRNA). These crRNAs anneal to trans-activating crRNAs (tracrRNAs) and direct sequence-specific cleavage and silencing of pathogenic DNA by Cas proteins. Recent work has shown that target recognition by the Cas9 protein requires a "seed" sequence within the crRNA and a conserved dinucleotide-containing protospacer adjacent motif (PAM) sequence upstream of the crRNA-binding region.

To direct Cas9 to cleave sequences of interest, crRNA-tracrRNA fusion transcripts, hereafter referred to as "guide RNAs" or "gRNAs" may be designed, from human U6 polymerase III promoter. CRISPR/CAS mediated genome editing and regulation, highlighted its transformative potential for basic science, cellular engineering and therapeutics.

The term "CRISPRi" refers to a CRISPR system for sequence specific gene repression or inhibition of gene expression, such as at the transcriptional level.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor antigen" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one aspect, a cancer associated with expression of a tumor antigen as described herein is a hematological cancer. In one aspect, a cancer associated with expression of a tumor antigen as described herein is a solid cancer. Further diseases associated with expression of a tumor antigen described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR of the invention can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules, such as downregulation of TGF-β, and/or reorganization of cytoskeletal structures, and the like.

The term "stimulatory molecule," refers to a molecule expressed by an immune effector cell (e.g., a T cell, NK cell, B cell) that provides the cytoplasmic signaling sequence(s) that regulate activation of the immune effector cell in a stimulatory way for at least some aspect of the immune effector cell signaling pathway, e.g., the T cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing primary cytoplasmic signaling sequence that is of particular use in the invention includes, but is not limited to, those derived from CD3 zeta, common FcR gamma (FCER1G), Fc gamma Rlla, FcR beta (Fc epsilon R1b), CD3 gamma, CD3 delta , CD3 epsilon, CD5, CD22, CD79a, CD79b, CD278 (also known as "ICOS"), FcεRI, DAP10, DAP12, and CD66d. In a specific CAR of the invention, the intracellular signaling domain in any one or more CARs of the invention comprises an intracellular signaling sequence, e.g., a primary signaling sequence of CD3-zeta. In a specific CAR of the invention, the primary signaling sequence of CD3-zeta is the sequence provided as SEQ ID NO:18, or the equivalent residues from a non-human species, e.g., mouse, rodent, monkey, ape and the like. In a specific CAR of the invention, the primary signaling sequence of CD3-zeta is the sequence as provided in SEQ ID NO:20, or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain generates a signal that promotes an immune effector function of the CAR-expressingcell, e.g., a CART cell or CAR-expressing NK cell. Examples of immune effector function, e.g., in a CART cell or CAR-expressing NK cell, include cytolytic activity and helper activity, including the secretion of cytokines. While the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. In an embodiment, the intracellular signaling domain is synthesized or engineered. For example, in the case of a CAR-expressing immune effector cell, e.g., CART cell or CAR-expressing NK cell, a primary intracellular signaling domain can comprise a cytoplasmic sequence of a T cell receptor, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, common FcR gamma (FCER1G), Fc gamma Rlla, FcR beta, CD3 gamma, CD3 delta, CD3 epsilon, CD5, CD22, CD79a, CD79b, CD278 ("ICOS"), FcεRI CD66d, DAP10 and DAP12.

The term "zeta" or alternatively "zeta chain", "CD3-zeta" or "TCR-zeta" is defined as the protein provided as GenBan Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO:18. In one aspect, the "zeta stimulatory domain" or a "CD3-zeta stimulatory domain" is the sequence provided as SEQ ID NO:20. Also encompassed herein are CD3 zeta domains comprising one or more mutations to the amino acid sequences described herein, e.g., SEQ ID NO: 20.

The term "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to an MHC class I molecule, a TNF receptor protein, an Immunoglobulin-like protein, a cytokine receptor, an integrin, a signaling lymphocytic activation molecule (SLAM protein), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain or costimulatory signaling domain can be the intracellular portion of a costimulatory molecule. The intracellular signaling domain can comprise the entire intracellular portion, or the entire native intracellular signaling domain, of the molecule from which it is derived, or a functional fragment thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the sequence provided as SEQ ID NO:14 or the equivalent residues from a non-human species, e.g., mouse, rodent, monkey, ape and the like.

The term "immune response" as used herein is defined as a cellular response to an antigen that occurs when lymphocytes identify antigenic molecules as foreign and induce the formation of antibodies and/or activate lymphocytes to remove the antigen.

When "an immunologically effective amount," "an autoimmune disease-inhibiting effective amount," or "therapeutic amount" is indicated, the precise amount of the compositions of the present invention to be administered can be determined by a physician or researcher with consideration of individual differences in age, weight, tumor size, extent of infection or metastasis, and condition of the patient (subject).

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloid-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and co-stimulation are examples of immune effector function or response.

The term "effector function" refers to a specialized function of a cell. Effector function of a T cell, for example, may be cytolytic activity or helper activity including the secretion of cytokines.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "effective amount" or "therapeutically effective amount" are used interchangeably herein, and refer to an amount of a compound, formulation, material, or composition, as described herein effective to achieve a particular biological result.

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTORO gene delivery technology from Oxford BioMedica, the LENTIMAX^{™} vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or antibody fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present disclosure, the following abbreviations for the commonly occurring nucleic acid bases are used. "A" refers to adenosine, "C" refers to cytosine, "G" refers to guanosine, "T" refers to thymidine, and "U" refers to uridine.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The terms "cancer associated antigen" or "tumor antigen" interchangeably refers to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, exemplary tumor antigens include: CD19; CD123; CD22; CD30; CD171; CS-1; C-type lectin-like molecule-1, CD33; epidermal growth factor receptor variant III (EGFRvIII); ganglioside G2 (GD2); ganglioside GD3; TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2; Mesothelin; Interleukin 11 receptor alpha (IL-11Ra); prostate stem cell antigen (PSCA); Protease Serine 21; vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CA!X); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3; transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51 E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1, melanoma antigen recognized by T cells 1; Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like, Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLL1); TNF receptor family member; Fms-Like Tyrosine Kinase 3 (FL T3); CD10; CD19; CD20; CD21; CD22; CD23; CD24; CD25; CD37; CD38; CD53; CD72; CD73; CD74; CD75; CD77; CD79a; CD79b; CD80; CD81; CD82; CD83; CD84; CD85; ROR1; BCMA; CD86; CD179b; CD1a; CD1b; CD1c; CD1d; CD2; CD5; CD6; CD9; CD11a; CD11b; CD11c; CD17; CD18; CD26; CD27; CD29; CD30; CD31; CD32a; CD32b; CD35; CD38; CD39; CD40; CD44; CD45; CD45RA; CD45RB; CD45RC; CD45RO; CD46; CD47; CD48; CD49b; CD49c; CD49d; CD50; CD52; CD54; CD55; CD58; CD60a; CD62L; CD63; CD63; CD68 CD69; CD70; CD85E; CD85I; CD85J; CD92; CD95; CD97; CD98; CD99; CD100; CD102; CD108; CD119; CD120a; CD120b; CD121b; CD122; CD124; CD125; CD126; CD130; CD132; CD137; CD138; CD139; CD147; CD148; CD150; CD152; CD162; CD164; CD166; CD167a; CD170; CD175; CD175s; CD180; CD184; CD185; CD192; CD196; CD197; CD200; CD205; CD210a; CDw210b; CD212; CD213a1; CD213a2; CD215; CD217; CD218a; CD218b; CD220; CD221; CD224; CD225; CD226; CD227; CD229; CD230; CD232; CD252; CD253; CD257; CD258; CD261; CD262; CD263; CD264; CD267; CD268; CD269; CD270; CD272; CD274; CD275; CD277; CD279; CD283; CD289; CD290; CD295; CD298; CD300a; CD300c; CD305; CD306; CD307a; CD307b; CD307c; CD307d; CD307e; CD314; CD315; CD316; CD317; CD319; CD321; CD327; CD328; CD329; CD338; CD351; CD352; CD353; CD354; CD355; CD357; CD358; CD360; CD361; CD362; CD363; and a peptide of any of these antigens presented on MHC. Particularly preferred antigens include: CD19, CD20, CD22, FcRn5, FcRn2, BCMA, CS-1, CD138, CLDN6, mesothelin, EGFRvIII, CD123, CD33 and CLL-1. In some embodiments, the CARs of the present disclosure includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library. Accordingly, the present disclosure provides CARs that comprise an antigen binding domain that binds to a MHC presented peptide of a molecule selected from the group of WT1, NY-ESO-1, LAGE-1a, MAGE-A1 and RAGE-1.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)n, where n is a positive integer equal to or greater than 1, for example, n=1, n=2, n=3, n=4, n=5 and n=6, n=7, n=8, n=9 or n=10 (SEQ ID NO:28). In one embodiment, the flexible polypeptide linkers include, but are not limited to, (Gly4 Ser)4 (SEQ ID NO:29) or (Gly4 Ser)3 (SEQ ID NO:30). In another embodiment, the linkers include multiple repeats of (Gly2Ser), (GlySer) or (Gly3Ser) (SEQ ID NO:31). Also included within the scope of the invention are linkers described in WO2012/138475.

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In the preferred embodiment of a construct for transient expression, the polyA is between 50 and 5000 (SEQ ID NO: 32), preferably greater than 64, more preferably greater than 100, most preferably greater than 300 or 400. Poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

As used herein, the terms "treat", "treatment" and "treating" refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a CAR of the invention). In specific embodiments, the terms "treat," "treatment" and "treating" refer to the amelioration of at least one measurable physical parameter of a proliferative disorder, such as growth of a tumor, not necessarily discernible by the patient. In other embodiments the terms "treat", "treatment" and "treating" - refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human). A "subject" or "patient," as used therein, may be a human or non-human mammal. Non-human mammals include, for example, livestock and pets, such as ovine, bovine, porcine, canine, feline and murine mammals. Preferably, the subject is human.

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

The term "suicide gene" as used herein refers to a suicide or apoptosis-inducing gene operably linked to a promoter, which may be constitutive or inducible. Examples of a suicide gene include, but are not limited to, a herpes simplex virus thymidine kinase (HSV-TK), the cytoplasmic domain of Fas, a caspase such as caspase-8 or caspase-9, cytosine deaminase, E1A, FHIT, and other known suicide or apoptosis-inducing genes.

The term "suicide gene product" or "suicide domain" as used herein refers to the expression product of the suicide gene.

The term "therapeutic" as used herein means a treatment. A therapeutic effect is obtained by reduction, suppression, remission, or eradication of a disease state.

The term "tolerance" or "immune tolerance" as used herein refers to a state in which a subject has a reduced or absent immune response to a specific antigen or group of antigens to which the subject is normally responsive to. Tolerance is achieved under conditions that suppress the immune reaction and is not just the absence of an immune response. In an embodiment, tolerance in a subject can be characterized by one or more of the following: a decreased level of a specific immunological response (e.g., mediated by antigen-specific effector T lymphocytes, B lymphocytes, or antibody); a delay in the onset or progression of a specific immunological response; or a reduced risk of the onset or progression of a specific immunological response, as compared to untreated subjects.

The term "prophylaxis" as used herein means the prevention of or protective treatment for a disease or disease state.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a cognate binding partner (e.g., a stimulatory and/or costimulatory molecule present on a T cell) protein present in a sample, but which antibody or ligand, does not substantially recognize or bind other molecules in the sample.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

"Relapsed" or "relapse" as used herein refers to the return or reappearance of a disease (e.g., cancer) or the signs and symptoms of a disease such as cancer after a period of improvement or responsiveness, e.g., after prior treatment of a therapy, e.g., cancer therapy. The initial period of responsiveness may involve the level of cancer cells falling below a certain threshold, e.g., below 20%, 1%, 10%, 5%, 4%, 3%, 2%, or 1%. The reappearance may involve the level of cancer cells rising above a certain threshold, e.g., above 20%, 1%, 10%, 5%, 4%, 3%, 2%, or 1%. For example, e.g., in the context of B-ALL, the reappearance may involve, e.g., a reappearance of blasts in the blood, bone marrow (> 5%), or any extramedullary site, after a complete response. A complete response, in this context, may involve < 5% BM blast. More generally, in an embodiment, a response (e.g., complete response or partial response) can involve the absence of detectable MRD (minimal residual disease). In an embodiment, the initial period of responsiveness lasts at least 1, 2, 3, 4, 5, or 6 days; at least 1, 2, 3, or 4 weeks; at least 1, 2, 3, 4, 6, 8, 10, or 12 months; or at least 1, 2, 3, 4, or 5 years.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes subranges such as 96-99%, 96-98%, 96-97%, 97-99%, 97-98% and 98-99% identity. This applies regardless of the breadth of the range.

### FUSION PROTEINS

Disclosed herein are fusion proteins including two protein domains separated by a heterologous protease cleavage site (e.g., a protease cleave site that is cleaved by a mammalian intracellular or extracellular protease), wherein a first of the protein domains is a conditional expression domain, e.g., a degradation domain or an aggregation domain. The fusion proteins described herein comprise three essential elements: a conditional expression domain, e.g., a degradation domain or an aggregation domain, a domain containing the protein of interest, and a protease cleavage domain separating the two. These elements can be arranged such that the conditional expression domain, e.g., a degradation domain or an aggregation domain, is located either N-terminal or C-terminal to the protein of interest. In the present invention, the conditional expression domain is a degradation domain.

In some embodiments, the fusion protein described herein includes a furin cleavage site. In some embodiments, the fusion proteins described herein include any one of furin cleavage sites listed in Table 20. In some embodiments, the fusion proteins described herein include a furin cleavage site selected from RTKR (SEQ ID NO: 123); GTGAEDPRPSRKRRSLGDVG (SEQ ID NO: 125) or a sequence having at least 90%, 95%, 97%, 98%, or 99% identity thereto; GTGAEDPRPSRKRR (SEQ ID NO: 127) or a sequence having at least 90%, 95%, 97%, 98%, or 99% identity thereto; LQWLEQQVAKRRTKR (SEQ ID NO: 129) or a sequence having at least 90%, 95%, 97%, 98%, or 99% identity thereto; GTGAEDPRPSRKRRSLGG (SEQ ID NO: 131) or a sequence having at least 90%, 95%, 97%, 98%, or 99% identity thereto; GTGAEDPRPSRKRRSLG (SEQ ID NO: 133) or a sequence having at least 90%, 95%, 97%, 98%, or 99% identity thereto; SLNLTESHNSRKKR (SEQ ID NO: 135) or a sequence having at least 90%, 95%, 97%, 98%, or 99% identity thereto; or CKINGYPKRGRKRR (SEQ ID NO: 137) or a sequence having at least 90%, 95%, 97%, 98%, or 99% identity thereto. In some embodiments, the fusion proteins described herein include a furin cleavage site selected from GTGAEDPRPSRKRRSLGDVG (SEQ ID NO: 125) or a sequence having at least 90%, 95%, 97%, 98%, or 99% identity thereto, or GTGAEDPRPSRKRR (SEQ ID NO: 127) or a sequence having at least 90%, 95%, 97%, 98%, or 99% identity thereto. In some embodiments, the fusion proteins described herein include the furin cleavage site of GTGAEDPRPSRKRRSLGDVG (SEQ ID NO: 125) or a sequence having at least 90%, 95%, 97%, 98%, or 99% identity thereto.

In some embodiments, the fusion proteins described herein include a furin cleavage site selected from RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); or CKINGYPKRGRKRR (SEQ ID NO: 137). In some embodiments, the fusion proteins described herein include a furin cleavage site selected from GTGAEDPRPSRKRRSLGDVG (SEQ ID NO: 125) or GTGAEDPRPSRKRR (SEQ ID NO: 127). In some embodiments, the fusion proteins described herein include the furin cleavage site of GTGAEDPRPSRKRRSLGDVG (SEQ ID NO: 125).

In some embodiments, the fusion protein of the invention includes a furin degron (FurON) domain, which comprises two components: a degron or degradation domain, which is a mutated protein domain unable to acquire a proper conformation in the absence of a small molecule ligand, and a furin cleavage site. The furin degron domain can be fused to a protein of interest that is expressed in the endoplasmic reticulum. The N- and/ or C-termini of the protein of interest can be used as site of fusion, provided that the furin degron domain faces the lumen of the endoplasmic reticulum. The protein of interest can be either a membrane protein (regardless of the number of transmembrane domains) or a soluble protein. The orientation of the furin cleavage site relative to the degron domain should be such that the cleavage results in the separation of the furin degron domain and protein of interest. In the absence of the small molecule ligand or stabilization compound, the furin degron domain leads to the destabilization or degradation of the entire fusion protein. In the presence of the small molecule ligand or stabilization compound, the fusion protein is spared from degradation. The furin degron domain is mutated such that the affinity to its natural endogenous ligand is abolished; the affinity to the small molecule ligand or stabilization compound is preserved; and protein instability is conferred when the small molecule is absent.

In some embodiments, the degron or degradation domain is derived from a protein listed in Table 21. In some embodiments, the degron or degradation domain is derived from an estrogen receptor. In some embodiments, the degron or degradation domain comprises an amino acid sequence selected from SEQ ID NO: 58 or a sequence having at least 90%, 95%, 97%, 98%, or 99% identity thereto, or SEQ ID NO: 121 or a sequence having at least 90%, 95%, 97%, 98%, or 99% identity thereto. In some embodiments, the degron or degradation domain comprises an amino acid sequence selected from SEQ ID NO: 58 or SEQ ID NO: 121.

In some embodiments, the degron or degradation domain is derived from an FKB protein (FKBP). In some embodiments, the degradation domain comprises an amino acid sequence of SEQ ID NO: 56 or a sequence having at least 90%, 95%, 97%, 98%, or 99% identity thereto. In some embodiments, the degradation domain comprises an amino acid sequence of SEQ ID NO: 56.

In some embodiments, the degron or degradation domain is derived from dihydrofolate reductase (DHFR). In some embodiments, the degradation domain comprises an amino acid sequence selected from SEQ ID NO: 57 or a sequence having at least 90%, 95%, 97%, 98%, or 99% identity thereto. In some embodiments, the degradation domain comprises an amino acid sequence selected from SEQ ID NO: 57. In some embodiments, the degron or degradation domain is not derived from an FKB protein, estrogen receptor, or DHFR.

### PROTEINS OF INTEREST

The fusion proteins disclosed herein can include virtually any protein of interest. In certain preferred instances, the protein of interest can be a transmembrane protein (e.g., a transmembrane receptor). The format of the fusion proteins disclosed herein are of particular use in such transmembrane proteins because inclusion of an intact conditional expression domain, e.g., degradation domain or, e.g., aggregation domain, on the N-terminus of a transmembrane receptor would possibly result in disruption of the activity of the protein of interest, while inclusion on the C-terminus may result in poor regulation of the protein of interest (because, e.g., integration into the membrane may result in sufficient stabilization of the protein or degradation domain to avoid degradation and/or aggreggation). One example of a class of proteins of interest is Chimeric Antigen T Cell Receptors as described in the section below.

### CHIMERIC ANTIGEN RECEPTOR

In the present invention, the protein of interest is a chimeric antigen receptor (CAR). In some embodiments, the protein of interest is a chimeric antigen receptor (CAR), wherein the CAR comprises an antigen binding domain (e.g., antibody or antibody fragment, TCR or TCR fragment) that binds to a tumor antigen as described herein, a transmembrane domain (e.g., a transmembrane domain described herein), and an intracellular signaling domain (e.g., an intracellular signaling domain described herein) (e.g., an intracellular signaling domain comprising a costimulatory domain (e.g., a costimulatory domain described herein) and/or a primary signaling domain (e.g., a primary signaling domain described herein). CAR nucleic acid constructs, encoded proteins, containing vectors, host cells, pharmaceutical compositions, and methods of administration and treatment related to the present disclosureare disclosed in detail in International Patent Application Publication No. WO2015142675.

In some embodiments, the protein of interest is a chimeric antigen receptor (CAR), wherein the CAR comprises an antigen binding domain (e.g., antibody or antibody fragment, TCR or TCR fragment) that binds to a tumor-supporting antigen (e.g., a tumor-supporting antigen as described herein), a transmembrane domain (e.g., a transmembrane domain described herein), and an intracellular signaling domain (e.g., an intracellular signaling domain described herein) (e.g., an intracellular signaling domain comprising a costimulatory domain (e.g., a costimulatory domain described herein) and/or a primary signaling domain (e.g., a primary signaling domain described herein). In some embodiments, the tumor-supporting antigen is an antigen present on a stromal cell or a myeloid-derived suppressor cell (MDSC). In other aspects, the invention features polypeptides encoded by such nucleic acids and host cells containing such nucleic acids and/or polypeptides.

In some embodiments, a CAR molecule comprises at least one intracellular signaling domain selected from a CD137 (4-1BB) signaling domain, a CD28 signaling domain, a CD27 signaling domain, an ICOS signaling domain, a CD3zeta signal domain, or any combination thereof. In some embodiments, a CAR molecule comprises at least one intracellular signaling domain selected from one or more costimulatory molecule(s) selected from CD137 (4-1BB), CD28, CD27, or ICOS.

Also disclosed herein is a method of modifying a T cell with a chimeric antigen receptor (CAR) and a suicide gene. Thus, the present invention encompasses a nucleic acid encoding a CAR or a modified T cell comprising a CAR, wherein the CAR includes an antigen binding domain, a transmembrane domain and an intracellular domain.

Example of CARs are described in U.S. Patent Nos.: 8,911,993, 8,906,682, 8,975,071, 8,916,381, 9,102,760, 9,101,584, and 9,102,761.

Sequences of non-limiting examples of various components that can be part of a CAR molecule are listed in Table 2, where "aa" stands for amino acids, and "na" stands for nucleic acids that encode the corresponding peptide.

**Table 2. Sequences of various components of CAR (aa - amino acids, na - nucleic acids that encodes the corresponding protein)**

| SEQ ID NO | description | Sequence |
|---|---|---|
| 1 | EF-1 promoter | CGTGAGGCTCCGGTGCCCGTCAGTGGGCAGAGCGCACATC GCCCACAGTCCCCGAGAAGTTGGGGGGAGGGGTCGGCAAT TGAACCGGTGCCTAGAGAAGGTGGCGCGGGGTAAACTGGG AAAGTGATGTCGTGTACTGGCTCCGCCTTTTTCCCGAGGGT GGGGGAGAACCGTATATAAGTGCAGTAGTCGCCGTGAACGT TCTTTTTCGCAACGGGTTTGCCGCCAGAACACAGGTAAGTG CCGTGTGTGGTTCCCGCGGGCCTGGCCTCTTTACGGGTTAT GGCCCTTGCGTGCCTTGAATTACTTCCACCTGGCTGCAGTA CGTGATTCTTGATCCCGAGCTTCGGGTTGGAAGTGGGTGGG AGAGTTCGAGGCCTTGCGCTTAAGGAGCCCCTTCGCCTCGT GCTTGAGTTGAGGCCTGGCCTGGGCGCTGGGGCCGCCGCG TGCGAATCTGGTGGCACCTTCGCGCCTGTCTCGCTGCTTTC GATAAGTCTCTAGCCATTTAAAATTTTTGATGACCTGCTGCG ACGCTTTTTTTCTGGCAAGATAGTCTTGTAAATGCGGGCCAA GATCTGCACACTGGTATTTCGGTTTTTGGGGCCGCGGGCGG CGACGGGGCCCGTGCGTCCCAGCGCACATGTTCGGCGAGG CGGGGCCTGCGAGCGCGGCCACCGAGAATCGGACGGGGG TAGTCTCAAGCTGGCCGGCCTGCTCTGGTGCCTGGCCTCGC GCCGCCGTGTATCGCCCCGCCCTGGGCGGCAAGGCTGGCC CGGTCGGCACCAGTTGCGTGAGCGGAAAGATGGCCGCTTC CCGGCCCTGCTGCAGGGAGCTCAAAATGGAGGACGCGGCG CTCGGGAGAGCGGGCGGGTGAGTCACCCACACAAAGGAAA AGGGCCTTTCCGTCCTCAGCCGTCGCTTCATGTGACTCCAC GGAGTACCGGGCGCCGTCCAGGCACCTCGATTAGTTCTCGA GCTTTTGGAGTACGTCGTCTTTAGGTTGGGGGGAGGGGTTT TATGCGATGGAGTTTCCCCACACTGAGTGGGTGGAGACTGA AGTTAGGCCAGCTTGGCACTTGATGTAATTCTCCTTGGAATT |
| | | TGCCCTTTTTGAGTTTGGATCTTGGTTCATTCTCAAGCCTCA GACAGTGGTTCAAAGTTTTTTTCTTCCATTTCAGGTGTCGTG A |
| 2 | Leader (aa) | MALPVTALLLPLALLLHAARP |
| 3 | Leader (na) | ATGGCCCTGCCTGTGACAGCCCTGCTGCTGCCTCTGGCTCT GCTGCTGCATGCCGCTAGACCC |
| 33 | Leader (na) | ATGGCCCTCCCTGTCACCGCCCTGCTGCTTCCGCTGGCTCT TCTGCTCCACGCCGCTCGGCCC |
| 4 | CD 8 hinge (aa) | TTTPAPRPPTPAPTIASQPLSLRPEACRPAAGGAVHTRGLDFA CD |
| 5 | CD8 hinge (na) | ACCACGACGCCAGCGCCGCGACCACCAACACCGGCGCCCA CCATCGCGTCGCAGCCCCTGTCCCTGCGCCCAGAGGCGTG CCGGCCAGCGGCGGGGGGCGCAGTGCACACGAGGGGGCT GGACTTCGCCTGTGAT |
| 6 | Ig4 hinge (aa) | ESKYGPPCPPCPAPEFLGGPSVFLFPPKPKDTLMISRTPEVTCV VVDVSQEDPEVQFNWYVDGVEVHNAKTKPREEQFNSTYRVVS VLTVLHQDWLNGKEYKCKVSNKGLPSSIEKTISKAKGQPREPQ VYTLPPSQEEMTKNQVSLTCLVKGFYPSDIAVEWESNGQPENN YKTTPPVLDSDGSFFLYSRLTVDKSRWQEGNVFSCSVMHEAL HNHYTQKSLSLSLGKM |
| 7 | Ig4 hinge (na) | GAGAGCAAGTACGGCCCTCCCTGCCCCCCTTGCCCTGCCC CCGAGTTCCTGGGCGGACCCAGCGTGTTCCTGTTCCCCCCC AAGCCCAAGGACACCCTGATGATCAGCCGGACCCCCGAGG TGACCTGTGTGGTGGTGGACGTGTCCCAGGAGGACCCCGA GGTCCAGTTCAACTGGTACGTGGACGGCGTGGAGGTGCAC AACGCCAAGACCAAGCCCCGGGAGGAGCAGTTCAATAGCA CCTACCGGGTGGTGTCCGTGCTGACCGTGCTGCACCAGGA CTGGCTGAACGGCAAGGAATACAAGTGTAAGGTGTCCAACA AGGGCCTGCCCAGCAGCATCGAGAAAACCATCAGCAAGGC CAAGGGCCAGCCTCGGGAGCCCCAGGTGTACACCCTGCCC CCTAGCCAAGAGGAGATGACCAAGAACCAGGTGTCCCTGAC CTGCCTGGTGAAGGGCTTCTACCCCAGCGACATCGCCGTG GAGTGGGAGAGCAACGGCCAGCCCGAGAACAACTACAAGA CCACCCCCCCTGTGCTGGACAGCGACGGCAGCTTCTTCCTG TACAGCCGGCTGACCGTGGACAAGAGCCGGTGGCAGGAGG GCAACGTCTTTAGCTGCTCCGTGATGCACGAGGCCCTGCAC AACCACTACACCCAGAAGAGCCTGAGCCTGTCCCTGGGCAA GATG |
| 8 | IgD hinge (aa) | RWPESPKAQASSVPTAQPQAEGSLAKATTAPATTRNTGRGGE EKKKEKEKEEQEERETKTPECPSHTQPLGVYLLTPAVQDLWLR DKATFTCFVVGSDLKDAHLTWEVAGKVPTGGVEEGLLERHSN GSQSQHSRLTLPRSLWNAGTSVTCTLNHPSLPPQRLMALREP AAQAPVKLSLNLLASSDPPEAASWLLCEVSGFSPPNILLMWLE DQREVNTSGFAPARPPPQPGSTTFWAWSVLRVPAPPSPQPAT YTCVVSHEDSRTLLNASRSLEVSYVTDH |
| 9 | IgD hinge (na) | AGGTGGCCCGAAAGTCCCAAGGCCCAGGCATCTAGTGTTCC TACTGCACAGCCCCAGGCAGAAGGCAGCCTAGCCAAAGCTA CTACTGCACCTGCCACTACGCGCAATACTGGCCGTGGCGG GGAGGAGAAGAAAAAGGAGAAAGAGAAAGAAGAACAGGAA GAGAGGGAGACCAAGACCCCTGAATGTCCATCCCATACCCA GCCGCTGGGCGTCTATCTCTTGACTCCCGCAGTACAGGACT TGTGGCTTAGAGATAAGGCCACCTTTACATGTTTCGTCGTGG GCTCTGACCTGAAGGATGCCCATTTGACTTGGGAGGTTGCC GGAAAGGTACCCACAGGGGGGGTTGAGGAAGGGTTGCTGG AGCGCCATTCCAATGGCTCTCAGAGCCAGCACTCAAGACTC |
| | | ACCCTTCCGAGATCCCTGTGGAACGCCGGGACCTCTGTCAC ATGTACTCTAAATCATCCTAGCCTGCCCCCACAGCGTCTGAT GGCCCTTAGAGAGCCAGCCGCCCAGGCACCAGTTAAGCTTA GCCTGAATCTGCTCGCCAGTAGTGATCCCCCAGAGGCCGCC AGCTGGCTCTTATGCGAAGTGTCCGGCTTTAGCCCGCCCAA CATCTTGCTCATGTGGCTGGAGGACCAGCGAGAAGTGAACA CCAGCGGCTTCGCTCCAGCCCGGCCCCCACCCCAGCCGGG TTCTACCACATTCTGGGCCTGGAGTGTCTTAAGGGTCCCAG CACCACCTAGCCCCCAGCCAGCCACATACACCTGTGTTGTG TCCCATGAAGATAGCAGGACCCTGCTAAATGCTTCTAGGAG TCTGGAGGTTTCCTACGTGACTGACCATT |
| 10 | GS hinge/linker (aa) | GGGGSGGGGS |
| 11 | GS hinge/linker (na) | GGTGGCGGAGGTTCTGGAGGTGGAGGTTCC |
| 12 | CD8TM (aa) | IYIWAPLAGTCGVLLLSLVITLYC |
| 13 | CD8 TM (na) | ATCTACATCTGGGCGCCCTTGGCCGGGACTTGTGGGGTCCT TCTCCTGTCACTGGTTATCACCCTTTACTGC |
| 34 | CD8 TM (na) | ATCTACATTTGGGCCCCTCTGGCTGGTACTTGCGGGGTCCT GCTGCTTTCACTCGTGATCACTCTTTACTGT |
| 14 | 4-1BB intracellular domain (aa) | KRGRKKLLYIFKQPFMRPVQTTQEEDGCSCRFPEEEEGGCEL |
| 15 | 4-1BB intracellular domain (na) | AAACGGGGCAGAAAGAAACTCCTGTATATATTCAAACAACCA TTTATGAGACCAGTACAAACTACTCAAGAGGAAGATGGCTGT AGCTGCCGATTTCCAGAAGAAGAAGAAGGAGGATGTGAACT G |
| 118 | 4-1BB intracellular domain (na) | AAGCGCGGTCGGAAGAAGCTGCTGTACATCTTTAAGCAACC CTTCATGAGGCCTGTGCAGACTACTCAAGAGGAGGACGGCT GTTCATGCCGGTTCCCAGAGGAGGAGGAAGGCGGCTGCGA ACTG |
| 16 | CD27 (aa) | QRRKYRSNKGESPVEPAEPCRYSCPREEEGSTIPIQEDYRKPE PACSP |
| 17 | CD27 (na) | AGGAGTAAGAGGAGCAGGCTCCTGCACAGTGACTACATGAA CATGACTCCCCGCCGCCCCGGGCCCACCCGCAAGCATTAC CAGCCCTATGCCCCACCACGCGACTTCGCAGCCTATCGCTC C |
| 18 | CD3-zeta (aa) | RVKFSRSADAPAYKQGQNQLYNELNLGRREEYDVLDKRRGRD PEMGGKPRRKNPQEGLYNELQKDKMAEAYSEIGMKGERRRG KGHDGLYQGLSTATKDTYDALHMQALPPR |
| 19 | CD3-zeta (na) | AGAGTGAAGTTCAGCAGGAGCGCAGACGCCCCCGCGTACA AGCAGGGCCAGAACCAGCTCTATAACGAGCTCAATCTAGGA CGAAGAGAGGAGTACGATGTTTTGGACAAGAGACGTGGCCG GGACCCTGAGATGGGGGGAAAGCCGAGAAGGAAGAACCCT CAGGAAGGCCTGTACAATGAACTGCAGAAAGATAAGATGGC GGAGGCCTACAGTGAGATTGGGATGAAAGGCGAGCGCCGG AGGGGCAAGGGGCACGATGGCCTTTACCAGGGTCTCAGTA CAGCCACCAAGGACACCTACGACGCCCTTCACATGCAGGCC CTGCCCCCTCGC |
| 119 | CD3-zeta (na) | CGCGTGAAATTCAGCCGCAGCGCAGATGCTCCAGCCTACAA GCAGGGGCAGAACCAGCTCTACAACGAACTCAATCTTGGTC GGAGAGAGGAGTACGACGTGCTGGACAAGCGGAGAGGACG GGACCCAGAAATGGGCGGGAAGCCGCGCAGAAAGAATCCC |
| | | CAAGAGGGCCTGTACAACGAGCTCCAAAAGGATAAGATGGC AGAAGCCTATAGCGAGATTGGTATGAAAGGGGAACGCAGAA GAGGCAAAGGCCACGACGGACTGTACCAGGGACTCAGCAC CGCCACCAAGGACACCTATGACGCTCTTCACATGCAGGCCC TGCCGCCTCGG |
| 20 | CD3-zeta (aa) | RVKFSRSADAPAYQQGQNQLYNELNLGRREEYDVLDKRRGRD PEMGGKPRRKNPQEGLYNELQKDKMAEAYSEIGMKGERRRG KGHDGLYQGLSTATKDTYDALHMQALPPR |
| 21 | CD3-zeta (na) | AGAGTGAAGTTCAGCAGGAGCGCAGACGCCCCCGCGTACC AGCAGGGCCAGAACCAGCTCTATAACGAGCTCAATCTAGGA CGAAGAGAGGAGTACGATGTTTTGGACAAGAGACGTGGCCG GGACCCTGAGATGGGGGGAAAGCCGAGAAGGAAGAACCCT CAGGAAGGCCTGTACAATGAACTGCAGAAAGATAAGATGGC GGAGGCCTACAGTGAGATTGGGATGAAAGGCGAGCGCCGG AGGGGCAAGGGGCACGATGGCCTTTACCAGGGTCTCAGTA CAGCCACCAAGGACACCTACGACGCCCTTCACATGCAGGCC CTGCCCCCTCGC |
| 22 | linker | GGGGS |
| 23 | linker | GGTGGCGGAGGTTCTGGAGGTGGAGGTTCC |
| 24 | PD-1 extracellular domain (aa) | |
| 25 | PD-1 extracellular domain (na) | Cccggatggtttctggactctccggatcgcccgtggaatcccccaaccttctcaccggca ctcttggttgtgactgagggcgataatgcgaccttcacgtgctcgttctccaacacctccga atcattcgtgctgaactggtaccgcatgagcccgtcaaaccagaccgacaagctcgccg cgtttccggaagatcggtcgcaaccgggacaggattgtcggttccgcgtgactcaactgc cgaatggcagagacttccacatgagcgtggtccgcgctaggcgaaacgactccggga cctacctgtgcggagccatctcgctggcgcctaaggcccaaatcaaagagagcttgagg gccgaactgagagtgaccgagcgcagagctgaggtgccaactgcacatccatccccat cgcctcggcctgcggggcagtttcagaccctggtc |
| 26 | PD-1 CAR (aa) with signal | Malpvtalllplalllhaarppgwfldspdrpwnpptfspallvvtegdnatftcsfsntsesfv lnwyrmspsnqtdklaafpedrsqpgqdcrfrvtqlpngrdfhmsvvrarrndsgtylcg aislapkaqikeslraelrvterraevptahpspsprpagqfqtlvtttpaprpptpaptiasq plslrpeacrpaaggavhtrgldfacdiyiwaplagtcgvlllslvitlyckrgrkkllyifkqpfm rpvqttqeedgcscrfpeeeeggcelrvkfsrsadapaykqgqnqlynelnlgrreeydvl dkrrgrdpemggkprrknpqeglynelqkdkmaeayseigmkgerrrgkghdglyqgl statkdtydalhmqalppr |
| 27 | PD-1 CAR (na) | Atggccctccctgtcactgccctgcttctccccctcgcactcctgctccacgccgctagacc acccggatggtttctggactctccggatcgcccgtggaatcccccaaccttctcaccggca ctcttggttgtgactgagggcgataatgcgaccttcacgtgctcgttctccaacacctccga atcattcgtgctgaactggtaccgcatgagcccgtcaaaccagaccgacaagctcgccg cgtttccggaagatcggtcgcaaccgggacaggattgtcggttccgcgtgactcaactgc cgaatggcagagacttccacatgagcgtggtccgcgctaggcgaaacgactccggga cctacctgtgcggagccatctcgctggcgcctaaggcccaaatcaaagagagcttgagg gccgaactgagagtgaccgagcgcagagctgaggtgccaactgcacatccatccccat cgcctcggcctgcggggcagtttcagaccctggtcacgaccactccggcgccgcgccc accgactccggccccaactatcgcgagccagcccctgtcgctgaggccggaagcatgc cgccctgccgccggaggtgctgtgcatacccggggattggacttcgcatgcgacatctac atttgggctcctctcgccggaacttgtggcgtgctccttctgtccctggtcatcaccctgtactg caagcggggtcggaaaaagcttctgtacattttcaagcagcccttcatgaggcccgtgca aaccacccaggaggaggacggttgctcctgccggttccccgaagaggaagaaggag gttgcgagctgcgcgtgaagttctcccggagcgccgacgcccccgcctataagcaggg ccagaaccagctgtacaacgaactgaacctgggacggcgggaagagtacgatgtgct ggacaagcggcgcggccgggaccccgaaatgggcgggaagcctagaagaaagaa ccctcaggaaggcctgtataacgagctgcagaaggacaagatggccgaggcctactc |
| | | cgaaattgggatgaagggagagcggcggaggggaaaggggcacgacggcctgtac caaggactgtccaccgccaccaaggacacatacgatgccctgcacatgcaggcccttc cccctcgc |
| 28 | linker | (Gly-Gly-Gly-Ser)n, where n = 1-10 |
| 29 | linker | (Gly4 Ser)4 |
| 30 | linker | (Glv4 Ser)3 |
| 31 | linker | (Gly3Ser) |
| 32 | polyA | [a]₅₀₋₅₀₀₀ |
| 39 | PD1 CAR (aa) | Pgwfldspdrpwnpptfspallvvtegdnatftcsfsntsesfvlnwyrmspsnqtdklaa fpedrsqpgqdcrfrvtqlpngrdfhmsvvrarrndsgtylcgaislapkaqikeslraelrvt erraevptahpspsprpagqfqtlvtttpaprpptpaptiasqplslrpeacrpaaggavht rgldfacdiyiwaplagtcgvlllslvitlyckrgrkkllyifkqpfmrpvqttqeedgcscrfpee eeggcelrvkfsrsadapaykqgqnqlynelnlgrreeydvldkrrgrdpemggkprrkn pqeglynelqkdkmaeayseigmkgerrrgkghdglyqglstatkdtydalhmqalppr |
| 120 | ICOS intracellular domain (aa) | TKKKYSSSVHDPNGEYMFMRAVNTAKKSRLTDVTL |
| 1111 | ICOS intracellular domain (na) | ACAAAAAAGAAGTATTCATCCAGTGTGCACGACCCTAACGGT GAATACATGTTCATGAGAGCAGTGAACACAGCCAAAAAATCC AGACTCACAGATGTGACCCTA |
| 972 | ICOS TM domain (aa) | TTTPAPRPPTPAPTIASQPLSLRPEACRPAAGGAVHTRGLDFA CDFWLPIGCAAFVVVCILGCILICWL |
| 973 | ICOS TM domain (na) | ACCACGACGCCAGCGCCGCGACCACCAACACCGGCGCCCA CCATCGCGTCGCAGCCCCTGTCCCTGCGCCCAGAGGCGTG CCGGCCAGCGGCGGGGGGCGCAGTGCACACGAGGGGGCT GGACTTCGCCTGTGATTTCTGGTTACCCATAGGATGTGCAG CCTTTGTTGTAGTCTGCATTTTGGGATGCATACTTATTTGTTG GCTT |
| 124 | CD28 intracellular domain (aa) | RSKRSRLLHSDYMNMTPRRPGPTRKHYQPYAPPRDFAAYRS |
| 1113 | CD28 intracellular domain (na) | AGGAGTAAGAGGAGCAGGCTCCTGCACAGTGACTACATGAA CATGACTCCCCGCCGCCCCGGGCCCACCCGCAAGCATTAC CAGCCCTATGCCCCACCACGCGACTTCGCAGCCTATCGCTC C |

In specific aspects, a CAR construct comprises a scFv domain, wherein the scFv may be preceded by an optional leader sequence such as provided in SEQ ID NO: 2, and followed by an optional hinge sequence such as provided in SEQ ID NO:4 or SEQ ID NO:6 or SEQ ID NO:8 or SEQ ID NO:10, a transmembrane region such as provided in SEQ ID NO:12, an intracellular signalling domain that includes any one of SEQ ID NO:14, SEQ ID NO:16, SEQ ID NO: 120, or SEQ ID NO: 124, and a CD3 zeta sequence that includes SEQ ID NO: 18 or SEQ ID NO: 20, e.g., wherein the domains are contiguous with and in the same reading frame to form a single fusion protein.

In one aspect, an exemplary CAR constructs comprise an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), and an intracellular stimulatory domain (e.g., an intracellular stimulatory domain described herein). In one aspect, an exemplary CAR construct comprises an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), an intracellular costimulatory signaling domain (e.g., a costimulatory signaling domain described herein) and/or an intracellular primary signaling domain (e.g., a primary signaling domain described herein).

An exemplary leader sequence is provided as SEQ ID NO: 2. An exemplary hinge/spacer sequence is provided as SEQ ID NO: 4 or SEQ ID NO: 6 or SEQ ID NO: 8 or SEQ ID NO: 10. An exemplary transmembrane domain sequence is provided as SEQ ID NO: 12. An exemplary sequence of the intracellular signaling domain of the 4-1BB protein is provided as SEQ ID NO: 14. An exemplary sequence of the intracellular signaling domain of CD27 is provided as SEQ ID NO: 16. An exemplary sequence of the intracellular signaling domain of ICOS is provided as SEQ ID NO: 120. An exemplary sequence of the intracellular signaling domain of CD28 is provided as SEQ ID NO: 124. An exemplary CD3zeta domain sequence is provided as SEQ ID NO: 18 or SEQ ID NO: 20.

The nucleic acid sequences coding for the desired molecules can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

### Antigen Binding Domain

In one aspect, a chimeric antigen receptor (CAR) comprises a target-specific binding element otherwise referred to as an antigen binding domain. The choice of moiety depends upon the type and number of ligands that define the surface of a target cell. For example, the antigen binding domain may be chosen or engineered to recognize a ligand that acts as a cell surface marker on target cells associated with a particular disease state, e.g., a tumor antigen associated with a particular cancer (e.g., an antigen binding domain that binds to a tumor antigen). In other embodiments, the antigen binding domain is chosen or engineered to recognize normal B cells, or a subpopulation of B cells, for depleting normal B cells or a target B cell population (e.g., an antigen binding domain that binds to a B cell antigen).

The antigen binding domain can be any domain that binds to the antigen including but not limited to a monoclonal antibody, a polyclonal antibody, a recombinant antibody, a bispecific antibody, a conjugated antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, and to an alternative scaffold known in the art to function as antigen binding domain, such as a recombinant fibronectin domain, a T cell receptor (TCR), a recombinant TCR with enhanced affinity, or a fragment there of, e.g., single chain TCR, and the like. In some instances, it is beneficial for the antigen binding domain to be derived from the same species in which the CAR will ultimately be used in. For example, for use in humans, it may be beneficial for the antigen binding domain of the CAR to comprise human or humanized residues for the antigen binding domain of an antibody or antibody fragment.

In some embodiments, the antigen binding domain of the encoded CAR molecule comprises an antibody, an antibody fragment, an scFv, a Fv, a Fab, a (Fab')2, a single domain antibody (SDAB), a VH or VL domain, a camelid VHH domain or a bi-functional (e.g. bi-specific) hybrid antibody (e.g., Lanzavecchia et al., Eur. J. Immunol. 17, 105 (1987)).

In some embodiments, the antigen binding domain of the encoded CAR molecule comprises an scFv. In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers.

In one aspect, the antigen binding domain of the CAR is a scFv antibody fragment that is humanized compared to the murine sequence of the scFv from which it is derived.

In one aspect, the antigen binding domain of a CAR of the invention (e.g., a scFv) is encoded by a nucleic acid molecule whose sequence has been codon optimized for expression in a mammalian cell. In one aspect, entire CAR construct of the invention is encoded by a nucleic acid molecule whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148. In one embodiment, the CAR comprises an antigen binding domain that binds to a B cell. Cell surface markers selectively found on B cells may act as an antigen that binds to the antigen binding domain of the CAR. The anti-B cell antigen binding domain can include any domain that binds to the B cell and may include, but is not limited to, a monoclonal antibody, a polyclonal antibody, a synthetic antibody, a human antibody, a humanized antibody, a non-human antibody, and any fragment thereof. Thus, in one embodiment, the antigen binding domain portion comprises a mammalian antibody or a fragment thereof, such as a single chain variable fragment (scFv). The antigen binding domain may bind one or more antigens, such as, but not limited to, any surface marker selectively found on a B cell, such as a pro-B cell, pre-B cell, immature B cell, mature B cell, memory B cell, and plasma cell. In one embodiment, the antigen binding domain binds at least one antigen, such as CD19, BCMA, and any combination thereof. In another embodiment, the antigen binding domain binds at least one antigen, such as CD20, CD21, CD27, CD38, CD138, , and any combination thereof. In some instances, it is beneficial for the antigen binding domain to be derived from the same species in which the CAR will ultimately be used in. For example, for use in humans, it may be beneficial for the antigen binding domain of the CAR to comprise a human antibody, humanized antibody as described elsewhere herein, or a fragment thereof.

### Tumor antigens

In some embodiments, an antigen binding domain of the CAR specifically targets a tumor antigen. There are two classes of tumor antigens (tumour antigens, TA) that can be targeted by CARs: (1) a tumor antigen that is expressed on the surface of cancer cells; and (2) a tumor antigen that itself is intracellular, however, a fragment of such antigen (peptide) is presented on the surface of the cancer cells by MHC (major histocompatibility complex).

In one embodiment, the tumor antigen is expressed on both normal cells and cancer cells, but is expressed at lower levels on normal cells. In one embodiment, the method further comprises selecting a CAR that binds a tumor antigen with an affinity that allows the cell engineered to express the CAR to bind and kill the cancer cells expressing a tumor antigen but less than 30%, 25%, 20%, 15%, 10%, 5% or less of the normal cells expressing a tumor antigen are killed, e.g., as determined by an assay described herein. For example, a killing assay such as flow cytometry based on Cr51 CTL can be used. In one embodiment, the selected CAR has an antigen binding domain that has a binding affinity K_{D} of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the selected antigen binding domain has a binding affinity that is at least five-fold, 10-fold, 20-fold, 30-fold, 50-fold, 100-fold or 1,000-fold less than a reference antibody, e.g., an antibody described herein.

Accordingly, a cell can be engineered to express a CAR comprising an antigen binding domain that can target, e.g., bind to, any one of the exemplary tumor antigens (tumour antigens): CD123, CD30, CD171, CS-1, CLL-1 (CLECL1), CD33, EGFRvIII, GD2, GD3, Tn Ag , sTn Ag, Tn-O-Glycopeptides, Stn-O-Glycopeptides, PSMA, FLT3, FAP, TAG72, CD44v6, CEA, EPCAM, B7H3, KIT, IL-13Ra2, Mesothelin, IL-11Ra, PSCA, VEGFR2, LewisY, PDGFR-beta, PRSS21, SSEA-4, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, LAIR1, FCAR, LILRA2, CD300LF, CLEC12A, BST2, EMR2, LY75, GPC3, FCRL5, IGLL1, CD10, CD19, CD20, CD21, CD22, CD23, CD24, CD25, CD37, CD38, CD53, CD72, CD73, CD74, CD75, CD77, CD79a, CD79b, CD80, CD81, CD82, CD83, CD84, CD85, ROR1, BCMA, CD86, and CD179b. Other B cell antigens that can be targeted by a CAR described herein include: CD1a, CD1b, CD1c, CD1d, CD2, CD5, CD6, CD9, CD11a, CD11b, CD11c, CD17, CD18, CD26, CD27, CD29, CD30, CD31, CD32a, CD32b, CD35, CD38, CD39, CD40, CD44, CD45, CD45RA, CD45RB, CD45RC, CD45RO, CD46, CD47, CD48, CD49b, CD49c, CD49d, CD50, CD52, CD54, CD55, CD58, CD60a, CD62L, CD63, CD63, CD68 CD69, CD70, CD85E, CD85I, CD85J, CD92, CD95, CD97, CD98, CD99, CD100, CD102, CD108, CD119, CD120a, CD120b, CD121b, CD122, CD124, CD125, CD126, CD130, CD132, CD137, CD138, CD139, CD147, CD148, CD150, CD152, CD162, CD164, CD166, CD167a, CD170, CD175, CD175s, CD180, CD184, CD185, CD192, CD196, CD197, CD200, CD205, CD210a, CDw210b, CD212, CD213a1, CD213a2, CD215, CD217, CD218a, CD218b, CD220, CD221 , CD224, CD225, CD226, CD227, CD229, CD230, CD232, CD252, CD253, CD257, CD258, CD261, CD262, CD263, CD264, CD267, CD268, CD269, CD270, CD272, CD274, CD275, CD277, CD279, CD283, CD289, CD290, CD295, CD298, CD300a, CD300c, CD305, CD306, CD307a, CD307b, CD307c, CD307d, CD307e, CD314, CD315, CD316, CD317, CD319, CD321, CD327, CD328, CD329, CD338, CD351, CD352, CD353, CD354, CD355, CD357, CD358, CD360, CD361, CD362, CD363 and peptides of these antigens presented on MHC.

In some embodiments, the antigen binding domain of a CAR targets a tumor antigen that is associated with a solid tumor, e.g., expressed by a solid tumor cell, referred to herein as a solid tumor associated antigen, e.g., an antigen associated with mesothelioma (e.g., malignant pleural mesothelioma), lung cancer (e.g., non-small cell lung cancer, small cell lung cancer, squamous cell lung cancer, or large cell lung cancer), pancreatic cancer (e.g., pancreatic ductal adenocarcinoma), esophageal adenocarcinoma, ovarian cancer, breast cancer, colorectal cancer and bladder cancer or any combination thereof. In one embodiment, the disease is pancreatic cancer, e.g., metastatic pancreatic ductal adenocarcinoma (PDA), e.g., in a subject who has progressed on at least one prior standard therapy. In one embodiment, the disease is mesothelioma (e.g., malignant pleural mesothelioma), e.g., in a subject who has progressed on at least one prior standard therapy. In one embodiment, the disease is ovarian cancer, e.g., serous epithelial ovarian cancer, e.g., in a subject who has progressed after at least one prior regimen of standard therapy.

Examples of tumor associated antigens (e.g., solid tumor antigens) include, without limitation: EGFRvIII, mesothelin, GD2, Tn antigen, sTn antigen, Tn-O-Glycopeptides, sTn-O-Glycopeptides, PSMA, CD97, TAG72, CD44v6, CEA, EPCAM, KIT, IL-13Ra2, leguman, GD3, CD171, IL-11Ra, PSCA, MAD-CT-1, MAD-CT-2, VEGFR2, LewisY, CD24, PDGFR-beta, SSEA-4, folate receptor alpha, ERBBs (e.g., ERBB2), Her2/neu, MUC1, EGFR, NCAM, Ephrin B2, CAIX, LMP2, sLe, HMWMAA, o-acetyl-GD2, folate receptor beta, TEM1/CD248, TEM7R, FAP, Legumain, HPV E6 or E7, ML-IAP, CLDN6, TSHR, GPRC5D, ALK, Polysialic acid, Fos-related antigen, neutrophil elastase, TRP-2, CYP1B1, sperm protein 17, beta human chorionic gonadotropin, AFP, thyroglobulin, PLAC1, globoH, RAGE1, MN-CA IX, human telomerase reverse transcriptase, intestinal carboxyl esterase, mut hsp 70-2, NA-17, NY-BR-1, UPK2, HAVCR1, ADRB3, PANX3, NY-ESO-1, GPR20, Ly6k, OR51E2, TARP, GFRα4, and a peptide of any of these antigens presented on MHC.

In an embodiment, the antigen binding domain of a CAR binds to human mesothelin. In an embodiment, the antigen binding domain is a murine scFv domain that binds to human mesothelin, e.g., SS1 as shown in Table 3. In an embodiment, the antigen binding domain is a humanized antibody or antibody fragment, e.g., scFv domain, derived from the murine SS1 scFv. In an embodiment, the antigen binding domain is a human antibody or antibody fragment that binds to human mesothelin. Exemplary human scFv domains (and their sequences) and the murine SS1 scFv that bind to mesothelin are provided in Table 3. CDR sequences are underlined. The scFv domain sequences provided in Table 3 include a light chain variable region (VL) and a heavy chain variable region (VH). The VL and VH are attached by a linker comprising the sequence GGGGSGGGGSGGGGS (SEQ ID NO: 30) (e.g., as shown in SS1 scFv domains) or GGGGSGGGGSGGGGSGGGGS (SEQ ID NO: 29) (e.g., as shown in M1, M2, M3, M4, M5, M6, M7, M8, M9, M10, M11, M12, M13, M14, M15, M16, M17, M18, M19, M20, M21, M22, M23, or M24 scFv domains). The scFv domains listed in Table 3 are in the following orientation: VL-linker-VH.

**Table 3. Antigen binding domains that bind to mesothelin**

| **Tumor antigen** | **Name** | **Amino acid sequence** | **SEQ ID NO:** |
|---|---|---|---|
| mesothelin | M5 (human) | QVQLVQSGAEVEKPGASVKVSCKASGYTFTDYYMHWVRQAPGQ GLEWMGWINPNSGGTNYAQKFQGRVTMTRDTSISTAYMELSRLR SDDTAVYYCASGWDFDYWGQGTLVTVSSGGGGSGGGGSGGGG SGGGGSDIVMTQSPSSLSASVGDRVTITCRASQSIRYYLSWYQQK PGKAPKLLIYTASILQNGVPSRFSGSGSGTDFTLTISSLQPEDFATY YCLQTYTTPDFGPGTKVEIK | 201 |
| mesothelin | M11 (human) | QVQLQQSGAEVKKPGASVKVSCKASGYTFTGYYMHWVRQAPGQ GLEWMGWINPNSGGTNYAQNFQGRVTMTRDTSISTAYMELRRLR SDDTAVYYCASGWDFDYWGQGTLVTVSSGGGGSGGGGSGGGG SGGGGSDIRMTQSPSSLSASVGDRVTITCRASQSIRYYLSWYQQK PGKAPKLLIYTASILQNGVPSRFSGSGSGTDFTLTISSLQPEDFATY YCLQTYTTPDFGPGTKVEIK | 202 |
| mesothelin | ss1 (murine) | QVQLQQSGPELEKPGASVKISCKASGYSFTGYTMNWVKQSHGKS LEWIGLITPYNGASSYNQKFRGKATLTVDKSSSTAYMDLLSLTSED SAVYFCARGGYDGRGFDYWGQGTTVTVSSGGGGSGGGGSGGG GSDIELTQSPAIMSASPGEKVTMTCSASSSVSYMHWYQQKSGTS PKRWIYDTSKLASGVPGRFSGSGSGNSYSLTISSVEAEDDATYYC QQWSGYPLTFGAGTKLEI | 203 |
| mesothelin | M1 (human) | QVQLQQSGAEVKKPGASVKVSCKASGYTFTGYYMHWVRQAPGQ GLEWMGRINPNSGGTNYAQKFQGRVTMTRDTSISTAYMELSRLR SEDTAVYYCARGRYYGMDVWGQGTMVTVSSGGGGSGGGGSGG | 204 |
| | | GGSGGGGSEIVLTQSPATLSLSPGERATISCRASQSVSSNFAWYQ QRPGQAPRLLIYDASNRATGIPPRFSGSGSGTDFTLTISSLEPEDF AAYYCHQRSNWLYTFGQGTKVDIK | |
| mesothelin | M2 (human) | QVQLVQSGAEVKKPGASVKVSCKASGYTFTGYYMHWVRQAPGQ GLEWMGWINPNSGGTNYAQKFQGRVTMTRDTSISTAYMELSRLR SDDTAVYYCARDLRRTVVTPRAYYGMDVWGQGTTVTVSSGGGG SGGGGSGGGGSGGGGSDIQLTQSPSTLSASVGDRVTITCQASQD ISNSLNWYQQKAGKAPKLLIYDASTLETGVPSRFSGSGSGTDFSFT ISSLQPEDIATYYCQQHDNLPLTFGQGTKVEIK | 205 |
| mesothelin | M3 (human) | QVQLVQSGAEVKKPGAPVKVSCKASGYTFTGYYMHWVRQAPGQ GLEWMGWINPNSGGTNYAQKFQGRVTMTRDTSISTAYMELSRLR SDDTAVYYCARGEWDGSYYYDYWGQGTLVTVSSGGGGSGGGG SGGGGSGGGGSDIVLTQTPSSLSASVGDRVTITCRASQSINTYLN WYQHKPGKAPKLLIYAASSLQSGVPSRFSGSGSGTDFTLTISSLQP EDFATYYCQQSFSPLTFGGGTKLEIK | 206 |
| mesothelin | M4 (human) | QVQLVESGGGLVQPGGSLRLSCAASGFTFSSYWMHWVRQVPGK GLVWVSRINTDGSTTTYADSVEGRFTISRDNAKNTLYLQMNSLRD DDTAVYYCVGGHWAVWGQGTTVTVSSGGGGSGGGGSGGGGS GGGGSDIQMTQSPSTLSASVGDRVTITCRASQSISDRLAWYQQKP GKAPKLLIYKASSLESGVPSRFSGSGSGTEFTLTISSLQPDDFAVY YCQQYGHLPMYTFGQGTKVEIK | 207 |
| mesothelin | M6 (human) | QVQLVQSGAEVKKPGASVKVSCKASGYTFTSYYMHWVRQAPGQ GLEWMGIINPSGGSTSYAQKFQGRVTMTRDTSTSTVYMELSSLRS EDTAVYYCARYRLIAVAGDYYYYGMDVWGQGTMVTVSSGGGGS GGGGSGGGGSGGGGSDIQMTQSPSSVASVGDRVTITCRASQGV GRWLAWYQQKPGTAPKLLIYAASTLQSGVPSRFSGSGSGTDFTLT INNLQPEDFATYYCQQANSFPLTFGGGTRLEIK | 208 |
| mesothelin | M7 (human) | QVQLVQSGGGVVQPGRSLRLSCAASGFTFSSYAMHWVRQAPGK GLEWVAVISYDGSNKYYADSVKGRFTISRDNSKNTLYLQMNSLRA EDTAVYYCARWKVSSSSPAFDYWGQGTLVTVSSGGGGSGGGGS GGGGSGGGGSEIVLTQSPATLSLSPGERAILSCRASQSVYTKYLG WYQQKPGQAPRLLIYDASTRATGIPDRFSGSGSGTDFTLTINRLEP EDFAVYYCQHYGGSPLITFGQGTRLEIK | 209 |
| mesothelin | M8 (human) | QVQLQQSGAEVKKPGASVKVSCKTSGYPFTGYSLHWVRQAPGQ GLEWMGWINPNSGGTNYAQKFQGRVTMTRDTSISTAYMELSRLR SDDTAVYYCARDHYGGNSLFYWGQGTLVTVSSGGGGSGGGGS GGGGSGGGGSDIQLTQSPSSISASVGDTVSITCRASQDSGTWLA WYQQKPGKAPNLLMYDASTLEDGVPSRFSGSASGTEFTLTVNRL QPEDSATYYCQQYNSYPLTFGGGTKVDIK | 210 |
| mesothelin | M9 (human) | QVQLVQSGAEVKKPGASVEVSCKASGYTFTSYYMHWVRQAPGQ GLEWMGIINPSGGSTGYAQKFQGRVTMTRDTSTSTVHMELSSLR SEDTAVYYCARGGYSSSSDAFDIWGQGTMVTVSSGGGGSGGGG SGGGGSGGGGSDIQMTQSPPSLSASVGDRVTITCRASQDISSALA WYQQKPGTPPKLLIYDASSLESGVPSRFSGSGSGTDFTLTISSLQP EDFATYYCQQFSSYPLTFGGGTRLEIK | 211 |
| mesothelin | M10 (human) | QVQLVQSGAEVKKPGASVKVSCKASGYTFTSYGISWVRQAPGQG LEWMGWISAYNGNTNYAQKLQGRVTMTTDTSTSTAYMELRSLRS DDTAVYYCARVAGGIYYYYGMDVWGQGTTITVSSGGGGSGGGG | 212 |
| | | SGGGGSGGGGSDIVMTQTPDSLAVSLGERATISCKSSHSVLYNR NNKNYLAWYQQKPGQPPKLLFYWASTRKSGVPDRFSGSGSGTD FTLTISSLQPEDFATYFCQQTQTFPLTFGQGTRLEIN | |
| mesothelin | M12 (human) | QVQLVQSGAEVKKPGASVKVSCKASGYTFTGYYMHWVRQAPGQ GLEWMGRINPNSGGTNYAQKFQGRVTMTTDTSTSTAYMELRSLR SDDTAVYYCARTTTSYAFDIWGQGTMVTVSSGGGGSGGGGSGG GGSGGGGSDIQLTQSPSTLSASVGDRVTITCRASQSISTWLAWYQ QKPGKAPNLLIYKASTLESGVPSRFSGSGSGTEFTLTISSLQPDDF ATYYCQQYNTYSPYTFGQGTKLEIK | 213 |
| mesothelin | M13 (human) | QVQLVQSGGGLVKPGGSLRLSCEASGFIFSDYYMGWIRQAPGKG LEWVSYIGRSGSSMYYADSVKGRFTFSRDNAKNSLYLQMNSLRA EDTAVYYCAASPVVAATEDFQHWGQGTLVTVSSGGGGSGGGGS GGGGSGGGGSDIVMTQTPATLSLSPGERATLSCRASQSVTSNYL AWYQQKPGQAPRLLLFGASTRATGIPDRFSGSGSGTDFTLTINRL EPEDFAMYYCQQYGSAPVTFGQGTKLEIK | 214 |
| mesothelin | M14 (human) | QVQLVQSGAEVRAPGASVKISCKASGFTFRGYYIHWVRQAPGQG LEWMGIINPSGGSRAYAQKFQGRVTMTRDTSTSTVYMELSSLRSD DTAMYYCARTASCGGDCYYLDYWGQGTLVTVSSGGGGSGGGG SGGGGSGGGGSDIQMTQSPPTLSASVGDRVTITCRASENVNIWL AWYQQKPGKAPKLLIYKSSSLASGVPSRFSGSGSGAEFTLTISSLQ PDDFATYYCQQYQSYPLTFGGGTKVDIK | 215 |
| mesothelin | M15 (human) | QVQLVQSGGGLVQPGRSLRLSCAASGFTFDDYAMHWVRQAPGK GLEWVSGISWNSGSIGYADSVKGRFTISRDNAKNSLYLQMNSLRA EDTAVYYCAKDGSSSWSWGYFDYWGQGTLVTVSSGGGGSGGG GSGGGGSSSELTQDPAVSVALGQTVRTTCQGDALRSYYASWYQ QKPGQAPMLVIYGKNNRPSGIPDRFSGSDSGDTASLTITGAQAED EADYYCNSRDSSGYPVFGTGTKVTVL | 216 |
| mesothelin | M16 (human) | EVQLVESGGGLVQPGRSLRLSCAASGFTFDDYAMHWVRQAPGK GLEWVSGISWNSGSTGYADSVKGRFTISRDNAKNSLYLQMNSLR AEDTALYYCAKDSSSWYGGGSAFDIWGQGTMVTVSSGGGGSGG GGSGGGGSSSELTQEPAVSVALGQTVRITCQGDSLRSYYASWYQ QKPGQAPVLVIFGRSRRPSGIPDRFSGSSSGNTASLIITGAQAEDE ADYYCNSRDNTANHYVFGTGTKLTVL | 217 |
| mesothelin | M17 (human) | EVQLVESGGGLVQPGRSLRLSCAASGFTFDDYAMHWVRQAPGK GLEWVSGISWNSGSTGYADSVKGRFTISRDNAKNSLYLQMNSLR AEDTALYYCAKDSSSWYGGGSAFDIWGQGTMVTVSSGGGGSGG GGSGGGGSSSELTQDPAVSVALGQTVRITCQGDSLRSYYASWYQ QKPGQAPVLVIYGKNNRPSGIPDRFSGSSSGNTASLTITGAQAED EADYYCNSRGSSGNHYVFGTGTKVTVL | 218 |
| mesothelin | M18 (human) | QVQLVQSGGGLVQPGGSLRLSCAASGFTFSSYWMHWVRQAPGK GLVWVSRINSDGSSTSYADSVKGRFTISRDNAKNTLYLQMNSLRA EDTAVYYCVRTGWVGSYYYYMDVWGKGTTVTVSSGGGGSGGG GSGGGGSGGGGSEIVLTQSPGTLSLSPGERATLSCRASQSVSSN YLAWYQQKPGQPPRLLIYDVSTRATGIPARFSGGGSGTDFTLTISS LEPEDFAVYYCQQRSNWPPWTFGQGTKVEIK | 219 |
| mesothelin | M19 (human) | QVQLVQSGGGVVQPGRSLRLSCAASGFTFSSYGMHWVRQAPGK GLEWVAVISYDGSNKYYADSVKGRFTISRDNSKNTLYLQMNSLRA EDTAVYYCAKGYSRYYYYGMDVWGQGTTVTVSSGGGGSGGGG | 220 |
| | | SGGGGSGGGGSEIVMTQSPATLSLSPGERAILSCRASQSVYTKYL GWYQQKPGQAPRLLIYDASTRATGIPDRFSGSGSGTDFTLTINRLE PEDFAVYYCQHYGGSPLITFGQGTKVDIK | |
| mesothelin | M20 (human) | QVQLVQSGGGLVQPGGSLRLSCAASGFTFSSYAMSWVRQAPGK GLEWVSAISGSGGSTYYADSVKGRFTISRDNSKNTLYLQMNSLRA EDTAVYYCAKREAAAGHDWYFDLWGRGTLVTVSSGGGGSGGGG SGGGGSGGGGSDIRVTQSPSSLSASVGDRVTITCRASQSISSYLN WYQQKPGKAPKLLIYAASSLQSGVPSRFSGSGSGTDFTLTISSLQ PEDFATYYCQQSYSIPLTFGQGTKVEIK | 221 |
| mesothelin | M21 (human) | QVQLVQSWAEVKKPGASVKVSCKASGYTFTSYYMHWVRQAPGQ GLEWMGIINPSGGSTSYAQKFQGRVTMTRDTSTSTVYMELSNLRS EDTAVYYCARSPRVTTGYFDYWGQGTLVTVSSGGGGSGGGGSG GGGSGGGGSDIQLTQSPSTLSASVGDRVTITCRASQSISSWLAWY QQKPGKAPKLLIYKASSLESGVPSRFSGSGSGTEFTLTISSLQPDD FATYYCQQYSSYPLTFGGGTRLEIK | 222 |
| mesothelin | M22 (human) | QVQLVQSGAEVRRPGASVKISCRASGDTSTRHYIHWLRQAPGQG PEWMGVINPTTGPATGSPAYAQMLQGRVTMTRDTSTRTVYMELR SLRFEDTAVYYCARSVVGRSAPYYFDYWGQGTLVTVSSGGGGS GGGGSGGGGSGGGGSDIQMTQSPSSLSASVGDRVTITCRASQGI SDYSAWYQQKPGKAPKLLIYAASTLQSGVPSRFSGSGSGTDFTLT ISYLQSEDFATYYCQQYYSYPLTFGGGTKVDIK | 223 |
| mesothelin | M23 (human) | QVQLQQSGAEVKKPGASVKVSCKASGYTFTNYYMHWVRQAPGQ GLEWMGIINPSGGYTTYAQKFQGRLTMTRDTSTSTVYMELSSLRS EDTAVYYCARIRSCGGDCYYFDNWGQGTLVTVSSGGGGSGGGG SGGGGSGGGGSDIQLTQSPSTLSASVGDRVTITCRASENVNIWLA WYQQKPGKAPKLLIYKSSSLASGVPSRFSGSGSGAEFTLTISSLQP DDFATYYCQQYQSYPLTFGGGTKVDIK | 224 |
| mesothelin | M24 (human) | QITLKESGPALVKPTQTLTLTCTFSGFSLSTAGVHVGWIRQPPGKA LEWLALISWADDKRYRPSLRSRLDITRVTSKDQVVLSMTNMQPED TATYYCALQGFDGYEANWGPGTLVTVSSGGGGSGGGGSGGGG SGGGGSDIVMTQSPSSLSASAGDRVTITCRASRGISSALAWYQQK PGKPPKLLIYDASSLESGVPSRFSGSGSGTDFTLTIDSLEPEDFAT YYCQQSYSTPWTFGQGTKVDIK | 225 |

The CDR sequences of the scFv domains of the mesothelin antigen binding domains provided in Table 3 are shown in Table 4 for the heavy chain variable domains and in Table 5 for the light chain variable domains.

**Table 4. Amino acid sequences for the heavy chain (HC) CDR1, CDR2, and CDR3 regions of human anti-mesothelin scFvs**

| **Descrip.** | **HC-CDR1** | **SEQ ID NO:** | **HC-CDR2** | **SEQ ID NO:** | **HC-CDR3** | **SEQ ID NO:** |
|---|---|---|---|---|---|---|
| M5 | GYTFTDYYMH | 226 | WINPNSGGTNYAQKFQG | 227 | GWDFDY | 228 |
| M11 | GYTFTGYYMH | 229 | WINPNSGGTNYAQNFQG | 230 | GWDFDY | 228 |
| SS1 | GYSFTGYTMN | 231 | LITPYNGASSYNQKFRG | 232 | GGYDGRGFDY | 233 |
| M1 | GYTFTGYYMH | 229 | RINPNSGGTNYAQKFQG | 234 | GRYYGMDV | 235 |
| M2 | GYTFTGYYMH | 229 | WINPNSGGTNYAQKFQG | 227 | DLRRTVVTPRAYY GMDV | 236 |
| M3 | GYTFTGYYMH | 229 | WINPNSGGTNYAQKFQG | 227 | GEWDGSYYYDY | 237 |
| M4 | GFTFSSYWMH | 238 | RINTDGSTTTYADSVEG | 239 | GHWAV | 240 |
| M6 | GYTFTSYYMH | 241 | IINPSGGSTSYAQKFQ | 242 | YRLIAVAGDYYYY GMDV | 243 |
| M7 | GFTFSSYAMH | 244 | VISYDGSNKYYADSVKG | 245 | WKVSSSSPAFDY | 246 |
| M8 | GYPFTGYSLH | 247 | WINPNSGGTNYAQKFQG | 227 | DHYGGNSLFY | 248 |
| M9 | GYTFTSYYMH | 241 | IINPSGGSTGYAQKFQG | 249 | GGYSSSSDAFDI | 250 |
| M10 | GYTFTSYGIS | 251 | WISAYNGNTNYAQKLQ | 252 | VAGGIYYYYGMD V | 253 |
| M12 | GYTFTGYYMH | 229 | RINPNSGGTNYAQKFQG | 234 | TTTSYAFDI | 254 |
| M13 | GFIFSDYYMG | 255 | YIGRSGSSMYYADSVKG | 256 | SPVVAATEDFQH | 257 |
| M14 | GFTFRGYYIH | 258 | IINPSGGSRAYAQKFQG | 259 | TASCGGDCYYLD Y | 260 |
| M15 | GFTFDDYAMH | 261 | GISWNSGSIGYADSVK | 262 | DGSSSWSWGYF DY | 263 |
| M16 | GFTFDDYAMH | 261 | GISWNSGSTGYADSVKG | 264 | DSSSWYGGGSAF DI | 265 |
| M17 | GFTFDDYAMH | 261 | GISWNSGSTGYADSVKG | 264 | DSSSWYGGGSAF DI | 265 |
| M18 | GFTFSSYWMH | 238 | RINSDGSSTSYADSVKG | 266 | TGWVGSYYYYMD V | 267 |
| M19 | GFTFSSYGMH | 268 | VISYDGSNKYYADSVKG | 245 | GYSRYYYYGMDV | 269 |
| M20 | GFTFSSYAMS | 270 | AISGSGGSTYYADSVKG | 271 | REAAAGHDWYFD L | 272 |
| M21 | GYTFTSYYMH | 241 | IINPSGGSTSYAQKFQG | 273 | SPRVTTGYFDY | 274 |
| M22 | GDTSTRHYIH | 275 | VINPTTGPATGSPAYAQ MLQG | 276 | SVVGRSAPYYFD Y | 277 |
| M23 | GYTFTNYYMH | 278 | IINPSGGYTTYAQKFQG | 279 | IRSCGGDCYYFDN | 280 |
| M24 | GFSLSTAGVH VG | 281 | LISWADDKRYRPSLRS | 282 | QGFDGYEAN | 283 |

**Table 5. Amino acid sequences for the light chain (LC) CDR1, CDR2, and CDR3 regions of human anti-mesothelin scFvs**

| **Description** | **LC-CDR1** | **SEQ ID NO:** | **LC-CDR2** | **SEQ ID NO:** | **LC-CDR3** | **SEQ ID NO:** |
|---|---|---|---|---|---|---|
| M5 | RASQSIRYYLS | 284 | TASILQN | 285 | LQTYTTPD | 286 |
| M11 | RASQSIRYYLS | 284 | TASILQN | 285 | LQTYTTPD | 286 |
| SS1 | SASSSVSYMH | 287 | DTSKLAS | 288 | QQWSGYPLT | 289 |
| M1 | RASQSVSSNFA | 290 | DASNRAT | 291 | HQRSNWLYT | 292 |
| M2 | QASQDISNSLN | 293 | DASTLET | 294 | QQHDNLPLT | 295 |
| M3 | RASQSINTYLN | 296 | AASSLQS | 297 | QQSFSPLT | 298 |
| M4 | RASQSISDRLA | 299 | KASSLES | 300 | QQYGHLPMYT | 301 |
| M6 | RASQGVGRWLA | 302 | AASTLQS | 303 | QQANSFPLT | 304 |
| M7 | RASQSVYTKYLG | 305 | DASTRAT | 306 | QHYGGSPLIT | 307 |
| M8 | RASQDSGTWLA | 308 | DASTLED | 309 | QQYNSYPLT | 310 |
| M9 | RASQDISSALA | 311 | DASSLES | 312 | QQFSSYPLT | 313 |
| M10 | KSSHSVLYNRNNKNYLA | 314 | WASTRKS | 315 | QQTQTFPLT | 316 |
| M12 | RASQSISTWLA | 317 | KASTLES | 318 | QQYNTYSPYT | 319 |
| M13 | RASQSVTSNYLA | 320 | GASTRAT | 321 | QQYGSAPVT | 322 |
| M14 | RASENVNIWLA | 323 | KSSSLAS | 324 | QQYQSYPLT | 325 |
| M15 | QGDALRSYYAS | 326 | GKNNRPS | 327 | NSRDSSGYPV | 328 |
| M16 | QGDSLRSYYAS | 329 | GRSRRPS | 330 | NSRDNTANHYV | 331 |
| M17 | QGDSLRSYYAS | 329 | GKNNRPS | 327 | NSRGSSGNHYV | 332 |
| M18 | RASQSVSSNYLA | 333 | DVSTRAT | 334 | QQRSNWPPWT | 335 |
| M19 | RASQSVYTKYLG | 305 | DASTRAT | 306 | QHYGGSPLIT | 307 |
| M20 | RASQSISSYLN | 336 | AASSLQS | 297 | QQSYSIPLT | 337 |
| M21 | RASQSISSWLA | 338 | KASSLES | 300 | QQYSSYPLT | 339 |
| M22 | RASQGISDYS | 340 | AASTLQS | 303 | QQYYSYPLT | 341 |
| M23 | RASENVNIWLA | 323 | KSSSLAS | 324 | QQYQSYPLT | 325 |
| M24 | RASRGISSALA | 342 | DASSLES | 312 | QQSYSTPWT | 343 |

Any known anti-mesothelin binding domain, from, for example, a known antibody, bispecific molecule or CAR, may be suitable for use in the CAR of the present invention. For example, the antigen binding domain against mesothelin is or may be derived from an antigen binding, e.g., CDRs or VH and VL, of an antibody, antigen-binding fragment or CAR described in, e.g., PCT publication WO2015/090230. In embodiments, the antigen binding domain against mesothelin is or is derived from an antigen binding portion, e.g., CDRs or VH and VL, of an antibody, antigen-binding fragment, or CAR described in, e.g., PCT publication WO1997/025068, WO1999/028471, WO2005/014652, WO2006/099141, WO2009/045957, WO2009/068204, WO2013/142034, WO2013/040557, or WO2013/063419.

In one embodiment, the mesothelin binding domain comprises one or more (e.g., all three) light chain complementary determining region 1 (LC CDR1), light chain complementary determining region 2 (LC CDR2), and light chain complementary determining region 3 (LC CDR3) of a mesothelin binding domain described herein, e.g., provided in Table 3 or 5, and/or one or more (e.g., all three) heavy chain complementary determining region 1 (HC CDR1), heavy chain complementary determining region 2 (HC CDR2), and heavy chain complementary determining region 3 (HC CDR3) of a mesothelin binding domain described herein, e.g., provided in Table 3 or 4. In one embodiment, the mesothelin binding domain comprises one, two, or all of LC CDR1, LC CDR2, and LC CDR3 of any amino acid sequences as provided in Table 5; and one, two or three of all of HC CDR1, HC CDR2 and HC CDR3, of any amino acid acid sequences as provided in Table 4.

In one embodiment, the mesothelin binding domain comprises a light chain variable region described herein (e.g., in Table 3) and/or a heavy chain variable region described herein (e.g., in Table 3). In one embodiment, the mesothelin binding domain is a scFv comprising a light chain and a heavy chain of an amino acid sequence listed in Table 3. In an embodiment, the mesothelin binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 3, or a sequence with 95-99% identity with an amino acid sequence provided in Table 3; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 3, or a sequence with 95-99% identity to an amino acid sequence provided in Table 3.

In one embodiment, the mesothelin binding domain comprises an amino acid sequence selected from any one of SEQ ID NOs: 201-225; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the mesothelin binding domain is a scFv, and a light chain variable region comprising an amino acid sequence described herein, e.g., in Table 3, is attached to a heavy chain variable region comprising an amino acid sequence described herein, e.g., in Table 3, via a linker, e.g., a linker described herein. In one embodiment, the mesothelin binding domain includes a (Gly4-Ser)n linker, wherein n is 1, 2, 3, 4, 5, or 6, preferably 4 (SEQ ID NO: 967). The light chain variable region and heavy chain variable region of a scFv can be, e.g., in any of the following orientations: light chain variable region-linker-heavy chain variable region or heavy chain variable region-linker-light chain variable region.

In an embodiment, the antigen binding domain of a CAR, e.g., a CAR expressed by a cell of the invention, binds to human EGFRvIII. In an embodiment, the antigen binding domain is a murine scFv domain that binds to human EGFRvIII such as, e.g., mu310C. In an embodiment, the antigen binding domain is a humanized antibody or antibody fragment, e.g., scFv domain, derived from the murine mu310C scFv. Exemplary humanized scFv domains (and their sequences) and murine SS1 scFv that bind to EGFRvIII are provided in Table 6.

In an embodiment, the antigen binding domain of a CAR binds to human claudin 6 (CLDN6). In an embodiment, the antigen binding domain is a murine scFv domain that binds to human CLDN6. In an embodiment, the antigen binding domain is a humanized antibody or antibody fragment. Exemplary scFv domains (and their sequences) that bind to CLDN6 are provided in Table 6. The scFv domain sequences provided in Table 6 include a light chain variable region (VL) and a heavy chain variable region (VH). The VL and VH are attached by a linker comprising the sequence GGGGSGGGGSGGGGSGGGGS (SEQ ID NO: 29), e.g., in the following orientation: VL-linker-VH.

**Table 6. Antigen binding domains that bind to the tumor antigen EGFRvIII or CLDN6**

| **Tumor antigen** | **Name** | **Amino acid sequence** | **SEQ ID NO:** |
|---|---|---|---|
| EGFR vIII | huscFv1 | Eiqlvqsgaevkkpgatvkisckgsgfniedyyihwvqqapgkglewmgridpendetk ygpifqgrvtitadtstntvymelsslrsedtavyycafrggvywgqgttvtvssggggsggg gsggggsggggsdvvmtqspdslavslgeratinckssqslldsdgktylnwlqqkpgqp pkrlislvskldsgvpdrfsgsgsgtdftltisslqaedvavyycwqgthfpgtfgggtkveik | 344 |
| EGFR vIII | huscFv2 | Dvvmtqspdslavslgeratinckssqslldsdgktylnwlqqkpgqppkrlislvskldsgv pdrfsgsgsgtdftltisslqaedvavyycwqgthfpgtfgggtkveikggggsggggsggg gsggggseiqlvqsgaevkkpgatvkisckgsgfniedyyihwvqqapgkglewmgrid pendetkygpifqgrvtitadtstntvymelsslrsedtavyycafrggvywgqgttvtvss | 345 |
| EGFR vIII | huscFv3 | Eiqlvqsgaevkkpgeslrisckgsgfniedyyihwvrqmpgkglewmgridpendetk ygpifqghvtisadtsintvylqwsslkasdtamyycafrggvywgqgttvtvssggggsg gggsggggsggggsdvvmtqsplslpvtlgqpasisckssqslldsdgktylnwlqqrpg qsprrlislvskldsgvpdrfsgsgsgtdftlkisrveaedvgvyycwqgthfpgtfgggtkvei k | 346 |
| EGFR vIII | huscFv4 | Dvvmtqsplslpvtlgqpasisckssqslldsdgktylnwlqqrpgqsprrlislvskldsgvp drfsgsgsgtdftlkisrveaedvgvyycwqgthfpgtfgggtkveikggggsggggsggg | 347 |
| | | gsggggseiqlvqsgaevkkpgeslrisckgsgfniedyyihwvrqmpgkglewmgrid pendetkygpifqghvtisadtsintvylqwsslkasdtamyycafrggvywgqgttvtvss | |
| EGFR vIII | huscFv5 | Eiqlvqsgaevkkpgatvkisckgsgfniedyyihwvqqapgkglewmgridpendetk ygpifqgrvtitadtstntvymelsslrsedtavyycafrggvywgqgttvtvssggggsggg gsggggsggggsdvvmtqsplslpvtlgqpasisckssqslldsdgktylnwlqqrpgqs prrlislvskldsgvpdrfsgsgsgtdftlkisrveaedvgvyycwqgthfpgtfgggtkveik | 348 |
| EGFR vIII | huscFv6 | Eiqlvqsgaevkkpgeslrisckgsgfniedyyihwvrqmpgkglewmgridpendetk ygpifqghvtisadtsintvylqwsslkasdtamyycafrggvywgqgttvtvssggggsg gggsggggsggggsdvvmtqspdslavslgeratinckssqslldsdgktylnwlqqkpg qppkrlislvskldsgvpdrfsgsgsgtdftltisslqaedvavyycwqgthfpgtfgggtkvei k | 349 |
| EGFR vIII | huscFv7 | Dvvmtqspdslavslgeratinckssqslldsdgktylnwlqqkpgqppkrlislvskldsgv pdrfsgsgsgtdftltisslqaedvavyycwqgthfpgtfgggtkveikggggsggggsggg gsggggseiqlvqsgaevkkpgeslrisckgsgfniedyyihwvrqmpgkglewmgrid pendetkygpifqghvtisadtsintvylqwsslkasdtamyycafrggvywgqgttvtvss | 350 |
| EGFR vIII | huscFv8 | Dvvmtqsplslpvtlgqpasisckssqslldsdgktylnwlqqrpgqsprrlislvskldsgvp drfsgsgsgtdftlkisrveaedvgvyycwqgthfpgtfgggtkveikggggsggggsggg gsggggseiqlvqsgaevkkpgatvkisckgsgfniedyyihwvqqapgkglewmgrid pendetkygpifqgrvtitadtstntvymelsslrsedtavyycafrggvywgqgttvtvss | 351 |
| EGFR vIII | Mu310C | eiqlqqsgaelvkpgasvklsctgsfniedyyihwvkqrteqglewigridpendetkygp ifqgratitadtssntvylqlssltsedtavyycafrggvywgpgttltvssggggsggggsgg ggshmdvvmtqspltlsvaigqsasisckssqslldsdgktylnwllqrpgqspkrlislvskl dsgvpdrftgsgsgtdftlrisrveaedlgiyycwqgthfpgtfgggtkleik | 352 |
| Claudin6 | muMAB 64A | EVQLQQSGPELVKPGASMKISCKASGYSFTGYTMNWVKQSHG KNLEWIGLINPYNGGTIYNQKFKGKATLTVDKSSSTAYMELLSLT SEDSAVYYCARDYGFVLDYWGQGTTLTVSSGGGGSGGGGSG GGGSGGGGSQIVLTQSPSIMSVSPGEKVTITCSASSSVSYMHW FQQKPGTSPKLCIYSTSNLASGVPARFSGRGSGTSYSLTISRVA AEDAATYYCQQRSNYPPWTFGGGTKLEIK | 353 |
| Claudin6 | mAb206-LCC | EVQLQQSGPELVKPGASMKISCKASGYSFTGYTMNWVKQSHG KNLEWIGLINPYNGGTIYNQKFKGKATLTVDKSSSTAYMELLSLT SEDSAVYYCARDYGFVLDYWGQGTTLTVSSGGGGSGGGGSG GGGSGGGGSQIVLTQSPAIMSASPGEKVTITCSASSSVSYLHWF QQKPGTSPKLWVYSTSNLPSGVPARFGGSGSGTSYSLTISRME AEDAATYYCQQRSIYPPWTFGGGTKLEIK | 354 |
| Claudin6 | mAb206-SUBG | EVQLQQSGPELVKPGASMKISCKASGYSFTGYTMNWVKQSHG KNLEWIGLINPYNGGTIYNQKFKGKATLTVDKSSSTAYMELLSLT SEDSAVYYCARDYGFVLDYWGQGTTLTVSSGGGGSGGGGSG GGGSGGGGSQIVLTQSPSIMSVSPGEKVTITCSASSSVSYMHW FQQKPGTSPKLGIYSTSNLASGVPARFSGRGSGTSYSLTISRVA AEDAATYYCQQRSNYPPWTFGGGTKLEIK | 355 |

In one embodiment, the EGFRvIII binding domain comprises one or more (e.g., all three) light chain complementary determining region 1 (LC CDR1), light chain complementary determining region 2 (LC CDR2), and light chain complementary determining region 3 (LC CDR3) of an EGFRvIII binding domain described herein, e.g., provided in Table 6, and/or one or more (e.g., all three) heavy chain complementary determining region 1 (HC CDR1), heavy chain complementary determining region 2 (HC CDR2), and heavy chain complementary determining region 3 (HC CDR3) of an EGFRvIII binding domain described herein, e.g., provided in Table 6.

In one embodiment, the EGFRvIII binding domain comprises a light chain variable region described herein (e.g., in Table 6) and/or a heavy chain variable region described herein (e.g., in Table 6). In one embodiment, the EGFRvIII binding domain is a scFv comprising a light chain and a heavy chain of an amino acid sequence listed in Table 6. In an embodiment, the EGFRvIII binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 6, or a sequence with 95-99% identity with an amino acid sequence provided in Table 6; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 6, or a sequence with 95-99% identity to an amino acid sequence provided in Table 6.

In one embodiment, the EGFRvIII binding domain comprises an amino acid sequence selected from any one of SEQ ID NOs: 344-352; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the EGFRvIII binding domain is a scFv, and a light chain variable region comprising an amino acid sequence described herein, e.g., in Table 6, is attached to a heavy chain variable region comprising an amino acid sequence described herein, e.g., in Table 6, via a linker, e.g., a linker described herein. In one embodiment, the EGFRvIII binding domain includes a (Gly4-Ser)n linker, wherein n is 1, 2, 3, 4, 5, or 6, preferably 4 (SEQ ID NO: 967). The light chain variable region and heavy chain variable region of a scFv can be, e.g., in any of the following orientations: light chain variable region-linker-heavy chain variable region or heavy chain variable region-linker-light chain variable region.

In one embodiment, the Claudin6 binding domain comprises one or more (e.g., all three) light chain complementary determining region 1 (LC CDR1), light chain complementary determining region 2 (LC CDR2), and light chain complementary determining region 3 (LC CDR3) of a Claudin6 binding domain described herein, e.g., provided in Table 6, and/or one or more (e.g., all three) heavy chain complementary determining region 1 (HC CDR1), heavy chain complementary determining region 2 (HC CDR2), and heavy chain complementary determining region 3 (HC CDR3) of a Claudin6 binding domain described herein, e.g., provided in Table 6.

In one embodiment, the Claudin-6 binding domain comprises a light chain variable region described herein (e.g., in Table 6) and/or a heavy chain variable region described herein (e.g., in Table 6). In one embodiment, the Claudin-6 binding domain is a scFv comprising a light chain and a heavy chain of an amino acid sequence listed in Table 6. In an embodiment, the Claudin-6 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 6, or a sequence with 95-99% identity with an amino acid sequence provided in Table 6; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 6, or a sequence with 95-99% identity to an amino acid sequence provided in Table 6.

In one embodiment, the Claudin6 binding domain comprises an amino acid sequence selected from any one of SEQ ID NOs: 353-355; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the Claudin6 binding domain is a scFv, and a light chain variable region comprising an amino acid sequence described herein, e.g., in Table 6, is attached to a heavy chain variable region comprising an amino acid sequence described herein, e.g., in Table 6, via a linker, e.g., a linker described herein. In one embodiment, the Claudin6 binding domain includes a (Gly4-Ser)n linker, wherein n is 1, 2, 3, 4, 5, or 6, preferably 4 (SEQ ID NO: 967). The light chain variable region and heavy chain variable region of a scFv can be, e.g., in any of the following orientations: light chain variable region-linker-heavy chain variable region or heavy chain variable region-linker-light chain variable region.

In one embodiment, an antigen binding domain against GD2 is an antigen binding portion, e.g., CDRs, of an antibody described in, e.g., Mujoo et al., Cancer Res. 47(4):1098-1104 (1987); Cheung et al., Cancer Res 45(6):2642-2649 (1985), Cheung et al., J Clin Oncol 5(9):1430-1440 (1987), Cheung et al., J Clin Oncol 16(9):3053-3060 (1998), Handgretinger et al., Cancer Immunol Immunother 35(3):199-204 (1992). In some embodiments, an antigen binding domain against GD2 is an antigen binding portion of an antibody selected from mAb 14.18, 14G2a, ch14.18, hu14.18, 3F8, hu3F8, 3G6, 8B6, 60C3, 10B8, ME36.1, and 8H9, see e.g., WO2012033885, WO2013040371, WO2013192294, WO2013061273, WO2013123061, WO2013074916, and WO201385552. In some embodiments, an antigen binding domain against GD2 is an antigen binding portion of an antibody described in US Publication No.: 20100150910 or PCT Publication No.: WO 2011160119.

In one embodiment, an antigen binding domain against the Tn antigen, the sTn antigen, a Tn-O-glycopeptide antigen, or a sTn-O-glycopeptide antigen is an antigen binding portion, e.g., CDRs, of an antibody described in, e.g., US 2014/0178365, US8,440,798, EP 2083868 A2, Brooks et al., PNAS 107(22):10056-10061 (2010), and Stone et al., Oncolmmunology 1(6):863-873(2012).

In one embodiment, an antigen binding domain against PSMA is an antigen binding portion, e.g., CDRs, of an antibody described in, e.g., Parker et al., Protein Expr Purif 89(2):136-145 (2013), US 20110268656 (J591 ScFv); Frigerio et al, European J Cancer 49(9):2223-2232 (2013) (scFvD2B); WO 2006125481 (mAbs 3/A12, 3/E7 and 3/F11) and single chain antibody fragments (scFv A5 and D7).

In one embodiment, an antigen binding domain against CD97 is an antigen binding portion, e.g., CDRs, of an antibody described in, e.g., US6,846,911 ;de Groot et al., J Immunol 183(6):4127-4134 (2009); or an antibody from R&D:MAB3734.

In one embodiment, an antigen binding domain against TAG72 is an antigen binding portion, e.g., CDRs, of an antibody described in, e.g., Hombach et al., Gastroenterology 113(4):1163-1170 (1997); and Abcam ab691.

In one embodiment, an antigen binding domain against CD44v6 is an antigen binding portion, e.g., CDRs, of an antibody described in, e.g., Casucci et al., Blood 122(20):3461-3472 (2013).

In one embodiment, an antigen binding domain against CEA is an antigen binding portion, e.g., CDRs, of an antibody described in, e.g., Chmielewski et al., Gastoenterology 143(4):1095-1107 (2012).

In one embodiment, an antigen binding domain against EPCAM is an antigen binding portion, e.g., CDRS, of an antibody selected from MT110, EpCAM-CD3 bispecific Ab (see, e.g., clinicaltrials.gov/ct2/show/NCT00635596); Edrecolomab; 3622W94; ING-1; and adecatumumab (MT201).

In one embodiment, an antigen binding domain against KIT is an antigen binding portion, e.g., CDRs, of an antibody described in, e.g., US7915391, US20120288506 , and several commercial catalog antibodies.

In one embodiment, an antigen binding domain against IL-13Ra2 is an antigen binding portion, e.g., CDRs, of an antibody described in, e.g., WO2008/146911, WO2004087758, several commercial catalog antibodies, and WO2004087758.

In one embodiment, an antigen binding domain against CD171 is an antigen binding portion, e.g., CDRs, of an antibody described in, e.g., Hong et al., J Immunother 37(2):93-104 (2014).

In one embodiment, an antigen binding domain against PSCA is an antigen binding portion, e.g., CDRs, of an antibody described in, e.g., Morgenroth et al., Prostate 67(10):1121-1131 (2007) (scFv 7F5); Nejatollahi et al., J of Oncology 2013(2013), article ID 839831 (scFv C5-II); and US Pat Publication No. 20090311181.

In one embodiment, an antigen binding domain against MAD-CT-2 is an antigen binding portion, e.g., CDRs, of an antibody described in, e.g., PMID: 2450952; US7635753.

In one embodiment, an antigen binding domain against Folate receptor alpha is an antigen binding portion, e.g., CDRs, of the antibody IMGN853, or an antibody described in US20120009181; US4851332, LK26: US5952484.

In one embodiment, an antigen binding domain against ERBB2 (Her2/neu) is an antigen binding portion, e.g., CDRs, of the antibody trastuzumab, or pertuzumab.

In one embodiment, an antigen binding domain against MUC1 is an antigen binding portion, e.g., CDRs, of the antibody SAR566658.

In one embodiment, the antigen binding domain against EGFR is antigen binding portion, e.g., CDRs, of the antibody cetuximab, panitumumab, zalutumumab, nimotuzumab, or matuzumab.

In one embodiment, an antigen binding domain against NCAM is an antigen binding portion, e.g., CDRs, of the antibody clone 2-2B: MAB5324 (EMD Millipore)

In one embodiment, an antigen binding domain against CAIX is an antigen binding portion, e.g., CDRs, of the antibody clone 303123 (R&D Systems).

In one embodiment, an antigen binding domain against Fos-related antigen 1 is an antigen binding portion, e.g., CDRs, of the antibody 12F9 (Novus Biologicals).

In one embodiment, an antigen binding domain against SSEA-4 is an antigen binding portion, e.g., CDRs, of antibody MC813 (Cell Signaling), or other commercially available antibodies.

In one embodiment, an antigen binding domain against PDGFR-beta is an antigen binding portion, e.g., CDRs, of an antibody Abcam ab32570.

In one embodiment, an antigen binding domain against ALK is an antigen binding portion, e.g., CDRs, of an antibody described in, e.g., Mino-Kenudson et al., Clin Cancer Res 16(5):1561-1571 (2010).

In one embodiment, an antigen binding domain against plysialic acid is an antigen binding portion, e.g., CDRs, of an antibody described in, e.g., Nagae et al., J Biol Chem 288(47):33784-33796 (2013).

In one embodiment, an antigen binding domain against PLAC1 is an antigen binding portion, e.g., CDRs, of an antibody described in, e.g., Ghods et al., Biotechnol Appl Biochem 2013 doi:10.1002/bab.1177.

In one embodiment, an antigen binding domain against GloboH is an antigen binding portion of the antibody VK9; or an antibody described in, e.g., Kudryashov V et al, Glycoconj J.15(3):243-9 (1998), Lou et al., Proc Natl Acad Sci USA 111(7):2482-2487 (2014) ; MBr1: Bremer E-G et al. J Biol Chem 259:14773-14777 (1984).

In one embodiment, an antigen binding domain against NY-BR-1 is an antigen binding portion, e.g., CDRs of an antibody described in, e.g., Jager et al., Appl Immunohistochem Mol Morphol 15(1):77-83 (2007).

In one embodiment, an antigen binding domain against sperm protein 17 is an antigen binding portion, e.g., CDRs, of an antibody described in, e.g., Song et al., Target Oncol 2013 Aug 14 (PMID: 23943313); Song et al., Med Oncol 29(4):2923-2931 (2012).

In one embodiment, an antigen binding domain against TRP-2 is an antigen binding portion, e.g., CDRs, of an antibody described in, e.g., Wang et al, J Exp Med. 184(6):2207-16 (1996).

In one embodiment, an antigen binding domain against CYP1B1 is an antigen binding portion, e.g., CDRs, of an antibody described in, e.g., Maecker et al, Blood 102 (9): 3287-3294 (2003).

In one embodiment, an antigen binding domain against RAGE-1 is an antigen binding portion, e.g., CDRs, of the antibody MAB5328 (EMD Millipore).

In one embodiment, an antigen binding domain against human telomerase reverse transcriptase is an antigen binding portion, e.g., CDRs, of the antibody cat no: LS-B95-100 (Lifespan Biosciences)

In one embodiment, an antigen binding domain against intestinal carboxyl esterase is an antigen binding portion, e.g., CDRs, of the antibody 4F12: cat no: LS-B6190-50 (Lifespan Biosciences).

In one embodiment, an antigen binding domain against mut hsp70-2 is an antigen binding portion, e.g., CDRs, of the antibody Lifespan Biosciences: monoclonal: cat no: LS-C133261-100 (Lifespan Biosciences).

In one embodiment, an antigen binding domain against MAD-CT-2 is an antigen binding portion, e.g., CDRs, of an antibody described in, e.g., PMID: 2450952; US7635753.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed above.

In some embodiments, the antigen binding domain of a CAR targets a tumor antigen that is an antigen expressed on a myeloid tumor (either a surface antigen or presented by MHC), and a cell comprising such a CAR recognizes a myeloid tumor antigen.

In an embodiment, the myeloid tumor antigen is an antigen that is preferentially or specifically expressed on the surface of a myeloid tumor cell.

In one embodiment, the antigen-binding domain of a CAR can be chosen such that a myeloid tumor population is targeted. Alternatively, when targeting of more than one type of myeloid tumor is desired, an antigen binding domain that targets a myeloid tumor antigen that is expressed by more than one, e.g., all, of the myeloid tumors to be targeted can be selected.

A CAR can target the following additional tumor antigens: CD123, CD34, Flt3, CD33 and CLL-1. In embodiments, the tumor antigen is selected from CD123, CD33 and CLL-1. In some embodiments, the tumor antigen is CD123. In some embodiments, the tumor antigen is CD33. In some embodiments, the tumor antigen is CD34. In some embodiments, the tumor antigen is Flt3. In embodiments, the tumor antigen is CLL-1. In embodiments, the antigen binding domain targets the human antigen.

In one aspect, the antigen-binding domain of a CAR binds to CD123, e.g., human CD123. Any known CD123 binding domain may be used in the invention. In one embodiment, an antigen binding domain against CD123 is an antigen binding portion, e.g., CDRs or VH and VL, of an antibody, antigen-binding fragment or CAR described in, e.g., PCT publication WO2014/130635. In one embodiment, an antigen binding domain against CD123 is an antigen binding portion, e.g., CDRs or VH and VL, of an antibody, antigen-binding fragment or CAR described in, e.g., PCT publication WO/2016/028896. In one embodiment, an antigen binding domain against CD123 is an antigen binding portion, e.g., CDRs, of an antibody, antigen-binding fragment, or CAR described in, e.g., PCT publication WO1997/024373, WO2008/127735 (e.g., a CD123 binding domain of 26292, 32701, 37716 or 32703), WO2014/138805 (e.g., a CD123 binding domain of CSL362), WO2014/138819, WO2013/173820, WO2014/144622, WO2001/66139, WO2010/126066 (e.g., the CD123 binding domain of any of Old4, Old5, Old17, Old19, New102, or Old6), WO2014/144622, WO2016/028896, or US2009/0252742. In embodiments, the antigen binding domain is or is derived from a murine anti-human CD123 binding domain. In embodiments, the antigen binding domain is a humanized antibody or antibody fragment, e.g., scFv domain. In an embodiment, the antigen binding domain is a human antibody or antibody fragment that binds to human CD123. In embodiments, the antigen binding domain is an scFv domain which includes a light chain variable region (VL) and a heavy chain variable region (VH). The VL and VH may attached by a linker described herein, e.g., comprising the sequence GGGGSGGGGSGGGGS (SEQ ID NO: 30), and may be in any orientation, e.g., VL-linker-VH, or VH-linker-VL.

In some embodiments, the antigen binding domain that binds to CD123 is a scFv domain. In some embodiments, the antigen binding domain that binds to CD123 is a murine scFv domain. In an embodiment, the antigen binding domain that binds to CD123 is a human or humanized scFv domain. Exemplary scFv domains (and their sequences) that bind to CLDN6 are provided in Table 19. The scFv domain sequences provided in Table 19 include a light chain variable region (VL) and a heavy chain variable region (VH). The VL and VH are attached by a linker comprising the sequence GGGGSGGGGSGGGGSGGGGS (SEQ ID NO: 29), e.g., in the following orientation: VL-linker-VH. In one embodiment, the CD123 binding domain comprises an amino acid sequence selected from any one of SEQ ID NOs: 751, 756, 761, or 766; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to an amino acid sequence selected from any one of SEQ ID NOs: 751, 756, 761, or 766.

In one aspect, the antigen-binding domain of a CAR binds to CD33, e.g., human CD33. Any known CD33 binding domain may be used in the invention. In one embodiment, an antigen binding domain against CD33 is an antigen binding portion, e.g., CDRs or VH and VL, of an antibody, antigen-binding fragment or CAR described in, e.g., PCT publication WO2016/014576 or US Patent Publication 2016-0096892-A1. In one embodiment, an antigen binding domain against CD33 is an antigen binding portion of or derived from Gemtuzumab ozogamicin (e.g., comprising an antigen binding domain comprising one or more, e.g., one, two, or three, CDRs of the heavy chain variable domain and/or one or more, e.g., one, two, or three, CDRs of the light chain variable domain, or the VH or VL, or the scFv sequence, of the scFv sequence of Gemtuzumab ozogamicin) (previously marketed as Mylotarg), e.g., Bross et al., Clin Cancer Res 7(6):1490-1496 (2001) (Gemtuzumab Ozogamicin, hP67.6). In one embodiment, an antigen binding domain against CD33 is an antigen binding portion of or derived from (e.g., comprising an antigen binding domain comprising one or more, e.g., one, two, or three, CDRs of the heavy chain variable domain and/or one or more, e.g., one, two, or three, CDRs of the light chain variable domain, or the VH or VL, or the scFv sequence) of the scFv sequence encoded by GenBank reference no. AM402974.1 (See, Wang et al., Mol. Ther., vol. 23:1, pp. 184-191 (2015). In one embodiment, an antigen binding domain against CD33 is an antigen binding portion, e.g., CDRs, of an antibody described in,, e.g., Caron et al., Cancer Res 52(24):6761-6767 (1992) (Lintuzumab, HuM195), Lapusan et al., Invest New Drugs 30(3):1121-1131 (2012) (AVE9633), Aigner et al., Leukemia 27(5): 1107-1115 (2013) (AMG330, CD33 BiTE), Dutour et al., Adv hematol 2012:683065 (2012), and Pizzitola et al., Leukemia doi:10.1038/Lue.2014.62 (2014). In embodiments, the antigen binding domain is or is derived from a murine anti-human CD33 binding domain. In embodiments, the antigen binding domain is a humanized antibody or antibody fragment, e.g., scFv domain. In an embodiment, the antigen binding domain is a human antibody or antibody fragment that binds to human CD33. In embodiments, the antigen binding domain is an scFv domain which includes a light chain variable region (VL) and a heavy chain variable region (VH). The VL and VH may attached by a linker described herein, e.g., comprising the sequence GGGGSGGGGSGGGGS (SEQ ID NO: 30), and may be in any orientation, e.g., VL-linker-VH, or VH-linker-VL.

In one aspect, the antigen-binding domain of a CAR binds to CLL-1, e.g., human CLL-1. Any known CLL-1 binding domain may be used in the invention. In one embodiment, an antigen binding domain against CLL-1 is an antigen binding portion, e.g., CDRs or VH and VL, of an antibody, antigen-binding fragment or CAR described in, e.g., PCT publication WO2016/014535 or US Patent Publication 2016-0051651-A1. In one embodiment, an antigen binding domain against CLL-1 is an antigen binding portion, e.g., CDRs, of an antibody available from R&D, ebiosciences, Abcam, for example, PE-CLL1-hu Cat# 353604 (BioLegend); and PE-CLL1 (CLEC12A) Cat# 562566 (BD). In embodiments, the antigen binding domain is or is derived from a murine anti-human CLL-1 binding domain. In embodiments, the antigen binding domain is a humanized antibody or antibody fragment, e.g., scFv domain. In an embodiment, the antigen binding domain is a human antibody or antibody fragment that binds to human CLL-1. In embodiments, the antigen binding domain is an scFv domain which includes a light chain variable region (VL) and a heavy chain variable region (VH). The VL and VH may attached by a linker described herein, e.g., comprising the sequence GGGGSGGGGSGGGGS (SEQ ID NO: 30), and may be in any orientation, e.g., VL-linker-VH, or VH-linker-VL.

In some embodiments, the antigen binding domain of a CAR targets a B-Cell antigen. In an embodiment, the B cell antigen is an antigen that is preferentially or specifically expressed on the surface of the B cell. The antigen can be expressed on the surface of any one of the following types of B cells: progenitor B cells (e.g., pre-B cells or pro-B cells), early pro-B cells, late pro-B cells, large pre-B cells, small pre-B cells, immature B cells, e.g., naïve B cells, mature B cells, plama B cells, plasmablasts, memory B cells, B-1 cells, B-2 cells, marginal-zone B cells, follicular B cells, germinal center B cells, or regulatory B cells (Bregs).

The present disclosure provides CARs that can target the following antigens: CD19; CD123; CD22; CD30; CD171; CS-1; C-type lectin-like molecule-1, CD33; epidermal growth factor receptor variant III (EGFRvlll); ganglioside G2 (GD2); ganglioside GD3; TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumorassociated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2; Mesothelin; Interleukin 11 receptor alpha (IL-11Ra); prostate stem cell antigen (PSCA); Protease Serine 21; vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3; transglutaminase 5 (TGS5); high molecular weightmelanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51 E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1, melanoma antigen recognized by T cells 1; Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like, Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); immunoglobulin lambda-like polypeptide 1 (IGLL1); TNF receptor family member; Fms-Like Tyrosine Kinase 3 (FL T3); CD10, CD19, CD20, CD21, CD22, CD23, CD24, CD25, CD37, CD38, CD53, CD72, CD73, CD74, CD75, CD77, CD79a, CD79b, CD80, CD81, CD82, CD83, CD84, CD85, ROR1, BCMA, CD86, and CD179b. Other B cell antigens that can be targeted by a CAR described herein include: CD1a, CD1b, CD1c, CD1d, CD2, CD5, CD6, CD9, CD11a, CD11b, CD11c, CD17, CD18, CD26, CD27, CD29, CD30, CD31, CD32a, CD32b, CD35, CD38, CD39, CD40, CD44, CD45, CD45RA, CD45RB, CD45RC, CD45RO, CD46, CD47, CD48, CD49b, CD49c, CD49d, CD50, CD52, CD54, CD55, CD58, CD60a, CD62L, CD63, CD63, CD68 CD69, CD70, CD85E, CD85I, CD85J, CD92, CD95, CD97, CD98, CD99, CD100, CD102, CD108, CD119, CD120a, CD120b, CD121b, CD122, CD124, CD125, CD126, CD130, CD132, CD137, CD138, CD139, CD147, CD148, CD150, CD152, CD162, CD164, CD166, CD167a, CD170, CD175, CD175s, CD180, CD184, CD185, CD192, CD196, CD197, CD200, CD205, CD210a, CDw210b, CD212, CD213a1, CD213a2, CD215, CD217, CD218a, CD218b, CD220, CD221, CD224, CD225, CD226, CD227, CD229, CD230, CD232, CD252, CD253, CD257, CD258, CD261, CD262, CD263, CD264, CD267, CD268, CD269, CD270, CD272, CD274, CD275, CD277, CD279, CD283, CD289, CD290, CD295, CD298, CD300a, CD300c, CD305, CD306, CD307a, CD307b, CD307c, CD307d, CD307e, CD314, CD315, CD316, CD317, CD319, CD321, CD327, CD328, CD329, CD338, CD351, CD352, CD353, CD354, CD355, CD357, CD358, CD360, CD361, CD362, and CD363.

In another embodiment, the antigen targeted by the CAR is chosen from CD19, BCMA, CD20, CD22, FcRn5, FcRn2, CS-1 and CD138. In an embodiment, the antigen targeted by the CAR is CD19. In an embodiment, the antigen targeted by the CAR is CD20. In an embodiment, the antigen targeted by the CAR is CD22. In an embodiment, the antigen targeted by the CAR is BCMA. In an embodiment, the antigen targeted by the CAR is FcRn5. In an embodiment, the antigen targeted by the CAR is FcRn2. In an embodiment, the antigen targeted by the CAR is CS-1. In an embodiment, the antigen targeted by the CAR is CD138.

In one embodiment, the antigen-binding domain of a CAR, e.g., the CAR expressed by a cell of the invention (e.g., a cell that also expresses a CAR), can be chosen such that a preferred B cell population is targeted. For example, in an embodiment where targeting of B regulatory cells is desired, an antigen binding domain is selected that targets an antigen that is expressed on regulatory B cells and not on other B cell populations, e.g., plasma B cells and memory B cells. Cell surface markers expressed on regulatory B cells include: CD19, CD24, CD25, CD38, or CD86, or markers described in He et al., 2014, J Immunology Research, Article ID 215471. When targeting of more than one type of B cells is desired, an antigen binding domain that targets an antigen that is expressed by all of the B cells to be targeted can be selected.

In an embodiment, the antigen-binding domain of a CAR, e.g., the CAR expressed by a cell of the invention, binds to CD19. CD19 is found on B cells throughout differentiation of the lineage from the pro/pre-B cell stage through the terminally differentiated plasma cell stage. In an embodiment, the antigen binding domain is a murine scFv domain that binds to human CD19, e.g., CTL019 (e.g., SEQ ID NO: 356). In an embodiment, the antigen binding domain is a humanized antibody or antibody fragment, e.g., scFv domain, derived from the murine CTL019 scFv. In an embodiment, the antigen binding domain is a human antibody or antibody fragment that binds to human CD19. Exemplary scFv domains (and their sequences, e.g., CDRs, VL and VH sequences) that bind to CD19 are provided in Table 7 and Table 15A. The scFv domain sequences provided in Table 7 include a light chain variable region (VL) and a heavy chain variable region (VH). The VL and VH are attached by a linker comprising the sequence GGGGSGGGGSGGGGS (SEQ ID NO: 30), e.g., in the following orientation: VL-linker-VH.

**Table 7. Antigen Binding domains that bind CD19**

| **Antigen** | **Name** | **Amino Acid Sequence** | **SEQ ID NO:** |
|---|---|---|---|
| CD19 | muCTL019 | DIQMTQTTSSLSASLGDRVTISCRASQDISKYLNWYQQKPDGTVKLLI YHTSRLHSGVPSRFSGSGSGTDYSLTISNLEQEDIATYFCQQGNTLP YTFGGGTKLEITGGGGSGGGGSGGGGSEVKLQESGPGLVAPSQSLS VTCTVSGVSLPDYGVSWIRQPPRKGLEWLGVIWGSETTYYNSALKSR LTIIKDNSKSQVFLKMNSLQTDDTAIYYCAKHYYYGGSYAMDYWGQG TSVTVSS | 356 |
| CD19 | huscFv1 | EIVMTQSPATLSLSPGERATLSCRASQDISKYLNWYQQKPGQAPRLLI YHTSRLHSGIPARFSGSGSGTDYTLTISSLQPEDFAVYFCQQGNTLPY TFGQGTKLEIKGGGGSGGGGSGGGGSQVQLQESGPGLVKPSETLSL TCTVSGVSLPDYGVSWIRQPPGKGLEWIGVIWGSETTYYSSSLKSRV TISKDNSKNQVSLKLSSVTAADTAVYYCAKHYYYGGSYAMDYWGQG TLVTVSS | 357 |
| CD19 | huscFv2 | EIVMTQSPATLSLSPGERATLSCRASQDISKYLNWYQQKPGQAPRLLI YHTSRLHSGIPARFSGSGSGTDYTLTISSLQPEDFAVYFCQQGNTLPY TFGQGTKLEIKGGGGSGGGGSGGGGSQVQLQESGPGLVKPSETLSL TCTVSGVSLPDYGVSWIRQPPGKGLEWIGVIWGSETTYYQSSLKSRV TISKDNSKNQVSLKLSSVTAADTAVYYCAKHYYYGGSYAMDYWGQG TLVTVSS | 358 |
| CD19 | huscFv3 | QVQLQESGPGLVKPSETLSLTCTVSGVSLPDYGVSWIRQPPGKGLE WIGVIWGSETTYYSSSLKSRVTISKDNSKNQVSLKLSSVTAADTAVYY CAKHYYYGGSYAMDYWGQGTLVTVSSGGGGSGGGGSGGGGSEIV MTQSPATLSLSPGERATLSCRASQDISKYLNWYQQKPGQAPRLLIYH TSRLHSGIPARFSGSGSGTDYTLTISSLQPEDFAVYFCQQGNTLPYTF GQGTKLEIK | 359 |
| CD19 | huscFv4 | QVQLQESGPGLVKPSETLSLTCTVSGVSLPDYGVSWIRQPPGKGLE WIGVIWGSETTYYQSSLKSRVTISKDNSKNQVSLKLSSVTAADTAVYY CAKHYYYGGSYAMDYWGQGTLVTVSSGGGGSGGGGSGGGGSEIV MTQSPATLSLSPGERATLSCRASQDISKYLNWYQQKPGQAPRLLIYH TSRLHSGIPARFSGSGSGTDYTLTISSLQPEDFAVYFCQQGNTLPYTF GQGTKLEIK | 360 |
| CD19 | huscFv5 | EIVMTQSPATLSLSPGERATLSCRASQDISKYLNWYQQKPGQAPRLLI YHTSRLHSGIPARFSGSGSGTDYTLTISSLQPEDFAVYFCQQGNTLPY TFGQGTKLEIKGGGGSGGGGSGGGGSGGGGSQVQLQESGPGLVK PSETLSLTCTVSGVSLPDYGVSWIRQPPGKGLEWIGVIWGSETTYYS SSLKSRVTISKDNSKNQVSLKLSSVTAADTAVYYCAKHYYYGGSYAM DYWGQGTLVTVSS | 361 |
| CD19 | huscFv6 | EIVMTQSPATLSLSPGERATLSCRASQDISKYLNWYQQKPGQAPRLLI YHTSRLHSGIPARFSGSGSGTDYTLTISSLQPEDFAVYFCQQGNTLPY | 362 |
| | | TFGQGTKLEIKGGGGSGGGGSGGGGSGGGGSQVQLQESGPGLVK PSETLSLTCTVSGVSLPDYGVSWIRQPPGKGLEWIGVIWGSETTYYQ SSLKSRVTISKDNSKNQVSLKLSSVTAADTAVYYCAKHYYYGGSYAM DYWGQGTLVTVSS | |
| CD19 | huscFv7 | QVQLQESGPGLVKPSETLSLTCTVSGVSLPDYGVSWIRQPPGKGLE WIGVIWGSETTYYSSSLKSRVTISKDNSKNQVSLKLSSVTAADTAVYY CAKHYYYGGSYAMDYWGQGTLVTVSSGGGGSGGGGSGGGGSGG GGSEIVMTQSPATLSLSPGERATLSCRASQDISKYLNWYQQKPGQAP RLLIYHTSRLHSGIPARFSGSGSGTDYTLTISSLQPEDFAVYFCQQGN TLPYTFGQGTKLEIK | 363 |
| CD19 | huscFv8 | QVQLQESGPGLVKPSETLSLTCTVSGVSLPDYGVSWIRQPPGKGLE WIGVIWGSETTYYQSSLKSRVTISKDNSKNQVSLKLSSVTAADTAVYY CAKHYYYGGSYAMDYWGQGTLVTVSSGGGGSGGGGSGGGGSGG GGSEIVMTQSPATLSLSPGERATLSCRASQDISKYLNWYQQKPGQAP RLLIYHTSRLHSGIPARFSGSGSGTDYTLTISSLQPEDFAVYFCQQGN TLPYTFGQGTKLEIK | 364 |
| CD19 | huscFv9 | EIVMTQSPATLSLSPGERATLSCRASQDISKYLNWYQQKPGQAPRLLI YHTSRLHSGIPARFSGSGSGTDYTLTISSLQPEDFAVYFCQQGNTLPY TFGQGTKLEIKGGGGSGGGGSGGGGSGGGGSQVQLQESGPGLVK PSETLSLTCTVSGVSLPDYGVSWIRQPPGKGLEWIGVIWGSETTYYN SSLKSRVTISKDNSKNQVSLKLSSVTAADTAVYYCAKHYYYGGSYAM DYWGQGTLVTVSS | 365 |
| CD19 | Hu scFv10 | QVQLQESGPGLVKPSETLSLTCTVSGVSLPDYGVSWIRQPPGKGLE WIGVIWGSETTYYNSSLKSRVTISKDNSKNQVSLKLSSVTAADTAVYY CAKHYYYGGSYAMDYWGQGTLVTVSSGGGGSGGGGSGGGGSGG GGSEIVMTQSPATLSLSPGERATLSCRASQDISKYLNWYQQKPGQAP RLLIYHTSRLHSGIPARFSGSGSGTDYTLTISSLQPEDFAVYFCQQGN TLPYTFGQGTKLEIK | 366 |
| CD19 | Hu scFv11 | EIVMTQSPATLSLSPGERATLSCRASQDISKYLNWYQQKPGQAPRLLI YHTSRLHSGIPARFSGSGSGTDYTLTISSLQPEDFAVYFCQQGNTLPY TFGQGTKLEIKGGGGSGGGGSGGGGSQVQLQESGPGLVKPSETLSL TCTVSGVSLPDYGVSWIRQPPGKGLEWIGVIWGSETTYYNSSLKSRV TISKDNSKNQVSLKLSSVTAADTAVYYCAKHYYYGGSYAMDYWGQG TLVTVSS | 367 |
| CD19 | Hu scFv12 | QVQLQESGPGLVKPSETLSLTCTVSGVSLPDYGVSWIRQPPGKGLE WIGVIWGSETTYYNSSLKSRVTISKDNSKNQVSLKLSSVTAADTAVYY CAKHYYYGGSYAMDYWGQGTLVTVSSGGGGSGGGGSGGGGSEIV MTQSPATLSLSPGERATLSCRASQDISKYLNWYQQKPGQAPRLLIYH TSRLHSGIPARFSGSGSGTDYTLTISSLQPEDFAVYFCQQGNTLPYTF GQGTKLEIK | 368 |

The CDR sequences of the scFv domains of the CD19 antigen binding domains provided in Table 7 are shown in Tables 8 and 15B for the heavy chain variable domains and in Tables 9 and 15C for the light chain variable domains. "ID" stands for the respective SEQ ID NO for each CDR.

**Table 8. Heavy Chain Variable Domain CDRs**

| Description | FW | HCDR1 | SEQ ID NO | HCDR2 | SEQ ID NO | HCDR3 | SEQ ID NO |
|---|---|---|---|---|---|---|---|
| murine_CART19 | | GVSLPDYGVS | 369 | VIWGSETTYYNSALKS | 370 | HYYYGGSYAMDY | 371 |
| humanized_CART19 a | VH4 | GVSLPDYGVS | 369 | VIWGSETTYY***S***S***S***LKS | 372 | HYYYGGSYAMDY | 371 |
| humanized_CART19 b | VH4 | GVSLPDYGVS | 369 | VIWGSETTYY***Q***S***S***LKS | 373 | HYYYGGSYAMDY | 371 |
| humanized_CART19 c | VH4 | GVSLPDYGVS | 369 | VIWGSETTYYNS***S***LKS | 374 | HYYYGGSYAMDY | 371 |

**Table 9. Light Chain Variable Domain CDRs**

| Description | FW | LCDR1 | SEQ ID NO | LCDR2 | SEQ ID NO | LCDR3 | SEQ ID NO |
|---|---|---|---|---|---|---|---|
| murine_CART19 | | RASQDISKYLN | 375 | HTSRLHS | 376 | QQGNTLPYT | 377 |
| humanized_CART19 a | VK3 | RASQDISKYLN | 375 | HTSRLHS | 376 | QQGNTLPYT | 377 |
| humanized_CART19 b | VK3 | RASQDISKYLN | 375 | HTSRLHS | 376 | QQGNTLPYT | 377 |
| humanized_CART19 c | VK3 | RASQDISKYLN | 375 | HTSRLHS | 376 | QQGNTLPYT | 377 |

In an embodiment, the antigen binding domain comprises an anti-CD19 antibody, or fragment thereof, e.g., an scFv. For example, the antigen binding domain comprises a variable heavy chain and a variable light chain listed in Table 10. The linker sequence joining the variable heavy and variable light chains can be any of the linker sequences described herein, or alternatively, can be GSTSGSGKPGSGEGSTKG (SEQ ID NO: 378). The light chain variable region and heavy chain variable region of a scFv can be, e.g., in any of the following orientations: light chain variable region-linker-heavy chain variable region or heavy chain variable region-linker-light chain variable region.

**Table 10. Additional Anti-CD19 antibody binding domains**

| **Ab Name** | **VH Sequence** | **VL Sequence** |
|---|---|---|
| SJ25-C1 | QVQLLESGAELVRPGSSVKISCKASG YAFSSYWMNWVKQRPGQGLEWIGQI YPGDGDTNYNGKFKGQATLTADKSS STAYMQLSGLTSEDSAVYSCARKTISS VVDFYFDYWGQGTTVT (SEQ ID NO: 379) | ELVLTQSPKFMSTSVGDRVSVTCKASQN VGTNVAWYQQKPGQSPKPLIYSATYRNS GVPDRFTGSGSGTDFTLTITNVQSKDLA DYFYFCQYNRYPYTSGGGTKLEIKRRS (SEQ ID NO: 380) |

| | **ScFv Sequence** | |
|---|---|---|
| SJ25-C1 scFv | QVQLLESGAELVRPGSSVKISCKASGYAFSSYWMNWVKQRPGQGLEWIGQIYPGD GDTNYNGKFKGQATLTADKSSSTAYMQLSGLTSEDSAVYSCARKTISSVVDFYFDY WGQGTTVTGSTSGSGKPGSGEGSTKGELVLTQSPKFMSTSVGDRVSVTCKASQNV GTNVAWYQQKPGQSPKPLIYSATYRNSGVPDRFTGSGSGTDFTLTITNVQSKDLAD YFYFCQYNRYPYTSGGGTKLEIKRRS (SEQ ID NO: 381) | |

In one embodiment, the CD19 binding domain comprises one or more (e.g., all three) light chain complementary determining region 1 (LC CDR1), light chain complementary determining region 2 (LC CDR2), and light chain complementary determining region 3 (LC CDR3) of a CD19 binding domain described herein, e.g., provided in Table 7 or 9, and/or one or more (e.g., all three) heavy chain complementary determining region 1 (HC CDR1), heavy chain complementary determining region 2 (HC CDR2), and heavy chain complementary determining region 3 (HC CDR3) of a CD19 binding domain described herein, e.g., provided in Table 7 or 8. In one embodiment, the CD19 binding domain comprises one, two, or all of LC CDR1, LC CDR2, and LC CDR3 of any amino acid sequences as provided in Table 9; and one, two or all of HC CDR1, HC CDR2, and HC CDR3 of any amino acid sequences as provided in Table 8.

In one embodiment, the CD19 binding domain comprises a light chain variable region described herein (e.g., in Table 7 or 10) and/or a heavy chain variable region described herein (e.g., in Table 7 or 10). In one embodiment, the CD19 binding domain is a scFv comprising a light chain and a heavy chain of an amino acid sequence listed in Table 7 or 10. In an embodiment, the CD19 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 7 or 10, or a sequence with 95-99% identity with an amino acid sequence provided in Table 7 or 10; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 7 or 10, or a sequence with 95-99% identity to an amino acid sequence provided in Table 7 or 10.

In one embodiment, the CD19 binding domain comprises an amino acid sequence selected from any one of SEQ ID NOs: 356-368 and 381; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the CD19 binding domain is a scFv, and a light chain variable region comprising an amino acid sequence described herein, e.g., in Table 7 or 10, is attached to a heavy chain variable region comprising an amino acid sequence described herein, e.g., in Table 7 or 10, via a linker, e.g., a linker described herein. In one embodiment, the CD19 binding domain includes a (Gly4-Ser)n linker, wherein n is 1, 2, 3, 4, 5, or 6, preferably 4 (SEQ ID NO: 967). The light chain variable region and heavy chain variable region of a scFv can be, e.g., in any of the following orientations: light chain variable region-linker-heavy chain variable region or heavy chain variable region-linker-light chain variable region.

Any known CAR, e.g., the CD19 antigen binding domain of any known CAR, in the art can be used in accordance with the instant invention to construct a CAR. For example, LG-740; CAR described in the US Pat. No. 8,399,645; US Pat. No. 7,446,190; Xu et al., Leuk Lymphoma. 2013 54(2):255-260(2012); Cruz et al., Blood 122(17):2965-2973 (2013); Brentjens et al., Blood, 118(18):4817-4828 (2011); Kochenderfer et al., Blood 116(20):4099-102 (2010); Kochenderfer et al., Blood 122 (25):4129-39(2013); and 16th Annu Meet Am Soc Gen Cell Ther (ASGCT) (May 15-18, Salt Lake City) 2013, Abst 10. In one embodiment, an antigen binding domain against CD19 is an antigen binding portion, e.g., CDRs, of a CAR, antibody or antigen-binding fragment thereof described in, e.g., PCT publication WO2012/079000; PCT publication WO2014/153270; Kochenderfer, J.N. et al., J. Immunother. 32 (7), 689-702 (2009); Kochenderfer, J.N., et al., Blood, 116 (20), 4099-4102 (2010); PCT publication WO2014/031687; Bejcek, Cancer Research, 55, 2346-2351, 1995; or U.S. Patent No. 7,446,190.

In an embodiment, the antigen-binding domain of a CAR, e.g., the CAR expressed by a cell of the invention, binds to BCMA. BCMA is found preferentially expressed in mature B lymphocytes. In an embodiment, the antigen binding domain is a murine scFv domain that binds to human BCMA. In an embodiment, the antigen binding domain is a humanized antibody or antibody fragment, e.g., scFv domain, that binds human BCMA. In an embodiment, the antigen binding domain is a human antibody or antibody fragment that binds to human BCMA. Exemplary scFv domains (and their sequences, e.g., CDRs, VL and VH sequences) that bind to BCMA are provided in Table 11, Table 12, Table 13 and Table 14. The scFv domain sequences provided in Table 11 and Table 12 include a light chain variable region (VL) and a heavy chain variable region (VH). The VL and VH are attached by a linker, e.g., in the following orientation: VH-linker-VL.

**Table 11. Antigen Binding domains that bind BCMA**

| The amino acid sequences variable heavy chain and variable light chain sequences for each scFv is also provided. | | |
|---|---|---|
| **Name/ Description** | **SEQ ID NO:** | **Sequence** |
| **139109** | | |
| **139109-aa ScFv domain** | 382 | EVQLVESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSDIQLTQSPSSLS ASVGDRVTITCRASQSISSYLNWYQQKPGKAPKLLIYAASSLQSGVPSRF SGSGSGTDFTLTISSLQPEDFATYYCQQSYSTPYTFGQGTKVEIK |
| **139109- nt ScFv domain** | 383 | GAAGTGCAATTGGTGGAATCAGGGGGAGGACTTGTGCAGCCTGGAG GATCGCTGAGACTGTCATGTGCCGTGTCCGGCTTTGCCCTGTCCAAC CACGGGATGTCCTGGGTCCGCCGCGCGCCTGGAAAGGGCCTCGAAT GGGTGTCGGGTATTGTGTACAGCGGTAGCACCTACTATGCCGCATCC GTGAAGGGGAGATTCACCATCAGCCGGGACAACTCCAGGAACACTCT GTACCTCCAAATGAATTCGCTGAGGCCAGAGGACACTGCCATCTACT ACTGCTCCGCGCATGGCGGAGAGTCCGACGTCTGGGGACAGGGGA CCACCGTGACCGTGTCTAGCGCGTCCGGCGGAGGCGGCAGCGGGG |
| | | GTCGGGCATCAGGGGGCGGCGGATCGGACATCCAGCTCACCCAGTC CCCGAGCTCGCTGTCCGCCTCCGTGGGAGATCGGGTCACCATCACG TGCCGCGCCAGCCAGTCGATTTCCTCCTACCTGAACTGGTACCAACA GAAGCCCGGAAAAGCCCCGAAGCTTCTCATCTACGCCGCCTCGAGC CTGCAGTCAGGAGTGCCCTCACGGTTCTCCGGCTCCGGTTCCGGTA CTGATTTCACCCTGACCATTTCCTCCCTGCAACCGGAGGACTTCGCT ACTTACTACTGCCAGCAGTCGTACTCCACCCCCTACACTTTCGGACAA GGCACCAAGGTCGAAATCAAG |
| **139109-aa VH** | 384 | EVQLVESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139109-aa VL** | 385 | DIQLTQSPSSLSASVGDRVTITCRASQSISSYLNWYQQKPGKAPKLLIYA ASSLQSGVPSRFSGSGSGTDFTLTISSLQPEDFATYYCQQSYSTPYTFG QGTKVEIK |

| **139103** | | |
|---|---|---|
| **139103-aa ScFv domain** | 386 | QVQLVESGGGLVQPGRSLRLSCAASGFTFSNYAMSWVRQAPGKGLGW VSGISRSGENTYYADSVKGRFTISRDNSKNTLYLQMNSLRDEDTAVYYC ARSPAHYYGGMDVWGQGTTVTVSSASGGGGSGGRASGGGGSDIVLTQ SPGTLSLSPGERATLSCRASQSISSSFLAWYQQKPGQAPRLLIYGASRR ATGIPDRFSGSGSGTDFTLTISRLEPEDSAVYYCQQYHSSPSWTFGQGT KLEIK |
| **139103-nt ScFv domain** | 387 | CAAGTGCAACTCGTGGAATCTGGTGGAGGACTCGTGCAACCCGGAA GATCGCTTAGACTGTCGTGTGCCGCCAGCGGGTTCACTTTCTCGAAC TACGCGATGTCCTGGGTCCGCCAGGCACCCGGAAAGGGACTCGGTT GGGTGTCCGGCATTTCCCGGTCCGGCGAAAATACCTACTACGCCGA CTCCGTGAAGGGCCGCTTCACCATCTCAAGGGACAACAGCAAAAACA CCCTGTACTTGCAAATGAACTCCCTGCGGGATGAAGATACAGCCGTG TACTATTGCGCCCGGTCGCCTGCCCATTACTACGGCGGAATGGACGT CTGGGGACAGGGAACCACTGTGACTGTCAGCAGCGCGTCGGGTGGC GGCGGCTCAGGGGGTCGGGCCTCCGGGGGGGGAGGGTCCGACATC GTGCTGACCCAGTCCCCGGGAACCCTGAGCCTGAGCCCGGGAGAGC GCGCGACCCTGTCATGCCGGGCATCCCAGAGCATTAGCTCCTCCTTT CTCGCCTGGTATCAGCAGAAGCCCGGACAGGCCCCGAGGCTGCTGA TCTACGGCGCTAGCAGAAGGGCTACCGGAATCCCAGACCGGTTCTC CGGCTCCGGTTCCGGGACCGATTTCACCCTTACTATCTCGCGCCTGG AACCTGAGGACTCCGCCGTCTACTACTGCCAGCAGTACCACTCATCC CCGTCGTGGACGTTCGGACAGGGCACCAAGCTGGAGATTAAG |
| **139103-aa VH** | 388 | QVQLVESGGGLVQPGRSLRLSCAASGFTFSNYAMSWVRQAPGKGLGW VSGISRSGENTYYADSVKGRFTISRDNSKNTLYLQMNSLRDEDTAVYYC ARSPAHYYGGMDVWGQGTTVTVSS |
| **139103-aa VL** | 389 | DIVLTQSPGTLSLSPGERATLSCRASQSISSSFLAWYQQKPGQAPRLLIY GASRRATGIPDRFSGSGSGTDFTLTISRLEPEDSAVYYCQQYHSSPSWT FGQGTKLEIK |

| **139105** | | |
|---|---|---|
| **139105-aa ScFv domain** | 390 | QVQLVESGGGLVQPGRSLRLSCAASGFTFDDYAMHWVRQAPGKGLEW VSGISWNSGSIGYADSVKGRFTISRDNAKNSLYLQMNSLRAEDTALYYC SVHSFLAYWGQGTLVTVSSASGGGGSGGRASGGGGSDIVMTQTPLSLP VTPGEPASISCRSSQSLLHSNGYNYLDWYLQKPGQSPQLLIYLGSNRAS GVPDRFSGSGSGTDFTLKISRVEAEDVGVYYCMQALQTPYTFGQGTKV EIK |
| **139105- nt ScFv domain** | 391 | CAAGTGCAACTCGTCGAATCCGGTGGAGGTCTGGTCCAACCTGGTAG AAGCCTGAGACTGTCGTGTGCGGCCAGCGGATTCACCTTTGATGACT ATGCTATGCACTGGGTGCGGCAGGCCCCAGGAAAGGGCCTGGAATG GGTGTCGGGAATTAGCTGGAACTCCGGGTCCATTGGCTACGCCGAC TCCGTGAAGGGCCGCTTCACCATCTCCCGCGACAACGCAAAGAACTC |
| | | CCTGTACTTGCAAATGAACTCGCTCAGGGCTGAGGATACCGCGCTGT ACTACTGCTCCGTGCATTCCTTCCTGGCCTACTGGGGACAGGGAACT CTGGTCACCGTGTCGAGCGCCTCCGGCGGCGGGGGCTCGGGTGGA CGGGCCTCGGGCGGAGGGGGGTCCGACATCGTGATGACCCAGACC CCGCTGAGCTTGCCCGTGACTCCCGGAGAGCCTGCATCCATCTCCT GCCGGTCATCCCAGTCCCTTCTCCACTCCAACGGATACAACTACCTC GACTGGTACCTCCAGAAGCCGGGACAGAGCCCTCAGCTTCTGATCTA CCTGGGGTCAAATAGAGCCTCAGGAGTGCCGGATCGGTTCAGCGGA TCTGGTTCGGGAACTGATTTCACTCTGAAGATTTCCCGCGTGGAAGC CGAGGACGTGGGCGTCTACTACTGTATGCAGGCGCTGCAGACCCCC TATACCTTCGGCCAAGGGACGAAAGTGGAGATCAAG |
| **139105- aa VH** | 392 | QVQLVESGGGLVQPGRSLRLSCAASGFTFDDYAMHWVRQAPGKGLEW VSGISWNSGSIGYADSVKGRFTISRDNAKNSLYLQMNSLRAEDTALYYC SVHSFLAYWGQGTLVTVSS |
| **139105- aa VL** | 393 | DIVMTQTPLSLPVTPGEPASISCRSSQSLLHSNGYNYLDWYLQKPGQSP QLLIYLGSNRASGVPDRFSGSGSGTDFTLKISRVEAEDVGVYYCMQALQ TPYTFGQGTKVEIK |

| **139111** | | |
|---|---|---|
| **139111-aa ScFv domain** | 394 | EVQLLESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSDIVMTQTPLSLS VTPGQPASISCKSSQSLLRNDGKTPLYWYLQKAGQPPQLLIYEVSNRFS GVPDRFSGSGSGTDFTLKISRVEAEDVGAYYCMQNIQFPSFGGGTKLEI K |
| **139111- nt ScFv domain** | 395 | GAAGTGCAATTGTTGGAATCTGGAGGAGGACTTGTGCAGCCTGGAG GATCACTGAGACTTTCGTGTGCGGTGTCAGGCTTCGCCCTGAGCAAC CACGGCATGAGCTGGGTGCGGAGAGCCCCGGGGAAGGGTCTGGAA TGGGTGTCCGGGATCGTCTACTCCGGTTCAACTTACTACGCCGCAAG CGTGAAGGGTCGCTTCACCATTTCCCGCGATAACTCCCGGAACACCC TGTACCTCCAAATGAACTCCCTGCGGCCCGAGGACACCGCCATCTAC TACTGTTCCGCGCATGGAGGAGAGTCCGATGTCTGGGGACAGGGCA CTACCGTGACCGTGTCGAGCGCCTCGGGGGGAGGAGGCTCCGGCG GTCGCGCCTCCGGGGGGGGTGGCAGCGACATTGTGATGACGCAGA CTCCACTCTCGCTGTCCGTGACCCCGGGACAGCCCGCGTCCATCTC GTGCAAGAGCTCCCAGAGCCTGCTGAGGAACGACGGAAAGACTCCT CTGTATTGGTACCTCCAGAAGGCTGGACAGCCCCCGCAACTGCTCAT CTACGAAGTGTCAAATCGCTTCTCCGGGGTGCCGGATCGGTTTTCCG GCTCGGGATCGGGCACCGACTTCACCCTGAAAATCTCCAGGGTCGA GGCCGAGGACGTGGGAGCCTACTACTGCATGCAAAACATCCAGTTCC CTTCCTTCGGCGGCGGCACAAAGCTGGAGATTAAG |
| **139111-aa VH** | 396 | EVQLLESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139111-aa VL** | 397 | DIVMTQTPLSLSVTPGQPASISCKSSQSLLRNDGKTPLYWYLQKAGQPP QLLIYEVSNRFSGVPDRFSGSGSGTDFTLKISRVEAEDVGAYYCMQNIQF PSFGGGTKLEIK |

| **139100** | | |
|---|---|---|
| **139100- aa ScFv domain** | 398 | QVQLVQSGAEVRKTGASVKVSCKASGYIFDNFGINWVRQAPGQGLEWM GWINPKNNNTNYAQKFQGRVTITADESTNTAYMEVSSLRSEDTAVYYCA RGPYYYQSYMDVWGQGTMVTVSSASGGGGSGGRASGGGGSDIVMTQ TPLSLPVTPGEPASISCRSSQSLLHSNGYNYLNWYLQKPGQSPQLLIYLG SKRASGVPDRFSGSGSGTDFTLHITRVGAEDVGVYYCMQALQTPYTFG QGTKLEIK |
| **139100- nt ScFv domain** | 399 | CAAGTCCAACTCGTCCAGTCCGGCGCAGAAGTCAGAAAAACCGGTG CTAGCGTGAAAGTGTCCTGCAAGGCCTCCGGCTACATTTTCGATAAC |
| | | TTCGGAATCAACTGGGTCAGACAGGCCCCGGGCCAGGGGCTGGAAT GGATGGGATGGATCAACCCCAAGAACAACAACACCAACTACGCACAG AAGTTCCAGGGCCGCGTGACTATCACCGCCGATGAATCGACCAATAC CGCCTACATGGAGGTGTCCTCCCTGCGGTCGGAGGACACTGCCGTG TATTACTGCGCGAGGGGCCCATACTACTACCAAAGCTACATGGACGT CTGGGGACAGGGAACCATGGTGACCGTGTCATCCGCCTCCGGTGGT GGAGGCTCCGGGGGGCGGGCTTCAGGAGGCGGAGGAAGCGATATT GTGATGACCCAGACTCCGCTTAGCCTGCCCGTGACTCCTGGAGAACC GGCCTCCATTTCCTGCCGGTCCTCGCAATCACTCCTGCATTCCAACG GTTACAACTACCTGAATTGGTACCTCCAGAAGCCTGGCCAGTCGCCC CAGTTGCTGATCTATCTGGGCTCGAAGCGCGCCTCCGGGGTGCCTG ACCGGTTTAGCGGATCTGGGAGCGGCACGGACTTCACTCTCCACATC ACCCGCGTGGGAGCGGAGGACGTGGGAGTGTACTACTGTATGCAGG CGCTGCAGACTCCGTACACATTCGGACAGGGCACCAAGCTGGAGAT CAAG |
| **139100- aa VH** | 400 | QVQLVQSGAEVRKTGASVKVSCKASGYIFDNFGINWVRQAPGQGLEWM GWINPKNNNTNYAQKFQGRVTITADESTNTAYMEVSSLRSEDTAVYYCA RGPYYYQSYMDVWGQGTMVTVSS |
| **139100- aa VL** | 401 | DIVMTQTPLSLPVTPGEPASISCRSSQSLLHSNGYNYLNWYLQKPGQSP QLLIYLGSKRASGVPDRFSGSGSGTDFTLHITRVGAEDVGVYYCMQALQ TPYTFGQGTKLEIK |

| **139101** | | |
|---|---|---|
| **139101- aa ScFv domain** | 402 | QVQLQESGGGLVQPGGSLRLSCAASGFTFSSDAMTWVRQAPGKGLEW VSVISGSGGTTYYADSVKGRFTISRDNSKNTLYLQMNSLRAEDTAVYYC AKLDSSGYYYARGPRYWGQGTLVTVSSASGGGGSGGRASGGGGSDIQ LTQSPSSLSASVGDRVTITCRASQSISSYLNWYQQKPGKAPKLLIYGAST LASGVPARFSGSGSGTHFTLTINSLQSEDSATYYCQQSYKRASFGQGTK VEIK |
| **139101- nt ScFv domain** | 403 | CAAGTGCAACTTCAAGAATCAGGCGGAGGACTCGTGCAGCCCGGAG GATCATTGCGGCTCTCGTGCGCCGCCTCGGGCTTCACCTTCTCGAGC GACGCCATGACCTGGGTCCGCCAGGCCCCGGGGAAGGGGCTGGAA TGGGTGTCTGTGATTTCCGGCTCCGGGGGAACTACGTACTACGCCGA TTCCGTGAAAGGTCGCTTCACTATCTCCCGGGACAACAGCAAGAACA CCCTTTATCTGCAAATGAATTCCCTCCGCGCCGAGGACACCGCCGTG TACTACTGCGCCAAGCTGGACTCCTCGGGCTACTACTATGCCCGGGG TCCGAGATACTGGGGACAGGGAACCCTCGTGACCGTGTCCTCCGCG TCCGGCGGAGGAGGGTCGGGAGGGCGGGCCTCCGGCGGCGGCGG TTCGGACATCCAGCTGACCCAGTCCCCATCCTCACTGAGCGCAAGCG TGGGCGACAGAGTCACCATTACATGCAGGGCGTCCCAGAGCATCAG CTCCTACCTGAACTGGTACCAACAGAAGCCTGGAAAGGCTCCTAAGC TGTTGATCTACGGGGCTTCGACCCTGGCATCCGGGGTGCCCGCGAG GTTTAGCGGAAGCGGTAGCGGCACTCACTTCACTCTGACCATTAACA GCCTCCAGTCCGAGGATTCAGCCACTTACTACTGTCAGCAGTCCTAC AAGCGGGCCAGCTTCGGACAGGGCACTAAGGTCGAGATCAAG |
| **139101-aa VH** | 404 | QVQLQESGGGLVQPGGSLRLSCAASGFTFSSDAMTWVRQAPGKGLEW VSVISGSGGTTYYADSVKGRFTISRDNSKNTLYLQMNSLRAEDTAVYYC AKLDSSGYYYARGPRYWGQGTLVTVSS |
| **139101-aa VL** | 405 | DIQLTQSPSSLSASVGDRVTITCRASQSISSYLNWYQQKPGKAPKLLIYG ASTLASGVPARFSGSGSGTHFTLTINSLQSEDSATYYCQQSYKRASFGQ GTKVEIK |

| **139102** | | |
|---|---|---|
| **139102- aa ScFv domain** | 406 | QVQLVQSGAEVKKPGASVKVSCKASGYTFSNYGITWVRQAPGQGLEW MGWISAYNGNTNYAQKFQGRVTMTRNTSISTAYMELSSLRSEDTAVYYC ARGPYYYYMDVWGKGTMVTVSSASGGGGSGGRASGGGGSEIVMTQS PLSLPVTPGEPASISCRSSQSLLYSNGYNYVDWYLQKPGQSPQLLIYLGS |
| | | NRASGVPDRFSGSGSGTDFKLQISRVEAEDVGIYYCMQGRQFPYSFGQ GTKVEIK |
| **139102- nt ScFv domain** | 407 | CAAGTCCAACTGGTCCAGAGCGGTGCAGAAGTGAAGAAGCCCGGAG CGAGCGTGAAAGTGTCCTGCAAGGCTTCCGGGTACACCTTCTCCAAC TACGGCATCACTTGGGTGCGCCAGGCCCCGGGACAGGGCCTGGAAT GGATGGGGTGGATTTCCGCGTACAACGGCAATACGAACTACGCTCAG AAGTTCCAGGGTAGAGTGACCATGACTAGGAACACCTCCATTTCCAC CGCCTACATGGAACTGTCCTCCCTGCGGAGCGAGGACACCGCCGTG TACTATTGCGCCCGGGGACCATACTACTACTACATGGATGTCTGGGG GAAGGGGACTATGGTCACCGTGTCATCCGCCTCGGGAGGCGGCGGA TCAGGAGGACGCGCCTCTGGTGGTGGAGGATCGGAGATCGTGATGA CCCAGAGCCCTCTCTCCTTGCCCGTGACTCCTGGGGAGCCCGCATC CATTTCATGCCGGAGCTCCCAGTCACTTCTCTACTCCAACGGCTATAA CTACGTGGATTGGTACCTCCAAAAGCCGGGCCAGAGCCCGCAGCTG CTGATCTACCTGGGCTCGAACAGGGCCAGCGGAGTGCCTGACCGGT TCTCCGGGTCGGGAAGCGGGACCGACTTCAAGCTGCAAATCTCGAG AGTGGAGGCCGAGGACGTGGGAATCTACTACTGTATGCAGGGCCGC CAGTTTCCGTACTCGTTCGGACAGGGCACCAAAGTGGAAATCAAG |
| **139102- aa VH** | 408 | QVQLVQSGAEVKKPGASVKVSCKASGYTFSNYGITWVRQAPGQGLEW MGWISAYNGNTNYAQKFQGRVTMTRNTSISTAYMELSSLRSEDTAVYYC ARGPYYYYMDVWGKGTMVTVSS |
| **139102- aa VL** | 409 | EIVMTQSPLSLPVTPGEPASISCRSSQSLLYSNGYNYVDWYLQKPGQSP QLLIYLGSNRASGVPDRFSGSGSGTDFKLQISRVEAEDVGIYYCMQGRQ FPYSFGQGTKVEIK |

| **139104** | | |
|---|---|---|
| **139104- aa ScFv domain** | 410 | EVQLLETGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSEIVLTQSPATLS VSPGESATLSCRASQSVSSNLAWYQQKPGQAPRLLIYGASTRASGIPDR FSGSGSGTDFTLTISSLQAEDVAVYYCQQYGSSLTFGGGTKVEIK |
| **139104- nt ScFv domain** | 411 | GAAGTGCAATTGCTCGAAACTGGAGGAGGTCTGGTGCAACCTGGAG GATCACTTCGCCTGTCCTGCGCCGTGTCGGGCTTTGCCCTGTCCAAC CATGGAATGAGCTGGGTCCGCCGCGCGCCGGGGAAGGGCCTCGAA TGGGTGTCCGGCATCGTCTACTCCGGCTCCACCTACTACGCCGCGTC CGTGAAGGGCCGGTTCACGATTTCACGGGACAACTCGCGGAACACC CTGTACCTCCAAATGAATTCCCTTCGGCCGGAGGATACTGCCATCTA CTACTGCTCCGCCCACGGTGGCGAATCCGACGTCTGGGGCCAGGGA ACCACCGTGACCGTGTCCAGCGCGTCCGGGGGAGGAGGAAGCGGG GGTAGAGCATCGGGTGGAGGCGGATCAGAGATCGTGCTGACCCAGT CCCCCGCCACCTTGAGCGTGTCACCAGGAGAGTCCGCCACCCTGTC ATGCCGCGCCAGCCAGTCCGTGTCCTCCAACCTGGCTTGGTACCAG CAGAAGCCGGGGCAGGCCCCTAGACTCCTGATCTATGGGGCGTCGA CCCGGGCATCTGGAATTCCCGATAGGTTCAGCGGATCGGGCTCGGG CACTGACTTCACTCTGACCATCTCCTCGCTGCAAGCCGAGGACGTGG CTGTGTACTACTGTCAGCAGTACGGAAGCTCCCTGACTTTCGGTGGC GGGACCAAAGTCGAGATTAAG |
| **139104- aa VH** | 412 | EVQLLETGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139104- aa VL** | 413 | EIVLTQSPATLSVSPGESATLSCRASQSVSSNLAWYQQKPGQAPRLLIY GASTRASGIPDRFSGSGSGTDFTLTISSLQAEDVAVYYCQQYGSSLTFG GGTKVEIK |

| **139106** | | |
|---|---|---|
| **139106- aa ScFv domain** | 414 | EVQLVETGGGLVQPGGSLRLSCAVSGFALSNHGMSWVRRAPGKGLEW VSGIVYSGSTYYAASVKGRFTISRDNSRNTLYLQMNSLRPEDTAIYYCSA |
| | | HGGESDVWGQGTTVTVSSASGGGGSGGRASGGGGSEIVMTQSPATLS VSPGERATLSCRASQSVSSKLAWYQQKPGQAPRLLMYGASIRATGIPDR FSGSGSGTEFTLTISSLEPEDFAVYYCQQYGSSSWTFGQGTKVEIK |
| **139106-nt ScFv domain** | 415 | GAAGTGCAATTGGTGGAAACTGGAGGAGGACTTGTGCAACCTGGAG GATCATTGAGACTGAGCTGCGCAGTGTCGGGATTCGCCCTGAGCAAC CATGGAATGTCCTGGGTCAGAAGGGCCCCTGGAAAAGGCCTCGAAT GGGTGTCAGGGATCGTGTACTCCGGTTCCACTTACTACGCCGCCTCC GTGAAGGGGCGCTTCACTATCTCACGGGATAACTCCCGCAATACCCT GTACCTCCAAATGAACAGCCTGCGGCCGGAGGATACCGCCATCTACT ACTGTTCCGCCCACGGTGGAGAGTCTGACGTCTGGGGCCAGGGAAC TACCGTGACCGTGTCCTCCGCGTCCGGCGGTGGAGGGAGCGGCGG CCGCGCCAGCGGCGGCGGAGGCTCCGAGATCGTGATGACCCAGAG CCCCGCTACTCTGTCGGTGTCGCCCGGAGAAAGGGCGACCCTGTCC TGCCGGGCGTCGCAGTCCGTGAGCAGCAAGCTGGCTTGGTACCAGC AGAAGCCGGGCCAGGCACCACGCCTGCTTATGTACGGTGCCTCCAT TCGGGCCACCGGAATCCCGGACCGGTTCTCGGGGTCGGGGTCCGG TACCGAGTTCACACTGACCATTTCCTCGCTCGAGCCCGAGGACTTTG CCGTCTATTACTGCCAGCAGTACGGCTCCTCCTCATGGACGTTCGGC CAGGGGACCAAGGTCGAAATCAAG |
| **139106-aa VH** | 416 | EVQLVETGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139106-aa VL** | 417 | EIVMTQSPATLSVSPGERATLSCRASQSVSSKLAWYQQKPGQAPRLLM YGASIRATGIPDRFSGSGSGTEFTLTISSLEPEDFAVYYCQQYGSSSWTF GQGTKVEIK |

| **139107** | | |
|---|---|---|
| **139107-aa ScFv domain** | 418 | EVQLVETGGGVVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSEIVLTQSPGTLS LSPGERATLSCRASQSVGSTNLAWYQQKPGQAPRLLIYDASNRATGIPD RFSGGGSGTDFTLTISRLEPEDFAVYYCQQYGSSPPWTFGQGTKVEIK |
| **139107-nt ScFv domain** | 419 | GAAGTGCAATTGGTGGAGACTGGAGGAGGAGTGGTGCAACCTGGAG GAAGCCTGAGACTGTCATGCGCGGTGTCGGGCTTCGCCCTCTCCAA CCACGGAATGTCCTGGGTCCGCCGGGCCCCTGGGAAAGGACTTGAA TGGGTGTCCGGCATCGTGTACTCGGGTTCCACCTACTACGCGGCCTC AGTGAAGGGCCGGTTTACTATTAGCCGCGACAACTCCAGAAACACAC TGTACCTCCAAATGAACTCGCTGCGGCCGGAAGATACCGCTATCTAC TACTGCTCCGCCCATGGGGGAGAGTCGGACGTCTGGGGACAGGGCA CCACTGTCACTGTGTCCAGCGCTTCCGGCGGTGGTGGAAGCGGGGG ACGGGCCTCAGGAGGCGGTGGCAGCGAGATTGTGCTGACCCAGTCC CCCGGGACCCTGAGCCTGTCCCCGGGAGAAAGGGCCACCCTCTCCT GTCGGGCATCCCAGTCCGTGGGGTCTACTAACCTTGCATGGTACCAG CAGAAGCCCGGCCAGGCCCCTCGCCTGCTGATCTACGACGCGTCCA ATAGAGCCACCGGCATCCCGGATCGCTTCAGCGGAGGCGGATCGGG CACCGACTTCACCCTCACCATTTCAAGGCTGGAACCGGAGGACTTCG CCGTGTACTACTGCCAGCAGTATGGTTCGTCCCCACCCTGGACGTTC GGCCAGGGGACTAAGGTCGAGATCAAG |
| **139107-aa VH** | 420 | EVQLVETGGGVVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139107- aa VL** | 421 | EIVLTQSPGTLSLSPGERATLSCRASQSVGSTNLAWYQQKPGQAPRLLI YDASNRATGIPDRFSGGGSGTDFTLTISRLEPEDFAVYYCQQYGSSPPW TFGQGTKVEIK |

| **139108** | | |
|---|---|---|
| **139108- aa** | 422 | QVQLVESGGGLVKPGGSLRLSCAASGFTFSDYYMSWIRQAPGKGLEWV |
| **ScFv domain** | | SYISSSGSTIYYADSVKGRFTISRDNAKNSLYLQMNSLRAEDTAVYYCAR ESGDGMDVWGQGTTVTVSSASGGGGSGGRASGGGGSDIQMTQSPSS LSASVGDRVTITCRASQSISSYLNWYQQKPGKAPKLLIYAASSLQSGVPS RFSGSGSGTDFTLTISSLQPEDFATYYCQQSYTLAFGQGTKVDIK |
| **139108- nt ScFv domain** | 423 | CAAGTGCAACTCGTGGAATCTGGTGGAGGACTCGTGAAACCTGGAG GATCATTGAGACTGTCATGCGCGGCCTCGGGATTCACGTTCTCCGAT TACTACATGAGCTGGATTCGCCAGGCTCCGGGGAAGGGACTGGAAT GGGTGTCCTACATTTCCTCATCCGGCTCCACCATCTACTACGCGGAC TCCGTGAAGGGGAGATTCACCATTAGCCGCGATAACGCCAAGAACAG CCTGTACCTTCAGATGAACTCCCTGCGGGCTGAAGATACTGCCGTCT ACTACTGCGCAAGGGAGAGCGGAGATGGGATGGACGTCTGGGGACA GGGTACCACTGTGACCGTGTCGTCGGCCTCCGGCGGAGGGGGTTCG GGTGGAAGGGCCAGCGGCGGCGGAGGCAGCGACATCCAGATGACC CAGTCCCCCTCATCGCTGTCCGCCTCCGTGGGCGACCGCGTCACCA TCACATGCCGGGCCTCACAGTCGATCTCCTCCTACCTCAATTGGTAT CAGCAGAAGCCCGGAAAGGCCCCTAAGCTTCTGATCTACGCAGCGT CCTCCCTGCAATCCGGGGTCCCATCTCGGTTCTCCGGCTCGGGCAG CGGTACCGACTTCACTCTGACCATCTCGAGCCTGCAGCCGGAGGACT TCGCCACTTACTACTGTCAGCAAAGCTACACCCTCGCGTTTGGCCAG GGCACCAAAGTGGACATCAAG |
| **139108- aa VH** | 424 | QVQLVESGGGLVKPGGSLRLSCAASGFTFSDYYMSWIRQAPGKGLEWV SYISSSGSTIYYADSVKGRFTISRDNAKNSLYLQMNSLRAEDTAVYYCAR ESGDGMDVWGQGTTVTVSS |
| **139108- aa VL** | 425 | DIQMTQSPSSLSASVGDRVTITCRASQSISSYLNWYQQKPGKAPKLLIYA ASSLQSGVPSRFSGSGSGTDFTLTISSLQPEDFATYYCQQSYTLAFGQG TKVDIK |

| **139110** | | |
|---|---|---|
| **139110- aa ScFv domain** | 426 | QVQLVQSGGGLVKPGGSLRLSCAASGFTFSDYYMSWIRQAPGKGLEW VSYISSSGNTIYYADSVKGRFTISRDNAKNSLYLQMNSLRAEDTAVYYCA RSTMVREDYWGQGTLVTVSSASGGGGSGGRASGGGGSDIVLTQSPLS LPVTLGQPASISCKSSESLVHNSGKTYLNWFHQRPGQSPRRLIYEVSNR DSGVPDRFTGSGSGTDFTLKISRVEAEDVGVYYCMQGTHWPGTFGQGT KLEIK |
| **139110- nt ScFv domain** | 427 | CAAGTGCAACTGGTGCAAAGCGGAGGAGGATTGGTCAAACCCGGAG GAAGCCTGAGACTGTCATGCGCGGCCTCTGGATTCACCTTCTCCGAT TACTACATGTCATGGATCAGACAGGCCCCGGGGAAGGGCCTCGAAT GGGTGTCCTACATCTCGTCCTCCGGGAACACCATCTACTACGCCGAC AGCGTGAAGGGCCGCTTTACCATTTCCCGCGACAACGCAAAGAACTC GCTGTACCTTCAGATGAATTCCCTGCGGGCTGAAGATACCGCGGTGT ACTATTGCGCCCGGTCCACTATGGTCCGGGAGGACTACTGGGGACA GGGCACACTCGTGACCGTGTCCAGCGCGAGCGGGGGTGGAGGCAG CGGTGGACGCGCCTCCGGCGGCGGCGGTTCAGACATCGTGCTGACT CAGTCGCCCCTGTCGCTGCCGGTCACCCTGGGCCAACCGGCCTCAA TTAGCTGCAAGTCCTCGGAGAGCCTGGTGCACAACTCAGGAAAGACT TACCTGAACTGGTTCCATCAGCGGCCTGGACAGTCCCCACGGAGGC TCATCTATGAAGTGTCCAACAGGGATTCGGGGGTGCCCGACCGCTTC ACTGGCTCCGGGTCCGGCACCGACTTCACCTTGAAAATCTCCAGAGT GGAAGCCGAGGACGTGGGCGTGTACTACTGTATGCAGGGTACCCAC TGGCCTGGAACCTTTGGACAAGGAACTAAGCTCGAGATTAAG |
| **139110-aa VH** | 428 | QVQLVQSGGGLVKPGGSLRLSCAASGFTFSDYYMSWIRQAPGKGLEW VSYISSSGNTIYYADSVKGRFTISRDNAKNSLYLQMNSLRAEDTAVYYCA RSTMVREDYWGQGTLVTVSS |
| **139110-aa VL** | 429 | DIVLTQSPLSLPVTLGQPASISCKSSESLVHNSGKTYLNWFHQRPGQSP RRLIYEVSNRDSGVPDRFTGSGSGTDFTLKISRVEAEDVGVYYCMQGTH WPGTFGQGTKLEIK |

| **139112** | | |
|---|---|---|
| **139112- aa ScFv domain** | 430 | QVQLVESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSDIRLTQSPSPLS ASVGDRVTITCQASEDINKFLNWYHQTPGKAPKLLIYDASTLQTGVPSRF SGSGSGTDFTLTINSLQPEDIGTYYCQQYESLPLTFGGGTKVEIK |
| **139112- nt ScFv domain** | 431 | CAAGTGCAACTCGTGGAATCTGGTGGAGGACTCGTGCAACCCGGTG GAAGCCTTAGGCTGTCGTGCGCCGTCAGCGGGTTTGCTCTGAGCAA CCATGGAATGTCCTGGGTCCGCCGGGCACCGGGAAAAGGGCTGGAA TGGGTGTCCGGCATCGTGTACAGCGGGTCAACCTATTACGCCGCGT CCGTGAAGGGCAGATTCACTATCTCAAGAGACAACAGCCGGAACACC CTGTACTTGCAAATGAATTCCCTGCGCCCCGAGGACACCGCCATCTA CTACTGCTCCGCCCACGGAGGAGAGTCGGACGTGTGGGGCCAGGG AACGACTGTGACTGTGTCCAGCGCATCAGGAGGGGGTGGTTCGGGC GGCCGGGCCTCGGGGGGAGGAGGTTCCGACATTCGGCTGACCCAG TCCCCGTCCCCACTGTCGGCCTCCGTCGGCGACCGCGTGACCATCA CTTGTCAGGCGTCCGAGGACATTAACAAGTTCCTGAACTGGTACCAC CAGACCCCTGGAAAGGCCCCCAAGCTGCTGATCTACGATGCCTCGA CCCTTCAAACTGGAGTGCCTAGCCGGTTCTCCGGGTCCGGCTCCGG CACTGATTTCACTCTGACCATCAACTCATTGCAGCCGGAAGATATCGG GACCTACTATTGCCAGCAGTACGAATCCCTCCCGCTCACATTCGGCG GGGGAACCAAGGTCGAGATTAAG |
| **139112-aa VH** | 432 | QVQLVESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139112-aa VL** | 433 | DIRLTQSPSPLSASVGDRVTITCQASEDINKFLNWYHQTPGKAPKLLIYDA STLQTGVPSRFSGSGSGTDFTLTINSLQPEDIGTYYCQQYESLPLTFGGG TKVEIK |

| **139113** | | |
|---|---|---|
| **139113- aa ScFv domain** | 434 | EVQLVETGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSETTLTQSPATLS VSPGERATLSCRASQSVGSNLAWYQQKPGQGPRLLIYGASTRATGIPAR FSGSGSGTEFTLTISSLQPEDFAVYYCQQYNDWLPVTFGQGTKVEIK |
| **139113- nt ScFv domain** | 435 | GAAGTGCAATTGGTGGAAACTGGAGGAGGACTTGTGCAACCTGGAG GATCATTGCGGCTCTCATGCGCTGTCTCCGGCTTCGCCCTGTCAAAT CACGGGATGTCGTGGGTCAGACGGGCCCCGGGAAAGGGTCTGGAAT GGGTGTCGGGGATTGTGTACAGCGGCTCCACCTACTACGCCGCTTC GGTCAAGGGCCGCTTCACTATTTCACGGGACAACAGCCGCAACACCC TCTATCTGCAAATGAACTCTCTCCGCCCGGAGGATACCGCCATCTAC TACTGCTCCGCACACGGCGGCGAATCCGACGTGTGGGGACAGGGAA CCACTGTCACCGTGTCGTCCGCATCCGGTGGCGGAGGATCGGGTGG CCGGGCCTCCGGGGGCGGCGGCAGCGAGACTACCCTGACCCAGTC CCCTGCCACTCTGTCCGTGAGCCCGGGAGAGAGAGCCACCCTTAGC TGCCGGGCCAGCCAGAGCGTGGGCTCCAACCTGGCCTGGTACCAGC AGAAGCCAGGACAGGGTCCCAGGCTGCTGATCTACGGAGCCTCCAC TCGCGCGACCGGCATCCCCGCGAGGTTCTCCGGGTCGGGTTCCGG GACCGAGTTCACCCTGACCATCTCCTCCCTCCAACCGGAGGACTTCG CGGTGTACTACTGTCAGCAGTACAACGATTGGCTGCCCGTGACATTT GGACAGGGGACGAAGGTGGAAATCAAA |
| **139113-aa VH** | 436 | EVQLVETGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139113-aa VL** | 437 | ETTLTQSPATLSVSPGERATLSCRASQSVGSNLAWYQQKPGQGPRLLIY GASTRATGIPARFSGSGSGTEFTLTISSLQPEDFAVYYCQQYNDWLPVT |
| | | FGQGTKVEIK |

| **139114** | | |
|---|---|---|
| **139114- aa ScFv domain** | 438 | EVQLVESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSSASGGGGSGGRASGGGGSEIVLTQSPGTLS LSPGERATLSCRASQSIGSSSLAWYQQKPGQAPRLLMYGASSRASGIPD RFSGSGSGTDFTLTISRLEPEDFAVYYCQQYAGSPPFTFGQGTKVEIK |
| **139114- nt ScFv domain** | 439 | GAAGTGCAATTGGTGGAATCTGGTGGAGGACTTGTGCAACCTGGAG GATCACTGAGACTGTCATGCGCGGTGTCCGGTTTTGCCCTGAGCAAT CATGGGATGTCGTGGGTCCGGCGCGCCCCCGGAAAGGGTCTGGAAT GGGTGTCGGGTATCGTCTACTCCGGGAGCACTTACTACGCCGCGAG CGTGAAGGGCCGCTTCACCATTTCCCGCGATAACTCCCGCAACACCC TGTACTTGCAAATGAACTCGCTCCGGCCTGAGGACACTGCCATCTAC TACTGCTCCGCACACGGAGGAGAATCCGACGTGTGGGGCCAGGGAA CTACCGTGACCGTCAGCAGCGCCTCCGGCGGCGGGGGCTCAGGCG GACGGGCTAGCGGCGGCGGTGGCTCCGAGATCGTGCTGACCCAGT CGCCTGGCACTCTCTCGCTGAGCCCCGGGGAAAGGGCAACCCTGTC CTGTCGGGCCAGCCAGTCCATTGGATCATCCTCCCTCGCCTGGTATC AGCAGAAACCGGGACAGGCTCCGCGGCTGCTTATGTATGGGGCCAG CTCAAGAGCCTCCGGCATTCCCGACCGGTTCTCCGGGTCCGGTTCC GGCACCGATTTCACCCTGACTATCTCGAGGCTGGAGCCAGAGGACTT CGCCGTGTACTACTGCCAGCAGTACGCGGGGTCCCCGCCGTTCACG TTCGGACAGGGAACCAAGGTCGAGATCAAG |
| **139114-aa VH** | 440 | EVQLVESGGGLVQPGGSLRLSCAVSGFALSNHGMSWVRRAPGKGLEW VSGIVYSGSTYYAASVKGRFTISRDNSRNTLYLQMNSLRPEDTAIYYCSA HGGESDVWGQGTTVTVSS |
| **139114-aa VL** | 441 | EIVLTQSPGTLSLSPGERATLSCRASQSIGSSSLAWYQQKPGQAPRLLM YGASSRASGIPDRFSGSGSGTDFTLTISRLEPEDFAVYYCQQYAGSPPF TFGQGTKVEIK |

| **149362** | | |
|---|---|---|
| **149362-aa ScFv domain** | 442 | Qvqlqesgpglvkpsetlsltctvsggsisssyyywgwirqppgkglewigsiyysgsayynpslksrv tisvdtsknqfslrlssvtaadtavyycarhwqewpdafdiwgqgtmvtvssggggsggggsggggs ettltqspafmsatpgdkviisckasqdiddamnwyqqkpgeaplfiiqsatspvpgipprfsgsgfgt dfsltinniesedaayyfclqhdnfpltfgqgtkleik |
| **149362-nt ScFv domain** | 443 | caagtgcagcttcaggaaagcggaccgggcctggtcaagccatccgaaactctctccctgacttgca ctgtgtctggcggttccatctcatcgtcgtactactactggggctggattaggcagccgcccggaaagg gactggagtggatcggaagcatctactattccggctcggcgtactacaaccctagcctcaagtcgaga gtgaccatctccgtggatacctccaagaaccagttttccctgcgcctgagctccgtgaccgccgctgac accgccgtgtactactgtgctcggcattggcaggaatggcccgatgccttcgacatttggggccagggc actatggtcactgtgtcatccgggggtggaggcagcgggggaggagggtccggggggggaggttca gagacaaccttgacccagtcacccgcattcatgtccgccactccgggagacaaggtcatcatctcgtg caaagcgtcccaggatatcgacgatgccatgaattggtaccagcagaagcctggcgaagcgccgct gttcattatccaatccgcaacctcgcccgtgcctggaatcccaccgcggttcagcggcagcggtttcgg aaccgacttttccctgaccattaacaacattgagtccgaggacgccgcctactacttctgcctgcaacac gacaacttccctctcacgttcggccagggaaccaagctggaaatcaag |
| **149362-aa VH** | 444 | QVQLQESGPGLVKPSETLSLTCTVSGGSISSSYYYWGWIRQPPGKGLE WIGSIYYSGSAYYNPSLKSRVTISVDTSKNQFSLRLSSVTAADTAVYYCA RHWQEWPDAFDIWGQGTMVTVSS |
| **149362-aa VL** | 445 | ETTLTQSPAFMSATPGDKVIISCKASQDIDDAMNWYQQKPGEAPLFIIQS ATSPVPGIPPRFSGSGFGTDFSLTINNIESEDAAYYFCLQHDNFPLTFGQ GTKLEIK |

| **149363** | | |
|---|---|---|
| **149363-aa ScFv domain** | 446 | QVNLRESGPALVKPTQTLTLTCTFSGFSLRTSGMCVSWIRQPPGKALEW LARIDWDEDKFYSTSLKTRLTISKDTSDNQVVLRMTNMDPADTATYYCA RSGAGGTSATAFDIWGPGTMVTVSSGGGGSGGGGSGGGGSDIQMTQS |
| | | PSSLSASVGDRVTITCRASQDIYNNLAWFQLKPGSAPRSLMYAANKSQS GVPSRFSGSASGTDFTLTISSLQPEDFATYYCQHYYRFPYSFGQGTKLEI K |
| **149363-nt ScFv domain** | 447 | caagtcaatctgcgcgaatccggccccgccttggtcaagcctacccagaccctcactctgacctgtact ttctccggcttctccctgcggacttccgggatgtgcgtgtcctggatcagacagcctccgggaaaggcc ctggagtggctcgctcgcattgactgggatgaggacaagttctactccacctcactcaagaccaggctg accatcagcaaagatacctctgacaaccaagtggtgctccgcatgaccaacatggacccagccgac actgccacttactactgcgcgaggagcggagcgggcggaacctccgccaccgccttcgatatttggg gcccgggtaccatggtcaccgtgtcaagcggaggaggggggtccgggggcggcggttccggggga ggcggatcggacattcagatgactcagtcaccatcgtccctgagcgctagcgtgggcgacagagtga caatcacttgccgggcatcccaggacatctataacaaccttgcgtggttccagctgaagcctggttccg caccgcggtcacttatgtacgccgccaacaagagccagtcgggagtgccgtcccggttttccggttcg gcctcgggaactgacttcaccctgacgatctccagcctgcaacccgaggatttcgccacctactactgc cagcactactaccgctttccctactcgttcggacagggaaccaagctggaaatcaag |
| **149363-aa VH** | 448 | QVNLRESGPALVKPTQTLTLTCTFSGFSLRTSGMCVSWIRQPPGKALEW LARIDWDEDKFYSTSLKTRLTISKDTSDNQVVLRMTNMDPADTATYYCA RSGAGGTSATAFDIWGPGTMVTVSS |
| **149363-aa VL** | 449 | DIQMTQSPSSLSASVGDRVTITCRASQDIYNNLAWFQLKPGSAPRSLMY AANKSQSGVPSRFSGSASGTDFTLTISSLQPEDFATYYCQHYYRFPYSF GQGTKLEIK |

| **149364** | | |
|---|---|---|
| **149364-aa ScFv domain** | 450 | evqlvesggglvkpggslrlscaasgftfssysmnwvrqapgkglewvssisssssyiyyadsvkgrft isrdnaknslylqmnslraedtavyycaktiaavyafdiwgqgttvtvssggggsggggsggggseivl tqsplslpvtpeepasiscrssqsllhsngynyldwylqkpgqspqlliylgsnrasgvpdrfsgsgsgtd ftlkisrveaedvgvyycmqalqtpytfgqgtkleik |
| **149364-nt ScFv domain** | 451 | gaagtgcagcttgtcgaatccggggggggactggtcaagccgggcggatcactgagactgtcctgcg ccgcgagcggcttcacgttctcctcctactccatgaactgggtccgccaagcccccgggaagggactg gaatgggtgtcctctatctcctcgtcgtcgtcctacatctactacgccgactccgtgaagggaagattcac catttcccgcgacaacgcaaagaactcactgtacttgcaaatgaactcactccgggccgaagatactg ctgtgtactattgcgccaagactattgccgccgtctacgctttcgacatctggggccagggaaccaccgt gactgtgtcgtccggtggtggtggctcgggcggaggaggaagcggcggcggggggtccgagattgt gctgacccagtcgccactgagcctccctgtgacccccgaggaacccgccagcatcagctgccggtcc agccagtccctgctccactccaacggatacaattacctcgattggtaccttcagaagcctggacaaag cccgcagctgctcatctacttgggatcaaaccgcgcgtcaggagtgcctgaccggttctccggctcgg gcagcggtaccgatttcaccctgaaaatctccagggtggaggcagaggacgtgggagtgtattactgt atgcaggcgctgcagactccgtacacatttgggcagggcaccaagctggagatcaag |
| **149364-aa VH** | 452 | EVQLVESGGGLVKPGGSLRLSCAASGFTFSSYSMNWVRQAPGKGLEW VSSISSSSSYIYYADSVKGRFTISRDNAKNSLYLQMNSLRAEDTAVYYCA KTIAAVYAFDIWGQGTTVTVSS |
| **149364-aa VL** | 453 | EIVLTQSPLSLPVTPEEPASISCRSSQSLLHSNGYNYLDWYLQKPGQSPQ LLIYLGSNRASGVPDRFSGSGSGTDFTLKISRVEAEDVGVYYCMQALQT PYTFGQGTKLEIK |

| **149365** | | |
|---|---|---|
| **149365-aa ScFv domain** | 454 | evqlvesggglvkpggslrlscaasgftfsdyymswirqapgkglewvsyisssgstiyyadsvkgrfti srdnaknslylqmnslraedtavyycardlrgafdiwgqgtmvtvssggggsggggsggggssyvlt qspsvsaapgytatiscggnnigtksvhwyqqkpgqapllvirddsvrpskipgrfsgsnsgnmatlti sgvqagdeadfycqvwdsdsehvvfgggtkltvl |
| **149365-nt ScFv domain** | 455 | gaagtccagctcgtggagtccggcggaggccttgtgaagcctggaggttcgctgagactgtcctgcgc cgcctccggcttcaccttctccgactactacatgtcctggatcagacaggccccgggaaagggcctgg aatgggtgtcctacatctcgtcatcgggcagcactatctactacgcggactcagtgaaggggcggttca ccatttcccgggataacgcgaagaactcgctgtatctgcaaatgaactcactgagggccgaggacac cgccgtgtactactgcgcccgcgatctccgcggggcatttgacatctggggacagggaaccatggtca cagtgtccagcggagggggaggatcgggtggcggaggttccgggggtggaggctcctcctacgtgct gactcagagcccaagcgtcagcgctgcgcccggttacacggcaaccatctcctgtggcggaaacaa cattgggaccaagtctgtgcactggtatcagcagaagccgggccaagctcccctgttggtgatccgcg |
| | | atgactccgtgcggcctagcaaaattccgggacggttctccggctccaacagcggcaatatggccact ctcaccatctcgggagtgcaggccggagatgaagccgacttctactgccaagtctgggactcagactc cgagcatgtggtgttcgggggcggaaccaagctgactgtgctc |
| **149365-aa VH** | 456 | EVQLVESGGGLVKPGGSLRLSCAASGFTFSDYYMSWIRQAPGKGLEWV SYISSSGSTIYYADSVKGRFTISRDNAKNSLYLQMNSLRAEDTAVYYCAR DLRGAFDIWGQGTMVTVSS |
| **149365-aa VL** | 457 | SYVLTQSPSVSAAPGYTATISCGGNNIGTKSVHWYQQKPGQAPLLVIRD DSVRPSKIPGRFSGSNSGNMATLTISGVQAGDEADFYCQVWDSDSEHV VFGGGTKLTVL |

| **149366** | | |
|---|---|---|
| **149366-aa ScFv domain** | 458 | qvqlvqsgaevkkpgasvkvsckpsgytvtshyihwvrrapgqglewmgminpsggvtaysqtlqg rvtmtsdtsssstvymelsslrsedtamyycaregsgsgwyfdfwgrgtlvtvssggggsggggsggg gssyvltqppsvsvspgqtasitcsgdglskkyvswyqqkagqspvvlisrdkerpsgipdrfsgsns adtatltisgtqamdeadyycqawddttvvfgggtkltvl |
| **149366-nt ScFv domain** | 459 | caagtgcagctggtgcagagcggggccgaagtcaagaagccgggagcctccgtgaaagtgtcctg caagccttcgggatacaccgtgacctcccactacattcattgggtccgccgcgcccccggccaagga ctcgagtggatgggcatgatcaaccctagcggcggagtgaccgcgtacagccagacgctgcaggg acgcgtgactatgacctcggatacctcctcctccaccgtctatatggaactgtccagcctgcggtccgag gataccgccatgtactactgcgcccgggaaggatcaggctccgggtggtatttcgacttctggggaag aggcaccctcgtgactgtgtcatctgggggagggggttccggtggtggcggatcgggaggaggcggt tcatcctacgtgctgacccagccaccctccgtgtccgtgagccccggccagactgcatcgattacatgt agcggcgacggcctctccaagaaatacgtgtcgtggtaccagcagaaggccggacagagcccggt ggtgctgatctcaagagataaggagcggcctagcggaatcccggacaggttctcgggttccaactcc gcggacactgctactctgaccatctcggggacccaggctatggacgaagccgattactactgccaag cctgggacgacactactgtcgtgtttggagggggcaccaagttgaccgtcctt |
| **149366-aa VH** | 460 | QVQLVQSGAEVKKPGASVKVSCKPSGYTVTSHYIHWVRRAPGQGLEW MGMINPSGGVTAYSQTLQGRVTMTSDTSSSTVYMELSSLRSEDTAMYY CAREGSGSGWYFDFWGRGTLVTVSS |
| **149366-aa VL** | 461 | SYVLTQPPSVSVSPGQTASITCSGDGLSKKYVSWYQQKAGQSPVVLISR DKERPSGIPDRFSGSNSADTATLTISGTQAMDEADYYCQAWDDTTVVFG GGTKLTVL |

| **149367** | | |
|---|---|---|
| **149367-aa ScFv domain** | 462 | QVQLQESGPGLVKPSQTLSLTCTVSGGSISSGGYYWSWIRQHPGKGLE WIGYIYYSGSTYYNPSLKSRVTISVDTSKNQFSLKLSSVTAADTAVYYCA RAGIAARLRGAFDIWGQGTMVTVSSGGGGSGGGGSGGGGSDIVMTQS PSSVSASVGDRVIITCRASQGIRNWLAWYQQKPGKAPNLLIYAASNLQS GVPSRFSGSGSGADFTLTISSLQPEDVATYYCQKYNSAPFTFGPGTKVDI K |
| **149367-nt ScFv domain** | 463 | caagtgcagcttcaggagagcggcccgggactcgtgaagccgtcccagaccctgtccctgacttgca ccgtgtcgggaggaagcatctcgagcggaggctactattggtcgtggattcggcagcaccctggaaa gggcctggaatggatcggctacatctactactccggctcgacctactacaacccatcgctgaagtcca gagtgacaatctcagtggacacgtccaagaatcagttcagcctgaagctctcttccgtgactgcggccg acaccgccgtgtactactgcgcacgcgctggaattgccgcccggctgaggggtgccttcgacatttgg ggacagggcaccatggtcaccgtgtcctccggcggcggaggttccgggggtggaggctcaggagg aggggggtccgacatcgtcatgactcagtcgccctcaagcgtcagcgcgtccgtcggggacagagtg atcatcacctgtcgggcgtcccagggaattcgcaactggctggcctggtatcagcagaagcccggaa aggcccccaacctgttgatctacgccgcctcaaacctccaatccggggtgccgagccgcttcagcgg ctccggttcgggtgccgatttcactctgaccatctcctccctgcaacctgaagatgtggctacctactactg ccaaaagtacaactccgcaccttttactttcggaccggggaccaaagtggacattaag |
| **149367-aa VH** | 464 | QVQLQESGPGLVKPSQTLSLTCTVSGGSISSGGYYWSWIRQHPGKGLE WIGYIYYSGSTYYNPSLKSRVTISVDTSKNQFSLKLSSVTAADTAVYYCA RAGIAARLRGAFDIWGQGTMVTVSS |
| **149367-aa VL** | 465 | DIVMTQSPSSVSASVGDRVIITCRASQGIRNWLAWYQQKPGKAPNLLIYA ASNLQSGVPSRFSGSGSGADFTLTISSLQPEDVATYYCQKYNSAPFTFG PGTKVDIK |

| **149368** | | |
|---|---|---|
| **149368-aa ScFv domain** | 466 | qvqlvqsgaevkkpgssvkvsckasggtfssyaiswvrqapgqglewmggiipifgtanyaqkfqgr vtitadeststaymelsslrsedtavyycarrggyqllrwdvgllrsafdiwgqgtmvtvssggggsggg gsggggssyvltqppsvsvapgqtaritcggnnigsksvhwyqqkpgqapvlvlygknnrpsgvpdr fsgsrsgttasltitgaqaedeadyycssrdssgdhlrvfgtgtkvtvl |
| **149368-nt ScFv domain** | 467 | caagtgcagctggtccagtcgggcgccgaggtcaagaagcccgggagctctgtgaaagtgtcctgc aaggcctccgggggcacctttagctcctacgccatctcctgggtccgccaagcaccgggtcaaggcct ggagtggatggggggaattatccctatcttcggcactgccaactacgcccagaagttccagggacgc gtgaccattaccgcggacgaatccacctccaccgcttatatggagctgtccagcttgcgctcggaagat accgccgtgtactactgcgcccggaggggtggataccagctgctgagatgggacgtgggcctcctgc ggtcggcgttcgacatctggggccagggcactatggtcactgtgtccagcggaggaggcggatcggg aggcggcggatcagggggaggcggttccagctacgtgcttactcaacccccttcggtgtccgtggccc cgggacagaccgccagaatcacttgcggaggaaacaacattgggtccaagagcgtgcattggtacc agcagaagccaggacaggcccctgtgctggtgctctacgggaagaacaatcggcccagcggagtg ccggacaggttctcgggttcacgctccggtacaaccgcttcactgactatcaccggggcccaggcag aggatgaagcggactactactgttcctcccgggattcatccggcgaccacctccgggtgttcggaacc ggaacgaaggtcaccgtgctg |
| **149368-aa VH** | 468 | QVQLVQSGAEVKKPGSSVKVSCKASGGTFSSYAISWVRQAPGQGLEW MGGIIPIFGTANYAQKFQGRVTITADESTSTAYMELSSLRSEDTAVYYCA RRGGYQLLRWDVGLLRSAFDIWGQGTMVTVSS |
| **149368-aa VL** | 469 | SYVLTQPPSVSVAPGQTARITCGGNNIGSKSVHWYQQKPGQAPVLVLY GKNNRPSGVPDRFSGSRSGTTASLTITGAQAEDEADYYCSSRDSSGDH LRVFGTGTKVTVL |

| **149369** | | |
|---|---|---|
| **149369-aa ScFv domain** | 470 | evqlqqsgpglvkpsqtlsltcaisgdsvssnsaawnwirqspsrglewlgrtyyrskwysfyaislksrii inpdtsknqfslqlksvtpedtavyycarsspeglflywfdpwgqgtlvtvssggdgsggggsggggss seltqdpavsvalgqtiritcqgdslgnyyatwyqqkpgqapvlviygtnnrpsgipdrfsasssgntasl titgaqaedeadyycnsrdssghhllfgtgtkvtvl |
| **149369-nt ScFv domain** | 471 | gaagtgcagctccaacagtcaggaccggggctcgtgaagccatcccagaccctgtccctgacttgtg ccatctcgggagatagcgtgtcatcgaactccgccgcctggaactggattcggcagagcccgtcccg cggactggagtggcttggaaggacctactaccggtccaagtggtactctttctacgcgatctcgctgaa gtcccgcattatcattaaccctgatacctccaagaatcagttctccctccaactgaaatccgtcaccccc gaggacacagcagtgtattactgcgcacggagcagccccgaaggactgttcctgtattggtttgaccc ctggggccaggggactcttgtgaccgtgtcgagcggcggagatgggtccggtggcggtggttcgggg ggcggcggatcatcatccgaactgacccaggacccggctgtgtccgtggcgctgggacaaaccatc cgcattacgtgccagggagactccctgggcaactactacgccacttggtaccagcagaagccgggc caagcccctgtgttggtcatctacgggaccaacaacagaccttccggcatccccgaccggttcagcgc ttcgtcctccggcaacactgccagcctgaccatcactggagcgcaggccgaagatgaggccgacta ctactgcaacagcagagactcctcgggtcatcacctcttgttcggaactggaaccaaggtcaccgtgct g |
| **149369-aa VH** | 472 | EVQLQQSGPGLVKPSQTLSLTCAISGDSVSSNSAAWNWIRQSPSRGLE WLGRTYYRSKWYSFYAISLKSRIIINPDTSKNQFSLQLKSVTPEDTAVYYC ARSSPEGLFLYWFDPWGQGTLVTVSS |
| **149369-aa VL** | 473 | SSELTQDPAVSVALGQTIRITCQGDSLGNYYATWYQQKPGQAPVLVIYG TNNRPSGIPDRFSASSSGNTASLTITGAQAEDEADYYCNSRDSSGHHLL FGTGTKVTVL |

| **BCMA_EBB-C1 978-A4** | | |
|---|---|---|
| **BCMA_EBB-C1978-A4 - aa ScFv domain** | 474 | EVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKVEGSGSLDYWGQGTLVTVSSGGGGSGGGGSGGGGSEIVMTQSPGT LSLSPGERATLSCRASQSVSSAYLAWYQQKPGQPPRLLISGASTRATGI PDRFGGSGSGTDFTLTISRLEPEDFAVYYCQHYGSSFNGSSLFTFGQGT RLEIK |
| **BCMA_EBB-C1978-A4 - nt** | 475 | GAAGTGCAGCTCGTGGAGTCAGGAGGCGGCCTGGTCCAGCCGGGA GGGTCCCTTAGACTGTCATGCGCCGCAAGCGGATTCACTTTCTCCTC |
| **ScFv domain** | | CTATGCCATGAGCTGGGTCCGCCAAGCCCCCGGAAAGGGACTGGAA TGGGTGTCCGCCATCTCGGGGTCTGGAGGCTCAACTTACTACGCTGA CTCCGTGAAGGGACGGTTCACCATTAGCCGCGACAACTCCAAGAACA CCCTCTACCTCCAAATGAACTCCCTGCGGGCCGAGGATACCGCCGTC TACTACTGCGCCAAAGTGGAAGGTTCAGGATCGCTGGACTACTGGGG ACAGGGTACTCTCGTGACCGTGTCATCGGGCGGAGGAGGTTCCGGC GGTGGCGGCTCCGGCGGCGGAGGGTCGGAGATCGTGATGACCCAG AGCCCTGGTACTCTGAGCCTTTCGCCGGGAGAAAGGGCCACCCTGT CCTGCCGCGCTTCCCAATCCGTGTCCTCCGCGTACTTGGCGTGGTAC CAGCAGAAGCCGGGACAGCCCCCTCGGCTGCTGATCAGCGGGGCC AGCACCCGGGCAACCGGAATCCCAGACAGATTCGGGGGTTCCGGCA GCGGCACAGATTTCACCCTGACTATTTCGAGGTTGGAGCCCGAGGAC TTTGCGGTGTATTACTGTCAGCACTACGGGTCGTCCTTTAATGGCTCC AGCCTGTTCACGTTCGGACAGGGGACCCGCCTGGAAATCAAG |
| **BCMA_EBB-C1978-A4 - aa VH** | 476 | EVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKVEGSGSLDYWGQGTLVTVSS |
| **BCMA_EBB-C1978-A4 - aa VL** | 477 | EIVMTQSPGTLSLSPGERATLSCRASQSVSSAYLAWYQQKPGQPPRLLI SGASTRATGIPDRFGGSGSGTDFTLTISRLEPEDFAVYYCQHYGSSFNG SSLFTFGQGTRLEIK |

| **BCMA_EBB-C1** 978-G1 | | |
|---|---|---|
| **BCMA_EBB-C1978-G1 - aa ScFv domain** | 478 | EVQLVETGGGLVQPGGSLRLSCAASGITFSRYPMSWVRQAPGKGLEWV SGISDSGVSTYYADSAKGRFTISRDNSKNTLFLQMSSLRDEDTAVYYCVT RAGSEASDIWGQGTMVTVSSGGGGSGGGGSGGGGSEIVLTQSPATLSL SPGERATLSCRASQSVSNSLAWYQQKPGQAPRLLIYDASSRATGIPDRF SGSGSGTDFTLTISRLEPEDFAIYYCQQFGTSSGLTFGGGTKLEIK |
| **BCMA_EBB-C1978-G1 - nt ScFv domain** | 479 | GAAGTGCAACTGGTGGAAACCGGTGGCGGCCTGGTGCAGCCTGGAG GATCATTGAGGCTGTCATGCGCGGCCAGCGGTATTACCTTCTCCCGG TACCCCATGTCCTGGGTCAGACAGGCCCCGGGGAAAGGGCTTGAAT GGGTGTCCGGGATCTCGGACTCCGGTGTCAGCACTTACTACGCCGA CTCCGCCAAGGGACGCTTCACCATTTCCCGGGACAACTCGAAGAACA CCCTGTTCCTCCAAATGAGCTCCCTCCGGGACGAGGATACTGCAGTG TACTACTGCGTGACCCGCGCCGGGTCCGAGGCGTCTGACATTTGGG GACAGGGCACTATGGTCACCGTGTCGTCCGGCGGAGGGGGCTCGG GAGGCGGTGGCAGCGGAGGAGGAGGGTCCGAGATCGTGCTGACCC AATCCCCGGCCACCCTCTCGCTGAGCCCTGGAGAAAGGGCAACCTT GTCCTGTCGCGCGAGCCAGTCCGTGAGCAACTCCCTGGCCTGGTAC CAGCAGAAGCCCGGACAGGCTCCGAGACTTCTGATCTACGACGCTTC GAGCCGGGCCACTGGAATCCCCGACCGCTTTTCGGGGTCCGGCTCA GGAACCGATTTCACCCTGACAATCTCACGGCTGGAGCCAGAGGATTT CGCCATCTATTACTGCCAGCAGTTCGGTACTTCCTCCGGCCTGACTTT CGGAGGCGGCACGAAGCTCGAAATCAAG |
| **BCMA_EBB-C1978-G1 -aa VH** | 480 | EVQLVETGGGLVQPGGSLRLSCAASGITFSRYPMSWVRQAPGKGLEWV SGISDSGVSTYYADSAKGRFTISRDNSKNTLFLQMSSLRDEDTAVYYCVT RAGSEASDIWGQGTMVTVSS |
| **BCMA_EBB-C1978-G1 - aa VL** | 481 | EIVLTQSPATLSLSPGERATLSCRASQSVSNSLAWYQQKPGQAPRLLIYD ASSRATGIPDRFSGSGSGTDFTLTISRLEPEDFAIYYCQQFGTSSGLTFG GGTKLEIK |

| **BCMA EBB-C1 979-C1** | | |
|---|---|---|
| **BCMA_EBB-C1979-C1 - aa ScFv domain** | 482 | QVQLVESGGGLVQPGGSLRLSCAASGFTFSSYAMSWVRQAPGKGLEW VSAISGSGGSTYYADSVKGRFTISRDNAKNSLYLQMNSLRAEDTAIYYCA RATYKRELRYYYGMDVWGQGTMVTVSSGGGGSGGGGSGGGGSEIVM TQSPGTVSLSPGERATLSCRASQSVSSSFLAWYQQKPGQAPRLLIYGAS SRATGIPDRFSGSGSGTDFTLTISRLEPEDSAVYYCQQYHSSPSWTFGQ |
| | | GTRLEIK |
| **BCMA_EBB-C1979-C1** - **nt ScFv domain** | 483 | CAAGTGCAGCTCGTGGAATCGGGTGGCGGACTGGTGCAGCCGGGG GGCTCACTTAGACTGTCCTGCGCGGCCAGCGGATTCACTTTCTCCTC CTACGCCATGTCCTGGGTCAGACAGGCCCCTGGAAAGGGCCTGGAA TGGGTGTCCGCAATCAGCGGCAGCGGCGGCTCGACCTATTACGCGG ATTCAGTGAAGGGCAGATTCACCATTTCCCGGGACAACGCCAAGAAC TCCTTGTACCTTCAAATGAACTCCCTCCGCGCGGAAGATACCGCAAT CTACTACTGCGCTCGGGCCACTTACAAGAGGGAACTGCGCTACTACT ACGGGATGGACGTCTGGGGCCAGGGAACCATGGTCACCGTGTCCAG CGGAGGAGGAGGATCGGGAGGAGGCGGTAGCGGGGGTGGAGGGT CGGAGATCGTGATGACCCAGTCCCCCGGCACTGTGTCGCTGTCCCC CGGCGAACGGGCCACCCTGTCATGTCGGGCCAGCCAGTCAGTGTCG TCAAGCTTCCTCGCCTGGTACCAGCAGAAACCGGGACAAGCTCCCC GCCTGCTGATCTACGGAGCCAGCAGCCGGGCCACCGGTATTCCTGA CCGGTTCTCCGGTTCGGGGTCCGGGACCGACTTTACTCTGACTATCT CTCGCCTCGAGCCAGAGGACTCCGCCGTGTATTACTGCCAGCAGTAC CACTCCTCCCCGTCCTGGACGTTCGGACAGGGCACAAGGCTGGAGA TTAAG |
| **BCMA_EBB-C1979-C1** - aa **VH** | 484 | QVQLVESGGGLVQPGGSLRLSCAASGFTFSSYAMSWVRQAPGKGLEW VSAISGSGGSTYYADSVKGRFTISRDNAKNSLYLQMNSLRAEDTAIYYCA RATYKRELRYYYGMDVWGQGTMVTVSS |
| **BCMA_EBB-C1979-C1** - aa **VL** | 485 | EIVMTQSPGTVSLSPGERATLSCRASQSVSSSFLAWYQQKPGQAPRLLI YGASSRATGIPDRFSGSGSGTDFTLTISRLEPEDSAVYYCQQYHSSPSW TFGQGTRLEIK |

| **BCMA EBB-C1978-C7** | | |
|---|---|---|
| **BCMA_EBB-C1978-C7** - **aa ScFv domain** | 486 | EVQLVETGGGLVQPGGSLRLSCAASGFTFSSYAMSWVRQAPGKGLEW VSAISGSGGSTYYADSVKGRFTISRDNSKNTLYLQMNTLKAEDTAVYYCA RATYKRELRYYYGMDVWGQGTTVTVSSGGGGSGGGGSGGGGSEIVLT QSPSTLSLSPGESATLSCRASQSVSTTFLAWYQQKPGQAPRLLIYGSSN RATGIPDRFSGSGSGTDFTLTIRRLEPEDFAVYYCQQYHSSPSWTFGQG TKVEIK |
| **BCMA_EBB-C1978-C7** - **nt ScFv domain** | 487 | GAGGTGCAGCTTGTGGAAACCGGTGGCGGACTGGTGCAGCCCGGA GGAAGCCTCAGGCTGTCCTGCGCCGCGTCCGGCTTCACCTTCTCCT CGTACGCCATGTCCTGGGTCCGCCAGGCCCCCGGAAAGGGCCTGGA ATGGGTGTCCGCCATCTCTGGAAGCGGAGGTTCCACGTACTACGCG GACAGCGTCAAGGGAAGGTTCACAATCTCCCGCGATAATTCGAAGAA CACTCTGTACCTTCAAATGAACACCCTGAAGGCCGAGGACACTGCTG TGTACTACTGCGCACGGGCCACCTACAAGAGAGAGCTCCGGTACTAC TACGGAATGGACGTCTGGGGCCAGGGAACTACTGTGACCGTGTCCT CGGGAGGGGGTGGCTCCGGGGGGGGCGGCTCCGGCGGAGGCGGT TCCGAGATTGTGCTGACCCAGTCACCTTCAACTCTGTCGCTGTCCCC GGGAGAGAGCGCTACTCTGAGCTGCCGGGCCAGCCAGTCCGTGTCC ACCACCTTCCTCGCCTGGTATCAGCAGAAGCCGGGGCAGGCACCAC GGCTCTTGATCTACGGGTCAAGCAACAGAGCGACCGGAATTCCTGAC CGCTTCTCGGGGAGCGGTTCAGGCACCGACTTCACCCTGACTATCC GGCGCCTGGAACCCGAAGATTTCGCCGTGTATTACTGTCAACAGTAC CACTCCTCGCCGTCCTGGACCTTTGGCCAAGGAACCAAAGTGGAAAT CAAG |
| **BCMA_EBB-C1978-C7** - **aa VH** | 488 | EVQLVETGGGLVQPGGSLRLSCAASGFTFSSYAMSWVRQAPGKGLEW VSAISGSGGSTYYADSVKGRFTISRDNSKNTLYLQMNTLKAEDTAVYYCA RATYKRELRYYYGMDVWGQGTTVTVSS |
| **BCMA_EBB-C1978-C7** - **aa VL** | 489 | EIVLTQSPSTLSLSPGESATLSCRASQSVSTTFLAWYQQKPGQAPRLLIY GSSNRATGIPDRFSGSGSGTDFTLTIRRLEPEDFAVYYCQQYHSSPSWT FGQGTKVEIK |

| **BCMA_EBB-C1978-D10** | | |
|---|---|---|
| **BCMA_EBB-C1978-D10** - **aa ScFv domain** | 490 | EVQLVETGGGLVQPGRSLRLSCAASGFTFDDYAMHWVRQAPGKGLEW VSGISWNSGSIGYADSVKGRFTISRDNAKNSLYLQMNSLRDEDTAVYYC ARVGKAVPDVWGQGTTVTVSSGGGGSGGGGSGGGGSDIVMTQTPSSL SASVGDRVTITCRASQSISSYLNWYQQKPGKAPKLLIYAASSLQSGVPSR FSGSGSGTDFTLTISSLQPEDFATYYCQQSYSTPYSFGQGTRLEIK |
| **BCMA_EBB-C1978-D10-nt ScFv domain** | 491 | TGGGLVQPGRSLRLSCAASGFTFDDYAMHWVRQAPGKGLEW NSGSIGYADSVKGRFTISRDNAKNSLYLQMNSLRDEDTAVYYC VPDVWGQGTTVTVSSGGGGSGGGGSGGGGSDIVMTQTPSSL RVTITCRASQSISSYLNWYQQKPGKAPKLLIYAASSLQSGVPSR GTDFTLTISSLQPEDFATYYCQQSYSTPYSFGQGTRLEIK CAGCTCGTGGAAACTGGAGGTGGACTCGTGCAGCCTGGAC TGCGGCTGAGCTGCGCTGCATCCGGCTTCACCTTCGACGA CATGCACTGGGTCAGACAGGCGCCAGGGAAGGGACTTGAG TCCGGTATCAGCTGGAATAGCGGCTCAATCGGATACGCGG TGAAGGGAAGGTTCACCATTTCCCGCGACAACGCCAAGAAC TACTTGCAAATGAACAGCCTCCGGGATGAGGACACTGCCGT CTGCGCCCGCGTCGGAAAAGCTGTGCCCGACGTCTGGGGC ACCACTGTGACCGTGTCCAGCGGCGGGGGTGGATCGGGC AGGGTCCGGTGGAGGGGGCTCAGATATTGTGATGACCCAGA TCGTCCCTGTCCGCCTCGGTCGGCGACCGCGTGACTATCAC AGCCTCGCAGAGCATCTCCAGCTACCTGAACTGGTATCAGC CCGGGGAAGGCCCCGAAGCTCCTGATCTACGCGGCATCATC ATCGGGAGTGCCGAGCCGGTTTTCCGGGTCCGGCTCCGGC CTTCACGCTGACCATTTCTTCCCTGCAACCCGAGGACTTCGC [?] |
| **BCMA_EBB-C1978-D10** - aa **VH** | 492 | EVQLVETGGGLVQPGRSLRLSCAASGFTFDDYAMHWVRQAPGKGLEW VSGISWNSGSIGYADSVKGRFTISRDNAKNSLYLQMNSLRDEDTAVYYC ARVGKAVPDVWGQGTTVTVSS |
| **BCMA_EBB-C1978-D10-**aa **VL** | 493 | DIVMTQTPSSLSASVGDRVTITCRASQSISSYLNWYQQKPGKAPKLLIYA ASSLQSGVPSRFSGSGSGTDFTLTISSLQPEDFATYYCQQSYSTPYSFG QGTRLEIK |

| **BCMA_EBB-C1979-C12** | | |
|---|---|---|
| **BCMA_EBB-C1979-C12-**aa **ScFv domain** | 494 | EVQLVESGGGLVQPGRSLRLSCTASGFTFDDYAMHWVRQRPGKGLEW VASINWKGNSLAYGDSVKGRFAISRDNAKNTVFLQMNSLRTEDTAVYYC ASHQGVAYYNYAMDVWGRGTLVTVSSGGGGSGGGGSGGGGSEIVLTQ SPGTLSLSPGERATLSCRATQSIGSSFLAWYQQRPGQAPRLLIYGASQR ATGIPDRFSGRGSGTDFTLTISRVEPEDSAVYYCQHYESSPSWTFGQGT KVEIK |
| **BCMA_EBB-C1979-C12** - **nt ScFv domain** | 495 | GAAGTGCAGCTCGTGGAGAGCGGGGGAGGATTGGTGCAGCCCGGA AGGTCCCTGCGGCTCTCCTGCACTGCGTCTGGCTTCACCTTCGACGA CTACGCGATGCACTGGGTCAGACAGCGCCCGGGAAAGGGCCTGGAA TGGGTCGCCTCAATCAACTGGAAGGGAAACTCCCTGGCCTATGGCGA CAGCGTGAAGGGCCGCTTCGCCATTTCGCGCGACAACGCCAAGAAC ACCGTGTTTCTGCAAATGAATTCCCTGCGGACCGAGGATACCGCTGT GTACTACTGCGCCAGCCACCAGGGCGTGGCATACTATAACTACGCCA TGGACGTGTGGGGAAGAGGGACGCTCGTCACCGTGTCCTCCGGGG GCGGTGGATCGGGTGGAGGAGGAAGCGGTGGCGGGGGCAGCGAAA TCGTGCTGACTCAGAGCCCGGGAACTCTTTCACTGTCCCCGGGAGAA CGGGCCACTCTCTCGTGCCGGGCCACCCAGTCCATCGGCTCCTCCT TCCTTGCCTGGTACCAGCAGAGGCCAGGACAGGCGCCCCGCCTGCT GATCTACGGTGCTTCCCAACGCGCCACTGGCATTCCTGACCGGTTCA GCGGCAGAGGGTCGGGAACCGATTTCACACTGACCATTTCCCGGGT GGAGCCCGAAGATTCGGCAGTCTACTACTGTCAGCATTACGAGTCCT CCCCTTCATGGACCTTCGGTCAAGGGACCAAAGTGGAGATCAAG |
| **BCMA_EBB-C1979-C12** - aa | 496 | EVQLVESGGGLVQPGRSLRLSCAASGFTFDDYAMHWVRQAPGKGLEW VASINWKGNSLAYGDSVKGRFAISRDNAKNTVFLQMNSLRTEDTAVYYC ASHQGVAYYNYAMDVWGRGTLVTVSS |
| **VH** | | |
| **BCMA_EBB-C1979-C12** - **aa VL** | 497 | EIVLTQSPGTLSLSPGERATLSCRATQSIGSSFLAWYQQRPGQAPRLLIY GASQRATGIPDRFSGRGSGTDFTLTISRVEPEDSAVYYCQHYESSPSWT FGQGTKVEIK |

| **BCMA EBB-C1980-G4** | | |
|---|---|---|
| **BCMA_EBB-C1980-G4-** aa **ScFv domain** | 498 | EVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKVVRDGMDVWGQGTTVTVSSGGGGSGGGGSGGGGSEIVLTQSPATL SLSPGERATLSCRASQSVSSSYLAWYQQKPGQAPRLLIYGASSRATGIP DRFSGNGSGTDFTLTISRLEPEDFAVYYCQQYGSPPRFTFGPGTKVDIK |
| **BCMA_EBB-C1980-G4- nt ScFv domain** | 499 | GAGGTGCAGTTGGTCGAAAGCGGGGGCGGGCTTGTGCAGCCTGGC GGATCACTGCGGCTGTCCTGCGCGGCATCAGGCTTCACGTTTTCTTC CTACGCCATGTCCTGGGTGCGCCAGGCCCCTGGAAAGGGACTGGAA TGGGTGTCCGCGATTTCGGGGTCCGGCGGGAGCACCTACTACGCCG ATTCCGTGAAGGGCCGCTTCACTATCTCGCGGGACAACTCCAAGAAC ACCCTCTACCTCCAAATGAATAGCCTGCGGGCCGAGGATACCGCCGT CTACTATTGCGCTAAGGTCGTGCGCGACGGAATGGACGTGTGGGGA CAGGGTACCACCGTGACAGTGTCCTCGGGGGGAGGCGGTAGCGGC GGAGGAGGAAGCGGTGGTGGAGGTTCCGAGATTGTGCTGACTCAAT CACCCGCGACCCTGAGCCTGTCCCCCGGCGAAAGGGCCACTCTGTC CTGTCGGGCCAGCCAATCAGTCTCCTCCTCGTACCTGGCCTGGTACC AGCAGAAGCCAGGACAGGCTCCGAGACTCCTTATCTATGGCGCATCC TCCCGCGCCACCGGAATCCCGGATAGGTTCTCGGGAAACGGATCGG GGACCGACTTCACTCTCACCATCTCCCGGCTGGAACCGGAGGACTTC GCCGTGTACTACTGCCAGCAGTACGGCAGCCCGCCTAGATTCACTTT CGGCCCCGGCACCAAAGTGGACATCAAG |
| **BCMA_EBB-C1980-G4-** aa **VH** | 500 | EVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKVVRDGMDVWGQGTTVTVSS |
| **BCMA_EBB-C1980-G4-** aa **VL** | 501 | EIVLTQSPATLSLSPGERATLSCRASQSVSSSYLAWYQQKPGQAPRLLIY GASSRATGIPDRFSGNGSGTDFTLTISRLEPEDFAVYYCQQYGSPPRFT FGPGTKVDIK |

| **BCMA EBB-C1980-D2** | | |
|---|---|---|
| **BCMA_EBB-C1980-D2-** aa **ScFv domain** | 502 | EVQLLESGGGLVQPGGSLRLSCAASGFTFSSYAMSWVRQAPGKGLEW VSAISGSGGSTYYADSVKGRFTISRDNSKNTLYLQMNSLRAEDTAVYYC AKIPQTGTFDYWGQGTLVTVSSGGGGSGGGGSGGGGSEIVLTQSPGTL SLSPGERATLSCRASQSVSSSYLAWYQQRPGQAPRLLIYGASSRATGIP DRFSGSGSGTDFTLTISRLEPEDFAVYYCQHYGSSPSWTFGQGTRLEIK |
| **BCMA_EBB-C1980-D2- nt ScFv domain** | 503 | GAAGTGCAGCTGCTGGAGTCCGGCGGTGGATTGGTGCAACCGGGG GGATCGCTCAGACTGTCCTGTGCGGCGTCAGGCTTCACCTTCTCGAG CTACGCCATGTCATGGGTCAGACAGGCCCCTGGAAAGGGTCTGGAA TGGGTGTCCGCCATTTCCGGGAGCGGGGGATCTACATACTACGCCG ATAGCGTGAAGGGCCGCTTCACCATTTCCCGGGACAACTCCAAGAAC ACTCTCTATCTGCAAATGAACTCCCTCCGCGCTGAGGACACTGCCGT GTACTACTGCGCCAAAATCCCTCAGACCGGCACCTTCGACTACTGGG GACAGGGGACTCTGGTCACCGTCAGCAGCGGTGGCGGAGGTTCGG GGGGAGGAGGAAGCGGCGGCGGAGGGTCCGAGATTGTGCTGACCC AGTCACCCGGCACTTTGTCCCTGTCGCCTGGAGAAAGGGCCACCCTT TCCTGCCGGGCATCCCAATCCGTGTCCTCCTCGTACCTGGCCTGGTA CCAGCAGAGGCCCGGACAGGCCCCACGGCTTCTGATCTACGGAGCA AGCAGCCGCGCGACCGGTATCCCGGACCGGTTTTCGGGCTCGGGCT CAGGAACTGACTTCACCCTCACCATCTCCCGCCTGGAACCCGAAGAT TTCGCTGTGTATTACTGCCAGCACTACGGCAGCTCCCCGTCCTGGAC GTTCGGCCAGGGAACTCGGCTGGAGATCAAG |
| **BCMA_EBB-C1980-D2- aa VH** | 504 | EVQLLESGGGLVQPGGSLRLSCAASGFTFSSYAMSWVRQAPGKGLEW VSAISGSGGSTYYADSVKGRFTISRDNSKNTLYLQMNSLRAEDTAVYYC AKIPQTGTFDYWGQGTLVTVSS |
| **BCMA_EBB-C1980-D2- aa VL** | 505 | EIVLTQSPGTLSLSPGERATLSCRASQSVSSSYLAWYQQRPGQAPRLLIY GASSRATGIPDRFSGSGSGTDFTLTISRLEPEDFAVYYCQHYGSSPSWT FGQGTRLEIK |

| **BCMA EBB-C1978-A10** | | |
|---|---|---|
| **BCMA_EBB-C1978-A10-aa ScFv domain** | 506 | EVQLVETGGGLVQPGGSLRLSCAASGFTFSSYAMSWVRQAPGKGLEW VSAISGSGGSTYYADSVKGRFTMSRENDKNSVFLQMNSLRVEDTGVYY CARANYKRELRYYYGMDVWGQGTMVTVSSGGGGSGGGGSGGGGSEI VMTQSPGTLSLSPGESATLSCRASQRVASNYLAWYQHKPGQAPSLLISG ASSRATGVPDRFSGSGSGTDFTLAISRLEPEDSAVYYCQHYDSSPSWTF GQGTKVEIK |
| **BCMA_EBB-C1978-A 10-nt ScFv domain** | 507 | GAAGTGCAACTGGTGGAAACCGGTGGAGGACTCGTGCAGCCTGGCG GCAGCCTCCGGCTGAGCTGCGCCGCTTCGGGATTCACCTTTTCCTCC TACGCGATGTCTTGGGTCAGACAGGCCCCCGGAAAGGGGCTGGAAT GGGTGTCAGCCATCTCCGGCTCCGGCGGATCAACGTACTACGCCGA CTCCGTGAAAGGCCGGTTCACCATGTCGCGCGAGAATGACAAGAACT CCGTGTTCCTGCAAATGAACTCCCTGAGGGTGGAGGACACCGGAGT GTACTATTGTGCGCGCGCCAACTACAAGAGAGAGCTGCGGTACTACT ACGGAATGGACGTCTGGGGACAGGGAACTATGGTGACCGTGTCATC CGGTGGAGGGGGAAGCGGCGGTGGAGGCAGCGGGGGCGGGGGTT CAGAAATTGTCATGACCCAGTCCCCGGGAACTCTTTCCCTCTCCCCC GGGGAATCCGCGACTTTGTCCTGCCGGGCCAGCCAGCGCGTGGCCT CGAACTACCTCGCATGGTACCAGCATAAGCCAGGCCAAGCCCCTTCC CTGCTGATTTCCGGGGCTAGCAGCCGCGCCACTGGCGTGCCGGATA GGTTCTCGGGAAGCGGCTCGGGTACCGATTTCACCCTGGCAATCTC GCGGCTGGAACCGGAGGATTCGGCCGTGTACTACTGCCAGCACTAT GACTCATCCCCCTCCTGGACATTCGGACAGGGCACCAAGGTCGAGAT CAAG |
| **BCMA_EBB-C1978-A10-aa VH** | 508 | EVQLVETGGGLVQPGGSLRLSCAASGFTFSSYAMSWVRQAPGKGLEW VSAISGSGGSTYYADSVKGRFTMSRENDKNSVFLQMNSLRVEDTGVYY CARANYKRELRYYYGMDVWGQGTMVTVSS |
| **BCMA_EBB-C1978-A10-aa VL** | 509 | EIVMTQSPGTLSLSPGESATLSCRASQRVASNYLAWYQHKPGQAPSLLI SGASSRATGVPDRFSGSGSGTDFTLAISRLEPEDSAVYYCQHYDSSPS WTFGQGTKVEIK |

| **BCMA EBB-C1978-D4** | | |
|---|---|---|
| **BCMA_EBB-C1978-D4- aa ScFv domain** | 510 | EVQLLETGGGLVQPGGSLRLSCAASGFSFSSYAMSWVRQAPGKGLEW VSAISGSGGSTYYADSVKGRFTISRDNSKNTLYLQMNSLRAEDTAVYYC AKALVGATGAFDIWGQGTLVTVSSGGGGSGGGGSGGGGSEIVLTQSPG TLSLSPGERATLSCRASQSLSSNFLAWYQQKPGQAPGLLIYGASNWAT GTPDRFSGSGSGTDFTLTITRLEPEDFAVYYCQYYGTSPMYTFGQGTKV EIK |
| **BCMA_EBB-C1978-D4- nt ScFv domain** | 511 | GAAGTGCAGCTGCTCGAAACCGGTGGAGGGCTGGTGCAGCCAGGG GGCTCCCTGAGGCTTTCATGCGCCGCTAGCGGATTCTCCTTCTCCTC TTACGCCATGTCGTGGGTCCGCCAAGCCCCTGGAAAAGGCCTGGAA TGGGTGTCCGCGATTTCCGGGAGCGGAGGTTCGACCTATTACGCCG ACTCCGTGAAGGGCCGCTTTACCATCTCCCGGGATAACTCCAAGAAC ACTCTGTACCTCCAAATGAACTCGCTGAGAGCCGAGGACACCGCCGT GTATTACTGCGCGAAGGCGCTGGTCGGCGCGACTGGGGCATTCGAC ATCTGGGGACAGGGAACTCTTGTGACCGTGTCGAGCGGAGGCGGCG GCTCCGGCGGAGGAGGGAGCGGGGGCGGTGGTTCCGAAATCGTGT TGACTCAGTCCCCGGGAACCCTGAGCTTGTCACCCGGGGAGCGGGC |
| | | CACTCTCTCCTGTCGCGCCTCCCAATCGCTCTCATCCAATTTCCTGGC CTGGTACCAGCAGAAGCCCGGACAGGCCCCGGGCCTGCTCATCTAC GGCGCTTCAAACTGGGCAACGGGAACCCCTGATCGGTTCAGCGGAA GCGGATCGGGTACTGACTTTACCCTGACCATCACCAGACTGGAACCG GAGGACTTCGCCGTGTACTACTGCCAGTACTACGGCACCTCCCCCAT GTACACATTCGGACAGGGTACCAAGGTCGAGATTAAG |
| **BCMA_EBB-C1978-D4- aa VH** | 512 | EVQLLETGGGLVQPGGSLRLSCAASGFSFSSYAMSWVRQAPGKGLEW VSAISGSGGSTYYADSVKGRFTISRDNSKNTLYLQMNSLRAEDTAVYYC AKALVGATGAFDIWGQGTLVTVSS |
| **BCMA_EBB-C1978-D4- aa VL** | 513 | EIVLTQSPGTLSLSPGERATLSCRASQSLSSNFLAWYQQKPGQAPGLLIY GASNWATGTPDRFSGSGSGTDFTLTITRLEPEDFAVYYCQYYGTSPMYT FGQGTKVEIK |

| **BCMA EBB-C1980-A2** | | |
|---|---|---|
| **BCMA_EBB-C1980-A2- aa ScFv domain** | 514 | EVQLLESGGGLVQPGGSLRLSCAASGFTFSSYAMSWVRQAPGKGLEW VSAISGSGGSTYYADSVKGRFTISRDNSKNTLYLQMNSLRAEDTAVYYC VLWFGEGFDPWGQGTLVTVSSGGGGSGGGGSGGGGSDIVLTQSPLSL PVTPGEPASISCRSSQSLLHSNGYNYLDWYLQKPGQSPQLLIYLGSNRA SGVPDRFSGSGSGTDFTLKISRVEAEDVGVYYCMQALQTPLTFGGGTKV DIK |
| **BCMA_EBB-C1980-A2- nt ScFv domain** | 515 | GAAGTGCAGCTGCTTGAGAGCGGTGGAGGTCTGGTGCAGCCCGGG GGATCACTGCGCCTGTCCTGTGCCGCGTCCGGTTTCACTTTCTCCTC GTACGCCATGTCGTGGGTCAGACAGGCACCGGGAAAGGGACTGGAA TGGGTGTCAGCCATTTCGGGTTCGGGGGGCAGCACCTACTACGCTG ACTCCGTGAAGGGCCGGTTCACCATTTCCCGCGACAACTCCAAGAAC ACCTTGTACCTCCAAATGAACTCCCTGCGGGCCGAAGATACCGCCGT GTATTACTGCGTGCTGTGGTTCGGAGAGGGATTCGACCCGTGGGGA CAAGGAACACTCGTGACTGTGTCATCCGGCGGAGGCGGCAGCGGTG GCGGCGGTTCCGGCGGCGGCGGATCTGACATCGTGTTGACCCAGTC CCCTCTGAGCCTGCCGGTCACTCCTGGCGAACCAGCCAGCATCTCCT GCCGGTCGAGCCAGTCCCTCCTGCACTCCAATGGGTACAACTACCTC GATTGGTATCTGCAAAAGCCGGGCCAGAGCCCCCAGCTGCTGATCTA CCTTGGGTCAAACCGCGCTTCCGGGGTGCCTGATAGATTCTCCGGGT CCGGGAGCGGAACCGACTTTACCCTGAAAATCTCGAGGGTGGAGGC CGAGGACGTCGGAGTGTACTACTGCATGCAGGCGCTCCAGACTCCC CTGACCTTCGGAGGAGGAACGAAGGTCGACATCAAGA |
| **BCMA_EBB-C1980-A2-** aa **VH** | 516 | EVQLLESGGGLVQPGGSLRLSCAASGFTFSSYAMSWVRQAPGKGLEW VSAISGSGGSTYYADSVKGRFTISRDNSKNTLYLQMNSLRAEDTAVYYC VLWFGEGFDPWGQGTLVTVSS |
| **BCMA_EBB-C1980-A2-** aa **VL** | 517 | DIVLTQSPLSLPVTPGEPASISCRSSQSLLHSNGYNYLDWYLQKPGQSP QLLIYLGSNRASGVPDRFSGSGSGTDFTLKISRVEAEDVGVYYCMQALQ TPLTFGGGTKVDIK |

| **BCMA_EBB-C1981-C3** | | |
|---|---|---|
| **BCMA_EBB-C1981-C3- aa ScFv domain** | 518 | QVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKVGYDSSGYYRDYYGMDVWGQGTTVTVSSGGGGSGGGGSGGGGS EIVLTQSPGTLSLSPGERATLSCRASQSVSSSYLAWYQQKPGQAPRLLIY GTSSRATGISDRFSGSGSGTDFTLTISRLEPEDFAVYYCQHYGNSPPKFT FGPGTKLEIK |
| **BCMA_EBB-C1981-C3- nt ScFv domain** | 519 | CAAGTGCAGCTCGTGGAGTCAGGCGGAGGACTGGTGCAGCCCGGG GGCTCCCTGAGACTTTCCTGCGCGGCATCGGGTTTTACCTTCTCCTC CTATGCTATGTCCTGGGTGCGCCAGGCCCCGGGAAAGGGACTGGAA TGGGTGTCCGCAATCAGCGGTAGCGGGGGCTCAACATACTACGCCG ACTCCGTCAAGGGTCGCTTCACTATTTCCCGGGACAACTCCAAGAAT ACCCTGTACCTCCAAATGAACAGCCTCAGGGCCGAGGATACTGCCGT GTACTACTGCGCCAAAGTCGGATACGATAGCTCCGGTTACTACCGGG |
| | | ACTACTACGGAATGGACGTGTGGGGACAGGGCACCACCGTGACCGT GTCAAGCGGCGGAGGCGGTTCAGGAGGGGGAGGCTCCGGCGGTGG AGGGTCCGAAATCGTCCTGACTCAGTCGCCTGGCACTCTGTCGTTGT CCCCGGGGGAGCGCGCTACCCTGTCGTGTCGGGCGTCGCAGTCCG TGTCGAGCTCCTACCTCGCGTGGTACCAGCAGAAGCCCGGACAGGC CCCTAGACTTCTGATCTACGGCACTTCTTCACGCGCCACCGGGATCA GCGACAGGTTCAGCGGCTCCGGCTCCGGGACCGACTTCACCCTGAC CATTAGCCGGCTGGAGCCTGAAGATTTCGCCGTGTATTACTGCCAAC ACTACGGAAACTCGCCGCCAAAGTTCACGTTCGGACCCGGAACCAAG CTGGAAATCAAG |
| **BCMA_EBB-C1981-C3- aa VH** | 520 | QVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKVGYDSSGYYRDYYGMDVWGQGTTVTVSS |
| **BCMA_EBB-C1981-C3- aa VL** | 521 | EIVLTQSPGTLSLSPGERATLSCRASQSVSSSYLAWYQQKPGQAPRLLIY GTSSRATGISDRFSGSGSGTDFTLTISRLEPEDFAVYYCQHYGNSPPKFT FGPGTKLEIK |

| **BCMA EBB-C1978-G4** | | |
|---|---|---|
| **BCMA_EBB-C1978-G4- aa ScFv domain** | 522 | EVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKMGWSSGYLGAFDIWGQGTTVTVSSGGGGSGGGGSGGGGSEIVLTQ SPGTLSLSPGERATLSCRASQSVASSFLAWYQQKPGQAPRLLIYGASGR ATGIPDRFSGSGSGTDFTLTISRLEPEDFAVYYCQHYGGSPRLTFGGGT KVDIK |
| **BCMA_EBB-C1978-G4- nt ScFv domain** | 523 | GAAGTCCAACTGGTGGAGTCCGGGGGAGGGCTCGTGCAGCCCGGA GGCAGCCTTCGGCTGTCGTGCGCCGCCTCCGGGTTCACGTTCTCAT CCTACGCGATGTCGTGGGTCAGACAGGCACCAGGAAAGGGACTGGA ATGGGTGTCCGCCATTAGCGGCTCCGGCGGTAGCACCTACTATGCC GACTCAGTGAAGGGAAGGTTCACTATCTCCCGCGACAACAGCAAGAA CACCCTGTACCTCCAAATGAACTCTCTGCGGGCCGAGGATACCGCG GTGTACTATTGCGCCAAGATGGGTTGGTCCAGCGGATACTTGGGAGC CTTCGACATTTGGGGACAGGGCACTACTGTGACCGTGTCCTCCGGG GGTGGCGGATCGGGAGGCGGCGGCTCGGGTGGAGGGGGTTCCGAA ATCGTGTTGACCCAGTCACCGGGAACCCTCTCGCTGTCCCCGGGAG AACGGGCTACACTGTCATGTAGAGCGTCCCAGTCCGTGGCTTCCTCG TTCCTGGCCTGGTACCAGCAGAAGCCGGGACAGGCACCCCGCCTGC TCATCTACGGAGCCAGCGGCCGGGCGACCGGCATCCCTGACCGCTT CTCCGGTTCCGGCTCGGGCACCGACTTTACTCTGACCATTAGCAGGC TTGAGCCCGAGGATTTTGCCGTGTACTACTGCCAACACTACGGGGGG AGCCCTCGCCTGACCTTCGGAGGCGGAACTAAGGTCGATATCAAAA |
| **BCMA_EBB-C1978-G4- aa VH** | 524 | EVQLVESGGGLVQPGGSLRLSCAASGFTFSSYAMSWVRQAPGKGLEW VSAISGSGGSTYYADSVKGRFTISRDNSKNTLYLQMNSLRAEDTAVYYC AKMGWSSGYLGAFDIWGQGTTVTVSS |
| **BCMA_EBB-C1978-G4- aa VL** | 525 | EIVLTQSPGTLSLSPGERATLSCRASQSVASSFLAWYQQKPGQAPRLLIY GASGRATGIPDRFSGSGSGTDFTLTISRLEPEDFAVYYCQHYGGSPRLT FGGGTKVDIK |

In some embodiments, additional exemplary CAR constructs are generated using the VH and VL sequences from PCT Publication WO2012/0163805. In some embodiments, additional exemplary CAR constructs are generated using the VH and VL sequences from PCT Publication WO2016/014565. In some embodiments, additional exemplary CAR constructs are generated using the VH and VL sequences from PCT Publication WO2014/122144.

In some embodiments, additional exemplary CAR constructs are generated using the CAR molecules, and/or the VH and VL sequences from PCT Publication WO2016/014789. In some embodiments, additional exemplary CAR constructs are generated using the CAR molecules, and/or the VH and VL sequences from PCT Publication WO2014/089335. In some embodiments, additional exemplary CAR constructs are generated using the CAR molecules, and/or the VH and VL sequences from PCT Publication WO2014/140248.

In some embodiments, additional exemplary CAR constructs can also be generated using the VH and VL sequences found in Table 12. The amino acid sequences of exemplary scFv domains comprising the VH and VL domains and a linker sequence, and full-length CARs are also found in Table 12.

**Table 12. Additional exemplary BCMA binding domain sequences**

| **Name** | **Sequence** | **SEQ ID NO:** |
|---|---|---|
| **A7D12.2 VH** | QIQLVQSGPDLKKPGETVKLSCKASGYTFTNFGMNWVKQAPGKGFKWMAWIN TYTGESYFADDFKGRFAFSVETSATTAYLQINNLKTEDTATYFCARGEIYYGYDG GFAYWGQGTLVTVSA | 526 |
| **A7D12.2 VL** | DVVMTQSHRFMSTSVGDRVSITCRASQDVNTAVSWYQQKPGQSPKLLIFSASY RYTGVPDRFTGSGSGADFTLTISSVQAEDLAVYYCQQHYSTPWTFGGGTKLDIK | 527 |
| **A7D12.2 scFv domain** | QIQLVQSGPDLKKPGETVKLSCKASGYTFTNFGMNWVKQAPGKGFKWMAWIN TYTGESYFADDFKGRFAFSVETSATTAYLQINNLKTEDTATYFCARGEIYYGYDG GFAYWGQGTLVTVSAGGGGSGGGGSGGGGSDVVMTQSHRFMSTSVGDRVSI TCRASQDVNTAVSWYQQKPGQSPKLLIFSASYRYTGVPDRFTGSGSGADFTLTI SSVQAEDLAVYYCQQHYSTPWTFGGGTKLDIK | 528 |
| **C11D5.3 VH** | QIQLVQSGPELKKPGETVKISCKASGYTFTDYSINWVKRAPGKGLKWMGWINTE TREPAYAYDFRGRFAFSLETSASTAYLQINNLKYEDTATYFCALDYSYAMDYWG QGTSVTVSS | 529 |
| **C11D5.3 VL** | DIVLTQSPASLAMSLGKRATISCRASESVSVIGAHLIHWYQQKPGQPPKLLIYLAS NLETGVPARFSGSGSGTDFTLTIDPVEEDDVAIYSCLQSRIFPRTFGGGTKLEIK | 530 |
| **C11D5.3 scFv domain** | QIQLVQSGPELKKPGETVKISCKASGYTFTDYSINWVKRAPGKGLKWMGWINTE TREPAYAYDFRGRFAFSLETSASTAYLQINNLKYEDTATYFCALDYSYAMDYWG QGTSVTVSSGGGGSGGGGSGGGGSQIQLVQSGPELKKPGETVKISCKASGYT FTDYSINWVKRAPGKGLKWMGWINTETREPAYAYDFRGRFAFSLETSASTAYL QINNLKYEDTATYFCALDYSYAMDYWGQGTSVTVSS | 531 |
| **C12A3.2 VH** | QIQLVQSGPELKKPGETVKISCKASGYTFRHYSMNWVKQAPGKGLKWMGRINT ESGVPIYADDFKGRFAFSVETSASTAYLVINNLKDEDTASYFCSNDYLYSLDFWG QGTALTVSS | 532 |
| **C12A3.2 VL** | DIVLTQSPPSLAMSLGKRATISCRASESVTILGSHLIYWYQQKPGQPPTLLIQLAS NVQTGVPARFSGSGSRTDFTLTIDPVEEDDVAVYYCLQSRTIPRTFGGGTKLEIK | 533 |
| **C12A3.2 scFv domain** | QIQLVQSGPELKKPGETVKISCKASGYTFRHYSMNWVKQAPGKGLKWMGRINT ESGVPIYADDFKGRFAFSVETSASTAYLVINNLKDEDTASYFCSNDYLYSLDFWG QGTALTVSSGGGGSGGGGSGGGGSDIVLTQSPPSLAMSLGKRATISCRASESV TILGSHLIYWYQQKPGQPPTLLIQLASNVQTGVPARFSGSGSRTDFTLTIDPVEE DDVAVYYCLQSRTIPRTFGGGTKLEIK | 534 |
| **C13F12.1** | QIQLVQSGPELKKPGETVKISCKASGYTFTHYSMNWVKQAPGKGLKWMGRINT | 535 |
| **VH** | ETGEPLYADDFKGRFAFSLETSASTAYLVINNLKNEDTATFFCSNDYLYSCDYW GQGTTLTVSS | |
| **C13F12.1 VL** | DIVLTQSPPSLAMSLGKRATISCRASESVTILGSHLIYWYQQKPGQPPTLLIQLAS NVQTGVPARFSGSGSRTDFTLTIDPVEEDDVAVYYCLQSRTIPRTFGGGTKLEIK | 536 |
| **C13F12.1 scFv domain** | QIQLVQSGPELKKPGETVKISCKASGYTFTHYSMNWVKQAPGKGLKWMGRINT ETGEPLYADDFKGRFAFSLETSASTAYLVINNLKNEDTATFFCSNDYLYSCDYW GQGTTLTVSSGGGGSGGGGSGGGGSDIVLTQSPPSLAMSLGKRATISCRASES VTILGSHLIYWYQQKPGQPPTLLIQLASNVQTGVPARFSGSGSRTDFTLTIDPVE EDDVAVYYCLQSRTIPRTFGGGTKLEIK | 537 |

The human CDR sequences of the scFv domains are shown in Table 13 for the heavy chain variable domains and in Table 14 for the light chain variable domains. "ID" stands for the respective SEQ ID NO for each CDR. The CDRs are shown according to the Kabat definition, however, the CDRs under other convention, for example, Chothia or the combined Kabat/Chothia definitions may be readily deduced based on the VH and VL sequences above.

**Table 13. Heavy Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **HCDR1** | **ID** | **HCDR2** | **ID** | **HCDR3** | **ID** |
|---|---|---|---|---|---|---|
| 139109 | NHGMS | 538 | GIVYSGSTYYAASV KG | 539 | HGGESDV | 540 |
| 139103 | NYAMS | 541 | GISRSGENTYYADS VKG | 542 | SPAHYYGGMDV | 543 |
| 139105 | DYAMH | 544 | GISWNSGSIGYADS VKG | 545 | HSFLAY | 546 |
| 139111 | NHGMS | 538 | GIVYSGSTYYAASV KG | 539 | HGGESDV | 540 |
| 139100 | NFGIN | 547 | WINPKNNNTNYAQK FQG | 548 | GPYYYQSYMDV | 549 |
| 139101 | SDAMT | 550 | VISGSGGTTYYADS VKG | 551 | LDSSGYYYARGPRY | 552 |
| 139102 | NYGIT | 553 | WISAYNGNTNYAQK FQG | 554 | GPYYYYMDV | 555 |
| 139104 | NHGMS | 538 | GIVYSGSTYYAASV KG | 539 | HGGESDV | 540 |
| 139106 | NHGMS | 538 | GIVYSGSTYYAASV KG | 539 | HGGESDV | 540 |
| 139107 | NHGMS | 538 | GIVYSGSTYYAASV KG | 539 | HGGESDV | 540 |
| 139108 | DYYMS | 556 | YISSSGSTIYYADSV KG | 557 | ESGDGMDV | 558 |
| 139110 | DYYMS | 556 | YISSSGNTIYYADSV KG | 559 | STMVREDY | 560 |
| 139112 | NHGMS | 538 | GIVYSGSTYYAASV KG | 539 | HGGESDV | 540 |
| 139113 | NHGMS | 538 | GIVYSGSTYYAASV KG | 539 | HGGESDV | 540 |
| 139114 | NHGMS | 538 | GIVYSGSTYYAASV KG | 539 | HGGESDV | 540 |
| 149362 | SSYYYWG | 561 | SIYYSGSAYYNPSL KS | 562 | HWQEWPDAFDI | 563 |
| 149363 | TSGMCVS | 564 | RIDWDEDKFYSTSL KT | 565 | SGAGGTSATAFDI | 566 |
| 149364 | SYSMN | 567 | SISSSSSYIYYADSV KG | 568 | TIAAVYAFDI | 569 |
| 149365 | DYYMS | 556 | YISSSGSTIYYADSV KG | 557 | DLRGAFDI | 570 |
| 149366 | SHYIH | 571 | MINPSGGVTAYSQT LQG | 572 | EGSGSGWYFDF | 573 |
| 149367 | SGGYYWS | 574 | YIYYSGSTYYNPSLK S | 575 | AGIAARLRGAFDI | 576 |
| 149368 | SYAIS | 577 | GIIPIFGTANYAQKF QG | 578 | RGGYQLLRWDVGLL RSAFDI | 579 |
| 149369 | SNSAAWN | 580 | RTYYRSKWYSFYAI SLKS | 581 | SSPEGLFLYWFDP | 582 |
| BCMA_EBB -C1978-A4 | SYAMS | 583 | AISGSGGSTYYADS VKG | 584 | VEGSGSLDY | 585 |
| BCMA_EBB -C 1978-G 1 | RYPMS | 586 | GISDSGVSTYYADS AKG | 587 | RAGSEASDI | 588 |
| BCMA_EBB -C1979-C1 | SYAMS | 583 | AISGSGGSTYYADS VKG | 584 | ATYKRELRYYYGMD V | 589 |
| BCMA_EBB -C1978-C7 | SYAMS | 583 | AISGSGGSTYYADS VKG | 584 | ATYKRELRYYYGMD V | 589 |
| BCMA_EBB -C1978-D10 | DYAMH | 544 | GISWNSGSIGYADS VKG | 545 | VGKAVPDV | 590 |
| BCMA_EBB -C1979-C12 | DYAMH | 544 | SINWKGNSLAYGDS VKG | ■ 591 | HQGVAYYNYAMDV | 592 |
| BCMA_EBB -C1980-G4 | SYAMS | 583 | AISGSGGSTYYADS VKG | 584 | VVRDGMDV | 593 |
| BCMA_EBB -C1980-D2 | SYAMS | 583 | AISGSGGSTYYADS VKG | 584 | IPQTGTFDY | 594 |
| BCMA_EBB -C1978-A10 | SYAMS | 583 | AISGSGGSTYYADS VKG | 584 | ANYKRELRYYYGMD V | 595 |
| BCMA_EBB -C1978-D4 | SYAMS | 583 | AISGSGGSTYYADS VKG | 584 | ALVGATGAFDI | 596 |
| BCMA_EBB -C1980-A2 | SYAMS | 583 | AISGSGGSTYYADS VKG | 584 | WFGEGFDP | 597 |
| BCMA_EBB -C1981-C3 | SYAMS | 583 | AISGSGGSTYYADS VKG | 584 | VGYDSSGYYRDYY GMDV | 598 |
| BCMA_EBB -C1978-G4 | SYAMS | 583 | AISGSGGSTYYADS VKG | 584 | MGWSSGYLGAFDI | 599 |
| A7D12.2 | NFGMN | 600 | WINTYTGESYFADD FKG | 601 | GEIYYGYDGGFAY | 602 |
| C11D5.3 | DYSIN | 603 | WINTETREPAYAYD FRG | 604 | DYSYAMDY | 605 |
| C12A3.2 | HYSMN | 606 | RINTESGVPIYADDF KG | 607 | DYLYSLDF | 608 |
| C13F12.1 | HYSMN | 606 | RINTETGEPLYADD FKG | 609 | DYLYSCDY | 610 |

**Table 14. Light Chain Variable Domain CDRs according to the Kabat numbering scheme (Kabat et al. (1991), "Sequences of Proteins of Immunological Interest," 5th Ed. Public Health Service, National Institutes of Health, Bethesda, MD)**

| **Candidate** | **LCDR1** | **ID** | **LCDR2** | **ID** | **LCDR3** | **ID** |
|---|---|---|---|---|---|---|
| 139109 | RASQSISSYLN | 611 | AASSLQS | 612 | QQSYSTPYT | 613 |
| 139103 | RASQSISSSFLA | 614 | GASRRAT | 615 | QQYHSSPSWT | 616 |
| 139105 | RSSQSLLHSNGYNYL D | 617 | LGSNRAS | 618 | MQALQTPYT | 619 |
| 139111 | KSSQSLLRNDGKTPL Y | 620 | EVSNRFS | 621 | MQNIQFPS | 622 |
| 139100 | RSSQSLLHSNGYNYL N | 623 | LGSKRAS | 624 | MQALQTPYT | 619 |
| 139101 | RASQSISSYLN | 611 | GASTLAS | 625 | QQSYKRAS | 626 |
| 139102 | RSSQSLLYSNGYNYV D | 627 | LGSNRAS | 618 | MQGRQFPYS | 628 |
| 139104 | RASQSVSSNLA | 629 | GASTRAS | 630 | QQYGSSLT | 631 |
| 139106 | RASQSVSSKLA | 632 | GASIRAT | 636 | QQYGSSSWT | 637 |
| 139107 | RASQSVGSTNLA | 638 | DASNRAT | 639 | QQYGSSPPWT | 640 |
| 139108 | RASQSISSYLN | 611 | AASSLQS | 612 | QQSYTLA | 641 |
| 139110 | KSSESLVHNSGKTYL N | 642 | EVSNRDS | 643 | MQGTHWPGT | 644 |
| 139112 | QASEDINKFLN | 645 | DASTLQT | 646 | QQYESLPLT | 647 |
| 139113 | RASQSVGSNLA | 648 | GASTRAT | 649 | QQYNDWLPVT | 650 |
| 139114 | RASQSIGSSSLA | 651 | GASSRAS | 652 | QQYAGSPPFT | 653 |
| 149362 | KASQDIDDAMN | 654 | SATSPVP | 655 | LQHDNFPLT | 656 |
| 149363 | RASQDIYNNLA | 657 | AANKSQS | 658 | QHYYRFPYS | 659 |
| 149364 | RSSQSLLHSNGYNYL D | 617 | LGSNRAS | 618 | MQALQTPYT | 619 |
| 149365 | GGNNIGTKSVH | 660 | DDSVRPS | 661 | QVWDSDSEHV V | 662 |
| 149366 | SGDGLSKKYVS | 663 | RDKERPS | 664 | QAWDDTTVV | 665 |
| 149367 | RASQGIRNWLA | 666 | AASNLQS | 667 | QKYNSAPFT | 668 |
| 149368 | GGNNIGSKSVH | 669 | GKNNRPS | 670 | SSRDSSGDHL RV | 671 |
| 149369 | QGDSLGNYYAT | 672 | GTNNRPS | 673 | NSRDSSGHHL L | 674 |
| BCMA_EBB-C1978-A4 | RASQSVSSAYLA | 675 | GASTRAT | 649 | QHYGSSFNGS SLFT | 676 |
| BCMA_EBB-C1978-G1 | RASQSVSNSLA | 677 | DASSRAT | 678 | QQFGTSSGLT | 679 |
| BCMA_EBB-C1979-C1 | RASQSVSSSFLA | 680 | GASSRAT | 681 | QQYHSSPSWT | 616 |
| BCMA_EBB-C1978-C7 | RASQSVSTTFLA | 682 | GSSNRAT | 683 | QQYHSSPSWT | 616 |
| BCMA_EBB-C1978-D10 | RASQSISSYLN | 611 | AASSLQS | 612 | QQSYSTPYS | 684 |
| BCMA_EBB-C1979-C12 | RATQSIGSSFLA | 685 | GASQRAT | 686 | QHYESSPSWT | 687 |
| BCMA_EBB-C1980-G4 | RASQSVSSSYLA | 688 | GASSRAT | 681 | QQYGSPPRFT | 689 |
| BCMA_EBB-C1980-D2 | RASQSVSSSYLA | 688 | GASSRAT | 681 | QHYGSSPSWT | 690 |
| BCMA_EBB-C1978-A10 | RASQRVASNYLA | 691 | GASSRAT | 681 | QHYDSSPSWT | 692 |
| BCMA_EBB-C1978-D4 | RASQSLSSNFLA | 693 | GASNWAT | 694 | QYYGTSPMYT | 695 |
| BCMA_EBB-C1980-A2 | RSSQSLLHSNGYNYL D | 617 | LGSNRAS | 618 | MQALQTPLT | 696 |
| BCMA_EBB-C1981-C3 | RASQSVSSSYLA | 688 | GTSSRAT | 697 | QHYGNSPPKF T | 698 |
| BCMA_EBB-C1978-G4 | RASQSVASSFLA | 699 | GASGRAT | 700 | QHYGGSPRLT | 701 |
| A7D12.2 | RASQDVNTAVS | 702 | SASYRYT | 703 | QQHYSTPWT | 704 |
| C11D5.3 | RASESVSVIGAHLIH | 705 | LASNLET | 706 | LQSRIFPRT | 707 |
| C12A3.2 | RASESVTILGSHLIY | 708 | LASNVQT | 709 | LQSRTIPRT | 710 |
| C13F12.1 | RASESVTILGSHLIY | 708 | LASNVQT | 709 | LQSRTIPRT | 710 |

In one embodiment, the BCMA binding domain comprises one or more (e.g., all three) light chain complementary determining region 1 (LC CDR1), light chain complementary determining region 2 (LC CDR2), and light chain complementary determining region 3 (LC CDR3) of a BCMA binding domain described herein, e.g., provided in Table 11, 12 or 14, and/or one or more (e.g., all three) heavy chain complementary determining region 1 (HC CDR1), heavy chain complementary determining region 2 (HC CDR2), and heavy chain complementary determining region 3 (HC CDR3) of a BCMA binding domain described herein, e.g., provided in Table 11, 12 or 13. In one embodiment, the BCMA binding domain comprises one, two, or all of LC CDR1, LC CDR2, and LC CDR3 of any amino acid sequences as provided in Table 11; and one, two or all of HC CDR1, HC CDR2, and HC CDR3 of any amino acid sequences as provided in Table 11.

In one embodiment, the BCMA binding domain comprises a light chain variable region described herein (e.g., in Table 11 or 12) and/or a heavy chain variable region described herein (e.g., in Table 11 or 12). In one embodiment, the BCMA binding domain is a scFv comprising a light chain and a heavy chain of an amino acid sequence listed in Table 11 or 12. In an embodiment, the BCMA binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 11 or 12, or a sequence with 95-99% identity with an amino acid sequence provided in Table 11 or 12; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 11 or 12, or a sequence with 95-99% identity to an amino acid sequence provided in Table 11 or 12.

In one embodiment, the BCMA binding domain comprises an amino acid sequence selected from any one of SEQ ID NOs: 382, 386, 390, 394, 398, 402, 406, 410, 414, 418, 422, 426, 430, 434, 438, 442, 446, 450, 454, 458, 462, 466, 470, 474, 478, 482, 486, 490, 494, 498, 502, 506, 510, 514, 518, 522, 528, 531, 534, or 537; or an amino acid sequence having at least one, two or three modifications (e.g., substitutions, e.g., conservative substitutions) but not more than 30, 20 or 10 modifications (e.g., substitutions, e.g., conservative substitutions) to any of the aforesaid sequences; or a sequence with 95-99% identity to any of the aforesaid sequences. In one embodiment, the BCMA binding domain is a scFv, and a light chain variable region comprising an amino acid sequence described herein, e.g., in Table 11 or 12, is attached to a heavy chain variable region comprising an amino acid sequence described herein, e.g., in Table 11 or 12, via a linker, e.g., a linker described herein. In one embodiment, the BCMA binding domain includes a (Gly4-Ser)n linker, wherein n is 1, 2, 3, 4, 5, or 6, preferably 4 (SEQ ID NO: 967). The light chain variable region and heavy chain variable region of a scFv can be, e.g., in any of the following orientations: light chain variable region-linker-heavy chain variable region or heavy chain variable region-linker-light chain variable region.

Any known CAR, e.g., the BCMA antigen binding domain of any known CAR, in the art can be used in accordance with the instant invention to construct a CAR. For example, those described herein.

In one embodiment, an antigen binding domain against ROR1 is an antigen binding portion, e.g., CDRs, of an antibody described in, e.g., Hudecek et al., Clin Cancer Res 19(12):3153-3164 (2013); WO 2011159847; and US20130101607.

In one embodiment, an antigen binding domain against CD22 is an antigen binding portion, e.g., CDRs, of an antibody described in, e.g., Haso et al., Blood, 121(7): 1165-1174 (2013); Wayne et al., Clin Cancer Res 16(6): 1894-1903 (2010); Kato et al., Leuk Res 37(1):83-88 (2013); Creative BioMart (creativebiomart.net): MOM-18047-S(P).

In one embodiment, an antigen binding domain against CD20 is an antigen binding portion, e.g., CDRs, of the antibody Rituximab, Ofatumumab, Ocrelizumab, Veltuzumab, or GA101, or derivatives thereof. In one embodiment, an antigen binding domain against CD20 is an antigen binding portion as described in WO2016/164731.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody that binds a tumor antigen listed above. In one embodiment, the antigen binding domain comprises a heavy chain variable region and/or a variable light chain region of an antibody that binds a tumor antigen listed above.

In one embodiment, the antigen binding domain of a CAR described herein is a scFv antibody fragment. In one aspect, such antibody fragments are functional in that they retain the equivalent binding affinity, e.g., they bind the same antigen with comparable efficacy, as the IgG antibody from which it is derived. In other embodiments, the antibody fragment has a lower binding affinity, e.g., it binds the same antigen with a lower binding affinity than the antibody from which it is derived, but is functional in that it provides a biological response described herein. In one embodiment, the CAR molecule comprises an antibody fragment that has a binding affinity K_{D} of 10⁻⁴ M to 10⁻⁸ M, e.g., 10⁻⁵ M to 10⁻⁷ M, e.g., 10⁻⁶ M or 10⁻⁷ M, for the target antigen. In one embodiment, the antibody fragment has a binding affinity that is at least fivefold, 10-fold, 20-fold, 30-fold, 50-fold, 100-fold or 1,000-fold less than a reference antibody, e.g., an antibody described herein.

In one embodiment, the antigen binding domain comprises a non-human antibody or antibody fragment, e.g., a mouse antibody or antibody fragment.

In another embodiment, the antigen binding domain comprises a humanized antibody or an antibody fragment. In some aspects, a non-human antibody is humanized, where specific sequences or regions of the antibody are modified to increase similarity to an antibody naturally produced in a human or fragment thereof. In one aspect, the antigen binding domain is humanized compared to the murine sequence of the antibody or antibody fragment, e.g., scFv, from which it is derived.

A humanized antibody can be produced using a variety of techniques known in the art, including but not limited to, CDR-grafting (see, e.g., European Patent No. EP 239,400; International Publication No. WO 91/09967; and U.S. Pat. Nos. 5,225,539, 5,530,101, and 5,585,089), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973), chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., J. Immunol., 169:1119-25 (2002), Caldas et al., Protein Eng., 13(5):353-60 (2000), Morea et al., Methods, 20(3):267-79 (2000), Baca et al., J. Biol. Chem., 272(16):10678-84 (1997), Roguska et al., Protein Eng., 9(10):895-904 (1996), Couto et al., Cancer Res., 55 (23 Supp):5973s-5977s (1995), Couto et al., Cancer Res., 55(8):1717-22 (1995), Sandhu J S, Gene, 150(2):409-10 (1994), and Pedersen et al., J. Mol. Biol., 235(3):959-73 (1994). Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323.)

A humanized antibody or antibody fragment has one or more amino acid residues remaining in it from a source which is nonhuman. These nonhuman amino acid residues are often referred to as "import" residues, which are typically taken from an "import" variable domain. As provided herein, humanized antibodies or antibody fragments comprise one or more CDRs from nonhuman immunoglobulin molecules and framework regions wherein the amino acid residues comprising the framework are derived completely or mostly from human germline. Multiple techniques for humanization of antibodies or antibody fragments are well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640). In such humanized antibodies and antibody fragments, substantially less than an intact human variable domain has been substituted by the corresponding sequence from a nonhuman species. Humanized antibodies are often human antibodies in which some CDR residues and possibly some framework (FR) residues are substituted by residues from analogous sites in rodent antibodies. Humanization of antibodies and antibody fragments can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

The choice of human variable domains, both light and heavy, to be used in making the humanized antibodies is to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody (Sims et al., J. Immunol., 151:2296 (1993); Chothia et al., J. Mol. Biol., 196:901 (1987)). Another method uses a particular framework derived from the consensus sequence of all human antibodies of a particular subgroup of light or heavy chains. The same framework may be used for several different humanized antibodies (see, e.g., Nicholson et al. Mol. Immun. 34 (16-17): 1157-1165 (1997); Carter et al., Proc. Natl. Acad. Sci. USA, 89:4285 (1992); Presta et al., J. Immunol., 151:2623 (1993)). In some embodiments, the framework region, e.g., all four framework regions, of the heavy chain variable region are derived from a VH4_4-59 germline sequence. In one embodiment, the framework region can comprise, one, two, three, four or five modifications, e.g., substitutions, e.g., from the amino acid at the corresponding murine sequence. In one embodiment, the framework region, e.g., all four framework regions of the light chain variable region are derived from a VK3_1.25 germline sequence. In one embodiment, the framework region can comprise, one, two, three, four or five modifications, e.g., substitutions, e.g., from the amino acid at the corresponding murine sequence.

In some aspects, the antibody fragment of a CAR is humanized with retention of high affinity for the target antigen and other favorable biological properties. According to one aspect of the invention, humanized antibodies and antibody fragments are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, e.g., the analysis of residues that influence the ability of the candidate immunoglobulin to bind the target antigen. In this way, FR residues can be selected and combined from the recipient and import sequences so that the desired antibody or antibody fragment characteristic, such as increased affinity for the target antigen, is achieved. In general, the CDR residues are directly and most substantially involved in influencing antigen binding.

A humanized antibody or antibody fragment may retain a similar antigenic specificity as the original antibody, e.g., in the present disclosure, the ability to bind human a tumor antigen as described herein. In some embodiments, a humanized antibody or antibody fragment may have improved affinity and/or specificity of binding to a tumor antigen as described herein. In some embodiments, a humanized antibody or antibody fragment may have lower affinity and/or specificity of a tumor antigen as described herein or a B cell antigen as described herein.

In one aspect, the antigen binding domain of the invention is characterized by particular functional features or properties of an antibody or antibody fragment. For example, in one aspect, the portion of a CAR of the invention that comprises an antigen binding domain specifically binds a tumor antigen as described herein.

In one aspect, the antigen binding domain is a fragment, e.g., a single chain variable fragment (scFv). In one aspect, the anti- tumor antigen as described herein binding domain is a Fv, a Fab, a (Fab')2, or a bifunctional (e.g. bi-specific) hybrid antibody (e.g., Lanzavecchia et al., Eur. J. Immunol. 17, 105 (1987)). In one aspect, the antibodies and fragments thereof of the disclosure binds a tumor antigen as described herein protein with wild-type or enhanced affinity.

In some instances, scFvs can be prepared according to method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:22). In one embodiment, the linker can be (Gly₄Ser)₄ (SEQ ID NO:29) or (Gly₄Ser)₃(SEQ ID NO:30). Variation in the linker length may retain or enhance activity, giving rise to superior efficacy in activity studies.

In another aspect, the antigen binding domain is a T cell receptor ("TCR"), an engineered TCR, or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012). For example, scTCR can be engineered that contains the Vα and Vβ genes from a T cell clone linked by a linker (e.g., a flexible peptide). This approach is very useful to cancer associated target that itself is intracellular, however, a fragment of such antigen (peptide) is presented on the surface of the cancer cells by MHC.

In one aspect, the antigen binding domain of the CAR comprises an amino acid sequence that is homologous to an antigen binding domain amino acid sequence described herein, and the antigen binding domain retains the desired functional properties of the antigen binding domain described herein.

In one specific aspect, the antigen binding domain of the CAR comprises an antibody fragment. In a further aspect, the antibody fragment comprises a scFv. In a further aspect, the antibody fragment comprises a variable heavy chain (VH) only.

In various aspects, the antigen binding domain of the CAR is engineered by modifying one or more amino acids within one or both variable regions (e.g., VH and/or VL), for example within one or more CDR regions and/or within one or more framework regions. In one specific aspect, the CAR composition of the invention comprises an antibody fragment. In a further aspect, the antibody fragment comprises an scFv. It will be understood by one of ordinary skill in the art that the antibody or antibody fragment of the disclosure may further be modified such that they vary in amino acid sequence (e.g., from wild-type), but not in desired activity. For example, additional nucleotide substitutions leading to amino acid substitutions at "non-essential" amino acid residues may be made to the protein. For example, a nonessential amino acid residue in a molecule may be replaced with another amino acid residue from the same side chain family. In another embodiment, a string of amino acids can be replaced with a structurally similar string that differs in order and/or composition of side chain family members, e.g., a conservative substitution, in which an amino acid residue is replaced with an amino acid residue having a similar side chain, may be made.

Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Percent identity in the context of two or more nucleic acids or polypeptide sequences, refers to two or more sequences that are the same. Two sequences are "substantially identical" if two sequences have a specified percentage of amino acid residues or nucleotides that are the same (e.g., 60% identity, optionally 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are well known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, Wl), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

In one aspect, the present disclosure contemplates modifications of the starting antibody or fragment (e.g., scFv) amino acid sequence that generate functionally equivalent molecules. For example, the VH or VL of an antigen binding domain to a tumor antigen described herein, e.g., scFv, comprised in the CAR can be modified to retain at least about 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity of the starting VH or VL framework region of the antigen binding domain to the tumor antigen described herein, e.g., scFv. The present disclosure contemplates modifications of the entire CAR construct, e.g., modifications in one or more amino acid sequences of the various domains of the CAR construct in order to generate functionally equivalent molecules. The CAR construct can be modified to retain at least about 70%, 71%. 72%. 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity of the starting CAR construct.

### Bispecific CARs

In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Protocols for generating bispecific or heterodimeric antibody molecules are known in the art; including but not limited to, for example, the "knob in a hole" approach described in, *e.g.,* US 5731168; the electrostatic steering Fc pairing as described in, *e.g.,* WO 09/089004, WO 06/106905 and WO 2010/129304; Strand Exchange Engineered Domains (SEED) heterodimer formation as described in, *e.g.,* WO 07/110205; Fab arm exchange as described in, *e.g.,* WO 08/119353, WO 2011/131746, and WO 2013/060867; double antibody conjugate, e.g., by antibody cross-linking to generate a bi-specific structure using a heterobifunctional reagent having an amine-reactive group and a sulfhydryl reactive group as described in, *e.g.,* US 4433059; bispecific antibody determinants generated by recombining half antibodies (heavy-light chain pairs or Fabs) from different antibodies through cycle of reduction and oxidation of disulfide bonds between the two heavy chains, as described in, *e.g.,* US 4444878; trifunctional antibodies, *e*.*g*., three Fab' fragments cross-linked through sulfhdryl reactive groups, as described in, *e*.*g*., US5273743; biosynthetic binding proteins, *e*.*g*., pair of scFvs cross-linked through C-terminal tails preferably through disulfide or amine-reactive chemical cross-linking, as described in, *e.g.,* US5534254; bifunctional antibodies, *e*.*g*., Fab fragments with different binding specificities dimerized through leucine zippers (*e*.*g*., c-fos and c-jun) that have replaced the constant domain, as described in, e.g., US5582996; bispecific and oligospecific mono-and oligovalent receptors, *e*.*g*., VH-CH1 regions of two antibodies (two Fab fragments) linked through a polypeptide spacer between the CH1 region of one antibody and the VH region of the other antibody typically with associated light chains, as described in, *e*.*g*., US5591828; bispecific DNA-antibody conjugates, *e*.*g*., crosslinking of antibodies or Fab fragments through a double stranded piece of DNA, as described in, *e*.*g*., US5635602; bispecific fusion proteins, *e.g.,* an expression construct containing two scFvs with a hydrophilic helical peptide linker between them and a full constant region, as described in, *e*.*g*., US5637481; multivalent and multispecific binding proteins, *e*.*g*., dimer of polypeptides having first domain with binding region of Ig heavy chain variable region, and second domain with binding region of Ig light chain variable region, generally termed diabodies (higher order structures are also encompassed creating for bispecifc, trispecific, or tetraspecific molecules, as described in, *e*.*g*., US5837242; minibody constructs with linked VL and VH chains further connected with peptide spacers to an antibody hinge region and CH3 region, which can be dimerized to form bispecific/multivalent molecules, as described in, *e*.*g*., US5837821; VH and VL domains linked with a short peptide linker (*e*.*g*., 5 or 10 amino acids) or no linker at all in either orientation, which can form dimers to form bispecific diabodies; trimers and tetramers, as described in, *e*.*g*., US5844094; String of VH domains (or VL domains in family members) connected by peptide linkages with crosslinkable groups at the C-terminus futher associated with VL domains to form a series of FVs (or scFvs), as described in, *e*.*g*., US5864019; and single chain binding polypeptides with both a VH and a VL domain linked through a peptide linker are combined into multivalent structures through non-covalent or chemical crosslinking to form, *e*.*g*., homobivalent, heterobivalent, trivalent, and tetravalent structures using both scFV or diabody type format, as described in, e.g., US5869620. Additional exemplary multispecific and bispecific molecules and methods of making the same are found, for example, in US5910573, US5932448, US5959083, US5989830, US6005079, US6239259, US6294353, US6333396, US6476198, US6511663, US6670453, US6743896, US6809185, US6833441, US7129330, US7183076, US7521056, US7527787, US7534866, US7612181, US2002004587A1, US2002076406A1, US2002103345A1, US2003207346A1, US2003211078A1, US2004219643A1, US2004220388A1, US2004242847A1, US2005003403A1, US2005004352A1, US2005069552A1, US2005079170A1, US2005100543A1, US2005136049A1, US2005136051A1, US2005163782A1, US2005266425A1, US2006083747A1, US2006120960A1, US2006204493A1, US2006263367A1, US2007004909A1, US2007087381A1, US2007128150A1, US2007141049A1, US2007154901A1, US2007274985A1, US2008050370A1, US2008069820A1, US2008152645A1, US2008171855A1, US2008241884A1, US2008254512A1, US2008260738A1, US2009130106A1, US2009148905A1, US2009155275A1, US2009162359A1, US2009162360A1, US2009175851A1, US2009175867A1, US2009232811A1, US2009234105A1, US2009263392A1, US2009274649A1, EP346087A2, WO0006605A2, WO02072635A2, WO04081051A1, WO06020258A2, WO2007044887A2, WO2007095338A2, WO2007137760A2, WO2008119353A1, WO2009021754A2, WO2009068630A1, WO9103493A1, WO9323537A1, WO9409131A1, WO9412625A2, WO9509917A1, WO9637621A2, WO9964460A1.

Within each antibody or antibody fragment (e.g., scFv) of a bispecific antibody molecule, the VH can be upstream or downstream of the VL. In some embodiments, the upstream antibody or antibody fragment (e.g., scFv) is arranged with its VH (VH₁) upstream of its VL (VL₁) and the downstream antibody or antibody fragment (e.g., scFv) is arranged with its VL (VL₂) upstream of its VH (VH₂), such that the overall bispecific antibody molecule has the arrangement VH₁-VL₁-VL₂-VH₂. In other embodiments, the upstream antibody or antibody fragment (e.g., scFv) is arranged with its VL (VL₁) upstream of its VH (VH₁) and the downstream antibody or antibody fragment (e.g., scFv) is arranged with its VH (VH₂) upstream of its VL (VL₂), such that the overall bispecific antibody molecule has the arrangement VL₁₋VH₁₋VH₂-VL₂. Optionally, a linker is disposed between the two antibodies or antibody fragments (e.g., scFvs), e.g., between VL₁ and VL₂ if the construct is arranged as VH₁-VL₁-VL₂-VH₂, or between VH₁ and VH₂ if the construct is arranged as VL₁-VH₁-VH₂-VL₂. The linker may be a linker as described herein, e.g., a (Gly₄-Ser)n linker, wherein n is 1, 2, 3, 4, 5, or 6, preferably 4 (SEQ ID NO: 967). In general, the linker between the two scFvs should be long enough to avoid mispairing between the domains of the two scFvs. Optionally, a linker is disposed between the VL and VH of the first scFv. Optionally, a linker is disposed between the VL and VH of the second scFv. In constructs that have multiple linkers, any two or more of the linkers can be the same or different. Accordingly, in some embodiments, a bispecific CAR comprises VLs, VHs, and optionally one or more linkers in an arrangement as described herein.

In one aspect, the chimeric antigen receptor comprises a bispecific antigen binding domain, a transmembrane domain (e.g., as described herein), and an intracellular signaling domain (e.g., as described herein). In embodiments, the bispecific antigen binding domain comprises a first immunoglobulin variable domain sequence, e.g., an scFv (or comprises the light chain CDRs and/or heavy chain CDRs from a scFv described herein), which binds an antigen, e.g., as described herein, e.g., (a CD19 binding domain or BCMA binding domain described herein, e.g., in Table 6 or Table 12), and a second immunoglobulin variable domain sequence, e.g., a scFv (or comprises the light chain CDRs and/or heavy chain CDRs from a scFv described herein), which has binding specificity for one or more antigens described herein, e.g., comprises a scFv as described herein, e.g., comprising a mesothelin binding domain or EGFRvIII binding domain (e.g., as described in Table 2 or Table 5). In embodiments, the bispecific antigen binding domain comprises a CD19 binding domain described herein and a mesothelin binding domain described herein. In embodiments, the bispecific antigen binding domain comprises a BCMA binding domain described herein and a mesothelin binding domain described herein. In embodiments, the bispecific antigen binding domain comprises a CD19 binding domain described herein and a EGFRvIII binding domain described herein. In embodiments, the bispecific antigen binding domain comprises a BCMA binding domain described herein and a EGFRvIII binding domain described herein. In another aspect, the invention provides a cell (e.g., a population of cells), e.g., an immune effector cell, e.g., a T cell or NK cell, e.g., as described herein, which is engineered to express (e.g., comprises) a bispecific CAR as described herein, e.g., a bispecific CAR comprising two antigen binding domains described herein. Without being bound by any theory, it is believed that cells expressing such bispecific CARs (e.g., comprising a two antigen binding domains, e.g., as described herein) are useful in the methods and compositions described herein.

### Chimeric TCR

In one aspect, the antigen binding domains described herein, e.g., the antibodies and antibody fragments, e.g., provided in the Tables herein, can be grafted to one or more constant domain of a T cell receptor ("TCR") chain, for example, a TCR alpha or TCR beta chain, to create an chimeric TCR that binds specificity to a tumor antigen described herein. Without being bound by theory, it is believed that chimeric TCRs will signal through the TCR complex upon antigen binding. For example, a mesothelin or CD19 scFv or a fragment there of, e.g., a VL domain, or VH domain, as disclosed herein, can be grafted to the constant domain, e.g., at least a portion of the extracellular constant domain, the transmembrane domain and the cytoplasmic domain, of a TCR chain, for example, the TCR alpha chain and/or the TCR beta chain. As another example, the CDRs of an antibody or antibody fragment, e.g., the CDRs of anyantibody or antibody fragment as described in Tables provided herein may be grafted into a TCR alpha and/or beta chain to create a chimeric TCR that binds specifically to a tumor antigen described herein. For example, the LCDRs disclosed herein may be grafted into the variable domain of a TCR alpha chain and the HCDRs disclosed herein may be grafted to the variable domain of a TCR beta chain, or vice versa. Such chimeric TCRs may be produced by methods known in the art (For example, Willemsen RA et al, Gene Therapy 2000; 7: 1369-1377; Zhang T et al, Cancer Gene Ther 2004; 11: 487-496; Aggen et al, Gene Ther. 2012 Apr;19(4):365-74).

### Transmembrane Domain

With respect to the transmembrane domain, in various embodiments, a CAR can be designed to comprise a transmembrane domain that is attached to the extracellular domain of the CAR, e.g., the antigen binding domain. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR, for example, the transmembrane domain is from the same protein as the intracellular signalling domain, e.g., the costimulatory domain. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect, the amino acid sequence of the transmembrane domain may be modified or substituted so as to minimize interactions with the binding domains of the native binding partner present in the same CAR-expressing cell.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this invention may include at least the transmembrane region(s) of e.g., the alpha, beta or zeta chain of the T-cell receptor, CD28, CD27, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge, e.g., an IgG4 hinge, or a CD8a hinge. In one embodiment, the hinge or spacer comprises (e.g., consists of) the amino acid sequence of SEQ ID NO:4. In one aspect, the transmembrane domain comprises (e.g., consists of) a transmembrane domain of SEQ ID NO: 12.

In one aspect, the hinge or spacer comprises an IgG4 hinge. For example, in one embodiment, the hinge or spacer comprises a hinge of the amino acid sequence SEQ ID NO: 6. In some embodiments, the hinge or spacer comprises a hinge encoded by a nucleotide sequence of SEQ ID NO: 7. In one aspect, the hinge or spacer comprises an IgD hinge. For example, in one embodiment, the hinge or spacer comprises a hinge of the amino acid sequence SEQ ID NO: 8. In some embodiments, the hinge or spacer comprises a hinge encoded by a nucleotide sequence of SEQ ID NO: 9.

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO:10). In some embodiments, the linker is encoded by a nucleotide sequence of GGTGGCGGAGGTTCTGGAGGTGGAGGTTCC (SEQ ID NO:11).

In one aspect, the hinge or spacer comprises a KIR2DS2 hinge.

### Cytoplasmic Domain

The cytoplasmic domain or region of the CAR includes an intracellular signaling domain. An intracellular signaling domain is generally responsible for activation of at least one of the normal effector functions of the immune cell in which the CAR has been introduced. The term "effector function" refers to a specialized function of a cell. Effector function of a T cell, for example, may be cytolytic activity or helper activity including the secretion of cytokines. Thus the term "intracellular signaling domain" refers to the portion of a protein which transduces the effector function signal and directs the cell to perform a specialized function. While usually the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

Examples of intracellular signaling domains for use in the CAR of the invention include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are insufficient for full activation of the T cell and that a secondary and/or costimulatory signal is also required. Thus, T cell activation can be said to be mediated by two distinct classes of cytoplasmic signaling sequences: those that initiate antigen-dependent primary activation through the TCR (primary intracellular signaling domains) and those that act in an antigen-independent manner to provide a secondary or costimulatory signal (secondary cytoplasmic domain, e.g., a costimulatory domain).

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the invention include those of TCR zeta, FcR gamma, FcR beta, CD3 gamma, CD3 delta , CD3 epsilon, CD5, CD22, CD79a, CD79b, CD278 (also known as "ICOS"), FcεRI, DAP10, DAP12,and CD66d. In one embodiment, a CAR of the invention comprises an intracellular signaling domain, e.g., a primary signaling domain of CD3-zeta, e.g., a CD3-zeta sequence described herein.

In one embodiment, a primary signaling domain comprises a modified ITAM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

The intracellular signaling domain of the CAR can comprise the CD3-zeta signaling domain by itself or it can be combined with any other desired intracellular signaling domain(s) useful in the context of a CAR of the invention. For example, the intracellular signaling domain of the CAR can comprise a CD3 zeta chain portion and a costimulatory signaling domain. The costimulatory signaling domain refers to a portion of the CAR comprising the intracellular domain of a costimulatory molecule. A costimulatory molecule is a cell surface molecule other than an antigen receptor or its ligands that is required for an efficient response of lymphocytes to an antigen. Examples of such molecules include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83, and the like. For example, CD27 costimulation has been demonstrated to enhance expansion, effector function, and survival of human CART cells in vitro and augments human T cell persistence and antitumor activity in vivo (Song et al. Blood. 2012; 119(3):696-706). Further examples of such costimulatory molecules include an MHC class I molecule, a TNF receptor protein, an Immunoglobulin-like protein, a cytokine receptor, an integrin, a signaling lymphocytic activation molecule (SLAM protein), an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

The intracellular signaling sequences within the cytoplasmic portion of the CAR of the invention may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequence. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 14. In one aspect, the signaling domain of CD3-zeta is a signaling domain of SEQ ID NO: 18.

In one aspect, the intracellular signaling domain is designed to comprise the signaling domain of CD3-zeta and the signaling domain of CD27. In one aspect, the signaling domain of CD27 comprises an amino acid sequence of SEQ ID NO:16. In one aspect, the signalling domain of CD27 is encoded by a nucleic acid sequence of SEQ ID NO:17.

In one aspect, the intracellular is designed to comprise the signaling domain of CD3-zeta and the signaling domain of CD28. In one aspect, the signaling domain of CD28 comprises an amino acid sequence of SEQ ID NO: 124. In one aspect, the signaling domain of CD28 is encoded by a nucleic acid sequence of SEQ ID NO: 1113.

In one aspect, the intracellular is designed to comprise the signaling domain of CD3-zeta and the signaling domain of ICOS. In one aspect, the signaling domain of ICOS comprises an amino acid sequence of SEQ ID NO: 120. In one aspect, the signaling domain of ICOS is encoded by a nucleic acid sequence of SEQ ID NO: 1111.

In one aspect, the cell of the invention, e.g., described herein, e.g., a cell expressing two different CARs, includes CARs that each include an antigen binding domain that binds a target antigen described herein, a transmembrane domain, a primary signaling domain, and a costimulatory signaling domain. In embodiments of the aforementioned aspect, the costimulatory domain of the two different CARs may be the same or different. In embodiments, one or more of the two different CARs comprise more than one costimulatory signalling domains. In other aspects, the cell of the invention, e.g., described herein, e.g., a cell expressing two different CARs, includes a first CAR that includes an antigen binding domain that binds a target antigen described herein, a transmembrane domain, a primary signaling domain, but does not include a costimulatory signaling domain, and a second CAR that includes an antigen binding domain that binds a target antigen described herein, a transmembrane domain, and a costimulatory signaling domain, but does not include a primary signaling domain.

In one aspect, the CAR-expressing cell described herein, e.g. a cell expressing two different CARs can further comprise another, e.g., a third, CAR, e.g., another CAR that includes a different antigen binding domain, e.g., to the same target or a different target (e.g., a target other than a tumor antigen described herein or a different tumor antigen described herein). For example, in an embodiment where the cell of the invention expresses a third CAR comprising an antigen binding domain that binds a second target tumor antigen, the third CAR includes an antigen binding domain to a target expressed the same cancer cell type as the tumor antigen targeted by the first or second CAR. In one embodiment, the CAR-expressing cell comprises a first CAR that targets a first tumor antigen and includes an intracellular signaling domain having a costimulatory signaling domain but not a primary signaling domain, and a third CAR that targets a second, different, tumor antigen and includes an intracellular signaling domain having a primary signaling domain but not a costimulatory signaling domain. While not wishing to be bound by theory, placement of a costimulatory signaling domain, e.g., 4-1BB, CD28, CD27 or OX-40, onto the first CAR, and the primary signaling domain, e.g., CD3 zeta, on the third CAR can limit the CAR activity to cells where both targets are expressed. In one embodiment, the cell of the invention comprises a first CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another embodiment, the cell of the invention comprises (i.e., is genetically engineered to express) a first CAR that includes an antigen binding domain that binds a target antigen described herein, a transmembrane domain and a primary signaling domain and a second CAR that targets a tumor antigen other than the first target antigen (e.g., an antigen expressed on the same cancer cell type as the first target antigen) and includes an antigen binding domain to the antigen, a transmembrane domain and a costimulatory signaling domain. In another embodiment, the cell of the invention comprises (i.e., is genetically engineered to express) a first CAR that includes an antigen binding domain that binds a target antigen described herein, a transmembrane domain, a costimulatory signaling domain and a primary signaling domain, and a second CAR that targets a tumor antigen other than the first target antigen (e.g., an antigen expressed on the same cancer cell type as the first target antigen) and includes an antigen binding domain to the antigen, a transmembrane domain, a costimulatory signaling domain and a primary signaling domain. In embodiments where both the first and second CAR include a costimulatory signaling domain, the costimulatory signaling domain of the first CAR and the second CAR may be derived from the same protein, e.g., from a costimulatory protein described herein, e.g., 4-1BB, CD28, or ICOS. In other embodiments, the costimulatory signaling domain of the first CAR and the second CAR may be derived from the different proteins, e.g., the first CAR includes a costimulatory signaling domain described herein, e.g., of 4-1BB, and the second CAR includes a different costimulatory signaling domain described herein, e.g., of CD28.

In one embodiment, the CAR-expressing cell comprises a CAR comprising antigen binding domain that bind target antigens described herein and an inhibitory CAR. In one embodiment, the inhibitory CAR comprises an antigen binding domain that binds an antigen found on normal cells but not cancer cells, e.g., normal cells that also express the tumor antigens targeted by the CAR comprising an antigen binding domain that binds a target antigen. In one embodiment, the inhibitory CAR comprises the antigen binding domain, a transmembrane domain and an intracellular domain of an inhibitory molecule. For example, the intracellular domain of the inhibitory CAR can be an intracellular domain of PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, or TGFR beta.

In one embodiment, the antigen binding domains of the different CARs can be such that the antigen binding domains do not interact with one another. For example, a cell expressing a first and second CAR can have an antigen binding domain of the first CAR, e.g., as a fragment, e.g., an scFv, that does not form an association with the antigen binding domain of the second CAR, e.g., the antigen binding domain of the second CAR is a VHH.

In some embodiments, the antigen binding domain comprises a single domain antigen binding (SDAB) molecules include molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies. SDAB molecules may be any of the art, or any future single domain molecules. SDAB molecules may be derived from any species including, but not limited to mouse, human, camel, llama, lamprey, fish, shark, goat, rabbit, and bovine. This term also includes naturally occurring single domain antibody molecules from species other than Camelidae and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from Camelidae species, for example in camel, llama, dromedary, alpaca and guanaco. Other species besides Camelidae may produce heavy chain molecules naturally devoid of light chain; such VHHs are within the scope of the invention.

The SDAB molecules can be recombinant, CDR-grafted, humanized, camelized, de-immunized and/or in vitro generated (e.g., selected by phage display).

It has also been discovered, that cells having a plurality of chimeric membrane embedded receptors comprising an antigen binding domain that interactions between the antigen binding domain of the receptors can be undesirable, e.g., because it inhibits the ability of one or more of the antigen binding domains to bind its cognate antigen. Accordingly, disclosed herein are cells having a first and a second non-naturally occurring chimeric membrane embedded receptor comprising antigen binding domains that minimize such interactions. Also disclosed herein are nucleic acids encoding a first and a second non-naturally occurring chimeric membrane embedded receptor comprising a antigen binding domains that minimize such interactions, as well as methods of making and using such cells and nucleic acids. In an embodiment the antigen binding domain of one of said first said second non-naturally occurring chimeric membrane embedded receptor, comprises an scFv, and the other comprises a single VH domain, e.g., a camelid, shark, or lamprey single VH domain, or a single VH domain derived from a human or mouse sequence.

In another aspect, the CAR-expressing cell of the invention can further express another agent, e.g., an agent which enhances the activity of a CAR-expressing cell. For example, in one embodiment, the agent can be an agent which inhibits an inhibitory molecule. Inhibitory molecules, e.g., PD1, can, in some embodiments, decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta.

In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

In one embodiment, the agent comprises the extracellular domain (ECD) of an inhibitory molecule, e.g., Programmed Death 1 (PD1), fused to a transmembrane domain and intracellular signaling domains such as 41BB and CD3 zeta (also referred to herein as a PD1 CAR). In one embodiment, the PD1 CAR, when used incombinations with a XCAR described herein, improves the persistence of the T cell. In one embodiment, the CAR is a PD1 CAR comprising the extracellular domain of PD1 indicated as underlined in SEQ ID NO: 26. In one embodiment, the PD1 CAR comprises the amino acid sequence of SEQ ID NO:26. In one embodiment, the PD1 CAR comprises the amino acid sequence of SEQ ID NO:39).

In one embodiment, the agent comprises a nucleic acid sequence encoding the PD1 CAR, e.g., the PD1 CAR described herein. In one embodiment, the nucleic acid sequence for the PD1 CAR is shown as SEQ ID NO: 27 in Table 1, with the sequence for PD1 ECD underlined.

In another aspect, the present disclosure provides a population of CAR-expressing cells. In some embodiments, the population of CAR-expressing cells comprises a mixture of cells expressing different CARs. For example, in one embodiment, the population of CART cells can include a first cell expressing a CAR having an antigen binding domain to a antigen described herein, and a second cell expressing a CAR having a different antigen binding domain, e.g., an antigen binding domain to a different antigen described herein, e.g., an antigen binding domain to a tumor antigen described herein that differs from the tumor antigen bound by the antigen binding domain of the CAR expressed by the first cell. As another example, the population of CAR-expressing cells can include a first cell expressing a CAR that includes an antigen binding domain to a tumor antigen described herein, and a second cell expressing a CAR that includes an antigen binding domain to a target other than a tumor antigen as described herein. In one embodiment, the population of CAR-expressing cells includes, e.g., a first cell expressing a CAR that includes a primary intracellular signaling domain, and a second cell expressing a CAR that includes a secondary signaling domain.

In another aspect, the present disclosure provides a population of cells wherein at least one cell in the population expresses a CAR having an antigen binding domain to a tumor antigen described herein, and a second cell expressing another agent, e.g., an agent which enhances the activity of a CAR-expressing cell. For example, in one embodiment, the agent can be an agent which inhibits an inhibitory molecule. Inhibitory molecules, e.g., PD-1, can, in some embodiments, decrease the ability of a CAR-expressing cell to mount an immune effector response. Examples of inhibitory molecules include PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGFR beta, or a fragment of any of these, and a second polypeptide which is an intracellular signaling domain described herein (e.g., comprising a costimulatory domain (e.g., 41BB, CD27, OX40 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28 signaling domain described herein and/or a CD3 zeta signaling domain described herein).

In one aspect, the present disclosure provides methods comprising administering a population of CAR-expressing cells, e.g., a mixture of cells expressing different CARs, in combination with another agent, e.g., a kinase inhibitor, such as a kinase inhibitor described herein. In another aspect, the present disclosure provides methods comprising administering a population of cells wherein at least one cell in the population expresses a CAR having an antigen binding domain of a tumor antigen described herein, and a second cell expressing another agent, e.g., an agent which enhances the activity of a CAR-expressing cell, in combination with another agent, e.g., a kinase inhibitor, such as a kinase inhibitor described herein.

### Exemplary CAR Molecules

The CAR molecules disclosed herein can comprise a binding domain that binds to a target, e.g., a target as described herein; a transmembrane domain, e.g., a transmembrane domain as described herein; and an intracellular signaling domain, e.g., an intracellular domain as described herein. In embodiments, the binding domain comprises a heavy chain complementary determining region 1 (HC CDR1), a heavy chain complementary determining region 2 (HC CDR2), and a heavy chain complementary determining region 3 (HC CDR3) of a heavy chain binding domain described herein, and/or a light chain complementary determining region 1 (LC CDR1), a light chain complementary determining region 2 (LC CDR2), and a light chain complementary determining region 3 (LC CDR3) of a light chain binding domain described herein.

In other embodiments, the CAR molecule comprises a CD19 CAR molecule described herein, e.g., a CD19 CAR molecule described in US-2015-0283178-A1, e.g., CTL019. In embodiments, the CD19 CAR comprises an amino acid, or has a nucleotide sequence shown in US-2015-0283178-A1, or a sequence substantially identical thereto (e.g., at least 85%, 90%, 95% or more identical thereto).

In one embodiment, the CAR T cell that specifically binds to CD19 has the USAN designation TISAGENLECLEUCEL-T. CTL019 is made by a gene modification of T cells is mediated by stable insertion via transduction with a self-inactivating, replication deficient Lentiviral (LV) vector containing the CTL019 transgene under the control of the EF-1 alpha promoter. CTL019 can be a mixture of transgene positive and negative T cells that are delivered to the subject on the basis of percent transgene positive T cells.

In one embodiment, the CAR19 molecule comprises (e.g., consists of) an amino acid sequence as provided in Table 15A, or in Table 3 of International Publication No. WO2014/153270, filed March 15, 2014. The amino acid and nucleotide sequences encoding the CD19 CAR molecules and antigen binding domains (e.g., including one, two, three VH CDRs; and one, two, three VL CDRs according to Kabat or Chothia), are specified in WO2014/153270. In embodiments, the CD19 CAR comprises an amino acid, or has a nucleotide sequence shown in WO2014/153270, or a sequence substantially identical to any of the aforesaid sequences (e.g., at least 85%, 90%, 95% or more identical to any of the aforesaid CD19 CAR sequences). In one embodiment, the CAR molecule comprises (e.g., consists of) an amino acid sequence selected from any one of SEQ ID NOs: 897, 902, 907, 912, 917, 922, 927, 932, 937, 942, 947, 952, 956; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence selected from any one of SEQ ID NOs: 897, 902, 907, 912, 917, 922, 927, 932, 937, 942, 947, 952, 956; or an amino acid sequence having 85%, 90%, 95%, 96%, 97%, 98%, 99% identity to an amino acid sequence selected from any one of SEQ ID NOs: 897, 902, 907, 912, 917, 922, 927, 932, 937, 942, 947, 952, 956.

In one embodiment, the parental murine scFv sequence is the CAR19 construct provided in PCT publication WO2012/079000 and provided herein in Table 15A. In one embodiment, the anti-CD19 binding domain is a scFv described in WO2012/079000 and provided herein in Table 15A.

In one embodiment, the CD19 CAR comprises an amino acid sequence provided as SEQ ID NO: 12 in PCT publication WO2012/079000. In embodiment, the amino acid sequence is:
MALPVTALLLPLALLLHAARPdiqmtqttsslsaslgdrvtiscrasqdiskylnwyqqkpdgtvklliyhtsrlhsgvpsrfsgsgsgtdyslti snleqediatyfcqqgntlpytfgggtkleitggggsggggsggggsevklqesgpglvapsqslsvtctvsgvslpdygvswirqpprkglewlgvi wgsettyynsalksrltiikdnsksqvflkmnslqtddtaiyycakhyyyggsyamdywgqgtsvtvsstttpaprpptpaptiasqplslrpeacrpa aggavhtrgldfacdiyiwaplagtcgvlllslvitlyckrgrkkllyifkqpfmrpvqttqeedgcscrfpeeeeggcelrvkfsrsadapaykqgqnqly nelnlgrreeydvldkrrgrdpemggkprrknpqeglynelqkdkmaeayseigmkgerrrgkghdglyqglstatkdtydalhmqalppr (SEQ ID NO: 891), or a sequence substantially identical thereto (e.g., at least 85%, 90% or 95% or higher identical thereto), with or without the signal peptide sequence indicated in capital letters.

In embodiment, the amino acid sequence is:
diqmtqttsslsaslgdrvtiscrasqdiskylnwyqqkpdgtvklliyhtsrlhsgvpsrfsgsgsgtdysltisnleqediatyfcqqgntlpytfgggtkle itggggsggggsggggsevklqesgpglvapsqslsvtctvsgvslpdygvswirqpprkglewlgviwgsettyynsalksrltiikdnsksqvflkm nslqtddtaiyycakhyyyggsyamdywgqgtsvtvsstttpaprpptpaptiasqplslrpeacrpaaggavhtrgldfacdiyiwaplagtcgvllls Ivitlyckrgrkkllyifkqpfmrpvqttqeedgcscrfpeeeeggcelrvkfsrsadapaykqgqnqlynelnlgrreeydvldkrrgrdpemggkprrk npqeglynelqkdkmaeayseigmkgerrrgkghdglyqglstatkdtydalhmqalppr (SEQ ID NO: 892), or a sequence substantially homologous thereto (e.g., at least 85%, 90% or 95% or higher identical thereto).

In embodiments, the CAR molecule is a CD19 CAR molecule described herein, e.g., a humanized CAR molecule described herein, e.g., a humanized CD19 CAR molecule of Table 15A or having CDRs as set out in Tables 15B and 15C.

In embodiments, the CAR molecule is a CD19 CAR molecule described herein, e.g., a murine CAR molecule described herein, e.g., a murine CD19 CAR molecule of Table 15A or having CDRs as set out in Tables 15B and 15C.

In some embodiments, the CAR molecule comprises one, two, and/or three CDRs from the heavy chain variable region and/or one, two, and/or three CDRs from the light chain variable region of the murine or humanized CD19 CAR of Tables 15B and 15C.

In one embodiment, the antigen binding domain comprises one, two three (e.g., all three) heavy chain CDRs, HC CDR1, HC CDR2 and HC CDR3, from an antibody listed herein, and/or one, two, three (e.g., all three) light chain CDRs, LC CDR1, LC CDR2 and LC CDR3, from an antibody listed herein. In one embodiment, the antigen binding domain comprises a heavy chain variable region and/or a variable light chain region of an antibody listed or described herein.

Exemplary CD19 CARs include any of the CD19 CARs or anti-CD19 binding domains described herein, e.g., in one or more tables (e.g., Table 15A) described herein (e.g., , or an anti-CD19 CAR described in Xu et al. Blood 123.24(2014):3750-9; Kochenderfer et al. Blood 122.25(2013):4129-39, Cruz et al. Blood 122.17(2013):2965-73, NCT00586391, NCT01087294, NCT02456350, NCT00840853, NCT02659943, NCT02650999, NCT02640209, NCT01747486, NCT02546739, NCT02656147, NCT02772198, NCT00709033, NCT02081937, NCT00924326, NCT02735083, NCT02794246, NCT02746952, NCT01593696, NCT02134262, NCT01853631, NCT02443831, NCT02277522, NCT02348216, NCT02614066, NCT02030834, NCT02624258, NCT02625480, NCT02030847, NCT02644655, NCT02349698, NCT02813837, NCT02050347, NCT01683279, NCT02529813, NCT02537977, NCT02799550, NCT02672501, NCT02819583, NCT02028455, NCT01840566, NCT01318317, NCT01864889, NCT02706405, NCT01475058, NCT01430390, NCT02146924, NCT02051257, NCT02431988, NCT01815749, NCT02153580, NCT01865617, NCT02208362, NCT02685670, NCT02535364, NCT02631044, NCT02728882, NCT02735291, NCT01860937, NCT02822326, NCT02737085, NCT02465983, NCT02132624, NCT02782351, NCT01493453, NCT02652910, NCT02247609, NCT01029366, NCT01626495, NCT02721407, NCT01044069, NCT00422383, NCT01680991, NCT02794961, or NCT02456207.

Exemplary CD19 CAR and antigen binding domain constructs that can be used in the methods described herein are shown in Table 15A. The light and heavy chain CDR sequences according to Kabat are shown by the bold and underlined text, and are also summarized in Tables 15A-C. The location of the signal sequence and histidine tag are also underlined. In embodiments, the CD19 CAR sequences and antigen binding fragments thereof do not include the signal sequence and/or histidine tag sequences.

In embodiments, the CD19 CAR comprises an anti- CD19 binding domain (e.g., murine or humanized anti- CD19 binding domain), a transmembrane domain, and an intracellular signaling domain, and wherein said anti- CD19 binding domain comprises a heavy chain complementary determining region 1 (HC CDR1), a heavy chain complementary determining region 2 (HC CDR2), and a heavy chain complementary determining region 3 (HC CDR3) of any anti- CD19 heavy chain binding domain amino acid sequences listed in Table 15A-C, or a sequence at least 85%, 90%, 95% or more identical thereto (e.g., having less than 5, 4, 3, 2 or 1 amino acid substitutions, e.g., conservative substitutions).

In one embodiment, the anti- CD19 binding domain comprises a light chain variable region described herein (e.g., in Table 15A) and/or a heavy chain variable region described herein (e.g., in Table 15A), or a sequence at least 85%, 90%, 95% or more identical thereto.

In one embodiment, the encoded anti- CD19 binding domain is a scFv comprising a light chain and a heavy chain of an amino acid sequence of Tables 5, or a sequence at least 85%, 90%, 95% or more identical thereto.

In an embodiment, the human or humanized anti- CD19 binding domain (e.g., an scFv) comprises: a light chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a light chain variable region provided in Table 15A, or a sequence at least 85%, 90%, 95% or more identical thereto; and/or a heavy chain variable region comprising an amino acid sequence having at least one, two or three modifications (e.g., substitutions, e.g., conservative substitutions) but not more than 30, 20 or 10 modifications (e.g., substitutions, e.g., conservative substitutions) of an amino acid sequence of a heavy chain variable region provided in Table 15A, or a sequence at least 85%, 90%, 95% or more identical thereto.

In one embodiment, the CAR molecule comprises a CD19 antigen binding domain (e.g., a murine, human or humanized antibody or antibody fragment that specifically binds to CD19), a transmembrane domain, and an intracellular signaling domain (e.g., an intracellular signaling domain comprising a costimulatory domain and/or a primary signaling domain).

Exemplary CAR19 molecules are provided in Table 15A (and also Table 7). The CAR molecules in Table 15A comprise a CD19 antigen binding domain, e.g., an amino acid sequence of any CD19 antigen binding domain provided in Table 7 or 10.

**Table 15A. Exemplary CD19 CAR molecules**

| **Name** | **SEQ ID NO:** | **Sequence** |
|---|---|---|
| **CAR 1** | | |
| **CAR1 scFv domain** | 893 | EIVMTQSPATLSLSPGERATLSCRASQDISKYLNWYQQKPGQAPRLLIYH TSRLHSGIPARFSGSGSGTDYTLTISSLQPEDFAVYFCQQGNTLPYTFGQ GTKLEIKGGGGSGGGGSGGGGSQVQLQESGPGLVKPSETLSLTCTVSGVS LPDYGVSWIRQPPGKGLEWIGVIWGSETTYYSSSLKSRVTISKDNSKNQV SLKLSSVTAADTAVYYCAKHYYYGGSYAMDYWGQGTLVTVSS |
| **103101 CAR1 Soluble scFv - nt** | 894 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagccaggtccaact ccaagaaagcggaccgggtcttgtgaagccatcagaaactctttcactgacttgt actgtgagcggagtgtctctccccgattacggggtgtcttggatcagacagccac cggggaagggtctggaatggattggagtgatttggggctctgagactacttacta ctcttcatccctcaagtcacgcgtcaccatctcaaaggacaactctaagaatcag gtgtcactgaaactgtcatctgtgaccgcagccgacaccgccgtgtactattgcg ctaagcattactattatggcgggagctacgcaatggattactggggacagggtac tctggtcaccgtgtccagccaccaccatcatcaccatcaccat |
| **103101 CAR1 Soluble scFv** | 895 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsqvqlqesgpglvkpsetlsltc tvsgvslpdygvswirqppgkglewigviwgsettyysssslksrvtiskdnskn q vslklssvtaadtavyycakhyyyggsyamdywgqgtlvtvsshhhhhhhh |
| **- aa** | | |
| **104875 CAR 1- Full - nt** | 896 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagccaggtccaact ccaagaaagcggaccgggtcttgtgaagccatcagaaactctttcactgacttgt actgtgagcggagtgtctctccccgattacggggtgtcttggatcagacagccac cggggaagggtctggaatggattggagtgatttggggctctgagactacttacta ctcttcatccctcaagtcacgcgtcaccatctcaaaggacaactctaagaatcag gtgtcactgaaactgtcatctgtgaccgcagccgacaccgccgtgtactattgcg ctaagcattactattatggcgggagctacgcaatggattactggggacagggtac tctggtcaccgtgtccagcaccactaccccagcaccgaggccacccaccccggct cctaccatcgcctcccagcctctgtccctgcgtccggaggcatgtagacccgcag ctggtggggccgtgcatacccggggtcttgacttcgcctgcgatatctacatttg ggcccctctggctggtacttgcggggtcctgctgctttcactcgtgatcactctt tactgtaagcgcggtcggaagaagctgctgtacatctttaagcaacccttcatga ggcctgtgcagactactcaagaggaggacggctgttcatgccggttcccagagga ggaggaaggcggctgcgaactgcgcgtgaaattcagccgcagcgcagatgctcca gcctacaagcaggggcagaaccagctctacaacgaactcaatcttggtcggagag aggagtacgacgtgctggacaagcggagaggacgggacccagaaatgggcgggaa gccgcgcagaaagaatccccaagagggcctgtacaacgagctccaaaaggataag atggcagaagcctatagcgagattggtatgaaaggggaacgcagaagaggcaaag gccacgacggactgtaccagggactcagcaccgccaccaaggacacctatgacgc tcttcacatgcaggccctgccgcctcgg |
| **104875 CAR 1- Full - aa** | 897 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsqvqlqesgpglvkpsetlsltc tvsgvslpdygvswirqppgkglewigviwgsettyyssslksrvtiskdnsknq vslklssvtaadtavyycakhyyyggsyamdywgqgtlvtvssttpaprpptpa ptiasqplslrpeacrpaaggavhtrgldfacdiyiwaplagtcgvlllslvitl yckrgrkkllyifkqpfmrpvqttqeedgcscrfpeeeeggcelrvkfsrsadap aykqgqnqlynelnlgrreeydvldkrrgrdpemggkprrknpqeglynelqkdk maeayseigmkgerrrgkghdglyqglstatkdtydalhmqalppr |

| **CAR 2** | | |
|---|---|---|
| **CAR2 scFv domain** | 898 | eivmtqspatlslspgeratlscrasqdiskylnwyqqkpgqaprlliyhtsrlh sgiparfsgsgsgtdytltisslqpedfavyfcqqgntlpytfgqgtkleikggg gsggggsggggsqvqlqesgpglvkpsetlsltctvsgvslpdygvswirqppgk glewigviwgsettyyqsslksrvtiskdnsknqvslklssvtaadtavyycakh yyyggsyamdywgqgtlvtvss |
| **103102** | 899 | atggccctccctgtcaccgccctgctgcttccgctggctcttctgctccacgccg ctcggcccgaaattgtgatgacccagtcacccgccactcttagcctttcacccgg |
| **CAR2 - Soluble scFv - nt** | | tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagccaggtccaact ccaagaaagcggaccgggtcttgtgaagccatcagaaactctttcactgacttgt actgtgagcggagtgtctctccccgattacggggtgtcttggatcagacagccac cggggaagggtctggaatggattggagtgatttggggctctgagactacttacta ccaatcatccctcaagtcacgcgtcaccatctcaaaggacaactctaagaatcag gtgtcactgaaactgtcatctgtgaccgcagccgacaccgccgtgtactattgcg ctaagcattactattatggcgggagctacgcaatggattactggggacagggtac tctggtcaccgtgtccagccaccaccatcatcaccatcaccat |
| **103102 CAR2 - Soluble scFv - aa** | 900 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsqvqlqesgpglvkpsetlsltc tvsgvslpdygvswirqppgkglewigviwgsettyyqsslksrvtiskdnsknq vslklssvtaadtavyycakhyyyggsyamdywgqgtlvtvsshhhhhhhh |
| **104876 CAR 2 - Full - nt** | 901 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagccaggtccaact ccaagaaagcggaccgggtcttgtgaagccatcagaaactctttcactgacttgt actgtgagcggagtgtctctccccgattacggggtgtcttggatcagacagccac cggggaagggtctggaatggattggagtgatttggggctctgagactacttacta ccaatcatccctcaagtcacgcgtcaccatctcaaaggacaactctaagaatcag gtgtcactgaaactgtcatctgtgaccgcagccgacaccgccgtgtactattgcg ctaagcattactattatggcgggagctacgcaatggattactggggacagggtac tctggtcaccgtgtccagcaccactaccccagcaccgaggccacccaccccggct cctaccatcgcctcccagcctctgtccctgcgtccggaggcatgtagacccgcag ctggtggggccgtgcatacccggggtcttgacttcgcctgcgatatctacatttg ggcccctctggctggtacttgcggggtcctgctgctttcactcgtgatcactctt tactgtaagcgcggtcggaagaagctgctgtacatctttaagcaacccttcatga ggcctgtgcagactactcaagaggaggacggctgttcatgccggttcccagagga ggaggaaggcggctgcgaactgcgcgtgaaattcagccgcagcgcagatgctcca gcctacaagcaggggcagaaccagctctacaacgaactcaatcttggtcggagag aggagtacgacgtgctggacaagcggagaggacgggacccagaaatgggcgggaa gccgcgcagaaagaatccccaagagggcctgtacaacgagctccaaaaggataag atggcagaagcctatagcgagattggtatgaaaggggaacgcagaagaggcaaag gccacgacggactgtaccagggactcagcaccgccaccaaggacacctatgacgc tcttcacatgcaggccctgccgcctcgg |
| **104876 CAR 2 - Full - aa** | 902 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsqvqlqesgpglvkpsetlsltc tvsgvslpdygvswirqppgkglewigviwgsettyyqsslksrvtiskdnsknq vslklssvtaadtavyycakhyyyggsyamdywgqgtlvtvsstttpaprpptpa ptiasqplslrpeacrpaaggavhtrgldfacdiyiwaplagtcgvlllslvitl yckrgrkkllyifkqpfmrpvqttqeedgcscrfpeeeeggcelrvkfsrsadap aykqgqnqlynelnlgrreeydvldkrrgrdpemggkprrknpqeglynelqkdk maeayseigmkgerrrgkghdglyqglstatkdtydalhmqalppr |

| **CAR 3** | | |
|---|---|---|
| **CARS scFv domain** | 903 | qvqlqesgpglvkpsetlsltctvsgvslpdygvswirqppgkglewigviwgse ttyysssslksrvtiskdnsknqvslklssvtaadtavyycakhyyyggsyamdyw gqgtlvtvssggggsggggsggggseivmtqspatlslspgeratlscrasqdis kylnwyqqkpgqaprlliyhtsrlhsgiparfsgsgsgtdytltisslqpedfav yfcqqgntlpytfgqgtkleik |
| **103104 CAR 3 - Soluble scfv** - **nt** | 904 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactattcatcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccgaaatcgtgatgacccagagccctgca accctgtccctttctcccggggaacgggctaccctttcttgtcgggcatcacaag atatctcaaaatacctcaattggtatcaacagaagccgggacaggcccctaggct tcttatctaccacacctctcgcctgcatagcgggattcccgcacgctttagcggg tctggaagcgggaccgactacactctgaccatctcatctctccagcccgaggact tcgccgtctacttctgccagcagggtaacaccctgccgtacaccttcggccaggg caccaagcttgagatcaaacatcaccaccatcatcaccatcac |
| **103104 CAR 3 - Soluble scFv - aa** | 905 | MALPVTALLLPLALLLHAARPqvqlqesgpglvkpsetlsltctvsgvslpdygv swirqppgkglewigviwgsettyyssslksrvtiskdnsknqvslklssvtaad tavyycakhyyyggsyamdywgqgtlvtvssggggsggggsggggseivmtqspa tlslspgeratlscrasqdiskylnwyqqkpgqaprlliyhtsrlhsgiparfsg sgsgtdytltisslqpedfavyfcqqgntlpytfgqgtkleikhhhhhhhh |
| **104877 CAR 3 - Full - nt** | 906 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactattcatcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccgaaatcgtgatgacccagagccctgca |
| | | accctgtccctttctcccggggaacgggctaccctttcttgtcgggcatcacaag atatctcaaaatacctcaattggtatcaacagaagccgggacaggcccctaggct tcttatctaccacacctctcgcctgcatagcgggattcccgcacgctttagcggg tctggaagcgggaccgactacactctgaccatctcatctctccagcccgaggact tcgccgtctacttctgccagcagggtaacaccctgccgtacaccttcggccaggg caccaagcttgagatcaaaaccactactcccgctccaaggccacccacccctgcc ccgaccatcgcctctcagccgctttccctgcgtccggaggcatgtagacccgcag ctggtggggccgtgcatacccggggtcttgacttcgcctgcgatatctacatttg ggcccctctggctggtacttgcggggtcctgctgctttcactcgtgatcactctt tactgtaagcgcggtcggaagaagctgctgtacatctttaagcaacccttcatga ggcctgtgcagactactcaagaggaggacggctgttcatgccggttcccagagga ggaggaaggcggctgcgaactgcgcgtgaaattcagccgcagcgcagatgctcca gcctacaagcaggggcagaaccagctctacaacgaactcaatcttggtcggagag aggagtacgacgtgctggacaagcggagaggacgggacccagaaatgggcgggaa gccgcgcagaaagaatccccaagagggcctgtacaacgagctccaaaaggataag atggcagaagcctatagcgagattggtatgaaaggggaacgcagaagaggcaaag gccacgacggactgtaccagggactcagcaccgccaccaaggacacctatgacgc tcttcacatgcaggccctgccgcctcgg |
| **104877 CAR 3 - Full - aa** | 907 | MALPVTALLLPLALLLHAARPqvqlqesgpglvkpsetlsltctvsgvslpdygv swirqppgkglewigviwgsettyyssslksrvtiskdnsknqvslklssvtaad tavyycakhyyyggsyamdywgqgtlvtvssggggsggggsggggseivmtqspa tlslspgeratlscrasqdiskylnwyqqkpgqaprlliyhtsrlhsgiparfsg sgsgtdytltisslqpedfavyfcqqgntlpytfgqgtkleikttttpaprpptpa ptiasqplslrpeacrpaaggavhtrgldfacdiyiwaplagtcgvlllslvitl yckrgrkkllyifkqpfmrpvqttqeedgcscrfpeeeeggcelrvkfsrsadap aykqgqnqlynelnlgrreeydvldkrrgrdpemggkprrknpqeglynelqkdk maeayseigmkgerrrgkghdglyqglstatkdtydalhmqalppr |

| **CAR 4** | | |
|---|---|---|
| **CAR4 scFv domain** | 908 | qvqlqesgpglvkpsetlsltctvsgvslpdygvswirqppgkglewigviwgse ttyyqsslksrvtiskdnsknqvslklssvtaadtavyycakhyyyggsyamdyw gqgtlvtvssggggsggggsggggseivmtqspatlslspgeratlscrasqdis kylnwyqqkpgqaprlliyhtsrlhsgiparfsgsgsgtdytltisslqpedfav yfcqqgntlpytfgqgtkleik |
| **103106 CAR4 - Soluble scFv - nt** | 909 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactatcaatcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccgaaatcgtgatgacccagagccctgca accctgtccctttctcccggggaacgggctaccctttcttgtcgggcatcacaag atatctcaaaatacctcaattggtatcaacagaagccgggacaggcccctaggct tcttatctaccacacctctcgcctgcatagcgggattcccgcacgctttagcggg tctggaagcgggaccgactacactctgaccatctcatctctccagcccgaggact |
| | | tcgccgtctacttctgccagcagggtaacaccctgccgtacaccttcggccaggg caccaagcttgagatcaaacatcaccaccatcatcaccatcac |
| **103106 CAR4 - Soluble scFv -aa** | 910 | MALPVTALLLPLALLLHAARPqvqlqesgpglvkpsetlsltctvsgvslpdygv swirqppgkglewigviwgsettyyqsslksrvtiskdnsknqvslklssvtaad tavyycakhyyyggsyamdywgqgtlvtvssggggsggggsggggseivmtqspa tlslspgeratlscrasqdiskylnwyqqkpgqaprlliyhtsrlhsgiparfsg sgsgtdytltisslqpedfavyfcqqgntlpytfgqgtkleikhhhhhhhh |
| **104878 CAR 4- Full - nt** | 911 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactatcaatcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccgaaatcgtgatgacccagagccctgca accctgtccctttctcccggggaacgggctaccctttcttgtcgggcatcacaag atatctcaaaatacctcaattggtatcaacagaagccgggacaggcccctaggct tcttatctaccacacctctcgcctgcatagcgggattcccgcacgctttagcggg tctggaagcgggaccgactacactctgaccatctcatctctccagcccgaggact tcgccgtctacttctgccagcagggtaacaccctgccgtacaccttcggccaggg caccaagcttgagatcaaaaccactactcccgctccaaggccacccacccctgcc ccgaccatcgcctctcagccgctttccctgcgtccggaggcatgtagacccgcag ctggtggggccgtgcatacccggggtcttgacttcgcctgcgatatctacatttg ggcccctctggctggtacttgcggggtcctgctgctttcactcgtgatcactctt tactgtaagcgcggtcggaagaagctgctgtacatctttaagcaacccttcatga ggcctgtgcagactactcaagaggaggacggctgttcatgccggttcccagagga ggaggaaggcggctgcgaactgcgcgtgaaattcagccgcagcgcagatgctcca gcctacaagcaggggcagaaccagctctacaacgaactcaatcttggtcggagag aggagtacgacgtgctggacaagcggagaggacgggacccagaaatgggcgggaa gccgcgcagaaagaatccccaagagggcctgtacaacgagctccaaaaggataag atggcagaagcctatagcgagattggtatgaaaggggaacgcagaagaggcaaag gccacgacggactgtaccagggactcagcaccgccaccaaggacacctatgacgc tcttcacatgcaggccctgccgcctcgg |
| **104878 CAR 4- Full - aa** | 912 | MALPVTALLLPLALLLHAARPqvqlqesgpglvkpsetlsltctvsgvslpdygv swirqppgkglewigviwgsettyyqsslksrvtiskdnsknqvslklssvtaad tavyycakhyyyggsyamdywgqgtlvtvssggggsggggsggggseivmtqspa tlslspgeratlscrasqdiskylnwyqqkpgqaprlliyhtsrlhsgiparfsg sgsgtdytltisslqpedfavyfcqqgntlpytfgqgtkleikttttpaprpptpa ptiasqplslrpeacrpaaggavhtrg_dfacdiyiwaplagtcgvlllslvitl yckrgrkkllyifkqpfmrpvqttqeedgcscrfpeeeeggcelrvkfsrsadap aykqgqnqlynelnlgrreeydvldkrrgrdpemggkprrknpqeglynelqkdk maeayseigmkgerrrgkghdglyqglstatkdtydalhmqalppr |

| **CAR 5** | | |
|---|---|---|
| **CARS scFv domain** | 913 | eivmtqspatlslspgeratlscrasqdiskylnwyqqkpgqaprlliyhtsrlh sgiparfsgsgsgtdytltisslqpedfavyfcqqgntlpytfgqgtkleikggg gsggggsggggsggggsqvqlqesgpglvkpsetlsltctvsgvslpdygvswir qppgkglewigviwgsettyyssslksrvtiskdnsknqvslklssvtaadtavy ycakhyyyggsyamdywgqgtlvtvss |
| **99789 CARS - Soluble scFv - nt** | 914 | atggccctcccagtgaccgctctgctgctgcctctcgcacttcttctccatgccg ctcggcctgagatcgtcatgacccaaagccccgctaccctgtccctgtcacccgg cgagagggcaaccctttcatgcagggccagccaggacatttctaagtacctcaac tggtatcagcagaagccagggcaggctcctcgcctgctgatctaccacaccagcc gcctccacagcggtatccccgccagattttccgggagcgggtctggaaccgacta caccctcaccatctcttctctgcagcccgaggatttcgccgtctatttctgccag caggggaatactctgccgtacaccttcggtcaaggtaccaagctggaaatcaagg gaggcggaggatcaggcggtggcggaagcggaggaggtggctccggaggaggagg ttcccaagtgcagcttcaagaatcaggacccggacttgtgaagccatcagaaacc ctctccctgacttgtaccgtgtccggtgtgagcctccccgactacggagtctctt ggattcgccagcctccggggaagggtcttgaatggattggggtgatttggggatc agagactacttactactcttcatcacttaagtcacgggtcaccatcagcaaagat aatagcaagaaccaagtgtcacttaagctgtcatctgtgaccgccgctgacaccg ccgtgtactattgtgccaaacattactattacggagggtcttatgctatggacta ctggggacaggggaccctggtgactgtctctagccatcaccatcaccaccatcat cac |
| **99789 CARS - Soluble scFv -aa** | 915 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsggggsqvqlqesgpglvkpset lsltctvsgvslpdygvswirqppgkg[?]ewigviwgsettyyssslksrvtiskd nsknqvslklssvtaadtavyycakhyyyggsyamdywgqgtlvtvsshhhhhh h |
| **104879 CAR 5 - Full - nt** | 916 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caaggaaacaccctgccctacacctttggacagggcaccaagctcgagattaaag gtggaggtggcagcggaggaggtgggtccggcggtggaggaagcggcggaggcgg gagccaggtccaactccaagaaagcggaccgggtcttgtgaagccatcagaaact ctttcactgacttgtactgtgagcggagtgtctctccccgattacggggtgtctt ggatcagacagccaccggggaagggtctggaatggattggagtgatttggggctc tgagactacttactactcttcatccctcaagtcacgcgtcaccatctcaaaggac aactctaagaatcaggtgtcactgaaactgtcatctgtgaccgcagccgacaccg ccgtgtactattgcgctaagcattactattatggcgggagctacgcaatggatta ctggggacagggtactctggtcaccgtgtccagcaccactaccccagcaccgagg ccacccaccccggctcctaccatcgcctcccagcctctgtccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg |
| | | ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **104879 CAR 5 - Full - aa** | 917 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsggggsqvqlqesgpglvkpset lsltctvsgvslpdygvswirqppgkglewigviwgsettyyssslksrvtiskd nsknqvslklssvtaadtavyycakhyyyggsyamdywgqgtlvtvssttpapr pptpaptiasqplslrpeacrpaaggavhtrgldfacdiyiwaplagtcgvllls lvitlyckrgrkkllyifkqpfmrpvqttqeedgcscrfpeeeeggcelrvkfsr sadapaykqgqnqlynelnlgrreeydvldkrrgrdpemggkprrknpqeglyne lqkdkmaeayseigmkgerrrgkghdglyqglstatkdtydalhmqalppr |

| **CAR 6** | | |
|---|---|---|
| **CAR6 scFv domain** | 918 | eivmtqspatlslspgeratlscrasqdiskylnwyqqkpgqaprlliyhtsrlh sgiparfsgsgsgtdytltisslqpedfavyfcqqgntlpytfgqgtkleikggg gsggggsggggsggggsqvqlqesgpglvkpsetlsltctvsgvslpdygvswir qppgkglewigviwgsettyyqsslksrvtiskdnsknqvslklssvtaadtavy ycakhyyyggsyamdywgqgtlvtvss |
| **99790 CAR6-Soluble scFv - nt** | 919 | atggccctcccagtgaccgctctgctgctgcctctcgcacttcttctccatgccg ctcggcctgagatcgtcatgacccaaagccccgctaccctgtccctgtcacccgg cgagagggcaaccctttcatgcagggccagccaggacatttctaagtacctcaac tggtatcagcagaagccagggcaggctcctcgcctgctgatctaccacaccagcc gcctccacagcggtatccccgccagattttccgggagcgggtctggaaccgacta caccctcaccatctcttctctgcagcccgaggatttcgccgtctatttctgccag caggggaatactctgccgtacaccttcggtcaaggtaccaagctggaaatcaagg gaggcggaggatcaggcggtggcggaagcggaggaggtggctccggaggaggagg ttcccaagtgcagcttcaagaatcaggacccggacttgtgaagccatcagaaacc ctctccctgacttgtaccgtgtccggtgtgagcctccccgactacggagtctctt ggattcgccagcctccggggaagggtcttgaatggattggggtgatttggggatc agagactacttactaccagtcatcacttaagtcacgggtcaccatcagcaaagat aatagcaagaaccaagtgtcacttaagctgtcatctgtgaccgccgctgacaccg ccgtgtactattgtgccaaacattactattacggagggtcttatgctatggacta ctggggacaggggaccctggtgactgtctctagccatcaccatcaccaccatcat cac |
| **99790 CAR6-Soluble scFv - aa** | 920 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsggggsqvqlqesgpglvkpset lsltctvsgvslpdygvswirqppgkg_ewigviwgsettyyqsslksrvtiskd nsknqvslklssvtaadtavyycakhyyyggsyamdywgqgtlvtvsshhhhhh h |
| **104880 CAR6 - Full - nt** | 921 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagcggaggcggagg gagccaggtccaactccaagaaagcggaccgggtcttgtgaagccatcagaaact ctttcactgacttgtactgtgagcggagtgtctctccccgattacggggtgtctt ggatcagacagccaccggggaagggtctggaatggattggagtgatttggggctc tgagactacttactaccaatcatccctcaagtcacgcgtcaccatctcaaaggac aactctaagaatcaggtgtcactgaaactgtcatctgtgaccgcagccgacaccg ccgtgtactattgcgctaagcattactattatggcgggagctacgcaatggatta ctggggacagggtactctggtcaccgtgtccagcaccactaccccagcaccgagg ccacccaccccggctcctaccatcgcctcccagcctctgtccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **104880 CAR6 - Full - aa** | 922 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsggggsqvqlqesgpglvkpset lsltctvsgvslpdygvswirqppgkglewigviwgsettyyqsslksrvtiskd nsknqvslklssvtaadtavyycakhyyyggsyamdywgqgtlvtvsstttpapr pptpaptiasqplslrpeacrpaaggavhtrgldfacdiyiwaplagtcgvllls lvitlyckrgrkkllyifkqpfmrpvqttqeedgcscrfpeeeeggcelrvkfsr sadapaykqgqnqlynelnlgrreeydvldkrrgrdpemggkprrknpqeglyne lqkdkmaeayseigmkgerrrgkghdglyqglstatkdtydalhmqalppr |

| **CAR 7** | | |
|---|---|---|
| **CAR7 scFv domain** | 923 | qvqlqesgpglvkpsetlsltctvsgvslpdygvswirqppgkglewigviwgse ttyysssslksrvtiskdnsknqvslklssvtaadtavyycakhyyyggsyamdyw gqgtlvtvssggggsggggsggggsggggseivmtqspatlslspgeratlscra sqdiskylnwyqqkpgqaprlliyhtsrlhsgiparfsgsgsgtdytltisslqp edfavyfcqqgntlpytfgqgtkleik |
| **100796 CAR7 -** | 924 | atggcactgcctgtcactgccctcctgctgcctctggccctccttctgcatgccg ccaggccccaagtccagctgcaagagtcaggacccggactggtgaagccgtctga gactctctcactgacttgtaccgtcagcggcgtgtccctccccgactacggagtg tcatggatccgccaacctcccgggaaagggcttgaatggattggtgtcatctggg |
| **Soluble scFv** - **nt** | | gttctgaaaccacctactactcatcttccctgaagtccagggtgaccatcagcaa ggataattccaagaaccaggtcagccttaagctgtcatctgtgaccgctgctgac accgccgtgtattactgcgccaagcactactattacggaggaagctacgctatgg actattggggacagggcactctcgtgactgtgagcagcggcggtggagggtctgg aggtggaggatccggtggtgggtcaggcggaggagggagcgagattgtgatg actcagtcaccagccaccctttctctttcacccggcgagagagcaaccctgagct gtagagccagccaggacatttctaagtacctcaactggtatcagcaaaaaccggg gcaggcccctcgcctcctgatctaccatacctcacgccttcactctggtatcccc gctcggtttagcggatcaggatctggtaccgactacactctgaccatttccagcc tgcagccagaagatttcgcagtgtatttctgccagcagggcaatacccttcctta caccttcggtcagggaaccaagctcgaaatcaagcaccatcaccatcatcaccac cat |
| **100796 CAR7 - Soluble scFv - aa** | 925 | MALPVTALLLPLALLLHAARPqvqlqesgpglvkpsetlsltctvsgvslpdygv swirqppgkglewigviwgsettyysslksrvtiskdnsknqvslklssvtaad tavyycakhyyyggsyamdywgqgtlvtvssggggsggggsggggsggggseivm tqspatlslspgeratlscrasqdiskylnwyqqkpgqaprlliyhtsrlhsgip arfsgsgsgtdytltisslqpedfavyfcqqgntlpytfgqgtkleikhhhhhh h |
| **104881 CAR 7 Full - nt** | 926 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactattcatcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccggaggtggcggaagcgaaatcgtgatg acccagagccctgcaaccctgtccctttctcccggggaacgggctaccctttctt gtcgggcatcacaagatatctcaaaatacctcaattggtatcaacagaagccggg acaggcccctaggcttcttatctaccacacctctcgcctgcatagcgggattccc gcacgctttagcgggtctggaagcgggaccgactacactctgaccatctcatctc tccagcccgaggacttcgccgtctacttctgccagcagggtaacaccctgccgta caccttcggccagggcaccaagcttgagatcaaaaccactactcccgctccaagg ccacccacccctgccccgaccatcgcctctcagccgctttccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **104881** | 927 | MALPVTALLLPLALLLHAARPqvqlqesgpglvkpsetlsltctvsgvslpdygv swirqppgkglewigviwgsettyyssslksrvtiskdnsknqvslklssvtaad |
| **CAR 7 Full** - **aa** | | tavyycakhyyyggsyamdywgqgtlvtvssggggsggggsggggsggggseivm tqspatlslspgeratlscrasqdiskylnwyqqkpgqaprlliyhtsrlhsgip arfsgsgsgtdytltisslqpedfavyfcqqgntlpytfgqgtkleikttttpapr pptpaptiasqplslrpeacrpaaggavhtrgldfacdiyiwaplagtcgvllls lvitlyckrgrkkllyifkqpfmrpvqttqeedgcscrfpeeeeggcelrvkfsr sadapaykqgqnqlynelnlgrreeydvldkrrgrdpemggkprrknpqeglyne lqkdkmaeayseigmkgerrrgkghdglyqglstatkdtydalhmqalppr |

| **CAR 8** | | |
|---|---|---|
| **CAR8 scFv domain** | 928 | qvqlqesgpglvkpsetlsltctvsgvslpdygvswirqppgkglewigviwgse ttyyqsslksrvtiskdnsknqvslklssvtaadtavyycakhyyyggsyamdyw gqgtlvtvssggggsggggsggggsggggseivmtqspatlslspgeratlscra sqdiskylnwyqqkpgqaprlliyhtsrlhsgiparfsgsgsgtdytltisslqp edfavyfcqqgntlpytfgqgtkleik |
| **100798 CAR8 - Soluble scFv** - **nt** | 929 | atggcactgcctgtcactgccctcctgctgcctctggccctccttctgcatgccg ccaggccccaagtccagctgcaagagtcaggacccggactggtgaagccgtctga gactctctcactgacttgtaccgtcagcggcgtgtccctccccgactacggagtg tcatggatccgccaacctcccgggaaagggcttgaatggattggtgtcatctggg gttctgaaaccacctactaccagtcttccctgaagtccagggtgaccatcagcaa ggataattccaagaaccaggtcagccttaagctgtcatctgtgaccgctgctgac accgccgtgtattactgcgccaagcactactattacggaggaagctacgctatgg actattggggacagggcactctcgtgactgtgagcagcggcggtggagggtctgg aggtggaggatccggtggtggtgggtcaggcggaggagggagcgagattgtgatg actcagtcaccagccaccctttctctttcacccggcgagagagcaaccctgagct gtagagccagccaggacatttctaagtacctcaactggtatcagcaaaaaccggg gcaggcccctcgcctcctgatctaccatacctcacgccttcactctggtatcccc gctcggtttagcggatcaggatctggtaccgactacactctgaccatttccagcc tgcagccagaagatttcgcagtgtatttctgccagcagggcaatacccttcctta caccttcggtcagggaaccaagctcgaaatcaagcaccatcaccatcatcatcac cac |
| **100798 CAR8 - Soluble scFv - aa** | 930 | MALPVTALLLPLALLLHAARPqvqlqesgpglvkpsetlsltctvsgvslpdygv swirqppgkglewigviwgsettyyqsslksrvtiskdnsknqvslklssvtaad tavyycakhyyyggsyamdywgqgtlvtvssggggsggggsggggsggggseivm tqspatlslspgeratlscrasqdiskylnwyqqkpgqaprlliyhtsrlhsgip arfsgsgsgtdytltisslqpedfavyfcqqgntlpytfgqgtkleikhhhhhh h |
| **104882 CAR 8-Full-nt** | 931 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactatcaatcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccggaggcggtgggtcagaaatcgtgatg acccagagccctgcaaccctgtccctttctcccggggaacgggctaccctttctt |
| | | gtcgggcatcacaagatatctcaaaatacctcaattggtatcaacagaagccggg acaggcccctaggcttcttatctaccacacctctcgcctgcatagcgggattccc gcacgctttagcgggtctggaagcgggaccgactacactctgaccatctcatctc tccagcccgaggacttcgccgtctacttctgccagcagggtaacaccctgccgta caccttcggccagggcaccaagcttgagatcaaaaccactactcccgctccaagg ccacccacccctgccccgaccatcgcctctcagccgctttccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **104882 CAR 8 - Full - aa** | 932 | MALPVTALLLPLALLLHAARPqvqlqesgpglvkpsetlsltctvsgvslpdygv swirqppgkglewigviwgsettyyqsslksrvtiskdnsknqvslklssvtaad tavyycakhyyyggsyamdywgqgtlvtvssggggsggggsggggsggggseivm tqspatlslspgeratlscrasqdiskylnwyqqkpgqaprlliyhtsrlhsgip arfsgsgsgtdytltisslqpedfavyfcqqgntlpytfgqgtkleikttttpapr pptpaptiasqplslrpeacrpaaggavhtrgldfacdiyiwaplagtcgvllls lvitlyckrgrkkllyifkqpfmrpvqttqeedgcscrfpeeeeggcelrvkfsr sadapaykqgqnqlynelnlgrreeydvldkrrgrdpemggkprrknpqeglyne lqkdkmaeayseigmkgerrrgkghdglyqglstatkdtydalhmqalppr |

| **CAR 9** | | |
|---|---|---|
| **CAR9 scFv domain** | 933 | eivmtqspatlslspgeratlscrasqdiskylnwyqqkpgqaprlliyhtsrlh sgiparfsgsgsgtdytltisslqpedfavyfcqqgntlpytfgqgtkleikggg gsggggsggggsggggsqvqlqesgpglvkpsetlsltctvsgvslpdygvswir qppgkglewigviwgsettyynsslksrvtiskdnsknqvslklssvtaadtavy ycakhyyyggsyamdywgqgtlvtvss |
| **99789 CAR9 - Soluble scFv** - **nt** | 934 | atggccctcccagtgaccgctctgctgctgcctctcgcacttcttctccatgccg ctcggcctgagatcgtcatgacccaaagccccgctaccctgtccctgtcacccgg cgagagggcaaccctttcatgcagggccagccaggacatttctaagtacctcaac tggtatcagcagaagccagggcaggctcctcgcctgctgatctaccacaccagcc gcctccacagcggtatccccgccagattttccgggagcgggtctggaaccgacta caccctcaccatctcttctctgcagcccgaggatttcgccgtctatttctgccag caggggaatactctgccgtacaccttcggtcaaggtaccaagctggaaatcaagg gaggcggaggatcaggcggtggcggaagcggaggaggtggctccggaggaggagg ttcccaagtgcagcttcaagaatcaggacccggacttgtgaagccatcagaaacc ctctccctgacttgtaccgtgtccggtgtgagcctccccgactacggagtctctt ggattcgccagcctccggggaagggtcttgaatggattggggtgatttggggatc agagactacttactacaattcatcacttaagtcacgggtcaccatcagcaaagat aatagcaagaaccaagtgtcacttaagctgtcatctgtgaccgccgctgacaccg ccgtgtactattgtgccaaacattactattacggagggtcttatgctatggacta |
| | | ctggggacaggggaccctggtgactgtctctagccatcaccatcaccaccatcat cac |
| **99789 CAR9 - Soluble scFv - aa** | 935 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsggggsqvqlqesgpglvkpset lsltctvsgvslpdygvswirqppgkg[?]ewigviwgsettyynsslksrvtiskd nsknqvslklssvtaadtavyycakhyyyggsyamdywgqgtlvtvsshhhhhh h |
| **105974 CAR 9 - Full - nt** | 936 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagcggaggcggtgg gagccaggtccaactccaagaaagcggaccgggtcttgtgaagccatcagaaact ctttcactgacttgtactgtgagcggagtgtctctccccgattacggggtgtctt ggatcagacagccaccggggaagggtctggaatggattggagtgatttggggctc tgagactacttactacaactcatccctcaagtcacgcgtcaccatctcaaaggac aactctaagaatcaggtgtcactgaaactgtcatctgtgaccgcagccgacaccg ccgtgtactattgcgctaagcattactattatggcgggagctacgcaatggatta ctggggacagggtactctggtcaccgtgtccagcaccactaccccagcaccgagg ccacccaccccggctcctaccatcgcctcccagcctctgtccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **105974 CAR 9 - Full - aa** | 937 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsggggsqvqlqesgpglvkpset lsltctvsgvslpdygvswirqppgkglewigviwgsettyynsslksrvtiskd nsknqvslklssvtaadtavyycakhyyyggsyamdywgqgtlvtvsstttpapr pptpaptiasqplslrpeacrpaaggavhtrgldfacdiyiwaplagtcgvllls lvitlyckrgrkkllyifkqpfmrpvqttqeedgcscrfpeeeeggcelrvkfsr sadapaykqgqnqlynelnlgrreeydvldkrrgrdpemggkprrknpqeglyne lqkdkmaeayseigmkgerrrgkghdglyqglstatkdtydalhmqalppr |

| **CAR10** | | |
|---|---|---|
| **CAR10 scFv domain** | 938 | qvqlqesgpglvkpsetlsltctvsgvslpdygvswirqppgkglewigviwgse ttyynsslksrvtiskdnsknqvslklssvtaadtavyycakhyyyggsyamdyw gqgtlvtvssggggsggggsggggsggggseivmtqspatlslspgeratlscra sqdiskylnwyqqkpgqaprlliyhtsrlhsgiparfsgsgsgtdytltisslqp edfavyfcqqgntlpytfgqgtkleik |
| **100796 CAR10 - Soluble scFv - nt** | 939 | atggcactgcctgtcactgccctcctgctgcctctggccctccttctgcatgccg ccaggccccaagtccagctgcaagagtcaggacccggactggtgaagccgtctga gactctctcactgacttgtaccgtcagcggcgtgtccctccccgactacggagtg tcatggatccgccaacctcccgggaaagggcttgaatggattggtgtcatctggg gttctgaaaccacctactacaactcttccctgaagtccagggtgaccatcagcaa ggataattccaagaaccaggtcagccttaagctgtcatctgtgaccgctgctgac accgccgtgtattactgcgccaagcactactattacggaggaagctacgctatgg actattggggacagggcactctcgtgactgtgagcagcggcggtggagggtctgg aggtggaggatccggtggtggtgggtcaggcggaggagggagcgagattgtgatg actcagtcaccagccaccctttctctttcacccggcgagagagcaaccctgagct gtagagccagccaggacatttctaagtacctcaactggtatcagcaaaaaccggg gcaggcccctcgcctcctgatctaccatacctcacgccttcactctggtatcccc gctcggtttagcggatcaggatctggtaccgactacactctgaccatttccagcc tgcagccagaagatttcgcagtgtatttctgccagcagggcaatacccttcctta caccttcggtcagggaaccaagctcgaaatcaagcaccatcaccatcatcaccac cat |
| **100796 CAR10 - Soluble scFv - aa** | 940 | MALPVTALLLPLALLLHAARPqvqlqesgpglvkpsetlsltctvsgvslpdygv swirqppgkglewigviwgsettyynsslksrvtiskdnsknqvslklssvtaad tavyycakhyyyggsyamdywgqgtlvtvssggggsggggsggggsggggseivm tqspatlslspgeratlscrasqdiskylnwyqqkpgqaprlliyhtsrlhsgip arfsgsgsgtdytltisslqpedfavyfcqqgntlpytfgqgtkleikhhhhhh h |
| **105975 CAR 10 Full - nt** | 941 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagcggaggcggtgg gagccaggtccaactccaagaaagcggaccgggtcttgtgaagccatcagaaact ctttcactgacttgtactgtgagcggagtgtctctccccgattacggggtgtctt ggatcagacagccaccggggaagggtctggaatggattggagtgatttggggctc tgagactacttactacaactcatccctcaagtcacgcgtcaccatctcaaaggac aactctaagaatcaggtgtcactgaaactgtcatctgtgaccgcagccgacaccg ccgtgtactattgcgctaagcattactattatggcgggagctacgcaatggatta ctggggacagggtactctggtcaccgtgtccagcaccactaccccagcaccgagg ccacccaccccggctcctaccatcgcctcccagcctctgtccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg |
| | | ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **105975 CAR 10 Full** - **aa** | 942 | MALPVTALLLPLALLLHAARPEIVMTQSPATLSLSPGERATLSCRASQDISKYLN WYQQKPGQAPRLLIYHTSRLHSGIPARFSGSGSGTDYTLTISSLQPEDFAVYFCQ QGNTLPYTFGQGTKLEIKGGGGSGGGGSGGGGSGGGGSQVQLQESGPGLVKPSET LSLTCTVSGVSLPDYGVSWIRQPPGKGLEWIGVIWGSETTYYNSSLKSRVTISKD NSKNQVSLKLSSVTAADTAVYYCAKHYYYGGSYAMDYWGQGTLVTVSSTTTPAPR PPTPAPTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNPQEGLYNE LQKDKMAEAYSEIGMKGERRRGKGHDGLYQGLSTATKDTYDALHMQALPPR |

| **CAR11** | | |
|---|---|---|
| **CAR11 scFv domain** | 943 | eivmtqspatlslspgeratlscrasqdiskylnwyqqkpgqaprlliyhtsrlh sgiparfsgsgsgtdytltisslqpedfavyfcqqgntlpytfgqgtkleikggg gsggggsggggsqvqlqesgpglvkpsetlsltctvsgvslpdygvswirqppgk glewigviwgsettyynsslksrvtiskdnsknqvslklssvtaadtavyycakh yyyggsyamdywgqgtlvtvss |
| **103101 CAR11** - **Soluble scFv** - **nt** | 944 | Atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagccaggtccaact ccaagaaagcggaccgggtcttgtgaagccatcagaaactctttcactgacttgt actgtgagcggagtgtctctccccgattacggggtgtcttggatcagacagccac cggggaagggtctggaatggattggagtgatttggggctctgagactacttacta caattcatccctcaagtcacgcgtcaccatctcaaaggacaactctaagaatcag gtgtcactgaaactgtcatctgtgaccgcagccgacaccgccgtgtactattgcg ctaagcattactattatggcgggagctacgcaatggattactggggacagggtac tctggtcaccgtgtccagccaccaccatcatcaccatcaccat |
| **103101 CAR11** - **Soluble scFv** | 945 | MALPVTALLLPLALLLHAARPeivmtqspatlslspgeratlscrasqdiskyln wyqqkpgqaprlliyhtsrlhsgiparfsgsgsgtdytltisslqpedfavyfcq qgntlpytfgqgtkleikggggsggggsggggsqvqlqesgpglvkpsetlsltc tvsgvslpdygvswirqppgkglewigviwgsettyynsslksrvtiskdnsknq |
| **- aa** | | vslklssvtaadtavyycakhyyyggsyamdywgqgtlvtvsshhhhhhhh |
| **105976 CAR 11 Full - nt** | 946 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactataactcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccggaggtggcggaagcgaaatcgtgatg acccagagccctgcaaccctgtccctttctcccggggaacgggctaccctttctt gtcgggcatcacaagatatctcaaaatacctcaattggtatcaacagaagccggg acaggcccctaggcttcttatctaccacacctctcgcctgcatagcgggattccc gcacgctttagcgggtctggaagcgggaccgactacactctgaccatctcatctc tccagcccgaggacttcgccgtctacttctgccagcagggtaacaccctgccgta caccttcggccagggcaccaagcttgagatcaaaaccactactcccgctccaagg ccacccacccctgccccgaccatcgcctctcagccgctttccctgcgtccggagg catgtagacccgcagctggtggggccgtgcatacccggggtcttgacttcgcctg cgatatctacatttgggcccctctggctggtacttgcggggtcctgctgctttca ctcgtgatcactctttactgtaagcgcggtcggaagaagctgctgtacatcttta agcaacccttcatgaggcctgtgcagactactcaagaggaggacggctgttcatg ccggttcccagaggaggaggaaggcggctgcgaactgcgcgtgaaattcagccgc agcgcagatgctccagcctacaagcaggggcagaaccagctctacaacgaactca atcttggtcggagagaggagtacgacgtgctggacaagcggagaggacgggaccc agaaatgggcgggaagccgcgcagaaagaatccccaagagggcctgtacaacgag ctccaaaaggataagatggcagaagcctatagcgagattggtatgaaaggggaac gcagaagaggcaaaggccacgacggactgtaccagggactcagcaccgccaccaa ggacacctatgacgctcttcacatgcaggccctgccgcctcgg |
| **105976 CAR 11 Full** - **aa** | 947 | MALPVTALLLPLALLLHAARPQVQLQESGPGLVKPSETLSLTCTVSGVSLPDYGV SWIRQPPGKGLEWIGVIWGSETTYYNSSLKSRVTISKDNSKNQVSLKLSSVTAAD TAVYYCAKHYYYGGSYAMDYWGQGTLVTVSSGGGGSGGGGSGGGGSGGGGSEIVM TQSPATLSLSPGERATLSCRASQDISKYLNWYQQKPGQAPRLLIYHTSRLHSGIP ARFSGSGSGTDYTLTISSLQPEDFAVYFCQQGNTLPYTFGQGTKLEIKTTTPAPR PPTPAPTIASQPLSLRPEACRPAAGGAVHTRGLDFACDIYIWAPLAGTCGVLLLS LVITLYCKRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNPQEGLYNE LQKDKMAEAYSEIGMKGERRRGKGHDGLYQGLSTATKDTYDALHMQALPPR |

| **CAR12** | | |
|---|---|---|
| **CAR12 scFv domain** | 948 | qvqlqesgpglvkpsetlsltctvsgvslpdygvswirqppgkglewigviwgse ttyynsslksrvtiskdnsknqvslklssvtaadtavyycakhyyyggsyamdyw gqgtlvtvssggggsggggsggggseivmtqspatlslspgeratlscrasqdis kylnwyqqkpgqaprlliyhtsrlhsgiparfsgsgsgtdytltisslqpedfav |
| | | yfcqqgntlpytfgqgtkleik |
| **103104 CAR12 - Soluble scFv - nt** | 949 | atggctctgcccgtgaccgcactcctcctgccactggctctgctgcttcacgccg ctcgcccacaagtccagcttcaagaatcagggcctggtctggtgaagccatctga gactctgtccctcacttgcaccgtgagcggagtgtccctcccagactacggagtg agctggattagacagcctcccggaaagggactggagtggatcggagtgatttggg gtagcgaaaccacttactataactcttccctgaagtcacgggtcaccatttcaaa ggataactcaaagaatcaagtgagcctcaagctctcatcagtcaccgccgctgac accgccgtgtattactgtgccaagcattactactatggagggtcctacgccatgg actactggggccagggaactctggtcactgtgtcatctggtggaggaggtagcgg aggaggcgggagcggtggaggtggctccgaaatcgtgatgacccagagccctgca accctgtccctttctcccggggaacgggctaccctttcttgtcgggcatcacaag atatctcaaaatacctcaattggtatcaacagaagccgggacaggcccctaggct tcttatctaccacacctctcgcctgcatagcgggattcccgcacgctttagcggg tctggaagcgggaccgactacactctgaccatctcatctctccagcccgaggact tcgccgtctacttctgccagcagggtaacaccctgccgtacaccttcggccaggg caccaagcttgagatcaaacatcaccaccatcatcaccatcac |
| **103104 CAR12 - Soluble scFv -aa** | 950 | MALPVTALLLPLALLLHAARPqvqlqesgpglvkpsetlsltctvsgvslpdygv swirqppgkglewigviwgsettyynsslksrvtiskdnsknqvslklssvtaad tavyycakhyyyggsyamdywgqgtlvtvssggggsggggsggggseivmtqspa tlslspgeratlscrasqdiskylnwyqqkpgqaprlliyhtsrlhsgiparfsg sgsgtdytltisslqpedfavyfcqqgntlpytfgqgtkleikhhhhhhhh |
| **105977 CAR 12 - Full - nt** | 951 | atggccctccctgtcaccgccctgctgcttccgctggctcttctgctccacgccg ctcggcccgaaattgtgatgacccagtcacccgccactcttagcctttcacccgg tgagcgcgcaaccctgtcttgcagagcctcccaagacatctcaaaataccttaat tggtatcaacagaagcccggacaggctcctcgccttctgatctaccacaccagcc ggctccattctggaatccctgccaggttcagcggtagcggatctgggaccgacta caccctcactatcagctcactgcagccagaggacttcgctgtctatttctgtcag caagggaacaccctgccctacacctttggacagggcaccaagctcgagattaaag gtggaggtggcagcggaggaggtgggtccggcggtggaggaagccaggtccaact ccaagaaagcggaccgggtcttgtgaagccatcagaaactctttcactgacttgt actgtgagcggagtgtctctccccgattacggggtgtcttggatcagacagccac cggggaagggtctggaatggattggagtgatttggggctctgagactacttacta caactcatccctcaagtcacgcgtcaccatctcaaaggacaactctaagaatcag gtgtcactgaaactgtcatctgtgaccgcagccgacaccgccgtgtactattgcg ctaagcattactattatggcgggagctacgcaatggattactggggacagggtac tctggtcaccgtgtccagcaccactaccccagcaccgaggccacccaccccggct cctaccatcgcctcccagcctctgtccctgcgtccggaggcatgtagacccgcag ctggtggggccgtgcatacccggggtcttgacttcgcctgcgatatctacatttg ggcccctctggctggtacttgcggggtcctgctgctttcactcgtgatcactctt tactgtaagcgcggtcggaagaagctgctgtacatctttaagcaacccttcatga ggcctgtgcagactactcaagaggaggacggctgttcatgccggttcccagagga ggaggaaggcggctgcgaactgcgcgtgaaattcagccgcagcgcagatgctcca gcctacaagcaggggcagaaccagctctacaacgaactcaatcttggtcggagag aggagtacgacgtgctggacaagcggagaggacgggacccagaaatgggcgggaa gccgcgcagaaagaatccccaagagggcctgtacaacgagctccaaaaggataag atggcagaagcctatagcgagattggtatgaaaggggaacgcagaagaggcaaag |
| | | gccacgacggactgtaccagggactcagcaccgccaccaaggacacctatgacgc tcttcacatgcaggccctgccgcctcgg |
| **105977 CAR 12 - Full - aa** | 952 | MALPVTALLLPLALLLHAARPEIVMTQSPATLSLSPGERATLSCRASQDISKYLN WYQQKPGQAPRLLIYHTSRLHSGIPARFSGSGSGTDYTLTISSLQPEDFAVYFCQ QGNTLPYTFGQGTKLEIKGGGGSGGGGSGGGGSQVQLQESGPGLVKPSETLSLTC TVSGVSLPDYGVSWIRQPPGKGLEWIGVIWGSETTYYNSSLKSRVTISKDNSKNQ VSLKLSSVTAADTAVYYCAKHYYYGGSYAMDYWGQGTLVTVSSTTTPAPRPPTPA PTIASQPLSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLYNELQKDK MAEAYSEIGMKGERRRGKGHDGLYQGLSTATKDTYDALHMQALPPR |

| **CTL019** | | |
|---|---|---|
| **CTL019** - **Soluble scFv-Histag - nt** | 953 | atggccctgcccgtcaccgctctgctgctgccccttgctctgcttcttcatgcag caaggccggacatccagatgacccaaaccacctcatccctctctgcctctcttgg agacagggtgaccatttcttgtcgcgccagccaggacatcagcaagtatctgaac tggtatcagcagaagccggacggaaccgtgaagctcctgatctaccatacctctc gcctgcatagcggcgtgccctcacgcttctctggaagcggatcaggaaccgatta ttctctcactatttcaaatcttgagcaggaagatattgccacctatttctgccag cagggtaataccctgccctacaccttcggaggagggaccaagctcgaaatcaccg gtggaggaggcagcggcggtggagggtctggtggaggtggttctgaggtgaagct gcaagaatcaggccctggacttgtggccccttcacagtccctgagcgtgacttgc accgtgtccggagtctccctgcccgactacggagtgtcatggatcagacaacctc cacggaaaggactggaatggctcggtgtcatctggggtagcgaaactacttacta caattcagccctcaaaagcaggctgactattatcaaggacaacagcaagtcccaa gtctttcttaagatgaactcactccagactgacgacaccgcaatctactattgtg ctaagcactactactacggaggatcctacgctatggattactggggacaaggtac ttccgtcactgtctcttcacaccatcatcaccatcaccatcac |
| **CTL019** - **Soluble scFv-Histag** - **aa** | 954 | MALPVTALLLPLALLLHAARPdiqmtqttsslsaslgdrvtiscrasqdiskyln wyqqkpdgtvklliyhtsrlhsgvpsrfsgsgsgtdysltisnleqediatyfcq qgntlpytfgggtkleitggggsggggsggggsevklqesgpglvapsqslsvtc tvsgvslpdygvswirqpprkglewlgviwgsettyynsalksrltiikdnsksq vflkmnslqtddtaiyycakhyyyggsyamdywgqgtsvtvsshhhhhhhh |
| **CTL019 Full** - **nt** | 955 | atggccttaccagtgaccgccttgctcctgccgctggccttgctgctccacgccg ccaggccggacatccagatgacacagactacatcctccctgtctgcctctctggg agacagagtcaccatcagttgcagggcaagtcaggacattagtaaatatttaaat tggtatcagcagaaaccagatggaactgttaaactcctgatctaccatacatcaa gattacactcaggagtcccatcaaggttcagtggcagtgggtctggaacagatta ttctctcaccattagcaacctggagcaagaagatattgccacttacttttgccaa cagggtaatacgcttccgtacacgttcggaggggggaccaagctggagatcacag gtggcggtggctcgggcggtggtgggtcgggtggcggcggatctgaggtgaaact |
| | | gcaggagtcaggacctggcctggtggcgccctcacagagcctgtccgtcacatgc actgtctcaggggtctcattacccgactatggtgtaagctggattcgccagcctc cacgaaagggtctggagtggctgggagtaatatggggtagtgaaaccacatacta taattcagctctcaaatccagactgaccatcatcaaggacaactccaagagccaa gttttcttaaaaatgaacagtctgcaaactgatgacacagccatttactactgtg ccaaacattattactacggtggtagctatgctatggactactggggccaaggaac ctcagtcaccgtctcctcaaccacgacgccagcgccgcgaccaccaacaccggcg cccaccatcgcgtcgcagcccctgtccctgcgcccagaggcgtgccggccagcgg cggggggcgcagtgcacacgagggggctggacttcgcctgtgatatctacatctg ggcgcccttggccgggacttgtggggtccttctcctgtcactggttatcaccctt tactgcaaacggggcagaaagaaactcctgtatatattcaaacaaccatttatga gaccagtacaaactactcaagaggaagatggctgtagctgccgatttccagaaga agaagaaggaggatgtgaactgagagtgaagttcagcaggagcgcagacgccccc gcgtacaagcagggccagaaccagctctataacgagctcaatctaggacgaagag aggagtacgatgttttggacaagagacgtggccgggaccctgagatggggggaaa gccgagaaggaagaaccctcaggaaggcctgtacaatgaactgcagaaagataag atggcggaggcctacagtgagattgggatgaaaggcgagcgccggaggggcaagg ggcacgatggcctttaccagggtctcagtacagccaccaaggacacctacgacgc ccttcacatgcaggccctgccccctcgc |
| **CTL019 Full** - **aa** | 956 | MALPVTALLLPLALLLHAARPdiqmtqttsslsaslgdrvtiscrasqdiskyln wyqqkpdgtvklliyhtsrlhsgvpsrfsgsgsgtdysltisnleqediatyfcq qgntlpytfgggtkleitggggsggggsggggsevklqesgpglvapsqslsvtc tvsgvslpdygvswirqpprkglewlgviwgsettyynsalksrltiikdnsksq vflkmnslqtddtaiyycakhyyyggsyamdywgqgtsvtvssttttpaprpptpa ptiasqplslrpeacrpaaggavhtrg[?]dfacdiyiwaplagtcgvlllslvitl yckrgrkkllyifkqpfmrpvqttqeedgcscrfpeeeeggcelrvkfsrsadap aykqgqnqlynelnlgrreeydvldkrrgrdpemggkprrknpqeglynelqkdk maeayseigmkgerrrgkghdglyqglstatkdtydalhmqalppr |
| **CTL019 scFv domain** | 957 | diqmtqttsslsaslgdrvtiscrasqdiskylnwyqqkpdgtvklliyhtsrlh sgvpsrfsgsgsgtdysltisnleqediatyfcqqgntlpytfgggtkleitggg gsggggsggggsevklqesgpglvapsqslsvtctvsgvslpdygvswirqpprk glewlgviwgsettyynsalksrltiikdnsksqvflkmnslqtddtaiyycakh yyyggsyamdywgqgtsvtvss |
| **mCAR1 scFv** | 983 | QVQLLESGAELVRPGSSVKISCKASGYAFSSYWMNWVKQRPGQGLEWIGQIYPGD GDTNYNGKFKGQATLTADKSSSTAYMQLSGLTSEDSAVYSCARKTISSVVDFYFD YWGQGTTVTGGGSGGGSGGGSGGGSELVLTQSPKFMSTSVGDRVSVTCKASQNVG TNVAWYQQKPGQSPKPLIYSATYRNSGVPDRFTGSGSGTDFTLTITNVQSKDLAD YFCQYNRYPYTSFFFTKLEIKRRS |
| **mCAR1 Full - aa** | 984 | QVQLLESGAELVRPGSSVKISCKASGYAFSSYWMNWVKQRPGQGLEWIGQIYPGD GDTNYNGKFKGQATLTADKSSSTAYMQLSGLTSEDSAVYSCARKTISSVVDFYFD YWGQGTTVTGGGSGGGSGGGSGGGSELVLTQSPKFMSTSVGDRVSVTCKASQNVG TNVAWYQQKPGQSPKPLIYSATYRNSGVPDRFTGSGSGTDFTLTITNVQSKDLAD YFCQYNRYPYTSFFFTKLEIKRRSKIEVMYPPPYLDNEKSNGTIIHVKGKHLCPS PLFPGPSKPFWVLVVVGGVLACYSLLVTVAFIIFWVRSKRSRLLHSDYMNMTPRR PGPTRKHYQPYAPPRDFAAYRSRVKFSRSADAPAYQQGQNQLYNELNLGRREEYD VLDKRRGRDPEMGGKPRRKNPQEGLYNELQKDKMAEAYSEIGMKGERRRGKGHDG |
| | | LYQGLSTATKDTYDALHMQALPPR |
| **mCAR2 scFv** | 985 | DIQMTQTTSSLSASLGDRVTISCRASQDISKYLNWYQQKPDGTVKLLIYHTSRLH SGVPSRFSGSGSGTDYSLTISNLEQED[?]ATYFCQQGNTLPYTFGGGTKLEITGST SGSGKPGSGEGSTKGEVKLQESGPGLVAPSQSLSVTCTVSGVSLPDYGVSWIRQP PRKGLEWLGVIWGSETTYYNSALKSRL[?]IIKDNSKSQVFLKMNSLQTDDTAIYYC AKHYYYGGSYAMDYWGQGTSVTVSSE |
| **mCAR2 CAR - aa** | 986 | DIQMTQTTSSLSASLGDRVTISCRASQDISKYLNWYQQKPDGTVKLLIYHTSRLH SGVPSRFSGSGSGTDYSLTISNLEQEDIATYFCQQGNTLPYTFGGGTKLEITGST SGSGKPGSGEGSTKGEVKLQESGPGLVAPSQSLSVTCTVSGVSLPDYGVSWIRQP PRKGLEWLGVIWGSETTYYNSALKSRLTIIKDNSKSQVFLKMNSLQTDDTAIY YCAKHYYYGGSYAMDYWGQGTSVTVSSESKYGPPCPPCPMFWVLVVVGGVLACYS LLVTVAFIIFWVKRGRKKLLYIFKQPFMRPVQTTQEEDGCSCRFEEEEGGCELRV KFSRSADAPAYQQGQNQLYNELNLGRREEYDVLDKRRGRDPEMGGKPRRKNPQEG LYNELQKDKMAEAYSEIGMKGERRRGKGHDGLYQGLSTATKDTYDALHMQALPPR L |
| **mCAR2 Full - aa** | 987 | DIQMTQTT SSLSASLGDR VTISCRASQD ISKYLNWYQQ KPDGTVKLLI YHTSRLHSGV PSRFSGSGSG TDYSL[?]ISNL EQEDIATYFC QQGNTLPYTF GGGTKLEITG STSGSGKPGS GEGSTKGEVK LQESGPGLVA PSQSLSVTCT VSGVSLPDYG VSWIRQPPRK GLEWLGVIWG SETTYYNSAL KSRLTIIKDN SKSQVFLKMN SLQTDDTAIY YCAKHYYYGG SYAMDYWGQG TSVTVSSESK YGPPCPPCPM FWVLVVVGGV LACYSLLVTV AFIIFWVKRG RKKLLYIFKQ PFMRPVQTTQ EEDGCSCRFE EEEGGCELRV KFSRSADAPA YQQGQNQLYN ELNLGRREEY DVLDKRRGRD PEMGGKPRRK NPQEGLYNEL QKDKMAEAYS EIGMKGERRR GKGHDGLYQG LSTATKDTYD ALHMQALPPR LEGGGEGRGS LLTCGDVEEN PGPRMLLLVT SLLLCELPHP AFLLIPRKVC NGIGIGEFKD SLSINATNIK HFKNCTSISG DLHILPVAFR GDSFTHTPPL DPQELDILKT VKEITGFLLI QAWPENRTDL HAFENLEIIR GRTKQHGQFS LAVVSLNITS LGLRSLKEIS DGDVIISGNK NLCYANTINW KKLFGTSGQK TKIISNRGEN SCKATGQVCH ALCSPEGCWG PEPRDCVSCR NVSRGRECVD KCNLLEGEPR EFVENSECIQ CHPECLPQAM NITCTGRGPD NCIQCAHYID GPHCVKTCPA GVMGENNTLV WKYADAGHVC HLCHPNCTYG CTGPGLEGCP TNGPKIPSIA TGMVGALLLL LVVALGIGLF M |
| **mCAR3 scFv** | 988 | DIQMTQTTSSLSASLGDRVTISCRASQDISKYLNWYQQKPDGTVKLLIYHTSRLH SGVPSRFSGSGSGTDYSLTISNLEQEDIATYFCQQGNTLPYTFGGGTKLEITGST SGSGKPGSGEGSTKGEVKLQESGPGLVAPSQSLSVTCTVSGVSLPDYGVSWIRQP PRKGLEWLGVIWGSETTYYNSALKSRLTIIKDNSKSQVFLKMNSLQTDDTAIYYC AKHYYYGGSYAMDYWGQGTSVTVSS |
| **mCAR3 Full - aa** | 989 | DIQMTQTTSSLSASLGDRVTISCRASQDISKYLNWYQQKPDGTVKLLIYHTSRLH SGVPSRFSGSGSGTDYSLTISNLEQEDIATYFCQQGNTLPYTFGGGTKLEITGST SGSGKPGSGEGSTKGEVKLQESGPGLVAPSQSLSVTCTVSGVSLPDYGVSWIRQP PRKGLEWLGVIWGSETTYYNSALKSRLTIIKDNSKSQVFLKMNSLQTDDTAIYYC AKHYYYGGSYAMDYWGQGTSVTVSSAAAIEVMYPPPYLDNEKSNGTIIHVKGKHL CPSPLFPGPSKPFWVLVVVGGVLACYSLLVTVAFIIFWVRSKRSRLLHSDYMNMT PRRPGPTRKHYQPYAPPRDFAAYRSRVKFSRSADAPAYQQGQNQLYNELNLGRRE EYDVLDKRRGRDPEMGGKPRRKNPQEGLYNELQKDKMAEAYSEIGMKGERRRGKG |
| | | HDGLYQGLSTATKDTYDALHMQALPPR |
| **SSJ25-C1 VH sequence** | 379 | QVQLLESGAELVRPGSSVKISCKASGYAFSSYWMNWVKQRPGQGLEWIGQIYPGD GDTNYNGKFKGQATLTADKSSSTAYMQLSGLTSEDSAVYSCARKTISSVVDFYFD YWGQGTTVT |
| **SSJ25-C1 VL sequence** | 380 | ELVLTQSPKFMSTSVGDRVSVTCKASQNVGTNVAWYQQKPGQSPKPLIYSATYRN SGVPDRFTGSGSGTDFTLTITNVQSKDLADYFYFCQYNRYPYTSGGGTKLEIKRR S |

In some embodiments, the CD19 CAR or binding domain includes the amino acid sequence of CTL019, or is encoded by the nucleotide sequence of CTL019 according to Table 5 with or without the leader sequence or the his tag, or a sequence substantially identical thereto (e.g., at least 85%, 90%, 95% or higher identity).

In some embodiments, the CDRs are defined according to the Kabat numbering scheme, the Chothia numbering scheme, or a combination thereof.

The sequences of humanized CDR sequences of the scFv domains are shown in Table 15B for the heavy chain variable domains and in Table 15C for the light chain variable domains. "ID" stands for the respective SEQ ID NO for each CDR.

**Table 15B. Heavy Chain Variable Domain CDRs (according to Kabat)**

| Candidate | FW | HCDR1 | SEQ ID | HCDR2 | SEQ ID | HCDR3 | SEQ ID |
|---|---|---|---|---|---|---|---|
| murine_CART19 | | DYGVS | 958 | VIWGSETTYYNSALKS | 959 | HYYYGGSYAMDY | 960 |
| humanized_CART19 a | VH4 | DYGVS | 958 | VIWGSETTYY***S***S***S***LKS | 961 | HYYYGGSYAMDY | 960 |
| humanized_CART19 b | VH4 | DYGVS | 958 | VIWGSETTYY***Q***S***S***LKS | 962 | HYYYGGSYAMDY | 960 |
| humanized_CART19 c | VH4 | DYGVS | 958 | VIWGSETTYYNS***S***LKS | 963 | HYYYGGSYAMDY | 960 |

**Table 15C Light Chain Variable Domain CDRs (according to Kabat)**

| Candidate | FW | LCDR1 | SEQ ID | LCDR2 | SEQ ID | LCDR3 | SEQ ID |
|---|---|---|---|---|---|---|---|
| murine_CART19 | | RASQDISKYLN | 964 | HTSRLHS | 965 | QQGNTLPYT | 966 |
| humanized_CART19 a | VK3 | RASQDISKYLN | 964 | HTSRLHS | 965 | QQGNTLPYT | 966 |
| humanized_CART19 b | VK3 | RASQDISKYLN | 964 | HTSRLHS | 965 | QQGNTLPYT | 966 |
| humanized_CART19 c | VK3 | RASQDISKYLN | 964 | HTSRLHS | 965 | QQGNTLPYT | 966 |

In one embodiment, the CAR comprises a CAR molecule comprising a BCMA antigen binding domain (e.g., a murine, human or humanized antibody or antibody fragment that specifically binds to BCMA, e.g., human BCMA), a transmembrane domain, and an intracellular signaling domain (e.g., an intracellular signaling domain comprising a costimulatory domain and/or a primary signaling domain).

Exemplary CAR molecules are provided in Table 16, or Table 1 of WO2016/014565, or as otherwise described herein. The CAR molecules in Table 16 comprise a BCMA antigen binding domain, e.g., an amino acid sequence of any BCMA antigen binding domain provided in Table 11 or 12.

**Table 16. Exemplary CAR molecules. Sequences are provided with a leader sequence.**

| **Name/ Description** | **SEQ ID NO:** | **Sequence** |
|---|---|---|
| **139109** | | |
| **139109-aa Full CAR** | 789 | MALPVTALLLPLALLLHAARPEVQLVESGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSDIQLTQSPSSLSASVGDRVTITCRASQSISSYLNWYQQKPGKAPKLL IYAASSLQSGVPSRFSGSGSGTDFTLTISSLQPEDFATYYCQQSYSTPYTF GQGTKVEIKTTTPAPRPPTPAPTIASQPLSLRPEACRPAAGGAVHTRGLDF ACDIYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREEY DVLDKRRGRDPEMGGKPRRKNPQEGLYNELQKDKMAEAYSEIGMKGER RRGKGHDGLYQGLSTATKDTYDALHMQALPPR |
| **139109-nt Full CAR** | 790 | ATGGCCCTCCCTGTCACCGCCCTGCTGCTTCCGCTGGCTCTTCTGCTC CACGCCGCTCGGCCCGAAGTGCAATTGGTGGAATCAGGGGGAGGACT TGTGCAGCCTGGAGGATCGCTGAGACTGTCATGTGCCGTGTCCGGCTT TGCCCTGTCCAACCACGGGATGTCCTGGGTCCGCCGCGCGCCTGGAA AGGGCCTCGAATGGGTGTCGGGTATTGTGTACAGCGGTAGCACCTACT ATGCCGCATCCGTGAAGGGGAGATTCACCATCAGCCGGGACAACTCCA GGAACACTCTGTACCTCCAAATGAATTCGCTGAGGCCAGAGGACACTG CCATCTACTACTGCTCCGCGCATGGCGGAGAGTCCGACGTCTGGGGA CAGGGGACCACCGTGACCGTGTCTAGCGCGTCCGGCGGAGGCGGCA GCGGGGGTCGGGCATCAGGGGGCGGCGGATCGGACATCCAGCTCAC CCAGTCCCCGAGCTCGCTGTCCGCCTCCGTGGGAGATCGGGTCACCA TCACGTGCCGCGCCAGCCAGTCGATTTCCTCCTACCTGAACTGGTACC |
| | | AACAGAAGCCCGGAAAAGCCCCGAAGCTTCTCATCTACGCCGCCTCGA GCCTGCAGTCAGGAGTGCCCTCACGGTTCTCCGGCTCCGGTTCCGGTA CTGATTTCACCCTGACCATTTCCTCCCTGCAACCGGAGGACTTCGCTAC TTACTACTGCCAGCAGTCGTACTCCACCCCCTACACTTTCGGACAAGGC ACCAAGGTCGAAATCAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTT CGCCTGCGATATCTACATTTGGGCCCCTCTGGCTGGTACTTGCGGGGT CCTGCTGCTTTCACTCGTGATCACTCTTTACTGTAAGCGCGGTCGGAAG AAGCTGCTGTACATCTTTAAGCAACCCTTCATGAGGCCTGTGCAGACTA CTCAAGAGGAGGACGGCTGTTCATGCCGGTTCCCAGAGGAGGAGGAA GGCGGCTGCGAACTGCGCGTGAAATTCAGCCGCAGCGCAGATGCTCC AGCCTACAAGCAGGGGCAGAACCAGCTCTACAACGAACTCAATCTTGG TCGGAGAGAGGAGTACGACGTGCTGGACAAGCGGAGAGGACGGGACC CAGAAATGGGCGGGAAGCCGCGCAGAAAGAATCCCCAAGAGGGCCTG TACAACGAGCTCCAAAAGGATAAGATGGCAGAAGCCTATAGCGAGATT GGTATGAAAGGGGAACGCAGAAGAGGCAAAGGCCACGACGGACTGTA CCAGGGACTCAGCACCGCCACCAAGGACACCTATGACGCTCTTCACAT GCAGGCCCTGCCGCCTCGG |

| **139103** | | |
|---|---|---|
| **139103- aa Full CAR** | 791 | MALPVTALLLPLALLLHAARPQVQLVESGGGLVQPGRSLRLSCAASGFTFS NYAMSWVRQAPGKGLGWVSGISRSGENTYYADSVKGRFTISRDNSKNTL YLQMNSLRDEDTAVYYCARSPAHYYGGMDVWGQGTTVTVSSASGGGGS GGRASGGGGSDIVLTQSPGTLSLSPGERATLSCRASQSISSSFLAWYQQK PGQAPRLLIYGASRRATGIPDRFSGSGSGTDFTLTISRLEPEDSAVYYCQQ YHSSPSWTFGQGTKLEIKTTTPAPRPPTPAPTIASQPLSLRPEACRPAAGG AVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNEL NLGRREEYDVLDKRRGRDPEMGGKPRRKNPQEGLYNELQKDKMAEAYS EIGMKGERRRGKGHDGLYQGLSTATKDTYDALHMQALPPR |
| **139103- nt Full CAR** | 792 | ATGGCCCTCCCTGTCACCGCCCTGCTGCTTCCGCTGGCTCTTCTGCTC CACGCCGCTCGGCCCCAAGTGCAACTCGTGGAATCTGGTGGAGGACT CGTGCAACCCGGAAGATCGCTTAGACTGTCGTGTGCCGCCAGCGGGTT CACTTTCTCGAACTACGCGATGTCCTGGGTCCGCCAGGCACCCGGAAA GGGACTCGGTTGGGTGTCCGGCATTTCCCGGTCCGGCGAAAATACCTA CTACGCCGACTCCGTGAAGGGCCGCTTCACCATCTCAAGGGACAACAG CAAAAACACCCTGTACTTGCAAATGAACTCCCTGCGGGATGAAGATACA GCCGTGTACTATTGCGCCCGGTCGCCTGCCCATTACTACGGCGGAATG GACGTCTGGGGACAGGGAACCACTGTGACTGTCAGCAGCGCGTCGGG TGGCGGCGGCTCAGGGGGTCGGGCCTCCGGGGGGGGAGGGTCCGAC ATCGTGCTGACCCAGTCCCCGGGAACCCTGAGCCTGAGCCCGGGAGA GCGCGCGACCCTGTCATGCCGGGCATCCCAGAGCATTAGCTCCTCCTT TCTCGCCTGGTATCAGCAGAAGCCCGGACAGGCCCCGAGGCTGCTGA TCTACGGCGCTAGCAGAAGGGCTACCGGAATCCCAGACCGGTTCTCCG GCTCCGGTTCCGGGACCGATTTCACCCTTACTATCTCGCGCCTGGAAC CTGAGGACTCCGCCGTCTACTACTGCCAGCAGTACCACTCATCCCCGT CGTGGACGTTCGGACAGGGCACCAAGCTGGAGATTAAGACCACTACCC CAGCACCGAGGCCACCCACCCCGGCTCCTACCATCGCCTCCCAGCCT CTGTCCCTGCGTCCGGAGGCATGTAGACCCGCAGCTGGTGGGGCCGT GCATACCCGGGGTCTTGACTTCGCCTGCGATATCTACATTTGGGCCCC TCTGGCTGGTACTTGCGGGGTCCTGCTGCTTTCACTCGTGATCACTCTT TACTGTAAGCGCGGTCGGAAGAAGCTGCTGTACATCTTTAAGCAACCCT TCATGAGGCCTGTGCAGACTACTCAAGAGGAGGACGGCTGTTCATGCC GGTTCCCAGAGGAGGAGGAAGGCGGCTGCGAACTGCGCGTGAAATTC AGCCGCAGCGCAGATGCTCCAGCCTACAAGCAGGGGCAGAACCAGCT |
| | | CTACAACGAACTCAATCTTGGTCGGAGAGAGGAGTACGACGTGCTGGA CAAGCGGAGAGGACGGGACCCAGAAATGGGCGGGAAGCCGCGCAGA AAGAATCCCCAAGAGGGCCTGTACAACGAGCTCCAAAAGGATAAGATG GCAGAAGCCTATAGCGAGATTGGTATGAAAGGGGAACGCAGAAGAGG CAAAGGCCACGACGGACTGTACCAGGGACTCAGCACCGCCACCAAGG ACACCTATGACGCTCTTCACATGCAGGCCCTGCCGCCTCGG |

| **139105** | | |
|---|---|---|
| **139105- aa Full CAR** | 793 | MALPVTALLLPLALLLHAARPQVQLVESGGGLVQPGRSLRLSCAASGFTFD DYAMHWVRQAPGKGLEWVSGISWNSGSIGYADSVKGRFTISRDNAKNSL YLQMNSLRAEDTALYYCSVHSFLAYWGQGTLVTVSSASGGGGSGGRASG GGGSDIVMTQTPLSLPVTPGEPASISCRSSQSLLHSNGYNYLDWYLQKPG QSPQLLIYLGSNRASGVPDRFSGSGSGTDFTLKISRVEAEDVGVYYCMQA LQTPYTFGQGTKVEIKTTTPAPRPPTPAPTIASQPLSLRPEACRPAAGGAV HTRGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNL GRREEYDVLDKRRGRDPEMGGKPRRKNPQEGLYNELQKDKMAEAYSEIG MKGERRRGKGHDGLYQGLSTATKDTYDALHMQALPPR |
| **139105- nt Full CAR** | 794 | ATGGCCCTCCCTGTCACCGCCCTGCTGCTTCCGCTGGCTCTTCTGCTC CACGCCGCTCGGCCCCAAGTGCAACTCGTCGAATCCGGTGGAGGTCT GGTCCAACCTGGTAGAAGCCTGAGACTGTCGTGTGCGGCCAGCGGATT CACCTTTGATGACTATGCTATGCACTGGGTGCGGCAGGCCCCAGGAAA GGGCCTGGAATGGGTGTCGGGAATTAGCTGGAACTCCGGGTCCATTG GCTACGCCGACTCCGTGAAGGGCCGCTTCACCATCTCCCGCGACAAC GCAAAGAACTCCCTGTACTTGCAAATGAACTCGCTCAGGGCTGAGGAT ACCGCGCTGTACTACTGCTCCGTGCATTCCTTCCTGGCCTACTGGGGA CAGGGAACTCTGGTCACCGTGTCGAGCGCCTCCGGCGGCGGGGGCTC GGGTGGACGGGCCTCGGGCGGAGGGGGGTCCGACATCGTGATGACC CAGACCCCGCTGAGCTTGCCCGTGACTCCCGGAGAGCCTGCATCCATC TCCTGCCGGTCATCCCAGTCCCTTCTCCACTCCAACGGATACAACTACC TCGACTGGTACCTCCAGAAGCCGGGACAGAGCCCTCAGCTTCTGATCT ACCTGGGGTCAAATAGAGCCTCAGGAGTGCCGGATCGGTTCAGCGGAT CTGGTTCGGGAACTGATTTCACTCTGAAGATTTCCCGCGTGGAAGCCG AGGACGTGGGCGTCTACTACTGTATGCAGGCGCTGCAGACCCCCTATA CCTTCGGCCAAGGGACGAAAGTGGAGATCAAGACCACTACCCCAGCAC CGAGGCCACCCACCCCGGCTCCTACCATCGCCTCCCAGCCTCTGTCCC TGCGTCCGGAGGCATGTAGACCCGCAGCTGGTGGGGCCGTGCATACC CGGGGTCTTGACTTCGCCTGCGATATCTACATTTGGGCCCCTCTGGCT GGTACTTGCGGGGTCCTGCTGCTTTCACTCGTGATCACTCTTTACTGTA AGCGCGGTCGGAAGAAGCTGCTGTACATCTTTAAGCAACCCTTCATGA GGCCTGTGCAGACTACTCAAGAGGAGGACGGCTGTTCATGCCGGTTCC CAGAGGAGGAGGAAGGCGGCTGCGAACTGCGCGTGAAATTCAGCCGC AGCGCAGATGCTCCAGCCTACAAGCAGGGGCAGAACCAGCTCTACAAC GAACTCAATCTTGGTCGGAGAGAGGAGTACGACGTGCTGGACAAGCG GAGAGGACGGGACCCAGAAATGGGCGGGAAGCCGCGCAGAAAGAATC CCCAAGAGGGCCTGTACAACGAGCTCCAAAAGGATAAGATGGCAGAAG CCTATAGCGAGATTGGTATGAAAGGGGAACGCAGAAGAGGCAAAGGCC ACGACGGACTGTACCAGGGACTCAGCACCGCCACCAAGGACACCTATG ACGCTCTTCACATGCAGGCCCTGCCGCCTCGG |

| **139111** | | |
|---|---|---|
| **139111-aa Full CAR** | 795 | MALPVTALLLPLALLLHAARPEVQLLESGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSDIVMTQTPLSLSVTPGQPASISCKSSQSLLRNDGKTPLYWYLQKAG QPPQLLIYEVSNRFSGVPDRFSGSGSGTDFTLKISRVEAEDVGAYYCMQNI QFPSFGGGTKLEIKTTTPAPRPPTPAPTIASQPLSLRPEACRPAAGGAVHT RGLDFACDIYIWAPLAGTCGVLLLSLVITLYCKRGRKKLLYIFKQPFMRPVQ |
| | | TTQEEDGCSCRFPEEEEGGCELRVKFSRSADAPAYKQGQNQLYNELNLG RREEYDVLDKRRGRDPEMGGKPRRKNPQEGLYNELQKDKMAEAYSEIGM KGERRRGKGHDGLYQGLSTATKDTYDALHMQALPPR |
| **139111- nt Full CAR** | 796 | ATGGCCCTCCCTGTCACCGCCCTGCTGCTTCCGCTGGCTCTTCTGCTC CACGCCGCTCGGCCCGAAGTGCAATTGTTGGAATCTGGAGGAGGACTT GTGCAGCCTGGAGGATCACTGAGACTTTCGTGTGCGGTGTCAGGCTTC GCCCTGAGCAACCACGGCATGAGCTGGGTGCGGAGAGCCCCGGGGAA GGGTCTGGAATGGGTGTCCGGGATCGTCTACTCCGGTTCAACTTACTA CGCCGCAAGCGTGAAGGGTCGCTTCACCATTTCCCGCGATAACTCCCG GAACACCCTGTACCTCCAAATGAACTCCCTGCGGCCCGAGGACACCGC CATCTACTACTGTTCCGCGCATGGAGGAGAGTCCGATGTCTGGGGACA GGGCACTACCGTGACCGTGTCGAGCGCCTCGGGGGGAGGAGGCTCC GGCGGTCGCGCCTCCGGGGGGGGTGGCAGCGACATTGTGATGACGC AGACTCCACTCTCGCTGTCCGTGACCCCGGGACAGCCCGCGTCCATCT CGTGCAAGAGCTCCCAGAGCCTGCTGAGGAACGACGGAAAGACTCCT CTGTATTGGTACCTCCAGAAGGCTGGACAGCCCCCGCAACTGCTCATC TACGAAGTGTCAAATCGCTTCTCCGGGGTGCCGGATCGGTTTTCCGGC TCGGGATCGGGCACCGACTTCACCCTGAAAATCTCCAGGGTCGAGGCC GAGGACGTGGGAGCCTACTACTGCATGCAAAACATCCAGTTCCCTTCC TTCGGCGGCGGCACAAAGCTGGAGATTAAGACCACTACCCCAGCACCG AGGCCACCCACCCCGGCTCCTACCATCGCCTCCCAGCCTCTGTCCCTG CGTCCGGAGGCATGTAGACCCGCAGCTGGTGGGGCCGTGCATACCCG GGGTCTTGACTTCGCCTGCGATATCTACATTTGGGCCCCTCTGGCTGG TACTTGCGGGGTCCTGCTGCTTTCACTCGTGATCACTCTTTACTGTAAG CGCGGTCGGAAGAAGCTGCTGTACATCTTTAAGCAACCCTTCATGAGG CCTGTGCAGACTACTCAAGAGGAGGACGGCTGTTCATGCCGGTTCCCA GAGGAGGAGGAAGGCGGCTGCGAACTGCGCGTGAAATTCAGCCGCAG CGCAGATGCTCCAGCCTACAAGCAGGGGCAGAACCAGCTCTACAACGA ACTCAATCTTGGTCGGAGAGAGGAGTACGACGTGCTGGACAAGCGGA GAGGACGGGACCCAGAAATGGGCGGGAAGCCGCGCAGAAAGAATCCC CAAGAGGGCCTGTACAACGAGCTCCAAAAGGATAAGATGGCAGAAGCC TATAGCGAGATTGGTATGAAAGGGGAACGCAGAAGAGGCAAAGGCCAC GACGGACTGTACCAGGGACTCAGCACCGCCACCAAGGACACCTATGAC GCTCTTCACATGCAGGCCCTGCCGCCTCGG |

| **139100** | | |
|---|---|---|
| **139100- aa Full CAR** | 797 | MALPVTALLLPLALLLHAARPQVQLVQSGAEVRKTGASVKVSCKASGYIFD NFGINWVRQAPGQGLEWMGWINPKNNNTNYAQKFQGRVTITADESTNTA YMEVSSLRSEDTAVYYCARGPYYYQSYMDVWGQGTMVTVSSASGGGGS GGRASGGGGSDIVMTQTPLSLPVTPGEPASISCRSSQSLLHSNGYNYLNW YLQKPGQSPQLLIYLGSKRASGVPDRFSGSGSGTDFTLHITRVGAEDVGVY YCMQALQTPYTFGQGTKLEIKTTTPAPRPPTPAPTIASQPLSLRPEACRPA AGGAVHTRGLDFACDIYIWAPLAGTCGVLLLSLVITLYCKRGRKKLLYIFKQ PFMRPVQTTQEEDGCSCRFPEEEEGGCELRVKFSRSADAPAYKQGQNQL YNELNLGRREEYDVLDKRRGRDPEMGGKPRRKNPQEGLYNELQKDKMA EAYSEIGMKGERRRGKGHDGLYQGLSTATKDTYDALHMQALPPR |
| **139100- nt Full CAR** | 798 | ATGGCCCTCCCTGTCACCGCCCTGCTGCTTCCGCTGGCTCTTCTGCTC CACGCCGCTCGGCCCCAAGTCCAACTCGTCCAGTCCGGCGCAGAAGT CAGAAAAACCGGTGCTAGCGTGAAAGTGTCCTGCAAGGCCTCCGGCTA CATTTTCGATAACTTCGGAATCAACTGGGTCAGACAGGCCCCGGGCCA GGGGCTGGAATGGATGGGATGGATCAACCCCAAGAACAACAACACCAA CTACGCACAGAAGTTCCAGGGCCGCGTGACTATCACCGCCGATGAATC GACCAATACCGCCTACATGGAGGTGTCCTCCCTGCGGTCGGAGGACAC TGCCGTGTATTACTGCGCGAGGGGCCCATACTACTACCAAAGCTACAT GGACGTCTGGGGACAGGGAACCATGGTGACCGTGTCATCCGCCTCCG GTGGTGGAGGCTCCGGGGGGCGGGCTTCAGGAGGCGGAGGAAGCGA TATTGTGATGACCCAGACTCCGCTTAGCCTGCCCGTGACTCCTGGAGA |
| | | ACCGGCCTCCATTTCCTGCCGGTCCTCGCAATCACTCCTGCATTCCAAC GGTTACAACTACCTGAATTGGTACCTCCAGAAGCCTGGCCAGTCGCCC CAGTTGCTGATCTATCTGGGCTCGAAGCGCGCCTCCGGGGTGCCTGAC CGGTTTAGCGGATCTGGGAGCGGCACGGACTTCACTCTCCACATCACC CGCGTGGGAGCGGAGGACGTGGGAGTGTACTACTGTATGCAGGCGCT GCAGACTCCGTACACATTCGGACAGGGCACCAAGCTGGAGATCAAGAC CACTACCCCAGCACCGAGGCCACCCACCCCGGCTCCTACCATCGCCTC CCAGCCTCTGTCCCTGCGTCCGGAGGCATGTAGACCCGCAGCTGGTG GGGCCGTGCATACCCGGGGTCTTGACTTCGCCTGCGATATCTACATTT GGGCCCCTCTGGCTGGTACTTGCGGGGTCCTGCTGCTTTCACTCGTGA TCACTCTTTACTGTAAGCGCGGTCGGAAGAAGCTGCTGTACATCTTTAA GCAACCCTTCATGAGGCCTGTGCAGACTACTCAAGAGGAGGACGGCTG TTCATGCCGGTTCCCAGAGGAGGAGGAAGGCGGCTGCGAACTGCGCG TGAAATTCAGCCGCAGCGCAGATGCTCCAGCCTACAAGCAGGGGCAGA ACCAGCTCTACAACGAACTCAATCTTGGTCGGAGAGAGGAGTACGACG TGCTGGACAAGCGGAGAGGACGGGACCCAGAAATGGGCGGGAAGCC GCGCAGAAAGAATCCCCAAGAGGGCCTGTACAACGAGCTCCAAAAGGA TAAGATGGCAGAAGCCTATAGCGAGATTGGTATGAAAGGGGAACGCAG AAGAGGCAAAGGCCACGACGGACTGTACCAGGGACTCAGCACCGCCA CCAAGGACACCTATGACGCTCTTCACATGCAGGCCCTGCCGCCTCGG |

| **139101** | | |
|---|---|---|
| **139101- aa Full CAR** | 799 | MALPVTALLLPLALLLHAARPQVQLQESGGGLVQPGGSLRLSCAASGFTFS SDAMTWVRQAPGKGLEWVSVISGSGGTTYYADSVKGRFTISRDNSKNTLY LQMNSLRAEDTAVYYCAKLDSSGYYYARGPRYWGQGTLVTVSSASGGGG SGGRASGGGGSDIQLTQSPSSLSASVGDRVTITCRASQSISSYLNWYQQK PGKAPKLLIYGASTLASGVPARFSGSGSGTHFTLTINSLQSEDSATYYCQQ SYKRASFGQGTKVEIKTTTPAPRPPTPAPTIASQPLSLRPEACRPAAGGAV HTRGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNL GRREEYDVLDKRRGRDPEMGGKPRRKNPQEGLYNELQKDKMAEAYSEIG MKGERRRGKGHDGLYQGLSTATKDTYDALHMQALPPR |
| **139101-nt Full CAR** | 800 | ATGGCCCTCCCTGTCACCGCCCTGCTGCTTCCGCTGGCTCTTCTGCTC CACGCCGCTCGGCCCCAAGTGCAACTTCAAGAATCAGGCGGAGGACT CGTGCAGCCCGGAGGATCATTGCGGCTCTCGTGCGCCGCCTCGGGCT TCACCTTCTCGAGCGACGCCATGACCTGGGTCCGCCAGGCCCCGGGG AAGGGGCTGGAATGGGTGTCTGTGATTTCCGGCTCCGGGGGAACTAC GTACTACGCCGATTCCGTGAAAGGTCGCTTCACTATCTCCCGGGACAA CAGCAAGAACACCCTTTATCTGCAAATGAATTCCCTCCGCGCCGAGGA CACCGCCGTGTACTACTGCGCCAAGCTGGACTCCTCGGGCTACTACTA TGCCCGGGGTCCGAGATACTGGGGACAGGGAACCCTCGTGACCGTGT CCTCCGCGTCCGGCGGAGGAGGGTCGGGAGGGCGGGCCTCCGGCGG CGGCGGTTCGGACATCCAGCTGACCCAGTCCCCATCCTCACTGAGCGC AAGCGTGGGCGACAGAGTCACCATTACATGCAGGGCGTCCCAGAGCAT CAGCTCCTACCTGAACTGGTACCAACAGAAGCCTGGAAAGGCTCCTAA GCTGTTGATCTACGGGGCTTCGACCCTGGCATCCGGGGTGCCCGCGA GGTTTAGCGGAAGCGGTAGCGGCACTCACTTCACTCTGACCATTAACA GCCTCCAGTCCGAGGATTCAGCCACTTACTACTGTCAGCAGTCCTACAA GCGGGCCAGCTTCGGACAGGGCACTAAGGTCGAGATCAAGACCACTA CCCCAGCACCGAGGCCACCCACCCCGGCTCCTACCATCGCCTCCCAG CCTCTGTCCCTGCGTCCGGAGGCATGTAGACCCGCAGCTGGTGGGGC CGTGCATACCCGGGGTCTTGACTTCGCCTGCGATATCTACATTTGGGC CCCTCTGGCTGGTACTTGCGGGGTCCTGCTGCTTTCACTCGTGATCAC TCTTTACTGTAAGCGCGGTCGGAAGAAGCTGCTGTACATCTTTAAGCAA CCCTTCATGAGGCCTGTGCAGACTACTCAAGAGGAGGACGGCTGTTCA TGCCGGTTCCCAGAGGAGGAGGAAGGCGGCTGCGAACTGCGCGTGAA ATTCAGCCGCAGCGCAGATGCTCCAGCCTACAAGCAGGGGCAGAACC |
| | | AGCTCTACAACGAACTCAATCTTGGTCGGAGAGAGGAGTACGACGTGC TGGACAAGCGGAGAGGACGGGACCCAGAAATGGGCGGGAAGCCGCG CAGAAAGAATCCCCAAGAGGGCCTGTACAACGAGCTCCAAAAGGATAA GATGGCAGAAGCCTATAGCGAGATTGGTATGAAAGGGGAACGCAGAAG AGGCAAAGGCCACGACGGACTGTACCAGGGACTCAGCACCGCCACCA AGGACACCTATGACGCTCTTCACATGCAGGCCCTGCCGCCTCGG |

| **139102** | | |
|---|---|---|
| **139102- aa Full CAR** | 801 | MALPVTALLLPLALLLHAARPQVQLVQSGAEVKKPGASVKVSCKASGYTFS NYGITWVRQAPGQGLEWMGWISAYNGNTNYAQKFQGRVTMTRNTSISTA YMELSSLRSEDTAVYYCARGPYYYYMDVWGKGTMVTVSSASGGGGSGG RASGGGGSEIVMTQSPLSLPVTPGEPASISCRSSQSLLYSNGYNYVDWYL QKPGQSPQLLIYLGSNRASGVPDRFSGSGSGTDFKLQISRVEAEDVGIYYC MQGRQFPYSFGQGTKVEIKTTTPAPRPPTPAPTIASQPLSLRPEACRPAAG GAVHTRGLDFACDIYIWAPLAGTCGVLLLSLVITLYCKRGRKKLLYIFKQPF MRPVQTTQEEDGCSCRFPEEEEGGCELRVKFSRSADAPAYKQGQNQLYN ELNLGRREEYDVLDKRRGRDPEMGGKPRRKNPQEGLYNELQKDKMAEAY SEIGMKGERRRGKGHDGLYQGLSTATKDTYDALHMQALPPR |
| **139102- nt Full CAR** | 802 | ATGGCCCTCCCTGTCACCGCCCTGCTGCTTCCGCTGGCTCTTCTGCTC CACGCCGCTCGGCCCCAAGTCCAACTGGTCCAGAGCGGTGCAGAAGT GAAGAAGCCCGGAGCGAGCGTGAAAGTGTCCTGCAAGGCTTCCGGGT ACACCTTCTCCAACTACGGCATCACTTGGGTGCGCCAGGCCCCGGGAC AGGGCCTGGAATGGATGGGGTGGATTTCCGCGTACAACGGCAATACGA ACTACGCTCAGAAGTTCCAGGGTAGAGTGACCATGACTAGGAACACCT CCATTTCCACCGCCTACATGGAACTGTCCTCCCTGCGGAGCGAGGACA CCGCCGTGTACTATTGCGCCCGGGGACCATACTACTACTACATGGATG TCTGGGGGAAGGGGACTATGGTCACCGTGTCATCCGCCTCGGGAGGC GGCGGATCAGGAGGACGCGCCTCTGGTGGTGGAGGATCGGAGATCGT GATGACCCAGAGCCCTCTCTCCTTGCCCGTGACTCCTGGGGAGCCCG CATCCATTTCATGCCGGAGCTCCCAGTCACTTCTCTACTCCAACGGCTA TAACTACGTGGATTGGTACCTCCAAAAGCCGGGCCAGAGCCCGCAGCT GCTGATCTACCTGGGCTCGAACAGGGCCAGCGGAGTGCCTGACCGGT TCTCCGGGTCGGGAAGCGGGACCGACTTCAAGCTGCAAATCTCGAGA GTGGAGGCCGAGGACGTGGGAATCTACTACTGTATGCAGGGCCGCCA GTTTCCGTACTCGTTCGGACAGGGCACCAAAGTGGAAATCAAGACCAC TACCCCAGCACCGAGGCCACCCACCCCGGCTCCTACCATCGCCTCCCA GCCTCTGTCCCTGCGTCCGGAGGCATGTAGACCCGCAGCTGGTGGGG CCGTGCATACCCGGGGTCTTGACTTCGCCTGCGATATCTACATTTGGG CCCCTCTGGCTGGTACTTGCGGGGTCCTGCTGCTTTCACTCGTGATCA CTCTTTACTGTAAGCGCGGTCGGAAGAAGCTGCTGTACATCTTTAAGCA ACCCTTCATGAGGCCTGTGCAGACTACTCAAGAGGAGGACGGCTGTTC ATGCCGGTTCCCAGAGGAGGAGGAAGGCGGCTGCGAACTGCGCGTGA AATTCAGCCGCAGCGCAGATGCTCCAGCCTACAAGCAGGGGCAGAAC CAGCTCTACAACGAACTCAATCTTGGTCGGAGAGAGGAGTACGACGTG CTGGACAAGCGGAGAGGACGGGACCCAGAAATGGGCGGGAAGCCGC GCAGAAAGAATCCCCAAGAGGGCCTGTACAACGAGCTCCAAAAGGATA AGATGGCAGAAGCCTATAGCGAGATTGGTATGAAAGGGGAACGCAGAA GAGGCAAAGGCCACGACGGACTGTACCAGGGACTCAGCACCGCCACC AAGGACACCTATGACGCTCTTCACATGCAGGCCCTGCCGCCTCGG |

| **139104** | | |
|---|---|---|
| **139104- aa Full CAR** | 803 | MALPVTALLLPLALLLHAARPEVQLLETGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSEIVLTQSPATLSVSPGESATLSCRASQSVSSNLAWYQQKPGQAPRL LIYGASTRASGIPDRFSGSGSGTDFTLTISSLQAEDVAVYYCQQYGSSLTF GGGTKVEIKTTTPAPRPPTPAPTIASQPLSLRPEACRPAAGGAVHTRGLDF ACDIYIWAPLAGTCGVLLLSLVITLYCKRGRKKLLYIFKQPFMRPVQTTQEE |
| | | DGCSCRFPEEEEGGCELRVKFSRSADAPAYKQGQNQLYNELNLGRREEY DVLDKRRGRDPEMGGKPRRKNPQEGLYNELQKDKMAEAYSEIGMKGER RRGKGHDGLYQGLSTATKDTYDALHMQALPPR |
| **139104-nt Full CAR** | 804 | ATGGCCCTCCCTGTCACCGCCCTGCTGCTTCCGCTGGCTCTTCTGCTC CACGCCGCTCGGCCCGAAGTGCAATTGCTCGAAACTGGAGGAGGTCT GGTGCAACCTGGAGGATCACTTCGCCTGTCCTGCGCCGTGTCGGGCTT TGCCCTGTCCAACCATGGAATGAGCTGGGTCCGCCGCGCGCCGGGGA AGGGCCTCGAATGGGTGTCCGGCATCGTCTACTCCGGCTCCACCTACT ACGCCGCGTCCGTGAAGGGCCGGTTCACGATTTCACGGGACAACTCG CGGAACACCCTGTACCTCCAAATGAATTCCCTTCGGCCGGAGGATACT GCCATCTACTACTGCTCCGCCCACGGTGGCGAATCCGACGTCTGGGG CCAGGGAACCACCGTGACCGTGTCCAGCGCGTCCGGGGGAGGAGGAA GCGGGGGTAGAGCATCGGGTGGAGGCGGATCAGAGATCGTGCTGACC CAGTCCCCCGCCACCTTGAGCGTGTCACCAGGAGAGTCCGCCACCCT GTCATGCCGCGCCAGCCAGTCCGTGTCCTCCAACCTGGCTTGGTACCA GCAGAAGCCGGGGCAGGCCCCTAGACTCCTGATCTATGGGGCGTCGA CCCGGGCATCTGGAATTCCCGATAGGTTCAGCGGATCGGGCTCGGGC ACTGACTTCACTCTGACCATCTCCTCGCTGCAAGCCGAGGACGTGGCT GTGTACTACTGTCAGCAGTACGGAAGCTCCCTGACTTTCGGTGGCGGG ACCAAAGTCGAGATTAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTT CGCCTGCGATATCTACATTTGGGCCCCTCTGGCTGGTACTTGCGGGGT CCTGCTGCTTTCACTCGTGATCACTCTTTACTGTAAGCGCGGTCGGAAG AAGCTGCTGTACATCTTTAAGCAACCCTTCATGAGGCCTGTGCAGACTA CTCAAGAGGAGGACGGCTGTTCATGCCGGTTCCCAGAGGAGGAGGAA GGCGGCTGCGAACTGCGCGTGAAATTCAGCCGCAGCGCAGATGCTCC AGCCTACAAGCAGGGGCAGAACCAGCTCTACAACGAACTCAATCTTGG TCGGAGAGAGGAGTACGACGTGCTGGACAAGCGGAGAGGACGGGACC CAGAAATGGGCGGGAAGCCGCGCAGAAAGAATCCCCAAGAGGGCCTG TACAACGAGCTCCAAAAGGATAAGATGGCAGAAGCCTATAGCGAGATT GGTATGAAAGGGGAACGCAGAAGAGGCAAAGGCCACGACGGACTGTA CCAGGGACTCAGCACCGCCACCAAGGACACCTATGACGCTCTTCACAT GCAGGCCCTGCCGCCTCGG |

| **139106** | | |
|---|---|---|
| **139106- aa Full CAR** | 805 | MALPVTALLLPLALLLHAARPEVQLVETGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSEIVMTQSPATLSVSPGERATLSCRASQSVSSKLAWYQQKPGQAPR LLMYGASIRATGIPDRFSGSGSGTEFTLTISSLEPEDFAVYYCQQYGSSSW TFGQGTKVEIKTTTPAPRPPTPAPTIASQPLSLRPEACRPAAGGAVHTRGL DFACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGE RRRGKGHDGLYQGLSTATKDTYDALHMQALPPR |
| **139106- nt Full CAR** | 806 | ATGGCCCTCCCTGTCACCGCCCTGCTGCTTCCGCTGGCTCTTCTGCTC CACGCCGCTCGGCCCGAAGTGCAATTGGTGGAAACTGGAGGAGGACT TGTGCAACCTGGAGGATCATTGAGACTGAGCTGCGCAGTGTCGGGATT CGCCCTGAGCAACCATGGAATGTCCTGGGTCAGAAGGCCCCCTGGAA AAGGCCTCGAATGGGTGTCAGGGATCGTGTACTCCGGTTCCACTTACT ACGCCGCCTCCGTGAAGGGGCGCTTCACTATCTCACGGGATAACTCCC GCAATACCCTGTACCTCCAAATGAACAGCCTGCGGCCGGAGGATACCG CCATCTACTACTGTTCCGCCCACGGTGGAGAGTCTGACGTCTGGGGCC AGGGAACTACCGTGACCGTGTCCTCCGCGTCCGGCGGTGGAGGGAGC GGCGGCCGCGCCAGCGGCGGCGGAGGCTCCGAGATCGTGATGACCC AGAGCCCCGCTACTCTGTCGGTGTCGCCCGGAGAAAGGGCGACCCTG |
| | | TCCTGCCGGGCGTCGCAGTCCGTGAGCAGCAAGCTGGCTTGGTACCA GCAGAAGCCGGGCCAGGCACCACGCCTGCTTATGTACGGTGCCTCCA TTCGGGCCACCGGAATCCCGGACCGGTTCTCGGGGTCGGGGTCCGGT ACCGAGTTCACACTGACCATTTCCTCGCTCGAGCCCGAGGACTTTGCC GTCTATTACTGCCAGCAGTACGGCTCCTCCTCATGGACGTTCGGCCAG GGGACCAAGGTCGAAATCAAGACCACTACCCCAGCACCGAGGCCACC CACCCCGGCTCCTACCATCGCCTCCCAGCCTCTGTCCCTGCGTCCGGA GGCATGTAGACCCGCAGCTGGTGGGGCCGTGCATACCCGGGGTCTTG ACTTCGCCTGCGATATCTACATTTGGGCCCCTCTGGCTGGTACTTGCG GGGTCCTGCTGCTTTCACTCGTGATCACTCTTTACTGTAAGCGCGGTCG GAAGAAGCTGCTGTACATCTTTAAGCAACCCTTCATGAGGCCTGTGCAG ACTACTCAAGAGGAGGACGGCTGTTCATGCCGGTTCCCAGAGGAGGA GGAAGGCGGCTGCGAACTGCGCGTGAAATTCAGCCGCAGCGCAGATG CTCCAGCCTACAAGCAGGGGCAGAACCAGCTCTACAACGAACTCAATC TTGGTCGGAGAGAGGAGTACGACGTGCTGGACAAGCGGAGAGGACGG GACCCAGAAATGGGCGGGAAGCCGCGCAGAAAGAATCCCCAAGAGGG CCTGTACAACGAGCTCCAAAAGGATAAGATGGCAGAAGCCTATAGCGA GATTGGTATGAAAGGGGAACGCAGAAGAGGCAAAGGCCACGACGGAC TGTACCAGGGACTCAGCACCGCCACCAAGGACACCTATGACGCTCTTC ACATGCAGGCCCTGCCGCCTCGG |

| **139107** | | |
|---|---|---|
| **139107- aa Full CAR** | 807 | MALPVTALLLPLALLLHAARPEVQLVETGGGVVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSEIVLTQSPGTLSLSPGERATLSCRASQSVGSTNLAWYQQKPGQAP RLLIYDASNRATGIPDRFSGGGSGTDFTLTISRLEPEDFAVYYCQQYGSSP PWTFGQGTKVEIKTTTPAPRPPTPAPTIASQPLSLRPEACRPAAGGAVHTR GLDFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRR EEYDVLDKRRGRDPEMGGKPRRKNPQEGLYNELQKDKMAEAYSEIGMKG ERRRGKGHDGLYQGLSTATKDTYDALHMQALPPR |
| **139107- nt Full CAR** | 808 | ATGGCCCTCCCTGTCACCGCCCTGCTGCTTCCGCTGGCTCTTCTGCTC CACGCCGCTCGGCCCGAAGTGCAATTGGTGGAGACTGGAGGAGGAGT GGTGCAACCTGGAGGAAGCCTGAGACTGTCATGCGCGGTGTCGGGCT TCGCCCTCTCCAACCACGGAATGTCCTGGGTCCGCCGGGCCCCTGGG AAAGGACTTGAATGGGTGTCCGGCATCGTGTACTCGGGTTCCACCTAC TACGCGGCCTCAGTGAAGGGCCGGTTTACTATTAGCCGCGACAACTCC AGAAACACACTGTACCTCCAAATGAACTCGCTGCGGCCGGAAGATACC GCTATCTACTACTGCTCCGCCCATGGGGGAGAGTCGGACGTCTGGGG ACAGGGCACCACTGTCACTGTGTCCAGCGCTTCCGGCGGTGGTGGAA GCGGGGGACGGGCCTCAGGAGGCGGTGGCAGCGAGATTGTGCTGAC CCAGTCCCCCGGGACCCTGAGCCTGTCCCCGGGAGAAAGGGCCACCC TCTCCTGTCGGGCATCCCAGTCCGTGGGGTCTACTAACCTTGCATGGT ACCAGCAGAAGCCCGGCCAGGCCCCTCGCCTGCTGATCTACGACGCG TCCAATAGAGCCACCGGCATCCCGGATCGCTTCAGCGGAGGCGGATC GGGCACCGACTTCACCCTCACCATTTCAAGGCTGGAACCGGAGGACTT CGCCGTGTACTACTGCCAGCAGTATGGTTCGTCCCCACCCTGGACGTT CGGCCAGGGGACTAAGGTCGAGATCAAGACCACTACCCCAGCACCGA GGCCACCCACCCCGGCTCCTACCATCGCCTCCCAGCCTCTGTCCCTGC GTCCGGAGGCATGTAGACCCGCAGCTGGTGGGGCCGTGCATACCCGG GGTCTTGACTTCGCCTGCGATATCTACATTTGGGCCCCTCTGGCTGGTA CTTGCGGGGTCCTGCTGCTTTCACTCGTGATCACTCTTTACTGTAAGCG CGGTCGGAAGAAGCTGCTGTACATCTTTAAGCAACCCTTCATGAGGCC TGTGCAGACTACTCAAGAGGAGGACGGCTGTTCATGCCGGTTCCCAGA GGAGGAGGAAGGCGGCTGCGAACTGCGCGTGAAATTCAGCCGCAGCG CAGATGCTCCAGCCTACAAGCAGGGGCAGAACCAGCTCTACAACGAAC |
| | | TCAATCTTGGTCGGAGAGAGGAGTACGACGTGCTGGACAAGCGGAGA GGACGGGACCCAGAAATGGGCGGGAAGCCGCGCAGAAAGAATCCCCA AGAGGGCCTGTACAACGAGCTCCAAAAGGATAAGATGGCAGAAGCCTA TAGCGAGATTGGTATGAAAGGGGAACGCAGAAGAGGCAAAGGCCACG ACGGACTGTACCAGGGACTCAGCACCGCCACCAAGGACACCTATGACG CTCTTCACATGCAGGCCCTGCCGCCTCGG |

| **139108** | | |
|---|---|---|
| **139108- aa Full CAR** | 809 | MALPVTALLLPLALLLHAARPQVQLVESGGGLVKPGGSLRLSCAASGFTFS DYYMSWIRQAPGKGLEWVSYISSSGSTIYYADSVKGRFTISRDNAKNSLYL QMNSLRAEDTAVYYCARESGDGMDVWGQGTTVTVSSASGGGGSGGRA SGGGGSDIQMTQSPSSLSASVGDRVTITCRASQSISSYLNWYQQKPGKAP KLLIYAASSLQSGVPSRFSGSGSGTDFTLTISSLQPEDFATYYCQQSYTLAF GQGTKVDIKTTTPAPRPPTPAPTIASQPLSLRPEACRPAAGGAVHTRGLDF ACDIYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREEY DVLDKRRGRDPEMGGKPRRKNPQEGLYNELQKDKMAEAYSEIGMKGER RRGKGHDGLYQGLSTATKDTYDALHMQALPPR |
| **139108- nt Full CAR** | 810 | ATGGCCCTCCCTGTCACCGCCCTGCTGCTTCCGCTGGCTCTTCTGCTC CACGCCGCTCGGCCCCAAGTGCAACTCGTGGAATCTGGTGGAGGACT CGTGAAACCTGGAGGATCATTGAGACTGTCATGCGCGGCCTCGGGATT CACGTTCTCCGATTACTACATGAGCTGGATTCGCCAGGCTCCGGGGAA GGGACTGGAATGGGTGTCCTACATTTCCTCATCCGGCTCCACCATCTA CTACGCGGACTCCGTGAAGGGGAGATTCACCATTAGCCGCGATAACGC CAAGAACAGCCTGTACCTTCAGATGAACTCCCTGCGGGCTGAAGATAC TGCCGTCTACTACTGCGCAAGGGAGAGCGGAGATGGGATGGACGTCT GGGGACAGGGTACCACTGTGACCGTGTCGTCGGCCTCCGGCGGAGGG GGTTCGGGTGGAAGGGCCAGCGGCGGCGGAGGCAGCGACATCCAGA TGACCCAGTCCCCCTCATCGCTGTCCGCCTCCGTGGGCGACCGCGTC ACCATCACATGCCGGGCCTCACAGTCGATCTCCTCCTACCTCAATTGGT ATCAGCAGAAGCCCGGAAAGGCCCCTAAGCTTCTGATCTACGCAGCGT CCTCCCTGCAATCCGGGGTCCCATCTCGGTTCTCCGGCTCGGGCAGC GGTACCGACTTCACTCTGACCATCTCGAGCCTGCAGCCGGAGGACTTC GCCACTTACTACTGTCAGCAAAGCTACACCCTCGCGTTTGGCCAGGGC ACCAAAGTGGACATCAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTT CGCCTGCGATATCTACATTTGGGCCCCTCTGGCTGGTACTTGCGGGGT CCTGCTGCTTTCACTCGTGATCACTCTTTACTGTAAGCGCGGTCGGAAG AAGCTGCTGTACATCTTTAAGCAACCCTTCATGAGGCCTGTGCAGACTA CTCAAGAGGAGGACGGCTGTTCATGCCGGTTCCCAGAGGAGGAGGAA GGCGGCTGCGAACTGCGCGTGAAATTCAGCCGCAGCGCAGATGCTCC AGCCTACAAGCAGGGGCAGAACCAGCTCTACAACGAACTCAATCTTGG TCGGAGAGAGGAGTACGACGTGCTGGACAAGCGGAGAGGACGGGACC CAGAAATGGGCGGGAAGCCGCGCAGAAAGAATCCCCAAGAGGGCCTG TACAACGAGCTCCAAAAGGATAAGATGGCAGAAGCCTATAGCGAGATT GGTATGAAAGGGGAACGCAGAAGAGGCAAAGGCCACGACGGACTGTA CCAGGGACTCAGCACCGCCACCAAGGACACCTATGACGCTCTTCACAT GCAGGCCCTGCCGCCTCGG |

| **139110** | | |
|---|---|---|
| **139110-aa Full CAR** | 811 | MALPVTALLLPLALLLHAARPQVQLVQSGGGLVKPGGSLRLSCAASGFTFS DYYMSWIRQAPGKGLEWVSYISSSGNTIYYADSVKGRFTISRDNAKNSLYL QMNSLRAEDTAVYYCARSTMVREDYWGQGTLVTVSSASGGGGSGGRAS GGGGSDIVLTQSPLSLPVTLGQPASISCKSSESLVHNSGKTYLNWFHQRP GQSPRRLIYEVSNRDSGVPDRFTGSGSGTDFTLKISRVEAEDVGVYYCMQ GTHWPGTFGQGTKLEIKTTTPAPRPPTPAPTIASQPLSLRPEACRPAAGGA VHTRGLDFACDIYIWAPLAGTCGVLLLSLVITLYCKRGRKKLLYIFKQPFMRP |
| | | VQTTQEEDGCSCRFPEEEEGGCELRVKFSRSADAPAYKQGQNQLYNELN LGRREEYDVLDKRRGRDPEMGGKPRRKNPQEGLYNELQKDKMAEAYSEI GMKGERRRGKGHDGLYQGLSTATKDTYDALHMQALPPR |
| **139110-nt Full CAR** | 812 | ATGGCCCTCCCTGTCACCGCCCTGCTGCTTCCGCTGGCTCTTCTGCTC CACGCCGCTCGGCCCCAAGTGCAACTGGTGCAAAGCGGAGGAGGATT GGTCAAACCCGGAGGAAGCCTGAGACTGTCATGCGCGGCCTCTGGATT CACCTTCTCCGATTACTACATGTCATGGATCAGACAGGCCCCGGGGAA GGGCCTCGAATGGGTGTCCTACATCTCGTCCTCCGGGAACACCATCTA CTACGCCGACAGCGTGAAGGGCCGCTTTACCATTTCCCGCGACAACGC AAAGAACTCGCTGTACCTTCAGATGAATTCCCTGCGGGCTGAAGATACC GCGGTGTACTATTGCGCCCGGTCCACTATGGTCCGGGAGGACTACTGG GGACAGGGCACACTCGTGACCGTGTCCAGCGCGAGCGGGGGTGGAG GCAGCGGTGGACGCGCCTCCGGCGGCGGCGGTTCAGACATCGTGCTG ACTCAGTCGCCCCTGTCGCTGCCGGTCACCCTGGGCCAACCGGCCTC AATTAGCTGCAAGTCCTCGGAGAGCCTGGTGCACAACTCAGGAAAGAC TTACCTGAACTGGTTCCATCAGCGGCCTGGACAGTCCCCACGGAGGCT CATCTATGAAGTGTCCAACAGGGATTCGGGGGTGCCCGACCGCTTCAC TGGCTCCGGGTCCGGCACCGACTTCACCTTGAAAATCTCCAGAGTGGA AGCCGAGGACGTGGGCGTGTACTACTGTATGCAGGGTACCCACTGGC CTGGAACCTTTGGACAAGGAACTAAGCTCGAGATTAAGACCACTACCC CAGCACCGAGGCCACCCACCCCGGCTCCTACCATCGCCTCCCAGCCT CTGTCCCTGCGTCCGGAGGCATGTAGACCCGCAGCTGGTGGGGCCGT GCATACCCGGGGTCTTGACTTCGCCTGCGATATCTACATTTGGGCCCC TCTGGCTGGTACTTGCGGGGTCCTGCTGCTTTCACTCGTGATCACTCTT TACTGTAAGCGCGGTCGGAAGAAGCTGCTGTACATCTTTAAGCAACCCT TCATGAGGCCTGTGCAGACTACTCAAGAGGAGGACGGCTGTTCATGCC GGTTCCCAGAGGAGGAGGAAGGCGGCTGCGAACTGCGCGTGAAATTC AGCCGCAGCGCAGATGCTCCAGCCTACAAGCAGGGGCAGAACCAGCT CTACAACGAACTCAATCTTGGTCGGAGAGAGGAGTACGACGTGCTGGA CAAGCGGAGAGGACGGGACCCAGAAATGGGCGGGAAGCCGCGCAGA AAGAATCCCCAAGAGGGCCTGTACAACGAGCTCCAAAAGGATAAGATG GCAGAAGCCTATAGCGAGATTGGTATGAAAGGGGAACGCAGAAGAGG CAAAGGCCACGACGGACTGTACCAGGGACTCAGCACCGCCACCAAGG ACACCTATGACGCTCTTCACATGCAGGCCCTGCCGCCTCGG |

| **139112** | | |
|---|---|---|
| **139112-aa Full CAR** | 813 | MALPVTALLLPLALLLHAARPQVQLVESGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSDIRLTQSPSPLSASVGDRVTITCQASEDINKFLNWYHQTPGKAPKLLI YDASTLQTGVPSRFSGSGSGTDFTLTINSLQPEDIGTYYCQQYESLPLTFG GGTKVEIKTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYKQGQNQLYNELNLGRREEYD VLDKRRGRDPEMGGKPRRKNPQEGLYNELQKDKMAEAYSEIGMKGERR RGKGHDGLYQGLSTATKDTYDALHMQALPPR |
| **139112- nt Full CAR** | 814 | ATGGCCCTCCCTGTCACCGCCCTGCTGCTTCCGCTGGCTCTTCTGCTC CACGCCGCTCGGCCCCAAGTGCAACTCGTGGAATCTGGTGGAGGACT CGTGCAACCCGGTGGAAGCCTTAGGCTGTCGTGCGCCGTCAGCGGGT TTGCTCTGAGCAACCATGGAATGTCCTGGGTCCGCCGGGCACCGGGA AAAGGGCTGGAATGGGTGTCCGGCATCGTGTACAGCGGGTCAACCTAT TACGCCGCGTCCGTGAAGGGCAGATTCACTATCTCAAGAGACAACAGC CGGAACACCCTGTACTTGCAAATGAATTCCCTGCGCCCCGAGGACACC GCCATCTACTACTGCTCCGCCCACGGAGGAGAGTCGGACGTGTGGGG CCAGGGAACGACTGTGACTGTGTCCAGCGCATCAGGAGGGGGTGGTT CGGGCGGCCGGGCCTCGGGGGGAGGAGGTTCCGACATTCGGCTGAC CCAGTCCCCGTCCCCACTGTCGGCCTCCGTCGGCGACCGCGTGACCA |
| | | TCACTTGTCAGGCGTCCGAGGACATTAACAAGTTCCTGAACTGGTACCA CCAGACCCCTGGAAAGGCCCCCAAGCTGCTGATCTACGATGCCTCGAC CCTTCAAACTGGAGTGCCTAGCCGGTTCTCCGGGTCCGGCTCCGGCAC TGATTTCACTCTGACCATCAACTCATTGCAGCCGGAAGATATCGGGACC TACTATTGCCAGCAGTACGAATCCCTCCCGCTCACATTCGGCGGGGGA ACCAAGGTCGAGATTAAGACCACTACCCCAGCACCGAGGCCACCCACC CCGGCTCCTACCATCGCCTCCCAGCCTCTGTCCCTGCGTCCGGAGGCA TGTAGACCCGCAGCTGGTGGGGCCGTGCATACCCGGGGTCTTGACTT CGCCTGCGATATCTACATTTGGGCCCCTCTGGCTGGTACTTGCGGGGT CCTGCTGCTTTCACTCGTGATCACTCTTTACTGTAAGCGCGGTCGGAAG AAGCTGCTGTACATCTTTAAGCAACCCTTCATGAGGCCTGTGCAGACTA CTCAAGAGGAGGACGGCTGTTCATGCCGGTTCCCAGAGGAGGAGGAA GGCGGCTGCGAACTGCGCGTGAAATTCAGCCGCAGCGCAGATGCTCC AGCCTACAAGCAGGGGCAGAACCAGCTCTACAACGAACTCAATCTTGG TCGGAGAGAGGAGTACGACGTGCTGGACAAGCGGAGAGGACGGGACC CAGAAATGGGCGGGAAGCCGCGCAGAAAGAATCCCCAAGAGGGCCTG TACAACGAGCTCCAAAAGGATAAGATGGCAGAAGCCTATAGCGAGATT GGTATGAAAGGGGAACGCAGAAGAGGCAAAGGCCACGACGGACTGTA CCAGGGACTCAGCACCGCCACCAAGGACACCTATGACGCTCTTCACAT GCAGGCCCTGCCGCCTCGG |

| **139113** | | |
|---|---|---|
| **139113-aa Full CAR** | 815 | MALPVTALLLPLALLLHAARPEVQLVETGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSETTLTQSPATLSVSPGERATLSCRASQSVGSNLAWYQQKPGQGPR LLIYGASTRATGIPARFSGSGSGTEFTLTISSLQPEDFAVYYCQQYNDWLPV TFGQGTKVEIKTTTPAPRPPTPAPTIASQPLSLRPEACRPAAGGAVHTRGL DFACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGE RRRGKGHDGLYQGLSTATKDTYDALHMQALPPR |
| **139113- nt Full CAR** | 816 | ATGGCCCTCCCTGTCACCGCCCTGCTGCTTCCGCTGGCTCTTCTGCTC CACGCCGCTCGGCCCGAAGTGCAATTGGTGGAAACTGGAGGAGGACT TGTGCAACCTGGAGGATCATTGCGGCTCTCATGCGCTGTCTCCGGCTT CGCCCTGTCAAATCACGGGATGTCGTGGGTCAGACGGCCCCCGGGAA AGGGTCTGGAATGGGTGTCGGGGATTGTGTACAGCGGCTCCACCTACT ACGCCGCTTCGGTCAAGGGCCGCTTCACTATTTCACGGGACAACAGCC GCAACACCCTCTATCTGCAAATGAACTCTCTCCGCCCGGAGGATACCG CCATCTACTACTGCTCCGCACACGGCGGCGAATCCGACGTGTGGGGA CAGGGAACCACTGTCACCGTGTCGTCCGCATCCGGTGGCGGAGGATC GGGTGGCCGGGCCTCCGGGGGCGGCGGCAGCGAGACTACCCTGACC CAGTCCCCTGCCACTCTGTCCGTGAGCCCGGGAGAGAGAGCCACCCT TAGCTGCCGGGCCAGCCAGAGCGTGGGCTCCAACCTGGCCTGGTACC AGCAGAAGCCAGGACAGGGTCCCAGGCTGCTGATCTACGGAGCCTCC ACTCGCGCGACCGGCATCCCCGCGAGGTTCTCCGGGTCGGGTTCCGG GACCGAGTTCACCCTGACCATCTCCTCCCTCCAACCGGAGGACTTCGC GGTGTACTACTGTCAGCAGTACAACGATTGGCTGCCCGTGACATTTGG ACAGGGGACGAAGGTGGAAATCAAAACCACTACCCCAGCACCGAGGC CACCCACCCCGGCTCCTACCATCGCCTCCCAGCCTCTGTCCCTGCGTC CGGAGGCATGTAGACCCGCAGCTGGTGGGGCCGTGCATACCCGGGGT CTTGACTTCGCCTGCGATATCTACATTTGGGCCCCTCTGGCTGGTACTT GCGGGGTCCTGCTGCTTTCACTCGTGATCACTCTTTACTGTAAGCGCG GTCGGAAGAAGCTGCTGTACATCTTTAAGCAACCCTTCATGAGGCCTGT GCAGACTACTCAAGAGGAGGACGGCTGTTCATGCCGGTTCCCAGAGGA GGAGGAAGGCGGCTGCGAACTGCGCGTGAAATTCAGCCGCAGCGCAG ATGCTCCAGCCTACAAGCAGGGGCAGAACCAGCTCTACAACGAACTCA |
| | | ATCTTGGTCGGAGAGAGGAGTACGACGTGCTGGACAAGCGGAGAGGA CGGGACCCAGAAATGGGCGGGAAGCCGCGCAGAAAGAATCCCCAAGA GGGCCTGTACAACGAGCTCCAAAAGGATAAGATGGCAGAAGCCTATAG CGAGATTGGTATGAAAGGGGAACGCAGAAGAGGCAAAGGCCACGACG GACTGTACCAGGGACTCAGCACCGCCACCAAGGACACCTATGACGCTC TTCACATGCAGGCCCTGCCGCCTCGG |

| **139114** | | |
|---|---|---|
| **139114-aa Full CAR** | 817 | MALPVTALLLPLALLLHAARPEVQLVESGGGLVQPGGSLRLSCAVSGFALS NHGMSWVRRAPGKGLEWVSGIVYSGSTYYAASVKGRFTISRDNSRNTLYL QMNSLRPEDTAIYYCSAHGGESDVWGQGTTVTVSSASGGGGSGGRASG GGGSEIVLTQSPGTLSLSPGERATLSCRASQSIGSSSLAWYQQKPGQAPR LLMYGASSRASGIPDRFSGSGSGTDFTLTISRLEPEDFAVYYCQQYAGSPP FTFGQGTKVEIKTTTPAPRPPTPAPTIASQPLSLRPEACRPAAGGAVHTRG LDFACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGE RRRGKGHDGLYQGLSTATKDTYDALHMQALPPR |
| **139114- nt Full CAR** | 818 | ATGGCCCTCCCTGTCACCGCCCTGCTGCTTCCGCTGGCTCTTCTGCTC CACGCCGCTCGGCCCGAAGTGCAATTGGTGGAATCTGGTGGAGGACTT GTGCAACCTGGAGGATCACTGAGACTGTCATGCGCGGTGTCCGGTTTT GCCCTGAGCAATCATGGGATGTCGTGGGTCCGGCGCGCCCCCGGAAA GGGTCTGGAATGGGTGTCGGGTATCGTCTACTCCGGGAGCACTTACTA CGCCGCGAGCGTGAAGGGCCGCTTCACCATTTCCCGCGATAACTCCC GCAACACCCTGTACTTGCAAATGAACTCGCTCCGGCCTGAGGACACTG CCATCTACTACTGCTCCGCACACGGAGGAGAATCCGACGTGTGGGGCC AGGGAACTACCGTGACCGTCAGCAGCGCCTCCGGCGGCGGGGGCTCA GGCGGACGGGCTAGCGGCGGCGGTGGCTCCGAGATCGTGCTGACCC AGTCGCCTGGCACTCTCTCGCTGAGCCCCGGGGAAAGGGCAACCCTG TCCTGTCGGGCCAGCCAGTCCATTGGATCATCCTCCCTCGCCTGGTAT CAGCAGAAACCGGGACAGGCTCCGCGGCTGCTTATGTATGGGGCCAG CTCAAGAGCCTCCGGCATTCCCGACCGGTTCTCCGGGTCCGGTTCCG GCACCGATTTCACCCTGACTATCTCGAGGCTGGAGCCAGAGGACTTCG CCGTGTACTACTGCCAGCAGTACGCGGGGTCCCCGCCGTTCACGTTCG GACAGGGAACCAAGGTCGAGATCAAGACCACTACCCCAGCACCGAGG CCACCCACCCCGGCTCCTACCATCGCCTCCCAGCCTCTGTCCCTGCGT CCGGAGGCATGTAGACCCGCAGCTGGTGGGGCCGTGCATACCCGGGG TCTTGACTTCGCCTGCGATATCTACATTTGGGCCCCTCTGGCTGGTACT TGCGGGGTCCTGCTGCTTTCACTCGTGATCACTCTTTACTGTAAGCGCG GTCGGAAGAAGCTGCTGTACATCTTTAAGCAACCCTTCATGAGGCCTGT GCAGACTACTCAAGAGGAGGACGGCTGTTCATGCCGGTTCCCAGAGGA GGAGGAAGGCGGCTGCGAACTGCGCGTGAAATTCAGCCGCAGCGCAG ATGCTCCAGCCTACAAGCAGGGGCAGAACCAGCTCTACAACGAACTCA ATCTTGGTCGGAGAGAGGAGTACGACGTGCTGGACAAGCGGAGAGGA CGGGACCCAGAAATGGGCGGGAAGCCGCGCAGAAAGAATCCCCAAGA GGGCCTGTACAACGAGCTCCAAAAGGATAAGATGGCAGAAGCCTATAG CGAGATTGGTATGAAAGGGGAACGCAGAAGAGGCAAAGGCCACGACG GACTGTACCAGGGACTCAGCACCGCCACCAAGGACACCTATGACGCTC TTCACATGCAGGCCCTGCCGCCTCGG |

| **149362** | | |
|---|---|---|
| **149362-aa Full CAR** | 819 | malpvtalllplalllhaarpqvqlqesgpglvkpsetlsltctvsggsisssyyywgwirqppgkglewigsi yysgsayynpslksrvtisvdtsknqfslrlssvtaadtavyycarhwqewpdafdiwgqgtmvtvssgg ggsggggsggggsettltqspafmsatpgdkviisckasqdiddamnwyqqkpgeaplfiiqsatspvp gipprfsgsgfgtdfsltinniesedaayyfclqhdnfpltfgqgtkleikttpaprpptpaptiasqplslrpea crpaaggavhtrgldfacdiyiwaplagtcgvlllslvitlyckrgrkkllyifkqpfmrpvqttqeedgcscrfpe eeeggcelrvkfsrsadapaykqgqnqlynelnlgrreeydvldkrrgrdpemggkprrknpqeglynel qkdkmaeayseigmkgerrrgkghdglyqglstatkdtydalhmqalppr |
| **149362-nt Full CAR** | 820 | atggccctccctgtcaccgccctgctgcttccgctggctcttctgctccacgccgctcggccccaagtgcag cttcaggaaagcggaccgggcctggtcaagccatccgaaactctctccctgacttgcactgtgtctggcg gttccatctcatcgtcgtactactactggggctggattaggcagccgcccggaaagggactggagtggat cggaagcatctactattccggctcggcgtactacaaccctagcctcaagtcgagagtgaccatctccgtg gatacctccaagaaccagttttccctgcgcctgagctccgtgaccgccgctgacaccgccgtgtactactg tgctcggcattggcaggaatggcccgatgccttcgacatttggggccagggcactatggtcactgtgtcatc cgggggtggaggcagcgggggaggagggtccggggggggaggttcagagacaaccttgacccagt cacccgcattcatgtccgccactccgggagacaaggtcatcatctcgtgcaaagcgtcccaggatatcga cgatgccatgaattggtaccagcagaagcctggcgaagcgccgctgttcattatccaatccgcaacctcg cccgtgcctggaatcccaccgcggttcagcggcagcggtttcggaaccgacttttccctgaccattaacaa cattgagtccgaggacgccgcctactacttctgcctgcaacacgacaacttccctctcacgttcggccagg gaaccaagctggaaatcaagaccactaccccagcaccgaggccacccaccccggctcctaccatcgc ctcccagcctctgtccctgcgtccggaggcatgtagacccgcagctggtggggccgtgcatacccggggt cttgacttcgcctgcgatatctacatttgggcccctctggctggtacttgcggggtcctgctgctttcactcgtga tcactctttactgtaagcgcggtcggaagaagctgctgtacatctttaagcaacccttcatgaggcctgtgca gactactcaagaggaggacggctgttcatgccggttcccagaggaggaggaaggcggctgcgaactg cgcgtgaaattcagccgcagcgcagatgctccagcctacaagcaggggcagaaccagctctacaacg aactcaatcttggtcggagagaggagtacgacgtgctggacaagcggagaggacgggacccagaaa tgggcgggaagccgcgcagaaagaatccccaagagggcctgtacaacgagctccaaaaggataag atggcagaagcctatagcgagattggtatgaaaggggaacgcagaagaggcaaaggccacgacgg actgtaccagggactcagcaccgccaccaaggacacctatgacgctcttcacatgcaggccctgccgcc tcgg |

| **149363** | | |
|---|---|---|
| **149363-aa Full CAR** | 821 | malpvtalllplalllhaarpqvnlresgpalvkptqtltltctfsgfslrtsgmcvswirqppgkalewlaridwd edkfystslktrltiskdtsdnqvvlrmtnmdpadtatyycarsgaggtsatafdiwgpgtmvtvssggggs ggggsggggsdiqmtqspsslsasvgdrvtitcrasqdiynnlawfqlkpgsaprslmyaanksqsgvp srfsgsasgtdftltisslqpedfatyycqhyyrfpysfgqgtkleikttpaprpptpaptiasqplslrpeacrp aaggavhtrgldfacdiyiwaplagtcgvlllslvitlyckrgrkkllyifkqpfmrpvqttqeedgcscrfpeee eggcelrvkfsrsadapaykqgqnqlynelnlgrreeydvldkrrgrdpemggkprrknpqeglynelqk dkmaeayseigmkgerrrgkghdglyqglstatkdtydalhmqalppr |
| **149363-nt Full CAR** | 822 | atggccctccctgtcaccgccctgctgcttccgctggctcttctgctccacgccgctcggccccaagtcaatc tgcgcgaatccggccccgccttggtcaagcctacccagaccctcactctgacctgtactttctccggcttctc cctgcggacttccgggatgtgcgtgtcctggatcagacagcctccgggaaaggccctggagtggctcgct cgcattgactgggatgaggacaagttctactccacctcactcaagaccaggctgaccatcagcaaagat acctctgacaaccaagtggtgctccgcatgaccaacatggacccagccgacactgccacttactactgc gcgaggagcggagcgggcggaacctccgccaccgccttcgatatttggggcccgggtaccatggtcac cgtgtcaagcggaggaggggggtccgggggcggcggttccgggggaggcggatcggacattcagat gactcagtcaccatcgtccctgagcgctagcgtgggcgacagagtgacaatcacttgccgggcatccca ggacatctataacaaccttgcgtggttccagctgaagcctggttccgcaccgcggtcacttatgtacgccg ccaacaagagccagtcgggagtgccgtcccggttttccggttcggcctcgggaactgacttcaccctgac gatctccagcctgcaacccgaggatttcgccacctactactgccagcactactaccgctttccctactcgttc ggacagggaaccaagctggaaatcaagaccactaccccagcaccgaggccacccaccccggctcct accatcgcctcccagcctctgtccctgcgtccggaggcatgtagacccgcagctggtggggccgtgcata cccggggtcttgacttcgcctgcgatatctacatttgggcccctctggctggtacttgcggggtcctgctgcttt cactcgtgatcactctttactgtaagcgcggtcggaagaagctgctgtacatctttaagcaacccttcatgag gcctgtgcagactactcaagaggaggacggctgttcatgccggttcccagaggaggaggaaggcggct gcgaactgcgcgtgaaattcagccgcagcgcagatgctccagcctacaagcaggggcagaaccagct ctacaacgaactcaatcttggtcggagagaggagtacgacgtgctggacaagcggagaggacgggac ccagaaatgggcgggaagccgcgcagaaagaatccccaagagggcctgtacaacgagctccaaaa ggataagatggcagaagcctatagcgagattggtatgaaaggggaacgcagaagaggcaaaggcca cgacggactgtaccagggactcagcaccgccaccaaggacacctatgacgctcttcacatgcaggccc tgccgcctcgg |

| **149364** | | |
|---|---|---|
| **149364-aa Full CAR** | 823 | malpvtalllplalllhaarpevqlvesggglvkpggslrlscaasgftfssysmnwvrqapgkglewvssis ssssyiyyadsvkgrftisrdnaknslylqmnslraedtavyycaktiaavyafdiwgqgttvtvssggggs ggggsggggseivltqsplslpvtpeepasiscrssqsllhsngynyldwylqkpgqspqlliylgsnrasg |
| | | vpdrfsgsgsgtdftlkisrveaedvgvyycmqalqtpytfgqgtkleikttpaprpptpaptiasqplslrpe acrpaaggavhtrgldfacdiyiwaplagtcgvlllslvitlyckrgrkkllyifkqpfmrpvqttqeedgcscrfp eeeeggcelrvkfsrsadapaykqgqnqlynelnlgrreeydvldkrrgrdpemggkprrknpqeglyn elqkdkmaeayseigmkgerrrgkghdglyqglstatkdtydalhmqalppr |
| **149364-nt Full CAR** | 824 | atggccctccctgtcaccgccctgctgcttccgctggctcttctgctccacgccgctcggcccgaagtgcag cttgtcgaatccggggggggactggtcaagccgggcggatcactgagactgtcctgcgccgcgagcgg cttcacgttctcctcctactccatgaactgggtccgccaagcccccgggaagggactggaatgggtgtcct ctatctcctcgtcgtcgtcctacatctactacgccgactccgtgaagggaagattcaccatttcccgcgaca acgcaaagaactcactgtacttgcaaatgaactcactccgggccgaagatactgctgtgtactattgcgcc aagactattgccgccgtctacgctttcgacatctggggccagggaaccaccgtgactgtgtcgtccggtgg tggtggctcgggcggaggaggaagcggcggcggggggtccgagattgtgctgacccagtcgccactg agcctccctgtgacccccgaggaacccgccagcatcagctgccggtccagccagtccctgctccactcc aacggatacaattacctcgattggtaccttcagaagcctggacaaagcccgcagctgctcatctacttggg atcaaaccgcgcgtcaggagtgcctgaccggttctccggctcgggcagcggtaccgatttcaccctgaa aatctccagggtggaggcagaggacgtgggagtgtattactgtatgcaggcgctgcagactccgtacac atttgggcagggcaccaagctggagatcaagaccactaccccagcaccgaggccacccaccccggct cctaccatcgcctcccagcctctgtccctgcgtccggaggcatgtagacccgcagctggtggggccgtgc atacccggggtcttgacttcgcctgcgatatctacatttgggcccctctggctggtacttgcggggtcctgctg ctttcactcgtgatcactctttactgtaagcgcggtcggaagaagctgctgtacatctttaagcaacccttcat gaggcctgtgcagactactcaagaggaggacggctgttcatgccggttcccagaggaggaggaaggc ggctgcgaactgcgcgtgaaattcagccgcagcgcagatgctccagcctacaagcaggggcagaacc agctctacaacgaactcaatcttggtcggagagaggagtacgacgtgctggacaagcggagaggacg ggacccagaaatgggcgggaagccgcgcagaaagaatccccaagagggcctgtacaacgagctcc aaaaggataagatggcagaagcctatagcgagattggtatgaaaggggaacgcagaagaggcaaa ggccacgacggactgtaccagggactcagcaccgccaccaaggacacctatgacgctcttcacatgca ggccctgccgcctcgg |

| **149365** | | |
|---|---|---|
| **149365-aa Full CAR** | 825 | malpvtalllplalllhaarpevqlvesggglvkpggslrlscaasgftfsdyymswirqapgkglewvsyis ssgstiyyadsvkgrftisrdnaknslylqmnslraedtavyycardlrgafdiwgqgtmvtvssggggsg gggsggggssyvltqspsvsaapgytatiscggnnigtksvhwyqqkpgqapllvirddsvrpskipgrfs gsnsgnmatltisgvqagdeadfycqvwdsdsehvvfgggtkltvltttpaprpptpaptiasqplslrpea crpaaggavhtrgldfacdiyiwaplagtcgvlllslvitlyckrgrkkllyifkqpfmrpvqttqeedgcscrfpe eeeggcelrvkfsrsadapaykqgqnqlynelnlgrreeydvldkrrgrdpemggkprrknpqeglynel qkdkmaeayseigmkgerrrgkghdglyqglstatkdtydalhmqalppr |
| **149365-nt Full CAR** | 826 | atggccctccctgtcaccgccctgctgcttccgctggctcttctgctccacgccgctcggcccgaagtccag ctcgtggagtccggcggaggccttgtgaagcctggaggttcgctgagactgtcctgcgccgcctccggctt caccttctccgactactacatgtcctggatcagacaggccccgggaaagggcctggaatgggtgtcctac atctcgtcatcgggcagcactatctactacgcggactcagtgaaggggcggttcaccatttcccgggataa cgcgaagaactcgctgtatctgcaaatgaactcactgagggccgaggacaccgccgtgtactactgcgc ccgcgatctccgcggggcatttgacatctggggacagggaaccatggtcacagtgtccagcggagggg gaggatcgggtggcggaggttccgggggtggaggctcctcctacgtgctgactcagagcccaagcgtc agcgctgcgcccggttacacggcaaccatctcctgtggcggaaacaacattgggaccaagtctgtgcac tggtatcagcagaagccgggccaagctcccctgttggtgatccgcgatgactccgtgcggcctagcaaa attccgggacggttctccggctccaacagcggcaatatggccactctcaccatctcgggagtgcaggccg gagatgaagccgacttctactgccaagtctgggactcagactccgagcatgtggtgttcgggggcggaa ccaagctgactgtgctcaccactaccccagcaccgaggccacccaccccggctcctaccatcgcctccc agcctctgtccctgcgtccggaggcatgtagacccgcagctggtggggccgtgcatacccggggtcttga cttcgcctgcgatatctacatttgggcccctctggctggtacttgcggggtcctgctgctttcactcgtgatcact ctttactgtaagcgcggtcggaagaagctgctgtacatctttaagcaacccttcatgaggcctgtgcagact actcaagaggaggacggctgttcatgccggttcccagaggaggaggaaggcggctgcgaactgcgcg tgaaattcagccgcagcgcagatgctccagcctacaagcaggggcagaaccagctctacaacgaact caatcttggtcggagagaggagtacgacgtgctggacaagcggagaggacgggacccagaaatggg cgggaagccgcgcagaaagaatccccaagagggcctgtacaacgagctccaaaaggataagatgg cagaagcctatagcgagattggtatgaaaggggaacgcagaagaggcaaaggccacgacggactgt accagggactcagcaccgccaccaaggacacctatgacgctcttcacatgcaggccctgccgcctcgg |

| 149366 | | |
|---|---|---|
| **149366-aa Full CAR** | 827 | malpvtalllplalllhaarpqvqlvqsgaevkkpgasvkvsckpsgytvtshyihwvrrapgqglewmg minpsggvtaysqtlqgrvtmtsdtsssstvymelsslrsedtamyycaregsgsgwyfdfwgrgtlvtvss ggggsggggsggggssyvltqppsvsvspgqtasitcsgdglskkyvswyqqkagqspvvlisrdker psgipdrfsgsnsadtatltisgtqamdeadyycqawddttvvfgggtkltvltttpaprpptpaptiasqpls lrpeacrpaaggavhtrgldfacdiyiwaplagtcgvlllslvitlyckrgrkkllyifkqpfmrpvqttqeedgcs crfpeeeeggcelrvkfsrsadapaykqgqnqlynelnlgrreeydvldkrrgrdpemggkprrknpqeg lynelqkdkmaeayseigmkgerrrgkghdglyqglstatkdtydalhmqalppr |
| **149366-nt Full CAR** | 828 | atggccctccctgtcaccgccctgctgcttccgctggctcttctgctccacgccgctcggccccaagtgcag ctggtgcagagcggggccgaagtcaagaagccgggagcctccgtgaaagtgtcctgcaagccttcgg gatacaccgtgacctcccactacattcattgggtccgccgcgcccccggccaaggactcgagtggatgg gcatgatcaaccctagcggcggagtgaccgcgtacagccagacgctgcagggacgcgtgactatgac ctcggatacctcctcctccaccgtctatatggaactgtccagcctgcggtccgaggataccgccatgtacta ctgcgcccgggaaggatcaggctccgggtggtatttcgacttctggggaagaggcaccctcgtgactgtg tcatctgggggagggggttccggtggtggcggatcgggaggaggcggttcatcctacgtgctgacccag ccaccctccgtgtccgtgagccccggccagactgcatcgattacatgtagcggcgacggcctctccaag aaatacgtgtcgtggtaccagcagaaggccggacagagcccggtggtgctgatctcaagagataagga gcggcctagcggaatcccggacaggttctcgggttccaactccgcggacactgctactctgaccatctcg gggacccaggctatggacgaagccgattactactgccaagcctgggacgacactactgtcgtgtttgga gggggcaccaagttgaccgtccttaccactaccccagcaccgaggccacccaccccggctcctaccat cgcctcccagcctctgtccctgcgtccggaggcatgtagacccgcagctggtggggccgtgcatacccg gggtcttgacttcgcctgcgatatctacatttgggcccctctggctggtacttgcggggtcctgctgctttcactc gtgatcactctttactgtaagcgcggtcggaagaagctgctgtacatctttaagcaacccttcatgaggcct gtgcagactactcaagaggaggacggctgttcatgccggttcccagaggaggaggaaggcggctgcg aactgcgcgtgaaattcagccgcagcgcagatgctccagcctacaagcaggggcagaaccagctcta caacgaactcaatcttggtcggagagaggagtacgacgtgctggacaagcggagaggacgggaccc agaaatgggcgggaagccgcgcagaaagaatccccaagagggcctgtacaacgagctccaaaagg ataagatggcagaagcctatagcgagattggtatgaaaggggaacgcagaagaggcaaaggccacg acggactgtaccagggactcagcaccgccaccaaggacacctatgacgctcttcacatgcaggccctg ccgcctcgg |

| **149367** | | |
|---|---|---|
| **149367-aa Full CAR** | 829 | malpvtalllplalllhaarpqvqlqesgpglvkpsqtlsltctvsggsissggyywswirqhpgkglewigyi yysgstyynpslksrvtisvdtsknqfslklssvtaadtavyycaragiaarlrgafdiwgqgtmvtvssggg gsggggsggggsdivmtqspssvsasvgdrviitcrasqgirnwlawyqqkpgkapnlliyaasnlqsg vpsrfsgsgsgadftltisslqpedvatyycqkynsapftfgpgtkvdikttpaprpptpaptiasqplslrpe acrpaaggavhtrgldfacdiyiwaplagtcgvlllslvitlyckrgrkkllyifkqpfmrpvqttqeedgcscrfp eeeeggcelrvkfsrsadapaykqgqnqlynelnlgrreeydvldkrrgrdpemggkprrknpqeglyn elqkdkmaeayseigmkgerrrgkghdglyqglstatkdtydalhmqalppr |
| **149367-nt Full CAR** | 830 | atggccctccctgtcaccgccctgctgcttccgctggctcttctgctccacgccgctcggccccaagtgcag cttcaggagagcggcccgggactcgtgaagccgtcccagaccctgtccctgacttgcaccgtgtcggga ggaagcatctcgagcggaggctactattggtcgtggattcggcagcaccctggaaagggcctggaatgg atcggctacatctactactccggctcgacctactacaacccatcgctgaagtccagagtgacaatctcagt ggacacgtccaagaatcagttcagcctgaagctctcttccgtgactgcggccgacaccgccgtgtactact gcgcacgcgctggaattgccgcccggctgaggggtgccttcgacatttggggacagggcaccatggtca ccgtgtcctccggcggcggaggttccgggggtggaggctcaggaggaggggggtccgacatcgtcatg actcagtcgccctcaagcgtcagcgcgtccgtcggggacagagtgatcatcacctgtcgggcgtcccag ggaattcgcaactggctggcctggtatcagcagaagcccggaaaggcccccaacctgttgatctacgcc gcctcaaacctccaatccggggtgccgagccgcttcagcggctccggttcgggtgccgatttcactctgac catctcctccctgcaacctgaagatgtggctacctactactgccaaaagtacaactccgcaccttttactttc ggaccggggaccaaagtggacattaagaccactaccccagcaccgaggccacccaccccggctcct accatcgcctcccagcctctgtccctgcgtccggaggcatgtagacccgcagctggtggggccgtgcata cccggggtcttgacttcgcctgcgatatctacatttgggcccctctggctggtacttgcggggtcctgctgcttt cactcgtgatcactctttactgtaagcgcggtcggaagaagctgctgtacatctttaagcaacccttcatgag gcctgtgcagactactcaagaggaggacggctgttcatgccggttcccagaggaggaggaaggcggct gcgaactgcgcgtgaaattcagccgcagcgcagatgctccagcctacaagcaggggcagaaccagct ctacaacgaactcaatcttggtcggagagaggagtacgacgtgctggacaagcggagaggacgggac ccagaaatgggcgggaagccgcgcagaaagaatccccaagagggcctgtacaacgagctccaaaa |
| | | ggataagatggcagaagcctatagcgagattggtatgaaaggggaacgcagaagaggcaaaggcca cgacggactgtaccagggactcagcaccgccaccaaggacacctatgacgctcttcacatgcaggccc tgccgcctcgg |

| **149368** | | |
|---|---|---|
| **149368-aa Full CAR** | 831 | malpvtalllplalllhaarpqvqlvqsgaevkkpgssvkvsckasggtfssyaiswvrqapgqglewmg giipifgtanyaqkfqgrvtitadeststaymelsslrsedtavyycarrggyqllrwdvgllrsafdiwgqgtm vtvssggggsggggsggggssyvltqppsvsvapgqtaritcggnnigsksvhwyqqkpgqapvlvly gknnrpsgvpdrfsgsrsgttasltitgaqaedeadyycssrdssgdhlrvfgtgtkvtvltttpaprpptpap tiasqplslrpeacrpaaggavhtrgldfacdiyiwaplagtcgvlllslvitlyckrgrkkllyifkqpfmrpvqtt qeedgcscrfpeeeeggcelrvkfsrsadapaykqgqnqlynelnlgrreeydvldkrrgrdpemggkp rrknpqeglynelqkdkmaeayseigmkgerrrgkghdglyqglstatkdtydalhmqalppr |
| **149368-nt Full CAR** | 832 | atggccctccctgtcaccgccctgctgcttccgctggctcttctgctccacgccgctcggccccaagtgcag ctggtccagtcgggcgccgaggtcaagaagcccgggagctctgtgaaagtgtcctgcaaggcctccgg gggcacctttagctcctacgccatctcctgggtccgccaagcaccgggtcaaggcctggagtggatggg gggaattatccctatcttcggcactgccaactacgcccagaagttccagggacgcgtgaccattaccgcg gacgaatccacctccaccgcttatatggagctgtccagcttgcgctcggaagataccgccgtgtactactg cgcccggaggggtggataccagctgctgagatgggacgtgggcctcctgcggtcggcgttcgacatctg gggccagggcactatggtcactgtgtccagcggaggaggcggatcgggaggcggcggatcaggggg aggcggttccagctacgtgcttactcaacccccttcggtgtccgtggccccgggacagaccgccagaatc acttgcggaggaaacaacattgggtccaagagcgtgcattggtaccagcagaagccaggacaggccc ctgtgctggtgctctacgggaagaacaatcggcccagcggagtgccggacaggttctcgggttcacgctc cggtacaaccgcttcactgactatcaccggggcccaggcagaggatgaagcggactactactgttcctc ccgggattcatccggcgaccacctccgggtgttcggaaccggaacgaaggtcaccgtgctgaccactac cccagcaccgaggccacccaccccggctcctaccatcgcctcccagcctctgtccctgcgtccggaggc atgtagacccgcagctggtggggccgtgcatacccggggtcttgacttcgcctgcgatatctacatttgggc ccctctggctggtacttgcggggtcctgctgctttcactcgtgatcactctttactgtaagcgcggtcggaaga agctgctgtacatctttaagcaacccttcatgaggcctgtgcagactactcaagaggaggacggctgttca tgccggttcccagaggaggaggaaggcggctgcgaactgcgcgtgaaattcagccgcagcgcagatg ctccagcctacaagcaggggcagaaccagctctacaacgaactcaatcttggtcggagagaggagtac gacgtgctggacaagcggagaggacgggacccagaaatgggcgggaagccgcgcagaaagaatc cccaagagggcctgtacaacgagctccaaaaggataagatggcagaagcctatagcgagattggtatg aaaggggaacgcagaagaggcaaaggccacgacggactgtaccagggactcagcaccgccacca aggacacctatgacgctcttcacatgcaggccctgccgcctcgg |

| **149369** | | |
|---|---|---|
| **149369-aa Full CAR** | 833 | malpvtalllplalllhaarpevqlqqsgpglvkpsqtlsltcaisgdsvssnsaawnwirqspsrglewlgrt yyrskwysfyaislksriiinpdtsknqfslqlksvtpedtavyycarsspeglflywfdpwgqgtlvtvssgg dgsggggsggggssseltqdpavsvalgqtiritcqgdslgnyyatwyqqkpgqapvlviygtnnrpsgi pdrfsasssgntasltitgaqaedeadyycnsrdssghhllfgtgtkvtvltttpaprpptpaptiasqplslrp eacrpaaggavhtrgldfacdiyiwaplagtcgvlllslvitlyckrgrkkllyifkqpfmrpvqttqeedgcscrf peeeeggcelrvkfsrsadapaykqgqnqlynelnlgrreeydvldkrrgrdpemggkprrknpqegly nelqkdkmaeayseigmkgerrrgkghdglyqglstatkdtydalhmqalppr |
| **149369-nt Full CAR** | 834 | atggccctccctgtcaccgccctgctgcttccgctggctcttctgctccacgccgctcggcccgaagtgcag ctccaacagtcaggaccggggctcgtgaagccatcccagaccctgtccctgacttgtgccatctcgggag atagcgtgtcatcgaactccgccgcctggaactggattcggcagagcccgtcccgcggactggagtggc ttggaaggacctactaccggtccaagtggtactctttctacgcgatctcgctgaagtcccgcattatcattaa ccctgatacctccaagaatcagttctccctccaactgaaatccgtcacccccgaggacacagcagtgtatt actgcgcacggagcagccccgaaggactgttcctgtattggtttgacccctggggccaggggactcttgtg accgtgtcgagcggcggagatgggtccggtggcggtggttcggggggcggcggatcatcatccgaact gacccaggacccggctgtgtccgtggcgctgggacaaaccatccgcattacgtgccagggagactccct gggcaactactacgccacttggtaccagcagaagccgggccaagcccctgtgttggtcatctacgggac caacaacagaccttccggcatccccgaccggttcagcgcttcgtcctccggcaacactgccagcctgac catcactggagcgcaggccgaagatgaggccgactactactgcaacagcagagactcctcgggtcatc acctcttgttcggaactggaaccaaggtcaccgtgctgaccactaccccagcaccgaggccacccaccc cggctcctaccatcgcctcccagcctctgtccctgcgtccggaggcatgtagacccgcagctggtggggc cgtgcatacccggggtcttgacttcgcctgcgatatctacatttgggcccctctggctggtacttgcggggtcc tgctgctttcactcgtgatcactctttactgtaagcgcggtcggaagaagctgctgtacatctttaagcaaccc |
| | | ttcatgaggcctgtgcagactactcaagaggaggacggctgttcatgccggttcccagaggaggaggaa ggcggctgcgaactgcgcgtgaaattcagccgcagcgcagatgctccagcctacaagcaggggcaga accagctctacaacgaactcaatcttggtcggagagaggagtacgacgtgctggacaagcggagagg acgggacccagaaatgggcgggaagccgcgcagaaagaatccccaagagggcctgtacaacgag ctccaaaaggataagatggcagaagcctatagcgagattggtatgaaaggggaacgcagaagaggc aaaggccacgacggactgtaccagggactcagcaccgccaccaaggacacctatgacgctcttcacat gcaggccctgccgcctcgg |

| **BCMA_EBB-C1978-A4** | | |
|---|---|---|
| **BCMA_EBB-C1978-A4 - aa Full CART** | 835 | MALPVTALLLPLALLLHAARPEVQLVESGGGLVQPGGSLRLSCAASGFTFS SYAMSWVRQAPGKGLEWVSAISGSGGSTYYADSVKGRFTISRDNSKNTLY LQMNSLRAEDTAVYYCAKVEGSGSLDYWGQGTLVTVSSGGGGSGGGGS GGGGSEIVMTQSPGTLSLSPGERATLSCRASQSVSSAYLAWYQQKPGQP PRLLISGASTRATGIPDRFGGSGSGTDFTLTISRLEPEDFAVYYCQHYGSSF NGSSLFTFGQGTRLEIKTTTPAPRPPTPAPTIASQPLSLRPEACRPAAGGA VHTRGLDFACDIYIWAPLAGTCGVLLLSLVITLYCKRGRKKLLYIFKQPFMRP VQTTQEEDGCSCRFPEEEEGGCELRVKFSRSADAPAYKQGQNQLYNELN LGRREEYDVLDKRRGRDPEMGGKPRRKNPQEGLYNELQKDKMAEAYSEI GMKGERRRGKGHDGLYQGLSTATKDTYDALHMQALPPR |
| **BCMA_EBB-C1978-A4 - nt Full CART** | 836 | ATGGCCCTCCCTGTCACCGCCCTGCTGCTTCCGCTGGCTCTTCTGCTC CACGCCGCTCGGCCCGAAGTGCAGCTCGTGGAGTCAGGAGGCGGCCT GGTCCAGCCGGGAGGGTCCCTTAGACTGTCATGCGCCGCAAGCGGAT TCACTTTCTCCTCCTATGCCATGAGCTGGGTCCGCCAAGCCCCCGGAA AGGGACTGGAATGGGTGTCCGCCATCTCGGGGTCTGGAGGCTCAACTT ACTACGCTGACTCCGTGAAGGGACGGTTCACCATTAGCCGCGACAACT CCAAGAACACCCTCTACCTCCAAATGAACTCCCTGCGGGCCGAGGATA CCGCCGTCTACTACTGCGCCAAAGTGGAAGGTTCAGGATCGCTGGACT ACTGGGGACAGGGTACTCTCGTGACCGTGTCATCGGGCGGAGGAGGT TCCGGCGGTGGCGGCTCCGGCGGCGGAGGGTCGGAGATCGTGATGA CCCAGAGCCCTGGTACTCTGAGCCTTTCGCCGGGAGAAAGGGCCACC CTGTCCTGCCGCGCTTCCCAATCCGTGTCCTCCGCGTACTTGGCGTGG TACCAGCAGAAGCCGGGACAGCCCCCTCGGCTGCTGATCAGCGGGGC CAGCACCCGGGCAACCGGAATCCCAGACAGATTCGGGGGTTCCGGCA GCGGCACAGATTTCACCCTGACTATTTCGAGGTTGGAGCCCGAGGACT TTGCGGTGTATTACTGTCAGCACTACGGGTCGTCCTTTAATGGCTCCAG CCTGTTCACGTTCGGACAGGGGACCCGCCTGGAAATCAAGACCACTAC CCCAGCACCGAGGCCACCCACCCCGGCTCCTACCATCGCCTCCCAGC CTCTGTCCCTGCGTCCGGAggcatgtagacccgcagctggtggggccgtgcatacccggg gtcttgacttcgcctgcgatatctacatttgggcccctctggctggtacttgcggggtcctgctgctttcactcgt gatcactctttactgtaagcgcggtcggaagaagctgctgtacatctttaagcaacccttcatgaggcctgt gcagactactcaagaggaggacggctgttcatgccggttcccagaggaggaggaaggcggctgcgaa ctgcgcgtgaaattcagccgcagcgcagatgctccagcctacaagcaggggcagaaccagctctaca acgaactcaatcttggtcggagagaggagtacgacgtgctggacaagcggagaggacgggacccag aaatgggcgggaagccgcgcagaaagaatccccaagagggcctgtacaacgagctccaaaaggat aagatggcagaagcctatagcgagattggtatgaaaggggaacgcagaagaggcaaaggccacga cggactgtaccagggactcagcaccgccaccaaggacacctatgacgctcttcacatgcaggccctgc cgcctcgg |

| **BCMA_EBB-C1978-G1** | | |
|---|---|---|
| **BCMA_EBB-C1978-G1 - aa Full CART** | 837 | MALPVTALLLPLALLLHAARPEVQLVETGGGLVQPGGSLRLSCAASGITFS RYPMSWVRQAPGKGLEWVSGISDSGVSTYYADSAKGRFTISRDNSKNTLF LQMSSLRDEDTAVYYCVTRAGSEASDIWGQGTMVTVSSGGGGSGGGGS GGGGSEIVLTQSPATLSLSPGERATLSCRASQSVSNSLAWYQQKPGQAPR LLIYDASSRATGIPDRFSGSGSGTDFTLTISRLEPEDFAIYYCQQFGTSSGLT FGGGTKLEIKTTTPAPRPPTPAPTIASQPLSLRPEACRPAAGGAVHTRGLD FACDIYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREEY DVLDKRRGRDPEMGGKPRRKNPQEGLYNELQKDKMAEAYSEIGMKGER |
| | | RRGKGHDGLYQGLSTATKDTYDALHMQALPPR |
| **BCMA_EBB-C1978-G1 - nt Full CART** | 838 | ATGGCCCTCCCTGTCACCGCCCTGCTGCTTCCGCTGGCTCTTCTGCTC CACGCCGCTCGGCCCGAAGTGCAACTGGTGGAAACCGGTGGCGGCCT GGTGCAGCCTGGAGGATCATTGAGGCTGTCATGCGCGGCCAGCGGTA TTACCTTCTCCCGGTACCCCATGTCCTGGGTCAGACAGGCCCCGGGGA AAGGGCTTGAATGGGTGTCCGGGATCTCGGACTCCGGTGTCAGCACTT ACTACGCCGACTCCGCCAAGGGACGCTTCACCATTTCCCGGGACAACT CGAAGAACACCCTGTTCCTCCAAATGAGCTCCCTCCGGGACGAGGATA CTGCAGTGTACTACTGCGTGACCCGCGCCGGGTCCGAGGCGTCTGAC ATTTGGGGACAGGGCACTATGGTCACCGTGTCGTCCGGCGGAGGGGG CTCGGGAGGCGGTGGCAGCGGAGGAGGAGGGTCCGAGATCGTGCTG ACCCAATCCCCGGCCACCCTCTCGCTGAGCCCTGGAGAAAGGGCAAC CTTGTCCTGTCGCGCGAGCCAGTCCGTGAGCAACTCCCTGGCCTGGTA CCAGCAGAAGCCCGGACAGGCTCCGAGACTTCTGATCTACGACGCTTC GAGCCGGGCCACTGGAATCCCCGACCGCTTTTCGGGGTCCGGCTCAG GAACCGATTTCACCCTGACAATCTCACGGCTGGAGCCAGAGGATTTCG CCATCTATTACTGCCAGCAGTTCGGTACTTCCTCCGGCCTGACTTTCGG AGGCGGCACGAAGCTCGAAATCAAGACCACTACCCCAGCACCGAGGC CACCCACCCCGGCTCCTACCATCGCCTCCCAGCCTCTGTCCCTGCGTC CGGAggcatgtagacccgcagctggtggggccgtgcatacccggggtcttgacttcgcctgcgatatct acatttgggcccctctggctggtacttgcggggtcctgctgctttcactcgtgatcactctttactgtaagcgcg gtcggaagaagctgctgtacatctttaagcaacccttcatgaggcctgtgcagactactcaagaggagga cggctgttcatgccggttcccagaggaggaggaaggcggctgcgaactgcgcgtgaaattcagccgca gcgcagatgctccagcctacaagcaggggcagaaccagctctacaacgaactcaatcttggtcggaga gaggagtacgacgtgctggacaagcggagaggacgggacccagaaatgggcgggaagccgcgca gaaagaatccccaagagggcctgtacaacgagctccaaaaggataagatggcagaagcctatagcg agattggtatgaaaggggaacgcagaagaggcaaaggccacgacggactgtaccagggactcagc accgccaccaaggacacctatgacgctcttcacatgcaggccctgccgcctcgg |

| **BCMA_EBB-C1979-C1** | | |
|---|---|---|
| **BCMA_EBB-C1979-C1 - aa Full CART** | 839 | MALPVTALLLPLALLLHAARPQVQLVESGGGLVQPGGSLRLSCAASGFTFS SYAMSWVRQAPGKGLEWVSAISGSGGSTYYADSVKGRFTISRDNAKNSL YLQMNSLRAEDTAIYYCARATYKRELRYYYGMDVWGQGTMVTVSSGGGG SGGGGSGGGGSEIVMTQSPGTVSLSPGERATLSCRASQSVSSSFLAWYQ QKPGQAPRLLIYGASSRATGIPDRFSGSGSGTDFTLTISRLEPEDSAVYYC QQYHSSPSWTFGQGTRLEIKTTTPAPRPPTPAPTIASQPLSLRPEACRPAA GGAVHTRGLDFACDIYIWAPLAGTCGVLLLSLVITLYCKRGRKKLLYIFKQPF MRPVQTTQEEDGCSCRFPEEEEGGCELRVKFSRSADAPAYKQGQNQLYN ELNLGRREEYDVLDKRRGRDPEMGGKPRRKNPQEGLYNELQKDKMAEAY SEIGMKGERRRGKGHDGLYQGLSTATKDTYDALHMQALPPR |
| **BCMA_EBB-C1979-C1 - nt Full CART** | 840 | ATGGCCCTCCCTGTCACCGCCCTGCTGCTTCCGCTGGCTCTTCTGCTC CACGCCGCTCGGCCCCAAGTGCAGCTCGTGGAATCGGGTGGCGGACT GGTGCAGCCGGGGGGCTCACTTAGACTGTCCTGCGCGGCCAGCGGAT TCACTTTCTCCTCCTACGCCATGTCCTGGGTCAGACAGGCCCCTGGAA AGGGCCTGGAATGGGTGTCCGCAATCAGCGGCAGCGGCGGCTCGACC TATTACGCGGATTCAGTGAAGGGCAGATTCACCATTTCCCGGGACAAC GCCAAGAACTCCTTGTACCTTCAAATGAACTCCCTCCGCGCGGAAGATA CCGCAATCTACTACTGCGCTCGGGCCACTTACAAGAGGGAACTGCGCT ACTACTACGGGATGGACGTCTGGGGCCAGGGAACCATGGTCACCGTG TCCAGCGGAGGAGGAGGATCGGGAGGAGGCGGTAGCGGGGGTGGAG GGTCGGAGATCGTGATGACCCAGTCCCCCGGCACTGTGTCGCTGTCC CCCGGCGAACGGGCCACCCTGTCATGTCGGGCCAGCCAGTCAGTGTC GTCAAGCTTCCTCGCCTGGTACCAGCAGAAACCGGGACAAGCTCCCCG CCTGCTGATCTACGGAGCCAGCAGCCGGGCCACCGGTATTCCTGACC GGTTCTCCGGTTCGGGGTCCGGGACCGACTTTACTCTGACTATCTCTC GCCTCGAGCCAGAGGACTCCGCCGTGTATTACTGCCAGCAGTACCACT CCTCCCCGTCCTGGACGTTCGGACAGGGCACAAGGCTGGAGATTAAGA |
| | | CCACTACCCCAGCACCGAGGCCACCCACCCCGGCTCCTACCATCGCCT CCCAGCCTCTGTCCCTGCGTCCGGAggcatgtagacccgcagctggtggggccgtgc atacccggggtcttgacttcgcctgcgatatctacatttgggcccctctggctggtacttgcggggtcctgctg ctttcactcgtgatcactctttactgtaagcgcggtcggaagaagctgctgtacatctttaagcaacccttcat gaggcctgtgcagactactcaagaggaggacggctgttcatgccggttcccagaggaggaggaaggc ggctgcgaactgcgcgtgaaattcagccgcagcgcagatgctccagcctacaagcaggggcagaacc agctctacaacgaactcaatcttggtcggagagaggagtacgacgtgctggacaagcggagaggacg ggacccagaaatgggcgggaagccgcgcagaaagaatccccaagagggcctgtacaacgagctcc aaaaggataagatggcagaagcctatagcgagattggtatgaaaggggaacgcagaagaggcaaa ggccacgacggactgtaccagggactcagcaccgccaccaaggacacctatgacgctcttcacatgca ggccctgccgcctcgg |

| **BCMA_EBB-C1978-C7** | | |
|---|---|---|
| **BCMA_EBB-C1978-C7 - aa Full CART** | 841 | MALPVTALLLPLALLLHAARPEVQLVETGGGLVQPGGSLRLSCAASGFTFS SYAMSWVRQAPGKGLEWVSAISGSGGSTYYADSVKGRFTISRDNSKNTLY LQMNTLKAEDTAVYYCARATYKRELRYYYGMDVWGQGTTVTVSSGGGGS GGGGSGGGGSEIVLTQSPSTLSLSPGESATLSCRASQSVSTTFLAWYQQK PGQAPRLLIYGSSNRATGIPDRFSGSGSGTDFTLTIRRLEPEDFAVYYCQQ YHSSPSWTFGQGTKVEIKTTTPAPRPPTPAPTIASQPLSLRPEACRPAAGG AVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNEL NLGRREEYDVLDKRRGRDPEMGGKPRRKNPQEGLYNELQKDKMAEAYS EIGMKGERRRGKGHDGLYQGLSTATKDTYDALHMQALPPR |
| **BCMA_EBB-C1978-C7 - nt Full CART** | 842 | ATGGCCCTCCCTGTCACCGCCCTGCTGCTTCCGCTGGCTCTTCTGCTC CACGCCGCTCGGCCCGAGGTGCAGCTTGTGGAAACCGGTGGCGGACT GGTGCAGCCCGGAGGAAGCCTCAGGCTGTCCTGCGCCGCGTCCGGCT TCACCTTCTCCTCGTACGCCATGTCCTGGGTCCGCCAGGCCCCCGGAA AGGGCCTGGAATGGGTGTCCGCCATCTCTGGAAGCGGAGGTTCCACG TACTACGCGGACAGCGTCAAGGGAAGGTTCACAATCTCCCGCGATAAT TCGAAGAACACTCTGTACCTTCAAATGAACACCCTGAAGGCCGAGGAC ACTGCTGTGTACTACTGCGCACGGGCCACCTACAAGAGAGAGCTCCGG TACTACTACGGAATGGACGTCTGGGGCCAGGGAACTACTGTGACCGTG TCCTCGGGAGGGGGTGGCTCCGGGGGGGGCGGCTCCGGCGGAGGC GGTTCCGAGATTGTGCTGACCCAGTCACCTTCAACTCTGTCGCTGTCC CCGGGAGAGAGCGCTACTCTGAGCTGCCGGGCCAGCCAGTCCGTGTC CACCACCTTCCTCGCCTGGTATCAGCAGAAGCCGGGGCAGGCACCAC GGCTCTTGATCTACGGGTCAAGCAACAGAGCGACCGGAATTCCTGACC GCTTCTCGGGGAGCGGTTCAGGCACCGACTTCACCCTGACTATCCGGC GCCTGGAACCCGAAGATTTCGCCGTGTATTACTGTCAACAGTACCACTC CTCGCCGTCCTGGACCTTTGGCCAAGGAACCAAAGTGGAAATCAAGAC CACTACCCCAGCACCGAGGCCACCCACCCCGGCTCCTACCATCGCCTC CCAGCCTCTGTCCCTGCGTCCGGAggcatgtagacccgcagctggtggggccgtgcat acccggggtcttgacttcgcctgcgatatctacatttgggcccctctggctggtacttgcggggtcctgctgctt tcactcgtgatcactctttactgtaagcgcggtcggaagaagctgctgtacatctttaagcaacccttcatga ggcctgtgcagactactcaagaggaggacggctgttcatgccggttcccagaggaggaggaaggcgg ctgcgaactgcgcgtgaaattcagccgcagcgcagatgctccagcctacaagcaggggcagaaccag ctctacaacgaactcaatcttggtcggagagaggagtacgacgtgctggacaagcggagaggacggg acccagaaatgggcgggaagccgcgcagaaagaatccccaagagggcctgtacaacgagctccaa aaggataagatggcagaagcctatagcgagattggtatgaaaggggaacgcagaagaggcaaagg ccacgacggactgtaccagggactcagcaccgccaccaaggacacctatgacgctcttcacatgcagg ccctgccgcctcgg |

| **BCMA_EBB-C1978-D10** | | |
|---|---|---|
| **BCMA_EBB-C1978-D10 - aa Full CART** | 843 | MALPVTALLLPLALLLHAARPEVQLVETGGGLVQPGRSLRLSCAASGFTFD DYAMHWVRQAPGKGLEWVSGISWNSGSIGYADSVKGRFTISRDNAKNSL YLQMNSLRDEDTAVYYCARVGKAVPDVWGQGTTVTVSSGGGGSGGGGS GGGGSDIVMTQTPSSLSASVGDRVTITCRASQSISSYLNWYQQKPGKAPK LLIYAASSLQSGVPSRFSGSGSGTDFTLTISSLQPEDFATYYCQQSYSTPYS |
| | | FGQGTRLEIKTTTPAPRPPTPAPTIASQPLSLRPEACRPAAGGAVHTRGLD FACDIYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREEY DVLDKRRGRDPEMGGKPRRKNPQEGLYNELQKDKMAEAYSEIGMKGER RRGKGHDGLYQGLSTATKDTYDALHMQALPPR |
| **BCMA_EBB-C1978-D10 - nt Full CART** | 844 | ATGGCCCTCCCTGTCACCGCCCTGCTGCTTCCGCTGGCTCTTCTGCTC CACGCCGCTCGGCCCGAAGTGCAGCTCGTGGAAACTGGAGGTGGACT CGTGCAGCCTGGACGGTCGCTGCGGCTGAGCTGCGCTGCATCCGGCT TCACCTTCGACGATTATGCCATGCACTGGGTCAGACAGGCGCCAGGGA AGGGACTTGAGTGGGTGTCCGGTATCAGCTGGAATAGCGGCTCAATCG GATACGCGGACTCCGTGAAGGGAAGGTTCACCATTTCCCGCGACAACG CCAAGAACTCCCTGTACTTGCAAATGAACAGCCTCCGGGATGAGGACA CTGCCGTGTACTACTGCGCCCGCGTCGGAAAAGCTGTGCCCGACGTCT GGGGCCAGGGAACCACTGTGACCGTGTCCAGCGGCGGGGGTGGATC GGGCGGTGGAGGGTCCGGTGGAGGGGGCTCAGATATTGTGATGACCC AGACCCCCTCGTCCCTGTCCGCCTCGGTCGGCGACCGCGTGACTATCA CATGTAGAGCCTCGCAGAGCATCTCCAGCTACCTGAACTGGTATCAGC AGAAGCCGGGGAAGGCCCCGAAGCTCCTGATCTACGCGGCATCATCA CTGCAATCGGGAGTGCCGAGCCGGTTTTCCGGGTCCGGCTCCGGCAC CGACTTCACGCTGACCATTTCTTCCCTGCAACCCGAGGACTTCGCCACT TACTACTGCCAGCAGTCCTACTCCACCCCTTACTCCTTCGGCCAAGGAA CCAGGCTGGAAATCAAGACCACTACCCCAGCACCGAGGCCACCCACC CCGGCTCCTACCATCGCCTCCCAGCCTCTGTCCCTGCGTCCGGAggcat gtagacccgcagctggtggggccgtgcatacccggggtcttgacttcgcctgcgatatctacatttgggcc cctctggctggtacttgcggggtcctgctgctttcactcgtgatcactctttactgtaagcgcggtcggaagaa gctgctgtacatctttaagcaacccttcatgaggcctgtgcagactactcaagaggaggacggctgttcat gccggttcccagaggaggaggaaggcggctgcgaactgcgcgtgaaattcagccgcagcgcagatg ctccagcctacaagcaggggcagaaccagctctacaacgaactcaatcttggtcggagagaggagtac gacgtgctggacaagcggagaggacgggacccagaaatgggcgggaagccgcgcagaaagaatc cccaagagggcctgtacaacgagctccaaaaggataagatggcagaagcctatagcgagattggtatg aaaggggaacgcagaagaggcaaaggccacgacggactgtaccagggactcagcaccgccacca aggacacctatgacgctcttcacatgcaggccctgccgcctcgg |

| **BCMA EBB-C1979-C12** | | |
|---|---|---|
| **BCMA_EBB-C1979-C12 - aa Full CART** | 845 | MALPVTALLLPLALLLHAARPEVQLVESGGGLVQPGRSLRLSCTASGFTFD DYAMHWVRQRPGKGLEWVASINWKGNSLAYGDSVKGRFAISRDNAKNTV FLQMNSLRTEDTAVYYCASHQGVAYYNYAMDVWGRGTLVTVSSGGGGS GGGGSGGGGSEIVLTQSPGTLSLSPGERATLSCRATQSIGSSFLAWYQQR PGQAPRLLIYGASQRATGIPDRFSGRGSGTDFTLTISRVEPEDSAVYYCQH YESSPSWTFGQGTKVEIKTTTPAPRPPTPAPTIASQPLSLRPEACRPAAGG AVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNEL NLGRREEYDVLDKRRGRDPEMGGKPRRKNPQEGLYNELQKDKMAEAYS EIGMKGERRRGKGHDGLYQGLSTATKDTYDALHMQALPPR |
| **BCMA_EBB-C1979-C12 - nt Full CART** | 846 | ATGGCCCTCCCTGTCACCGCCCTGCTGCTTCCGCTGGCTCTTCTGCTC CACGCCGCTCGGCCCGAAGTGCAGCTCGTGGAGAGCGGGGGAGGATT GGTGCAGCCCGGAAGGTCCCTGCGGCTCTCCTGCACTGCGTCTGGCT TCACCTTCGACGACTACGCGATGCACTGGGTCAGACAGCGCCCGGGA AAGGGCCTGGAATGGGTCGCCTCAATCAACTGGAAGGGAAACTCCCTG GCCTATGGCGACAGCGTGAAGGGCCGCTTCGCCATTTCGCGCGACAA CGCCAAGAACACCGTGTTTCTGCAAATGAATTCCCTGCGGACCGAGGA TACCGCTGTGTACTACTGCGCCAGCCACCAGGGCGTGGCATACTATAA CTACGCCATGGACGTGTGGGGAAGAGGGACGCTCGTCACCGTGTCCT CCGGGGGCGGTGGATCGGGTGGAGGAGGAAGCGGTGGCGGGGGCA GCGAAATCGTGCTGACTCAGAGCCCGGGAACTCTTTCACTGTCCCCGG GAGAACGGGCCACTCTCTCGTGCCGGGCCACCCAGTCCATCGGCTCC TCCTTCCTTGCCTGGTACCAGCAGAGGCCAGGACAGGCGCCCCGCCT |
| | | GCTGATCTACGGTGCTTCCCAACGCGCCACTGGCATTCCTGACCGGTT CAGCGGCAGAGGGTCGGGAACCGATTTCACACTGACCATTTCCCGGGT GGAGCCCGAAGATTCGGCAGTCTACTACTGTCAGCATTACGAGTCCTC CCCTTCATGGACCTTCGGTCAAGGGACCAAAGTGGAGATCAAGACCAC TACCCCAGCACCGAGGCCACCCACCCCGGCTCCTACCATCGCCTCCCA GCCTCTGTCCCTGCGTCCGGAggcatgtagacccgcagctggtggggccgtgcataccc ggggtcttgacttcgcctgcgatatctacatttgggcccctctggctggtacttgcggggtcctgctgctttcac tcgtgatcactctttactgtaagcgcggtcggaagaagctgctgtacatctttaagcaacccttcatgaggcc tgtgcagactactcaagaggaggacggctgttcatgccggttcccagaggaggaggaaggcggctgcg aactgcgcgtgaaattcagccgcagcgcagatgctccagcctacaagcaggggcagaaccagctcta caacgaactcaatcttggtcggagagaggagtacgacgtgctggacaagcggagaggacgggaccc agaaatgggcgggaagccgcgcagaaagaatccccaagagggcctgtacaacgagctccaaaagg ataagatggcagaagcctatagcgagattggtatgaaaggggaacgcagaagaggcaaaggccacg acggactgtaccagggactcagcaccgccaccaaggacacctatgacgctcttcacatgcaggccctg ccgcctcgg |

| **BCMA_EBB-C1980-G4** | | |
|---|---|---|
| **BCMA_EBB-C1980-G4- aa Full CART** | 847 | MALPVTALLLPLALLLHAARPEVQLVESGGGLVQPGGSLRLSCAASGFTFS SYAMSWVRQAPGKGLEWVSAISGSGGSTYYADSVKGRFTISRDNSKNTLY LQMNSLRAEDTAVYYCAKVVRDGMDVWGQGTTVTVSSGGGGSGGGGS GGGGSEIVLTQSPATLSLSPGERATLSCRASQSVSSSYLAWYQQKPGQAP RLLIYGASSRATGIPDRFSGNGSGTDFTLTISRLEPEDFAVYYCQQYGSPP RFTFGPGTKVDIKTTTPAPRPPTPAPTIASQPLSLRPEACRPAAGGAVHTR GLDFACDIYIWAPLAGTCGVLLLSLVITLYCKRGRKKLLYIFKQPFMRPVQTT QEEDGCSCRFPEEEEGGCELRVKFSRSADAPAYKQGQNQLYNELNLGRR EEYDVLDKRRGRDPEMGGKPRRKNPQEGLYNELQKDKMAEAYSEIGMKG ERRRGKGHDGLYQGLSTATKDTYDALHMQALPPR |
| **BCMA_EBB-C1980-G4- nt Full CART** | 848 | ATGGCCCTCCCTGTCACCGCCCTGCTGCTTCCGCTGGCTCTTCTGCTC CACGCCGCTCGGCCCGAGGTGCAGTTGGTCGAAAGCGGGGGCGGGC TTGTGCAGCCTGGCGGATCACTGCGGCTGTCCTGCGCGGCATCAGGC TTCACGTTTTCTTCCTACGCCATGTCCTGGGTGCGCCAGGCCCCTGGA AAGGGACTGGAATGGGTGTCCGCGATTTCGGGGTCCGGCGGGAGCAC CTACTACGCCGATTCCGTGAAGGGCCGCTTCACTATCTCGCGGGACAA CTCCAAGAACACCCTCTACCTCCAAATGAATAGCCTGCGGGCCGAGGA TACCGCCGTCTACTATTGCGCTAAGGTCGTGCGCGACGGAATGGACGT GTGGGGACAGGGTACCACCGTGACAGTGTCCTCGGGGGGAGGCGGTA GCGGCGGAGGAGGAAGCGGTGGTGGAGGTTCCGAGATTGTGCTGACT CAATCACCCGCGACCCTGAGCCTGTCCCCCGGCGAAAGGGCCACTCT GTCCTGTCGGGCCAGCCAATCAGTCTCCTCCTCGTACCTGGCCTGGTA CCAGCAGAAGCCAGGACAGGCTCCGAGACTCCTTATCTATGGCGCATC CTCCCGCGCCACCGGAATCCCGGATAGGTTCTCGGGAAACGGATCGG GGACCGACTTCACTCTCACCATCTCCCGGCTGGAACCGGAGGACTTCG CCGTGTACTACTGCCAGCAGTACGGCAGCCCGCCTAGATTCACTTTCG GCCCCGGCACCAAAGTGGACATCAAGACCACTACCCCAGCACCGAGG CCACCCACCCCGGCTCCTACCATCGCCTCCCAGCCTCTGTCCCTGCGT CCGGAggcatgtagacccgcagctggtggggccgtgcatacccggggtcttgacttcgcctgcgatat ctacatttgggcccctctggctggtacttgcggggtcctgctgctttcactcgtgatcactctttactgtaagcgc ggtcggaagaagctgctgtacatctttaagcaacccttcatgaggcctgtgcagactactcaagaggagg acggctgttcatgccggttcccagaggaggaggaaggcggctgcgaactgcgcgtgaaattcagccgc agcgcagatgctccagcctacaagcaggggcagaaccagctctacaacgaactcaatcttggtcggag agaggagtacgacgtgctggacaagcggagaggacgggacccagaaatgggcgggaagccgcgc agaaagaatccccaagagggcctgtacaacgagctccaaaaggataagatggcagaagcctatagc gagattggtatgaaaggggaacgcagaagaggcaaaggccacgacggactgtaccagggactcag caccgccaccaaggacacctatgacgctcttcacatgcaggccctgccgcctcgg |

| **BCMA EBB-C1980-D2** | | |
|---|---|---|
| **BCMA_EBB-C1980-D2- aa** | 849 | MALPVTALLLPLALLLHAARPEVQLLESGGGLVQPGGSLRLSCAASGFTFS SYAMSWVRQAPGKGLEWVSAISGSGGSTYYADSVKGRFTISRDNSKNTLY |
| **Full CART** | | LQMNSLRAEDTAVYYCAKIPQTGTFDYWGQGTLVTVSSGGGGSGGGGSG GGGSEIVLTQSPGTLSLSPGERATLSCRASQSVSSSYLAWYQQRPGQAPR LLIYGASSRATGIPDRFSGSGSGTDFTLTISRLEPEDFAVYYCQHYGSSPS WTFGQGTRLEIKTTTPAPRPPTPAPTIASQPLSLRPEACRPAAGGAVHTRG LDFACDIYIWAPLAGTCGVLLLSLVITLYCKRGRKKLLYIFKQPFMRPVQTTQ EEDGCSCRFPEEEEGGCELRVKFSRSADAPAYKQGQNQLYNELNLGRRE EYDVLDKRRGRDPEMGGKPRRKNPQEGLYNELQKDKMAEAYSEIGMKGE RRRGKGHDGLYQGLSTATKDTYDALHMQALPPR |
| **BCMA_EBB-C1980-D2- nt Full CART** | 850 | ATGGCCCTCCCTGTCACCGCCCTGCTGCTTCCGCTGGCTCTTCTGCTC CACGCCGCTCGGCCCGAAGTGCAGCTGCTGGAGTCCGGCGGTGGATT GGTGCAACCGGGGGGATCGCTCAGACTGTCCTGTGCGGCGTCAGGCT TCACCTTCTCGAGCTACGCCATGTCATGGGTCAGACAGGCCCCTGGAA AGGGTCTGGAATGGGTGTCCGCCATTTCCGGGAGCGGGGGATCTACA TACTACGCCGATAGCGTGAAGGGCCGCTTCACCATTTCCCGGGACAAC TCCAAGAACACTCTCTATCTGCAAATGAACTCCCTCCGCGCTGAGGACA CTGCCGTGTACTACTGCGCCAAAATCCCTCAGACCGGCACCTTCGACT ACTGGGGACAGGGGACTCTGGTCACCGTCAGCAGCGGTGGCGGAGGT TCGGGGGGAGGAGGAAGCGGCGGCGGAGGGTCCGAGATTGTGCTGA CCCAGTCACCCGGCACTTTGTCCCTGTCGCCTGGAGAAAGGGCCACCC TTTCCTGCCGGGCATCCCAATCCGTGTCCTCCTCGTACCTGGCCTGGT ACCAGCAGAGGCCCGGACAGGCCCCACGGCTTCTGATCTACGGAGCA AGCAGCCGCGCGACCGGTATCCCGGACCGGTTTTCGGGCTCGGGCTC AGGAACTGACTTCACCCTCACCATCTCCCGCCTGGAACCCGAAGATTT CGCTGTGTATTACTGCCAGCACTACGGCAGCTCCCCGTCCTGGACGTT CGGCCAGGGAACTCGGCTGGAGATCAAGACCACTACCCCAGCACCGA GGCCACCCACCCCGGCTCCTACCATCGCCTCCCAGCCTCTGTCCCTGC GTCCGGAggcatgtagacccgcagctggtggggccgtgcatacccggggtcttgacttcgcctgcg atatctacatttgggcccctctggctggtacttgcggggtcctgctgctttcactcgtgatcactctttactgtaa gcgcggtcggaagaagctgctgtacatctttaagcaacccttcatgaggcctgtgcagactactcaagag gaggacggctgttcatgccggttcccagaggaggaggaaggcggctgcgaactgcgcgtgaaattcag ccgcagcgcagatgctccagcctacaagcaggggcagaaccagctctacaacgaactcaatcttggtc ggagagaggagtacgacgtgctggacaagcggagaggacgggacccagaaatgggcgggaagcc gcgcagaaagaatccccaagagggcctgtacaacgagctccaaaaggataagatggcagaagccta tagcgagattggtatgaaaggggaacgcagaagaggcaaaggccacgacggactgtaccagggact cagcaccgccaccaaggacacctatgacgctcttcacatgcaggccctgccgcctcgg |

| **BCMA_EBB-C1978-A10** | | |
|---|---|---|
| **BCMA_EBB-C1978-A10-aa Full CART** | 851 | MALPVTALLLPLALLLHAARPEVQLVETGGGLVQPGGSLRLSCAASGFTFS SYAMSWVRQAPGKGLEWVSAISGSGGSTYYADSVKGRFTMSRENDKNS VFLQMNSLRVEDTGVYYCARANYKRELRYYYGMDVWGQGTMVTVSSGG GGSGGGGSGGGGSEIVMTQSPGTLSLSPGESATLSCRASQRVASNYLAW YQHKPGQAPSLLISGASSRATGVPDRFSGSGSGTDFTLAISRLEPEDSAVY YCQHYDSSPSWTFGQGTKVEIKTTTPAPRPPTPAPTIASQPLSLRPEACRP AAGGAVHTRGLDFACDIYIWAPLAGTCGVLLLSLVITLYCKRGRKKLLYIFK QPFMRPVQTTQEEDGCSCRFPEEEEGGCELRVKFSRSADAPAYKQGQN QLYNELNLGRREEYDVLDKRRGRDPEMGGKPRRKNPQEGLYNELQKDK MAEAYSEIGMKGERRRGKGHDGLYQGLSTATKDTYDALHMQALPPR |
| **BCMA_EBB-C1978-A10-nt Full CART** | 852 | ATGGCCCTCCCTGTCACCGCCCTGCTGCTTCCGCTGGCTCTTCTGCTC CACGCCGCTCGGCCCGAAGTGCAACTGGTGGAAACCGGTGGAGGACT CGTGCAGCCTGGCGGCAGCCTCCGGCTGAGCTGCGCCGCTTCGGGAT TCACCTTTTCCTCCTACGCGATGTCTTGGGTCAGACAGGCCCCCGGAA AGGGGCTGGAATGGGTGTCAGCCATCTCCGGCTCCGGCGGATCAACG TACTACGCCGACTCCGTGAAAGGCCGGTTCACCATGTCGCGCGAGAAT GACAAGAACTCCGTGTTCCTGCAAATGAACTCCCTGAGGGTGGAGGAC ACCGGAGTGTACTATTGTGCGCGCGCCAACTACAAGAGAGAGCTGCGG TACTACTACGGAATGGACGTCTGGGGACAGGGAACTATGGTGACCGTG TCATCCGGTGGAGGGGGAAGCGGCGGTGGAGGCAGCGGGGGCGGG |
| | | GTTCAGAAATTGTCATGACCCAGTCCCCGGGAACTCTTTCCCTCTCCCC CGGGGAATCCGCGACTTTGTCCTGCCGGGCCAGCCAGCGCGTGGCCT CGAACTACCTCGCATGGTACCAGCATAAGCCAGGCCAAGCCCCTTCCC TGCTGATTTCCGGGGCTAGCAGCCGCGCCACTGGCGTGCCGGATAGG TTCTCGGGAAGCGGCTCGGGTACCGATTTCACCCTGGCAATCTCGCGG CTGGAACCGGAGGATTCGGCCGTGTACTACTGCCAGCACTATGACTCA TCCCCCTCCTGGACATTCGGACAGGGCACCAAGGTCGAGATCAAGACC ACTACCCCAGCACCGAGGCCACCCACCCCGGCTCCTACCATCGCCTCC CAGCCTCTGTCCCTGCGTCCGGAggcatgtagacccgcagctggtggggccgtgcata cccggggtcttgacttcgcctgcgatatctacatttgggcccctctggctggtacttgcggggtcctgctgcttt cactcgtgatcactctttactgtaagcgcggtcggaagaagctgctgtacatctttaagcaacccttcatgag gcctgtgcagactactcaagaggaggacggctgttcatgccggttcccagaggaggaggaaggcggct gcgaactgcgcgtgaaattcagccgcagcgcagatgctccagcctacaagcaggggcagaaccagct ctacaacgaactcaatcttggtcggagagaggagtacgacgtgctggacaagcggagaggacgggac ccagaaatgggcgggaagccgcgcagaaagaatccccaagagggcctgtacaacgagctccaaaa ggataagatggcagaagcctatagcgagattggtatgaaaggggaacgcagaagaggcaaaggcca cgacggactgtaccagggactcagcaccgccaccaaggacacctatgacgctcttcacatgcaggccc tgccgcctcgg |

| **BCMA EBB-C1978-D4** | | |
|---|---|---|
| **BCMA_EBB-C1978-D4- aa Full CART** | 853 | MALPVTALLLPLALLLHAARPEVQLLETGGGLVQPGGSLRLSCAASGFSFS SYAMSWVRQAPGKGLEWVSAISGSGGSTYYADSVKGRFTISRDNSKNTLY LQMNSLRAEDTAVYYCAKALVGATGAFDIWGQGTLVTVSSGGGGSGGGG SGGGGSEIVLTQSPGTLSLSPGERATLSCRASQSLSSNFLAWYQQKPGQA PGLLIYGASNWATGTPDRFSGSGSGTDFTLTITRLEPEDFAVYYCQYYGTS PMYTFGQGTKVEIKTTTPAPRPPTPAPTIASQPLSLRPEACRPAAGGAVHT RGLDFACDIYIWAPLAGTCGVLLLSLVITLYCKRGRKKLLYIFKQPFMRPVQ TTQEEDGCSCRFPEEEEGGCELRVKFSRSADAPAYKQGQNQLYNELNLG RREEYDVLDKRRGRDPEMGGKPRRKNPQEGLYNELQKDKMAEAYSEIGM KGERRRGKGHDGLYQGLSTATKDTYDALHMQALPPR |
| **BCMA_EBB-C1978-D4- nt Full CART** | 854 | ATGGCCCTCCCTGTCACCGCCCTGCTGCTTCCGCTGGCTCTTCTGCTC CACGCCGCTCGGCCCGAAGTGCAGCTGCTCGAAACCGGTGGAGGGCT GGTGCAGCCAGGGGGCTCCCTGAGGCTTTCATGCGCCGCTAGCGGAT TCTCCTTCTCCTCTTACGCCATGTCGTGGGTCCGCCAAGCCCCTGGAA AAGGCCTGGAATGGGTGTCCGCGATTTCCGGGAGCGGAGGTTCGACC TATTACGCCGACTCCGTGAAGGGCCGCTTTACCATCTCCCGGGATAAC TCCAAGAACACTCTGTACCTCCAAATGAACTCGCTGAGAGCCGAGGAC ACCGCCGTGTATTACTGCGCGAAGGCGCTGGTCGGCGCGACTGGGGC ATTCGACATCTGGGGACAGGGAACTCTTGTGACCGTGTCGAGCGGAGG CGGCGGCTCCGGCGGAGGAGGGAGCGGGGGCGGTGGTTCCGAAATC GTGTTGACTCAGTCCCCGGGAACCCTGAGCTTGTCACCCGGGGAGCG GGCCACTCTCTCCTGTCGCGCCTCCCAATCGCTCTCATCCAATTTCCTG GCCTGGTACCAGCAGAAGCCCGGACAGGCCCCGGGCCTGCTCATCTA CGGCGCTTCAAACTGGGCAACGGGAACCCCTGATCGGTTCAGCGGAA GCGGATCGGGTACTGACTTTACCCTGACCATCACCAGACTGGAACCGG AGGACTTCGCCGTGTACTACTGCCAGTACTACGGCACCTCCCCCATGT ACACATTCGGACAGGGTACCAAGGTCGAGATTAAGACCACTACCCCAG CACCGAGGCCACCCACCCCGGCTCCTACCATCGCCTCCCAGCCTCTGT CCCTGCGTCCGGAggcatgtagacccgcagctggtggggccgtgcatacccggggtcttgactt cgcctgcgatatctacatttgggcccctctggctggtacttgcggggtcctgctgctttcactcgtgatcactctt tactgtaagcgcggtcggaagaagctgctgtacatctttaagcaacccttcatgaggcctgtgcagactac tcaagaggaggacggctgttcatgccggttcccagaggaggaggaaggcggctgcgaactgcgcgtg aaattcagccgcagcgcagatgctccagcctacaagcaggggcagaaccagctctacaacgaactca atcttggtcggagagaggagtacgacgtgctggacaagcggagaggacgggacccagaaatgggcg ggaagccgcgcagaaagaatccccaagagggcctgtacaacgagctccaaaaggataagatggca gaagcctatagcgagattggtatgaaaggggaacgcagaagaggcaaaggccacgacggactgtac cagggactcagcaccgccaccaaggacacctatgacgctcttcacatgcaggccctgccgcctcgg |

| **BCMA_EBB-C1980-A2** | | |
|---|---|---|
| **BCMA_EBB-C1980-A2- aa Full CART** | 855 | MALPVTALLLPLALLLHAARPEVQLLESGGGLVQPGGSLRLSCAASGFTFS SYAMSWVRQAPGKGLEWVSAISGSGGSTYYADSVKGRFTISRDNSKNTLY LQMNSLRAEDTAVYYCVLWFGEGFDPWGQGTLVTVSSGGGGSGGGGSG GGGSDIVLTQSPLSLPVTPGEPASISCRSSQSLLHSNGYNYLDWYLQKPG QSPQLLIYLGSNRASGVPDRFSGSGSGTDFTLKISRVEAEDVGVYYCMQA LQTPLTFGGGTKVDIKTTTPAPRPPTPAPTIASQPLSLRPEACRPAAGGAV HTRGLDFACDIYIWAPLAGTCGVLLLSLVITLYCKRGRKKLLYIFKQPFMRPV QTTQEEDGCSCRFPEEEEGGCELRVKFSRSADAPAYKQGQNQLYNELNL GRREEYDVLDKRRGRDPEMGGKPRRKNPQEGLYNELQKDKMAEAYSEIG MKGERRRGKGHDGLYQGLSTATKDTYDALHMQALPPR |
| **BCMA_EBB-C1980-A2- nt Full CART** | 856 | ATGGCCCTCCCTGTCACCGCCCTGCTGCTTCCGCTGGCTCTTCTGCTC CACGCCGCTCGGCCCGAAGTGCAGCTGCTTGAGAGCGGTGGAGGTCT GGTGCAGCCCGGGGGATCACTGCGCCTGTCCTGTGCCGCGTCCGGTT TCACTTTCTCCTCGTACGCCATGTCGTGGGTCAGACAGGCACCGGGAA AGGGACTGGAATGGGTGTCAGCCATTTCGGGTTCGGGGGGCAGCACC TACTACGCTGACTCCGTGAAGGGCCGGTTCACCATTTCCCGCGACAAC TCCAAGAACACCTTGTACCTCCAAATGAACTCCCTGCGGGCCGAAGAT ACCGCCGTGTATTACTGCGTGCTGTGGTTCGGAGAGGGATTCGACCCG TGGGGACAAGGAACACTCGTGACTGTGTCATCCGGCGGAGGCGGCAG CGGTGGCGGCGGTTCCGGCGGCGGCGGATCTGACATCGTGTTGACCC AGTCCCCTCTGAGCCTGCCGGTCACTCCTGGCGAACCAGCCAGCATCT CCTGCCGGTCGAGCCAGTCCCTCCTGCACTCCAATGGGTACAACTACC TCGATTGGTATCTGCAAAAGCCGGGCCAGAGCCCCCAGCTGCTGATCT ACCTTGGGTCAAACCGCGCTTCCGGGGTGCCTGATAGATTCTCCGGGT CCGGGAGCGGAACCGACTTTACCCTGAAAATCTCGAGGGTGGAGGCC GAGGACGTCGGAGTGTACTACTGCATGCAGGCGCTCCAGACTCCCCTG ACCTTCGGAGGAGGAACGAAGGTCGACATCAAGACCACTACCCCAGCA CCGAGGCCACCCACCCCGGCTCCTACCATCGCCTCCCAGCCTCTGTCC CTGCGTCCGGAggcatgtagacccgcagctggtggggccgtgcatacccggggtcttgacttcgc ctgcgatatctacatttgggcccctctggctggtacttgcggggtcctgctgctttcactcgtgatcactctttact gtaagcgcggtcggaagaagctgctgtacatctttaagcaacccttcatgaggcctgtgcagactactca agaggaggacggctgttcatgccggttcccagaggaggaggaaggcggctgcgaactgcgcgtgaaa ttcagccgcagcgcagatgctccagcctacaagcaggggcagaaccagctctacaacgaactcaatct tggtcggagagaggagtacgacgtgctggacaagcggagaggacgggacccagaaatgggcggga agccgcgcagaaagaatccccaagagggcctgtacaacgagctccaaaaggataagatggcagaa gcctatagcgagattggtatgaaaggggaacgcagaagaggcaaaggccacgacggactgtaccag ggactcagcaccgccaccaaggacacctatgacgctcttcacatgcaggccctgccgcctcgg |

| **BCMA_EBB-C1981-C3** | | |
|---|---|---|
| **BCMA_EBB-C1981-C3- aa Full CART** | 857 | MALPVTALLLPLALLLHAARPQVQLVESGGGLVQPGGSLRLSCAASGFTFS SYAMSWVRQAPGKGLEWVSAISGSGGSTYYADSVKGRFTISRDNSKNTLY LQMNSLRAEDTAVYYCAKVGYDSSGYYRDYYGMDVWGQGTTVTVSSGG GGSGGGGSGGGGSEIVLTQSPGTLSLSPGERATLSCRASQSVSSSYLAW YQQKPGQAPRLLIYGTSSRATGISDRFSGSGSGTDFTLTISRLEPEDFAVYY CQHYGNSPPKFTFGPGTKLEIKTTTPAPRPPTPAPTIASQPLSLRPEACRPA AGGAVHTRGLDFACDIYIWAPLAGTCGVLLLSLVITLYCKRGRKKLLYIFKQ PFMRPVQTTQEEDGCSCRFPEEEEGGCELRVKFSRSADAPAYKQGQNQL YNELNLGRREEYDVLDKRRGRDPEMGGKPRRKNPQEGLYNELQKDKMA EAYSEIGMKGERRRGKGHDGLYQGLSTATKDTYDALHMQALPPR |
| **BCMA_EBB-C1981-C3- nt Full CART** | 858 | ATGGCCCTCCCTGTCACCGCCCTGCTGCTTCCGCTGGCTCTTCTGCTC CACGCCGCTCGGCCCCAAGTGCAGCTCGTGGAGTCAGGCGGAGGACT GGTGCAGCCCGGGGGCTCCCTGAGACTTTCCTGCGCGGCATCGGGTT TTACCTTCTCCTCCTATGCTATGTCCTGGGTGCGCCAGGCCCCGGGAA AGGGACTGGAATGGGTGTCCGCAATCAGCGGTAGCGGGGGCTCAACA TACTACGCCGACTCCGTCAAGGGTCGCTTCACTATTTCCCGGGACAAC TCCAAGAATACCCTGTACCTCCAAATGAACAGCCTCAGGGCCGAGGAT |
| | | ACTGCCGTGTACTACTGCGCCAAAGTCGGATACGATAGCTCCGGTTAC TACCGGGACTACTACGGAATGGACGTGTGGGGACAGGGCACCACCGT GACCGTGTCAAGCGGCGGAGGCGGTTCAGGAGGGGGAGGCTCCGGC GGTGGAGGGTCCGAAATCGTCCTGACTCAGTCGCCTGGCACTCTGTCG TTGTCCCCGGGGGAGCGCGCTACCCTGTCGTGTCGGGCGTCGCAGTC CGTGTCGAGCTCCTACCTCGCGTGGTACCAGCAGAAGCCCGGACAGG CCCCTAGACTTCTGATCTACGGCACTTCTTCACGCGCCACCGGGATCA GCGACAGGTTCAGCGGCTCCGGCTCCGGGACCGACTTCACCCTGACC ATTAGCCGGCTGGAGCCTGAAGATTTCGCCGTGTATTACTGCCAACACT ACGGAAACTCGCCGCCAAAGTTCACGTTCGGACCCGGAACCAAGCTGG AAATCAAGACCACTACCCCAGCACCGAGGCCACCCACCCCGGCTCCTA CCATCGCCTCCCAGCCTCTGTCCCTGCGTCCGGAggcatgtagacccgcagctg gtggggccgtgcatacccggggtcttgacttcgcctgcgatatctacatttgggcccctctggctggtacttg cggggtcctgctgctttcactcgtgatcactctttactgtaagcgcggtcggaagaagctgctgtacatcttta agcaacccttcatgaggcctgtgcagactactcaagaggaggacggctgttcatgccggttcccagagg aggaggaaggcggctgcgaactgcgcgtgaaattcagccgcagcgcagatgctccagcctacaagca ggggcagaaccagctctacaacgaactcaatcttggtcggagagaggagtacgacgtgctggacaag cggagaggacgggacccagaaatgggcgggaagccgcgcagaaagaatccccaagagggcctgt acaacgagctccaaaaggataagatggcagaagcctatagcgagattggtatgaaaggggaacgca gaagaggcaaaggccacgacggactgtaccagggactcagcaccgccaccaaggacacctatgac gctcttcacatgcaggccctgccgcctcgg |

| **BCMA_EBB-C1978-G4** | | |
|---|---|---|
| **BCMA_EBB-C1978-G4- aa Full CART** | 859 | MALPVTALLLPLALLLHAARPEVQLVESGGGLVQPGGSLRLSCAASGFTFS SYAMSWVRQAPGKGLEWVSAISGSGGSTYYADSVKGRFTISRDNSKNTLY LQMNSLRAEDTAVYYCAKMGWSSGYLGAFDIWGQGTTVTVSSGGGGSG GGGSGGGGSEIVLTQSPGTLSLSPGERATLSCRASQSVASSFLAWYQQK PGQAPRLLIYGASGRATGIPDRFSGSGSGTDFTLTISRLEPEDFAVYYCQH YGGSPRLTFGGGTKVDIKTTTPAPRPPTPAPTIASQPLSLRPEACRPAAGG AVHTRGLDFACDIYIWAPLAGTCGVLLLSLVITLYCKRGRKKLLYIFKQPFMR PVQTTQEEDGCSCRFPEEEEGGCELRVKFSRSADAPAYKQGQNQLYNEL NLGRREEYDVLDKRRGRDPEMGGKPRRKNPQEGLYNELQKDKMAEAYS EIGMKGERRRGKGHDGLYQGLSTATKDTYDALHMQALPPR |
| **BCMA_EBB-C1978-G4- nt Full CART** | 860 | ATGGCCCTCCCTGTCACCGCCCTGCTGCTTCCGCTGGCTCTTCTGCTC CACGCCGCTCGGCCCGAAGTCCAACTGGTGGAGTCCGGGGGAGGGCT CGTGCAGCCCGGAGGCAGCCTTCGGCTGTCGTGCGCCGCCTCCGGGT TCACGTTCTCATCCTACGCGATGTCGTGGGTCAGACAGGCACCAGGAA AGGGACTGGAATGGGTGTCCGCCATTAGCGGCTCCGGCGGTAGCACC TACTATGCCGACTCAGTGAAGGGAAGGTTCACTATCTCCCGCGACAAC AGCAAGAACACCCTGTACCTCCAAATGAACTCTCTGCGGGCCGAGGAT ACCGCGGTGTACTATTGCGCCAAGATGGGTTGGTCCAGCGGATACTTG GGAGCCTTCGACATTTGGGGACAGGGCACTACTGTGACCGTGTCCTCC GGGGGTGGCGGATCGGGAGGCGGCGGCTCGGGTGGAGGGGGTTCCG AAATCGTGTTGACCCAGTCACCGGGAACCCTCTCGCTGTCCCCGGGAG AACGGGCTACACTGTCATGTAGAGCGTCCCAGTCCGTGGCTTCCTCGT TCCTGGCCTGGTACCAGCAGAAGCCGGGACAGGCACCCCGCCTGCTC ATCTACGGAGCCAGCGGCCGGGCGACCGGCATCCCTGACCGCTTCTC CGGTTCCGGCTCGGGCACCGACTTTACTCTGACCATTAGCAGGCTTGA GCCCGAGGATTTTGCCGTGTACTACTGCCAACACTACGGGGGGAGCCC TCGCCTGACCTTCGGAGGCGGAACTAAGGTCGATATCAAAACCACTAC CCCAGCACCGAGGCCACCCACCCCGGCTCCTACCATCGCCTCCCAGC CTCTGTCCCTGCGTCCGGAggcatgtagacccgcagctggtggggccgtgcatacccggg gtcttgacttcgcctgcgatatctacatttgggcccctctggctggtacttgcggggtcctgctgctttcactcgt gatcactctttactgtaagcgcggtcggaagaagctgctgtacatctttaagcaacccttcatgaggcctgt gcagactactcaagaggaggacggctgttcatgccggttcccagaggaggaggaaggcggctgcgaa ctgcgcgtgaaattcagccgcagcgcagatgctccagcctacaagcaggggcagaaccagctctaca acgaactcaatcttggtcggagagaggagtacgacgtgctggacaagcggagaggacgggacccag |
| | | aaatgggcgggaagccgcgcagaaagaatccccaagagggcctgtacaacgagctccaaaaggat aagatggcagaagcctatagcgagattggtatgaaaggggaacgcagaagaggcaaaggccacga cggactgtaccagggactcagcaccgccaccaaggacacctatgacgctcttcacatgcaggccctgc cgcctcgg |

In one embodiment, the CAR comprises (e.g., consists of) an amino acid sequence provided in Table 16, or Table 1 of WO2016/014565, or as otherwise described herein. In one embodiment, the CAR comprises (e.g., consists of) an amino acid sequence selected from any one of SEQ ID NOs: 789, 791, 793, 795, 797, 799, 801 , 803, 805, 807, 809, 81 1, 813, 815, 817, 819, 821, 823, 825, 827, 829, 831, 833, 835, 837, 839, 841, 843, 845, 847, 849, 851, 853, 855, 857, 859; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence selected from any one of SEQ ID NOs: 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, 859; or an amino acid sequence having 85%, 90%, 95%, 96%, 97%, 98%, 99% identity to an amino acid sequence selected from any one of SEQ ID NOs: 789, 791, 793, 795, 797, 799, 801, 803, 805, 807, 809, 811, 813, 815, 817, 819, 821 ,823, 825, 827, 829, 831, 833, 835, 837, 839, 841, 843, 845, 847, 849, 851, 853, 855, 857, 859.

In one embodiment, the CAR molecule comprises a mesothelin antigen binding domain (e.g., a murine, human or humanized antibody or antibody fragment that specifically binds to mesothelin), a transmembrane domain, and an intracellular signaling domain (e.g., an intracellular signaling domain comprising a costimulatory domain and/or a primary signaling domain).

Exemplary CAR molecules that target mesothelin are described herein, and are provided in Table 17. The CAR molecules in Table 17 comprise a mesothelin antigen binding domain, e.g., an amino acid sequence of any mesothelin antigen binding domain provided in Table 3. The leader sequence is in bold and underlined, CDRs are underlined, and the linker sequence between the heavy and light chain of the antigen binding region is shaded in grey.

**Table 17. Exemplary CAR molecules**

| **Name** | **Amino Acid Sequence** | **SEQ ID NO:** |
|---|---|---|
| M5 CAR | MALPVTALLLPLALLLHAARPQVQLVQSGAEVEKPGASVKVSCKASGYTFTDYY MHWVRQAPGQGLEWMGWINPNSGGTNYAQKFQGRVTMTRDTSISTAYMELSRL RSDDTAVYYCASGWDFDYWGQGTLVTVSSGGGGSGGGGSGGGGSGGGGSDIV MTQSPSSLSASVGDRVTITCRASQSIRYYLSWYQQKPGKAPKLLIYTASILQNGVP SRFSGSGSGTDFTLTISSLQPEDFATYYCLQTYTTPDFGPGTKVEIKTTTPAPRPPT PAPTIASQPLSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLYNELQK DKMAEAYSEIGMKGERRRGKGHDGLYQGLSTATKDTYDALHMQALPPR | **724** |
| M11 CAR | MALPVTALLLPLALLLHAARPQVQLQQSGAEVKKPGASVKVSCKASGYTFTGYY MHWVRQAPGQGLEWMGWINPNSGGTNYAQNFQGRVTMTRDTSISTAYMELRRL RSDDTAVYYCASGWDFDYWGQGTLVTVSSGGGGSGGGGSGGGGSGGGGSDIR MTQSPSSLSASVGDRVTITCRASQSIRYYLSWYQQKPGKAPKLLIYTASILQNGVP SRFSGSGSGTDFTLTISSLQPEDFATYYCLQTYTTPDFGPGTKVEIKTTTPAPRPPT PAPTIASQPLSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKFSRSADAP AYKQGQNQLYNELNLGRREEYDVLDKRRGRDPEMGGKPRRKNPQEGLYNELQK DKMAEAYSEIGMKGERRRGKGHDGLYQGLSTATKDTYDALHMQALPPR | **725** |
| SS1 CAR | MALPVTALLLPLALLLHAARPQVQLQQSGPELEKPGASVKISCKASGYSFTGYTM NWVKQSHGKSLEWIGLITPYNGASSYNQKFRGKATLTVDKSSSTAYMDLLSLTSE DSAVYFCARGGYDGRGFDYWGQGTTVTVSSGGGGSGGGGSGGGGSDIELTQS PAIMSASPGEKVTMTCSASSSVSYMHWYQQKSGTSPKRWIYDTSKLASGVPGRF SGSGSGNSYSLTISSVEAEDDATYYCQQWSGYPLTFGAGTKLEITTTPAPRPPTPA PTIASQPLSLRPEACRPAAGGAVHTRGLDFACDIYIWAPLAGTCGVLLLSLVITLYC KRGRKKLLYIFKQPFMRPVQTTQEEDGCSCRFPEEEEGGCELRVKFSRSADAPA | **726** |
| M1 CAR | MALPVTALLLPLALLLHAARPQVQLQQSGAEVKKPGASVKVSCKASGYTFTGYY MHWVRQAPGQGLEWMGRINPNSGGTNYAQKFQGRVTMTRDTSISTAYMELSRL RSEDTAVYYCARGRYYGMDVWGQGTMVTVSSGGGGSGGGGSGGGGSGGGGS EIVLTQSPATLSLSPGERATISCRASQSVSSNFAWYQQRPGQAPRLLIYDASNRAT GIPPRFSGSGSGTDFTLTISSLEPEDFAAYYCHQRSNWLYTFGQGTKVDIKTTTPA PRPPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNPQEGLY NELQKDKMAEAYSEIGMKGERRRGKGHDGLYQGLSTATKDTYDALHMQALPPR | **727** |
| M2 CAR | MALPVTALLLPLALLLHAARPQVQLVQSGAEVKKPGASVKVSCKASGYTFTGYY MHWVRQAPGQGLEWMGWINPNSGGTNYAQKFQGRVTMTRDTSISTAYMELSRL RSDDTAVYYCARDLRRTVVTPRAYYGMDVWGQGTTVTVSSGGGGSGGGGSGG GGSGGGGSDIQLTQSPSTLSASVGDRVTITCQASQDISNSLNWYQQKAGKAPKLLI YDASTLETGVPSRFSGSGSGTDFSFTISSLQPEDIATYYCQQHDNLPLTFGQGTKV EIKTTTPAPRPPTPAPTIASQPLSLRPEACRPAAGGAVHTRGLDFACDIYIWAPLAG TCGVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRK NPQEGLYNELQKDKMAEAYSEIGMKGERRRGKGHDGLYQGLSTATKDTYDALHM QALPPR | **728** |
| M3 CAR | MALPVTALLLPLALLLHAARPQVQLVQSGAEVKKPGAPVKVSCKASGYTFTGYY MHWVRQAPGQGLEWMGWINPNSGGTNYAQKFQGRVTMTRDTSISTAYMELSRL RSDDTAVYYCARGEWDGSYYYDYWGQGTLVTVSSGGGGSGGGGSGGGGSGG GGSDIVLTQTPSSLSASVGDRVTITCRASQSINTYLNWYQHKPGKAPKLLIYAASSL QSGVPSRFSGSGSGTDFTLTISSLQPEDFATYYCQQSFSPLTFGGGTKLEIKTTTP APRPPTPAPTIASQPLSLRPEACRPAAGGAVHTRGLDFACDIYIWAPLAGTCGVLL LSLVITLYCKRGRKKLLYIFKQPFMRPVQTTQEEDGCSCRFPEEEEGGCELRVKFS RSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **729** |
| M4 CAR | MALPVTALLLPLALLLHAARPQVQLVESGGGLVQPGGSLRLSCAASGFTFSSYW MHWVRQVPGKGLVWVSRINTDGSTTTYADSVEGRFTISRDNAKNTLYLQMNSLR DDDTAVYYCVGGHWAVWGQGTTVTVSSGGGGSGGGGSGGGGSGGGGSDIQM TQSPSTLSASVGDRVTITCRASQSISDRLAWYQQKPGKAPKLLIYKASSLESGVPS RFSGSGSGTEFTLTISSLQPDDFAVYYCQQYGHLPMYTFGQGTKVEIKTTTPAPRP PTPAPTIASQPLSLRPEACRPAAGGAVHTRGLDFACDIYIWAPLAGTCGVLLLSLVI TLYCKRGRKKLLYIFKQPFMRPVQTTQEEDGCSCRFPEEEEGGCELRVKFSRSAD APAYKQGQNQLYNELNLGRREEYDVLDKRRGRDPEMGGKPRRKNPQEGLYNEL QKDKMAEAYSEIGMKGERRRGKGHDGLYQGLSTATKDTYDALHMQALPPR | **730** |
| M6 CAR | MALPVTALLLPLALLLHAARPQVQLVQSGAEVKKPGASVKVSCKASGYTFTSYY MHWVRQAPGQGLEWMGIINPSGGSTSYAQKFQGRVTMTRDTSTSTVYMELSSLR | **731** |
| | SEDTAVYYCARYRLIAVAGDYYYYGMDVWGQGTMVTVSSGGGGSGGGGSGGG GSGGGGSDIQMTQSPSSVSASVGDRVTITCRASQGVGRWLAWYQQKPGTAPKL LIYAASTLQSGVPSRFSGSGSGTDFTLTINNLQPEDFATYYCQQANSFPLTFGGGT RLEIKTTTPAPRPPTPAPTIASQPLSLRPEACRPAAGGAVHTRGLDFACDIYIWAPL AGTCGVLLLSLVITLYCKRGRKKLLYIFKQPFMRPVQTTQEEDGCSCRFPEEEEGG CELRVKFSRSADAPAYKQGQNQLYNELNLGRREEYDVLDKRRGRDPEMGGKPR RKNPQEGLYNELQKDKMAEAYSEIGMKGERRRGKGHDGLYQGLSTATKDTYDAL HMQALPPR | |
| M7 CAR | MALPVTALLLPLALLLHAARPQVQLVQSGGGVVQPGRSLRLSCAASGFTFSSYA MHWVRQAPGKGLEWVAVISYDGSNKYYADSVKGRFTISRDNSKNTLYLQMNSLR AEDTAVYYCARWKVSSSSPAFDYWGQGTLVTVSSGGGGSGGGGSGGGGSGGG GSEIVLTQSPATLSLSPGERAILSCRASQSVYTKYLGWYQQKPGQAPRLLIYDAST RATGIPDRFSGSGSGTDFTLTINRLEPEDFAVYYCQHYGGSPLITFGQGTRLEIKTT TPAPRPPTPAPTIASQPLSLRPEACRPAAGGAVHTRGLDFACDIYIWAPLAGTCGV LLLSLVITLYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALP PR | **732** |
| M8 CAR | MALPVTALLLPLALLLHAARPQVQLQQSGAEVKKPGASVKVSCKTSGYPFTGYS LHWVRQAPGQGLEWMGWINPNSGGTNYAQKFQGRVTMTRDTSISTAYMELSRL RSDDTAVYYCARDHYGGNSLFYWGQGTLVTVSSGGGGSGGGGSGGGGSGGG GSDIQLTQSPSSISASVGDTVSITCRASQDSGTWLAWYQQKPGKAPNLLMYDAST LEDGVPSRFSGSASGTEFTLTVNRLQPEDSATYYCQQYNSYPLTFGGGTKVDIKT TTPAPRPPTPAPTIASQPLSLRPEACRPAAGGAVHTRGLDFACDIYIWAPLAGTCG VLLLSLVITLYCKRGRKKLLYIFKQPFMRPVQTTQEEDGCSCRFPEEEEGGCELRV KFSRSADAPAYKQGQNQLYNELNLGRREEYDVLDKRRGRDPEMGGKPRRKNPQ EGLYNELQKDKMAEAYSEIGMKGERRRGKGHDGLYQGLSTATKDTYDALHMQAL PPR | **733** |
| M9 CAR | MALPVTALLLPLALLLHAARPQVQLVQSGAEVKKPGASVEVSCKASGYTFTSYY MHWVRQAPGQGLEWMGIINPSGGSTGYAQKFQGRVTMTRDTSTSTVHMELSSL RSEDTAVYYCARGGYSSSSDAFDIWGQGTMVTVSSGGGGSGGGGSGGGGSGG GGSDIQMTQSPPSLSASVGDRVTITCRASQDISSALAWYQQKPGTPPKLLIYDASS LESGVPSRFSGSGSGTDFTLTISSLQPEDFATYYCQQFSSYPLTFGGGTRLEIKTT TPAPRPPTPAPTIASQPLSLRPEACRPAAGGAVHTRGLDFACDIYIWAPLAGTCGV LLLSLVITLYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALP PR | **734** |
| M10 CAR | MALPVTALLLPLALLLHAARPQVQLVQSGAEVKKPGASVKVSCKASGYTFTSYGI SWVRQAPGQGLEWMGWISAYNGNTNYAQKLQGRVTMTTDTSTSTAYMELRSLR SDDTAVYYCARVAGGIYYYYGMDVWGQGTTITVSSGGGGSGGGGSGGGGSGG GGSDIVMTQTPDSLAVSLGERATISCKSSHSVLYNRNNKNYLAWYQQKPGQPPKL LFYWASTRKSGVPDRFSGSGSGTDFTLTISSLQPEDFATYFCQQTQTFPLTFGQG TRLEINTTTPAPRPPTPAPTIASQPLSLRPEACRPAAGGAVHTRGLDFACDIYIWAP LAGTCGVLLLSLVITLYCKRGRKKLLYIFKQPFMRPVQTTQEEDGCSCRFPEEEEG GCELRVKFSRSADAPAYKQGQNQLYNELNLGRREEYDVLDKRRGRDPEMGGKP RRKNPQEGLYNELQKDKMAEAYSEIGMKGERRRGKGHDGLYQGLSTATKDTYDA LHMQALPPR | **735** |
| M12 CAR | MALPVTALLLPLALLLHAARPQVQLVQSGAEVKKPGASVKVSCKASGYTFTGYY MHWVRQAPGQGLEWMGRINPNSGGTNYAQKFQGRVTMTTDTSTSTAYMELRSL RSDDTAVYYCARTTTSYAFDIWGQGTMVTVSSGGGGSGGGGSGGGGSGGGGS DIQLTQSPSTLSASVGDRVTITCRASQSISTWLAWYQQKPGKAPNLLIYKASTLESG VPSRFSGSGSGTEFTLTISSLQPDDFATYYCQQYNTYSPYTFGQGTKLEIKTTTPA PRPPTPAPTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCELRVKFSR SADAPAYKQGQNQLYNELNLGRREEYDVLDKRRGRDPEMGGKPRRKNPQEGLY | **736** |
| | NELQKDKMAEAYSEIGMKGERRRGKGHDGLYQGLSTATKDTYDALHMQALPPR | |
| M13 CAR | MALPVTALLLPLALLLHAARPQVQLVQSGGGLVKPGGSLRLSCEASGFIFSDYYM GWIRQAPGKGLEWVSYIGRSGSSMYYADSVKGRFTFSRDNAKNSLYLQMNSLRA EDTAVYYCAASPVVAATEDFQHWGQGTLVTVSSGGGGSGGGGSGGGGSGGGG SDIVMTQTPATLSLSPGERATLSCRASQSVTSNYLAWYQQKPGQAPRLLLFGAST RATGIPDRFSGSGSGTDFTLTINRLEPEDFAMYYCQQYGSAPVTFGQGTKLEIKTT TPAPRPPTPAPTIASQPLSLRPEACRPAAGGAVHTRGLDFACDIYIWAPLAGTCGV LLLSLVITLYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALP PR | **737** |
| M14 CAR | MALPVTALLLPLALLLHAARPQVQLVQSGAEVRAPGASVKISCKASGFTFRGYYI HWVRQAPGQGLEWMGIINPSGGSRAYAQKFQGRVTMTRDTSTSTVYMELSSLRS DDTAMYYCARTASCGGDCYYLDYWGQGTLVTVSSGGGGSGGGGSGGGGSGG GGSDIQMTQSPPTLSASVGDRVTITCRASENVNIWLAWYQQKPGKAPKLLIYKSSS LASGVPSRFSGSGSGAEFTLTISSLQPDDFATYYCQQYQSYPLTFGGGTKVDIKTT TPAPRPPTPAPTIASQPLSLRPEACRPAAGGAVHTRGLDFACDIYIWAPLAGTCGV LLLSLVITLYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALP PR | **738** |
| M15 CAR | MALPVTALLLPLALLLHAARPQVQLVQSGGGLVQPGRSLRLSCAASGFTFDDYA MHWVRQAPGKGLEWVSGISWNSGSIGYADSVKGRFTISRDNAKNSLYLQMNSLR AEDTAVYYCAKDGSSSWSWGYFDYWGQGTLVTVSSGGGGSGGGGSGGGGSS SELTQDPAVSVALGQTVRTTCQGDALRSYYASWYQQKPGQAPMLVIYGKNNRPS GIPDRFSGSDSGDTASLTITGAQAEDEADYYCNSRDSSGYPVFGTGTKVTVLTTTP APRPPTPAPTIASQPLSLRPEACRPAAGGAVHTRGLDFACDIYIWAPLAGTCGVLL LSLVITLYCKRGRKKLLYIFKQPFMRPVQTTQEEDGCSCRFPEEEEGGCELRVKFS RSADAPAYKQGQNQLYNELNLGRREEYDVLDKRRGRDPEMGGKPRRKNPQEGL YNELQKDKMAEAYSEIGMKGERRRGKGHDGLYQGLSTATKDTYDALHMQALPPR | **739** |
| M16 CAR | MALPVTALLLPLALLLHAARPEVQLVESGGGLVQPGRSLRLSCAASGFTFDDYA MHWVRQAPGKGLEWVSGISWNSGSTGYADSVKGRFTISRDNAKNSLYLQMNSL RAEDTALYYCAKDSSSWYGGGSAFDIWGQGTMVTVSSGGGGSGGGGSGGGGS SSELTQEPAVSVALGQTVRITCQGDSLRSYYASWYQQKPGQAPVLVIFGRSRRPS GIPDRFSGSSSGNTASLIITGAQAEDEADYYCNSRDNTANHYVFGTGTKLTVLTTT PAPRPPTPAPTIASQPLSLRPEACRPAAGGAVHTRGLDFACDIYIWAPLAGTCGVL LLSLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEG LYNELQKDKMAEAYSEIGMKGERRRGKGHDGLYQGLSTATKDTYDALHMQALPP R | **740** |
| M17 CAR | MALPVTALLLPLALLLHAARPEVQLVESGGGLVQPGRSLRLSCAASGFTFDDYA MHWVRQAPGKGLEWVSGISWNSGSTGYADSVKGRFTISRDNAKNSLYLQMNSL RAEDTALYYCAKDSSSWYGGGSAFDIWGQGTMVTVSSGGGGSGGGGSGGGGS SSELTQDPAVSVALGQTVRITCQGDSLRSYYASWYQQKPGQAPVLVIYGKNNRPS GIPDRFSGSSSGNTASLTITGAQAEDEADYYCNSRGSSGNHYVFGTGTKVTVLTT TPAPRPPTPAPTIASQPLSLRPEACRPAAGGAVHTRGLDFACDIYIWAPLAGTCGV LLLSLVITLYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALP PR | **741** |
| M18 CAR | MALPVTALLLPLALLLHAARPQVQLVQSGGGLVQPGGSLRLSCAASGFTFSSYW MHWVRQAPGKGLVWVSRINSDGSSTSYADSVKGRFTISRDNAKNTLYLQMNSLR AEDTAVYYCVRTGWVGSYYYYMDVWGKGTTVTVSSGGGGSGGGGSGGGGSG GGGSEIVLTQSPGTLSLSPGERATLSCRASQSVSSNYLAWYQQKPGQPPRLLIYD VSTRATGIPARFSGGGSGTDFTLTISSLEPEDFAVYYCQQRSNWPPWTFGQGTKV EIKTTTPAPRPPTPAPTIASQPLSLRPEACRPAAGGAVHTRGLDFACDIYIWAPLAG | **742** |
| | TCGVLLLSLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMGGKPRRK NPQEGLYNELQKDKMAEAYSEIGMKGERRRGKGHDGLYQGLSTATKDTYDALHM QALPPR | |
| M19 CAR | MALPVTALLLPLALLLHAARPQVQLVQSGGGVVQPGRSLRLSCAASGFTFSSYG MHWVRQAPGKGLEWVAVISYDGSNKYYADSVKGRFTISRDNSKNTLYLQMNSLR AEDTAVYYCAKGYSRYYYYGMDVWGQGTTVTVSSGGGGSGGGGSGGGGSGG GGSEIVMTQSPATLSLSPGERAILSCRASQSVYTKYLGWYQQKPGQAPRLLIYDAS TRATGIPDRFSGSGSGTDFTLTINRLEPEDFAVYYCQHYGGSPLITFGQGTKVDIKT TTPAPRPPTPAPTIASQPLSLRPEACRPAAGGAVHTRGLDFACDIYIWAPLAGTCG VLLLSLVITLYCKRGRKKLLYIFKQPFMRPVQTTQEEDGCSCRFPEEEEGGCELRV KFSRSADAPAYKQGQNQLYNELNLGRREEYDVLDKRRGRDPEMGGKPRRKNPQ EGLYNELQKDKMAEAYSEIGMKGERRRGKGHDGLYQGLSTATKDTYDALHMQAL PPR | **743** |
| M20 CAR | MALPVTALLLPLALLLHAARPQVQLVQSGGGLVQPGGSLRLSCAASGFTFSSYA MSWVRQAPGKGLEWVSAISGSGGSTYYADSVKGRFTISRDNSKNTLYLQMNSLR AEDTAVYYCAKREAAAGHDWYFDLWGRGTLVTVSSGGGGSGGGGSGGGGSGG GGSDIRVTQSPSSLSASVGDRVTITCRASQSISSYLNWYQQKPGKAPKLLIYAASS LQSGVPSRFSGSGSGTDFTLTISSLQPEDFATYYCQQSYSIPLTFGQGTKVEIKTTT PAPRPPTPAPTIASQPLSLRPEACRPAAGGAVHTRGLDFACDIYIWAPLAGTCGVL LLSLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEG LYNELQKDKMAEAYSEIGMKGERRRGKGHDGLYQGLSTATKDTYDALHMQALPP R | **744** |
| M21 CAR | MALPVTALLLPLALLLHAARPQVQLVQSWAEVKKPGASVKVSCKASGYTFTSYY MHWVRQAPGQGLEWMGIINPSGGSTSYAQKFQGRVTMTRDTSTSTVYMELSNL RSEDTAVYYCARSPRVTTGYFDYWGQGTLVTVSSGGGGSGGGGSGGGGSGGG GSDIQLTQSPSTLSASVGDRVTITCRASQSISSWLAWYQQKPGKAPKLLIYKASSL ESGVPSRFSGSGSGTEFTLTISSLQPDDFATYYCQQYSSYPLTFGGGTRLEIKTTT PAPRPPTPAPTIASQPLSLRPEACRPAAGGAVHTRGLDFACDIYIWAPLAGTCGVL LLSLVITLYCKRGRKKLLYIFKQPFMRPVQTTQEEDGCSCRFPEEEEGGCELRVKF SRSADAPAYKQGQNQLYNELNLGRREEYDVLDKRRGRDPEMGGKPRRKNPQEG LYNELQKDKMAEAYSEIGMKGERRRGKGHDGLYQGLSTATKDTYDALHMQALPP R | **745** |
| M22 CAR | MALPVTALLLPLALLLHAARPQVQLVQSGAEVRRPGASVKISCRASGDTSTRHYI HWLRQAPGQGPEWMGVINPTTGPATGSPAYAQMLQGRVTMTRDTSTRTVYMEL RSLRFEDTAVYYCARSVVGRSAPYYFDYWGQGTLVTVSSGGGGSGGGGSGGG GSGGGGSDIQMTQSPSSLSASVGDRVTITCRASQGISDYSAWYQQKPGKAPKLLI YAASTLQSGVPSRFSGSGSGTDFTLTISYLQSEDFATYYCQQYYSYPLTFGGGTK VDIKTTTPAPRPPTPAPTIASQPLSLRPEACRPAAGGAVHTRGLDFACDIYIWAPLA GTCGVLLLSLVITLYCKRGRKKLLYIFKQPFMRPVQTTQEEDGCSCRFPEEEEGGC ELRVKFSRSADAPAYKQGQNQLYNELNLGRREEYDVLDKRRGRDPEMGGKPRR KNPQEGLYNELQKDKMAEAYSEIGMKGERRRGKGHDGLYQGLSTATKDTYDALH MQALPPR | **746** |
| M23 CAR | MALPVTALLLPLALLLHAARPQVQLQQSGAEVKKPGASVKVSCKASGYTFTNYY MHWVRQAPGQGLEWMGIINPSGGYTTYAQKFQGRLTMTRDTSTSTVYMELSSLR SEDTAVYYCARIRSCGGDCYYFDNWGQGTLVTVSSGGGGSGGGGSGGGGSGG GGSDIQLTQSPSTLSASVGDRVTITCRASENVNIWLAWYQQKPGKAPKLLIYKSSS LASGVPSRFSGSGSGAEFTLTISSLQPDDFATYYCQQYQSYPLTFGGGTKVDIKTT TPAPRPPTPAPTIASQPLSLRPEACRPAAGGAVHTRGLDFACDIYIWAPLAGTCGV LLLSLVITLYCKRGRKKLLYIFKQPFMRPVQTTQEEDGCSCRFPEEEEGGCELRVK FSRSADAPAYKQGQNQLYNELNLGRREEYDVLDKRRGRDPEMGGKPRRKNPQE GLYNELQKDKMAEAYSEIGMKGERRRGKGHDGLYQGLSTATKDTYDALHMQALP PR | **747** |
| M24 CAR | MALPVTALLLPLALLLHAARPQITLKESGPALVKPTQTLTLTCTFSGFSLSTAGVH VGWIRQPPGKALEWLALISWADDKRYRPSLRSRLDITRVTSKDQVVLSMTNMQPE | **748** |
| | DTATYYCALQGFDGYEANWGPGTLVTVSSGGGGSGGGGSGGGGSGGGGSDIV MTQSPSSLSASAGDRVTITCRASRGISSALAWYQQKPGKPPKLLIYDASSLESGVP SRFSGSGSGTDFTLTIDSLEPEDFATYYCQQSYSTPWTFGQGTKVDIKTTTPAPRP PTPAPTIASQPLSLRPEACRPAAGGAVHTRGLDFACDIYIWAPLAGTCGVLLLSLVI TLYCKRGRKKLLYIFKQPFMRPVQTTQEEDGCSCRFPEEEEGGCELRVKFSRSAD APAYKQGQNQLYNELNLGRREEYDVLDKRRGRDPEMGGKPRRKNPQEGLYNEL QKDKMAEAYSEIGMKGERRRGKGHDGLYQGLSTATKDTYDALHMQALPPR | |

In one embodiment, a CAR molecule that binds mesothelin, comprises (e.g., consists of) an amino acid sequence as provided in Table 17, or Table 2 of International Publication No. WO2015/090230, filed December 19, 2014. In one embodiment, the CAR molecule comprises (e.g., consists of) an amino acid sequence selected from any one of SEQ ID NOs: 724-748; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence selected from any one of SEQ ID NOs: 724-748; or an amino acid sequence having 85%, 90%, 95%, 96%, 97%, 98%, 99% identity to an amino acid sequence selected from any one of SEQ ID NOs: 724-748.

In one embodiment, the CAR molecule comprises an EGFRvIII antigen binding domain (e.g., a murine, human or humanized antibody or antibody fragment that specifically binds to EGFRvlll), a transmembrane domain, and an intracellular signaling domain (e.g., an intracellular signaling domain comprising a costimulatory domain and/or a primary signaling domain).

Exemplary CAR molecules that target EGFRvIII are described herein, and are provided in Table 18, or in Table 2 of WO/2014/130657 or as described in WO2016/014789.

**Table 18. Exemplary Humanized CAR Constructs. Sequences are provided with a leader, and the CDRs are underlined. Nt stands for nucleic acid and aa stands for amino acid**

| **Name** | **SEQ ID NO:** | **Sequence** |
|---|---|---|
| **CAR 1** | | |
| CAR 1 - Full - nt | 769 | atggccctccctgtcaccgccctgctgcttccgctggctcttctgctccacgccgctcggcccgagatc cagctggtgcagtcgggagctgaagtcaaaaagcctggcgcaaccgtcaagatctcgtgcaaag gatcagggttcaacatcgaggactactacatccattgggtgcaacaggcacccggaaaaggcctg gagtggatggggaggattgacccagaaaatgacgaaaccaagtacggaccgatcttccaagga cgggtgaccatcacggctgacacttccactaacaccgtctacatggaactctcgagccttcgctcgg aagataccgcggtgtactactgcgcctttagaggtggagtctactggggacaagggactaccgtca ccgtgtcgtcaggtggcggaggatcaggcggaggcggctccggtggaggaggaagcggagga ggtggctccgacgtggtgatgacgcagtcaccggactccttggcggtgagcctgggtgaacgcgcc actatcaactgcaagagctcccagagcttgctggactccgatggaaagacttatctcaattggctgca acagaagcctggccagccgccaaagagactcatctcactggtgagcaagctggatagcggagtg ccagatcggttttcgggatcgggctcaggcaccgacttcaccctgactatttcctccctccaagccga ggatgtggccgtctactactgttggcaggggactcacttcccggggaccttcggtggaggcactaag gtggagatcaaaaccactaccccagcaccgaggccacccaccccggctcctaccatcgcctccc agcctctgtccctgcgtccggaggcatgtagacccgcagctggtggggccgtgcatacccggggtc ttgacttcgcctgcgatatctacatttgggcccctctggctggtacttgcggggtcctgctgctttcactcg tgatcactctttactgtaagcgcggtcggaagaagctgctgtacatctttaagcaacccttcatgaggc |
| | | ctgtgcagactactcaagaggaggacggctgttcatgccggttcccagaggaggaggaaggcgg ctgcgaactgcgcgtgaaattcagccgcagcgcagatgctccagcctacaagcaggggcagaac cagctctacaacgaactcaatcttggtcggagagaggagtacgacgtgctggacaagcggagag gacgggacccagaaatgggcgggaagccgcgcagaaagaatccccaagagggcctgtacaa cgagctccaaaaggataagatggcagaagcctatagcgagattggtatgaaaggggaacgcag aagaggcaaaggccacgacggactgtaccagggactcagcaccgccaccaaggacacctatg acgctcttcacatgcaggccctgccgcctcgg |
| CAR 1 - Full - aa | 770 | malpvtalllplalllhaarpeiqlvqsgaevkkpgatvkisckgsgfniedyyihwvqqapgkglew mgridpendetkyqpifqgrvtitadtstntvymelsslrsedtavyycafrggvywgqgttvtvss ggggsggggsggggsggggsdvvmtqspdslavslgeratinckssqsllldsdgktylnwlqq kpgqppkrlislvskldsgvpdrfsgsgsgtdftltisslqaedvavyycwqgthfpqtfgggtkveik tttpaprpptpaptiasqplslrpeacrpaaggavhtrgldfacdiyiwaplagtcgvlllslvitlyckrgrk kllyifkqpfmrpvqttqeedgcscrfpeeeeggcelrvkfsrsadapaykqgqnqlynelnlgrree ydvldkrrgrdpemggkprrknpqeglynelqkdkmaeayseigmkgerrrgkghdglyqglsta tkdtydalhmqalppr |

| **CAR 2** | | |
|---|---|---|
| CAR 2 - Full - nt | 771 | atggccctccctgtcaccgccctgctgcttccgctggctcttctgctccacgccgctcggcccgacgtg gtcatgactcaaagcccagattccttggctgtctcccttggagaaagagcaacgatcaattgcaaaa gctcgcagtccctgttggactccgatggaaaaacctacctcaactggctgcagcagaagccggga caaccaccaaagcggctgatttccctcgtgtccaagctggacagcggcgtgccggatcgcttctcgg gcagcggctcgggaaccgattttactctcactatttcgtcactgcaagcggaggacgtggcggtgtat tactgctggcagggcactcacttcccgggtacttttggtggaggtaccaaagtcgaaatcaagggtg gaggcgggagcggaggaggcgggtcgggaggaggaggatcgggtggcggaggctcagaaat ccagctggtgcagtcaggtgccgaagtgaagaagcctggggccacggtgaagatctcgtgcaag gggagcggattcaacatcgaggattactacatccattgggtgcaacaggcccctggcaaagggct ggaatggatgggaaggatcgaccccgagaatgacgagactaagtacggcccgatcttccaagga cgggtgaccatcactgcagacacttcaaccaacaccgtctacatggaactctcctcgctgcgctccg aggacaccgccgtgtactactgtgctttcagaggaggagtctactggggacagggaacgaccgtg accgtcagctcaaccactaccccagcaccgaggccacccaccccggctcctaccatcgcctccca gcctctgtccctgcgtccggaggcatgtagacccgcagctggtggggccgtgcatacccggggtctt gacttcgcctgcgatatctacatttgggcccctctggctggtacttgcggggtcctgctgctttcactcgt gatcactctttactgtaagcgcggtcggaagaagctgctgtacatctttaagcaacccttcatgaggcc tgtgcagactactcaagaggaggacggctgttcatgccggttcccagaggaggaggaaggcggct gcgaactgcgcgtgaaattcagccgcagcgcagatgctccagcctacaagcaggggcagaacc agctctacaacgaactcaatcttggtcggagagaggagtacgacgtgctggacaagcggagagg acgggacccagaaatgggcgggaagccgcgcagaaagaatccccaagagggcctgtacaac gagctccaaaaggataagatggcagaagcctatagcgagattggtatgaaaggggaacgcaga agaggcaaaggccacgacggactgtaccagggactcagcaccgccaccaaggacacctatga cgctcttcacatgcaggccctgccgcctcgg |
| CAR 2 - Full - aa | 772 | malpvtalllplalllhaarpdvvmtqspdslavslgeratinckssqslldsdgktylnwlqqkpgq ppkrlislvskldsgvpdrfsgsgsgtdftltisslqaedvavyycwqgthfpqtfgggtkveikggg gsggggsggggsggggseiqlvqsgaevkkpgatvkisckgsgfniedyyihwvqqapgkgle wmgridpendetkygpifqgrvtitadtstntvymelsslrsedtavyycafrggvywgqgttvtv sstttpaprpptpaptiasqplslrpeacrpaaggavhtrgldfacdiyiwaplagtcgvlllslvitlyckr grkkllyifkqpfmrpvqttqeedgcscrfpeeeeggcelrvkfsrsadapaykqgqnqlynelnlgrr eeydvldkrrgrdpemggkprrknpqeglynelqkdkmaeayseigmkgerrrgkghdglyqgl statkdtydalhmqalppr |

| **CAR 3** | | |
|---|---|---|
| CAR 3 - Full - nt | 773 | atggccctccctgtcaccgccctgctgcttccgctggctcttctgctccacgccgctcggcccgaaatc cagctggtgcaaagcggagccgaggtgaagaagcccggagaatccctgcgcatctcgtgtaagg gttccggctttaacatcgaggattactacatccactgggtgagacagatgccgggcaaaggtctgga atggatgggccgcatcgacccggagaacgacgaaaccaaatacggaccaatcttccaaggaca tgtgactatttccgcggatacctccatcaacactgtctacttgcagtggagctcgctcaaggcgtcgga taccgccatgtactactgcgcattcagaggaggtgtgtactggggccagggcactacggtcaccgtg tcctcgggaggtggagggtcaggaggcggaggctcgggcggtggaggatcaggcggaggagg aagcgatgtggtcatgactcaatccccactgtcactgcctgtcactctggggcaaccggcttccatctc |
| | | atgcaagtcaagccaatcgctgctcgactccgacggaaaaacctacctcaattggcttcagcagcg cccaggccagtcgcctcggaggctgatctcactcgtgtcgaagcttgactccggggtgccggatcg gtttagcggaagcggatcggggaccgacttcacgttgaagattagccgggtggaagccgaggac gtgggagtctattactgctggcaggggacccacttcccggggactttcggaggaggcaccaaagtc gagattaagaccactaccccagcaccgaggccacccaccccggctcctaccatcgcctcccagc ctctgtccctgcgtccggaggcatgtagacccgcagctggtggggccgtgcatacccggggtcttga cttcgcctgcgatatctacatttgggcccctctggctggtacttgcggggtcctgctgctttcactcgtgat cactctttactgtaagcgcggtcggaagaagctgctgtacatctttaagcaacccttcatgaggcctgt gcagactactcaagaggaggacggctgttcatgccggttcccagaggaggaggaaggcggctgc gaactgcgcgtgaaattcagccgcagcgcagatgctccagcctacaagcaggggcagaaccag ctctacaacgaactcaatcttggtcggagagaggagtacgacgtgctggacaagcggagaggac gggacccagaaatgggcgggaagccgcgcagaaagaatccccaagagggcctgtacaacga gctccaaaaggataagatggcagaagcctatagcgagattggtatgaaaggggaacgcagaag aggcaaaggccacgacggactgtaccagggactcagcaccgccaccaaggacacctatgacg ctcttcacatgcaggccctgccgcctcgg |
| **CAR 3 -** Full - aa | 774 | malpvtalllplalllhaarpeiqlvqsgaevkkpgeslrisckgsgfniedyyihwvrqmpgkglew mgridpendetkygpifqghvtisadtsintvylqwsslkasdtamyycafrggvywgqgttvtv ssggggsggggsggggsggggsdvvmtqsplslpvtlgqpasisckssqslldsdgktylnwlq qrpgqsprrlislvskldsgvpdrfsgsgsgtdftlkisrveaedvgvyycwqgthfpqtfgggtkvei ktttpaprpptpaptiasqplslrpeacrpaaggavhtrgldfacdiyiwaplagtcgvlllslvitlyckrgr kkllyifkqpfmrpvqttqeedgcscrfpeeeeggcelrvkfsrsadapaykqgqnqlynelnlgrre eydvldkrrgrdpemggkprrknpqeglynelqkdkmaeayseigmkgerrrgkghdglyqglst atkdtydalhmqalppr |

| **CAR 4** | | |
|---|---|---|
| CAR 4 - Full - nt | 775 | atggccctccctgtcaccgccctgctgcttccgctggctcttctgctccacgccgctcggcccgacgtc gtcatgacccaatcccctctctccctgccggtcaccctgggtcagccggcgtcgatctcatgcaaaa gctcacagtccctgctggattcggacggaaaaacctacttgaactggctccaacagaggccgggtc agtcccctcgcagactgatctcgctggtgagcaagctcgactcgggtgtgccggatcggttctccgg gtcaggatcgggcaccgactttacgctcaagatttcgagagtggaggccgaggatgtgggagtgta ctattgctggcagggcacgcatttccccgggacctttggaggcgggactaaggtggaaatcaaggg aggtggcggatcaggcggaggaggcagcggcggaggtggatcaggaggcggagggtcagag atccagctggtccaaagcggagcagaggtgaagaagccaggcgagtcccttcgcatttcgtgcaa agggagcggcttcaacattgaagattactacatccactgggtgcggcaaatgccaggaaagggtct ggaatggatgggacggatcgacccagaaaatgatgaaactaagtacggaccgatcttccaagga cacgtcactatctccgcggacacttcgatcaacaccgtgtacctccagtggagcagcttgaaagcct ccgacaccgctatgtactactgtgccttccgcggaggagtctactggggacaggggactactgtgac cgtgtcgtccaccactaccccagcaccgaggccacccaccccggctcctaccatcgcctcccagc ctctgtccctgcgtccggaggcatgtagacccgcagctggtggggccgtgcatacccggggtcttga cttcgcctgcgatatctacatttgggcccctctggctggtacttgcggggtcctgctgctttcactcgtgat cactctttactgtaagcgcggtcggaagaagctgctgtacatctttaagcaacccttcatgaggcctgt gcagactactcaagaggaggacggctgttcatgccggttcccagaggaggaggaaggcggctgc gaactgcgcgtgaaattcagccgcagcgcagatgctccagcctacaagcaggggcagaaccag ctctacaacgaactcaatcttggtcggagagaggagtacgacgtgctggacaagcggagaggac gggacccagaaatgggcgggaagccgcgcagaaagaatccccaagagggcctgtacaacga gctccaaaaggataagatggcagaagcctatagcgagattggtatgaaaggggaacgcagaag aggcaaaggccacgacggactgtaccagggactcagcaccgccaccaaggacacctatgacg ctcttcacatgcaggccctgccgcctcgg |
| CAR 4 - Full - aa | 776 | malpvtalllplalllhaarpdvvmtqsplslpvtlgqpasisckssqslldsdgktylnwlqqrpgqs prrlislvskldsgvpdrfsgsgsgtdftlkisrveaedvgvyycwqgthfpqtfgggtkveikgggg sggggsggggsggggseiqlvqsgaevkkpgeslrisckgsgfniedyyihwvrqmpgkglew mgridpendetkygpifqghvtisadtsintvylqwsslkasdtamyycafrggvywgqgttvtv sstttpaprpptpaptiasqplslrpeacrpaaggavhtrgldfacdiyiwaplagtcgvlllslvitlyckr grkkllyifkqpfmrpvqttqeedgcscrfpeeeeggcelrvkfsrsadapaykqgqnqlynelnlgrr eeydvldkrrgrdpemggkprrknpqeglynelqkdkmaeayseigmkgerrrgkghdglyqgl statkdtydalhmqalppr |

| **CAR 5** | | |
|---|---|---|
| CAR 5 - Full - nt | 777 | atggccctccctgtcaccgccctgctgcttccgctggctcttctgctccacgccgctcggcccgaaatc cagctcgtgcagagcggagccgaggtcaagaaaccgggtgctaccgtgaagatttcatgcaagg gatcgggcttcaacatcgaggattactacatccactgggtgcagcaggcaccaggaaaaggactt gaatggatgggccggatcgacccggaaaatgacgagactaagtacggccctatcttccaaggac gggtgacgatcaccgcagacactagcaccaacaccgtctatatggaactctcgtccctgaggtccg aagatactgccgtgtactactgtgcgtttcgcggaggtgtgtactggggacagggtaccaccgtcac cgtgtcatcgggcggtggaggctccggtggaggagggtcaggaggcggtggaagcggaggagg cggcagcgacgtggtcatgactcaatcgccgctgtcgctgcccgtcactctgggacaacccgcgtc catcagctgcaaatcctcgcagtcactgcttgactccgatggaaagacctacctcaactggctgcag caacgcccaggccaatccccaagacgcctgatctcgttggtgtcaaagctggactcaggggtgcc ggaccggttctccgggagcgggtcgggcacggatttcactctcaagatctccagagtggaagccg aggatgtgggagtctactactgctggcagggaacccatttccctggaacttttggcggaggaactaa ggtcgagattaaaaccactaccccagcaccgaggccacccaccccggctcctaccatcgcctccc agcctctgtccctgcgtccggaggcatgtagacccgcagctggtggggccgtgcatacccggggtc ttgacttcgcctgcgatatctacatttgggcccctctggctggtacttgcggggtcctgctgctttcactcg tgatcactctttactgtaagcgcggtcggaagaagctgctgtacatctttaagcaacccttcatgaggc ctgtgcagactactcaagaggaggacggctgttcatgccggttcccagaggaggaggaaggcgg ctgcgaactgcgcgtgaaattcagccgcagcgcagatgctccagcctacaagcaggggcagaac cagctctacaacgaactcaatcttggtcggagagaggagtacgacgtgctggacaagcggagag gacgggacccagaaatgggcgggaagccgcgcagaaagaatccccaagagggcctgtacaa cgagctccaaaaggataagatggcagaagcctatagcgagattggtatgaaaggggaacgcag aagaggcaaaggccacgacggactgtaccagggactcagcaccgccaccaaggacacctatg acgctcttcacatgcaggccctgccgcctcgg |
| CAR 5 - Full - aa | 778 | malpvtalllplalllhaarpeiqlvqsgaevkkpgatvkisckgsgfniedyyihwvqqapgkglew mgridpendetkyqpifqgrvtitadtstntvymelsslrsedtavyycafrggvywgqgttvtvss ggggsggggsggggsggggsdvvmtqsplslpvtlgqpasisckssqsllldsdgktylnwlqqr pgqsprrlislvsklldsgvpdrfsgsgsgtdftlkisrveaedvgvyycwqgthfpqtfgggtkveikt ttpaprpptpaptiasqplslrpeacrpaaggavhtrgldfacdiyiwaplagtcgvlllslvitlyckrgrk kllyifkqpfmrpvqttqeedgcscrfpeeeeggcelrvkfsrsadapaykqgqnqlynelnlgrree ydvldkrrgrdpemggkprrknpqeglynelqkdkmaeayseigmkgerrrgkghdglyqglsta tkdtydalhmqalppr |

| **CAR 6** | | |
|---|---|---|
| CAR6 - Full - nt | 779 | atggccctccctgtcaccgccctgctgcttccgctggctcttctgctccacgccgctcggcccgagatt cagctcgtgcaatcgggagcggaagtcaagaagccaggagagtccttgcggatctcatgcaagg gtagcggctttaacatcgaggattactacatccactgggtgaggcagatgccggggaagggactcg aatggatgggacggatcgacccagaaaacgacgaaactaagtacggtccgatcttccaaggcca tgtgactattagcgccgatacttcaatcaataccgtgtatctgcaatggtcctcattgaaagcctcagat accgcgatgtactactgtgctttcagaggaggggtctactggggacagggaactaccgtgactgtct cgtccggcggaggcgggtcaggaggtggcggcagcggaggaggagggtccggcggaggtgg gtccgacgtcgtgatgacccagagccctgacagcctggcagtgagcctgggcgaaagagctacc attaactgcaaatcgtcgcagagcctgctggactcggacggaaaaacgtacctcaattggctgcag caaaagcctggccagccaccgaagcgccttatctcactggtgtcgaagctggattcgggagtgccc gatcgcttctccggctcgggatcgggtactgacttcaccctcactatctcctcgcttcaagcagaggac gtggccgtctactactgctggcagggaacccactttccgggaaccttcggcggagggacgaaagt ggagatcaagaccactaccccagcaccgaggccacccaccccggctcctaccatcgcctcccag cctctgtccctgcgtccggaggcatgtagacccgcagctggtggggccgtgcatacccggggtcttg acttcgcctgcgatatctacatttgggcccctctggctggtacttgcggggtcctgctgctttcactcgtg atcactctttactgtaagcgcggtcggaagaagctgctgtacatctttaagcaacccttcatgaggcct gtgcagactactcaagaggaggacggctgttcatgccggttcccagaggaggaggaaggcggct gcgaactgcgcgtgaaattcagccgcagcgcagatgctccagcctacaagcaggggcagaacc agctctacaacgaactcaatcttggtcggagagaggagtacgacgtgctggacaagcggagagg acgggacccagaaatgggcgggaagccgcgcagaaagaatccccaagagggcctgtacaac gagctccaaaaggataagatggcagaagcctatagcgagattggtatgaaaggggaacgcaga agaggcaaaggccacgacggactgtaccagggactcagcaccgccaccaaggacacctatga cgctcttcacatgcaggccctgccgcctcgg |
| CAR6 - | 780 | malpvtalllplalllhaarpeiqlvqsgaevkkpgeslrisckgsgfniedyyihwvrqmpgkglew |
| Full **- aa** | | mgridpendetkyqpifqghvtisadtsintvylqwsslkasdtamyycafrggvywgqgttvtv ssggggsggggsggggsggggsdvvmtqspdslavslgeratinckssqslldsdgktylnwl qqkpgqppkrlislvskldsgvpdrfsgsgsgtdftltisslqaedvavyycwqgthfpqtfgggtkv eikttttpaprpptpaptiasqplslrpeacrpaaggavhtrgldfacdiyiwaplagtcgvlllslvitlyckr grkkllyifkqpfmrpvqttqeedgcscrfpeeeeggcelrvkfsrsadapaykqgqnqlynelnlgrr eeydvldkrrgrdpemggkprrknpqeglynelqkdkmaeayseigmkgerrrgkghdglyqgl statkdtydalhmqalppr |

| **CAR 7** | | |
|---|---|---|
| CAR 7 Full - nt | 781 | atggccctccctgtcaccgccctgctgcttccgctggctcttctgctccacgccgctcggcccgacgtg gtgatgactcagtcgcctgactcgctggctgtgtcccttggagagcgggccactatcaattgcaagtc atcccagtcgctgctggattccgacgggaaaacctacctcaattggctgcagcaaaaaccgggac agcctccaaagcggctcatcagcctggtgtccaagttggacagcggcgtgccagaccgcttctccg gttcgggaagcggtactgatttcacgctgaccatctcatccctccaagcggaggatgtggcagtctac tactgttggcagggcacgcattttccgggcacttttggaggagggaccaaggtcgaaatcaaggga ggaggtggctcgggcggaggaggctcgggaggaggaggatcaggaggcggtggaagcgaga ttcaactggtccagagcggcgcagaagtcaagaagccgggtgaatcgctcagaatctcgtgcaaa ggatcgggattcaacatcgaggactactacattcactgggtcagacaaatgccgggcaaagggct ggaatggatggggaggatcgaccccgaaaacgatgaaaccaagtacggaccaatcttccaagg gcacgtgaccatttcggcggacacctcaatcaacactgtgtacctccagtggagctcacttaaggcc agcgataccgccatgtactattgcgctttccgcggaggggtgtactggggacagggcactactgtga ccgtgtcatccaccactaccccagcaccgaggccacccaccccggctcctaccatcgcctcccag cctctgtccctgcgtccggaggcatgtagacccgcagctggtggggccgtgcatacccggggtcttg acttcgcctgcgatatctacatttgggcccctctggctggtacttgcggggtcctgctgctttcactcgtg atcactctttactgtaagcgcggtcggaagaagctgctgtacatctttaagcaacccttcatgaggcct gtgcagactactcaagaggaggacggctgttcatgccggttcccagaggaggaggaaggcggct gcgaactgcgcgtgaaattcagccgcagcgcagatgctccagcctacaagcaggggcagaacc agctctacaacgaactcaatcttggtcggagagaggagtacgacgtgctggacaagcggagagg acgggacccagaaatgggcgggaagccgcgcagaaagaatccccaagagggcctgtacaac gagctccaaaaggataagatggcagaagcctatagcgagattggtatgaaaggggaacgcaga agaggcaaaggccacgacggactgtaccagggactcagcaccgccaccaaggacacctatga cgctcttcacatgcaggccctgccgcctcgg |
| CAR 7 Full - aa | 782 | malpvtalllplalllhaarpdvvmtqspdslavslgeratinckssqslldsdgktylnwlqqkpgq ppkrlislvskldsgvpdrfsgsgsgtdftltisslqaedvavyycwqgthfpqtfgggtkveikggg gsggggsggggsggggseiqlvqsgaevkkpgeslrisckgsgfniedyyihwvrqmpgkgle wmgridpendetkyqpifqghvtisadtsintvylqwsslkasdtamyycafrggvywgqgttvt vssttpaprpptpaptiasqplslrpeacrpaaggavhtrgldfacdiyiwaplagtcgvlllslvitlyck rgrkkllyifkqpfmrpvqttqeedgcscrfpeeeeggcelrvkfsrsadapaykqgqnqlynelnlgr reeydvldkrrgrdpemggkprrknpqeglynelqkdkmaeayseigmkgerrrgkghdglyqgl statkdtydalhmqalppr |

| **CAR 8** | | |
|---|---|---|
| CAR 8 - Full - nt | 783 | atggccctccctgtcaccgccctgctgcttccgctggctcttctgctccacgccgctcggcccgatgtg gtcatgacgcagtcaccactgtccctccccgtgacccttggacagccagcgtcgattagctgcaagt catcccaatccctgctcgattcggatggaaagacctatctcaactggctgcagcaaagacccggtc agagccctaggagactcatctcgttggtgtcaaagctggacagcggagtgccggaccggttttccg gttcgggatcggggacggacttcactctgaagatttcacgggtggaagctgaggatgtgggagtgta ctactgctggcagggaacccatttccctggcacttttggcggaggaactaaggtcgaaatcaaggg aggaggtggctcgggaggaggcggatcgggcggaggcgggagcggcggaggagggtccgaa atccaacttgtccagtcaggagccgaagtgaagaaaccgggagccaccgtcaaaatcagctgta agggatcgggattcaatatcgaggactactacatccactgggtgcagcaagctccgggcaaagga ctggagtggatggggcgcatcgacccagagaacgacgaaaccaaatacggcccgatcttccaa gggcgggtgaccatcaccgcggacacctcaactaacactgtgtacatggagctgagctccctgcg ctccgaagatactgcagtctactactgcgccttccgcggtggtgtgtactggggacagggcaccact gtgactgtcagctcgaccactaccccagcaccgaggccacccaccccggctcctaccatcgcctcc cagcctctgtccctgcgtccggaggcatgtagacccgcagctggtggggccgtgcatacccggggt cttgacttcgcctgcgatatctacatttgggcccctctggctggtacttgcggggtcctgctgctttcactc gtgatcactctttactgtaagcgcggtcggaagaagctgctgtacatctttaagcaacccttcatgagg |
| | | cctgtgcagactactcaagaggaggacggctgttcatgccggttcccagaggaggaggaaggcg gctgcgaactgcgcgtgaaattcagccgcagcgcagatgctccagcctacaagcaggggcagaa ccagctctacaacgaactcaatcttggtcggagagaggagtacgacgtgctggacaagcggaga ggacgggacccagaaatgggcgggaagccgcgcagaaagaatccccaagagggcctgtaca acgagctccaaaaggataagatggcagaagcctatagcgagattggtatgaaaggggaacgca gaagaggcaaaggccacgacggactgtaccagggactcagcaccgccaccaaggacacctat gacgctcttcacatgcaggccctgccgcctcgg |
| CAR 8 - Full - aa | 784 | malpvtalllplalllhaarpdvvmtqsplslpvtlgqpasisckssqslldsdgktylnwlqqrpgqs prrlislvskldsgvpdrfsgsgsgtdftlkisrveaedvgvyycwqgthfpqtfgggtkveikgggg sggggsggggsggggseiqlvqsgaevkkpgatvkisckgsgfniedyyihwvqqapgkglew mgridpendetkygpifqgrvtitadtstntvymelsslrsedtavyycafrggvywgqgttvtvss tttpaprpptpaptiasqplslrpeacrpaaggavhtrgldfacdiyiwaplagtcgvlllslvitlyckrgrk kllyifkqpfmrpvqttqeedgcscrfpeeeeggcelrvkfsrsadapaykqgqnqlynelnlgrree ydvldkrrgrdpemggkprrknpqeglynelqkdkmaeayseigmkgerrrgkghdglyqglsta tkdtydalhmqalppr |

| **CAR 9** | | Mouse anti-EGFRvIII clone 3C10 |
|---|---|---|
| CAR 9 - Full - nt | 785 | atggccctccctgtcaccgccctgctgcttccgctggctcttctgctccacgccgctcggcccgagatc cagctccaacagagcggagccgaactggtcaaaccgggagcgtcggtgaagttgtcatgcactg gatcgggcttcaacatcgaggattactacatccactgggtcaagcaacgcaccgagcaggggctg gaatggatcggacggatcgaccccgaaaacgatgaaaccaagtacggccctatcttccaaggac gggccaccattacggctgacacgtcaagcaataccgtctacctccagctttccagcctgacctccga ggacactgccgtgtactactgcgccttcagaggaggcgtgtactggggaccaggaaccactttgac cgtgtccagcggaggcggtggatcaggaggaggaggctcaggcggtggcggctcgcacatgga cgtggtcatgactcagtccccgctgaccctgtcggtggcaattggacagagcgcatccatctcgtgc aagagctcacagtcgctgctggattccgacggaaagacttatctgaactggctgctccaaagacca gggcaatcaccgaaacgccttatctccctggtgtcgaaactcgactcgggtgtgccggatcggtttac cggtagcgggtccggcacggacttcactctccgcatttcgagggtggaagcggaggatctcgggat ctactactgttggcagggaacccacttccctgggacttttggaggcggaactaagctggaaatcaag accactaccccagcaccgaggccacccaccccggctcctaccatcgcctcccagcctctgtccctg cgtccggaggcatgtagacccgcagctggtggggccgtgcatacccggggtcttgacttcgcctgc gatatctacatttgggcccctctggctggtacttgcggggtcctgctgctttcactcgtgatcactctttact gtaagcgcggtcggaagaagctgctgtacatctttaagcaacccttcatgaggcctgtgcagactac tcaagaggaggacggctgttcatgccggttcccagaggaggaggaaggcggctgcgaactgcgc gtgaaattcagccgcagcgcagatgctccagcctacaagcaggggcagaaccagctctacaacg aactcaatcttggtcggagagaggagtacgacgtgctggacaagcggagaggacgggacccag aaatgggcgggaagccgcgcagaaagaatccccaagagggcctgtacaacgagctccaaaag gataagatggcagaagcctatagcgagattggtatgaaaggggaacgcagaagaggcaaagg ccacgacggactgtaccagggactcagcaccgccaccaaggacacctatgacgctcttcacatgc aggccctgccgcctcgg |
| **CAR 9** - Full - aa | 786 | malpvtalllplalllhaarpeiqlqqsgaelvkpgasvklsctgsgfniedyyihwvkqrteqglewig ridpendetkygpifqgratitadtssntvylqlssltsedtavyycafrggvywgpgttltvssgggg sggggsggggshmdvvmtqspltlsvaigqsasisckssqslldsdgktylnwllqrpgqspkrli slvskldsgvpdrftgsgsgtdftlrisrveaedlgiyycwqgthfpqtfgggtkleikttpaprpptpa ptiasqplslrpeacrpaaggavhtrgldfacdiyiwaplagtcgvlllslvitlyckrgrkkllyifkqpfmr pvqttqeedgcscrfpeeeeggcelrvkfsrsadapaykqgqnqlynelnlgrreeydvldkrrgrd pemggkprrknpqeglynelqkdkmaeayseigmkgerrrgkghdglyqglstatkdtydalhm qalppr |

| **CAR10** | | Anti-EGFRvIII clone 139 |
|---|---|---|
| CAR 10 Full - nt | 787 | atggccctccctgtcaccgccctgctgcttccgctggctcttctgctccacgccgctcggcccgatatcc aaatgactcagagcccttcatccctgagcgccagcgtcggagacagggtgaccatcacgtgccgg gcatcccaaggcattagaaataacttggcgtggtatcagcaaaaaccaggaaaggccccgaagc gcctgatctacgcggcctccaaccttcagtcaggagtgccctcgcgcttcaccgggagcggtagcg gaactgagtttacccttatcgtgtcgtccctgcagccagaggacttcgcgacctactactgcctccagc atcactcgtacccgttgacttcgggaggcggaaccaaggtcgaaatcaaacgcactggctcgacgt cagggtccggtaaaccgggatcgggagaaggatcggaagtccaagtgctggagagcggaggc ggactcgtgcaacctggcgggtcgctgcggctcagctgtgccgcgtcgggttttactttcagctcgtac |
| | | gctatgtcatgggtgcggcaggctccgggaaaggggctggaatgggtgtccgctatttccggctcgg gtggaagcaccaattacgccgactccgtgaagggacgcttcaccatctcacgggataactccaag aatactctgtacctccagatgaactcgctgagagccgaggacaccgcagtgtactactgcgcaggg tcaagcggctggtccgaatactggggacagggcaccctcgtcactgtcagctccaccactacccca gcaccgaggccacccaccccggctcctaccatcgcctcccagcctctgtccctgcgtccggaggca tgtagacccgcagctggtggggccgtgcatacccggggtcttgacttcgcctgcgatatctacatttgg gcccctctggctggtacttgcggggtcctgctgctttcactcgtgatcactctttactgtaagcgcggtcg gaagaagctgctgtacatctttaagcaacccttcatgaggcctgtgcagactactcaagaggagga cggctgttcatgccggttcccagaggaggaggaaggcggctgcgaactgcgcgtgaaattcagcc gcagcgcagatgctccagcctacaagcaggggcagaaccagctctacaacgaactcaatcttggt cggagagaggagtacgacgtgctggacaagcggagaggacgggacccagaaatgggcggga agccgcgcagaaagaatccccaagagggcctgtacaacgagctccaaaaggataagatggca gaagcctatagcgagattggtatgaaaggggaacgcagaagaggcaaaggccacgacggact gtaccagggactcagcaccgccaccaaggacacctatgacgctcttcacatgcaggccctgccgc ctcgg |
| CAR 10 Full - aa | 788 | malpvtalllplalllhaarpdiqmtqspsslsasvgdrvtitcrasqgirnnlawyqqkpgkapkrliya asnlqsgvpsrftgsgsgteftlivsslqpedfatyyclqhhsypltsgggtkveikrtgstsgsgkpgsg egsevqvlesggglvqpggslrlscaasgftfssyamswvrqapgkglewvsaisgsggstnyad svkgrftisrdnskntlylqmnslraedtavyycagssgwseywgqgtlvtvsstttpaprpptpapti asqplslrpeacrpaaggavhtrgldfacdiyiwaplagtcgvlllslvitlyckrgrkkllyifkqpfmrpv qttqeedgcscrfpeeeeggcelrvkfsrsadapaykqgqnqlynelnlgrreeydvldkrrgrdpe mggkprrknpqeglynelqkdkmaeayseigmkgerrrgkghdglyqglstatkdtydalhmqal ppr |

In one embodiment, a CAR molecule that binds EGFRvIII comprises (e.g., consists of) an amino acid sequence as provided in Table 18. In one embodiment, the CAR that binds EGFRvIII comprises (e.g., consists of) an amino acid sequence selected from any one of SEQ ID NOs: 770, 772, 774, 776, 778, 780, 782, 784, 786, 788; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence selected from any one of SEQ ID NOs: 770, 772, 774, 776, 778, 780, 782, 784, 786, 788; or an amino acid sequence having 85%, 90%, 95%, 96%, 97%, 98%, 99% identity to an amino acid sequence selected from any one of SEQ ID NOs: 770, 772, 774, 776, 778, 780, 782, 784, 786, 788.

In one embodiment, a CAR molecule comprises a CD123 antigen binding domain (e.g., a murine, human or humanized antibody or antibody fragment that specifically binds to CD123), a transmembrane domain, and an intracellular signaling domain (e.g., an intracellular signaling domain comprising a costimulatory domain and/or a primary signaling domain). Exemplary CAR molecules that target CD123 include those provided in Table 19, and those provided in Tables 2, 6 and 9 of WO2016/028896. Other exemplary CAR molecules that target CD123 are described in WO/2014/130635 (e.g., Table 1 of WO/2014/130635). Other exemplary CAR molecules that target CD123 are described in WO/2014/144622.

In one embodiment, a CAR molecule that binds CD123 comprises (e.g., consists of) an amino acid sequence as provided in Table 19. In one embodiment, the CAR that binds CD123 comprises (e.g., consists of) an amino acid sequence selected from any one of SEQ ID NOs: 750, 755, 760, 765; or an amino acid sequence having at least one, two, three, four, five, 10, 15, 20 or 30 modifications (e.g., substitutions, e.g., conservative substitutions) but not more than 60, 50, or 40 modifications (e.g., substitutions, e.g., conservative substitutions) of an amino acid sequence selected from any one of SEQ ID NOs: 750, 755, 760, 765; or an amino acid sequence having 85%, 90%, 95%, 96%, 97%, 98%, 99% identity to an amino acid sequence selected from any one of SEQ ID NOs: 750, 755, 760, 765.

**Table 19. Exemplary CAR sequences**

| **Name** | **SEQ ID** | **Sequence** |
|---|---|---|
| **CAR123-2 NT** | 749 | atggccctccctgtcaccgccctgctgcttccgctggctcttctgctccacgccgctcggccccaagtgcaa ctcgtccaaagcggagcggaagtcaagaaacccggagcgagcgtgaaagtgtcctgcaaagcctccg gctacacctttacgggctactacatgcactgggtgcgccaggcaccaggacagggtcttgaatggatgg gatggatcaaccctaattcgggcggaactaactacgcacagaagttccaggggagagtgactctgactc gggatacctccatctcaactgtctacatggaactctcccgcttgcggtcagatgatacggcagtgtactact gcgcccgcgacatgaatatcctggctaccgtgccgttcgacatctggggacaggggactatggttactgtc tcatcgggcggtggaggttcaggaggaggcggctcgggaggcggaggttcggacattcagatgaccca gtccccatcctctctgtcggccagcgtcggagatagggtgaccattacctgtcgggcctcgcaaagcatct cctcgtacctcaactggtatcagcaaaagccgggaaaggcgcctaagctgctgatctacgccgcttcga gcttgcaaagcggggtgccatccagattctcgggatcaggctcaggaaccgacttcaccctgaccgtga acagcctccagccggaggactttgccacttactactgccagcagggagactccgtgccgcttactttcggg gggggtacccgcctggagatcaagaccactaccccagcaccgaggccacccaccccggctcctacca tcgcctcccagcctctgtccctgcgtccggaggcatgtagacccgcagctggtggggccgtgcatacccg gggtcttgacttcgcctgcgatatctacatttgggcccctctggctggtacttgcggggtcctgctgctttcactc gtgatcactctttactgtaagcgcggtcggaagaagctgctgtacatctttaagcaacccttcatgaggcct gtgcagactactcaagaggaggacggctgttcatgccggttcccagaggaggaggaaggcggctgcg aactgcgcgtgaaattcagccgcagcgcagatgctccagcctacaagcaggggcagaaccagctcta caacgaactcaatcttggtcggagagaggagtacgacgtgctggacaagcggagaggacgggaccc agaaatgggcgggaagccgcgcagaaagaatccccaagagggcctgtacaacgagctccaaaagg ataagatggcagaagcctatagcgagattggtatgaaaggggaacgcagaagaggcaaaggccacg acggactgtaccagggactcagcaccgccaccaaggacacctatgacgctcttcacatgcaggccctg ccgcctcgg |
| **CAR123-2 AA** | 750 | malpvtalllplalllhaarpqvqlvqsgaevkkpgasvkvsckasgytftgyymhwvrqapgqglewm gwinpnsggtnyaqkfqgrvtltrdtsistvymelsrlrsddtavyycardmnilatvpfdiwgqgtmvtvss ggggsggggsggggsdiqmtqspsslsasvgdrvtitcrasqsissylnwyqqkpgkapklliyaasslq sgvpsrfsgsgsgtdftltvnslqpedfatyycqqgdsvpltfgggtrleikttttpaprpptpaptiasqplslrp eacrpaaggavhtrgldfacdiyiwaplagtcgvlllslvitlyckrgrkkllyifkqpfmrpvqttqeedgcscrf peeeeggcelrvkfsrsadapaykqgqnqlynelnlgrreeydvldkrrgrdpemggkprrknpqegly nelqkdkmaeayseigmkgerrrgkghdglyqglstatkdtydalhmqalppr |
| **CAR123-2 scFv** | 751 | malpvtalllplalllhaarpqvqlvqsgaevkkpgasvkvsckasgytftgyymhwvrqapgqglewm gwinpnsggtnyaqkfqgrvtltrdtsistvymelsrlrsddtavyycardmnilatvpfdiwgqgtmvtvss ggggsggggsggggsdiqmtqspsslsasvgdrvtitcrasqsissylnwyqqkpgkapklliyaasslq sgvpsrfsgsgsgtdftltvnslqpedfatyycqqgdsvpltfgggtrleik |
| **CAR123-2 VH** | 752 | QVQLVQSGAEVKKPGASVKVSCKASGYTFTGYYMHWVRQAPGQGLEWM GWINPNSGGTNYAQKFQGRVTLTRDTSISTVYMELSRLRSDDTAVYYCAR DMNILATVPFDIWGQGTMVTVSS |
| **CAR123-2 VL** | 753 | DIQMTQSPSSLSASVGDRVTITCRASQSISSYLNWYQQKPGKAPKLLIYAA SSLQSGVPSRFSGSGSGTDFTLTVNSLQPEDFATYYCQQGDSVPLTFGG GTRLEIK |
| **CAR123-3 NT** | 754 | atggccctccctgtcaccgccctgctgcttccgctggctcttctgctccacgccgctcggccccaagtccaa ctcgttcaatccggcgcagaagtcaagaagccaggagcatcagtgaaagtgtcctgcaaagcctcagg ctacatcttcacgggatactacatccactgggtgcgccaggctccgggccagggccttgagtggatgggc tggatcaaccctaactctgggggaaccaactacgctcagaagttccaggggagggtcactatgactcgc gatacctccatctccactgcgtacatggaactctcgggactgagatccgacgatcctgccgtgtactactgc |
| | | gcccgggacatgaacatcttggcgaccgtgccgtttgacatttggggacagggcaccctcgtcactgtgtc gagcggtggaggaggctcggggggtggcggatcaggagggggaggaagcgacatccagctgactc agagcccatcgtcgttgtccgcgtcggtgggggatagagtgaccattacttgccgcgccagccagagcat ctcatcatatctgaattggtaccagcagaagcccggaaaggccccaaaactgctgatctacgctgcaag cagcctccaatcgggagtgccgtcacggttctccgggtccggttcgggaactgactttaccctgaccgtga attcgctgcaaccggaggatttcgccacgtactactgtcagcaaggagactccgtgccgctgaccttcggt ggaggcaccaaggtcgaaatcaagaccactaccccagcaccgaggccacccaccccggctcctacc atcgcctcccagcctctgtccctgcgtccggaggcatgtagacccgcagctggtggggccgtgcataccc ggggtcttgacttcgcctgcgatatctacatttgggcccctctggctggtacttgcggggtcctgctgctttcac tcgtgatcactctttactgtaagcgcggtcggaagaagctgctgtacatctttaagcaacccttcatgaggcc tgtgcagactactcaagaggaggacggctgttcatgccggttcccagaggaggaggaaggcggctgcg aactgcgcgtgaaattcagccgcagcgcagatgctccagcctacaagcaggggcagaaccagctcta caacgaactcaatcttggtcggagagaggagtacgacgtgctggacaagcggagaggacgggaccc agaaatgggcgggaagccgcgcagaaagaatccccaagagggcctgtacaacgagctccaaaagg ataagatggcagaagcctatagcgagattggtatgaaaggggaacgcagaagaggcaaaggccacg acggactgtaccagggactcagcaccgccaccaaggacacctatgacgctcttcacatgcaggccctg ccgcctcgg |
| **CAR123-3 AA** | 755 | malpvtalllplalllhaarpqvqlvqsgaevkkpgasvkvsckasgyiftgyyihwvrqapgqglewmg winpnsggtnyaqkfqgrvtmtrdtsistaymelsglrsddpavyycardmnilatvpfdiwgqgtlvtvss ggggsggggsggggsdiqltqspsslsasvgdrvtitcrasqsissylnwyqqkpgkapklliyaasslqs gvpsrfsgsgsgtdftltvnslqpedfatyycqqgdsvpltfgggtkveikttpaprpptpaptiasqplslrp eacrpaaggavhtrgldfacdiyiwaplagtcgvlllslvitlyckrgrkkllyifkqpfmrpvqttqeedgcscrf peeeeggcelrvkfsrsadapaykqgqnqlynelnlgrreeydvldkrrgrdpemggkprrknpqegly nelqkdkmaeayseigmkgerrrgkghdglyqglstatkdtydalhmqalppr |
| **CAR123-3 scFv** | 756 | malpvtalllplalllhaarpqvqlvqsgaevkkpgasvkvsckasgyiftgyyihwvrqapgqglewmg winpnsggtnyaqkfqgrvtmtrdtsistaymelsglrsddpavyycardmnilatvpfdiwgqgtlvtvss ggggsggggsggggsdiqltqspsslsasvgdrvtitcrasqsissylnwyqqkpgkapklliyaasslqs gvpsrfsgsgsgtdftltvnslqpedfatyycqqgdsvpltfgggtkveik |
| **CAR123-3 VH** | 757 | QVQLVQSGAEVKKPGASVKVSCKASGYIFTGYYIHWVRQAPGQGLEWMG WINPNSGGTNYAQKFQGRVTMTRDTSISTAYMELSGLRSDDPAVYYCARD MNILATVPFDIWGQGTLVTVSS |
| **CAR123-3 VL** | 758 | DIQLTQSPSSLSASVGDRVTITCRASQSISSYLNWYQQKPGKAPKLLIYAAS SLQSGVPSRFSGSGSGTDFTLTVNSLQPEDFATYYCQQGDSVPLTFGGGT KVEIK |
| **CAR123-4 NT** | 759 | atggccctccctgtcaccgccctgctgcttccgctggctcttctgctccacgccgctcggccccaagtccaa ctccaacagtcaggcgcagaagtgaaaaagagcggtgcatcggtgaaagtgtcatgcaaagcctcgg gctacaccttcactgactactatatgcactggctgcggcaggcaccgggacagggacttgagtggatgg gatggatcaacccgaattcaggggacactaactacgcgcagaagttccaggggagagtgaccctgac gagggacacctcaatttcgaccgtctacatggaattgtcgcgcctgagatcggacgatactgctgtgtact actgtgcccgcgacatgaacatcctcgcgactgtgccttttgatatctggggacaggggactatggtcacc gtttcctccgcttccggtggcggaggctcgggaggccgggcctccggtggaggaggcagcgacatcca gatgactcagagcccttcctcgctgagcgcctcagtgggagatcgcgtgaccatcacttgccgggccagc cagtccatttcgtcctacctcaattggtaccagcagaagccgggaaaggcgcccaagctcttgatctacg ctgcgagctccctgcaaagcggggtgccgagccgattctcgggttccggctcgggaaccgacttcactct gaccatctcatccctgcaaccagaggactttgccacctactactgccaacaaggagattctgtcccactga cgttcggcggaggaaccaaggtcgaaatcaagaccactaccccagcaccgaggccacccaccccgg ctcctaccatcgcctcccagcctctgtccctgcgtccggaggcatgtagacccgcagctggtggggccgtg catacccggggtcttgacttcgcctgcgatatctacatttgggcccctctggctggtacttgcggggtcctgct gctttcactcgtgatcactctttactgtaagcgcggtcggaagaagctgctgtacatctttaagcaacccttca tgaggcctgtgcagactactcaagaggaggacggctgttcatgccggttcccagaggaggaggaaggc ggctgcgaactgcgcgtgaaattcagccgcagcgcagatgctccagcctacaagcaggggcagaacc agctctacaacgaactcaatcttggtcggagagaggagtacgacgtgctggacaagcggagaggacg ggacccagaaatgggcgggaagccgcgcagaaagaatccccaagagggcctgtacaacgagctcc aaaaggataagatggcagaagcctatagcgagattggtatgaaaggggaacgcagaagaggcaaa ggccacgacggactgtaccagggactcagcaccgccaccaaggacacctatgacgctcttcacatgca ggccctgccgcctcgg |
| **CAR123-4 AA** | 760 | malpvtalllplalllhaarpqvqlqqsgaevkksgasvkvsckasgytftdyymhwlrqapgqglewmg winpnsgdtnyaqkfqgrvtltrdtsistvymelsrlrsddtavyycardmnilatvpfdiwgqgtmvtvssa sggggsggrasggggsdiqmtqspsslsasvgdrvtitcrasqsissylnwyqqkpgkapklliyaassl qsgvpsrfsgsgsgtdftltisslqpedfatyycqqgdsvpltfgggtkveiktttpaprpptpaptiasqplslr peacrpaaggavhtrgldfacdiyiwaplagtcgvlllslvitlyck |
| **CAR123-4 scFv** | 761 | MALPVTALLLPLALLLHAARPQVQLQQSGAEVKKSGASVKVSCKASGYTFT DYYMHWLRQAPGQGLEWMGWINPNSGDTNYAQKFQGRVTLTRDTSISTV YMELSRLRSDDTAVYYCARDMNILATVPFDIWGQGTMVTVSSASGGGGS GGRASGGGGSDIQMTQSPSSLSASVGDRVTITCRASQSISSYLNWYQQKP GKAPKLLIYAASSLQSGVPSRFSGSGSGTDFTLTISSLQPEDFATYYCQQG DSVPLTFGGGTKVEIK |
| **CAR123-4 VH** | 762 | QVQLQQSGAEVKKSGASVKVSCKASGYTFTDYYMHWLRQAPGQGLEWM GWINPNSGDTNYAQKFQGRVTLTRDTSISTVYMELSRLRSDDTAVYYCAR DMNILATVPFDIWGQGTMVTVSS |
| **CAR123-4 VL** | 763 | DIQMTQSPSSLSASVGDRVTITCRASQSISSYLNWYQQKPGKAPKLLIYAA SSLQSGVPSRFSGSGSGTDFTLTISSLQPEDFATYYCQQGDSVPLTFGGG TKVEIK |
| **CAR123-1 NT** | 764 | atggccctccctgtcaccgccctgctgcttccgctggctcttctgctccacgccgctcggccccaagtccaa ctcgtccagtcaggagcggaagtcaagaagcccggagcgtcagtcaaagtgtcatgcaaagcctcgg gctacactttcactgggtactacatgcactgggtgcgccaggctccaggacagggactggaatggatgg gatggatcaacccgaactccggtggcaccaattacgcccagaagttccaggggagggtgaccatgact cgcgacacgtcgatcagcaccgcatacatggagctgtcaagactccggtccgacgatactgccgtgtac tactgcgcacgggacatgaacattctggccaccgtgccttttgacatctggggtcagggaactatggttacc gtgtcctctggtggaggcggctccggcggggggggaagcggaggcggtggaagcgacattcagatga cccagtcgccttcatccctttcggcgagcgtgggagatcgcgtcactatcacttgtcgggcctcgcagtcca tctccacctacctcaattggtaccagcagaagccaggaaaagcaccgaatctgctgatctacgccgcgtt ttccttgcaatcgggagtgccaagcagattcagcggatcgggatcaggcactgatttcaccctcaccatca actcgctgcaaccggaggatttcgctacgtactattgccaacaaggagacagcgtgccgctcaccttcgg cggagggactaagctggaaatcaagaccactaccccagcaccgaggccacccaccccggctcctac catcgcctcccagcctctgtccctgcgtccggaggcatgtagacccgcagctggtggggccgtgcatacc cggggtcttgacttcgcctgcgatatctacatttgggcccctctggctggtacttgcggggtcctgctgctttca ctcgtgatcactctttactgtaagcgcggtcggaagaagctgctgtacatctttaagcaacccttcatgaggc ctgtgcagactactcaagaggaggacggctgttcatgccggttcccagaggaggaggaaggcggctgc gaactgcgcgtgaaattcagccgcagcgcagatgctccagcctacaagcaggggcagaaccagctct acaacgaactcaatcttggtcggagagaggagtacgacgtgctggacaagcggagaggacgggacc cagaaatgggcgggaagccgcgcagaaagaatccccaagagggcctgtacaacgagctccaaaag gataagatggcagaagcctatagcgagattggtatgaaaggggaacgcagaagaggcaaaggccac gacggactgtaccagggactcagcaccgccaccaaggacacctatgacgctcttcacatgcaggccct gccgcctcgg |
| **CAR123-1 AA** | 765 | malpvtalllplalllhaarpqvqlvqsgaevkkpgasvkvsckasgytftgyymhwvrqapgqglewm gwinpnsggtnyaqkfqgrvtmtrdtsistaymelsrlrsddtavyycardmnilatvpfdiwgqgtmvtvs sggggsggggsggggsdiqmtqspsslsasvgdrvtitcrasqsistylnwyqqkpgkapnlliyaafslq sgvpsrfsgsgsgtdftltinslqpedfatyycqqgdsvpltfgggtkleikttpaprpptpaptiasqplslrp eacrpaaggavhtrgldfacdiyiwaplagtcgvlllslvitlyckrgrkkllyifkqpfmrpvqttqeedgcscrf peeeeggcelrvkfsrsadapaykqgqnqlynelnlgrreeydvldkrrgrdpemggkprrknpqegly nelqkdkmaeayseigmkgerrrgkghdglyqglstatkdtydalhmqalppr |
| **CAR123-1 scFv** | 766 | malpvtalllplalllhaarpqvqlvqsgaevkkpgasvkvsckasgytftgyymhwvrqapgqglewm gwinpnsggtnyaqkfqgrvtmtrdtsistaymelsrlrsddtavyycardmnilatvpfdiwgqgtmvtvs sggggsggggsggggsdiqmtqspsslsasvgdrvtitcrasqsistylnwyqqkpgkapnllliyaafslq sgvpsrfsgsgsgtdftltinslqpedfatyycqqgdsvpltfgggtkleik |
| **CAR123-1 VH** | 767 | QVQLVQSGAEVKKPGASVKVSCKASGYTFTGYYMHWVRQAPGQGLEWM GWINPNSGGTNYAQKFQGRVTMTRDTSISTAYMELSRLRSDDTAVYYCAR DMNILATVPFDIWGQGTMVTVSS |
| **CAR123-1 VL** | 768 | DIQMTQSPSSLSASVGDRVTITCRASQSISTYLNWYQQKPGKAPNLLIYAAF SLQSGVPSRFSGSGSGTDFTLTINSLQPEDFATYYCQQGDSVPLTFGGGT |
| | | KLEIK |

In one embodiment, the CAR molecule comprises a CD33 antigen binding domain (e.g., a murine, human or humanized antibody or antibody fragment that specifically binds to CD33), a transmembrane domain, and an intracellular signaling domain (e.g., an intracellular signaling domain comprising a costimulatory domain and/or a primary signaling domain). Exemplary CAR molecules that target CD33 are described herein, and are provided in WO2016/014576, e.g., in Table 2 of WO2016/014576.

In one embodiment, the CAR molecule comprises a CLL-1 antigen binding domain (e.g., a murine, human or humanized antibody or antibody fragment that specifically binds to CLL-1), a transmembrane domain, and an intracellular signaling domain (e.g., an intracellular signaling domain comprising a costimulatory domain and/or a primary signaling domain). Exemplary CAR molecules that target CLL-1 are described herein, and are provided in WO/2016/014535, e.g., in Table 2 of WO2016/014535.

### Natural Killer Cell Receptor (NKR) CARs

In an embodiment, the CAR molecule described herein comprises one or more components of a natural killer cell receptor (NKR). The NKR component can be a transmembrane domain, a hinge domain, or a cytoplasmic domain from any of the following natural killer cell receptors: killer cell immunoglobulin-like receptor (KIR), e.g., KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1; natural cyotoxicity receptor (NCR), e.g., NKp30, NKp44, NKp46; signaling lymphocyte activation molecule (SLAM) family of immune cell receptors, e.g., CD48, CD229, 2B4, CD84, NTB-A, CRACC, BLAME, and CD2F-10; Fc receptor (FcR), e.g., CD16, and CD64; and Ly49 receptors, e.g., LY49A, LY49C. The CAR molecules comprising components of NKRs described herein may interact with an adaptor molecule or intracellular signaling domain, e.g., DAP12. Exemplary configurations and sequences of CAR molecules comprising NKR components are described in International Publication No. WO2014/145252.

### Split CAR

In some embodiments, a CAR-expressing cell of the invention uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41 BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens. In embodiments the first antigen binding domain recognizes an antigen described herein, e.g., comprises an antigen binding domain described herein, and the second antigen binding domain recognizes a second antigen described herein.

### Exemplary characteristics of the temporally expressed CARs of the invention

In some embodiments, it is beneficial that the antigen binding domain is operably linked to another domain of the CAR, such as the transmembrane domain or the intracellular domain, both described elsewhere herein, for expression in the cell. In one embodiment, a nucleic acid encoding the antigen binding domain is operably linked to a nucleic acid encoding a transmembrane domain and a nucleic acid encoding an intracellular domain.

Between the antigen binding domain and the transmembrane domain of the CAR, or between the intracellular domain and the transmembrane domain of the CAR, a spacer domain may be incorporated. As used herein, the term "spacer domain" generally means any oligo- or polypeptide that functions to link the transmembrane domain to, either the antigen binding domain or, the intracellular domain in the polypeptide chain. In one embodiment, the spacer domain may comprise up to 300 amino acids, preferably 10 to 100 amino acids and most preferably 25 to 50 amino acids. In another embodiment, a short oligo- or polypeptide linker, preferably between 2 and 10 amino acids in length may form the linkage between the transmembrane domain and the intracellular domain of the CAR. An example of a linker includes a glycine-serine doublet.

In some embodiments, the CAR further comprises a signal peptide. In one embodiment, the signal peptide comprises a nucleic acid sequence comprising
ATGGCCTTACCAGTGACCGCCTTGCTCCTGCCGCTGGCCTTGCTGCTCCACGCCGCCAGGCCG or SEQ ID NO: 3034. In another embodiment, the signal peptide comprises the amino acid sequence comprising MALPVTALLLPLALLLHAARP or SEQ ID NO: 3035.

In some instances disclosed herein which are not part of the claimed invention, the CAR further comprises a dimerization domain, such a dimerization domain from FKBP or similar molecule. In such an instance, the presence of CAR molecules on the surface of the modified T cell is prevented by spontaneous aggregation of the CAR molecules in the cytoplasm or other internal location in the cell.

In yet another instance, the dimerization domain comprises the nucleic acid sequence comprising

CGGGGCGTGCAGGTTGAGACAATTTCCCCAGGAGATGGGCGAACGTTCCCCAAGCGCGGACAGAC
ATGCGTTGTGCACTACACAGGAATGTTGGAGGACGGAAAGAAAATGGACAGTTCAAGAGATCGGAA
CAAACCATTCAAATTCATGTTGGGAAAACAGGAAGTGATACGGGGCTGGGAAGAGGGTGTAGCGCA
AATGTCCGTTGGTCAACGAGCAAAACTCACGATAAGTCCCGATTATGCTTACGGCGCAACCGGTCAC
CCGGGCATCATACCGCCTCATGCGACTTTGGTCTTTGATGTGGAGCTGTTGAAACTTGAAACTCGCG
GAGTACAGGTTGAAACAATATCACCCGGGGACGGGCGGACTTTTCCGAAGAGAGGTCAGACCTGCG

TCGTCCATTATACCGGTATGCTGGAGGACGGAAAGAAAATGGACAGCTCACGGGACCGAAATAAAC
CATTCAAATTTATGTTGGGGAAACAAGAGGTTATCAGGGGCTGGGAGGAGGGTGTGGCCCAGATGT
CTGTCGGTCAGCGCGCGAAACTCACAATCTCTCCGGATTATGCGTATGGGGCGACAGGGCATCCGG
GAATTATCCCTCCCCACGCTACCTTGGTTTTCGATGTTGAGCTTCTGAAGTTGGAGACCAGAGGAGT
TCAAGTGGAGACAATATCTCCTGGGGATGGACGGACGTTCCCCAAGCGCGGCCAGACCTGTGTAGT
CCACTACACAGGGATGCTTGAAGACGGAAAAAAGATGGATAGCAGTAGAGATCGCAACAAACCATTT
AAGTTCATGCTGGGGAAGCAGGAAGTAATACGCGGCTGGGAGGAAGGCGTGGCACAGATGAGTGT
TGGTCAACGGGCCAAACTTACTATTTCTCCCGATTATGCGTATGGAGCCACCGGGCACCCTGGCATT
ATCCCACCCCATGCCACATTGGTTTTTGACGTTGAATTGCTTAAATTGGAGACCAGGGGAGTCCAAG
TGGAAACAATATCACCGGGGGATGGTCGGACTTTTCCTAAAAGGGGCCAAACCTGTGTAGTCCATTA
TACCGGAATGCTCGAAGACGGAAAGAAAATGGACTCTTCTAGAGACCGCAATAAGCCCTTCAAGTTC
ATGTTGGGTAAGCAAGAGGTGATCCGGGGCTGGGAAGAGGGGGTCGCTCAAATGTCCGTCGGTCA
GCGAGCTAAACTGACTATTTCCCCAGACTACGCATATGGAGCGACTGGCCACCCCGGTATTATTCCT
CCCCATGCGACTCTCGTGTTCGACGTAGAACTCTTGAAATTGGAAACG or SEQ ID NO: 3015.

In another such instance, the dimerization domain comprises the amino acid sequence comprising RGVQVETISPGDGRTFPKRGQTCVVHYTGMLEDGKKMDSSRDRNKPFKFMLGKQEVIRGWEEGVAQM SVGQRAKLTISPDYAYGATGHPGIIPPHATLVFDVELLKLETRGVQVETISPGDGRTFPKRGQTCVVHYTG MLEDGKKMDSSRDRNKPFKFMLGKQEVIRGWEEGVAQMSVGQRAKLTISPDYAYGATGHPGIIPPHATL VFDVELLKLETRGVQVETISPGDGRTFPKRGQTCVVHYTGMLEDGKKMDSSRDRNKPFKFMLGKQEVIR GWEEGVAQMSVGQRAKLTISPDYAYGATGHPGIIPPHATLVFDVELLKLETRGVQVETISPGDGRTFPKR GQTCVVHYTGMLEDGKKMDSSRDRNKPFKFMLGKQEVIRGWEEGVAQMSVGQRAKLTISPDYAYGAT GHPGIIPPHATLVFDVELLKLET or SEQ ID NO: 980 (described elsewhere herein).

Solubilization of the dimerization domains with a solubilizing agent, administered to the cell or to a mammal comprising the cell, prevents dimerization and allows the construct to egress through the secretory system, where it is processed via furin cleavage to express a functional cell surface CAR.

In the presence of a solubilizing agent, the CAR molecules are separated and aggregation is prevented. Administration of a solubilizing agent would prevent dimerization or aggregation of the CAR molecules and allow the CAR molecules to be presented on the surface of the T cell. In one instance, the furin cleavage site comprises the nucleic acid sequence comprising TCAGCCCGGAACAGGCGGAAGAGA or SEQ ID NO:3016. In another instance, the furin cleavage site comprises the amino acid sequence SARNRRKR or SEQ ID NO: 3017.

### Composition

Also disclosed herein but not forming part of the claimed invention is an isolated nucleic acid sequence comprising a nucleic acid sequence comprising a suicide gene comprising a nucleic acid sequence selected from the group consisting of SEQ ID NOs: 3001-3004; and a nucleic acid sequence encoding a CAR comprising an anti-B cell binding domain, a transmembrane domain, a costimulatory domain and an intracellular signaling domain. In some instances of the disclosure, the isolated nucleic acid sequence comprises SEQ ID NO: 3018, 3020, 3024, 3026, 3028 or 3030.

In one instance, the disclosure includes an isolated polypeptide comprising an amino acid sequence encoded by a suicide gene wherein the amino acid sequence is selected from the group consisting of SEQ ID NOs: 3005-3007; and a CAR comprising an anti-B cell binding domain, a transmembrane domain, a costimulatory domain and an intracellular signaling domain. In some instances, the isolated polypeptide comprises an amino acid sequence of SEQ ID NO: 3019, 3021, 3026, 3028, 3030 or 3034.

In another instance, the disclosure includes an isolated nucleic acid sequence comprising a nucleic acid encoding a dimerization domain; and a CAR comprising an anti-B cell binding domain, a transmembrane domain, a costimulatory domain and an intracellular signaling domain. In some instances, the dimerization domain comprises the amino acid sequence of SEQ ID NO: 980. In other instances, the isolated nucleic acid sequence comprises SEQ ID NO: 977 or 3032.

In yet another instance, the disclosure includes an isolated polypeptide comprising a dimerization domain; and a CAR comprising an anti-B cell binding domain, a transmembrane domain, a costimulatory domain and an intracellular signaling domain. In some instances, the isolated polypeptide comprises an amino acid sequence of SEQ ID NO: 978 or 3033.

### SIGNAL PEPTIDE

In certain preferred embodiments, the fusion proteins of the invention further include a signal peptide. Signal peptides are useful if it is desirable to have the protein follow the secretory pathway. In preferred embodiments, this signal peptide will be engineered to be present at the very N-terminus of the fusion protein. Exemplary signal peptides are set forth below:
CD8: MALPVTALLLPLALLLHAARP (SEQ ID NO: 2)
GMCSFR: MLLLVTSLLLCELPHPAFLLIP (SEQ ID NO: 43)
IL2: MYRMQLLSCIALSLALVTNS (SEQ ID NO: 44)
IgK chain: MAQVKLQESGTELAKPGAAVK (SEQ ID NO: 45)
NPC2: MRFLAATFLLLALSTAAQA (SEQ ID NO: 46)
LAMB1: MGLLQLLAFSFLALCRARVRA (SEQ ID NO: 1114)
P3IP1: MLLAWVQAFLVSNMLLAEAYG (SEQ ID NO: 1115)
DMKN: MKFQGPLACLLLALCLGSGEA (SEQ ID NO: 1116)
TPA: MDAMKRGLCCVLLLCGAVFVSP (SEQ ID NO: 1117)
PCSK9: MGTVSSRRSWWPLPLLLLLLLLLGPAGARAQEDED (SEQ ID NO: 1118)

KDEL (SEQ ID NO: 47) or KKXX and derivatives at the very C-terminus of the protein of interest can be engineered if the protein of interest is an ER-resident protein. These sequences must be inserted together with the signal peptide.

Proteins of interest can be engineered to include glycosylation patterns for internalization via mannose-6-phosphate receptor and targeting to the endosomal/ lysosomal system. These should be included in the protein of interest itself, if this is a protein resident in that compartment. Consensus for N-glycosylation is Asn-X-Ser/Thr, where X is any aminoacid except proline (Pro), serine (Ser), and threonine (Thr).

In embodiments where it is desirable to have proteins of interested targeted at the peroxisome, the fusion protein can be engineered to include a C-terminal peroxisomal targeting signal (e.g., PTS1: -SKL).

### CLEAVAGE SITES

Each fusion protein of the invention includes a cleavage site. The cleavage site can either be selfcleavage sites and/or protease cleavage sites. The cleavage site can be designed to be cleaved by any site-specific protease that is expressed in a cell of interest (either through recombinant expression or endogenous expression) at adequate levels to cleave off the conditional expression domain, e.g., a degradation domain or an aggregation domain. In important aspects of the invention, the protease cleavage site is chosen to correspond to a protease natively (or by virtue of cell engineering) to be present in a cellular compartment relevant to the expression of the protein of interest. I.e., the intracellular trafficking of the protease should overlap or partially overlap with the intracellular trafficking of the protein of interest that contains the conditional expression domain, e.g., a degradation domain or an aggregation domain employed. For example, if the protein of interest is located at the cell surface, the enzyme to cleave it can be added exogenous to the cell.

If the protein of interest resides in the endosomal/lysosomal system a protease cleavage site for an enzyme resident in those compartments can be used. Such protease/consensus motifs include, e.g.,
Furin: RX(K/R)R consensus motif
PCSK1: RX(K/R)R consensus motif
PCSK5: RX(K/R)R consensus motif
PCSK6: RX(K/R)R consensus motif
PCSK7: RXXX[KR]R consensus motif
Cathepsin B : RRX
Granzyme B : I-E-P-D-X (SEQ ID NO: 35)
Factor XA: lie - Glu/Asp-Gly-Arg
Enterokinase: Asp-Asp-Asp-Asp-Lys (SEQ ID NO: 36)
Genenase: Pro-Gly-Ala-Ala-His-Tyr (SEQ ID NO: 37)
Sortase: LPXTG/A
PreScission protease: Leu-Glu-Val-Phe-Gln-Gly-Pro (SEQ ID NO: 38)
Thrombin: Leu-Val-Pro-Arg-Gly-Ser (SEQ ID NO: 40)
TEV protease: E-N-L-Y-F-Q-G (SEQ ID NO: 41)
Elastase 1 [AGSV]-x (SEQ ID NO: 42)

In some embodiments, the fusion protein of the invention includes a furin cleavage site. In some embodiments, the fusion proteins of the invention include any one of furin cleavage sites listed in Table 20.

In some embodiments, the fusion proteins of the invention include a furin cleavage site selected from RTKR (SEQ ID NO: 123) or a sequence having at least 90%, 95%, 97%, 98%, or 99% identity thereto; GTGAEDPRPSRKRRSLGDVG (SEQ ID NO: 125) or a sequence having at least 90%, 95%, 97%, 98%, or 99% identity thereto; GTGAEDPRPSRKRR (SEQ ID NO: 127) or a sequence having at least 90%, 95%, 97%, 98%, or 99% identity thereto; LQWLEQQVAKRRTKR (SEQ ID NO: 129) or a sequence having at least 90%, 95%, 97%, 98%, or 99% identity thereto; GTGAEDPRPSRKRRSLGG (SEQ ID NO: 131) or a sequence having at least 90%, 95%, 97%, 98%, or 99% identity thereto; GTGAEDPRPSRKRRSLG (SEQ ID NO: 133) or a sequence having at least 90%, 95%, 97%, 98%, or 99% identity thereto; SLNLTESHNSRKKR (SEQ ID NO: 135) or a sequence having at least 90%, 95%, 97%, 98%, or 99% identity thereto; or CKINGYPKRGRKRR (SEQ ID NO: 137) or a sequence having at least 90%, 95%, 97%, 98%, or 99% identity thereto.

In some embodiments, the fusion proteins of the invention include a furin cleavage site selected from RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); or CKINGYPKRGRKRR (SEQ ID NO: 137).

In some embodiments, the fusion proteins of the invention include a furin cleavage site selected from GTGAEDPRPSRKRRSLGDVG (SEQ ID NO: 125) or a sequence having at least 90%, 95%, 97%, 98%, or 99% identity thereto, or GTGAEDPRPSRKRR (SEQ ID NO: 127) or a sequence having at least 90%, 95%, 97%, 98%, or 99% identity thereto.

In some embodiments, the fusion proteins of the invention include a furin cleavage site selected from GTGAEDPRPSRKRRSLGDVG (SEQ ID NO: 125) or GTGAEDPRPSRKRR (SEQ ID NO: 127).

In some embodiments, the fusion proteins of the invention include the furin cleavage site of GTGAEDPRPSRKRRSLGDVG (SEQ ID NO: 125).

**Table 20. Exemplary furin cleavage site**

| | **Amino acid sequence** | **Nucleic acid sequence** |
|---|---|---|
| Furin cleavage site1 | RTKR (SEQ ID NO: 123) | cgtactaaaaga (SEQ ID NO: 1112) |
| Furin cleavage site2 | GTGAEDPRPSRKRRSLGDVG (SEQ ID NO: 125) | ggaaccggcgcggaagacccccggccctccaggaag cgaaggtccctcggagacgtgggt (SEQ ID NO: 126) |
| Furin cleavage site3 | GTGAEDPRPSRKRR (SEQ ID NO: 127) | ggaaccggcgcggaagacccccggccctccaggaag cgaagg (SEQ ID NO: 128) |
| Furin cleavage site4 | LQWLEQQVAKRRTKR (SEQ ID NO: 129) | ctgcaatggctggagcagcaggtggcgaagcggagaa ctaagcgg (SEQ ID NO: 130) |
| Furin cleavage site5 | GTGAEDPRPSRKRRSLGG (SEQ ID NO: 131) | ggcacaggtgccgaggaccctcggccaagccgcaaaa ggaggtcacttggcggc (SEQ ID NO: 132) |
| Furin cleavage site6 | GTGAEDPRPSRKRRSLG (SEQ ID NO: 133) | ggaaccggagcagaagatcccagaccaagccggaaa aggcggtccctgggt (SEQ ID NO: 134) |
| Furin cleavage site7 | SLNLTESHNSRKKR (SEQ ID NO: 135) | agtctcaatttgactgagtcacacaattccaggaagaaaa gg (SEQ ID NO: 136) |
| Furin cleavage site8 | CKINGYPKRGRKRR (SEQ ID NO: 137) | tgcaagatcaacggctaccctaagaggggcagaaagc ggcgg (SEQ ID NO: 138) |

### CONDITIONAL EXPRESSION DOMAINS

The fusion protein of the disclosure can include a conditional expression domain. In some instances disclosed herein, a conditional expression domain has a first state and a second state, e.g., states of aggregation or conformational states, e.g., states of stabilization/destabilization, or states of folding/misfolding. The first state is associated with, causes, or mediates cell surface expression or extracellular expression of one or more (e.g., all) portions of the fusion protein at a first rate or level and the second state is associated with, causes, or mediates cell surface expression or extracellular expression of one or more (e.g., all) portions of the fusion protein at a second rate or level. In an instance, the second state has a level or rate that is greater, e.g., 2, 3, 4, 5, 6, 7, 8, 9, 10, 20, or 30 fold greater, than the rate or level of the first state. In an instance one of the states, e.g., the second state, is associated with, maintained by, or caused by the presence of an expression compound. In an instance, the presence of the expression compound can be associated with, cause, or mediate a change in a physical property, e.g., the second state is associated with an altered level of the physical property, e.g., presence of the expression compound is associated with, causes, or mediates the transformation of a first state with first value for a physical property to a second state with a second value for a physical property, e.g., wherein the property can comprise molecular weight, stability, absorbance at a preselected wavelength, the ability to interact with another molecule, or solubility. In an instance, the presence of the expression compound can be associated with, cause, or mediate the transformation of a first folding state to a second folding state, e.g., from misfolded to more properly folded state, e.g., a first state susceptable to degradation to a second state less susceptable to degradation than the first state; or from a first folding state that has a first level of degradation to a second folding state what has a second, lessor, level of degradation, e.g., in a cell of interest. In an instance, the presence of the expression compound can be associated with, cause, or mediate disassociation or disaggregation of a first and a second conditional expression domains, e.g., from a higher order association to a lower order association, e.g., from a dimer to monomers, or from tetramers to lower order structures, e.g., dimers or monomers. In an instance, the presence of the expression compound can be associated with, cause, or mediate a change in solubility, e.g., the second state is associated with a higher level of solubility, e.g., presence of the expression compound is associated with, causes, or mediates the transformation of a first state with lower solubility to a second state with higher solubility. In an instance, the presence of the expression compound can be associated with, cause, or mediate the transformation of a first state of localization or compartmentalization of one or more or all portions of the fusion protein to a second state of localization or compartmentalization of one or more or all portions of the fusion protein, e.g., from the golgi or ER to another compartment or localization, e.g., the cytosol, a membrane, the cell surface, or extracellular space.

In an instance, the presence of the expression compound can be associated with, cause, or mediate, the transformation from or of a first folding state to a second folding state, e.g., from a misfolded state to a more properly folded state, e.g., a first state susceptable to degradation to a second state less susceptable to degradation than the first state; or from a first folding state that has a first level of degradation to a second folding state that has a second, lessor, level of degradation, e.g., in a cell of interest.

In the present invention the conditional expression domain is a degradation domain, wherein the degradation domain has a first state associated with a first level of surface expression of the fusion protein and a second state associated with a second level of surface expression of the fusion protein, wherein the second level is increased, e.g., by at least 2, 3, 4, 5, 10, 20 or 30 fold over the first level in the presence of a stabilization compound.

In an embodiment, one of the states, e.g., the second state, is associated with, causes, or mediates cleavage at the cleavage site. While not wishing to be bound by theory, it is believed, that in some embodiments, cleavage results or is associated with cell surface expression or extracellular expresson of one or more (e.g., all) portions of the fusion protein. In an embodiment, absent an expression compound, when expressed in a cell of interest, fusion protein, e.g., a first level, e.g., a large fraction, of the fusion protein, comprising a conditional expression domain is not detectable either on the cell surface or extracellularly, e.g., by steric hinderance of the cleavage by the another entity, e.g., the conditional expression domain. Conversely, in the presence of an expression compound, surface expression and/or extracellular expression of one or more (e.g., all) domains of the fusion protein is transformed to a second level which is higher than the first level, e.g., substantially increased, e.g., by 2, 3, 4, 5, or 10 fold over the first level or increased, e.g., by at least 10, 20, 30, 40, 50, 60, 70, 80, 90, 100, 150, 200, 300, 400, 500, 600, 700, 800, 900, or 1000% over the first level. Thus, in an embodiment, a conditional expression domain is identifiable or characterized by the following characteristics: (1) it is not naturally occurring in the context of the fusion protein, e.g., it is recombinant or engineered; (2) surface expression and/or extracellular expression is regulated co-translationally or post-translationally; (3) the rate of surface expression and/or extracellular expression is substantially increased in the presence of an expression compound.

In an instance disclosed herein, addition of expression compound, e.g., deaggregation compound or stabilization compound, to a plurality of cells, e.g., host cells or cells comprising fusion proteins described herein, causes a transformation of a sub-plurality of cells from the first state to the second state, e.g., states of aggregation or conformational states, e.g., states of stabilization/destabilization, or states of folding/misfolding as described herein. In an instance, in the absence of expression compound, e.g., deaggregation compound or stabilization compound, less than 10, 9, 8, 7, 6, 5, 4, 3, 2, or 1% of the cells in the plurality comprise the second state, and greater than or equal to 90, 91, 92, 93, 94, 95, 96, 97, 98, or 99% of the cells in the plurality comprise the first state. In an instance, in the presence of expression compound, e.g., deaggregation compound or stabilization compound, greater than or equal to 20, 30, 40, 50, 60, 70, 80, 90, or 95% of the cells in the plurality comprise the second state, and less than 20, 15, 10, 9, 8, 7, 6, 5, 4, 3, 2, or 1% of the cells in the plurality comprise the first state. Determination of the percentages of cells in a plurality comprising a state can be made using methods described throughout the specification, for example, as used in Figure 4.

In the present invention, a conditional expression domain is a degradation domain, for example, as described herein. In other instances disclosed herein which do not form part of the claimed invention, a conditional expression domain is an aggregation domain, for example as described herein. In an instance, a conditional expression domain does not comprise a degradation domain. In an embodiment, a conditional expression domain does not comprise an aggregation domain. In the present invention, the fusion protein comprising the conditional expression domain comprises a transmembrane protein, which is a chimeric antigen receptor (CAR), e.g., as described herein. In the present invention, the conditional expression domain is a degradation domain. In some instances, the conditional expression domain is an aggregation domain.

### AGGREGATION DOMAINS

In some instances disclosed herein which do not form part of the claimed invention, the conditional expression domain is an aggregation domain. Methods of generating aggregation domains that cause intracellular aggregation in the absence of an expression compound, e.g., a deaggregation compound, are known in the art and discussed further below.

The present disclosure encompasses aggregation domains derived from any naturally occurring protein. Preferably, fusion proteins of the disclosure will include an aggregation domain for which no deaggregation compound is natively expressed or provided in the cell compartments of interest. For example, if the fusion protein is designed for expression in T-cells, it is preferable to select an aggregation domain which is paired with a deaggregation compound that is not naturally present in T-cells. Thus, the aggregation domain, when expressed in the cell of interest, will only be deaggregated in the presence of deaggregation compound. Notably, this property can be engineered by either engineering the aggregation domain to no longer bind a natively expressed/present deaggregation compound (in which case the aggregation domain will only deaggregate in the presence of a synthetic compound) or by expressing the aggregation domain in a compartment where the natively expressed/present ligand does not occur (e.g., the aggregation domain can be derived from a species other than the species in which the fusion protein will be expressed).

Aggregation domain -deaggregation compound pairs can be derived from any naturally occurring or synthetically developed protein. Deaggregation compounds can be any naturally occurring or synthetic compounds. In certain instances, the deaggregation compounds will be existing prescription or over-the-counter medicines. Examples of proteins that can be engineered to possess the properties of an aggregation domain are set forth below along with a corresponding deaggregation compound.

In one instance, the aggregation domain is a dimerization domain, e.g., is derived from an an FKB protein (FKBP). In some instances, the aggregation domain comprises an amino acid sequence selected from SEQ ID NO: 975 or a sequence having at least 90%, 95%, 97%, 98%, or 99% identity thereto, or SEQ ID NO: 976 or a sequence having at least 90%, 95%, 97%, 98%, or 99% identity thereto. In some instances, the aggregation domain comprises an amino acid sequence selected from SEQ ID NO: 979 or a sequence having at least 90%, 95%, 97%, 98%, or 99% identity thereto, or SEQ ID NO: 980 or a sequence having at least 90%, 95%, 97%, 98%, or 99% identity thereto. When the aggregation domain is derived from an FKBP, the deaggregation compound can be selected from AP21998 or AP22542, known in the art and described in Rollins et al 2000 PNAS vol. 97 no. 13, 7096-7101, WO/2000/023600, and Rivera, V. M., et al. (2000) Regulation of protein secretion through controlled aggregation in the endoplasmic reticulum. Science 287: 826-30. While not wishing to be bound by theory, FKBP domains comprising F36M substitutions associate into dimers. The FKBP F36M dimers can be disassociated, e.g., deaggregated, by addition of ligand, e.g., FK506, rapamycin, AP22542, AP21998, or Shield-1.

GVQVETISPGDGRTFPKRGQTCVVHYTGMLEDGKKFDSSRDRNKPFKFMLGKQEVIRGWEEGVAQMS
VGQRAKLTISPDYAYGATGHPGIIPPHATLVFDVELLKLE (SEQ ID NO: 975)

GVQVETISPGDGRTFPKRGQTCVVHYTGMLEDGKKMDSSRDRNKPFKFMLGKQEVIRGWEEGVAQMS
VGQRAKLTISPDYAYGATGHPGIIPPHATLVFDVELLKLE (SEQ ID NO: 976)

RGVQVETISPGDGRTFPKRGQTCVVHYTGMLEDGKKFDSSRDRNKPFKFMLGKQEVIRGWEEGVAQM
SVGQRAKLTISPDYAYGATGHPGIIPPHATLVFDVELLKLETRGVQVETISPGDGRTFPKRGQTCVVHYTG
MLEDGKKMDSSRDRNKPFKFMLGKQEVIRGWEEGVAQMSVGQRAKLTISPDYAYGATGHPGIIPPHATL
VFDVELLKLETRGVQVETISPGDGRTFPKRGQTCVVHYTGMLEDGKKMDSSRDRNKPFKFMLGKQEVIR
GWEEGVAQMSVGQRAKLTISPDYAYGATGHPGIIPPHATLVFDVELLKLETRGVQVETISPGDGRTFPKR
GQTCVVHYTGMLEDGKKMDSSRDRNKPFKFMLGKQEVIRGWEEGVAQMSVGQRAKLTISPDYAYGAT
GHPGIIPPHATLVFDVELLKLET (SEQ ID NO: 979)

RGVQVETISPGDGRTFPKRGQTCVVHYTGMLEDGKKMDSSRDRNKPFKFMLGKQEVIRGWEEGVAQM
SVGQRAKLTISPDYAYGATGHPGIIPPHATLVFDVELLKLETRGVQVETISPGDGRTFPKRGQTCVVHYTG
MLEDGKKMDSSRDRNKPFKFMLGKQEVIRGWEEGVAQMSVGQRAKLTISPDYAYGATGHPGIIPPHATL
VFDVELLKLETRGVQVETISPGDGRTFPKRGQTCVVHYTGMLEDGKKMDSSRDRNKPFKFMLGKQEVIR
GWEEGVAQMSVGQRAKLTISPDYAYGATGHPGIIPPHATLVFDVELLKLETRGVQVETISPGDGRTFPKR
GQTCVVHYTGMLEDGKKMDSSRDRNKPFKFMLGKQEVIRGWEEGVAQMSVGQRAKLTISPDYAYGAT
GHPGIIPPHATLVFDVELLKLET (SEQ ID NO: 980)

In another instance, the aggregation domain is derived from a UVR8 protein. UVR8 is a plant photoreceptor protein (Chen et al. 2013. J. Cell. Biol. v201 no. 4: 631-640; Rizzini, L., et al. 2011. Science. 332:103-106; Christie, J.M., et al. 2012. Science. 335:1492-1496; Wu, D., et al. 2012. Nature. 484:214-219). UVR8 constitutively forms photolabile homodimers which dissociate upon absorption of ultraviolet B (UVB) radiation (280-320 nm). In an instance, the aggregation domain is derived from a UVR8 domain, and the deaggregation compound is a suitable dose of UVB radiation. In an instance, the suitable dose of UVB radiation is sufficient to dissociate at least 25, 50, 75, or 100% of the UVR8 homodimers. In an instance, the suitable dose of UVB is minimally toxic to a cell comprising the fusion protein.

In some instances, a fusion protein of the present disclosure comprises multiple, e.g., 2, 3, 4, 5, 6, 7, 8, 9, 10, or more, copies of a conditional expression domain, e.g., an aggregation domain or degradation domain. In some instances, a fusion protein of the present disclosure comprises multiple, e.g., 2, 3, 4, 5, 6, 7, 8, 9, 10, or more, aggregation domains.

In some instances, each of the multiple aggregation domains is a copy of the same aggregation domain. Such aggregation domains would bind to each other to form homooligomers, e.g., homodimers,

In some instances, a fusion protein of the present disclosure comprises a plurality, e.g., more than one, of aggregation domains, wherein the plurality comprises more than one type, e.g., a first type and a second type, of aggregation domain. In some instances, the first type of aggregation domain associates, e.g., binds to and promotes oligomerization or aggregation, with the second type of aggregation domain; such association represents heteromeric association or heterooligomerization of aggregation domains, e.g., into heterodimers. In some instances, a fusion protein of the present disclosure comprises two, four, six, or eight aggregation domains, wherein the fusion protein comprises at least one of each type, e.g., a first type and a second type, of aggregation domain. In one instance, a fusion protein comprising a plurality of aggregation domains, wherein the plurality comprises more than one type, e.g., a first type and a second type, of aggregation domain, comprises equal numbers of each type of aggregation domain. In some instances, wherein a fusion protein comprises four or more (e.g., six, eight, ten, or more) aggregation domains, the aggregation domains are disposed in an alternating pattern in the fusion protein, e.g., type 1, type 2, type 1, type 2. In some instances, wherein a fusion protein comprises four or more (e.g., six, eight, ten, or more) aggregation domains, the aggregation domains are disposed in a block pattern in the fusion protein, e.g., type 1, type 1, type 2, type 2. In some instances, the first type of aggregation domain does not appreciably associate with other copies of the first type of aggregation domain and the second type of aggregation domain does not appreciably associate with other copies of the second type of aggregation domain. In some instances, the first type of aggregation domain only appreciably associates with other copies of the first type of aggregation domain and the second type of aggregation domain only appreciably associates with other copies of the second type of aggregation domain.

In some instances, the expression compound, e.g., deaggregation compound, is AP21998 or AP22542 (chemical structure shown here; AP21998 and AP22542 are the same in the portion left of the dashed line and differ in the portion right of the dashed line). Preparation and use of AP21998 and AP22542 can be found in the art and in Rollins et al. 2000, Amara, J. F., et al. (1997) Proc. Natl. Acad. Sci. USA 94, 10618-10623, Clackson, T., et al. (1998) Proc. Natl. Acad. Sci. USA 95, 10437-10442, and Yang, W., et al. (2000) J. Med. Chem. 43, 1135-1142.

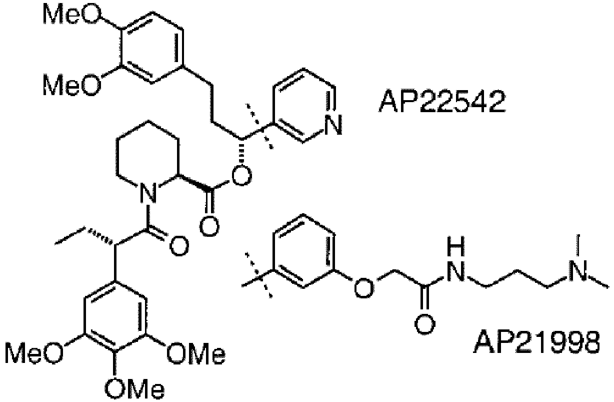

In some instances, the expression compound, e.g., deaggregation compound, is FK506 (tacrolimus) or rapamycin (sirolimus). In some instances, the conditional expression domain, e.g., aggregation domain, is derived from FKBP and the expression compound, e.g., deaggregation compound, is FK506 (tacrolimus) or rapamycin (sirolimus). Preparation and use of FK506 and rapamycin can be found in the art.

In some instances, the expression compound, e.g., deaggregation compound, inhibits interaction between aggregation domains. In some instances, wherein the deaggregation compound is FK506, rapamycin, AP22542, or AP21998, the deaggregation compound inhibits interaction between fusion proteins comprising an aggregation domain derived from FKBP F36M.

An exemplary aggregation domain-deaggregation compound pair has been generated and is featured in the present disclosure (SEQ ID NOs: 977 (nucleic acid) and 978 (amino acid)). This aggregation domain comprises an FKBP F36M domain as described in: "A ligand-reversible dimerization system for controlling protein-protein interactions." Rollins et al. PNAS vol. 97 no. 13, 7096-7101, and WO/2000/023600.

### CD19bbz on-CAR nucleotide sequence, CD19 on-CAR (SEQ ID NO:977)

Signal peptide-conditional aggregation domains (4 repeats)- Furin site-FMC63bbz

ATGGCCTTACCAGTGACCGCCTTGCTCCTGCCGCTGGCCTTGCTGCTCCACGCCGCCAGGCCGGGATCCCGGGGC
GTGCAGGTTGAGACAATTTCCCCAGGAGATGGGCGAACGTTCCCCAAGCGCGGACAGACATGCGTTGTGCACTAC
ACAGGAATGTTGGAGGACGGAAAGAAAATGGACAGTTCAAGAGATCGGAACAAACCATTCAAATTCATGTTGGG
AAAACAGGAAGTGATACGGGGCTGGGAAGAGGGTGTAGCGCAAATGTCCGTTGGTCAACGAGCAAAACTCACG
AT

AAGTCCCGATTATGCTTACGGCGCAACCGGTCACCCGGGCATCATACCGCCTCATGCGACTTTGGTCTTTGATGTG
GAGCTGTTGAAACTTGAAACTCGCGGAGTACAGGTTGAAACAATATCACCCGGGGACGGGCGGACTTTTCCGAAG
AGAGGTCAGACCTGCGTCGTCCATTATACCGGTATGCTGGAGGACGGAAAGAAAATGGACAGCTCACGGGACCG
AAATAAACCATTCAAATTTATGTTGGGGAAACAAGAGGTTATCAGGGGCTGGGAGGAGGGTGTGGCCCAGATGT
CTGTCGGTCAGCGCGCGAAACTCACAATCTCTCCGGATTATGCGTATGGGGCGACAGGGCATCCGGGAATTATCC
CTCCCCACGCTACCTTGGTTTTCGATGTTGAGCTTCTGAAGTTGGAGACCAGAGGAGTTCAAGTGGAGACAATATC
TCCTGGGGATGGACGGACGTTCCCCAAGCGCGGCCAGACCTGTGTAGTCCACTACACAGGGATGCTTGAAGACG
GAAAAAAGATGGATAGCAGTAGAGATCGCAACAAACCATTTAAGTTCATGCTGGGGAAGCAGGAAGTAATACGC
GGCTGGGAGGAAGGCGTGGCACAGATGAGTGTTGGTCAACGGGCCAAACTTACTATTTCTCCCGATTATGCGTAT
GGAGCCACCGGGCACCCTGGCATTATCCCACCCCATGCCACATTGGTTTTTGACGTTGAATTGCTTAAATTGGAGA
CCAGGGGAGTCCAAGTGGAAACAATATCACCGGGGGATGGTCGGACTTTTCCTAAAAGGGGCCAAACCTGTGTA
GTCCATTATACCGGAATGCTCGAAGACGGAAAGAAAATGGACTCTTCTAGAGACCGCAATAAGCCCTTCAAGTTC
ATGTTGGGTAAGCAAGAGGTGATCCGGGGCTGGGAAGAGGGGGTCGCTCAAATGTCCGTCGGTCAGCGAGCTAA
ACTGACTATTTCCCCAGACTACGCATATGGAGCGACTGGCCACCCCGGTATTATTCCTCCCCATGCGACTCTCGTGT
TCGACGTAGAACTCTTGAAATTGGAAACGTCAGCCCGGAACAGGCGGAAGAGAGGATCCGACATCCAGATGACA
CAGACTACATCCTCCCTGTCTGCCTCTCTGGGAGACAGAGTCACCATCAGTTGCAGGGCAAGTCAGGACATTAGTA
AATATTTAAATTGGTATCAGCAGAAACCAGATGGAACTGTTAAACTCCTGATCTACCATACATCAAGATTACACTCA
GGAGTCCCATCAAGGTTCAGTGGCAGTGGGTCTGGAACAGATTATTCTCTCACCATTAGCAACCTGGAGCAAGAA
GATATTGCCACTTACTTTTGCCAACAGGGTAATACGCTTCCGTACACGTTCGGAGGGGGGACTAAGTTGGAAATAA
CAGGTGGCGGTGGCTCGGGCGGTGGTGGGTCGGGTGGCGGCGGATCTGAGGTGAAACTGCAGGAGTCAGGACC
TGGCCTGGTGGCGCCCTCACAGAGCCTGTCCGTCACATGCACTGTCTCAGGGGTCTCATTACCCGACTATGGTGTA
AGCTGGATTCGCCAGCCTCCACGAAAGGGTCTGGAGTGGCTGGGAGTAATATGGGGTAGTGAAACCACATACTAT
AATTCAGCTCTCAAATCCAGACTGACCATCATCAAGGACAACTCCAAGAGCCAAGTTTTCTTAAAAATGAACAGTC
TGCAAACTGATGACACAGCCATTTACTACTGTGCCAAACATTATTACTACGGTGGTAGCTATGCTATGGACTACTG
GGGTCAAGGAACCTCAGTCACCGTCTCCTCAGCTAGCACCACGACGCCAGCGCCGCGACCACCAACACCGGCGCC
CACCATCGCGTCGCAGCCCCTGTCCCTGCGCCCAGAGGCGTGCCGGCCAGCGGCGGGGGGCGCAGTGCACACGA
GGGGGCTGGACTTCGCCTGTGATTCCGGAATCTACATCTGGGCGCCCTTGGCCGGGACTTGTGGGGTCCTTCTCCT
GTCACTGGTTATCACCCTTTACTGCAAACGGGGCAGAAAGAAACTCCTGTATATATTCAAACAACCATTTATGAGA
CCAGTACAAACTACTCAAGAGGAAGATGGCTGTAGCTGCCGATTTCCAGAAGAAGAAGGAGGATGTGAACT
GAGAGTGAAGTTCAGCAGGAGCGCAGACGCCCCCGCGTACCAGCAGGGCCAGAACCAGCTCTATAACGAGCTCA
ATCTAGGACGAAGAGAGGAGTACGATGTTTTGGACAAGAGACGTGGCCGGGACCCTGAGATGGGGGGAAAGCC
GAGAAGGAAGAACCCTCAGGAAGGCCTGTACAATGAACTGCAGAAAGATAAGATGGCGGAGGCCTACAGTGAG
ATTGGGATGAAAGGCGAGCGCCGGAGGGGCAAGGGGCACGATGGCCTTTACCAGGGTCTCAGTACAGCCACCAA
GGACACCTACGACGCCCTTCACATGCAGGCCCTGCCCCCTCGCTAA

### CD19bbz on-CAR amino acid sequence (SEQ ID NO:978)

Signal peptide-conditional aggregation domains (4 repeats)-Furin site-FMC63bbz

MALPVTALLLPLALLLHAARPGSRGVQVETISPGDGRTFPKRGQTCVVHYTGMLEDGKKMDSSRDRNKPFKFMLGKQ
EVIRGWEEGVAQMSVGQRAKLTISPDYAYGATGHPGIIPPHATLVFDVELLKLETRGVQVETISPGDGRTFPKRGQTCV

VHYTGMLEDGKKMDSSRDRNKPFKFMLGKQEVIRGWEEGVAQMSVGQRAKLTISPDYAYGATGHPGIIPPHATLVFD
VELLKLETRGVQVETISPGDGRTFPKRGQTCVVHYTGMLEDGKKMDSSRDRNKPFKFMLGKQEVIRGWEEGVAQMS
VGQRAKLTISPDYAYGATGHPGIIPPHATLVFDVELLKLETRGVQVETISPGDGRTFPKRGQTCVVHYTGMLEDGKKMD
SSRDRNKPFKFMLGKQEVIRGWEEGVAQMSVGQRAKLTISPDYAYGATGHPGIIPPHATLVFDVELLKLETSARNRRKR
GSDIQMTQTTSSLSASLGDRVTISCRASQDISKYLNWYQQKPDGTVKLLIYHTSRLHSGVPSRFSGSGSGTDYSLTISNLE
QEDIATYFCQQGNTLPYTFGGGTKLEITGGGGSGGGGSGGGGSEVKLQESGPGLVAPSQSLSVTCTVSGVSLPDYGVS
WIRQPPRKGLEWLGVIWGSETTYYNSALKSRLTIIKDNSKSQVFLKMNSLQTDDTAIYYCAKHYYYGGSYAMDYWGQG
TSVTVSSASTTTPAPRPPTPAPTIASQPLSLRPEACRPAAGGAVHTRGLDFACDSGIYIWAPLAGTCGVLLLSLVITLYCKR
GRKKLLYIFKQPFMRPVQTTQEEDGCSCRFPEEEEGGCELRVKFSRSADAPAYQQGQNQLYNELNLGRREEYDVLDKR
RGRDPEMGGKPRRKNPQEGLYNELQKDKMAEAYSEIGMKGERRRGKGHDGLYQGLSTATKDTYDALHMQALPPRZ

### DEGRADATION DOMAINS

In the present invention, the conditional expression domain is a degradation domain. Methods of generating degradation domains that are selectively stable in the presence of a stabilization compound are well known in the art and discussed further below. Several such domain-stabilization compound pairs have been generated to date and are featured in the present invention. These include degradation domains based on FKBP (e.g., using a "Shield" stabilization compound) as described in: A Rapid, Reversible, and Tunable Method to Regulate Protein Function in Living Cells Using Synthetic Small Molecules." Banaszynski, L. A.; Chen, L.-C.; Maynard-Smith, L. A.; Ooi, A. G. L.; Wandless, T. J. Cell, 2006, 126, 995-1004; domains based on DHFR (e.g., using trimethoprim as a stabilization compound) as described in A general chemical method to regulate protein stability in the mammalian central nervous system. Iwamoto, M.; Björklund, T.; Lundberg, C.; Kirik, D.; Wandless, T. J. Chemistry & Biology, 2010, 17, 981-988; and domains based on estrogen receptor alpha (e.g., where 4OHT is used as a stabilization compound) as described in Destabilizing domains derived from the human estrogen receptor Y Miyazaki, H Imoto, L-c Chen, TJ Wandless J. Am. Chem. Soc. 2012, 134, 3942-3945.

The present disclosure encompasses degradation domains derived from any naturally occurring protein. Preferably, fusion proteins of the invention will include a degradation domain for which there is no ligand natively expressed in the cell compartments of interest. For example, if the fusion protein is designed for expression in T-cells, it is preferable to select a degradation domain for which there is no naturally occurring ligand present in T cells. Thus, the degradation domain, when expressed in the cell of interest, will only be stabilized in the presence of an exogenously added compound. Notably, this property can be engineered by either engineering the degradation domain to no longer bind a natively expressed ligand (in which case the degradation domain will only be stable in the presence of a synthetic compound) or by expressing the degradation domain in a compartment where the natively expressed ligand does not occur (e.g., the degradation domain can be derived from a species other than the species in which the fusion protein will be expressed).

Degradation domain -stabilization compound pairs can be derived from any naturally occurring or synthetically developed protein. Stabilization compounds can be any naturally occurring or synthetic compounds. In certain embodiments, the stabilization compounds will be existing prescription or over-the-counter medicines. Examples of proteins that can be engineered to possess the properties of a degradation domain are set forth in Table 21 below along with a corresponding stabilization compound.

In some embodiments, the degradation domain is derived from a protein listed in Table 21.

In some embodiments, the degradation domain is derived from an estrogen receptor (ER). In some embodiments, the degradation domain comprises an amino acid sequence selected from SEQ ID NO: 58 or a sequence having at least 90%, 95%, 97%, 98%, or 99% identity thereto, or SEQ ID NO: 121 or a sequence having at least 90%, 95%, 97%, 98%, or 99% identity thereto. In some embodiments, the degradation domain comprises an amino acid sequence selected from SEQ ID NO: 58, or SEQ ID NO: 121. When the degradation domain is derived from an estrogen receptor, the stabilization compound can be selected from Bazedoxifene or 4-hydroxy tamoxifen (4-OHT). In some embodiments, the stabilization compound is Bazedoxifene. Tamoxifen and Bazedoxifene are FDA approved drugs, and thus are safe to use in human.

In some embodiments, the degradation domain is derived from an FKB protein (FKBP). In some embodiments, the degradation domain comprises an amino acid sequence of SEQ ID NO: 56 or a sequence having at least 90%, 95%, 97%, 98%, or 99% identity thereto. In some embodiments, the degradation domain comprises an amino acid sequence of SEQ ID NO: 56. When the degradation domain is derived from a FKBP, the stabilization compound can be Shield-1.

In some embodiments, the degradation domain is derived from dihydrofolate reductase (DHFR). In some embodiments, the degradation domain comprises an amino acid sequence selected from SEQ ID NO: 57 or a sequence having at least 90%, 95%, 97%, 98%, or 99% identity thereto. In some embodiments, the degradation domain comprises an amino acid sequence selected from SEQ ID NO: 57. When the degradation domain is derived from a DHFR, the stabilization compound can be Trimethoprim.

In some embodiments, the degradation domain is not derived from an FKB protein, estrogen receptor, or DHFR.

**Table 21. Exemplary proteins for generating degradation domains**

| **Type** | **Activity of drug** | **Drug examples** |
|---|---|---|
| ***Oxidoreductases*** | | |
| Aldehyde dehydrogenase | Inhibitor | Disulfiram |
| Monoamine oxidases (MAOs) | MAO-A inhibitor | Tranylcypromine, moclobemide |
| | MAO-B inhibitor | Tranylcypromine |
| Cyclooxygenases (COXs) | COX1 inhibitor | Acetylsalicylic acid, profens, acetaminophen and dipyrone (as arachidonylamides) |
| | COX2 inhibitor | Acetylsalicylic acid, profens, acetaminophen and dipyrone (as arachidonylamides) |
| Vitamin K epoxide reductase | Inhibitor | Warfarin, phenprocoumon |
| Aromatase | Inhibitor | Exemestane |
| Lanosterol demethylase (fungal) | Inhibitor | Azole antifungals |
| Lipoxygenases | Inhibitor | Mesalazine |
| | 5-lipoxygenase inhibitor | Zileuton |
| Thyroidal peroxidase | Inhibitor | Thiouracils |
| lodothyronine-5' deiodinase | Inhibitor | Propylthiouracil |
| Inosine monophosphate dehydrogenase | Inhibitor | Mycophenolate mofetil |
| HMG-CoA reductase | Inhibitor | Statins |
| α-5-Testosterone reductase | Inhibitor | Finasteride, dutasteride |
| Dihydrofolate reductase (bacterial) | Inhibitor | Trimethoprim |
| Dihydrofolate reductase (human) | Inhibitor | Methotrexate, pemetrexed |
| Dihydrofolate reductase (parasitic) | Inhibitor | Proguanil |
| Dihydroorotate reductase | Inhibitor | Leflunomide |
| Enoyl reductase (mycobacterial) | Inhibitor | Isoniazid |
| Squalene epoxidase (fungal) | Inhibitor | Terbinafin |
| Δ-14 reductase (fungal) | Inhibitor | Amorolfin |
| Xanthine oxidase | Inhibitor | Allopurinol |
| 4-Hydroxyphenylpyruvate dioxygenase | Inhibitor | Nitisinone |
| Ribonucleoside diphosphate reductase | Inhibitor | Hydroxycarbamide |

| ***Transferases*** | | |
|---|---|---|
| Protein kinase C | Inhibitor | Miltefosine |
| Bacterial peptidyl transferase | Inhibitor | Chloramphenicol |
| Catecholamine-*O-*methyltransferase | Inhibitor | Entacapone |
| RNA polymerase (bacterial) | Inhibitor | Ansamycins |
| Reverse transcriptases (viral) | Competitive inhibitors | Zidovudine |
| | Allosteric inhibitors | Efavirenz |
| DNA polymerases | Inhibitor | Acyclovir, suramin |
| GABA transaminase | Inhibitor | Valproic acid, vigabatrin |
| Tyrosine kinases | PDGFR/ABL/KIT inhibitor | Imatinib |
| | EGFR inhibitor | Erlotinib |
| | β-VEGFR2/PDGFR/KIT/FLT3 | Sunitinib |
| | β-VEGFR2/PDGFR/RAF | Sorafenib |
| Glycinamide ribonucleotide formyl transferase | Inhibitor | Pemetrexed |
| Phosphoenolpyruvate transferase (MurA, bacterial) | Inhibitor | Fosfomycin |
| Human cytosolic branched-chain aminotransferase (hBCATc) | Inhibitor | Gabapentin |

| ***Hydrolases (proteases)*** | | |
|---|---|---|
| Aspartyl proteases (viral) | HIV protease inhibitor | Saquinavir, indinavir |

| ***Hydrolases (serine proteases)*** | | |
|---|---|---|
| Unspecific | Unspecific inhibitors | Aprotinine |
| Bacterial serine protease | Direct inhibitor | β-lactams |
| Bacterial serine protease | Indirect inhibitor | Glycopeptides |
| Bacterial lactamases | Direct inhibitor | Sulbactam |
| Human antithrombin | Activator | Heparins |
| Human plasminogen | Activator | Streptokinase |
| Human coagulation factor | Activator | Factor IX complex, Factor VIII |
| Human factor Xa | Inhibitor | Fondaparinux |

| ***Hydrolases (metalloproteases)*** | | |
|---|---|---|
| Human ACE | Inhibitor | Captopril |
| Human HRD | Inhibitor | Cilastatin |
| Human carboxypeptidase A (Zn) | Inhibitor | Penicillamine |
| Human enkephalinase | Inhibitor | Racecadotril |

| ***Hydrolases (other)*** | | |
|---|---|---|
| 26S proteasome | Inhibitor | Bortezomib |
| Esterases | AChE inhibitor | Physostigmine |
| | AChE reactivators | Obidoxime |
| | PDE inhibitor | Caffeine |
| | PDE3 inhibitor | Amrinon, milrinone |
| | PDE4 inhibitor | Papaverine |
| | PDE5 inhibitor | Sildenafil |
| | HDAC inhibitor | Valproic acid |
| | HDAC3/HDAC7 inhibitor | Carbamezepine |
| Glycosidases (viral) | α-glycosidase inhibitor | Zanamivir, oseltamivir |
| Glycosidases (human) | α-glycosidase inhibitor | Acarbose |
| Lipases | Gastrointestinal lipases inhibitor | Orlistat |
| Phosphatases | Calcineurin inhibitor | Cyclosporin |
| | Inositol polyphosphate phosphatase inhibitor | Lithium ions |
| GTPases | Rac1 inhibitor | 6-Thio-GTP (azathioprine metabolite) |
| Phosphorylases | Bacterial C55-lipid phosphate dephosphorylase inhibitor | Bacitracin |

| ***Lyases*** | | |
|---|---|---|
| DOPA decarboxylase | Inhibitor | Carbidopa |
| Carbonic anhydrase | Inhibitor | Acetazolamide |
| Histidine decarboxylase | Inhibitor | Tritoqualine |
| Ornithine decarboxylase | Inhibitor | Eflornithine |
| Soluble guanylyl cyclase | Activator | Nitric acid esters, molsidomine |

| ***Isomerases*** | | |
|---|---|---|
| Alanine racemase | Inhibitor | D-Cycloserine |
| DNA gyrases (bacterial) | Inhibitor | Fluoroquinolones |
| Topoisomerases | Topoisomerase I inhibitor | Irinotecan |
| | Topoisomerase II inhibitor | Etoposide |
| 8,7 isomerase (fungal) | Inhibitor | Amorolfin |

| ***Ligases (also known as synthases)*** | | |
|---|---|---|
| Dihydropteroate synthase | Inhibitor | Sulphonamides |
| Thymidylate synthase (fungal and human) | Inhibitor | Fluorouracil |
| Thymidylate synthase (human) | Inhibitor | Methotrexate, pemetrexed |
| Phosphofructokinase | Inhibitor | Antimony compounds |
| mTOR | Inhibitor | Rapamycin |
| Haem polymerase (Plasmodium) | Inhibitor | Quinoline antimalarials |
| β-1,3--D-glucansynthase (fungi) | Inhibitor | Caspofungin |
| Glucosylceramide synthase | Inhibitor | Miglustat |
| | | |

| **Substrate** | **Drug substance** | |
|---|---|---|
| Asparagine | Asparaginase | |
| Urate | Rasburicase (a urate oxidase) | |
| VAM P-syn aptobrevi n, SNAP25, Syntaxin | Light chain of the botulinum neurotoxin (Zn-endopeptidase) | |
| | | |

| **Type** | **Activity of drug** | **Drug examples** |
|---|---|---|
| ***Direct ligand-gated ion channel receptors*** | | |
| GABA_{A} receptors | Barbiturate binding site agonists | Barbiturate |
| | Benzodiazepine binding site agonists | Benzodiazepines |
| | Benzodiazepine binding site antagonists | Flumazenil |
| Acetylcholine receptors | Nicotinic receptor agonists | Pyrantel (of *Angiostrongylus*), levamisole |
| | Nicotinic receptor stabilizing antagonists | Alcuronium |
| | Nicotinic receptor depolarizing antagonists | Suxamethonium |
| | Nicotinic receptor allosteric modulators | Galantamine |
| Glutamate receptors (ionotropic) | NMDA subtype antagonists | Memantine |
| | NMDA subtype expression modulators | Acamprosate |
| | NMDA subtype phencyclidine binding site antagonists | Ketamine |

| ***G-protein-coupled receptors*** | | |
|---|---|---|
| Acetylcholine receptors | Muscarinic receptor agonists | Pilocarpine |
| | Muscarinic receptor antagonists | Tropane derivatives |
| | Muscarinic receptor M₃ antagonists | Darifenacine |
| Adenosine receptors | Agonists | Adenosine |
| | Adenosine A₁ receptor agonists | Lignans from valerian |
| | Adenosine A1 receptor antagonists | Caffeine, theophylline |
| | Adenosine A_{2A} receptor antagonists | Caffeine, theophylline |
| Adrenoceptors | Agonists | Adrenaline, noradrenaline, ephedrine |
| | α₁- and α₂-receptors agonists | Xylometazoline |
| | α₁-receptor antagonists | Ergotamine |
| | α₂-receptor, central agonists | Methyldopa (as methylnoradrenaline) |
| | β-adrenoceptor antagonists | Isoprenaline |
| | β₁-receptor antagonists | Propranolol, atenolol |
| | β₂-receptor agonists | Salbutamol |
| | β₂-receptor antagonists | Propranolol |
| Angiotensin receptors | AT₁-receptors antagonists | Sartans |
| Calcium-sensing receptor | Agonists | Strontium ions |
| | Allosteric activators | Cinacalcet |
| Cannabinoid receptors | CB₁ - and CB₂-receptors agonists | Dronabinol |
| Cysteinyl-leukotriene receptors | Antagonists | Montelukast |
| Dopamine receptors | Dopamine receptor subtype direct agonists | Dopamine, levodopa |
| | D₂, D₃ and D₄ agonists | Apomorphine |
| | D₂, D₃ and D₄ antagonists | Chlorpromazine, fluphenazine, haloperidol, metoclopramide, ziprasidone |
| Endothelin receptors (ET_{A}, ET_{B}) | Antagonists | Bosentan |
| GABA_{B} receptors | Agonists | Baclofen |
| Glucagon receptors | Agonists | Glucagon |
| Glucagon-like peptide-1 receptor | Agonists | Exenatide |
| Histamine receptors | H₁-antagonists | Diphenhydramine |
| | H₂-antagonists | Cimetidine |
| Opioid receptors | µ-opioid agonists | Morphine, buprenorphine |
| | µ-, κ- and δ-opioid antagonists | Naltrexone |
| | κ-opioid antagonists | Buprenorphine |
| Neurokinin receptors | NK₁ receptor antagonists | Aprepitant |
| Prostanoid receptors | Agonists | Misoprostol, sulprostone, iloprost |
| Prostamide receptors | Agonists | Bimatoprost |
| Purinergic receptors | P₂Y₁₂ antagonists | Clopidogrel |
| Serotonin receptors | Subtype-specific (partial) agonists | Ergometrine, ergotamine |
| | 5-HT_{1A} partial agonists | Buspirone |
| | 5-HT_{1B/1D} agonists | Triptans |
| | 5-HT_{2A} antagonists | Quetiapine, ziprasidone |
| | 5-HT₃ antagonists | Granisetron |
| | 5-HT₄ partial agonists | Tegaserode |
| Vasopressin receptors | Agonists | Vasopressin |
| | V₁ agonists | Terlipressin |
| | V₂ agonists | Desmopressin |
| | OT agonists | Oxytocin |
| | OT antagonists | Atosiban |

| ***Cytokine receptors*** | | |
|---|---|---|
| Class I cytokine receptors | Growth hormone receptor antagonists | Pegvisomant |
| | Erythropoietin receptor agonists | Erythropoietin |
| | Granulocyte colony stimulating factor agonists | Filgrastim |
| | Granulocyte-macrophage colony stimulating factor agonists | Molgramostim |
| | Interleukin-1 receptor antagonists | Anakinra |
| | Interleukin-2 receptor agonists | Aldesleukin |
| TNFα receptors | Mimetics (soluble) | Etanercept |

| ***Integrin receptors*** | | |
|---|---|---|
| Glycoprotein IIb/IIIa receptor | Antagonists | Tirofiban |

| ***Receptors associated with a tyrosine kinase*** | | |
|---|---|---|
| Insulin receptor | Direct agonists | Insulin |
| Insulin receptor | Sensitizers | Biguanides |

| ***Nuclear receptors (steroid hormone receptors)*** | | |
|---|---|---|
| Mineralocorticoid receptor | Agonists | Aldosterone |
| | Antagonists | Spironolactone |
| Glucocorticoid receptor | Agonists | Glucocorticoids |
| Progesterone receptor | Agonists | Gestagens |
| Estrogen receptor | Agonists | Oestrogens |
| | (Partial) antagonists | Clomifene |
| | Antagonists | Fulvestrant |
| | Modulators | Tamoxifen, raloxifene |
| | Androgen receptor Agonists | Testosterone |
| | Antagonists | Cyproterone acetate |
| Vitamin D receptor | Agonists | Retinoids |
| ACTH receptor agonists | Agonists | Tetracosactide (also known as cosyntropin) |

| ***Nuclear receptors (other)*** | | |
|---|---|---|
| | α-Retinoic acid receptors RAR agonists | Isotretinoin |
| | β-RAR agonists | Adapalene, isotretinoin |
| | γ-RAR agonists | Adapalene, isotretinoin |
| Peroxisome proliferator-activated receptor (PPAR) | α-PPAR agonists | Fibrates |
| | γ-PPAR agonists | Glitazones |
| Thyroid hormone receptors | Agonists | L-Thyroxine |
| | | |
| | | |

| ***Voltage-gated Ca²⁺ channels*** | | |
|---|---|---|
| General | Inhibitor | Oxcarbazepine |
| In *Schistosoma sp.* | Inhibitor | Praziquantel |
| L-type channels | Inhibitor | Dihydropyridines, diltiazem, lercanidipine, pregabalin, verapamil |
| T-type channels | Inhibitor | Succinimides |
| K+ channels | | |
| Epithelial K⁺ channels | Opener Inhibitor | Diazoxide, minoxidil |
| | | Nateglinide, sulphonylureas |
| Voltage-gated K⁺ channels | Inhibitor | Amiodarone |

| ***Na⁺ channels*** | | |
|---|---|---|
| Epithelial Na+ channels (ENaC) | Inhibitor | Amiloride, bupivacaine, lidocaine, procainamide, quinidine |
| Voltage-gated Na⁺ channels | Inhibitor | Carbamazepine, flecainide, lamotrigine, phenytoin, propafenone, topiramate, valproic acid |

| ***Ryanodine-inositol 1,4,5-triphosphate receptor Ca²⁺ channel (RIR-CaC) family*** | | |
|---|---|---|
| Ryanodine receptors | Inhibitor | Dantrolene |

| ***Transient receptor potential Ca²⁺ channel (TRP-CC) family*** | | |
|---|---|---|
| TRPV1 receptors | Inhibitor | Acetaminophen (as arachidonylamide) |
| Cl- channels | | |
| Cl⁻channel | Inhibitor (mast cells) Opener (parasites) | Cromolyn sodium Ivermectin |
| | | |
| Cation-chloride cotransporter (CCC) family | Thiazide-sensitive NaCl symporter, human inhibitor | Thiazide diuretics |
| | Bumetanide-sensitive NaCl/KCl symporters, human inhibitor | Furosemide |
| Na⁺/H⁺ antiporters | Inhibitor | Amiloride, triamterene |
| Proton pumps | Ca²⁺-dependent ATPase (PfATP6; Plasmodia) inhibitor | Artemisinin and derivatives |
| H⁺/K⁺-ATPase | Inhibitor | Omeprazole |
| Na⁺/K⁺ ATPase | Inhibitor | Cardiac glycosides |
| Eukaryotic (putative) sterol transporter (EST) family | Niemann-Pick C1 like 1 (NPC1L1) protein inhibitor | Ezetimibe |
| Neurotransmitter/Na⁺ symporter (NSS) family | Serotonin/Na⁺ symporter inhibitor | Cocaine, tricyclic antidepressants, paroxetine |
| | Noradrenaline/Na⁺ symporter inhibitor | Bupropion, venlafaxine |
| | Dopamine/Na⁺ symporter inhibitor | Tricyclic antidepressants, cocaine, amphetamines |
| | Vesicular monoamine transporter inhibitor | Reserpine |

| ***Nucleic acids*** | | |
|---|---|---|
| DNA and RNA | Alkylation | Chlorambucil, cyclophosphamide, dacarbazine |
| | Complexation | Cisplatin |
| | Intercalation | Doxorubicin |
| | Oxidative degradation | Bleomycin |
| | Strand breaks | Nitroimidazoles |
| RNA | Interaction with 16S-rRNA | Aminoglycoside antiinfectives |
| | Interaction with 23S-rRNA | Macrolide antiinfectives |
| | 23S-rRNA/tRNA/2-polypeptide complex | Oxazolidinone antiinfectives |
| Spindle | Inhibition of development | Vinca alkaloids |
| | Inhibition of desaggregation | Taxanes |
| Inhibition of mitosis | - | Colchicine |
| Ribosome | | |
| 30S subunit (bacterial) | Inhibitors | Tetracyclines |
| 50S subunit (bacterial) | Inhibitors | Lincosamides, quinupristindalfopristin |

Degradation domains can be engineered from known proteins (e.g., those proteins set forth in the above table) through any of a variety of routine methods known in the art. Generally, such methods employ first creating a library of interest including proteins derived from the, e.g., naturally occurring protein. Second, cells or cell populations expressing proteins from individual library constructs will be selected on the basis of whether the expression of the derived protein is dependent on the presence of the desired stabilization compound. The process of derivation and selection can be repeated in as many cycles necessary to identify a suitable candidate.

For example, a library can be created through rational protein design based on sampling different structures and putative affinities of the protein domain to the selected compound. Alternatively, a library can be generated by random mutagenesis of the target protein. In either case, e.g., Jurkat cells can be transduced with a lentiviral library generated from the constructs. Jurkat cells can then undergo a round of FACS sorting, to eliminate cells that constitutively express the protein of interest. In the next stage, the sorted cells are incubated with the compound of choice for 24 hrs and positive cells are FACS sorted. These are expanded through single cell cloning. From there, individual transduced clones will be assessed for the ability to induce expression of the protein of interest in a compound-dependent manner.

### SUICIDE GENES AND DOMAINS

The present disclosure, in part, utilizes CAR technology for methods of selectively ablating or activating modified T cells from a subject after adoptive transfer. In one instance of the disclosure which does not form part of the claimed invention, a modified CAR T cell is selectively ablated in the subject by inducing activation of a suicide domain in the modified T cell. In another instance, a modified T cell is selectively activated in the subject by actively preventing the apoptosis of modified CAR T cell during therapy. In yet another instance, the modified T cell is selectively activated in the subject by allowing cell-surface expression of the CAR construct.

Some of the potential side effects of non-target cell recognition by CAR T cells can be overcome by the co-expression of a suicide gene in the CAR T cell. In addition, the CAR T cells can be selectively ablated after targeting B cells for depletion to treat autoantibody or alloantibody diseases or conditions.

Also disclosed herein but not forming part of the claimed invention is an isolated nucleic acid comprising a suicide gene. Examples of suicide genes include, but are not limited to, herpes simplex virus thymidine kinase (HSV-TK), the cytoplasmic domain of Fas, a caspase such as caspase-8 or caspase-9, cytosine deaminase, E1A, FHIT, and other known suicide or apoptosis-inducing genes (Straathof et al., 2005, Blood 105:4247-4254; Cohen et al., 1999, Leuk. Lymphoma 34:473-480; Thomis et al., 2001, Blood 97:1249-1257; Tey et al., 2007, Biol. Blood Marrow Transplant 13:913-924; and Di Stasi et al., 2011, N. Engl. J. Med. 365:1673-1683).

The suicide gene may be operably linked to a promoter, such as an inducible promoter sequence. Examples of inducible promoters include, but are not limited to, a heat shock promoter, a tetracycline-regulated promoter, a steroid-regulated promoter, a metal-regulated promoter, an estrogen receptor-regulated promoter, and others known in the art. In one aspect, the disclosure includes an isolated nucleic acid sequence comprising a nucleic acid sequence comprising a suicide gene and a nucleic acid encoding a chimeric antigen receptor. In another aspect, the disclosure includes an isolated nucleic acid sequence comprising a nucleic acid sequence comprising a suicide gene and a nucleic acid encoding a chimeric antigen receptor. In one instance of the disclosure which does not form part of the claimed invention, the suicide gene comprises the nucleic acid selected from the group consisting of

ATGCTCGAGGGGGTTCAGGTGGAGACTATCAGCCCGGGCGACGGACGGACATTCCCAAAGCGCGG
GCAGACGTGTGTGGTGCATTACACCGGGATGCTTGAGGACGGAAAGAAAGTGGACTCTTCCCGAGA
CCGAAATAAACCATTCAAGTTCATGTTGGGCAAGCAGGAGGTTATCAGAGGGTGGGAGGAGGGCGT
CGCTCAGATGAGTGTCGGACAGAGGGCCAAGCTCACGATCTCCCCTGATTACGCCTACGGGGCAAC
TGGTCACCCCGGAATCATCCCCCCTCACGCAACCCTCGTGTTCGACGTCGAGCTGCTCAAACTGGA
ATCAGGCGGAGGCAGTGGCGCTAGCGGGTTTGGCGATGTCGGTGCCCTTGAAAGCTTGAGAGGAA
ATGCCGATCTCGCTTACATCTTGAGCATGGAGCCCTGTGGGCACTGTCTGATCATCAACAATGTTAA
CTTTTGCCGGGAGTCCGGCCTGCGCACACGCACAGGCTCCAACATTGACTGCGAAAAACTTCGAAG
GAGGTTTAGCTCTCTGCATTTCATGGTAGAGGTGAAGGGGGATCTGACCGCCAAGAAAATGGTTCTC
GCCCTTCTCGAGCTTGCGCAGCAGGACCATGGAGCGCTTGACTGTTGTGTCGTTGTGATACTGAGC
CATGGCTGTCAGGCTTCCCATCTCCAGTTTCCAGGGGCCGTGTACGGAACCGATGGATGCCCTGTG
TCAGTTGAAAAGATCGTAAACATCTTTAACGGAACATCTTGCCCGAGCCTCGGCGGTAAACCGAAGC
TTTTTTTTATCCAGGCCTGCGGCGGTGAACAGAAAGATCATGGCTTCGAGGTTGCCAGTACCAGCCC
TGAAGACGAATCCCCCGGGTCAAATCCTGAACCAGATGCGACCCCTTTCCAGGAAGGACTCCGCAC
TTTTGACCAGCTTGACGCCATTTCCTCCCTGCCAACACCTTCCGACATATTTGTAAGCTACTCCACCT
TTCCAGGATTCGTGAGCTGGCGCGACCCAAAATCCGGCAGTTGGTATGTTGAAACCCTGGACGATA
TTTTCGAACAATGGGCCCACAGTGAGGACCTGCAGTCCCTTCTTCTGCGCGTAGCCAATGCCGTGT
CAGTCAAAGGGATTTACAAGCAGATGCCAGGCTGCTTTAATTTCCTGCGCAAGAAACTGTTTTTTAAG
ACCAGT or SEQ ID NO: 3001;

CGGACGGACATTCCCAAAGCGCGGGCAGACGTGTGTGGTGCATTACACCGGGATGCTTGAGGACG
GAAAGAAAGTGGACTCTTCCCGAGACCGAAATAAACCATTCAAGTTCATGTTGGGCAAGCAGGAGGT
TATCAGAGGGTGGGAGGAGGGCGTCGCTCAGATGAGTGTCGGACAGAGGGCCAAGCTCACGATCT
CCCCTGATTACGCCTACGGGGCAACTGGTCACCCCGGAATCATCCCCCCTCACGCAACCCTCGTGT
TCGACGTCGAGCTGCTCAAACTGGAATCAGGCGGAGGCAGTGGCGGGTTTGGCGATGTCGGTGCC
CTTGAAAGCTTGAGAGGAAATGCCGATCTCGCTTACATCTTGAGCATGGAGCCCTGTGGGCACTGTC
TGATCATCAACAATGTTAACTTTTGCCGGGAGTCCGGCCTGCGCACACGCACAGGCTCCAACATTGA
CTGCGAAAAACTTCGAAGGAGGTTTAGCTCTCTGCATTTCATGGTAGAGGTGAAGGGGGATCTGACC
GCCAAGAAAATGGTTCTCGCCCTTCTCGAGCTTGCGCAGCAGGACCATGGAGCGCTTGACTGTTGT
GTCGTTGTGATACTGAGCCATGGCTGTCAGGCTTCCCATCTCCAGTTTCCAGGGGCCGTGTACGGA
ACCGATGGATGCCCTGTGTCAGTTGAAAAGATCGTAAACATCTTTAACGGAACATCTTGCCCGAGCC
TCGGCGGTAAACCGAAGCTTTTTTTTATCCAGGCCTGCGGCGGTGAACAGAAAGATCATGGCTTCGA
GGTTGCCAGTACCAGCCCTGAAGACGAATCCCCCGGGTCAAATCCTGAACCAGATGCGACCCCTTT
CCAGGAAGGACTCCGCACTTTTGACCAGCTTGACGCCATTTCCTCCCTGCCAACACCTTCCGACATA
TTTGTAAGCTACTCCACCTTTCCAGGATTCGTGAGCTGGCGCGACCCAAAATCCGGCAGTTGGTATG

TTGAAACCCTGGACGATATCTTCGAACAATGGGCCCACAGTGAGGACCTGCAGTCCCTTCTTCTGCG
CGTAGCCAATGCCGTGTCAGTCAAAGGGATTTACAAGCAGATGCCAGGCTGCTTTAATTTCCTGCGC
AAGAAACTGTTTTTTAAGACCAGTTGAGTCGACGGAGGAGGAG or SEQ ID NO: 3002;

GGGGTTCAGGTGGAGACTATCAGCCCGGGCGACGGACGGACATTCCCAAAGCGCGGGCAGACGTG
TGTGGTGCATTACACCGGGATGCTTGAGGACGGAAAGAAAGTGGACTCTTCCCGAGACCGAAATAA
ACCATTCAAGTTCATGTTGGGCAAGCAGGAGGTTATCAGAGGGTGGGAGGAGGGCGTCGCTCAGAT
GAGTGTCGGACAGAGGGCCAAGCTCACGATCTCCCCTGATTACGCCTACGGGGCAACTGGTCACCC
CGGAATCATCCCCCCTCACGCAACCCTCGTGTTCGACGTCGAGCTGCTCAAACTGGAATCAGGCGG
AGGCAGTGGCGGGTTTGGCGATGTCGGTGCCCTTGAAAGCTTGAGAGGAAATGCCGATCTCGCTTA
CATCTTGAGCATGGAGCCCTGTGGGCACTGTCTGATCATCAACAATGTTAACTTTTGCCGGGAGTCC
GGCCTGCGCACACGCACAGGCTCCAACATTGACTGCGAAAAACTTCGAAGGAGGTTTAGCTCTCTG
CATTTCATGGTAGAGGTGAAGGGGGATCTGACCGCCAAGAAAATGGTTCTCGCCCTTCTCGAGCTT
GCGCAGCAGGACCATGGAGCGCTTGACTGTTGTGTCGTTGTGATACTGAGCCATGGCTGTCAGGCT
TCCCATCTCCAGTTTCCAGGGGCCGTGTACGGAACCGATGGATGCCCTGTGTCAGTTGAAAAGATC
GTAAACATCTTTAACGGAACATCTTGCCCGAGCCTCGGCGGTAAACCGAAGCTTTTTTTTATCCAGG
CCTGCGGCGGTGAACAGAAAGATCATGGCTTCGAGGTTGCCAGTACCAGCCCTGAAGACGAATCCC
CCGGGTCAAATCCTGAACCAGATGCGACCCCTTTCCAGGAAGGACTCCGCACTTTTGACCAGCTTG
ACGCCATTTCCTCCCTGCCAACACCTTCCGACATATTTGTAAGCTACTCCACCTTTCCAGGATTCGTG
AGCTGGCGCGACCCAAAATCCGGCAGTTGGTATGTTGAAACCCTGGACGATATCTTCGAACAATGG
GCCCACAGTGAGGACCTGCAGTCCCTTCTTCTGCGCGTAGCCAATGCCGTGTCAGTCAAAGGGATT
TACAAGCAGATGCCAGGCTGCTTTAATTTCCTGCGCAAGAAACTGTTTTTTAAGACCAGTTGA or SEQ
ID NO: 3003; and

AGAGGCGTGCAGGTGGAAACCATCTCTCCCGGCGACGGCAGAACCTTCCCTAAGAGGGGCCAGAC
CTGCGTGGTGCACTACACCGGCATGCTGGAAGATGGCAAGAAGATGGACAGCTCCCGGGACCGGA
ACAAGCCCTTCAAGTTCATGCTGGGCAAGCAGGAAGTGATCCGGGGCTGGGAAGAGGGCGTGGCA
CAGATGTCTGTGGGCCAGAGAGCCAAGCTGACCATCAGCCCCGATTACGCCTACGGCGCCACAGG
CCACCCTGGCATCATTCCTCCACACGCCACACTGGTGTTCGATGTGGAACTGCTGAAGCTGGAAAC
CCGGGGAGTGCAGGTGGAAACAATCAGCCCTGGCGACGGCCGGACCTTTCCAAAACGGGGACAGA
CATGTGTGGTGCATTATACAGGGATGCTGGAAGATGGAAAAAATGGATAGCAGCCGCGACCGCA
ACAAACCTTTTAAGTTTATGCTGGGGAAACAGGAAGTGATTAGAGGCTGGGAAGAGGGGGTGGCAC
AGATGAGCGTGGGACAGCGGGCCAAACTGACAATCTCCCCCGACTATGCCTATGGGGCCACCGGA
CACCCCGGAATCATCCCACCTCATGCTACCCTGGTGTTTGACGTGGAACTGCTGAAACTGGAAACAA
GCGGCGGAGGCAGCGGCGGCTTTGGAGATGTGGGAGCCCTGGAAAGCCTGCGGGGCAATGCCGA
TCTGGCCTACATCCTGAGCATGGAACCCTGCGGCCACTGCCTGATTATCAACAACGTGAACTTCTGC
AGAGAGAGCGGCCTGCGGACCAGAACCGGCAGCAACATCGACTGCGAGAAGCTGCGGCGGAGATT
CAGCAGCCTGCACTTCATGGTGGAAGTGAAGGGGGACCTGACCGCCAAGAAAATGGTGCTGGCTCT
GCTGGAACTGGCCCAGCAGGATCATGGCGCCCTGGACTGTTGCGTGGTCGTGATCCTGAGCCACG

GCTGCCAGGCCAGCCATCTGCAGTTTCCCGGCGCTGTGTATGGCACCGATGGCTGCCCTGTGTCC
GTGGAAAAGATCGTGAATATCTTCAACGGCACCAGCTGCCCCAGCCTGGGCGGAAAGCCTAAGCTG
TTCTTTATTCAAGCCTGTGGGGGCGAGCAGAAGGACCACGGATTTGAGGTGGCCAGCACCTCCCCC
GAGGATGAGAGCCCTGGCAGCAACCCTGAGCCTGACGCCACCCCATTCCAGGAAGGACTGCGGAC
CTTCGACCAGCTGGACGCCATCTCTAGCCTGCCCACCCCCAGCGACATCTTCGTGTCCTACAGCAC
CTTCCCTGGCTTTGTGTCCTGGCGGGACCCCAAGTCCGGCTCTTGGTACGTGGAAACCCTGGACGA
CATCTTTGAGCAGTGGGCCCATAGCGAGGACCTGCAGAGCCTGCTGCTGAGGGTGGCCAATGCCG
TGTCCGTGAAGGGCATCTACAAGCAGATGCCCGGCTGCTTCAACTTCCTGCGGAAGAAGCTGTTTTT
CAAGACCAGC or SEQ ID NO: 3004.

In another instance, the suicide gene encodes an amino acid sequence selected from the group consisting of MLEGVQVETISPGDGRTFPKRGQTCVVHYTGMLEDGKKVDSSRDRNKPFKFMLGKQEVIRGWEEGVAQ MSVGQRAKLTISPDYAYGATGHPGIIPPHATLVFDVELLKLESGGGSGASGFGDVGALESLRGNADLAYIL SMEPCGHCLIINNVNFCRESGLRTRTGSNIDCEKLRRRFSSLHFMVEVKGDLTAKKMVLALLELAQQDHG ALDCCVVVILSHGCQASHLQFPGAVYGTDGCPVSVEKIVNIFNGTSCPSLGGKPKLFFIQACGGEQKDHG FEVASTSPEDESPGSNPEPDATPFQEGLRTFDQLDAISSLPTPSDIFVSYSTFPGFVSWRDPKSGSWYVE TLDDIFEQWAHSEDLQSLLLRVANAVSVKGIYKQMPGCFNFLRKKLFFKTS or SEQ ID NO: 3005; GVQVETISPGDGRTFPKRGQTCVVHYTGMLEDGKKVDSSRDRNKPFKFMLGKQEVIRGWEEGVAQMS VGQRAKLTISPDYAYGATGHPGIIPPHATLVFDVELLKLESGGGSGGFGDVGALESLRGNADLAYILSMEP CGHCLIINNVNFCRESGLRTRTGSNIDCEKLRRRFSSLHFMVEVKGDLTAKKMVLALLELAQQDHGALDC CVVVILSHGCQASHLQFPGAVYGTDGCPVSVEKIVNIFNGTSCPSLGGKPKLFFIQACGGEQKDHGFEVA STSPEDESPGSNPEPDATPFQEGLRTFDQLDAISSLPTPSDIFVSYSTFPGFVSWRDPKSGSWYVETLDD IFEQWAHSEDLQSLLLRVANAVSVKGIYKQMPGCFNFLRKKLFFKTS or SEQ ID NO: 3006; and RGVQVETISPGDGRTFPKRGQTCVVHYTGMLEDGKKMDSSRDRNKPFKFMLGKQEVIRGWEEGVAQM SVGQRAKLTISPDYAYGATGHPGIIPPHATLVFDVELLKLETRGVQVETISPGDGRTFPKRGQTCVVHYTG MLEDGKKMDSSRDRNKPFKFMLGKQEVIRGWEEGVAQMSVGQRAKLTISPDYAYGATGHPGIIPPHATL VFDVELLKLETSGGGSGGFGDVGALESLRGNADLAYILSMEPCGHCLIINNVNFCRESGLRTRTGSNIDC EKLRRRFSSLHFMVEVKGDLTAKKMVLALLELAQQDHGALDCCVVVILSHGCQASHLQFPGAVYGTDGC PVSVEKIVNIFNGTSCPSLGGKPKLFFIQACGGEQKDHGFEVASTSPEDESPGSNPEPDATPFQEGLRTF DQLDAISSLPTPSDIFVSYSTFPGFVSWRDPKSGSWYVETLDDIFEQWAHSEDLQSLLLRVANAVSVKGIY KQMPGCFNFLRKKLFFKTS or SEQ ID NO: 3007. In yet another instance, the suicide gene has an inducible promoter.

In some instances, the suicide gene is in an expression vector. In an exemplary instance, the present disclosure includes a vector comprising a nucleic acid sequence comprising a suicide gene comprising SEQ ID NOs: 3001, 3002, 3003 or 3004. The expression vector may also include other genes, such as a chimeric antigen receptor and/or CRISPR system disclosed elsewhere herein.

Also disclosed herein but not forming part of the claimed invention is a cell comprising the suicide gene. In an exemplary aspect, the present disclosure includes a modified cell comprising a nucleic acid comprising a suicide gene encoding an amino acid sequence selected from the group consisting of SEQ ID NO: 3005, 3006, and 3007 and a nucleic acid encoding a chimeric antigen receptor. In another aspect, the present disclosure includes a modified cell comprising a nucleic acid comprising a suicide gene selected from the group consisting of SEQ ID NO: 3001, 3002, 3003 and 3004 and a nucleic acid encoding a chimeric antigen receptor. In one instance, the suicide gene encodes an amino acid sequence selected from the group consisting of SEQ ID NO: 3005, 3006, and 3007. In another instance, the suicide gene has an inducible promoter.

In one instance, the CAR modified T cell comprises nucleic acids encoding a suicide gene as a separate nucleic acid sequence from the CAR construct described elsewhere herein. For example, HSV-TK, i-Casp9, the cytoplasmic domain of Fas, or a caspase can be incorporated into genetically engineered T cells separate from the CAR construct. In another instance, the CAR modified T cell comprises a suicide gene in the same construct as the nucleic acids encoding the CAR. In this instance, the nucleic acids comprising the suicide gene may be upstream or downstream of the nucleic acids encoding the CAR.

In one instance, expression of the suicide gene is activated in the cell by contacting the cell with an inducing agent administered to the cell or to a mammal comprising the cell. The inducing agent then activates an inducible promoter to express the suicide gene. In such an instance, the inducing agent is administered to the subject to induce expression of the suicide gene.

In another instance, a suicide gene product that is expressed from the suicide gene is activated by an activating agent, such as a dimerization agent. For example, the dimerization agent, such as AP20187, promotes dimerization and activation of caspase-9 molecules.

In some instances with constitutive expression of the suicide gene, expression of the suicide gene may be turned off in the cell by contacting the cell with an inhibiting agent administered to the cell or to a mammal comprising the cell. The inhibiting agent selectively turns off expression. For example, caspase-9 is constitutively expressed in the cell and the addition of an inhibiting agent represses expression or activation of caspase-9. In one instance, the inhibiting agent is administered to the subject to repress expression of the suicide gene.

In some instances with constitutive expression of the suicide gene, activation of the suicide gene product may be repressed in the cell by contacting the cell with an inhibiting agent, such as a solubilizing agent, administered to the cell or to a mammal comprising the cell. The inhibiting agent represses activation of the suicide gene product, such as by preventing dimerization of the caspase-9 molecules. In one instance, the solubilizing agent is administered to the subject to repress activation of the suicide gene product.

In some aspects, the suicide gene is not immunogenic to the cell comprising the suicide gene or host harboring the suicide gene. Although thymidine kinase (TK) may be employed, it can be immunogenic. Alternatively, examples of suicide genes that are not immunogenic to the host include caspase-9, caspase-8, and cytosine deaminase.

In yet another instance, suicide gene expression is linked in tandem to one or more dimerization domains, which cause aggregation of the fusion protein containing the suicide domain and the dimerization domain, preventing cell-surface expression and hence function of the suicide gene.

In yet another instance, the nucleic acid sequence encoding a dimerization domain, linked to a suicide gene, comprises the nucleic acid sequence selected from the group consisting of 
GGAAGCGGCGCCACCAATTTCAGCCTGCTGAAACAGGCCGGCGACGTGGAAGAGAACCCTGGCCC
T or SEQ ID NO: 3008;
 
AGTGGCTCCGGCGCAACAAATTTCTCCTTGCTGAAACAGGCAGGCGACGTTGAGGAAAATCCCGGC
CCA or SEQ ID NO: 3009;
 
GGCTCCGGCGCAACAAATTTCTCCTTGCTGAAACAGGCAGGCGACGTTGAGGAAAATCCCGGCCCA
or SEQ ID NO: 3010; GGGGTTCAGGTGGAGACTATCAGCCCGGGCGA or SEQ ID NO: 3011; and
 GGCTCCGGCGCAACAAATTTCTCCTTGCTGAAACAGGCAGGCGACGTTGAGGAAAATCCCGGCCCA or SEQ ID NO: 3012. In another such instance, the dimerization domain, linked to a suicide domain, comprises the amino acid sequence selected from the group consisting of
GSGATNFSLLKQAGDVEENPGP or SEQ ID NO: 3013; and RKRRGSGATNFSLLKQAGDVEENPGP or SEQ ID NO: 3014.

Solubilization of the dimerization domains, with a solubilizing agent, administered to the cell or to a mammal comprising the cell, prevents aggregation and allows the construct to egress through the secretory system.

Also disclosed herein but not forming part of the claimed invention is a method of modifying a T cell with a chimeric antigen receptor (CAR) and a suicide gene. Thus, the present disclosure encompasses a nucleic acid encoding a CAR or a modified T cell comprising a CAR, wherein the CAR includes an antigen binding domain, a transmembrane domain and an intracellular domain.

One or more domains or a fragment of a domain of the CAR may be human. In one instance, the present disclosure includes a fully human CAR. The nucleic acid sequences coding for the desired domains can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than as a cloned molecule.

Example of CARs are described in U.S. Patent Nos.: 8,911,993, 8,906,682, 8,975,071, 8,916,381, 9,102,760, 9,101,584, and 9,102,761.

### NUCLEIC ACID AND VECTORS

In another aspect, the invention pertains to a nucleic acid encoding any of the fusion proteins of the invention, or a vector comprising such a nucleic acid. In one embodiment, the vector is chosen from a DNA vector, an RNA vector, a plasmid, a lentivirus vector, adenoviral vector, or a retrovirus vector. In one embodiment, the vector is a lentivirus vector.

In some embodiments, the nucleic acids of the invention include a sequence encoding a furin cleavage site. In some embodiments, the nucleic acids of the invention include any one of SEQ ID NOs: 1112, 126, 128, 130, 132, 134, 136, or 138, or a sequence having at least 90%, 95%, 97%, 98%, or 99% identity thereto. In some embodiments, the nucleic acids of the invention include any one of SEQ ID NOs: 1112, 126, 128, 130, 132, 134, 136, or 138. In some embodiments, the nucleic acids of the invention include SEQ ID NO: 126 or SEQ ID NO: 128. In some embodiments, the nucleic acids of the invention include SEQ ID NO: 126.

In some instances, the nucleic acids described herein include a sequence encoding a conditional expression domain, e.g., an aggregation domain or a degradation domain. In some instances of the disclosure which do not form part of the claimed invention, the nucleic acids described herein include a sequence encoding an aggregation domain derived from FKBP, e.g., FKBP F36M. In the present invention, the nucleic acids described herein include a sequence encoding a degradation domain derived from an estrogen receptor (ER). In some embodiments, the nucleic acids described herein include SEQ ID NO: 1110 or SEQ ID NO: 122, or a sequence having at least 90%, 95%, 97%, 98%, or 99% identity thereto. In some embodiments, the nucleic acids described herein include SEQ ID NO: 1110 or SEQ ID NO: 122. In some embodiments, the nucleic acids described herein include SEQ ID NO: 122.

The present disclosure also provides vectors in which a DNA of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713.

In another embodiment, the vector comprising the nucleic acid encoding the desired fusion protein of the invention is an adenoviral vector (A5/35). In another embodiment, the expression of nucleic acids encoding chimeric molecules can be accomplished using of transposons such as sleeping beauty, crisper, CAS9, and zinc finger nucleases. See below June et al. 2009 Nature Reviews Immunology 9.10: 704-716.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Disclosed herein are methods for producing an in vitro transcribed RNA chimeric molecule. The present disclosurealso includes a chimeric molecule encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-5000 bases in length (SEQ ID NO:32). RNA so produced can efficiently transfect different kinds of cells. In one aspect, the template includes sequences for the CAR.

### Nucleic Acid Constructs Encoding a fusion protein comprising a CAR

The present disclosure also provides nucleic acid molecules encoding a fusion protein, e.g., as described herein, comprising a domain that includes one or more of the CAR constructs targeting an antigen described herein. In one aspect, the nucleic acid molecule is provided as a messenger RNA transcript. In one aspect, the nucleic acid molecule is provided as a DNA construct.

Accordingly, in one aspect, the invention pertains to a nucleic acid molecule encoding a fusion protein comprising a chimeric antigen receptor (CAR), wherein the CAR comprises an antigen binding domain that binds to an antigen described herein, a transmembrane domain (e.g., a transmembrane domain described herein), and an intracellular signaling domain (e.g., an intracellular signaling domain described herein) comprising a stimulatory domain, e.g., a costimulatory signaling domain (e.g., a costimulatory signaling domain described herein) and/or a primary signaling domain (e.g., a primary signaling domain described herein, e.g., a zeta chain described herein). In one embodiment, the transmembrane domain is transmembrane domain of a protein selected from the group consisting of the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 and CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp , NKG2D, and NKG2C.

In one embodiment, the transmembrane domain comprises a sequence of SEQ ID NO: 12, or a sequence with 95-99% identity thereof. In one embodiment, the antigen binding domain is connected to the transmembrane domain by a hinge region, e.g., a hinge described herein. In one embodiment, the hinge region comprises SEQ ID NO:4 or SEQ ID NO:6 or SEQ ID NO:8 or SEQ ID NO:10, or a sequence with 95-99% identity thereof. In one embodiment, the isolated nucleic acid molecule further comprises a sequence encoding a costimulatory domain. In one embodiment, the costimulatory domain is a functional signaling domain of a protein selected from the group consisting of OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKG2D, and NKG2C. In one embodiment, the intracellular signaling domain comprises a functional signaling domain of 4-1BB and a functional signaling domain of CD3 zeta. In one embodiment, the intracellular signaling domain comprises the sequence of SEQ ID NO: 14, 16, 120, or 124, or a sequence with 95-99% identity thereof, and the sequence of SEQ ID NO: 18 or SEQ ID NO:20, or a sequence with 95-99% identity thereof, wherein the sequences comprising the intracellular signaling domain are expressed in the same frame and as a single polypeptide chain.

In another aspect, the invention pertains to an isolated nucleic acid molecule encoding a fusion protein comprising a domain that includes a CAR construct comprising a leader sequence of SEQ ID NO: 2, a scFv domain as described herein, a hinge region of SEQ ID NO:4 or SEQ ID NO:6 or SEQ ID NO:8 or SEQ ID NO:10 (or a sequence with 95-99% identity thereof), a transmembrane domain having a sequence of SEQ ID NO: 12 (or a sequence with 95-99% identity thereof), a 4-1BB costimulatory domain having a sequence of SEQ ID NO:14, a CD27 costimulatory domain having a sequence of SEQ ID NO:16 (or a sequence with 95-99% identity thereof), a ICOS costimulatory domain having a sequence of SEQ ID NO: 120 (or a sequence with 95-99% identity thereof) or a CD28 costimulatory domain having a sequence of SEQ ID NO:124, and a CD3 zeta stimulatory domain having a sequence of SEQ ID NO:18 or SEQ ID NO:20 (or a sequence with 95-99% identity thereof).

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present disclosure also provides vectors in which a nucleic acid of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

In another embodiment, the vector comprising the nucleic acid encoding a fusion protein comprising a domain that includes aCAR of the invention is an adenoviral vector (A5/35). In another embodiment, the expression of nucleic acids encoding CARs can be accomplished using of transposons such as sleeping beauty, crispr/CAS9, and zinc finger nucleases. See below June et al. 2009Nature Reviews Immunology 9.10: 704-716.

In brief summary, the expression of natural or synthetic nucleic acids encoding a fusion protein, e.g., as described herein, comprising a domain that includes CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The expression constructs of the present disclosure may also be used for nucleic acid immunization and gene therapy, using standard gene delivery protocols. Methods for gene delivery are known in the art. See, e.g., U.S. Pat. Nos. 5,399,346, 5,580,859, 5,589,466. In another embodiment, the invention provides a gene therapy vector.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno- associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. Exemplary promoters include the CMV IE gene, EF-1α, ubiquitin C, or phosphoglycerokinase (PGK) promoters.

An example of a promoter that is capable of expressing a fusion protein, e.g., as described herein, comprising a domain that includes aCAR encoding nucleic acid molecule in a mammalian T cell is the EF1a promoter. The native EF1a promoter drives expression of the alpha subunit of the elongation factor-1 complex, which is responsible for the enzymatic delivery of aminoacyl tRNAs to the ribosome. The EF1a promoter has been extensively used in mammalian expression plasmids and has been shown to be effective in driving expression of a fusion protein, e.g., as described herein, a fusion protein comprising a domain that includes a CAR, from nucleic acid molecules cloned into a lentiviral vector. See, e.g., Milone et al., Mol. Ther. 17(8): 1453-1464 (2009). In one aspect, the EF1a promoter comprises the sequence provided as SEQ ID NO:1.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1□promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Another example of a promoter is the phosphoglycerate kinase (PGK) promoter. In embodiments, a truncated PGK promoter (e.g., a PGK promoter with one or more, e.g., 1, 2, 5, 10, 100, 200, 300, or 400, nucleotide deletions when compared to the wild-type PGK promoter sequence) may be desired. The nucleotide sequences of exemplary PGK promoters are provided below.

### WT PGK Promoter

ACCCCTCTCTCCAGCCACTAAGCCAGTTGCTCCCTCGGCTGACGGCTGCACGCGAGGCCTCCGAAC
GTCTTACGCCTTGTGGCGCGCCCGTCCTTGTCCCGGGTGTGATGGCGGGGTGTGGGGCGGAGGG
CGTGGCGGGGAAGGGCCGGCGACGAGAGCCGCGCGGGACGACTCGTCGGCGATAACCGGTGTCG
GGTAGCGCCAGCCGCGCGACGGTAACGAGGGACCGCGACAGGCAGACGCTCCCATGATCACTCTG
CACGCCGAAGGCAAATAGTGCAGGCCGTGCGGCGCTTGGCGTTCCTTGGAAGGGCTGAATCCCCG
CCTCGTCCTTCGCAGCGGCCCCCCGGGTGTTCCCATCGCCGCTTCTAGGCCCACTGCGACGCTTG
CCTGCACTTCTTACACGCTCTGGGTCCCAGCCGCGGCGACGCAAAGGGCCTTGGTGCGGGTCTCG
TCGGCGCAGGGACGCGTTTGGGTCCCGACGGAACCTTTTCCGCGTTGGGGTTGGGGCACCATAAG
CT(SEQ ID NO: 185)

Exemplary truncated PGK Promoters:
PGK100: 
ACCCCTCTCTCCAGCCACTAAGCCAGTTGCTCCCTCGGCTGACGGCTGCACGCGAGGCCTCCGAAC
GTCTTACGCCTTGTGGCGCGCCCGTCCTTGTCCCGGGTGTGATGGCGGGGTG (SEQ ID NO: 186)
PGK200: 
ACCCCTCTCTCCAGCCACTAAGCCAGTTGCTCCCTCGGCTGACGGCTGCACGCGAGGCCTCCGAAC
GTCTTACGCCTTGTGGCGCGCCCGTCCTTGTCCCGGGTGTGATGGCGGGGTGTGGGGCGGAGGG
CGTGGCGGGGAAGGGCCGGCGACGAGAGCCGCGCGGGACGACTCGTCGGCGATAACCGGTGTCG
GGTAGCGCCAGCCGCGCGACGGTAACG (SEQ ID NO: 187)
PGK300: 
ACCCCTCTCTCCAGCCACTAAGCCAGTTGCTCCCTCGGCTGACGGCTGCACGCGAGGCCTCCGAAC
GTCTTACGCCTTGTGGCGCGCCCGTCCTTGTCCCGGGTGTGATGGCGGGGTGTGGGGCGGAGGG
CGTGGCGGGGAAGGGCCGGCGACGAGAGCCGCGCGGGACGACTCGTCGGCGATAACCGGTGTCG
GGTAGCGCCAGCCGCGCGACGGTAACGAGGGACCGCGACAGGCAGACGCTCCCATGATCACTCTG
CACGCCGAAGGCAAATAGTGCAGGCCGTGCGGCGCTTGGCGTTCCTTGGAAGGGCTGAATCCCCG
(SEQ ID NO: 188)
PGK400:

ACCCCTCTCTCCAGCCACTAAGCCAGTTGCTCCCTCGGCTGACGGCTGCACGCGAGGCCTCCGAAC
GTCTTACGCCTTGTGGCGCGCCCGTCCTTGTCCCGGGTGTGATGGCGGGGTGTGGGGCGGAGGG
CGTGGCGGGGAAGGGCCGGCGACGAGAGCCGCGCGGGACGACTCGTCGGCGATAACCGGTGTCG
GGTAGCGCCAGCCGCGCGACGGTAACGAGGGACCGCGACAGGCAGACGCTCCCATGATCACTCTG
CACGCCGAAGGCAAATAGTGCAGGCCGTGCGGCGCTTGGCGTTCCTTGGAAGGGCTGAATCCCCG
CCTCGTCCTTCGCAGCGGCCCCCCGGGTGTTCCCATCGCCGCTTCTAGGCCCACTGCGACGCTTG
CCTGCACTTCTTACACGCTCTGGGTCCCAGCCG (SEQ ID NO: 189)

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a fusion protein, e.g., as described herein, comprising a domain that includes a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

In some embodiments, the a vector comprising a nucleic acid sequence encoding a fusion protein of the invention, e.g., a fusion protein comprising a CAR molecule described herein, can further comprise a second nucleic acid sequence encoding a polypeptide, e.g., an agent that increases the activity of thefusion protein, e.g., as described herein, comprising a domain that includes CAR molecule. In some embodiments a single nucleic acid molecule, or vector comprising said nucleic acid molecule, encodes multiple fusion proteins, each comprising domains that include a CAR, described herein. In some embodiments, the nucleic acid encoding the a first fusion protein is under separate regulatory control (e.g., by a promoter described herein) from the nucleic acid endocing a second fusion protein (e.g., by a promoter described herein). In other embodiments, the two or more nucleic acid sequences are encoded by a single nucleic molecule in the same frame and as a single polypeptide chain. In this aspect, the two or more fusion proteins, each comprising a domain that includes a CAR, can, e.g., be separated by one or more peptide cleavage sites (e.g., an auto-cleavage site or a substrate for an intracellular protease). Examples of peptide cleavage sites include the following, wherein the GSG residues are optional:

| | |
|---|---|
| T2A: | (GSG) E G R G S L L T C G D V E E N P G P (SEQ ID NO: 190) |
| P2A: | (GSG) A T N F S L L K Q A G D V E E N P G P (SEQ ID NO: 191) |
| E2A: | (GSG) Q C T N Y A L L K L A G D V E S N P G P (SEQ ID NO: 192) |
| F2A: | (GSG) V K Q T L N F D L L K L A G D V E S N P G P (SEQ ID NO: 193) |

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A preferred method for the introduction of a polynucleotide into a host cell is calcium phosphate transfection or electroporation.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about -20ºC. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present disclosure, in order to confirm the presence of the recombinant DNA sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

The present disclosure further provides a vector comprising a fusion protein comprising a domain that includes a CAR, -encoding nucleic acid molecule. In one embodiment, the vector comprises a CAR encoding nucleic acid molecule, e.g., as described herein. In one embodiment, the vector comprises twoCAR encoding nucleic acid molecules. In one aspect, the one or more CAR vectors (e.g., the vector comprising a firstCAR encoding nucleic acid molecule and the vector comprising a second CAR encoding nucleic acid molecule, or the vector comprising both a first and second CAR encoding nucleic acids) can be directly transduced into a cell, *e.g.,* a T cell or a NK cell. In one aspect, the vector is a cloning or expression vector, *e.g.,* a vector including, but not limited to, one or more plasmids (*e.g.,* expression plasmids, cloning vectors, minicircles, minivectors, double minute chromosomes), retroviral and lentiviral vector constructs. In one aspect, the vector is capable of expressing the CAR construct in mammalian immune effector cells (e.g., T cells, NK cells).

In one embodiment, where stable expression of one or more (e.g., one or two)fusion proteins, each comprising a domain that includes a CAR, is desired, a vector comprising one or more (e.g., one or two) CAR-encoding nucleic acid molecules is transduced into an immune effector cell. For example, immune effector cells with stable expression of two fusion proteins, each comprising a domain that include a CAR, can be generated using lentiviral vectors. Cells that exhibit stable expression of a two fusion proteins, each comprising a domain that includes a CAR, express the CARs for at least 1 week, 2 weeks, 3 weeks, 4 weeks, 5 weeks, 6 weeks, 7 weeks, 8 weeks, 3 months, 6 months, 9 months, or 12 months after transduction.

In one embodiment, where transient expression of one or more (e.g., one or two) fusion proteins comprising a domain that includes a CAR is desired, one or more (e.g., one or two)a fusion protein - encoding nucleic acid molecules is transfected into an immune effector cell. The one or more (e.g., one or two) fusion proteins comprising a domain that includes a CAR, -encoding nucleic acid molecules may be a vector comprising a one or more (e.g., one or two) CAR encoding nucleic acid molecules, or an in vitro transcribed RNA one or more (e.g., one or two) CARs. In vitro transcribed RNA CARs and methods for transfection into immune effector cells are further described below. Cells that exhibit transient expression of a one or more (e.g., one or two) CAR express the one or more (e.g., one or two) CAR for 4, 5, 6, 7, 8, 9, 10, 11, 12, 13, 14, 15 days after transfection.

### RNA Transfection

Disclosed herein are methods for producing an in vitro transcribed RNA encoding a fusion protein, e.g., as described herein, comprising a domain that includes a CAR. The present disclosure also includes a fusion protein, e.g., as described herein, comprising a domain that includes CAR, -encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-5000 bases in length (SEQ ID NO: 32). RNA so produced can efficiently transfect different kinds of cells. In one aspect, the template includes sequences for the CAR.

In one aspect, a fusion protein, e.g., as described herein, comprising a domain that includes a CAR, of the present disclosure is encoded by a messenger RNA (mRNA). In one aspect, the mRNA encoding a CAR described herein is introduced into a T cell or a NK cell.

In one embodiment, the *in vitro* transcribed RNA encoding a fusion protein comprising a domain that includes a CAR can be introduced to a cell as a form of transient transfection. The RNA is produced by *in vitro* transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired template for *in vitro* transcription is a fusion protein, e.g., as described herein, comprising a domain that includes a CAR,described herein. For example, the template for the RNA CAR comprises an extracellular region comprising a single chain variable domain of an antibody to an antigen described herein; a hinge region (e.g., a hinge region described herein), a transmembrane domain (e.g., a transmembrane domain described herein such as a transmembrane domain of CD8a); and a cytoplasmic region that includes an intracellular signaling domain, e.g., an intracellular signaling domain described herein, e.g., comprising the signaling domain of CD3-zeta and the signaling domain of 4-1BB.

In one embodiment, the DNA to be used for PCR contains an open reading frame. The DNA can be from a naturally occurring DNA sequence from the genome of an organism. In one embodiment, the nucleic acid can include some or all of the 5' and/or 3' untranslated regions (UTRs). The nucleic acid can include exons and introns. In one embodiment, the DNA to be used for PCR is a human nucleic acid sequence. In another embodiment, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one embodiment, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyAlT segment of the transcriptional DNA template can be produced during PCR by using a reverse primer containing a polyT tail, such as 100T tail (SEQ ID NO: 968) (size can be 50-5000 T (SEQ ID NO: 974)), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines (SEQ ID NO: 82).

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides (SEQ ID NO: 969) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### Non-viral delivery methods

In some aspects, non-viral methods can be used to deliver a nucleic acid encoding a chimeric molecule or fusion protein described herein into a cell or tissue or a subject.

In some embodiments, the non-viral method includes the use of a transposon (also called a transposable element). In some embodiments, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition.

In some embodiments, cells, e.g., T or NK cells, are generated that express a chimeric molecule or fusion protein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease re-engineered homing endonucleases).

In some embodiments, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### HOST CELLS

Also provided herein are cells, e.g., immune effector cells (e.g., a population of cells, e.g., a population of immune effector cells) comprising a nucleic acid molecule, a fusion protein molecule, or a vector, e.g., as described herein. In some embodiments, the provided cells comprise a fusion protein comprising a domain that includes a CAR, a nucleic acid molecule encoding a fusion protein comprising a domain that includes a CAR, or a vector comprising the same.

In certain aspects, immune effector cells, e.g., T cells or NK cells, can be obtained from a unit of blood collected from a subject using any number of techniques known to the skilled artisan, such as Ficoll^{™} separation. In one preferred aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi:10.1038/cti.2014.31.

In one aspect, T cells are isolated from peripheral blood lymphocytes by lysing the red blood cells and depleting the monocytes, for example, by centrifugation through a PERCOLL^{™} gradient or by counterflow centrifugal elutriation.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

In one embodiment, T regulatory cells, e.g., CD25+ T cells, are removed from the population using an anti-CD25 antibody, or fragment thereof, or a CD25-binding ligand, IL-2. In one embodiment, the anti-CD25 antibody, or fragment thereof, or CD25-binding ligand is conjugated to a substrate, e.g., a bead, or is otherwise coated on a substrate, e.g., a bead. In one embodiment, the anti-CD25 antibody, or fragment thereof, is conjugated to a substrate as described herein.

In one embodiment, the T regulatory cells, e.g., CD25+ T cells, are removed from the population using CD25 depletion reagent from Miltenyi^{™}. In one embodiment, the ratio of cells to CD25 depletion reagent is 1e7 cells to 20 uL, or 1e7 cells to15 uL, or 1e7 cells to 10 uL, or 1e7 cells to 5 uL, or 1e7 cells to 2.5 uL, or 1e7 cells to 1.25 uL. In one embodiment, e.g., for T regulatory cells, e.g., CD25+ depletion, greater than 500 million cells/ml is used. In a further aspect, a concentration of cells of 600, 700, 800, or 900 million cells/ml is used.

In one embodiment, the population of immune effector cells to be depleted includes about 6 x 10⁹ CD25+ T cells. In other aspects, the population of immune effector cells to be depleted include about 1 × 10⁹ to 1× 10¹⁰ CD25+ T cell, and any integer value in between. In one embodiment, the resulting population T regulatory depleted cells has 2 x 10⁹ T regulatory cells, e.g., CD25+ cells, or less (e.g., 1 × 10⁹, 5 × 10⁸, 1 × 10⁸, 5 × 10⁷, 1 × 10⁷, or less CD25+ cells).

In one embodiment, the T regulatory cells, e.g., CD25+ cells, are removed from the population using the CliniMAC system with a depletion tubing set, such as, e.g., tubing 162-01. In one embodiment, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a fusion protein, e.g., as described herein, comprising a domain that includes a CAR, -expressing cell product can reduce the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, and combinations thereof.

In some embodiments, the manufacturing methods comprise reducing the number of (e.g., depleting) T_{REG} cells prior to manufacturing of the fusion protein comprising a domain that includes a CAR, - expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an embodiment, a subject is pre-treated with one or more therapies that reduce T_{REG} cells prior to collection of cells for fusion protein, e.g., as described herein, comprising a domain that includes a CAR, - expressing cell product manufacturing, thereby reducing the risk of subject relapse to fusion protein, e.g., as described herein, comprising a domain that includes a CAR, -expressing cell treatment. In an embodiment, methods of decreasing T_{REG} cells include, but are not limited to, administration to the subject of one or more of cyclophosphamide, anti-GITR antibody, CD25-depletion, or a combination thereof. Administration of one or more of cyclophosphamide, anti-GITR antibody, CD25-depletion, or a combination thereof, can occur before, during or after an infusion of the CAR-expressing cell product.

In an embodiment, a subject is pre-treated with cyclophosphamide prior to collection of cells for CAR-expressing cell product manufacturing, thereby reducing the risk of subject relapse to fusion protein, e.g., as described herein, comprising a domain that includes a CAR, -expressing cell treatment. In an embodiment, a subject is pre-treated with an anti-GITR antibody prior to collection of cells for CAR-expressing cell product manufacturing, thereby reducing the risk of subject relapse to CAR-expressing cell treatment.

In one embodiment, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one embodiment, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a fusion protein, e.g., as described herein, comprising a domain that includes a CAR, e.g., a CAR described herein. In one embodiment, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other embodiments, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include B7-H1, B7-1, CD160, P1H, 2B4, PD1, TIM3, CEACAM (e.g., CEACAM-1, CEACAM-3 and/or CEACAM-5), LAG3, TIGIT, CTLA-4, BTLA and LAIR1. In one embodiment, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other embodiments, the removal of T regulatory cells, e.g., CD25+ cells, and the removal of the check point inhibitor expressing cells is sequential, and can occur, e.g., in either order.

Methods described herein can include a positive selection step. For example, T cells can isolated by incubation with anti-CD3/anti-CD28 (e.g., 3x28)-conjugated beads, such as DYNABEADS^{®} M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another embodiment, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one embodiment, a T cell population can be selected that expresses one or more of IFN-γ, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 × 10⁶/ml. In other aspects, the concentration used can be from about 1 × 10⁵/ml to 1 × 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present invention.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present invention, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present disclosureto collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one embodiment, the immune effector cells expressing a fusion protein comprising a domain that includes a CAR molecule, e.g., a CAR molecule described herein, are obtained from a subject that has received a low, immune enhancing dose of an mTOR inhibitor. In an embodiment, the population of immune effector cells, e.g., T cells, to be engineered to express a CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other embodiments, population of immune effector cells, e.g., T cells, which have, or will be engineered to express a fusion protein comprising a domain that includes a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an embodiment, the NK cells are obtained from the subject. In another embodiment, the NK cells are an NK cell line, e.g., NK-92 cell line (Conkwest).

### Additional Expressed Agents

In another embodiment, a fusion protein comprising a domain that includes a CAR, -expressing immune effector cell described herein can further express another agent, e.g., an agent which enhances the activity of a CAR-expressing cell. For example, in one embodiment, the agent can be an agent which inhibits an inhibitory molecule. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta, e.g., as described herein. In one embodiment, the agent that inhibits an inhibitory molecule comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta, or a fragment of any of these, and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28, CD27, OX40 or 4-IBB signaling domain described herein and/or a CD3 zeta signaling domain described herein).

In one embodiment, the fusion protein comprising a domain that includes a CAR, -expressing immune effector cell described herein can further comprise a second fusion protein, e.g., as described herein, comprising a domain that includes a CAR, e.g., a second CAR that includes a different antigen binding domain, e.g., to the same target (e.g., a target described above) or a different target. In one embodiment, the second CAR includes an antigen binding domain to a target expressed on the same cancer cell type as the target of the first CAR. In one embodiment, the CAR-expressing immune effector cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain.

While not wishing to be bound by theory, placement of a costimulatory signaling domain, e.g., 4-1BB, CD28, CD27 or OX-40, onto the first CAR, and the primary signaling domain, e.g., CD3 zeta, on the second CAR can limit the CAR activity to cells where both targets are expressed. In one embodiment, the CAR expressing immune effector cell comprises a first CAR that includes an antigen binding domain that targets, e.g., a target described above, a transmembrane domain and a costimulatory domain and a second CAR that targets an antigen other than antigen targeted by the first CAR (e.g., an antigen expressed on the same cancer cell type as the first target) and includes an antigen binding domain, a transmembrane domain and a primary signaling domain. In another embodiment, the CAR expressing immune effector cell comprises a first CAR that includes an antigen binding domain that targets, e.g., a target described above, a transmembrane domain and a primary signaling domain and a second CAR that targets an antigen other than antigen targeted by the first CAR (e.g., an antigen expressed on the same cancer cell type as the first target) and includes an antigen binding domain to the antigen, a transmembrane domain and a costimulatory signaling domain.

In one embodiment, the CAR-expressing immune effector cell comprises a CAR described herein, e.g., a CAR to a target described above, and an inhibitory CAR. In one embodiment, the inhibitory CAR comprises an antigen binding domain that binds an antigen found on normal cells but not cancer cells, e.g., normal cells that also express the target. In one embodiment, the inhibitory CAR comprises the antigen binding domain, a transmembrane domain and an intracellular domain of an inhibitory molecule.

For example, the intracellular domain of the inhibitory CAR can be an intracellular domain of PD1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta.

In one embodiment, an immune effector cell (e.g., T cell, NK cell) comprises a first CAR comprising an antigen binding domain that binds to a tumor antigen as described herein, and a second CAR comprising a PD1 extracellular domain or a fragment thereof.

In one embodiment, the cell further comprises an inhibitory molecule as described above.

In one embodiment, the second CAR in the cell is an inhibitory CAR, wherein the inhibitory CAR comprises an antigen binding domain, a transmembrane domain, and an intracellular domain of an inhibitory molecule. The inhibitory molecule can be chosen from one or more of: PD1, PD-L1, CTLA-4, TIM-3, LAG-3, VISTA, BTLA, TIGIT, LAIR1, CD160, 2B4, TGFR beta, CEACAM-1, CEACAM-3, and CEACAM-5. In one embodiment, the second CAR molecule comprises the extracellular domain of PD1 or a fragment thereof.

In embodiments, the second CAR molecule in the cell further comprises an intracellular signaling domain comprising a primary signaling domain and/or an intracellular signaling domain.

In other embodiments, the intracellular signaling domain in the cell comprises a primary signaling domain comprising the functional domain of CD3 zeta and a costimulatory signaling domain comprising the functional domain of 4-1BB.

In one embodiment, the second CAR molecule in the cell comprises the amino acid sequence of SEQ ID NO: 26.

In certain embodiments, the antigen binding domain of the first CAR molecule comprises a scFv and the antigen binding domain of the second CAR molecule does not comprise a scFv. For example, the antigen binding domain of the first CAR molecule comprises a scFv and the antigen binding domain of the second CAR molecule comprises a camelid VHH domain.

### Split CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens.

### Multiple CAR expression

In one aspect, the fusion protein, e.g., as described herein, comprising a domain that includes a CAR, - expressing cell described herein can further comprise a second fusion protein, e.g., as described herein, comprising a domain that includes a CAR, e.g., a second CAR that includes a different antigen binding domain, e.g., to the same target or a different target (e.g., a target other than a cancer associated antigen described herein or a different cancer associated antigen described herein). In one embodiment, the second CAR includes an antigen binding domain to a target expressed the same cancer cell type as the cancer associated antigen. In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. While not wishing to be bound by theory, placement of a costimulatory signaling domain, e.g., 4-1BB, CD28, CD27 or OX-40, onto the first CAR, and the primary signaling domain, e.g.,CD3 zeta, on the second CAR can limit the CAR activity to cells where both targets are expressed. In one embodiment, the CAR expressing cell comprises a first cancer associated antigen CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another embodiment, the CAR expressing cell comprises a first CAR that includes an antigen binding domain that binds a target antigen described herein, a transmembrane domain and a primary signaling domain and a second CAR that targets an antigen other than the first target antigen (e.g., an antigen expressed on the same cancer cell type as the first target antigen) and includes an antigen binding domain to the antigen, a transmembrane domain and a costimulatory signaling domain.

In some embodiments, the claimed invention comprises a first and second CAR, wherein the antigen binding domain of one of said first CAR said second CAR does not comprise a variable light domain and a variable heavy domain. In some embodiments, the antigen binding domain of one of said first CAR said second CAR is an scFv, and the other is not an scFv. In some embodiments, the antigen binding domain of one of said first CAR said second CAR comprises a single VH domain, e.g., a camelid, shark, or lamprey single VH domain, or a single VH domain derived from a human or mouse sequence. In some embodiments, the antigen binding domain of one of said first CAR said second CAR comprises a nanobody. In some embodiments, the antigen binding domain of one of said first CAR said second CAR comprises a camelid VHH domain.

### Allogeneic cells

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II or beta 2 microglobulin (B2M).

A T cell lacking a functional TCR can be, e.g., engineered such that it does not express any functional TCR on its surface, engineered such that it does not express one or more subunits that comprise a functional TCR, e.g., TRAC, TRBC1, TRBC2, CD3E, CD3G, or CD3D, or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, or subunit or regulator of HLA expression, e.g., B2M, is downregulated.

A T cell described herein can be, e.g., engineered such that it does not express a functional B2M on its surface. For example, a T cell described herein, can be engineered such that cell surface expression of B2M is downregulated.

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. by any mehod described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, CEACAM (e.g., CEACAM-1, CEACAM-3 and/or CEACAM-5), LAG3, VISTA, BTLA, TIGIT, LAIR1, CD160, 2B4 and TGFR beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA

In some embodiments, TCR expression and/or HLA or B2M expression can be inhibited using siRNA or shRNA that targets a nucleic acid encoding a TCR and/or HLA in a T cell.

### CRISPR

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA or B2M gene.

Artificial CRISPR/Cas systems can be generated which inhibit TCR and/or HLA, using technology known in the art, e.g., that described in U.S. Publication No. 20140068797, and Cong (2013) Science 339: 819-823. Other artificial CRISPR/Cas systems that are known in the art may also be generated which inhibit TCR and/or HLA, e.g., that described in Tsai (2014) Nature Biotechnol., 32:6 569-576, U.S. Patent No.: 8,871,445; 8,865,406; 8,795,965; 8,771,945; and 8,697,359.

### TALEN

"TALEN" or "TALEN to HLA and/or TCR" or "TALEN to inhibit HLA and/or TCR" refers to a transcription activator-like effector nuclease, an artificial nuclease which can be used to edit the HLA or B2M and/or TCR gene.

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALENs specific to sequences in HLA or TCR can be constructed using any method known in the art, including various schemes using modular components. Zhang et al. (2011) Nature Biotech. 29: 149-53; Geibler et al. (2011) PLoS ONE 6: e19509.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR or B2M gene.

ZFNs specific to sequences in HLA AND/OR TCR can be constructed using any method known in the art. See, e.g., Provasi (2011) Nature Med. 18: 807-815; Torikai (2013) Blood 122: 1341-1349; Cathomen et al. (2008) Mol. Ther. 16: 1200-7; Guo et al. (2010) J. Mol. Biol. 400: 96; U.S. Patent Publication 2011/0158957; and U.S. Patent Publication 2012/0060230.

### Telomerase expression

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an embodiment, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

### Expansion and Activation

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells e.g., T regulatory cell depleted cells, may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present invention.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3:CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one. In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one preferred aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a preferred particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain aspects, it may be desirable to significantly decrease the volume in which particles and cells are mixed together (i.e., increase the concentration of cells), to ensure maximum contact of cells and particles. For example, in one aspect, a concentration of about 10 billion cells/ml, 9 billion/ml, 8 billion/ml, 7 billion/ml, 6 billion/ml, 5 billion/ml, or 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one embodiment, cells transduced with a nucleic acid encoding a fusion protein comprising a domain that includes a CAR, e.g., a CAR described herein, are expanded, e.g., by a method described herein. In one embodiment, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one embodiment, the cells are expanded for a period of 4 to 9 days. In one embodiment, the cells are expanded for a period of 8 days or less, e.g., 7, 6 or 5 days. In one embodiment, the cells are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one embodiment, the cells are expanded for 5 days show at least a one, two, three or four fold increase in cells doublings upon antigen stimulation as compared to the same cells expanded in culture for 9 days under the same culture conditions. In one embodiment, the cells are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one embodiment, the cells expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-y, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one embodiment, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one embodiment, the cells are expanded in the presence of IL-15 and/or IL-7 (e.g., IL-15 and IL-7).

In embodiments, methods described herein, e.g., fusion protein comprising a domain that includes a CAR, -expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In embodiments, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some embodiments a fusion protein comprising a domain that includes a CAR, -expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetlL-15, during the manufacturing of the CAR-expressing cell, e.g., ex vivo. In embodiments, a CAR-expressing cell of the invention is contacted with a composition comprising a IL-15 polypeptide during the manufacturing of the CAR-expressing cell, e.g., ex vivo. In embodiments, a CAR-expressing cell of the invention is contacted with a composition comprising a combination of both a IL-15 polypeptide and a IL-15 Ra polypeptide during the manufacturing of the CAR-expressing cell, e.g., ex vivo. In embodiments, a CAR-expressing cell of the invention is contacted with a composition comprising hetlL-15 during the manufacturing of the CAR-expressing cell, e.g., ex vivo.

In one embodiment the fusion protein comprising a domain that includes a CAR, -expressing cell described herein is contacted with a composition comprising hetlL-15 during ex vivo expansion. In an embodiment, the CAR-expressing cell of the invention is contacted with a composition comprising an IL-15 polypeptide during ex vivo expansion. In an embodiment, the CAR-expressing cell of the invention is contacted with a composition comprising both an IL-15 polypeptide and an IL-15Ra polypeptide during ex vivo expansion. In one embodiment the contacting results in the survival and proliferation of a lymphocyte subpopulation, e.g., CD8+ T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

Once a fusion protein, e.g., as described herein, comprising a domain that includes a CAR, described herein is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a cars of the present disclosureare described in further detail below

Western blot analysis of fusion protein, e.g., as described herein, comprising a domain that includes a CAR, expression in primary T cells can be used to detect the presence of monomers and dimers. See, *e.g.,* Milone et al., Molecular Therapy 17(8): 1453-1464 (2009). Very briefly, T cells (1:1 mixture of CD4⁺ and CD8⁺ T cells) expressing the CARs are expanded *in vitro* for more than 10 days followed by lysis and SDS-PAGE under reducing conditions. CARs containing the full length TCR-ζ cytoplasmic domain and the endogenous TCR-ζ chain are detected by western blotting using an antibody to the TCR-ζ chain. The same T cell subsets are used for SDS-PAGE analysis under non-reducing conditions to permit evaluation of covalent dimer formation.

*In vitro* expansion of fusion protein⁺, e.g., as described herein, comprising a domain that includes a CAR, e.g., CAR⁺, T cells following antigen stimulation can be measured by flow cytometry. For example, a mixture of CD4⁺ and CD8⁺ T cells are stimulated with αCD3/αCD28 aAPCs followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, e.g., Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with CAR on day 1 using a bicistronic lentiviral vector expressing CAR along with eGFP using a 2A ribosomal skipping sequence. Cultures are re-stimulated with either a cancer associated antigen as described herein⁺ K562 cells (K562 expressing a cancer associated antigen as described herein), wildtype K562 cells (K562 wild type) or K562 cells expressing hCD32 and 4-1BBL in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, *e.g*., Milone et al., Molecular Therapy 17(8): 1453-1464 (2009).

Sustained fusion protein⁺, e.g., as described herein, comprising a domain that includes a CAR, e.g., CAR⁺, T cell expansion in the absence of re-stimulation can also be measured. See, *e.g.,* Milone et al., Molecular Therapy 17(8): 1453-1464 (2009). Briefly, mean T cell volume (fl) is measured on day 8 of culture using a Coulter Multisizer III particle counter, a Nexcelom Cellometer Vision or Millipore Scepter, following stimulation with αCD3/αCD28 coated magnetic beads on day 0, and transduction with the indicated CAR on day 1.

Animal models can also be used to measure a CART activity. For example, xenograft model using human a cancer associated antigen described herein-specific CAR⁺ T cells to treat a primary human pre-B ALL in immunodeficient mice can be used. See, e.g., Milone et al., Molecular Therapy 17(8): 1453-1464 (2009). Very briefly, after establishment of ALL, mice are randomized as to treatment groups. Different numbers of a cancer associated antigen -specific CARengineered T cells are coinjected at a 1:1 ratio into NOD-SCID-γ^{-/-} mice bearing B-ALL. The number of copies of a cancer associated antigen - specific CAR vector in spleen DNA from mice is evaluated at various times following T cell injection. Animals are assessed for leukemia at weekly intervals. Peripheral blood a cancer associate antigen as described herein⁺ B-ALL blast cell counts are measured in mice that are injected with a cancer associated antigen described herein-ζ CAR⁺ T cells or mock-transduced T cells. Survival curves for the groups are compared using the log-rank test. In addition, absolute peripheral blood CD4⁺ and CD8⁺ T cell counts 4 weeks following T cell injection in NOD-SCID-γ^{-/-} mice can also be analyzed. Mice are injected with leukemic cells and 3 weeks later are injected with T cells engineered to express CAR by a bicistronic lentiviral vector that encodes the CAR linked to eGFP. T cells are normalized to 45-50% input GFP⁺ T cells by mixing with mock-transduced cells prior to injection, and confirmed by flow cytometry. Animals are assessed for leukemia at 1-week intervals. Survival curves for the CAR⁺ T cell groups are compared using the log-rank test.

Dose dependent fusion protein, e.g., as described herein, comprising a domain that includes a CAR, treatment response can be evaluated. See, e.g., Milone et al., Molecular Therapy 17(8): 1453-1464 (2009). For example, peripheral blood is obtained 35-70 days after establishing leukemia in mice injected on day 21 with CAR T cells, an equivalent number of mock-transduced T cells, or no T cells. Mice from each group are randomly bled for determination of peripheral blood a cancer associate antigen as described herein⁺ ALL blast counts and then killed on days 35 and 49. The remaining animals are evaluated on days 57 and 70.

Assessment of cell proliferation and cytokine production has been previously described, e.g., at Milone et al., Molecular Therapy 17(8): 1453-1464 (2009). Briefly, assessment of CAR-mediated proliferation is performed in microtiter plates by mixing washed T cells with K562 cells expressing a cancer associated antigen described herein (K19) or CD32 and CD137 (KT32-BBL) for a final T-cell:K562 ratio of 2:1. K562 cells are irradiated with gamma-radiation prior to use. Anti-CD3 (clone OKT3) and anti- CD28 (clone 9.3) monoclonal antibodies are added to cultures with KT32-BBL cells to serve as a positive control for stimulating T-cell proliferation since these signals support long-term CD8⁺ T cell expansion *ex vivo.* T cells are enumerated in cultures using CountBright^{™} fluorescent beads (Invitrogen, Carlsbad, CA) and flow cytometry as described by the manufacturer. CAR⁺ T cells are identified by GFP expression using T cells that are engineered with eGFP-2A linked CAR-expressing lentiviral vectors. For CAR+ T cells not expressing GFP, the CAR+ T cells are detected with biotinylated recombinant a cancer associate antigen as described herein protein and a secondary avidin-PE conjugate. CD4+ and CD8⁺ expression on T cells are also simultaneously detected with specific monoclonal antibodies (BD Biosciences). Cytokine measurements are performed on supernatants collected 24 hours following re-stimulation using the human TH1/TH2 cytokine cytometric bead array kit (BD Biosciences, San Diego, CA) according the manufacturer's instructions. Fluorescence is assessed using a FACScalibur flow cytometer, and data is analyzed according to the manufacturer's instructions.

Cytotoxicity can be assessed by a standard 51 Cr-release assay. See, e.g., Milone et al., Molecular Therapy 17(8): 1453-1464 (2009). Briefly, target cells (K562 lines and primary pro-B-ALL cells) are loaded with 51 Cr (as NaCrO4, New England Nuclear, Boston, MA) at 37°C for 2 hours with frequent agitation, washed twice in complete RPMI and plated into microtiter plates. Effector T cells are mixed with target cells in the wells in complete RPMI at varying ratios of effector cell:target cell (E:T). Additional wells containing media only (spontaneous release, SR) or a 1% solution of triton-X 100 detergent (total release, TR) are also prepared. After 4 hours of incubation at 37°C, supernatant from each well is harvested. Released 51 Cr is then measured using a gamma particle counter (Packard Instrument Co., Waltham, MA). Each condition is performed in at least triplicate, and the percentage of lysis is calculated using the formula: % Lysis = (ER- SR) / (TR - SR), where ER represents the average 51 Cr released for each experimental condition.

Imaging technologies can be used to evaluate specific trafficking and proliferation of CARs in tumorbearing animal models. Such assays have been described, for example, in Barrett et al., Human Gene Therapy 22:1575-1586 (2011). Briefly, NOD/SCID/γc^{-/-} (NSG) mice are injected IV with Nalm-6 cells followed 7 days later with T cells 4 hour after electroporation with the CAR constructs. The T cells are stably transfected with a lentiviral construct to express firefly luciferase, and mice are imaged for bioluminescence. Alternatively, therapeutic efficacy and specificity of a single injection of CAR⁺ T cells in Nalm-6 xenograft model can be measured as the following: NSG mice are injected with Nalm-6 transduced to stably express firefly luciferase, followed by a single tail-vein injection of T cells electroporated with cars of the present disclosure7 days later. Animals are imaged at various time points post injection. For example, photon-density heat maps of firefly luciferasepositive leukemia in representative mice at day 5 (2 days before treatment) and day 8 (24 hr post CAR⁺ PBLs) can be generated.

Other assays, including those described in the Example section herein as well as those that are known in the art can also be used to evaluate the CARs described herein.

### Methods of making CAR-expressing cells

In another aspect, the invention pertains to a method of making a cell (e.g., an immune effector cell or population thereof) comprising introducing into (e.g., transducing) a cell, e.g., a T cell or a NK cell described herein, with a vector of comprising a nucleic acid encoding a fusion protein of the invention, comprising a domain that includes a CAR, e.g., a CAR described herein; or a nucleic acid encoding a CAR molecule e.g., a CAR described herein.

The cell in the methods is an immune effector cell (e.g., aT cell or a NK cell, or a combination thereof). In some embodiments, the cell in the methods is diaglycerol kinase (DGK) and/or Ikaros deficient.

In some embodiment, the introducing the nucleic acid molecule encoding a CAR comprises transducing a vector comprising the nucleic acid molecule encoding a fusion protein comprising a domain that includes a CAR, or transfecting the nucleic acid molecule encoding a CAR, wherein the nucleic acid molecule is an in vitro transcribed RNA.

In some embodiments, the method further comprises:
providing a population of immune effector cells (e.g., T cells or NK cells); and
removing T regulatory cells from the population, thereby providing a population of T regulatory-depleted cells;
wherein steps a) and b) are performed prior to introducing the nucleic acid encoding the fusion protein, comprising a domain that includes a CAR, to the population.

In embodiments of the methods, the T regulatory cells comprise CD25+ T cells, and are removed from the cell population using an anti-CD25 antibody, or fragment thereof. The anti-CD25 antibody, or fragment thereof, can be conjugated to a substrate, e.g., a bead.

In other embodiments, the population of T regulatory-depleted cells provided from step (b) contains less than 30%, 25%, 20%, 15%, 10%, 5%, 4%, 3%, 2%, 1% of CD25+ cells.

In yet other embodiments, the method further comprises removing cells from the population which express a tumor antigen that does not comprise CD25 to provide a population of T regulatory-depleted and tumor antigen depleted cells prior to introducing the nucleic acid encoding a fusion protein, comprising a domain that includes a CAR, to the population. The tumor antigen can be selected from CD19, CD30, CD38, CD123, CD20, CD14 or CD11b, or a combination thereof.

In other embodiments, the method further comprises removing cells from the population which express a checkpoint inhibitor, to provide a population of T regulatory-depleted and inhibitory molecule depleted cells prior to introducing the nucleic acid encoding a fusion protein, comprising a domain that includes a CAR, to the population. The checkpoint inhibitor can be chosen from PD-1, LAG-3, TIM3, B7-H1, CD160, P1H, 2B4, CEACAM (e.g., CEACAM-1, CEACAM-3, and/or CEACAM-5), TIGIT, CTLA-4, BTLA, and LAIR1.

Further embodiments disclosed herein encompass providing a population of immune effector cells. The population of immune effector cells provided can be selected based upon the expression of one or more of CD3, CD28, CD4, CD8, CD45RA, and/or CD45RO. In certain embodiments, the population of immune effector cells provided are CD3+ and/or CD28+.

In certain embodiments of the method, the method further comprises expanding the population of cells after the nucleic acid molecule encoding a fusion protein, comprising a domain that includes a CAR, has been introduced.

In embodiments, the population of cells is expanded for a period of 8 days or less.

In certain embodiments, the population of cells is expanded in culture for 5 days, and the resulting cells are more potent than the same cells expanded in culture for 9 days under the same culture conditions.

In other embodiments, the population of cells is expanded in culture for 5 days show at least a one, two, three or four fold increase in cell doublings upon antigen stimulation as compared to the same cells expanded in culture for 9 days under the same culture conditions.

In yet other embodiments, the population of cells is expanded in culture for 5 days, and the resulting cells exhibit higher proinflammatory IFN-y and/or GM-CSF levels, as compared to the same cells expanded in culture for 9 days under the same culture conditions.

In other embodiments, the population of cells is expanded by culturing the cells in the presence of an agent that stimulates a CD3/TCR complex associated signal and/or a ligand that stimulates a costimulatory molecule on the surface of the cells. The agent can be a bead conjugated with anti-CD3 antibody, or a fragment thereof, and/or anti-CD28 antibody, or a fragment thereof.

In other embodiments, the population of cells is expanded in an appropriate media that includes one or more interleukin that result in at least a 200-fold, 250-fold, 300-fold, or 350-fold increase in cells over a 14 day expansion period, as measured by flow cytometry.

In other embodiments, the population of cells is expanded in the presence IL-15 and/or IL-7.

In certain embodiments, the method further includes cryopresercing he population of the cells after the appropriate expansion period.

In yet other embodiments, the method of making of the invention further comprises contacting the population of immune effector cells with a nucleic acid encoding a telomerase subunit, e.g., hTERT. The the nucleic acid encoding the telomerase subunit can be DNA.

The present disclosurealso provides a method of generating a population of RNA-engineered cells, e.g., cells described herein, e.g., immune effector cells (e.g., T cells, NK cells), transiently expressing exogenous RNA. The method comprises introducing an *in vitro* transcribed RNA or synthetic RNA into a cell, where the RNA comprises a nucleic acid encoding a fusion protein, e.g., as described herein, comprising a domain that includes a CAR molecule, described herein.

In another aspect, the invention pertains to providing an anti-tumor immunity in a subject comprising administering to the subject an effective amount of a cell comprising a fusion protein comprising a domain that includes a CAR molecule, e.g., a cell expressing a CAR molecule described herein. In one embodiment, the cell is an autologous T cell or NK cell. In one embodiment, the cell is an allogeneic T cell or NK cell. In one embodiment, the subject is a human.

In one aspect, the invention includes a population of autologous cells that are transfected or transduced with a vector comprising a nucleic acid molecule encoding a fusion protein of the invention comprising a domain that includes a CAR molecule, e.g., as described herein. In one embodiment, the vector is a retroviral vector. In one embodiment, the vector is a self-inactivating lentiviral vector as described elsewhere herein. In one embodiment, the vector is delivered (e.g., by transfecting or electroporating) to a cell, e.g., a T cell or a NK cell, wherein the vector comprises a nucleic acid molecule encoding a CAR of the present disclosureas described herein, which is transcribed as an mRNA molecule, and the CARs of the present disclosure is translated from the RNA molecule and expressed on the surface of the cell.

In another aspect, the present disclosure provides a population of fusion protein comprising a domain that includes a CAR, -expressing cells, e.g., CAR-expressing immune effector cells (e.g., T cells or NK cells). In some embodiments, the population of CAR-expressing cells comprises a mixture of cells expressing different CARs. For example, in one embodiment, the population of CAR-expressing immune effector cells (e.g., T cells or NK cells) can include a first cell expressing a CAR having an antigen binding domain that binds to a first tumor antigen as described herein, and a second cell expressing a CAR having a different antigen binding domain that binds to a second tumor antigen as described herein. As another example, the population of CAR-expressing cells can include a first cell expressing a CAR that includes an antigen binding domain that binds to a tumor antigen as described herein, and a second cell expressing a CAR that includes an antigen binding domain to a target other than a tumor antigen as described herein. In one embodiment, the population of CAR-expressing cells includes, e.g., a first cell expressing a CAR that includes a primary intracellular signaling domain, and a second cell expressing a CAR that includes a secondary signaling domain, e.g., a costimulatory signaling domain.

In another aspect, the present disclosure provides a population of cells wherein at least one cell in the population expresses a fusion protein comprising a domain that includes a CAR having an antigen binding domain that binds to a tumor antigen as described herein, and a second cell expressing another agent, e.g., an agent which enhances the activity of a CAR-expressing cell. For example, in one embodiment, the agent can be an agent which inhibits an inhibitory molecule. Examples of inhibitory molecules include PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta. In one embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, LAG-3, CTLA-4, CD160, BTLA, LAIR1, TIM-3, CEACAM (e.g., CEACAM-1, CEACAM-3 and/or CEACAM-5), 2B4 and TIGIT, or a fragment of any of these, and a second polypeptide which is an intracellular signaling domain described herein (e.g., comprising a costimulatory domain (e.g., 41 BB, CD27 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28, CD27, OX40 or 4-IBB signaling domain described herein and/or a CD3 zeta signaling domain described herein).

In one embodiment, the nucleic acid molecule encoding a fusion protein comprising a domain that includes a CAR of the present disclosure, e.g., as described herein, is expressed as an mRNA molecule. In one embodiment, the genetically modified CAR of the present invention -expressing cells, e.g., immune effector cells (e.g., T cells, NK cells), can be generated by transfecting or electroporating an RNA molecule encoding the desired CARs (e.g., without a vector sequence) into the cell. In one embodiment, a CAR of the present disclosuremolecule is translated from the RNA molecule once it is incorporated and expressed on the surface of the recombinant cell.

A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-5000 bases in length (SEQ ID NO:32). RNA so produced can efficiently transfect different kinds of cells. In one embodiment, the template includes sequences for the fusion protein, e.g., as described herein, comprising a domain that includes a CAR. In an embodiment, an RNA fusion protein, comprising a domain that includes a CAR, vector is transduced into a cell, e.g., a T cell or a NK cell, by electroporation.

In one embodiment, the fusion protein, comprising a domain that includes a CAR, is introduced into immune effector cells (e.g., T cells, NK cells), e.g., using in vitro transcription, and the subject (e.g., human) receives an initial administration of fusion protein, comprising a domain that includes a CAR, immune effector cells (e.g., T cells, NK cells) of the invention, and one or more subsequent administrations of the fusion protein, comprising a domain that includes a CAR, immune effector cells (e.g., T cells, NK cells) of the invention, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the invention are administered to the subject (e.g., human) per week, e.g., 2, 3, or 4 administrations of the CAR immune effector cells (e.g., T cells, NK cells) of the invention are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

In one aspect, CAR-expressing cells are generated using lentiviral viral vectors, such as lentivirus. Cells, e.g., CARTs, generated that way will have stable CAR expression.

In one aspect, CAR-expressing cells, e.g., CARTs, are generated using a viral vector such as a gammaretroviral vector, e.g., a gammaretroviral vector described herein. CARTs generated using these vectors can have stable CAR expression.

In one aspect, CARTs transiently express CAR vectors for 4, 5, 6, 7, 8, 9, 10, 11, 12, 13, 14, 15 days after transduction. Transient expression of CARs can be effected by RNA CAR vector delivery. In one aspect, the CAR RNA is transduced into the T cell by electroporation.

A potential issue that can arise in patients being treated using transiently expressing CAR immune effector cells (e.g., T cells, NK cells) (particularly with murine scFv bearing CARTs) is anaphylaxis after multiple treatments.

Without being bound by this theory, it is believed that such an anaphylactic response might be caused by a patient developing humoral anti-CAR response, i.e., anti-CAR antibodies having an anti-IgE isotype. It is thought that a patient's antibody producing cells undergo a class switch from IgG isotype (that does not cause anaphylaxis) to IgE isotype when there is a ten to fourteen day break in exposure to antigen. If a patient is at high risk of generating an anti-CAR antibody response during the course of transient CAR therapy (such as those generated by RNA transductions), CART infusion breaks should not last more than ten to fourteen days.

### METHODS OF CONDITIONALLY EXPRESSING A PROTEIN AND THERAPEUTIC APPLICATION

Disclosed herein are also methods of conditionally expressing a fusion protein (e.g., comprising a CAR polypeptide). Such methods can include contacting a host cell comprising any of the fusion proteins described herein or a nucleic acid encoding such a fusion protein with a expression compound, e.g., a deaggregation compound or stabilization compound. In the present invention the fusion protein comprises a CAR polypeptide and the expression compound is a stabilization compound.

In the presence of the stabilization compound, the degradation domain of the fusion protein assumes a conformation resistant to cellular degradation, and a protease cleaves the protease cleavage site within the fusion protein, thereby resulting in cleavage of the degradation domain from the desired protein and the expression of the desired protein. In the absence of the stabilization compound, the degradation domain of the fusion protein assumes a conformation permissive to cellular degradation, which results in degradation of the fusion protein. In some embodiments, the host cell is contacted with the stabilization compound in vivo. In some embodiments, the host cell is contacted with the stabilization compound ex vivo.

In the presence of the deaggregation compound, the deaggregation domain of the fusion protein assumes a conformation resistant to oligomerization (e.g., dimerization), causing the fusion protein to enter a monomeric state accessible to a protease, and a protease cleaves the protease cleavage site within the fusion protein, thereby resulting in cleavage of the aggregation domain from the desired protein and the expression of the desired protein. In the absence of the deaggregation compound, the deaggregation domain of the fusion protein assumes a conformation permissive to dimerization, causing the fusion protein to enter into an oligomeric, e.g., dimeric, e.g., aggregated state. The oligomeric, e.g., dimeric, e.g., aggregated state prevents the fusion protein from moving between cellular compartments or from being secreted. The oligomeric, e.g., dimeric, e.g., aggregated state is inaccessible to protease. In some instances of the disclosure which do not form part of the invention as claimed, the host cell is contacted with the deaggregation compound in vivo. In some instances, the host cell is contacted with the deaggregation compound ex vivo.

The stabilization compound can be selected based on the degradation domain. For example, when the degradation domain is derived from an estrogen receptor, the stabilization compound can be selected from Bazedoxifene or 4-hydroxy tamoxifen (4-OHT). Tamoxifen and Bazedoxifene are FDA approved drugs, and thus are safe to use in human. If the degradation domain is derived from an FKB protein, the stabilization compound can be Shield1.

The deaggregation compound can be selected based on the deaggregation domain. For example, when the deaggregation domain is derived from FKBP, the deaggregation compound can be selected from FK506, rapamycin, AP22542, or AP21998.

In another aspect, the present disclosure provides fusion proteins of the invention for use in methods comprising administering the fusion protein of the invention as a therapy. Typically, such administration will be in the form of host cells (e.g., autologous or allogeneic host cells) expressing the fusion protein of invention to the subject. Accordingly, through administration of a corresponding stabilization compound (either in vivo or ex vivo), the expression of the therapeutic (i.e., the protein remaining after cleavage of the degradation domain) can be regulated. Accordingly, through administration of a corresponding deaggregation compound (either in vivo or ex vivo), the expression of the therapeutic (i.e., the protein remaining after cleavage of the aggregation domain) can be regulated. Thus, expression of known synthetic therapeutic proteinsor transmembrane receptors (e.g., a fusion protein, e.g., as described herein, comprising a domain that includes a CAR molecule described herein) can be regulated. In one embodiment, the subject has a disorder described herein, e.g., the subject has cancer, e.g., the subject has a cancer which expresses a target antigen described herein. In one embodiment, the subject is a human.

Provided herein are host cells of the invention or nucleic acids of the invention for use in methods of treating a subject having a disease associated with expression of a tumor antigen by administering to the subject an effective amount of a host cell comprising any of the fusion proteins of the invention or a nucleic acid encoding such a fusion protein. In some embodiments, the fusion protein comprises a chimeric antigen receptor (CAR), which comprises, in an N-terminal to C-terminal direction, an antigen binding domain that specifically binds the tumor antigen, a transmembrane domain, and one or more intracellular signaling domains. In some embodiments, the host cell is autologous to the subject. In some embodiments, the host cell is allogenic to said subject. In some embodiments, the host cell is contacted with an expression compound, which is a stabilization compound. In the presence of the stabilization compound, the degradation domain of the fusion protein assumes a conformation resistant to cellular degradation, and a protease cleaves the protease cleavage site within the fusion protein, thereby results in cleavage of the degradation domain from the desired protein and the expression of the desired protein. In the absence of the stabilization compound, the degradation domain of the fusion protein assumes a conformation permissive to cellular degradation, which results in degradation of the fusion protein. In some instances not forming part of the claimed invention, a host cell is contacted with a deaggregation compound. In the presence of the deaggregation compound, the aggregation domain of the fusion protein assumes a conformation resistant to oligomerization or aggregation, and a protease cleaves the protease cleavage site within the fusion protein, thereby resulting in cleavage of the aggregation domain from the desired protein and the expression of the desired protein. In the absence of the deaggregation compound, the aggregation domain of the fusion protein assumes a conformation permissive to oligomerization or aggregation, which results in aggregation of the fusion protein.

In another aspect, the invention pertains to treating a subject having a disease associated with expression of a cancer associated antigen as described herein comprising administering to the subject an effective amount of a cell comprising a fusion protein comprising a CAR molecule, e.g., a fusion protein comprising a CAR molecule described herein.

In yet another aspect, the invention features a cell of the invention for use in a method of treating a subject having a disease associated with expression of a tumor antigen (e.g., an antigen described herein), the method comprising administering to the subject an effective amount of the cell, e.g., an immune effector cell *(e.g.,* a population of immune effector cells) which comprises a fusion protein comprising a CAR molecule, wherein the CAR molecule comprises an antigen binding domain, a transmembrane domain, and an intracellular domain, said intracellular domain comprises a costimulatory domain and/or a primary signaling domain, wherein said antigen binding domain binds to the tumor antigen associated with the disease, e.g. a tumor antigen as described herein.

In a related aspect, the invention features a cell of the invention for use in a method of treating a subject having a disease associated with expression of a tumor antigen. The method comprises administering to the subject an effective amount of the cell, e.g., an immune effector cell (*e.g.,* a population of immune effector cells), comprising a fusion protein comprising a CAR molecule, in combination with an agent that increases the efficacy of the immune cell, wherein:
the agent that increases the efficacy of the immune cell is chosen from one or more of:
(i) a protein phosphatase inhibitor;
(ii) a kinase inhibitor;
(iii) a cytokine;
(iv) an inhibitor of an immune inhibitory molecule; or
(v) an agent that decreases the level or activity of a T_{REG} cell.

In another aspect, the invention features a composition comprising an immune effector cell (*e.g.,* a population of immune effector cells) comprising a fusion protein comprising a CAR molecule (e.g., a fusion protein comprising a CAR molecule as described herein) for use in the treatment of a subject having a disease associated with expression of a tumor antigen, e.g., a disorder as described herein.

In certain embodiments of any of the aforesaid methods or uses, the disease associated with a tumor antigen, e.g., a tumor antigen described herein, is selected from a proliferative disease such as a cancer or malignancy or a precancerous condition such as a myelodysplasia, a myelodysplastic syndrome or a preleukemia, or is a non-cancer related indication associated with expression of a tumor antigen described herein. In one embodiment, the disease is a cancer described herein, e.g., a cancer described herein as being associated with a target described herein. In one embodiment, the disease is a hematologic cancer. In one embodiment, the hematologic cancer is leukemia. In one embodiment, the cancer is selected from the group consisting of one or more acute leukemias including but not limited to B-cell acute lymphoid leukemia ("BALL"), T-cell acute lymphoid leukemia ("TALL"), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and to disease associated with expression of a tumor antigen described herein include, but not limited to, atypical and/or non-classical cancers, malignancies, precancerous conditions or proliferative diseases expressing a tumor antigen as described herein; and any combination thereof. In another embodiment, the disease associated with a tumor antigen described herein is a solid tumor.

In certain embodiments, the methods or uses are carried out in combination with an agent that increases the efficacy of the immune effector cell, e.g., an agent as described herein.

In any of the aforesaid methods or uses, the disease associated with expression of the tumor antigen is selected from the group consisting of a proliferative disease, a precancerous condition, a cancer, and a non-cancer related indication associated with expression of the tumor antigen.

The cancer can be a hematologic cancer, e.g., a cancer chosen from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or pre-leukemia.

The cancer can also be chosen from colon cancer, rectal cancer, renal-cell carcinoma, liver cancer, nonsmall cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.

In certain embodiments of the methods or uses described herein, the cell is administered in combination with an agent that increases the efficacy of the cell, e.g., one or more of a protein phosphatase inhibitor, a kinase inhibitor, a cytokine, an inhibitor of an immune inhibitory molecule; or an agent that decreases the level or activity of a T_{REG} cell.

In certain embodiments of the methods or uses described herein, the protein phosphatase inhibitor is a SHP-1 inhibitor and/or an SHP-2 inhibitor.

In other embodiments of the methods or uses described herein, kinase inhibitor is chosen from one or more of a CDK4 inhibitor, a CDK4/6 inhibitor (e.g., palbociclib), a BTK inhibitor (e.g., ibrutinib or RN-486), an mTOR inhibitor (e.g., rapamycin or everolimus (RAD001)), an MNK inhibitor, or a dual P13K/mTOR inhibitor. In one embodiment, the BTK inhibitor does not reduce or inhibit the kinase activity of interleukin-2-inducible kinase (ITK).

In other embodiments of the methods or uses described herein, the agent that inhibits the immune inhibitory molecule comprises an antibody or antibody fragment, an inhibitory nucleic acid, a clustered regularly interspaced short palindromic repeats (CRISPR), a transcription-activator like effector nuclease (TALEN), or a zinc finger endonuclease (ZFN) that inhibits the expression of the inhibitory molecule.

In other embodiments of the methods or uses described herein, the agent that decreases the level or activity of the TREG cells is chosen from cyclophosphamide, anti-GITR antibody, CD25-depletion, or a combination thereof.

In certain embodiments of the methods or uses described herein, the immune inhibitory molecule is selected from the group consisting of PD1, PD-L1, CTLA-4, TIM-3, LAG-3, VISTA, BTLA, TIGIT, LAIR1, CD160, 2B4, TGFR beta, CEACAM-1, CEACAM-3, and CEACAM-5.

In other embodiments, the agent that inhibits the inhibitory molecule comprises a first polypeptide comprising an inhibitory molecule or a fragment thereof and a second polypeptide that provides a positive signal to the cell, and wherein the first and second polypeptides are expressed on the CAR-containing immune cells, wherein (i) the first polypeptide comprises PD1, PD-L1, CTLA-4, TIM-3, LAG3, VISTA, BTLA, TIGIT, LAIR1, CD160, 2B4, TGFR beta, CEACAM-1, CEACAM-3, and CEACAM-5 or a fragment thereof; and/or (ii) the second polypeptide comprises an intracellular signaling domain comprising a primary signaling domain and/or a costimulatory signaling domain. In one embodiment, the primary signaling domain comprises a functional domain of CD3 zeta; and/or the costimulatory signaling domain comprises a functional domain of a protein selected from 41 BB, CD27 and CD28.

In other embodiments, the cytokine is chosen from IL-7, IL-15, IL-18, or IL-21, or a combination thereof.

In other embodiments, the immune effector cell comprising the fusion protein and a second, e.g., any of the combination therapies disclosed herein (e.g., the agent that that increases the efficacy of the immune effector cell) are administered substantially simultaneously or sequentially.

In other embodiments, the immune cell comprising the fusion protein is administered in combination with a molecule that targets GITR and/or modulates GITR function. In certain embodiments, the molecule targeting GITR and/or modulating GITR function is administered prior to the CAR-expressing cell or population of cells, or prior to apheresis.

In one embodiment, lymphocyte infusion, for example allogeneic lymphocyte infusion, is used in the treatment of the cancer, wherein the lymphocyte infusion comprises at least one CAR-expressing cell of the present invention. In one embodiment, autologous lymphocyte infusion is used in the treatment of the cancer, wherein the autologous lymphocyte infusion comprises at least one CAR-expressing cell described herein.

In one embodiment, the cell is a T cell and the T cell is diaglycerol kinase (DGK) deficient. In one embodiment, the cell is a T cell and the T cell is Ikaros deficient. In one embodiment, the cell is a T cell and the T cell is both DGK and Ikaros deficient.

In one embodiment, the method includes administering a cell expressing the fusion protein comprising a CAR molecule, as described herein, in combination with an agent which enhances the activity of a CAR-expressing cell, wherein the agent is a cytokine, e.g., IL-7, IL-15, IL-18, IL-21, or a combination thereof. The cytokine can be delivered in combination with, e.g., simultaneously or shortly after, administration of the CAR-expressing cell. Alternatively, the cytokine can be delivered after a prolonged period of time after administration of the CAR-expressing cell, e.g., after assessment of the subject's response to the CAR-expressing cell. In one embodiment the cytokine is administered to the subject simultaneously (e.g., administered on the same day) with or shortly after administration (e.g., administered 1 day, 2 days, 3 days, 4 days, 5 days, 6 days, or 7 days after administration) of the cell or population of cells of any of claims 61-80. In other embodiments, the cytokine is administered to the subject after a prolonged period of time (e.g., e.g., at least 2 weeks, 3 weeks, 4 weeks, 6 weeks, 8 weeks, 10 weeks, or more) after administration of the cell or population of cells of any of claims 61-80, or after assessment of the subject's response to the cell.

In other embodiments, the cells expressing a fusion protein comprising a CAR molecule are administered in combination with an agent that ameliorates one or more side effects associated with administration of a cell expressing a CAR molecule. Side effects associated with the CAR-expressing cell can be chosen from cytokine release syndrome (CRS) or hemophagocytic lymphohistiocytosis (HLH).

In embodiments of any of the aforeseaid methods or uses, the cells expressing the CAR molecule are administered in combination with an agent that treats the disease associated with expression of the tumor antigen, e.g., any of the second or third therapies disclosed herein. Additional exemplary combinations include one or more of the following.

In another embodiment, the cell expressing the CAR molecule, e.g., as described herein, can be administered in combination with another agent, e.g., a kinase inhibitor and/or checkpoint inhibitor described herein. In an embodiment, a cell expressing the CAR molecule can further express another agent, e.g., an agent which enhances the activity of a CAR-expressing cell.

For example, in one embodiment, the agent that enhances the activity of a CAR-expressing cell can be an agent which inhibits an inhibitory molecule (e.g., an immune inhibitor molecule). Examples of inhibitory molecules include PD1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta.

In one embodiment, the agent that inhibits the inhibitory molecule is an inhibitory nucleic acid is a dsRNA, a siRNA, or a shRNA. In embodiments, the inhibitory nucleic acid is linked to the nucleic acid that encodes a component of the CAR molecule. For example, the inhibitory molecule can be expressed on the CAR-expressing cell.

In another embodiment, the agent which inhibits an inhibitory molecule, e.g., is a molecule described herein, e.g., an agent that comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 or TGFR beta, or a fragment of any of these (e.g., at least a portion of the extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of the extracellular domain of PD1), and a second polypeptide of an intracellular signaling domain described herein (e.g., a CD28 signaling domain described herein and/or a CD3 zeta signaling domain described herein).

In one embodiment, the cell of the present invention, e.g., T cell or NK cell, is administered to a subject that has received a previous stem cell transplantation, e.g., autologous stem cell transplantation.

In one embodiment, the cell of the present invention, e.g., T cell or NK cells, is administered to a subject that has received a previous dose of melphalan.

In one embodiment, the cell expressing a fusion protein comprising a CAR molecule, e.g., a CAR molecule described herein, is administered in combination with an agent that increases the efficacy of a cell expressing a CAR molecule, e.g., an agent described herein.

In one embodiment, the cells expressing a fusion protein comprising a CAR molecule, e.g., a CAR molecule described herein, are administered in combination with a low, immune enhancing dose of an mTOR inhibitor. While not wishing to be bound by theory, it is believed that treatment with a low, immune enhancing, dose (e.g., a dose that is insufficient to completely suppress the immune system but sufficient to improve immune function) is accompanied by a decrease in PD-1 positive T cells or an increase in PD-1 negative cells. PD-1 positive T cells, but not PD-1 negative T cells, can be exhausted by engagement with cells which express a PD-1 ligand, e.g., PD-L1 or PD-L2.

In an embodiment this approach can be used to optimize the performance of CAR cells of the invention in the subject. While not wishing to be bound by theory, it is believed that, in an embodiment, the performance of endogenous, non-modified immune effector cells, e.g., T cells or NK cells, is improved. While not wishing to be bound by theory, it is believed that, in an embodiment, the performance of a target antigen CAR- expressing cell is improved. In other embodiments, cells, e.g., T cells or NK cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In an embodiment, administration of a low, immune enhancing, dose of an mTOR inhibitor, e.g., an allosteric inhibitor, e.g., RAD001, or a catalytic inhibitor, is initiated prior to administration of an CAR expressing cell of the invention, e.g., T cells or NK cells. In an embodiment, the CAR cells are administered after a sufficient time, or sufficient dosing, of an mTOR inhibitor, such that the level of PD1 negative immune effector cells, e.g., T cells or NK cells, or the ratio of PD1 negative immune effector cells, e.g., T cells/ PD1 positive immune effector cells, e.g., T cells, has been, at least transiently, increased.

In an embodiment, the cell, e.g., T cell or NK cell, to be engineered to express a CAR, is harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In one embodiment, the cell expressing a fusion protein comprising a CAR molecule, e.g., a CAR molecule described herein, is administered in combination with an agent that ameliorates one or more side effect associated with administration of a cell expressing a CAR molecule, e.g., an agent described herein.

In one embodiment, the cell expressing a fusion protein comprising a CAR molecule, e.g., a CAR molecule described herein, is administered in combination with an agent that treats the disease associated with a cancer associated antigen as described herein, e.g., an agent described herein.

In one embodiment, a cell expressing two or more fusion proteins comprising CAR molecules, e.g., as described herein, is administered to a subject in need thereof to treat cancer. In one embodiment, a population of cells including a fusion protein comprising a CAR expressing cell is administered to a subject in need thereof to treat cancer.

In one embodiment, the cell expressing a fusion protein comprising a CAR molecule, e.g., a CAR molecule described herein, is administered at a dose and/or dosing schedule described herein.

In one embodiment, the fusion protein comprising a CAR molecule is introduced into immune effector cells (e.g., T cells, NK cells), e.g., using *in vitro* transcription, and the subject (e.g., human) receives an initial administration of cells comprising a fusion protein comprising a CAR molecule, and one or more subsequent administrations of cells comprising a fusion protein comprising a CAR molecule, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of cells comprising a fusion protein comprising a CAR molecule are administered to the subject (e.g., human) per week, e.g., 2, 3, or 4 administrations of cells comprising a fusion protein comprising a CAR molecule are administered per week. In one embodiment, the subject (e.g., human subject) receives more than one administration of cells comprising a fusion protein comprising a CAR molecule per week (e.g., 2, 3 or 4 administrations per week) (also referred to herein as a cycle), followed by a week of no administration of cells comprising a fusion protein comprising a CAR molecule, and then one or more additional administration of cells comprising a CAR molecule (e.g., more than one administration of the cells comprising a CAR molecule per week) is administered to the subject. In another embodiment, the subject (e.g., human subject) receives more than one cycle of cells comprising a fusion protein comprising a CAR molecule, and the time between each cycle is less than 10, 9, 8, 7, 6, 5, 4, or 3 days. In one embodiment, the cells comprising a fusion protein comprising a CAR molecule are administered every other day for 3 administrations per week. In one embodiment, the cells comprising a fusion protein comprising a CAR molecule are administered for at least two, three, four, five, six, seven, eight or more weeks.

In one embodiment, the cells expressing a fusion protein comprising a CAR molecule, e.g., a CAR molecule described herein, are administered as a first line treatment for the disease, e.g., the cancer, e.g., the cancer described herein. In another embodiment, the cells expressing a fusion protein comprising a CAR molecule, e.g., a CAR molecule described herein, are administered as a second, third, fourth line treatment for the disease, e.g., the cancer, e.g., the cancer described herein.

In one embodiment, a population of cells of the invention is administered.

In another aspect, the invention pertains to a cell expressing a fusion protein comprising a CAR molecule described herein for use as a medicament in combination with a kinase inhibitor and/or a checkpoint inhibitor as described herein. In another aspect, the invention pertains to a kinase inhibitor and/or a checkpoint inhibitor described herein for use as a medicament in combination with a cell expressing a CAR molecule described herein.

In another aspect, the invention pertains to a cell expressing a fusion protein comprising a CAR molecule described herein for use in combination with a cytokine, e.g., IL-7, IL-15 and/or IL-21 as described herein, in the treatment of a disease expressing a tumor antigen targeted by the CAR. In another aspect, the disclosure pertains to a cytokine described herein for use in combination with a cell expressing a fusion protein comprising a CAR molecule described herein, in the treatment of a disease expressing a tumor antigen targeted by the CAR.

In another aspect, the invention pertains to a cell expressing a fusion protein comprising a CAR molecule described herein for use in combination with a kinase inhibitor and/or a checkpoint inhibitor as described herein, in the treatment of a disease expressing a tumor antigen targeted by the CAR. In another aspect, the invention pertains to a kinase inhibitor and/or a checkpoint inhibitor described herein for use in combination with a cell expressing a fusion protein comprising a CAR molecule described herein, in the treatment of a disease expressing a tumor antigen targeted by the CAR.

In another aspect, the present disclosure provides a method comprising administering a fusion protein comprising a CAR molecule, e.g., a CAR molecule described herein, or a cell comprising a nucleic acid encoding a fusion protein comprising a CAR molecule, e.g., a CAR molecule described herein. In one instance, the subject has a disorder described herein, e.g., the subject has cancer, e.g., the subject has a cancer and has tumor-supporting cells which express a tumor-supporting antigen described herein. In one instance, the subject is a human.

In one embodiment of the methods or uses described herein, the fusion protein comprising CAR molecule is administered in combination with another agent. In one embodiment, the agent can be a kinase inhibitor, e.g., a CDK4/6 inhibitor, a BTK inhibitor, an mTOR inhibitor, a MNK inhibitor, or a dual PI3K/mTOR inhibitor, and combinations thereof. In one embodiment, the kinase inhibitor is a CDK4 inhibitor, e.g., a CDK4 inhibitor described herein, e.g., a CD4/6 inhibitor, such as, e.g., 6-Acetyl-8-cyclopentyl-5-methyl-2-(5-piperazin-1-yl-pyridin-2-ylamino)-8*H*-pyrido[2,3-*d*]pyrimidin-7-one, hydrochloride (also referred to as palbociclib or PD0332991). In one embodiment, the kinase inhibitor is a BTK inhibitor, e.g., a BTK inhibitor described herein, such as, e.g., ibrutinib. In one embodiment, the kinase inhibitor is an mTOR inhibitor, e.g., an mTOR inhibitor described herein, such as, e.g., rapamycin, a rapamycin analog, OSI-027. The mTOR inhibitor can be, e.g., an mTORC1 inhibitor and/or an mTORC2 inhibitor, e.g., an mTORC1 inhibitor and/or mTORC2 inhibitor described herein. In one embodiment, the kinase inhibitor is a MNK inhibitor, e.g., a MNK inhibitor described herein, such as, e.g., 4-amino-5-(4-fluoroanilino)-pyrazolo [3,4-*d*] pyrimidine. The MNK inhibitor can be, e.g., a MNK1a, MNK1b, MNK2a and/or MNK2b inhibitor. The dual PI3K/mTOR inhibitor can be, e.g., PF-04695102.

In one embodiment of the methods or uses described herein, the kinase inhibitor is a CDK4 inhibitor selected from aloisine A; flavopiridol or HMR-1275, 2-(2-chlorophenyl)-5,7-dihydroxy-8-[(3S,4R)-3-hydroxy-1-methyl-4-piperidinyl]-4-chromenone; crizotinib (PF-02341066; 2-(2-Chlorophenyl)-5,7-dihydroxy-8-[(2*R*,3*S*)-2-(hydroxymethyl)-1-methyl-3-pyrrolidinyl]- 4*H*-1-benzopyran-4-one, hydrochloride (P276-00); 1-methyl-5-[[2-[5-(trifluoromethyl)-1*H*-imidazol-2-yl]-4-pyridinyl]oxy]-*N*-[4-(trifluoromethyl)phenyl]-1*H*-benzimidazol-2-amine (RAF265); indisulam (E7070); roscovitine (CYC202); palbociclib (PD0332991); dinaciclib (SCH727965); N-[5-[[(5-*tert*-butyloxazol-2-yl)methyl]thio]thiazol-2-yl]piperidine-4-carboxamide (BMS 387032); 4-[[9-chloro-7-(2,6-difluorophenyl)-5*H*-pyrimido[5,4-*d*][2]benzazepin-2-yl]amino]-benzoic acid (MLN8054); 5-[3-(4,6-difluoro-1H-benzimidazol-2-yl)-1H-indazol-5-yl]-N-ethyl-4-methyl-3-pyridinemethanamine (AG-024322); 4-(2,6-dichlorobenzoylamino)-1H-pyrazole-3-carboxylic acid N-(piperidin-4-yl)amide (AT7519); 4-[2-methyl-1-(1-methylethyl)-1H-imidazol-5-yl]-N-[4-(methylsulfonyl)phenyl]- 2-pyrimidinamine (AZD5438); and XL281 (BMS908662).

In one embodiment of the methods or uses described herein, the kinase inhibitor is a CDK4 inhibitor, e.g., palbociclib (PD0332991), and the palbociclib is administered at a dose of about 50 mg, 60 mg, 70 mg, 75 mg, 80 mg, 90 mg, 100 mg, 105 mg, 110 mg, 115 mg, 120 mg, 125 mg, 130 mg, 135 mg (e.g., 75 mg, 100 mg or 125 mg) daily for a period of time, e.g., daily for 14-21 days of a 28 day cycle, or daily for 7-12 days of a 21 day cycle. In one embodiment, 1, 2, 3, 4, 5, 6, 7, 8, 9, 10, 11, 12 or more cycles of palbociclib are administered.

In one embodiment of the methods or uses described herein, the kinase inhibitor is a BTK inhibitor selected from ibrutinib (PCI-32765); GDC-0834; RN-486; CGI-560; CGI-1764; HM-71224; CC-292; ONO-4059; CNX-774; and LFM-A13. In one embodiment, the BTK inhibitor does not reduce or inhibit the kinase activity of interleukin-2-inducible kinase (ITK), and is selected from GDC-0834; RN-486; CGI-560; CGI-1764; HM-71224; CC-292; ONO-4059; CNX-774; and LFM-A13.

In one embodiment of the methods or uses described herein, the kinase inhibitor is a BTK inhibitor, e.g., ibrutinib (PCI-32765), and the ibrutinib is administered at a dose of about 250 mg, 300 mg, 350 mg, 400 mg, 420 mg, 440 mg, 460 mg, 480 mg, 500 mg, 520 mg, 540 mg, 560 mg, 580 mg, 600 mg (e.g., 250 mg, 420 mg or 560 mg) daily for a period of time, e.g., daily for 21 day cycle, or daily for 28 day cycle. In one embodiment, 1, 2, 3, 4, 5, 6, 7, 8, 9, 10, 11, 12 or more cycles of ibrutinib are administered.

In one embodiment of the methods or uses described herein, the kinase inhibitor is a BTK inhibitor that does not inhibit the kinase activity of ITK, e.g., RN-486, and RN-486 is administered at a dose of about 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg (e.g., 150 mg, 200 mg or 250 mg) daily for a period of time, e.g., daily a 28 day cycle. In one embodiment, 1, 2, 3, 4, 5, 6, 7, or more cycles of RN-486 are administered.

In one embodiment of the methods or uses described herein, the kinase inhibitor is an mTOR inhibitor selected from temsirolimus; ridaforolimus (1*R*,2*R*,4*S*)-4-[(2*R*)-2 [(1*R*,9*S*,12*S*,15*R*,16*E*,18*R*,19*R*,21*R,* 23*S*,24*E*,26*E*,28*Z*,30*S*,32*S*,35*R*)-1,18-dihydroxy-19,30-dimethoxy-15,17,21,23, 29,35-hexamethyl-2,3,10,14,20-pentaoxo-11,36-dioxa-4-azatricyclo[30.3.1.0^{1,9}] hexatriaconta-16,24,26,28-tetraen-12-yl]propyl]-2-methoxycyclohexyl dimethylphosphinate, also known as AP23573 and MK8669; everolimus (RAD001); rapamycin (AY22989); simapimod; (5-{2,4-bis[(3S)-3-methylmorpholin-4-yl]pyrido[2,3-d]pyrimidin-7-yl}-2-methoxyphenyl)methanol (AZD8055); 2-amino-8-[trans-4-(2-hydroxyethoxy)cyclohexyl]-6-(6-methoxy-3-pyridinyl)-4-methyl-pyrido[2,3-*d*]pyrimidin-7(8*H*)-one (PF04691502); and *N*²-[1,4-dioxo-4-[[4-(4-oxo-8-phenyl-4*H*-1-benzopyran-2-yl)morpholinium-4-yl]methoxy]butyl]-L-arginylglycyl-L-α-aspartylL-serine- (SEQ ID NO: 982), inner salt (SF1126); and XL765.

In one embodiment of the methods or uses described herein, the kinase inhibitor is an mTOR inhibitor, e.g., rapamycin, and the rapamycin is administered at a dose of about 3 mg, 4 mg, 5 mg, 6 mg, 7 mg, 8 mg, 9 mg, 10 mg (e.g., 6 mg) daily for a period of time, e.g., daily for 21 day cycle, or daily for 28 day cycle. In one embodiment, 1, 2, 3, 4, 5, 6, 7, 8, 9, 10, 11, 12 or more cycles of rapamycin are administered. In one embodiment, the kinase inhibitor is an mTOR inhibitor, e.g., everolimus and the everolimus is administered at a dose of about 2 mg, 2.5 mg, 3 mg, 4 mg, 5 mg, 6 mg, 7 mg, 8 mg, 9 mg, 10 mg, 11 mg, 12 mg, 13 mg, 14 mg, 15 mg (e.g., 10 mg) daily for a period of time, e.g., daily for 28 day cycle. In one embodiment, 1, 2, 3, 4, 5, 6, 7, 8, 9, 10, 11, 12 or more cycles of everolimus are administered.

In one embodiment of the methods or uses described herein, the kinase inhibitor is an MNK inhibitor selected from CGP052088; 4-amino-3-(p-fluorophenylamino)-pyrazolo [3,4-*d*] pyrimidine (CGP57380); cercosporamide; ETC-1780445-2; and 4-amino-5-(4-fluoroanilino)-pyrazolo [3,4-*d*] pyrimidine.

In one embodiment of the methods or uses described herein, the kinase inhibitor is a dual phosphatidylinositol 3-kinase (PI3K) and mTOR inhibitor selected from 2-Amino-8-[trans-4-(2-hydroxyethoxy)cyclohexyl]-6-(6-methoxy-3-pyridinyl)-4-methyl-pyrido[2,3-**d**]pyrimidin-7(8H)-one (PF-04691502); N-[4-[[4-(Dimethylamino)-1-piperidinyl]carbonyl]phenyl]-N'-[4-(4,6-di-4-morpholinyl-1,3,5-triazin-2-yl)phenyl]urea (PF-05212384, PKI-587); 2-Methyl-2-{4-[3-methyl-2-oxo-8-(quinolin-3-yl)-2,3-dihydro-1*H*-imidazo[4,5-c]quinolin-1-yl]phenyl}propanenitrile (BEZ-235); apitolisib (GDC-0980, RG7422); 2,4-Difluoro-N-{2-(methyloxy)-5-[4-(4-pyridazinyl)-6-quinolinyl]-3-pyridinyl}benzenesulfonamide (GSK2126458); 8-(6-methoxypyridin-3-yl)-3-methyl-1-(4-(piperazin-1-yl)-3-(trifluoromethyl)phenyl)-1H-imidazo[4,5-c]quinolin-2(3H)-one Maleic acid (NVP-BGT226); 3-[4-(4-Morpholinylpyrido[3',2':4,5]furo[3,2-d]pyrimidin-2-yl]phenol (PI-103); 5-(9-isopropyl-8-methyl-2-morpholino-9H-purin-6-yl)pyrimidin-2-amine (VS-5584, SB2343); and N-[2-[(3,5-Dimethoxyphenyl)amino]quinoxalin-3-yl]-4-[(4-methyl-3-methoxyphenyl)carbonyl]aminophenylsulfonamide (XL765).

In one embodiment of the methods or uses described herein, a cell comprising a fusion protein of the invention is administered to a subject in combination with a protein tyrosine phosphatase inhibitor, e.g., a protein tyrosine phosphatase inhibitor described herein. In one embodiment, the protein tyrosine phosphatase inhibitor is an SHP-1 inhibitor, e.g., an SHP-1 inhibitor described herein, such as, e.g., sodium stibogluconate. In one embodiment, the protein tyrosine phosphatase inhibitor is an SHP-2 inhibitor.

In one embodiment of the methods or uses described herein, a cell comprising a fusion protein of the invention is administered in combination with another agent, and the agent is a cytokine. The cytokine can be, e.g., IL-7, IL-15, IL-21, or a combination thereof. In another embodiment, a cell comprising a fusion protein described herein is administered in combination with a checkpoint inhibitor, e.g., a checkpoint inhibitor described herein. For example, in one embodiment, the check point inhibitor inhibits an inhibitory molecule selected from PD-1, PD-L1, CTLA-4, TIM-3, CEACAM (e.g., CEACAM-1, CEACAM-3 and/or CEACAM-5), LAG-3, VISTA, BTLA, TIGIT, LAIR1, CD160, 2B4 and TGFR beta.

In one aspect, the fusion protein described herein can be used to eradicate a normal cell that express a tumor antigen as described herein, thereby applicable for use as a cellular conditioning therapy prior to cell transplantation. In one aspect, the normal cell that expresses a tumor antigen as described herein is a normal stem cell and the cell transplantation is a stem cell transplantation.

### METHODS OF TREATMENT OF AUTOANTIBODY OR ALLOANTIBODY CONDITIONS

Also disclosed but not forming part of the invention as claimed are methods for treating autoantibody or alloantibody diseases or conditions in a subject by targeting B cells, including the autoantibody or alloantibody producing B cells, with anti-B cell modified T cells. In one instance of the disclosure which does not form part of the invention as claimed, after B cell depletion by the modified T cells, or following the onset of an adverse reaction to the modified T cells, the modified T cells are then selectively ablated in the subject by activating a suicide gene that has been inserted into the modified T cells.

In one aspect, the disclosure includes a method for treating an autoantibody or alloantibody disease or condition in a subject in need thereof comprising administering an effective amount of a pharmaceutical composition comprising a modified T cell to the subject. The modified T cell comprises a nucleic acid comprising a suicide gene and a nucleic acid encoding a chimeric antigen receptor comprising an anti-B cell binding domain, a transmembrane domain, a costimulatory domain and an intracellular signaling domain.

In one instance, the method further comprises inducing expression of the suicide gene to produce a suicide gene product that induces cell death of the modified T cell. In one such instance, administering an inducing agent induces expression of the suicide gene. In another instance, inducing expression of the suicide gene occurs after the modified T cell exerts cytotoxic function against B cells or after an onset of an adverse reaction in the subject to the modified T cell.

In another instance, the method further comprises activating a suicide gene product of the suicide gene to induce cell death of the modified T cell. In one such instance, administering an activating agent to promote dimerization of the suicide gene product to activate the suicide gene product. In another instance, activating the suicide gene product occurs after the modified T cell exerts cytotoxic function against B cells or after an onset of an adverse reaction in the subject to the modified T cell or after therapeutic effect is achieved.

In yet another instance, the method further comprises inhibiting expression of the suicide gene to inhibit cell death of the modified T cell. In one such instance, administering an inhibiting agent inhibits expression of the suicide gene. In another instance, administering the inhibiting agent occurs concurrently with administration of the modified T cell and continues as the modified T cell exerts cytotoxic function against B cells and may be ceased after an onset of an adverse reaction in the subject to the modified T cell.

In still another instance, the method comprises repressing activation of a suicide gene product of the suicide gene to repress cell death of the modified T cell. In one such instance, administering a solubilizing agent prevents dimerization of the suicide gene product to prevent activation the suicide gene product. In another instance, administering the solubilizing agent occurs concurrently with administration of the modified T cell and continues as the modified T cell exerts cytotoxic function against B cells and may be ceased after an onset of an adverse reaction in the subject to the modified T cell or after therapeutic effect is achieved.

In yet another instance, the method further comprises administering a solubilizing agent to prevent dimerization of the CAR, wherein the CAR is linked to a dimerization domain, such as a dimerization domain of FKBP or similar molecule. In such an instance, the CAR molecules spontaneously aggregate in the cytoplasm or other internal location, thereby preventing presentation of the CAR molecules on the surface of the modified T cell. In another instance, administering the solubilizing agent occurs concurrently with administration of the modified T cell and continues as the modified T cell exerts cytotoxic function against B cells and may be ceased after an onset of an adverse reaction in the subject to the modified T cell or after therapeutic effect is achieved.

The modified T cells can be administered to an animal, preferably a mammal, even more preferably a human, to suppress an immune reaction, such as those common to an autoimmune disease or condition or an alloantibody disease or condition.

In addition, the modified T cells of the present disclosure can be used for the treatment of any condition in which a diminished or otherwise inhibited immune response, especially a cell-mediated immune response, is desired in order to treat or alleviate the disease. In one aspect, the disclosure includes treating a condition, such as an autoantibody or alloantibody disease, in a subject, comprising administering to the subject a therapeutically effective amount of a pharmaceutical composition comprising the modified T cell described herein.

Examples of autoantibody diseases or conditions include, but are not limited to, bullous pemphigoid, epidermolysis bullosa acquisita, p200 pemphigoid, linear IgA bullous dermatosis, other pemphigoid group diseases, dermatitis herpetiformis, celiac disease, myasthenia gravis, Goodpasture's syndrome, granulomatosis with polyangiitis and other ANCA+ vasculitidies, autoimmune limbic encephalitis, anti-N-methyl-D-aspartate receptor encephalitis, neuromyelitis optica, autoimmune hemolytic anemia, autoantibody-associated end-organ damage in lupus and other connective tissue diseases (due to antidsDNA, anti-Ro, and other autoantibodies), Graves' and Hashimoto's thyroiditis, anti-insulin antibodies in diabetes, anti-insulin receptor antibodies in autoimmune hypoglycemia, cryoglobulinemia, rheumatoid arthritis, multiple sclerosis, Sjogren's syndrome, dermatomyositis, anti-Fc-epsilon receptor antibodies in chronic idiopathic urticaria, anti-folate receptor antibodies, anti-endothelial receptor or anti-adrenergic receptor antibodies in pulmonary arterial hypertension, refractory hypertension, dilated cadiomyopathy, and an autoinflammatory syndrome.

An example of alloantibody disease or condition, includes, but are not limited to, an immune reaction resulting from organ transplant, blood transfusion and production of donor-specific HLA and/or b2-microglobulin antibodies, pregnancy sensitization against blood group or Rh antigens or protein replacement therapy, such as with Factor VIII, α-L-iduronidase, type VII collagen, etc. including, but not limited to, the replacement proteins listed in Gorzelany, et al. (Sci. Transl. Med., 5(178):178s10, 2013).

In one instance, the disease or condition is pemphigus. Longitudinal analysis of B cell repertoires in pemphigus patients during treatment has shown that pemphigus patients relapse with the same anti-Dsg B cell clones observed in active disease. Collectively these data indicate that a single, but truly complete, B cell depletion eliminating all anti-Dsg B cell clones by targeting a pan-B cell marker, such as CD20 or alternatively CD19, may be curative in pemphigus patients. The pathologic clones will be eliminated, a healthy B cell repertoire will reform, and another rare break in tolerance is unlikely to recur.

By analogy, this same approach should also be effective for any B-cell or antibody-mediated disease; for example diseases caused by alloantibodies resulting from blood transfusion or sensitization during pregnancy, donor-specific HLA alloantibodies that complicate organ transplant, and alloantibodies resulting from protein replacement therapy for genetic diseases. Even if alloantibodies recur due to reexposure to alloantigen, intermittent re-treatment to completely, but transiently, eliminate B cells (and consequently alloantibodies) will be beneficial in these diseases.

### PHARMACEUTICAL COMPOSITIONS

Pharmaceutical compositions of the present disclosure may comprise any fusion protein, nucleic acid encoding such a fusion protein, as described herein, and one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are in one aspect formulated for intravenous administration.

Pharmaceutical compositions of the present disclosure may be administered in a manner appropriate to the disease to be treated (or prevented). The quantity and frequency of administration will be determined by such factors as the condition of the patient, and the type and severity of the patient's disease, although appropriate dosages may be determined by clinical trials.

In one aspect, the disclosure includes a pharmaceutical composition formulated for use in the method as described herein, the composition comprising a modified T cell comprising a nucleic acid encoding a suicide gene and a nucleic acid encoding a chimeric antigen receptor comprising an anti-B cell binding domain, a transmembrane domain, a costimulatory domain and an intracellular signaling domain.

In another aspect, the disclosure includes a pharmaceutical composition formulated for use in the method as described herein, the composition comprising a modified T cell comprising a nucleic acid encoding a dimerization domain and a chimeric antigen receptor (CAR) comprising an anti-B cell binding domain, a transmembrane domain, a costimulatory domain and an intracellular signaling domain In one instance, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one instance, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-tumor effective amount," "a tumor-inhibiting effective amount," or "therapeutic amount" is indicated, the precise amount of the compositions of the present disclosureto be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In certain aspects, it may be desired to administer activated immune effector cells (e.g., T cells, NK cells) to a subject and then subsequently redraw blood (or have an apheresis performed), activate immune effector cells (e.g., T cells, NK cells) therefrom according to the present disclosure, and reinfuse the patient with these activated and expanded immune effector cells (e.g., T cells, NK cells). This process can be carried out multiple times every few weeks. In certain aspects, immune effector cells (e.g., T cells, NK

cells) can be activated from blood draws of from 10cc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

The administration of the subject compositions may be carried out in any convenient manner, including by aerosol inhalation, injection, ingestion, transfusion, implantation or transplantation. The compositions described herein may be administered to a patient transarterially, subcutaneously, intradermally, intratumorally, intranodally, intramedullary, intramuscularly, by intravenous (i.v.) injection, or intraperitoneally. In one aspect, the cell compositions of the present disclosureare administered to a patient by intradermal or subcutaneous injection. In one aspect, the cell compositions of the present disclosure are administered by i.v. injection. The compositions of immune effector cells (e.g., T cells, NK cells) may be injected directly into a tumor, lymph node, or site of infection.

In a particular exemplary aspect, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more CAR constructs of the disclosure may be introduced, thereby creating a CAR T cell of the disclosure. Subjects in need thereof may subsequently undergo standard treatment with high dose chemotherapy followed by peripheral blood stem cell transplantation. In certain aspects, following or concurrent with the transplant, subjects receive an infusion of the expanded CAR T cells of the present disclosure. In an additional aspect, expanded cells are administered before or following surgery.

The dosage of the above treatments to be administered to a patient will vary with the precise nature of the condition being treated and the recipient of the treatment. The scaling of dosages for human administration can be performed according to art-accepted practices.

### EXAMPLES

### EXAMPLE 1: MATERIALS AND METHODS FOR EXAMPLES 1-13.

### Constructs

The following constructs shown in Table 22 were used in Examples 1-13.

Note that the invention encompasses both orientations of heavy and light chain.

**Table 22. Sequences of various constructs (in bold is the signal peptide used; underlined are the furin cleavage sites.)**

| **Construct** | **Protein Sequence** | **DNA sequence** |
|---|---|---|
| ERₘᵤₜ_Furin_ Flexi-12 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLR TKRIWELKKDVYVVELDWYPDA PGEMVVLTCDTPEEDGITWTLD QSSEVLGSGKTLTIQVKEFGDA GQYTCHKGGEVLSHSLLLLHKK EDGIWSTDILKDQKEPKNKTFLR CEAKNYSGRFTCWWLTTISTDL TFSVKSSRGSSDPQGVTCGAAT LSAERVRGDNKEYEYSVECQED SACPAAEESLPIEVMVDAVHKLK YENYTSSFFIRDIIKPDPPKNLQL KPLKNSRQVEVSWEYPDTWST PHSYFSLTFCVQVQGKSKREKK DRVFTDKTSATVICRKNASISVR AQDRYYSSSWSEWASVPCSGG GSGGGSGGGSRNLPVATPDPG MFPCLHHSQNLLRAVSNMLQKA RQTLEFYPCTSEEIDHEDITKDK TSTVEACLPLELTKNESCLNSRE TSFITNGSCLASRKTSFMMALCL SSIYEDLKMYQVEFKTMNAKLL MDPKRQIFLDQNMLAVIDELMQ ALNFNSETVPQKSSLEEPDFYK TKIKLCILLHAFRIRAVTIDRVMSY LNAS (SEQ ID NO: 48) | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgatt aactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctgc tgattctgagccacatccggcacatgtcgtccaagaggatggaac acctgtacagcatgaagtgcaagaacgtcgtgcctctgtccgatct gctcctggaaatgctggacgcgcacagactccgtactaaaaga (SEQ ID NO: 49) |
| linker | GGGSGGGSGGGSRNLPV (SEQ ID NO: 52) | gtggtggcggtggaagcggcggtggcggaagcggcggtggcg gcagcagaaacctccccgtgg (SEQ ID NO: 53) |
| FKBP L106P | GVQVETISPGDGRTFPKRGQTC VVHYTGMLEDGKKVDSSRDRN KPFKFMLGKQEVIRGWEEGVAQ MSVGQRAKLTISPDYAYGATGH PGIIPPHATLVFDVELLKPE (SEQ ID NO: 56) | |
| DHFR R12Y/G27S/ Y100I | ISLIAALAVDYVIGMENAMPWNL PADLAWFKRNTLNKPVIMGRHT WESIGRPLPGRKNIILSSQPSTD DRVTWVKSVDEAIAACGDVPEI MVIGGGRVIEQFLPKAQKLYLTH IDAEVEGDTHFPDYEPDDWESV FSEFHDADAQNSHSYCFEILER R (SEQ ID NO: 57) | |
| ER | RSSLALSLTADQMVSALLDAEP | |
| T371A_L384 M_M421G_N 519S_G521R _Y537S | PILYSEYDPTRPFSEASMMGLLT NLADRELVHMINWAKRVPGFVD LALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRL (SEQ ID NO: 1119) | |
| CG#c43 FKBPₘᵤₜ_HA_ Furin_CAR19 | MALPVTALLLPLALLLHAARPG VQVETISPGDGRTFPKRGQTCV VHYTGMLEDGKKVDSSRDRNK PFKFMLGKQEVIRGWEEGVAQ MSVGQRAKLTISPDYAYGATGH PGIIPPHATLVFDVELLKPEYPYD VPDYAFPVDRTKREIVMTQSPA TLSLSPGERATLSCRASQDISKY LNWYQQKPGQAPRLLIYHTSRL HSGIPARFSGSGSGTDYTLTISS LQPEDFAVYFCQQGNTLPYTFG QGTKLEIKGGGGSGGGGSGGG GSQVQLQESGPGLVKPSETLSL TCTVSGVSLPDYGVSWIRQPPG KGLEWIGVIWGSETTYYSSSLKS RVTISKDNSKNQVSLKLSSVTAA DTAVYYCAKHYYYGGSYAMDY WGQGTLVTVSSTTTPAPRPPTP APTIASQPLSLRPEACRPAAGG AVHTRGLDFACDIYIWAPLAGTC GVLLLSLVITLYCKRGRKKLLYIF KQPFMRPVQTTQEEDGCSCRF PEEEEGGCELRVKFSRSADAPA YKQGQNQLYNELNLGRREEYD VLDKRRGRDPEMGGKPRRKNP QEGLYNELQKDKMAEAYSEIGM KGERRRGKGHDGLYQGLSTAT KDTYDALHMQALPPR (SEQ ID NO: 59) | atggccctccctgtcaccgccctgctgcttccgctggctcttctgctc cacgccgctcggcccggagtgcaggtggaaaccatctccccag gagacgggcgcaccttccccaagcgcggccagacctgtgtggt gcactacaccgggatgcttgaagatggaaagaaagtcgattcct cccgggacagaaacaagccctttaagtttatgctaggcaagcag gaggtgatccgaggctgggaagaaggggttgcccagatgagtg tgggtcagagagccaaactgactatatctccagattatgcctatgg tgccactgggcacccaggcatcatcccaccacatgccactctcgt cttcgatgtggagcttctaaaaccggaatacccttatgatgttcctg attatgctttccccgtggatcgtactaaaagagaaattgtgatgacc cagtcacccgccactcttagcctttcacccggtgagcgcgcaacc ctgtcttgcagagcctcccaagacatctcaaaataccttaattggta tcaacagaagcccggacaggctcctcgccttctgatctaccacac cagccggctccattctggaatccctgccaggttcagcggtagcgg atctgggaccgactacaccctcactatcagctcactgcagccaga ggacttcgctgtctatttctgtcagcaagggaacaccctgccctac acctttggacagggcaccaagctcgagattaaaggtggaggtgg cagcggaggaggtgggtccggcggtggaggaagccaggtcca actccaagaaagcggaccgggtcttgtgaagccatcagaaactc tttcactgacttgtactgtgagcggagtgtctctccccgattacgggg tgtcttggatcagacagccaccggggaagggtctggaatggattg gagtgatttggggctctgagactacttactactcttcatccctcaagt cacgcgtcaccatctcaaaggacaactctaagaatcaggtgtca ctgaaactgtcatctgtgaccgcagccgacaccgccgtgtactatt gcgctaagcattactattatggcgggagctacgcaatggattactg gggacagggtactctggtcaccgtgtccagcaccactaccccag caccgaggccacccaccccggctcctaccatcgcctcccagcct ctgtccctgcgtccggaggcatgtagacccgcagctggtggggc cgtgcatacccggggtcttgacttcgcctgcgatatctacatttggg cccctctggctggtacttgcggggtcctgctgctttcactcgtgatca ctctttactgtaagcgcggtcggaagaagctgctgtacatctttaag caacccttcatgaggcctgtgcagactactcaagaggaggacgg ctgttcatgccggttcccagaggaggaggaaggcggctgcgaa ctgcgcgtgaaattcagccgcagcgcagatgctccagcctacaa gcaggggcagaaccagctctacaacgaactcaatcttggtcgga gagaggagtacgacgtgctggacaagcggagaggacgggac ccagaaatgggcgggaagccgcgcagaaagaatccccaaga gggcctgtacaacgagctccaaaaggataagatggcagaagc ctatagcgagattggtatgaaaggggaacgcagaagaggcaa aggccacgacggactgtaccagggactcagcaccgccaccaa ggacacctatgacgctcttcacatgcaggccctgccgcctcgg (SEQ ID NO: 60) |
| CG#c44 FKBPₘᵤₜ _HA_Furinmu | MALPVTALLLPLALLLHAARPG VQVETISPGDGRTFPKRGQTCV VHYTGMLEDGKKVDSSRDRNK | atggccctccctgtcaccgccctgctgcttccgctggctcttctgctc cacgccgctcggcccggagtgcaggtggaaaccatctccccag gagacgggcgcaccttccccaagcgcggccagacctgtgtggt |
| t_CAR19 | PFKFMLGKQEVIRGWEEGVAQ MSVGQRAKLTISPDYAYGATGH PGIIPPHATLVFDVELLKPEYPYD VPDYAFPVDATKREIVMTQSPA TLSLSPGERATLSCRASQDISKY LNWYQQKPGQAPRLLIYHTSRL HSGIPARFSGSGSGTDYTLTISS LQPEDFAVYFCQQGNTLPYTFG QGTKLEIKGGGGSGGGGSGGG GSQVQLQESGPGLVKPSETLSL TCTVSGVSLPDYGVSWIRQPPG KGLEWIGVIWGSETTYYSSSLKS RVTISKDNSKNQVSLKLSSVTAA DTAVYYCAKHYYYGGSYAMDY WGQGTLVTVSSTTTPAPRPPTP APTIASQPLSLRPEACRPAAGG AVHTRGLDFACDIYIWAPLAGTC GVLLLSLVITLYCKRGRKKLLYIF KQPFMRPVQTTQEEDGCSCRF PEEEEGGCELRVKFSRSADAPA YKQGQNQLYNELNLGRREEYD VLDKRRGRDPEMGGKPRRKNP QEGLYNELQKDKMAEAYSEIGM KGERRRGKGHDGLYQGLSTAT KDTYDALHMQALPPR (SEQ ID NO: 61) | gcactacaccgggatgcttgaagatggaaagaaagtcgattcct cccgggacagaaacaagccctttaagtttatgctaggcaagcag gaggtgatccgaggctgggaagaaggggttgcccagatgagtg tgggtcagagagccaaactgactatatctccagattatgcctatgg tgccactgggcacccaggcatcatcccaccacatgccactctcgt cttcgatgtggagcttctaaaaccggaatacccttatgatgttcctg attatgctttccccgtggatgctactaaaagagaaattgtgatgacc cagtcacccgccactcttagcctttcacccggtgagcgcgcaacc ctgtcttgcagagcctcccaagacatctcaaaataccttaattggta tcaacagaagcccggacaggctcctcgccttctgatctaccacac cagccggctccattctggaatccctgccaggttcagcggtagcgg atctgggaccgactacaccctcactatcagctcactgcagccaga ggacttcgctgtctatttctgtcagcaagggaacaccctgccctac acctttggacagggcaccaagctcgagattaaaggtggaggtgg cagcggaggaggtgggtccggcggtggaggaagccaggtcca actccaagaaagcggaccgggtcttgtgaagccatcagaaactc tttcactgacttgtactgtgagcggagtgtctctccccgattacgggg tgtcttggatcagacagccaccggggaagggtctggaatggattg gagtgatttggggctctgagactacttactactcttcatccctcaagt cacgcgtcaccatctcaaaggacaactctaagaatcaggtgtca ctgaaactgtcatctgtgaccgcagccgacaccgccgtgtactatt gcgctaagcattactattatggcgggagctacgcaatggattactg gggacagggtactctggtcaccgtgtccagcaccactaccccag caccgaggccacccaccccggctcctaccatcgcctcccagcct ctgtccctgcgtccggaggcatgtagacccgcagctggtggggc cgtgcatacccggggtcttgacttcgcctgcgatatctacatttggg cccctctggctggtacttgcggggtcctgctgctttcactcgtgatca ctctttactgtaagcgcggtcggaagaagctgctgtacatctttaag caacccttcatgaggcctgtgcagactactcaagaggaggacgg ctgttcatgccggttcccagaggaggaggaaggcggctgcgaa ctgcgcgtgaaattcagccgcagcgcagatgctccagcctacaa gcaggggcagaaccagctctacaacgaactcaatcttggtcgga gagaggagtacgacgtgctggacaagcggagaggacgggac ccagaaatgggcgggaagccgcgcagaaagaatccccaaga gggcctgtacaacgagctccaaaaggataagatggcagaagc ctatagcgagattggtatgaaaggggaacgcagaagaggcaa aggccacgacggactgtaccagggactcagcaccgccaccaa ggacacctatgacgctcttcacatgcaggccctgccgcctcgg (SEQ ID NO: 62) |
| CG#c45 DHFRₘᵤₜ_HA _Furin_CAR1 9 | MALPVTALLLPLALLLHAARPIS LIAALAVDYVIGMENAMPWNLPA DLAWFKRNTLNKPVIMGRHTWE SIGRPLPGRKNIILSSQPSTDDR VTWVKSVDEAIAACGDVPEIMVI GGGRVIEQFLPKAQKLYLTHIDA EVEGDTHFPDYEPDDWESVFS EFHDADAQNSHSYCFEILERRY PYDVPDYAFPVDRTKREIVMTQ SPATLSLSPGERATLSCRASQDI SKYLNWYQQKPGQAPRLLIYHT SRLHSGIPARFSGSGSGTDYTL TISSLQPEDFAVYFCQQGNTLPY TFGQGTKLEIKGGGGSGGGGS GGGGSQVQLQESGPGLVKPSE TLSLTCTVSGVSLPDYGVSWIR QPPGKGLEWIGVIWGSETTYYS | atggccctccctgtcaccgccctgctgcttccgctggctcttctgctc cacgccgctcggcccatctctctgattgccgctctggccgtggact acgtgatcgggatggaaaacgctatgccatggaatctgcccgcc gatctggcttggttcaagaggaacaccctgaacaagccagtgat catgggcagacacacttgggagtccattggccggcccctgcctg gacgcaagaacatcattctgagctcccagccctctaccgacgac agggtgacatgggtgaaaagtgtggacgaagccattgccgcttg cggagatgtgcccgagatcatggtcatcggcggagggagagtg atcgagcagttcctgcctaaggcccagaaactgtacctgactcac attgacgctgaggtggaaggggacacccattttcctgattatgagc cagacgattgggaaagcgtgttctccgagtttcacgacgccgatg ctcagaactctcatagttattgctttgagatcctggaaaggagatac ccttatgatgttcctgattatgctttccccgtggatcgtactaaaaga gaaattgtgatgacccagtcacccgccactcttagcctttcacccg gtgagcgcgcaaccctgtcttgcagagcctcccaagacatctca aaataccttaattggtatcaacagaagcccggacaggctcctcgc cttctgatctaccacaccagccggctccattctggaatccctgcca |
| | SSLKSRVTISKDNSKNQVSLKLS SVTAADTAVYYCAKHYYYGGSY AMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY SEIGMKGERRRGKGHDGLYQG LSTATKDTYDALHMQALPPR (SEQ ID NO: 63) | ggttcagcggtagcggatctgggaccgactacaccctcactatca gctcactgcagccagaggacttcgctgtctatttctgtcagcaagg gaacaccctgccctacacctttggacagggcaccaagctcgag attaaaggtggaggtggcagcggaggaggtgggtccggcggtg gaggaagccaggtccaactccaagaaagcggaccgggtcttgt gaagccatcagaaactctttcactgacttgtactgtgagcggagtg tctctccccgattacggggtgtcttggatcagacagccaccgggg aagggtctggaatggattggagtgatttggggctctgagactactt actactcttcatccctcaagtcacgcgtcaccatctcaaaggacaa ctctaagaatcaggtgtcactgaaactgtcatctgtgaccgcagcc gacaccgccgtgtactattgcgctaagcattactattatggcggga gctacgcaatggattactggggacagggtactctggtcaccgtgt ccagcaccactaccccagcaccgaggccacccaccccggctc ctaccatcgcctcccagcctctgtccctgcgtccggaggcatgtag acccgcagctggtggggccgtgcatacccggggtcttgacttcgc ctgcgatatctacatttgggcccctctggctggtacttgcggggtcct gctgctttcactcgtgatcactctttactgtaagcgcggtcggaaga agctgctgtacatctttaagcaacccttcatgaggcctgtgcagact actcaagaggaggacggctgttcatgccggttcccagaggagg aggaaggcggctgcgaactgcgcgtgaaattcagccgcagcgc agatgctccagcctacaagcaggggcagaaccagctctacaac gaactcaatcttggtcggagagaggagtacgacgtgctggacaa gcggagaggacgggacccagaaatgggcgggaagccgcgc agaaagaatccccaagagggcctgtacaacgagctccaaaag gataagatggcagaagcctatagcgagattggtatgaaagggg aacgcagaagaggcaaaggccacgacggactgtaccaggga ctcagcaccgccaccaaggacacctatgacgctcttcacatgca ggccctgccgcctcgg (SEQ ID NO: 64) |
| CG#c46 DHFRₘᵤₜ _HA_Furinmu t_CAR19 | MALPVTALLLPLALLLHAARPIS LIAALAVDYVIGMENAMPWNLPA DLAWFKRNTLNKPVIMGRHTWE SIGRPLPGRKNIILSSQPSTDDR VTWVKSVDEAIAACGDVPEIMVI GGGRVIEQFLPKAQKLYLTHIDA EVEGDTHFPDYEPDDWESVFS EFHDADAQNSHSYCFEILERRY PYDVPDYAFPVDATKREIVMTQ SPATLSLSPGERATLSCRASQDI SKYLNWYQQKPGQAPRLLIYHT SRLHSGIPARFSGSGSGTDYTL TISSLQPEDFAVYFCQQGNTLPY TFGQGTKLEIKGGGGSGGGGS GGGGSQVQLQESGPGLVKPSE TLSLTCTVSGVSLPDYGVSWIR QPPGKGLEWIGVIWGSETTYYS SSLKSRVTISKDNSKNQVSLKLS SVTAADTAVYYCAKHYYYGGSY AMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY | atggccctccctgtcaccgccctgctgcttccgctggctcttctgctc cacgccgctcggcccatctctctgattgccgctctggccgtggact acgtgatcgggatggaaaacgctatgccatggaatctgcccgcc gatctggcttggttcaagaggaacaccctgaacaagccagtgat catgggcagacacacttgggagtccattggccggcccctgcctg gacgcaagaacatcattctgagctcccagccctctaccgacgac agggtgacatgggtgaaaagtgtggacgaagccattgccgcttg cggagatgtgcccgagatcatggtcatcggcggagggagagtg atcgagcagttcctgcctaaggcccagaaactgtacctgactcac attgacgctgaggtggaaggggacacccattttcctgattatgagc cagacgattgggaaagcgtgttctccgagtttcacgacgccgatg ctcagaactctcatagttattgctttgagatcctggaaaggagatac ccttatgatgttcctgattatgctttccccgtggatgctactaaaaga gaaattgtgatgacccagtcacccgccactcttagcctttcacccg gtgagcgcgcaaccctgtcttgcagagcctcccaagacatctca aaataccttaattggtatcaacagaagcccggacaggctcctcgc cttctgatctaccacaccagccggctccattctggaatccctgcca ggttcagcggtagcggatctgggaccgactacaccctcactatca gctcactgcagccagaggacttcgctgtctatttctgtcagcaagg gaacaccctgccctacacctttggacagggcaccaagctcgag attaaaggtggaggtggcagcggaggaggtgggtccggcggtg gaggaagccaggtccaactccaagaaagcggaccgggtcttgt gaagccatcagaaactctttcactgacttgtactgtgagcggagtg tctctccccgattacggggtgtcttggatcagacagccaccgggg aagggtctggaatggattggagtgatttggggctctgagactactt actactcttcatccctcaagtcacgcgtcaccatctcaaaggacaa ctctaagaatcaggtgtcactgaaactgtcatctgtgaccgcagcc gacaccgccgtgtactattgcgctaagcattactattatggcggga |
| | SEIGMKGERRRGKGHDGLYQG LSTATKDTYDALHMQALPPR (SEQ ID NO: 65) | gctacgcaatggattactggggacagggtactctggtcaccgtgt ccagcaccactaccccagcaccgaggccacccaccccggctc ctaccatcgcctcccagcctctgtccctgcgtccggaggcatgtag acccgcagctggtggggccgtgcatacccggggtcttgacttcgc ctgcgatatctacatttgggcccctctggctggtacttgcggggtcct gctgctttcactcgtgatcactctttactgtaagcgcggtcggaaga agctgctgtacatctttaagcaacccttcatgaggcctgtgcagact actcaagaggaggacggctgttcatgccggttcccagaggagg aggaaggcggctgcgaactgcgcgtgaaattcagccgcagcgc agatgctccagcctacaagcaggggcagaaccagctctacaac gaactcaatcttggtcggagagaggagtacgacgtgctggacaa gcggagaggacgggacccagaaatgggcgggaagccgcgc agaaagaatccccaagagggcctgtacaacgagctccaaaag gataagatggcagaagcctatagcgagattggtatgaaagggg aacgcagaagaggcaaaggccacgacggactgtaccaggga ctcagcaccgccaccaaggacacctatgacgctcttcacatgca ggccctgccgcctcgg (SEQ ID NO: 66) |
| CG#c47 ERₘᵤₜ_HA_Fu rin-CAR19 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLY PYDVPDYAFPVDRTKREIVMTQ SPATLSLSPGERATLSCRASQDI SKYLNWYQQKPGQAPRLLIYHT SRLHSGIPARFSGSGSGTDYTL TISSLQPEDFAVYFCQQGNTLPY TFGQGTKLEIKGGGGSGGGGS GGGGSQVQLQESGPGLVKPSE TLSLTCTVSGVSLPDYGVSWIR QPPGKGLEWIGVIWGSETTYYS SSLKSRVTISKDNSKNQVSLKLS SVTAADTAVYYCAKHYYYGGSY AMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY SEIGMKGERRRGKGHDGLYQG LSTATKDTYDALHMQALPPR (SEQ ID NO: 67) | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactctacccttatgatg ttcctgattatgctttccccgtggatcgtactaaaagagaaattgtga tgacccagtcacccgccactcttagcctttcacccggtgagcgcg caaccctgtcttgcagagcctcccaagacatctcaaaatacctta attggtatcaacagaagcccggacaggctcctcgccttctgatcta ccacaccagccggctccattctggaatccctgccaggttcagcgg tagcggatctgggaccgactacaccctcactatcagctcactgca gccagaggacttcgctgtctatttctgtcagcaagggaacaccctg ccctacacctttggacagggcaccaagctcgagattaaaggtgg aggtggcagcggaggaggtgggtccggcggtggaggaagcca ggtccaactccaagaaagcggaccgggtcttgtgaagccatcag aaactctttcactgacttgtactgtgagcggagtgtctctccccgatt acggggtgtcttggatcagacagccaccggggaagggtctgga atggattggagtgatttggggctctgagactacttactactcttcatc cctcaagtcacgcgtcaccatctcaaaggacaactctaagaatc aggtgtcactgaaactgtcatctgtgaccgcagccgacaccgcc gtgtactattgcgctaagcattactattatggcgggagctacgcaat ggattactggggacagggtactctggtcaccgtgtccagcaccac taccccagcaccgaggccacccaccccggctcctaccatcgcct cccagcctctgtccctgcgtccggaggcatgtagacccgcagctg gtggggccgtgcatacccggggtcttgacttcgcctgcgatatcta catttgggcccctctggctggtacttgcggggtcctgctgctttcact |
| | | cgtgatcactctttactgtaagcgcggtcggaagaagctgctgtac atctttaagcaacccttcatgaggcctgtgcagactactcaagagg aggacggctgttcatgccggttcccagaggaggaggaaggcgg ctgcgaactgcgcgtgaaattcagccgcagcgcagatgctccag cctacaagcaggggcagaaccagctctacaacgaactcaatctt ggtcggagagaggagtacgacgtgctggacaagcggagagg acgggacccagaaatgggcgggaagccgcgcagaaagaatc cccaagagggcctgtacaacgagctccaaaaggataagatgg cagaagcctatagcgagattggtatgaaaggggaacgcagaa gaggcaaaggccacgacggactgtaccagggactcagcaccg ccaccaaggacacctatgacgctcttcacatgcaggccctgccg cctcgg (SEQ ID NO: 68) |
| CG#c48 ERₘᵤₜ_HA_Fu rinMmut_CA R19 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLY PYDVPDYAFPVDATKREIVMTQ SPATLSLSPGERATLSCRASQDI SKYLNWYQQKPGQAPRLLIYHT SRLHSGIPARFSGSGSGTDYTL TISSLQPEDFAVYFCQQGNTLPY TFGQGTKLEIKGGGGSGGGGS GGGGSQVQLQESGPGLVKPSE TLSLTCTVSGVSLPDYGVSWIR QPPGKGLEWIGVIWGSETTYYS SSLKSRVTISKDNSKNQVSLKLS SVTAADTAVYYCAKHYYYGGSY AMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY SEIGMKGERRRGKGHDGLYQG LSTATKDTYDALHMQALPPR (SEQ ID NO: 69) | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactctacccttatgatg ttcctgattatgctttccccgtggatgctactaaaagagaaattgtga tgacccagtcacccgccactcttagcctttcacccggtgagcgcg caaccctgtcttgcagagcctcccaagacatctcaaaatacctta attggtatcaacagaagcccggacaggctcctcgccttctgatcta ccacaccagccggctccattctggaatccctgccaggttcagcgg tagcggatctgggaccgactacaccctcactatcagctcactgca gccagaggacttcgctgtctatttctgtcagcaagggaacaccctg ccctacacctttggacagggcaccaagctcgagattaaaggtgg aggtggcagcggaggaggtgggtccggcggtggaggaagcca ggtccaactccaagaaagcggaccgggtcttgtgaagccatcag aaactctttcactgacttgtactgtgagcggagtgtctctccccgatt acggggtgtcttggatcagacagccaccggggaagggtctgga atggattggagtgatttggggctctgagactacttactactcttcatc cctcaagtcacgcgtcaccatctcaaaggacaactctaagaatc aggtgtcactgaaactgtcatctgtgaccgcagccgacaccgcc gtgtactattgcgctaagcattactattatggcgggagctacgcaat ggattactggggacagggtactctggtcaccgtgtccagcaccac taccccagcaccgaggccacccaccccggctcctaccatcgcct cccagcctctgtccctgcgtccggaggcatgtagacccgcagctg gtggggccgtgcatacccggggtcttgacttcgcctgcgatatcta catttgggcccctctggctggtacttgcggggtcctgctgctttcact cgtgatcactctttactgtaagcgcggtcggaagaagctgctgtac atctttaagcaacccttcatgaggcctgtgcagactactcaagagg aggacggctgttcatgccggttcccagaggaggaggaaggcgg ctgcgaactgcgcgtgaaattcagccgcagcgcagatgctccag cctacaagcaggggcagaaccagctctacaacgaactcaatctt |
| | | ggtcggagagaggagtacgacgtgctggacaagcggagagg acgggacccagaaatgggcgggaagccgcgcagaaagaatc cccaagagggcctgtacaacgagctccaaaaggataagatgg cagaagcctatagcgagattggtatgaaaggggaacgcagaa gaggcaaaggccacgacggactgtaccagggactcagcaccg ccaccaaggacacctatgacgctcttcacatgcaggccctgccg cctcgg (SEQ ID NO: 70) |
| CG#c71 FKBPₘᵤₜ_HA_ noFurin_CAR 19 | MALPVTALLLPLALLLHAARPG VQVETISPGDGRTFPKRGQTCV VHYTGMLEDGKKVDSSRDRNK PFKFMLGKQEVIRGWEEGVAQ MSVGQRAKLTISPDYAYGATGH PGIIPPHATLVFDVELLKPEYPYD VPDYAFPVDEIVMTQSPATLSLS PGERATLSCRASQDISKYLNWY QQKPGQAPRLLIYHTSRLHSGIP ARFSGSGSGTDYTLTISSLQPED FAVYFCQQGNTLPYTFGQGTKL EIKGGGGSGGGGSGGGGSQV QLQESGPGLVKPSETLSLTCTV SGVSLPDYGVSWIRQPPGKGLE WIGVIWGSETTYYSSSLKSRVTI SKDNSKNQVSLKLSSVTAADTA VYYCAKHYYYGGSYAMDYWGQ GTLVTVSSTTTPAPRPPTPAPTI ASQPLSLRPEACRPAAGGAVHT RGLDFACDIYIWAPLAGTCGVLL LSLVITLYCKRGRKKLLYIFKQPF MRPVQTTQEEDGCSCRFPEEE EGGCELRVKFSRSADAPAYKQ GQNQLYNELNLGRREEYDVLDK RRGRDPEMGGKPRRKNPQEGL YNELQKDKMAEAYSEIGMKGER RRGKGHDGLYQGLSTATKDTY DALHMQALPPR (SEQ ID NO: 71) | atggccctccctgtcaccgccctgctgcttccgctggctcttctgctc cacgccgctcggcccggagtgcaggtggaaaccatctccccag gagacgggcgcaccttccccaagcgcggccagacctgtgtggt gcactacaccgggatgcttgaagatggaaagaaagtcgattcct cccgggacagaaacaagccctttaagtttatgctaggcaagcag gaggtgatccgaggctgggaagaaggggttgcccagatgagtg tgggtcagagagccaaactgactatatctccagattatgcctatgg tgccactgggcacccaggcatcatcccaccacatgccactctcgt cttcgatgtggagcttctaaaaccggaatacccttatgatgttcctg attatgctttccccgtggatgaaattgtgatgacccagtcacccgcc actcttagcctttcacccggtgagcgcgcaaccctgtcttgcagag cctcccaagacatctcaaaataccttaattggtatcaacagaagc ccggacaggctcctcgccttctgatctaccacaccagccggctcc attctggaatccctgccaggttcagcggtagcggatctgggaccg actacaccctcactatcagctcactgcagccagaggacttcgctgt ctatttctgtcagcaagggaacaccctgccctacacctttggacag ggcaccaagctcgagattaaaggtggaggtggcagcggagga ggtgggtccggcggtggaggaagccaggtccaactccaagaa agcggaccgggtcttgtgaagccatcagaaactctttcactgactt gtactgtgagcggagtgtctctccccgattacggggtgtcttggatc agacagccaccggggaagggtctggaatggattggagtgatttg gggctctgagactacttactactcttcatccctcaagtcacgcgtca ccatctcaaaggacaactctaagaatcaggtgtcactgaaactgt catctgtgaccgcagccgacaccgccgtgtactattgcgctaagc attactattatggcgggagctacgcaatggattactggggacagg gtactctggtcaccgtgtccagcaccactaccccagcaccgagg ccacccaccccggctcctaccatcgcctcccagcctctgtccctgc gtccggaggcatgtagacccgcagctggtggggccgtgcatacc cggggtcttgacttcgcctgcgatatctacatttgggcccctctggct ggtacttgcggggtcctgctgctttcactcgtgatcactctttactgta agcgcggtcggaagaagctgctgtacatctttaagcaacccttcat gaggcctgtgcagactactcaagaggaggacggctgttcatgcc ggttcccagaggaggaggaaggcggctgcgaactgcgcgtga aattcagccgcagcgcagatgctccagcctacaagcaggggca gaaccagctctacaacgaactcaatcttggtcggagagaggagt acgacgtgctggacaagcggagaggacgggacccagaaatg ggcgggaagccgcgcagaaagaatccccaagagggcctgtac aacgagctccaaaaggataagatggcagaagcctatagcgag attggtatgaaaggggaacgcagaagaggcaaaggccacgac ggactgtaccagggactcagcaccgccaccaaggacacctatg acgctcttcacatgcaggccctgccgcctcgg (SEQ ID NO: 72) |
| CG#c72 ERₘᵤₜ_HA_no FurinCAR19 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat |
| | MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLY PYDVPDYAFPVDEIVMTQSPATL SLSPGERATLSCRASQDISKYLN WYQQKPGQAPRLLIYHTSRLHS GIPARFSGSGSGTDYTLTISSLQ PEDFAVYFCQQGNTLPYTFGQG TKLEIKGGGGSGGGGSGGGGS QVQLQESGPGLVKPSETLSLTC TVSGVSLPDYGVSWIRQPPGKG LEWIGVIWGSETTYYSSSLKSRV TISKDNSKNQVSLKLSSVTAADT AVYYCAKHYYYGGSYAMDYWG QGTLVTVSSTTTPAPRPPTPAPT IASQPLSLRPEACRPAAGGAVH TRGLDFACDIYIWAPLAGTCGVL LLSLVITLYCKRGRKKLLYIFKQP FMRPVQTTQEEDGCSCRFPEE EEGGCELRVKFSRSADAPAYKQ GQNQLYNELNLGRREEYDVLDK RRGRDPEMGGKPRRKNPQEGL YNELQKDKMAEAYSEIGMKGER RRGKGHDGLYQGLSTATKDTY DALHMQALPPR (SEQ ID NO: 73) | gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactctacccttatgatg ttcctgattatgctttccccgtggatgaaattgtgatgacccagtcac ccgccactcttagcctttcacccggtgagcgcgcaaccctgtcttg cagagcctcccaagacatctcaaaataccttaattggtatcaaca gaagcccggacaggctcctcgccttctgatctaccacaccagcc ggctccattctggaatccctgccaggttcagcggtagcggatctgg gaccgactacaccctcactatcagctcactgcagccagaggactt cgctgtctatttctgtcagcaagggaacaccctgccctacacctttg gacagggcaccaagctcgagattaaaggtggaggtggcagcg gaggaggtgggtccggcggtggaggaagccaggtccaactcc aagaaagcggaccgggtcttgtgaagccatcagaaactctttca ctgacttgtactgtgagcggagtgtctctccccgattacggggtgtct tggatcagacagccaccggggaagggtctggaatggattggagt gatttggggctctgagactacttactactcttcatccctcaagtcacg cgtcaccatctcaaaggacaactctaagaatcaggtgtcactgaa actgtcatctgtgaccgcagccgacaccgccgtgtactattgcgct aagcattactattatggcgggagctacgcaatggattactgggga cagggtactctggtcaccgtgtccagcaccactaccccagcacc gaggccacccaccccggctcctaccatcgcctcccagcctctgtc cctgcgtccggaggcatgtagacccgcagctggtggggccgtgc atacccggggtcttgacttcgcctgcgatatctacatttgggcccct ctggctggtacttgcggggtcctgctgctttcactcgtgatcactcttt actgtaagcgcggtcggaagaagctgctgtacatctttaagcaac ccttcatgaggcctgtgcagactactcaagaggaggacggctgtt catgccggttcccagaggaggaggaaggcggctgcgaactgc gcgtgaaattcagccgcagcgcagatgctccagcctacaagca ggggcagaaccagctctacaacgaactcaatcttggtcggaga gaggagtacgacgtgctggacaagcggagaggacgggaccc agaaatgggcgggaagccgcgcagaaagaatccccaagagg gcctgtacaacgagctccaaaaggataagatggcagaagccta tagcgagattggtatgaaaggggaacgcagaagaggcaaagg ccacgacggactgtaccagggactcagcaccgccaccaagga cacctatgacgctcttcacatgcaggccctgccgcctcgg (SEQ ID NO: 74) |
| CG#c73 ERₘᵤₜ_noHa_ FurinCAR19 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLR | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg |
| | TKREIVMTQSPATLSLSPGERAT LSCRASQDISKYLNWYQQKPGQ APRLLIYHTSRLHSGIPARFSGS GSGTDYTLTISSLQPEDFAVYFC QQGNTLPYTFGQGTKLEIKGGG GSGGGGSGGGGSQVQLQESG PGLVKPSETLSLTCTVSGVSLPD YGVSWIRQPPGKGLEWIGVIWG SETTYYSSSLKSRVTISKDNSKN QVSLKLSSVTAADTAVYYCAKH YYYGGSYAMDYWGQGTLVTVS STTTPAPRPPTPAPTIASQPLSL RPEACRPAAGGAVHTRGLDFA CDIYIWAPLAGTCGVLLLSLVITL YCKRGRKKLLYIFKQPFMRPVQ TTQEEDGCSCRFPEEEEGGCEL RVKFSRSADAPAYKQGQNQLY NELNLGRREEYDVLDKRRGRDP EMGGKPRRKNPQEGLYNELQK DKMAEAYSEIGMKGERRRGKG HDGLYQGLSTATKDTYDALHMQ ALPPR (SEQ ID NO: 75) | accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactccgtactaaaag agaaattgtgatgacccagtcacccgccactcttagcctttcaccc ggtgagcgcgcaaccctgtcttgcagagcctcccaagacatctc aaaataccttaattggtatcaacagaagcccggacaggctcctcg ccttctgatctaccacaccagccggctccattctggaatccctgcc aggttcagcggtagcggatctgggaccgactacaccctcactatc agctcactgcagccagaggacttcgctgtctatttctgtcagcaag ggaacaccctgccctacacctttggacagggcaccaagctcga gattaaaggtggaggtggcagcggaggaggtgggtccggcggt ggaggaagccaggtccaactccaagaaagcggaccgggtctt gtgaagccatcagaaactctttcactgacttgtactgtgagcggag tgtctctccccgattacggggtgtcttggatcagacagccaccggg gaagggtctggaatggattggagtgatttggggctctgagactact tactactcttcatccctcaagtcacgcgtcaccatctcaaaggaca actctaagaatcaggtgtcactgaaactgtcatctgtgaccgcagc cgacaccgccgtgtactattgcgctaagcattactattatggcggg agctacgcaatggattactggggacagggtactctggtcaccgtg tccagcaccactaccccagcaccgaggccacccaccccggctc ctaccatcgcctcccagcctctgtccctgcgtccggaggcatgtag acccgcagctggtggggccgtgcatacccggggtcttgacttcgc ctgcgatatctacatttgggcccctctggctggtacttgcggggtcct gctgctttcactcgtgatcactctttactgtaagcgcggtcggaaga agctgctgtacatctttaagcaacccttcatgaggcctgtgcagact actcaagaggaggacggctgttcatgccggttcccagaggagg aggaaggcggctgcgaactgcgcgtgaaattcagccgcagcgc agatgctccagcctacaagcaggggcagaaccagctctacaac gaactcaatcttggtcggagagaggagtacgacgtgctggacaa gcggagaggacgggacccagaaatgggcgggaagccgcgc agaaagaatccccaagagggcctgtacaacgagctccaaaag gataagatggcagaagcctatagcgagattggtatgaaagggg aacgcagaagaggcaaaggccacgacggactgtaccaggga ctcagcaccgccaccaaggacacctatgacgctcttcacatgca ggccctgccgcctcgg (SEQ ID NO: 76) |
| CG#c74 ERₘᵤₜ_noHa_ noFurin-CAR19 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLEI VMTQSPATLSLSPGERATLSCR ASQDISKYLNWYQQKPGQAPRL LIYHTSRLHSGIPARFSGSGSGT DYTLTISSLQPEDFAVYFCQQG NTLPYTFGQGTKLEIKGGGGSG GGGSGGGGSQVQLQESGPGLV | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactcgaaattgtgatg |
| | KPSETLSLTCTVSGVSLPDYGV SWIRQPPGKGLEWIGVIWGSET TYYSSSLKSRVTISKDNSKNQVS LKLSSVTAADTAVYYCAKHYYY GGSYAMDYWGQGTLVTVSSTT TPAPRPPTPAPTIASQPLSLRPE ACRPAAGGAVHTRGLDFACDIYI WAPLAGTCGVLLLSLVITLYCKR GRKKLLYIFKQPFMRPVQTTQE EDGCSCRFPEEEEGGCELRVKF SRSADAPAYKQGQNQLYNELNL GRREEYDVLDKRRGRDPEMGG KPRRKNPQEGLYNELQKDKMA EAYSEIGMKGERRRGKGHDGL YQGLSTATKDTYDALHMQALPP R (SEQ ID NO: 77) | acccagtcacccgccactcttagcctttcacccggtgagcgcgca accctgtcttgcagagcctcccaagacatctcaaaataccttaatt ggtatcaacagaagcccggacaggctcctcgccttctgatctacc acaccagccggctccattctggaatccctgccaggttcagcggta gcggatctgggaccgactacaccctcactatcagctcactgcagc cagaggacttcgctgtctatttctgtcagcaagggaacaccctgcc ctacacctttggacagggcaccaagctcgagattaaaggtggag gtggcagcggaggaggtgggtccggcggtggaggaagccagg tccaactccaagaaagcggaccgggtcttgtgaagccatcagaa actctttcactgacttgtactgtgagcggagtgtctctccccgattac ggggtgtcttggatcagacagccaccggggaagggtctggaatg gattggagtgatttggggctctgagactacttactactcttcatccctc aagtcacgcgtcaccatctcaaaggacaactctaagaatcaggt gtcactgaaactgtcatctgtgaccgcagccgacaccgccgtgta ctattgcgctaagcattactattatggcgggagctacgcaatggatt actggggacagggtactctggtcaccgtgtccagcaccactacc ccagcaccgaggccacccaccccggctcctaccatcgcctccc agcctctgtccctgcgtccggaggcatgtagacccgcagctggtg gggccgtgcatacccggggtcttgacttcgcctgcgatatctacatt tgggcccctctggctggtacttgcggggtcctgctgctttcactcgtg atcactctttactgtaagcgcggtcggaagaagctgctgtacatctt taagcaacccttcatgaggcctgtgcagactactcaagaggagg acggctgttcatgccggttcccagaggaggaggaaggcggctg cgaactgcgcgtgaaattcagccgcagcgcagatgctccagcct acaagcaggggcagaaccagctctacaacgaactcaatcttggt cggagagaggagtacgacgtgctggacaagcggagaggacg ggacccagaaatgggcgggaagccgcgcagaaagaatcccc aagagggcctgtacaacgagctccaaaaggataagatggcag aagcctatagcgagattggtatgaaaggggaacgcagaagag gcaaaggccacgacggactgtaccagggactcagcaccgcca ccaaggacacctatgacgctcttcacatgcaggccctgccgcctc gg (SEQ ID NO: 78) |
| | | |
| | | |
| | | |
| | | |
| | | |
| | | |
| | | |
| | | |
| | | |
| | | |
| | | |
| ERₘᵤₜ.FurinCAR19=Alt1 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLSL | Atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg |
| | NLTESHNSRKKREIVMTQSPATL SLSPGERATLSCRASQDISKYLN WYQQKPGQAPRLLIYHTSRLHS GIPARFSGSGSGTDYTLTISSLQ PEDFAVYFCQQGNTLPYTFGQG TKLEIKGGGGSGGGGSGGGGS QVQLQESGPGLVKPSETLSLTC TVSGVSLPDYGVSWIRQPPGKG LEWIGVIWGSETTYYSSSLKSRV TISKDNSKNQVSLKLSSVTAADT AVYYCAKHYYYGGSYAMDYWG QGTLVTVSSTTTPAPRPPTPAPT IASQPLSLRPEACRPAAGGAVH TRGLDFACDIYIWAPLAGTCGVL LLSLVITLYCKRGRKKLLYIFKQP FMRPVQTTQEEDGCSCRFPEE EEGGCELRVKFSRSADAPAYKQ GQNQLYNELNLGRREEYDVLDK RRGRDPEMGGKPRRKNPQEGL YNELQKDKMAEAYSEIGMKGER RRGKGHDGLYQGLSTATKDTY DALHMQALPPR (SEQ ID NO: 103) | accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactcagtctcaatttga ctgagtcacacaattccaggaagaaaagggaaattgtgatgacc cagtcacccgccactcttagcctttcacccggtgagcgcgcaacc ctgtcttgcagagcctcccaagacatctcaaaataccttaattggta tcaacagaagcccggacaggctcctcgccttctgatctaccacac cagccggctccattctggaatccctgccaggttcagcggtagcgg atctgggaccgactacaccctcactatcagctcactgcagccaga ggacttcgctgtctatttctgtcagcaagggaacaccctgccctac acctttggacagggcaccaagctcgagattaaaggtggaggtgg cagcggaggaggtgggtccggcggtggaggaagccaggtcca actccaagaaagcggaccgggtcttgtgaagccatcagaaactc tttcactgacttgtactgtgagcggagtgtctctccccgattacgggg tgtcttggatcagacagccaccggggaagggtctggaatggattg gagtgatttggggctctgagactacttactactcttcatccctcaagt cacgcgtcaccatctcaaaggacaactctaagaatcaggtgtca ctgaaactgtcatctgtgaccgcagccgacaccgccgtgtactatt gcgctaagcattactattatggcgggagctacgcaatggattactg gggacagggtactctggtcaccgtgtccagcaccactaccccag caccgaggccacccaccccggctcctaccatcgcctcccagcct ctgtccctgcgtccggaggcatgtagacccgcagctggtggggc cgtgcatacccggggtcttgacttcgcctgcgatatctacatttggg cccctctggctggtacttgcggggtcctgctgctttcactcgtgatca ctctttactgtaagcgcggtcggaagaagctgctgtacatctttaag caacccttcatgaggcctgtgcagactactcaagaggaggacgg ctgttcatgccggttcccagaggaggaggaaggcggctgcgaa ctgcgcgtgaaattcagccgcagcgcagatgctccagcctacaa gcaggggcagaaccagctctacaacgaactcaatcttggtcgga gagaggagtacgacgtgctggacaagcggagaggacgggac ccagaaatgggcgggaagccgcgcagaaagaatccccaaga gggcctgtacaacgagctccaaaaggataagatggcagaagc ctatagcgagattggtatgaaaggggaacgcagaagaggcaa aggccacgacggactgtaccagggactcagcaccgccaccaa ggacacctatgacgctcttcacatgcaggccctgccgcctcgg (SEQ ID NO: 104) |
| ERₘᵤₜ.FurinCAR19=Alt2 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLG TGAEDPRPSRKRREIVMTQSPA TLSLSPGERATLSCRASQDISKY LNWYQQKPGQAPRLLIYHTSRL HSGIPARFSGSGSGTDYTLTISS LQPEDFAVYFCQQGNTLPYTFG | atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat |
| | QGTKLEIKGGGGSGGGGSGGG GSQVQLQESGPGLVKPSETLSL TCTVSGVSLPDYGVSWIRQPPG KGLEWIGVIWGSETTYYSSSLKS RVTISKDNSKNQVSLKLSSVTAA DTAVYYCAKHYYYGGSYAMDY WGQGTLVTVSSTTTPAPRPPTP APTIASQPLSLRPEACRPAAGG AVHTRGLDFACDIYIWAPLAGTC GVLLLSLVITLYCKRGRKKLLYIF KQPFMRPVQTTQEEDGCSCRF PEEEEGGCELRVKFSRSADAPA YKQGQNQLYNELNLGRREEYD VLDKRRGRDPEMGGKPRRKNP QEGLYNELQKDKMAEAYSEIGM KGERRRGKGHDGLYQGLSTAT KDTYDALHMQALPPR (SEQ ID NO: 105) | ctgctcctggaaatgctggacgcgcacagactcgggacgggag ctgaggatccacgacccagcagaaagcgacgggaaattgtgat gacccagtcacccgccactcttagcctttcacccggtgagcgcgc aaccctgtcttgcagagcctcccaagacatctcaaaataccttaat tggtatcaacagaagcccggacaggctcctcgccttctgatctac cacaccagccggctccattctggaatccctgccaggttcagcggt agcggatctgggaccgactacaccctcactatcagctcactgca gccagaggacttcgctgtctatttctgtcagcaagggaacaccctg ccctacacctttggacagggcaccaagctcgagattaaaggtgg aggtggcagcggaggaggtgggtccggcggtggaggaagcca ggtccaactccaagaaagcggaccgggtcttgtgaagccatcag aaactctttcactgacttgtactgtgagcggagtgtctctccccgatt acggggtgtcttggatcagacagccaccggggaagggtctgga atggattggagtgatttggggctctgagactacttactactcttcatc cctcaagtcacgcgtcaccatctcaaaggacaactctaagaatc aggtgtcactgaaactgtcatctgtgaccgcagccgacaccgcc gtgtactattgcgctaagcattactattatggcgggagctacgcaat ggattactggggacagggtactctggtcaccgtgtccagcaccac taccccagcaccgaggccacccaccccggctcctaccatcgcct cccagcctctgtccctgcgtccggaggcatgtagacccgcagctg gtggggccgtgcatacccggggtcttgacttcgcctgcgatatcta catttgggcccctctggctggtacttgcggggtcctgctgctttcact cgtgatcactctttactgtaagcgcggtcggaagaagctgctgtac atctttaagcaacccttcatgaggcctgtgcagactactcaagagg aggacggctgttcatgccggttcccagaggaggaggaaggcgg ctgcgaactgcgcgtgaaattcagccgcagcgcagatgctccag cctacaagcaggggcagaaccagctctacaacgaactcaatctt ggtcggagagaggagtacgacgtgctggacaagcggagagg acgggacccagaaatgggcgggaagccgcgcagaaagaatc cccaagagggcctgtacaacgagctccaaaaggataagatgg cagaagcctatagcgagattggtatgaaaggggaacgcagaa gaggcaaaggccacgacggactgtaccagggactcagcaccg ccaccaaggacacctatgacgctcttcacatgcaggccctgccg cctcgg (SEQ ID NO: 106) |
| ERₘᵤₜ.FurinCAR19=Alt3 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLC KINGYPKRGRKRREIVMTQSPA TLSLSPGERATLSCRASQDISKY LNWYQQKPGQAPRLLIYHTSRL HSGIPARFSGSGSGTDYTLTISS LQPEDFAVYFCQQGNTLPYTFG QGTKLEIKGGGGSGGGGSGGG GSQVQLQESGPGLVKPSETLSL TCTVSGVSLPDYGVSWIRQPPG KGLEWIGVIWGSETTYYSSSLKS RVTISKDNSKNQVSLKLSSVTAA | Atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactctgcaagatcaa cggctaccctaagaggggcagaaagcggcgggaaattgtgatg acccagtcacccgccactcttagcctttcacccggtgagcgcgca accctgtcttgcagagcctcccaagacatctcaaaataccttaatt ggtatcaacagaagcccggacaggctcctcgccttctgatctacc |
| | DTAVYYCAKHYYYGGSYAMDY WGQGTLVTVSSTTTPAPRPPTP APTIASQPLSLRPEACRPAAGG AVHTRGLDFACDIYIWAPLAGTC GVLLLSLVITLYCKRGRKKLLYIF KQPFMRPVQTTQEEDGCSCRF PEEEEGGCELRVKFSRSADAPA YKQGQNQLYNELNLGRREEYD VLDKRRGRDPEMGGKPRRKNP QEGLYNELQKDKMAEAYSEIGM KGERRRGKGHDGLYQGLSTAT KDTYDALHMQALPPR (SEQ ID NO: 107) | acaccagccggctccattctggaatccctgccaggttcagcggta gcggatctgggaccgactacaccctcactatcagctcactgcagc cagaggacttcgctgtctatttctgtcagcaagggaacaccctgcc ctacacctttggacagggcaccaagctcgagattaaaggtggag gtggcagcggaggaggtgggtccggcggtggaggaagccagg tccaactccaagaaagcggaccgggtcttgtgaagccatcagaa actctttcactgacttgtactgtgagcggagtgtctctccccgattac ggggtgtcttggatcagacagccaccggggaagggtctggaatg gattggagtgatttggggctctgagactacttactactcttcatccctc aagtcacgcgtcaccatctcaaaggacaactctaagaatcaggt gtcactgaaactgtcatctgtgaccgcagccgacaccgccgtgta ctattgcgctaagcattactattatggcgggagctacgcaatggatt actggggacagggtactctggtcaccgtgtccagcaccactacc ccagcaccgaggccacccaccccggctcctaccatcgcctccc agcctctgtccctgcgtccggaggcatgtagacccgcagctggtg gggccgtgcatacccggggtcttgacttcgcctgcgatatctacatt tgggcccctctggctggtacttgcggggtcctgctgctttcactcgtg atcactctttactgtaagcgcggtcggaagaagctgctgtacatctt taagcaacccttcatgaggcctgtgcagactactcaagaggagg acggctgttcatgccggttcccagaggaggaggaaggcggctg cgaactgcgcgtgaaattcagccgcagcgcagatgctccagcct acaagcaggggcagaaccagctctacaacgaactcaatcttggt cggagagaggagtacgacgtgctggacaagcggagaggacg ggacccagaaatgggcgggaagccgcgcagaaagaatcccc aagagggcctgtacaacgagctccaaaaggataagatggcag aagcctatagcgagattggtatgaaaggggaacgcagaagag gcaaaggccacgacggactgtaccagggactcagcaccgcca ccaaggacacctatgacgctcttcacatgcaggccctgccgcctc gg (SEQ ID NO: 108) |
| ERₘᵤₜ.FurinCAR19=Alt4 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLL QWLEQQVAKRRTKREIVMTQSP ATLSLSPGERATLSCRASQDISK YLNWYQQKPGQAPRLLIYHTSR LHSGIPARFSGSGSGTDYTLTIS SLQPEDFAVYFCQQGNTLPYTF GQGTKLEIKGGGGSGGGGSGG GGSQVQLQESGPGLVKPSETLS LTCTVSGVSLPDYGVSWIRQPP GKGLEWIGVIWGSETTYYSSSL KSRVTISKDNSKNQVSLKLSSVT AADTAVYYCAKHYYYGGSYAM DYWGQGTLVTVSSTTTPAPRPP TPAPTIASQPLSLRPEACRPAAG GAVHTRGLDFACDIYIWAPLAGT CGVLLLSLVITLYCKRGRKKLLYI | Atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactcctgcaatggctg gagcagcaggtggcgaagcggagaactaagcgggaaattgtg atgacccagtcacccgccactcttagcctttcacccggtgagcgc gcaaccctgtcttgcagagcctcccaagacatctcaaaatacctt aattggtatcaacagaagcccggacaggctcctcgccttctgatct accacaccagccggctccattctggaatccctgccaggttcagcg gtagcggatctgggaccgactacaccctcactatcagctcactgc agccagaggacttcgctgtctatttctgtcagcaagggaacaccct gccctacacctttggacagggcaccaagctcgagattaaaggtg gaggtggcagcggaggaggtgggtccggcggtggaggaagcc |
| | FKQPFMRPVQTTQEEDGCSCR FPEEEEGGCELRVKFSRSADAP AYKQGQNQLYNELNLGRREEY DVLDKRRGRDPEMGGKPRRKN PQEGLYNELQKDKMAEAYSEIG MKGERRRGKGHDGLYQGLSTA TKDTYDALHMQALPPR (SEQ ID NO: 109) | aggtccaactccaagaaagcggaccgggtcttgtgaagccatca gaaactctttcactgacttgtactgtgagcggagtgtctctccccgat tacggggtgtcttggatcagacagccaccggggaagggtctgga atggattggagtgatttggggctctgagactacttactactcttcatc cctcaagtcacgcgtcaccatctcaaaggacaactctaagaatc aggtgtcactgaaactgtcatctgtgaccgcagccgacaccgcc gtgtactattgcgctaagcattactattatggcgggagctacgcaat ggattactggggacagggtactctggtcaccgtgtccagcaccac taccccagcaccgaggccacccaccccggctcctaccatcgcct cccagcctctgtccctgcgtccggaggcatgtagacccgcagctg gtggggccgtgcatacccggggtcttgacttcgcctgcgatatcta catttgggcccctctggctggtacttgcggggtcctgctgctttcact cgtgatcactctttactgtaagcgcggtcggaagaagctgctgtac atctttaagcaacccttcatgaggcctgtgcagactactcaagagg aggacggctgttcatgccggttcccagaggaggaggaaggcgg ctgcgaactgcgcgtgaaattcagccgcagcgcagatgctccag cctacaagcaggggcagaaccagctctacaacgaactcaatctt ggtcggagagaggagtacgacgtgctggacaagcggagagg acgggacccagaaatgggcgggaagccgcgcagaaagaatc cccaagagggcctgtacaacgagctccaaaaggataagatgg cagaagcctatagcgagattggtatgaaaggggaacgcagaa gaggcaaaggccacgacggactgtaccagggactcagcaccg ccaccaaggacacctatgacgctcttcacatgcaggccctgccg cctcgg (SEQ ID NO: 110) |
| ERₘᵤₜ.FurinCAR19=Alt5 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLG TGAEDPRPSRKRRSLGDVGEIV MTQSPATLSLSPGERATLSCRA SQDISKYLNWYQQKPGQAPRLL IYHTSRLHSGIPARFSGSGSGTD YTLTISSLQPEDFAVYFCQQGNT LPYTFGQGTKLEIKGGGGSGGG GSGGGGSQVQLQESGPGLVKP SETLSLTCTVSGVSLPDYGVSWI RQPPGKGLEWIGVIWGSETTYY SSSLKSRVTISKDNSKNQVSLKL SSVTAADTAVYYCAKHYYYGGS YAMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY | Atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactcggaaccggcg cggaagacccccggccctccaggaagcgaaggtccctcggag acgtgggtgaaattgtgatgacccagtcacccgccactcttagcct ttcacccggtgagcgcgcaaccctgtcttgcagagcctcccaaga catctcaaaataccttaattggtatcaacagaagcccggacaggc tcctcgccttctgatctaccacaccagccggctccattctggaatcc ctgccaggttcagcggtagcggatctgggaccgactacaccctc actatcagctcactgcagccagaggacttcgctgtctatttctgtca gcaagggaacaccctgccctacacctttggacagggcaccaag ctcgagattaaaggtggaggtggcagcggaggaggtgggtccg gcggtggaggaagccaggtccaactccaagaaagcggaccg ggtcttgtgaagccatcagaaactctttcactgacttgtactgtgag cggagtgtctctccccgattacggggtgtcttggatcagacagcca ccggggaagggtctggaatggattggagtgatttggggctctgag actacttactactcttcatccctcaagtcacgcgtcaccatctcaaa |
| | SEIGMKGERRRGKGHDGLYQG LSTATKDTYDALHMQALPPR (SEQ ID NO: 111) | ggacaactctaagaatcaggtgtcactgaaactgtcatctgtgac cgcagccgacaccgccgtgtactattgcgctaagcattactattat ggcgggagctacgcaatggattactggggacagggtactctggt caccgtgtccagcaccactaccccagcaccgaggccacccacc ccggctcctaccatcgcctcccagcctctgtccctgcgtccggagg catgtagacccgcagctggtggggccgtgcatacccggggtcttg acttcgcctgcgatatctacatttgggcccctctggctggtacttgcg gggtcctgctgctttcactcgtgatcactctttactgtaagcgcggtc ggaagaagctgctgtacatctttaagcaacccttcatgaggcctgt gcagactactcaagaggaggacggctgttcatgccggttcccag aggaggaggaaggcggctgcgaactgcgcgtgaaattcagcc gcagcgcagatgctccagcctacaagcaggggcagaaccagc tctacaacgaactcaatcttggtcggagagaggagtacgacgtg ctggacaagcggagaggacgggacccagaaatgggcgggaa gccgcgcagaaagaatccccaagagggcctgtacaacgagct ccaaaaggataagatggcagaagcctatagcgagattggtatg aaaggggaacgcagaagaggcaaaggccacgacggactgta ccagggactcagcaccgccaccaaggacacctatgacgctcttc acatgcaggccctgccgcctcgg (SEQ ID NO: 112) |
| ERₘᵤₜ.FurinCAR19=Alt6 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLG TGAEDPRPSRKRRSLGGEIVMT QSPATLSLSPGERATLSCRASQ DISKYLNWYQQKPGQAPRLLIY HTSRLHSGIPARFSGSGSGTDY TLTISSLQPEDFAVYFCQQGNTL PYTFGQGTKLEIKGGGGSGGG GSGGGGSQVQLQESGPGLVKP SETLSLTCTVSGVSLPDYGVSWI RQPPGKGLEWIGVIWGSETTYY SSSLKSRVTISKDNSKNQVSLKL SSVTAADTAVYYCAKHYYYGGS YAMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY SEIGMKGERRRGKGHDGLYQG LSTATKDTYDALHMQALPPR (SEQ ID NO: 113) | Atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactcggcacaggtgc cgaggaccctcggccaagccgcaaaaggaggtcacttggcgg cgaaattgtgatgacccagtcacccgccactcttagcctttcaccc ggtgagcgcgcaaccctgtcttgcagagcctcccaagacatctc aaaataccttaattggtatcaacagaagcccggacaggctcctcg ccttctgatctaccacaccagccggctccattctggaatccctgcc aggttcagcggtagcggatctgggaccgactacaccctcactatc agctcactgcagccagaggacttcgctgtctatttctgtcagcaag ggaacaccctgccctacacctttggacagggcaccaagctcga gattaaaggtggaggtggcagcggaggaggtgggtccggcggt ggaggaagccaggtccaactccaagaaagcggaccgggtctt gtgaagccatcagaaactctttcactgacttgtactgtgagcggag tgtctctccccgattacggggtgtcttggatcagacagccaccggg gaagggtctggaatggattggagtgatttggggctctgagactact tactactcttcatccctcaagtcacgcgtcaccatctcaaaggaca actctaagaatcaggtgtcactgaaactgtcatctgtgaccgcagc cgacaccgccgtgtactattgcgctaagcattactattatggcggg agctacgcaatggattactggggacagggtactctggtcaccgtg tccagcaccactaccccagcaccgaggccacccaccccggctc ctaccatcgcctcccagcctctgtccctgcgtccggaggcatgtag |
| | | acccgcagctggtggggccgtgcatacccggggtcttgacttcgc ctgcgatatctacatttgggcccctctggctggtacttgcggggtcct gctgctttcactcgtgatcactctttactgtaagcgcggtcggaaga agctgctgtacatctttaagcaacccttcatgaggcctgtgcagact actcaagaggaggacggctgttcatgccggttcccagaggagg aggaaggcggctgcgaactgcgcgtgaaattcagccgcagcgc agatgctccagcctacaagcaggggcagaaccagctctacaac gaactcaatcttggtcggagagaggagtacgacgtgctggacaa gcggagaggacgggacccagaaatgggcgggaagccgcgc agaaagaatccccaagagggcctgtacaacgagctccaaaag gataagatggcagaagcctatagcgagattggtatgaaagggg aacgcagaagaggcaaaggccacgacggactgtaccaggga ctcagcaccgccaccaaggacacctatgacgctcttcacatgca ggccctgccgcctcgg (SEQ ID NO: 114) |
| ERₘᵤₜ.FurinCAR19=Alt7 | MALPVTALLLPLALLLHAARPR SSLALSLTADQMVSALLDAEPPI LYSEYDPTRPFSEASMMGLLTN LADRELVHMINWAKRVPGFVDL ALHDQVHLLECAWMEILMIGLV WRSMEHPGKLLFAPNLLLDRNQ GKCVEGGVEIFDMLLATSSRFR MMNLQGEEFVCLKSIILLNSGVY TFLSSTLKSLEEKDHIHRVLDKIT DTLIHLMAKAGLTLQQQHQRLA QLLLILSHIRHMSSKRMEHLYSM KCKNVVPLSDLLLEMLDAHRLG TGAEDPRPSRKRRSLGEIVMTQ SPATLSLSPGERATLSCRASQDI SKYLNWYQQKPGQAPRLLIYHT SRLHSGIPARFSGSGSGTDYTL TISSLQPEDFAVYFCQQGNTLPY TFGQGTKLEIKGGGGSGGGGS GGGGSQVQLQESGPGLVKPSE TLSLTCTVSGVSLPDYGVSWIR QPPGKGLEWIGVIWGSETTYYS SSLKSRVTISKDNSKNQVSLKLS SVTAADTAVYYCAKHYYYGGSY AMDYWGQGTLVTVSSTTTPAP RPPTPAPTIASQPLSLRPEACRP AAGGAVHTRGLDFACDIYIWAPL AGTCGVLLLSLVITLYCKRGRKK LLYIFKQPFMRPVQTTQEEDGC SCRFPEEEEGGCELRVKFSRSA DAPAYKQGQNQLYNELNLGRR EEYDVLDKRRGRDPEMGGKPR RKNPQEGLYNELQKDKMAEAY SEIGMKGERRRGKGHDGLYQG LSTATKDTYDALHMQALPPR (SEQ ID NO: 115) | Atggccctccctgtcaccgccctgctgcttccgctggctcttctgctc cacgccgctcggccccggtcgtcgttggcactttccctgactgccg accagatggtgtccgcccttctggacgccgagcctccaattctgta ctcggagtacgatccgactcgcccgttctccgaagccagcatgat gggcctgttgactaacctggcggaccgcgagttggtgcacatgat taactgggctaagcgggtgccgggcttcgtggacctggccctgca cgaccaagtgcacctcctggaatgcgcctggatggaaatcctcat gatcggcctcgtgtggagatccatggagcatcccggaaagctcct gtttgcacccaacctcctgcttgatcgcaaccagggaaaatgcgt ggaagggggtgtcgagattttcgacatgctgctcgccacctcttcc cggttccggatgatgaatctgcagggagaagagttcgtgtgtctga agtcaatcatcctgctgaactccggggtctataccttcctgagctcg accctcaagtcactggaggaaaaagaccacatccatcgcgtgct cgataagatcaccgacacccttatccatctcatggcgaaggctgg actgaccctgcaacagcagcaccagaggctggcccagttgctg ctgattctgagccacatccggcacatgtcgtccaagaggatggaa cacctgtacagcatgaagtgcaagaacgtcgtgcctctgtccgat ctgctcctggaaatgctggacgcgcacagactcggaaccggag cagaagatcccagaccaagccggaaaaggcggtccctgggtg aaattgtgatgacccagtcacccgccactcttagcctttcacccggt gagcgcgcaaccctgtcttgcagagcctcccaagacatctcaaa ataccttaattggtatcaacagaagcccggacaggctcctcgcctt ctgatctaccacaccagccggctccattctggaatccctgccaggt tcagcggtagcggatctgggaccgactacaccctcactatcagct cactgcagccagaggacttcgctgtctatttctgtcagcaagggaa caccctgccctacacctttggacagggcaccaagctcgagatta aaggtggaggtggcagcggaggaggtgggtccggcggtggag gaagccaggtccaactccaagaaagcggaccgggtcttgtgaa gccatcagaaactctttcactgacttgtactgtgagcggagtgtctct ccccgattacggggtgtcttggatcagacagccaccggggaagg gtctggaatggattggagtgatttggggctctgagactacttactact cttcatccctcaagtcacgcgtcaccatctcaaaggacaactctaa gaatcaggtgtcactgaaactgtcatctgtgaccgcagccgacac cgccgtgtactattgcgctaagcattactattatggcgggagctac gcaatggattactggggacagggtactctggtcaccgtgtccagc accactaccccagcaccgaggccacccaccccggctcctacca tcgcctcccagcctctgtccctgcgtccggaggcatgtagacccg cagctggtggggccgtgcatacccggggtcttgacttcgcctgcg atatctacatttgggcccctctggctggtacttgcggggtcctgctgc tttcactcgtgatcactctttactgtaagcgcggtcggaagaagctg ctgtacatctttaagcaacccttcatgaggcctgtgcagactactca agaggaggacggctgttcatgccggttcccagaggaggaggaa |
| | | ggcggctgcgaactgcgcgtgaaattcagccgcagcgcagatg ctccagcctacaagcaggggcagaaccagctctacaacgaact caatcttggtcggagagaggagtacgacgtgctggacaagcgg agaggacgggacccagaaatgggcgggaagccgcgcagaa agaatccccaagagggcctgtacaacgagctccaaaaggata agatggcagaagcctatagcgagattggtatgaaaggggaacg cagaagaggcaaaggccacgacggactgtaccagggactca gcaccgccaccaaggacacctatgacgctcttcacatgcaggcc ctgccgcctcgg (SEQ ID NO: 116) |

### General Methods

The following general materials and methods were used for the assays described in Examples 1-13.

### Generation of CAR constructs

The scFvs used in the CAR constructs that were assessed in Examples 3 were independently synthesized based on an anti-CD19 scFv sequence described above. The scFv was cloned in a heavy-to-light chain direction, with a flexible linker connecting the VH and VL domains (e.g., GGGGSGGGGSGGGGSGGGGS (SEQ ID NO: 29)), into a vector backbone containing the CD8 hinge region along with the 4-1BB molecule and the CD3zeta molecule. To this, an N-terminal furin cleavage site fused to a destabilization domain was added upstream of the scFv. The proteins used in the furindegron constructs that were assessed in Examples 4-5 were independently synthesized based on publicly available sequence data. To this, an N-terminal furin cleavage site fused to a destabilization domain was added upstream of the protein.

### Cell lines and cell transduction

Jurkat cells were obtained from the ATCC and maintained in media according to supplier's recommendations. Jurkat cell lines were transduced by spinoculation with lentiviral supernatant from 293T cells transfected with pELPS using the protein-furin domain regulated plasmid DNA.

### Cancer Cells

NALM6 and K562 cell lines were obtained from the ATCC and maintained in media according to supplier's recommendations. To produce a bioluminescent model for T cell killing assays, all cells were transduced with a luciferase lentiviral construct and kept under antibiotic selection.

### Flow cytometry

Cells were isolated from *in vitro* culture, washed once in PBS supplemented with 0.5% bovine serum albumin, and stained on ice using either biotinylated Protein L followed by incubated with a fluorochromeconjugated streptavidin reagent or an antibody recognizing the given target antigen. In all analyses, the population of interest was gated based on forward vs side scatter characteristics, followed by singlet gating, and live cells were gated. Flow cytometry was performed on a four laser Fortessa (Becton-Dickinson).

### Compound treatments

In all instances, either 4-OHT or Shield1 was resuspended in ethanol. Trimethoprim (TMP) was resuspended in DMSO. In all instances where concentrations not indicated, compounds were added for 24 hours in total prior to analysis by flow cytometry. 3 µM 4-OHT (ERα), 1 µM Shield1 (FKBP), or 100 µM TMP (ecDHFR) was used.

### Killing assay

In brief, luciferase transduced target cell lines were incubated at the indicated ratios with effector CD19CAR or CD19CAR-FurON T cells for 18 hours. Remaining luciferase activity was measured by adding Bright-Glo (Promega) on a luminescence reader (Envision). Percentage killing was calculated using negative controls.

### Cytokine secretion

Effector and target cells were incubated at a 3:1 ratio in RPMI containing 10% FBS for 18 hours. Supernatant was analysed by 3-plex array according the manufacturer's instructions (Invitrogen).

### EXAMPLE 2: FURIN IS THE MOST HIGHLY EXPRESSED PROPROTEIN CONVERTASE IN PRIMARY HUMAN T CELLS

Literature sources indicated that FURIN (FUR; PACE; PCSK3; SPC1) has a broad expression profile (Seidah, et al Nature Reviews Drug Discovery 11, 367-383 (May 2012)). The expression of the proprotein convertase family members was analysed by qRT-PCR. RNA was harvested from normal donor T cells on days 0, 4, and 11 following stimulation with anti-CD3/anti-CD28 activation beads. An additional group was supplemented with 100U/mL IL-2 during culture and RNA was harvested on day 11. These data demonstrate that following activation with anti-CD3/anti-CD28 beads, FURIN mRNA is expressed more highly than other members of the proprotein convertase family (FIG. 1).

### EXAMPLE 3: EXPRESSION OF CD19 CAR CONTROLLED BY COMPOUND ADDITION

Jurkat T cells were transduced with a furin degron domain (FKBP_{FD}, ERα_{FD}, or DHFR_{FD}) fused to an antiCD19 scFv CAR construct followed by treatment with the corresponding compound. FKBP_{FD} transduced cells treated with 1 µM Shield1. ERα_{FD} transduced cells treated with 1µM Bazedoxifene. DHFR_{FD} transduced cells treated with 1mM TMP. Expression of the anti-CD19 scFv is inducible in the presence of compound (FIG. 2). Black=UTD; Gray=construct, no compound; White=construct, compound. These data demonstrate that multiple degron domains, when combined with a furin cleavage domain, result in strict compound-dependent regulation of CAR 19 expression.

### EXAMPLE 4: KINETICS OF CAR EXPRESSION IN JURKAT CELLS FOLLOWING COMPOUND ADDITION

Jurkat T cells were transduced with the indicated furin degron domain (FKBP_{FD} or ERα_{FD}) fused to an anti-CD19 scFv CAR construct. These cells were treated with either 1 µM Shield1 or 1 µM 4-OHT for the time indicated and CAR expression was determined by FACS. These data demonstrate that induction of expression using a furin degron CAR 19 occurs rapidly following compound addition and is stable throughout the period of compound treatment (FIG. 3).

### EXAMPLE 5: COMPOUND INDUCIBLE CAR EXPRESSION IN PRIMARY HUMAN T CELLS

Primary human T cells were transduced with ERα furin degron domain (ERα_{FD} domain) fused to an antiCD19 scFv CAR construct. 100 U/mL IL-2 was added on day 9 following activation with anti-CD3/CD28 stimulation beads. Bazedoxifene was added on day 10, and CAR expression was determined by FACS on day 11. These data demonstrate that a furin degron domain (e.g., ERα_{FD} domain) can regulate CAR 19 expression in a compound-dependent manner in primary human T cells (FIG. 4), and that stabilization is enhanced in the presence of IL-2 in vitro (FIG. 4).

### EXAMPLE 6: KINETICS OF CAR EXPRESSION FOLLOWING COMPOUND WASHOUT IN PRIMARY T CELLS

Primary human T cells were transduced with the indicated furin degron domain (FKBP_{FD} or ERα_{FD}) fused to an anti-CD19 scFv CAR construct. 100 U/mL IL-2 was added on day 9 following activation with antiCD3/CD28 stimulation beads. Bazedoxifene was added on day 10, and T cells were frozen on day 11. T cells were thawed, extensively washed, and placed in culture for the times indicated followed by determination of CAR expression by FACS. These data demonstrate that removal of compound results in a reduction of CAR 19 expression using a furin degron domain (FIG. 5).

### EXAMPLE 7: MULTIPLE ER-ALPHA TARGETING DRUGS STABILIZE ER-ALPHA FURON CARTS

Jurkat T cells were transduced with the ERα_{FD} degradation domain fused to an anti-CD19 scFv CAR construct followed by treatment with the indicated compounds for 24 hours. Compounds were used at 10 µM for 4-OHT, 1 µM for Bazedoxifene, or 1 µM for Lasofoxifene. These data demonstrate that multiple ER-alpha targeting compounds can induce CAR 19 expression using an estrogen receptor-based furin degron (FurON) system (FIG. 6).

### EXAMPLE 8: DOSE RESPONSE WITH BAZEDOXIFENE IN HUMAN T CELLS

Primary human T cells were transduced with ERα_{FD} furin degron domain fused to an anti-CD19 scFv CAR construct or the parental CD19 CAR construct. 100 U/mL IL-2 was added on day 9 following activation with anti-CD3/CD28 stimulation beads, and T cells were frozen on day 11. T cells were thawed and placed in culture for 48 hrs with Bazedoxifene at the concenrations indicated. CAR expression was determined by FACS. These data demonstrate that physiologically relevant concentrations of Bazedoxifene can stabilize furin degron CAR 19 expression in a level that is similar to parental CAR 19 (FIG. 7).

### EXAMPLE 9: COMPOUND-DEPENDENT TARGET SPECIFIC CELL KILLING BY ER-ALPHA FURON CART

Primary human T cells were transduced with ERα_{FD} furin degron domain fused to an anti-CD19 scFv CAR construct or the parental CD19 CAR construct. 100 U/mL IL-2 and Bazedoxifene were added on day 9 following activation with anti-CD3/CD28 stimulation beads, and T cells were frozen on day 11. T cells were thawed and incubated for 20 hours with the indicated luciferized cell line targets, K562 (CD19-) or NALM6 (CD19+). Percent killing was determined by analysis of remaining luciferase activity. These data demonstrate that CAR19 with an estrogen receptor-based furin degron domain specifically kills CD19+ tumor cells in a manner that is compound-dependent and is noninferior to the parental CAR19 (FIG. 8).

### EXAMPLE 10: COMPOUND-DEPENDENT TARGET SPECIFIC CELL KILLING BY FKBP FURON CART

Primary human T cells were transduced with FKBP_{FD} furin degron domain fused to an anti-CD19 scFv CAR construct or the parental CD19 CAR construct. 100U/mL IL-2 and Shield1 were added on day 9 following activation with anti-CD3/CD28 stimulation beads, and T cells were frozen on day 11. T cells were thawed and incubated for 20 hours with the indicated luciferized cell line targets, K562 (CD19-) or NALM6 (CD19+). Percent killing was determined by analysis of remaining luciferase activity. These data demonstrate that CAR19 with an FKBP-based furin degron domain specifically kills CD19+ tumor cells in a manner that is compound-dependent and is noninferior to the parental CAR19 (FIG. 9).

### EXAMPLE 11: COMPOUND-DEPENDENT CYTOKINE PRODUCTION OF ER-ALPHA FURON CART

Primary human T cells were transduced with ERα_{FD} furin degron domain fused to an anti-CD19 scFv CAR construct or the parental CD19 CAR construct. 100 U/mL IL-2 and Bazedoxifene were added on day 9 following activation with anti-CD3/CD28 stimulation beads, and T cells were frozen on day 11. T cells were thawed and incubated with the indicated cell line targets for 20 hours. Supernatants were harvested and analyzed by cytokine bead array. These data demonstrate that CAR19 with an estrogen receptor-based furin degron domain specifically produces cytokines in the presence of CD19+ tumor cells in a manner that is compound-dependent and is noninferior to the parental CAR19 (FIG. 10).

### EXAMPLE 12: COMPOUND-DEPENDENT PROLIFERATION OF ER-ALPHA FURON CART

Primary human T cells were transduced with ERα_{FD} furin degron domain fused to an anti-CD19 scFv CAR construct or the parental CD19 CAR construct. 100 U/mL IL-2 and Bazedoxifene were added on day 9 following activation with anti-CD3/CD28 stimulation beads, and T cells were frozen on day 11. T cells were thawed and incubated with the indicated cell line targets for 4 days. T cell FurON-CAR numbers were analyzed by FACS. These data demonstrate that CAR19 with an estrogen receptor-based furin degron domain specifically proliferates in the presence of CD19+ tumor cells in a manner that is compound-dependent and is noninferior to the parental CAR19 (FIG. 11).

EXAMPLE 13: MATERIALS AND METHODS FOR EXAMPLES 14-26.

The following general materials and methods were used for the assays described in Examples 14-26.

### FurON constructs

In some embodiments, the fusion protein described herein includes a furin degron (FurON) domain, which comprises two components: a degron or degradation domain, which is a mutated protein domain unable to acquire a proper conformation in the absence of a small molecule ligand, and a furin cleavage site. The furin degron domain can be fused to a protein of interest that is expressed in the endoplasmic reticulum. The N- and/ or C-termini of the protein of interest can be used as site of fusion, provided that the furin degron domain faces the lumen of the endoplasmic reticulum. The protein of interest can be either a membrane protein (regardless of the number of transmembrane domains) or a soluble protein . The orientation of the furin cleavage site relative to the degron domain should be such that the cleavage results in the separation of the furin degron domain and protein of interest. In the absence of the small molecule ligand or stabilization compound, the furin degron domain leads to the destabilization or degradation of the entire fusion protein. In the presence of the small molecule ligand or stabilization compound, the fusion protein is spared from degradation. The furin degron domain is mutated such that the affinity to its natural endogenous ligand is abolished; the affinity to the small molecule ligand or stabilization compound is preserved; and protein instability is conferred when the small molecule is absent.

Several constructs comprising different mutations and different furin cleavage sites within the furin degron domain were created and fused to the N-terminus of the anti-CD19 scFV CAR or anti-CD123 scFV CAR. Regardless the orientation of the scFV heavy and light chains, the fusion of the furin degron domain occurs between the signal peptide leader and the scFV. The mutated protein selected within the furin degron domain was a truncated version of the estrogen receptor alpha. The small molecule ligands chosen to stabilize the furin degron domain belong to the family of selective estrogen receptor modulators or downregulators.

The efficacy of the furin degron domain at destabilizing or stabilizing the CAR structure was assessed, in the absence or presence of a small molecule ligand. The potency of the CART cell effector function, when the furin degron domain was fused, was also evaluated in the absence or presence of a small molecule ligand, and compared to the parental CAR19 construct. Cells were normalized to the same percentage of CAR expressing cells, with the addition of untransduced T cells (UTDs) from the same donor T cell expansion. The normalized populations were then used in co-cultured assays to measure CAR-mediated cytolytic function, cytokine production, and proliferation.

### Generation of FurON CAR constructs

The scFvs used in the CAR constructs assessed in the examples were independently synthesized based on anti-CD19 or anti-CD123 scFV sequences described above. The scFVs were cloned in a light-toheavy chain direction (anti-CD19) or heavy-to-light chain direction (anti-CD123), with a flexible linker connecting the VH and VL domains (e.g., GGGGSGGGGSGGGGSGGGGS, SEQ ID NO: 29), into a vector backbone containing the CD8 hinge region along with the 4-1BB molecule and the CD3zeta molecule. To this, an N-terminal furin cleavage site fused to a destabilization domain was added upstream of the scFV. All constructs are generated in a lentiviral vector. Sequences of various FurON CAR constructs and components thereof are provided in Table 23.

**Table 23. Sequences of FurON CAR constructs and components thereof**

| | **Amino acid sequence** | **DNA sequence** |
|---|---|---|
| ER1 WT (305aa-549aa) | SLALSLTADQMVSALLDAEPPI LYSEYDPTRPFSEASMMGLLT NLADRELVHMINWAKRVPGF VDLTLHDQVHLLECAWLEILMI GLVWRSMEHPGKLLFAPNLLL DRNQGKCVEGMVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKGMEHLYSMKCKNVVPLY DLLLEMLDAHRL (SEQ ID NO: 970) | tcgttggcactttccctgactgccgaccagatggtgtccgcc cttctggacgccgagcctccaattctgtactcggagtacgat ccgactcgcccgttctccgaagccagcatgatgggcctgtt gactaacctggcggaccgcgagttggtgcacatgattaac tgggctaagcgggtgccgggcttcgtggacctgactctgc acgaccaagtgcacctcctggaatgcgcctggctggaaa tcctcatgatcggcctcgtgtggagatccatggagcatccc ggaaagctcctgtttgcacccaacctcctgcttgatcgcaa ccagggaaaatgcgtggaagggatggtcgagattttcga catgctgctcgccacctcttcccggttccggatgatgaatct gcagggagaagagttcgtgtgtctgaagtcaatcatcctgc tgaactccggggtctataccttcctgagctcgaccctcaagt cactggaggaaaaagaccacatccatcgcgtgctcgata agatcaccgacacccttatccatctcatggcgaaggctgg actgaccctgcaacagcagcaccagaggctggcccagtt gctgctgattctgagccacatccggcacatgtcgaacaag gggatggaacacctgtacagcatgaagtgcaagaacgtc gtgcctctgtacgatctgctcctggaaatgctggacgcgca cagactc (SEQ ID NO: 971) |
| ERmut1 (6 mutations) | SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRL (SEQ ID NO: 58) | tcgttggcactttccctgactgccgaccagatggtgtccgcc cttctggacgccgagcctccaattctgtactcggagtacgat ccgactcgcccgttctccgaagccagcatgatgggcctgtt gactaacctggcggaccgcgagttggtgcacatgattaac tgggctaagcgggtgccgggcttcgtggacctggccctgc acgaccaagtgcacctcctggaatgcgcctggatggaaa tcctcatgatcggcctcgtgtggagatccatggagcatccc ggaaagctcctgtttgcacccaacctcctgcttgatcgcaa ccagggaaaatgcgtggaagggggtgtcgagattttcga catgctgctcgccacctcttcccggttccggatgatgaatct gcagggagaagagttcgtgtgtctgaagtcaatcatcctgc tgaactccggggtctataccttcctgagctcgaccctcaagt cactggaggaaaaagaccacatccatcgcgtgctcgata agatcaccgacacccttatccatctcatggcgaaggctgg actgaccctgcaacagcagcaccagaggctggcccagtt gctgctgattctgagccacatccggcacatgtcgtccaaga ggatggaacacctgtacagcatgaagtgcaagaacgtcg tgcctctgtccgatctgctcctggaaatgctggacgcgcac agactc (SEQ ID NO: 1110) |
| ERmut2 (4 mutations) | SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRL (SEQ ID NO: 121) | tcgttggcactttccctgactgccgaccagatggtgtccgcc cttctggacgccgagcctccaattctgtactcggagtacgat ccgactcgcccgttctccgaagccagcatgatgggcctgtt gactaacctggcggaccgcgagttggtgcacatgattaac tgggctaagcgggtgccgggcttcgtggacctgaccctgc acgaccaagtgcacctcctggaatgcgcctggatggaaa tcctcatgatcggcctcgtgtggagatccatggagcatccc ggaaagctcctgtttgcacccaacctcctgcttgatcgcaa ccagggaaaatgcgtggaagggggtgtcgagattttcga catgctgctcgccacctcttcccggttccggatgatgaatct gcagggagaagagttcgtgtgtctgaagtcaatcatcctgc tgaactccggggtctataccttcctgagctcgaccctcaagt cactggaggaaaaagaccacatccatcgcgtgctcgata agatcaccgacacccttatccatctcatggcgaaggctgg |
| | | actgaccctgcaacagcagcaccagaggctggcccagtt gctgctgattctgagccacatccggcacatgtcgaacaag aggatggaacacctgtacagcatgaagtgcaagaacgtc gtgcctctgtccgatctgctcctggaaatgctggacgcgca cagactc (SEQ ID NO: 122) |
| Furin cleavage site1 | RTKR (SEQ ID NO: 123) | cgtactaaaaga (SEQ ID NO: 1112) |
| Furin cleavage site2 | GTGAEDPRPSRKRRSLGDVG (SEQ ID NO: 125) | ggaaccggcgcggaagacccccggccctccaggaagc gaaggtccctcggagacgtgggt (SEQ ID NO: 126) |
| Furin cleavage site3 | GTGAEDPRPSRKRR (SEQ ID NO: 127) | ggaaccggcgcggaagacccccggccctccaggaagc gaagg (SEQ ID NO: 128) |
| Furin cleavage site4 | LQWLEQQVAKRRTKR (SEQ ID NO: 129) | ctgcaatggctggagcagcaggtggcgaagcggagaac taagcgg (SEQ ID NO: 130) |
| Furin cleavage site5 | GTGAEDPRPSRKRRSLGG (SEQ ID NO: 131) | ggcacaggtgccgaggaccctcggccaagccgcaaaa ggaggtcacttggcggc (SEQ ID NO: 132) |
| Furin cleavage site6 | GTGAEDPRPSRKRRSLG (SEQ ID NO: 133) | ggaaccggagcagaagatcccagaccaagccggaaa aggcggtccctgggt (SEQ ID NO: 134) |
| Furin cleavage site7 | SLNLTESHNSRKKR (SEQ ID NO: 135) | agtctcaatttgactgagtcacacaattccaggaagaaaa gg (SEQ ID NO: 136) |
| Furin cleavage site8 | CKINGYPKRGRKRR (SEQ ID NO: 137) | tgcaagatcaacggctaccctaagaggggcagaaagcg gcgg (SEQ ID NO: 138) |
| Leader sequence/ Signal peptide (SP) | MALPVTALLLPLALLLHAARP (SEQ ID NO: 139) | atggccctccctgtcaccgccctgctgcttccgctggctcttc tgctccacgccgctcggccc (SEQ ID NO: 140) |
| SP-Linker 1-ERmut1-Furin cleavage site 1-CD19 CAR1 | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLRTKREIVM TQSPATLSLSPGERATLSCRA SQDISKYLNWYQQKPGQAPR LLIYHTSRLHSGIPARFSGSGS GTDYTLTISSLQPEDFAVYFC QQGNTLPYTFGQGTKLEIKGG GGSGGGGSGGGGSQVQLQE SGPGLVKPSETLSLTCTVSGV SLPDYGVSWIRQPPGKGLEWI GVIWGSETTYYSSSLKSRVTIS KDNSKNQVSLKLSSVTAADTA VYYCAKHYYYGGSYAMDYW GQGTLVTVSSTTTPAPRPPTP APTIASQPLSLRPEACRPAAG GAVHTRGLDFACDIYIWAPLA GTCGVLLLSLVITLYCKRGRKK LLYIFKQPFMRPVQTTQEEDG CSCRFPEEEEGGCELRVKFS RSADAPAYKQGQNQLYNELN LGRREEYDVLDKRRGRDPEM | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactccgtacta aaagagaaattgtgatgacccagtcacccgccactcttag cctttcacccggtgagcgcgcaaccctgtcttgcagagcct cccaagacatctcaaaataccttaattggtatcaacagaa gcccggacaggctcctcgccttctgatctaccacaccagc cggctccattctggaatccctgccaggttcagcggtagcgg atctgggaccgactacaccctcactatcagctcactgcag ccagaggacttcgctgtctatttctgtcagcaagggaacac cctgccctacacctttggacagggcaccaagctcgagatt aaaggtggaggtggcagcggaggaggtgggtccggcg gtggaggaagccaggtccaactccaagaaagcggacc gggtcttgtgaagccatcagaaactctttcactgacttgtact gtgagcggagtgtctctccccgattacggggtgtcttggatc |
| | GGKPRRKNPQEGLYNELQKD KMAEAYSEIGMKGERRRGKG HDGLYQGLSTATKDTYDALH MQALPPR (SEQ ID NO: 141) | agacagccaccggggaagggtctggaatggattggagt gatttggggctctgagactacttactactcttcatccctcaag tcacgcgtcaccatctcaaaggacaactctaagaatcag gtgtcactgaaactgtcatctgtgaccgcagccgacaccg ccgtgtactattgcgctaagcattactattatggcgggagct acgcaatggattactggggacagggtactctggtcaccgt gtccagcaccactaccccagcaccgaggccacccaccc cggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctacaagcaggggcagaaccagctct acaacgaactcaatcttggtcggagagaggagtacgacg tgctggacaagcggagaggacgggacccagaaatggg cgggaagccgcgcagaaagaatccccaagagggcctg tacaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 142) |
| SP-Linker 1-**ERmut1-Furin cleavage site 2-CD19 CAR1 (construct 106)** | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRRSLGDVGEIVMTQSPA TLSLSPGERATLSCRASQDIS KYLNWYQQKPGQAPRLLIYHT SRLHSGIPARFSGSGSGTDYT LTISSLQPEDFAVYFCQQGNT LPYTFGQGTKLEIKGGGGSG GGGSGGGGSQVQLQESGPG LVKPSETLSLTCTVSGVSLPD YGVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYKQGQNQLYNELNLGR | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca |
| | REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 143) | ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctacaagcaggggcaga accagctctacaacgaactcaatcttggtcggagagagg agtacgacgtgctggacaagcggagaggacgggaccc agaaatgggcgggaagccgcgcagaaagaatccccaa gagggcctgtacaacgagctccaaaaggataagatggc agaagcctatagcgagattggtatgaaaggggaacgca gaagaggcaaaggccacgacggactgtaccagggact cagcaccgccaccaaggacacctatgacgctcttcacat gcaggccctgccgcctcgg (SEQ ID NO: 144) |
| SP-Linker 1-ERmut1-Furin cleavage site 2-CD19 CAR2 | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRRSLGDVGEIVMTQSPA TLSLSPGERATLSCRASQDIS KYLNWYQQKPGQAPRLLIYHT SRLHSGIPARFSGSGSGTDYT LTISSLQPEDFAVYFCQQGNT LPYTFGQGTKLEIKGGGGSG GGGSGGGGSQVQLQESGPG LVKPSETLSLTCTVSGVSLPD YGVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga |
| | DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 145) | aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 146) |
| SP-Linker 1-**ERmut1-Furin cleavage site 3-CD19 CAR1 (construct 103)** | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRREIVMTQSPATLSLSP GERATLSCRASQDISKYLNWY QQKPGQAPRLLIYHTSRLHSG IPARFSGSGSGTDYTLTISSLQ PEDFAVYFCQQGNTLPYTFG QGTKLEIKGGGGSGGGGSGG GGSQVQLQESGPGLVKPSE TLSLTCTVSGVSLPDYGVS WIRQPPGKGLEWIGVIWGS ETTYYSSSLKSRVTISKDNS KNQVSLKLSSVTAADTAVY YCAKHYYYGGSYAMDYW GQGTLVTVSSTTTPAPRPP TPAPTIASQPLSLRPEACRP AAGGAVHTRGLDFACDIYI WAPLAGTCGVLLLSLVITLY | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcgggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg acaggctcctcgccttctgatctaccacaccagccggctcc attctggaatccctgccaggttcagcggtagcggatctggg accgactacaccctcactatcagctcactgcagccagag gacttcgctgtctatttctgtcagcaagggaacaccctgccc tacacctttggacagggcaccaagctcgagattaaaggtg gaggtggcagcggaggaggtgggtccggcggtggagg |
| | CKRGRKKLLYIFKQPFMRP VQTTQEEDGCSCRFPEEE EGGCELRVKFSRSADAPA YKQGQNQLYNELNLGRRE EYDVLDKRRGRDPEMGGK PRRKNPQEGLYNELQKDK MAEAYSEIGMKGERRRGK GHDGLYQGLSTATKDTYD ALHMQALPPR (SEQ ID NO: 147) | aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctacaagcaggggcagaaccagctctacaacgaa ctcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 148) |
| SP-Linker 1-ERmut1-Furin cleavage site 3-CD19 CAR2 | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRREIVMTQSPATLSLSP GERATLSCRASQDISKYLNWY QQKPGQAPRLLIYHTSRLHSG IPARFSGSGSGTDYTLTISSLQ PEDFAVYFCQQGNTLPYTFG QGTKLEIKGGGGSGGGGSGG GGSQVQLQESGPGLVKPSET LSLTCTVSGVSLPDYGVSWIR QPPGKGLEWIGVIWGSETTYY SSSLKSRVTISKDNSKNQVSL KLSSVTAADTAVYYCAKHYYY GGSYAMDYWGQGTLVTVSST TTPAPRPPTPAPTIASQPLSLR PEACRPAAGGAVHTRGLDFA CDIYIWAPLAGTCGVLLLSLVIT LYCKRGRKKLLYIFKQPFMRP | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcgggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg acaggctcctcgccttctgatctaccacaccagccggctcc attctggaatccctgccaggttcagcggtagcggatctggg accgactacaccctcactatcagctcactgcagccagag gacttcgctgtctatttctgtcagcaagggaacaccctgccc tacacctttggacagggcaccaagctcgagattaaaggtg |
| | VQTTQEEDGCSCRFPEEEEG GCELRVKFSRSADAPAYQQG QNQLYNELNLGRREEYDVLD KRRGRDPEMGGKPRRKNPQ EGLYNELQKDKMAEAYSEIGM KGERRRGKGHDGLYQGLSTA TKDTYDALHMQALPPR (SEQ ID NO: 149) | gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 150) |
| SP-Linker 1-ERmut2-Furin cleavage site 1-CD19 CAR1 | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLRTKREIVM TQSPATLSLSPGERATLSCRA SQDISKYLNWYQQKPGQAPR LLIYHTSRLHSGIPARFSGSGS GTDYTLTISSLQPEDFAVYFC QQGNTLPYTFGQGTKLEIKGG GGSGGGGSGGGGSQVQLQE SGPGLVKPSETLSLTCTVSGV SLPDYGVSWIRQPPGKGLEWI GVIWGSETTYYSSSLKSRVTIS KDNSKNQVSLKLSSVTAADTA VYYCAKHYYYGGSYAMDYW GQGTLVTVSSTTTPAPRPPTP APTIASQPLSLRPEACRPAAG GAVHTRGLDFACDIYIWAPLA GTCGVLLLSLVITLYCKRGRKK | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactccgtact aaaagagaaattgtgatgacccagtcacccgccactctta gcctttcacccggtgagcgcgcaaccctgtcttgcagagc ctcccaagacatctcaaaataccttaattggtatcaacaga agcccggacaggctcctcgccttctgatctaccacaccag ccggctccattctggaatccctgccaggttcagcggtagcg gatctgggaccgactacaccctcactatcagctcactgca gccagaggacttcgctgtctatttctgtcagcaagggaaca ccctgccctacacctttggacagggcaccaagctcgagat |
| | LLYIFKQPFMRPVQTTQEEDG CSCRFPEEEEGGCELRVKFS RSADAPAYKQGQNQLYNELN LGRREEYDVLDKRRGRDPEM GGKPRRKNPQEGLYNELQKD KMAEAYSEIGMKGERRRGKG HDGLYQGLSTATKDTYDALH MQALPPR (SEQ ID NO: 151) | taaaggtggaggtggcagcggaggaggtgggtccggcg gtggaggaagccaggtccaactccaagaaagcggacc gggtcttgtgaagccatcagaaactctttcactgacttgtact gtgagcggagtgtctctccccgattacggggtgtcttggatc agacagccaccggggaagggtctggaatggattggagt gatttggggctctgagactacttactactcttcatccctcaag tcacgcgtcaccatctcaaaggacaactctaagaatcag gtgtcactgaaactgtcatctgtgaccgcagccgacaccg ccgtgtactattgcgctaagcattactattatggcgggagct acgcaatggattactggggacagggtactctggtcaccgt gtccagcaccactaccccagcaccgaggccacccaccc cggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctacaagcaggggcagaaccagctct acaacgaactcaatcttggtcggagagaggagtacgacg tgctggacaagcggagaggacgggacccagaaatggg cgggaagccgcgcagaaagaatccccaagagggcctg tacaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 152) |
| SP-Linker 1-**ERmut2-Furin cleavage site 2-CD19 CAR1 (construct 130)** | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLGTGAEDPR PSRKRRSLGDVGEIVMTQSPA TLSLSPGERATLSCRASQDIS KYLNWYQQKPGQAPRLLIYHT SRLHSGIPARFSGSGSGTDYT LTISSLQPEDFAVYFCQQGNT LPYTFGQGTKLEIKGGGGSG GGGSGGGGSQVQLQESGPG LVKPSETLSLTCTVSGVSLPD YGVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc |
| | GVLLLSLVITLYCKRGRKKLLYI FKQPFMRPVQTTQEEDGCSC RFPEEEEGGCELRVKFSRSA DAPAYKQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 153) | aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctacaagcaggggcaga accagctctacaacgaactcaatcttggtcggagagagg agtacgacgtgctggacaagcggagaggacgggaccc agaaatgggcgggaagccgcgcagaaagaatccccaa gagggcctgtacaacgagctccaaaaggataagatggc agaagcctatagcgagattggtatgaaaggggaacgca gaagaggcaaaggccacgacggactgtaccagggact cagcaccgccaccaaggacacctatgacgctcttcacat gcaggccctgccgcctcgg (SEQ ID NO: 154) |
| SP-Linker 1-ERmut2-Furin cleavage site 2-CD19 CAR2 | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLGTGAEDPR PSRKRRSLGDVGEIVMTQSPA TLSLSPGERATLSCRASQDIS KYLNWYQQKPGQAPRLLIYHT SRLHSGIPARFSGSGSGTDYT LTISSLQPEDFAVYFCQQGNT LPYTFGQGTKLEIKGGGGSG GGGSGGGGSQVQLQESGPG LVKPSETLSLTCTVSGVSLPD YGVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca |
| | HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 155) | gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 156) |
| SP-Linker 1-ERmut2-Furin cleavage site 3-CD19 CAR1 | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLGTGAEDPR PSRKRREIVMTQSPATLSLSP GERATLSCRASQDISKYLNWY QQKPGQAPRLLIYHTSRLHSG IPARFSGSGSGTDYTLTISSLQ PEDFAVYFCQQGNTLPYTFG QGTKLEIKGGGGSGGGGSGG GGSQVQLQESGPGLVKPSET LSLTCTVSGVSLPDYGVSWIR QPPGKGLEWIGVIWGSETTYY SSSLKSRVTISKDNSKNQVSL KLSSVTAADTAVYYCAKHYYY GGSYAMDYWGQGTLVTVSST TTPAPRPPTPAPTIASQPLSLR | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactcGgga cgggagctgaagatccacgacccagcagaaagcgacg ggaaattgtgatgacccagtcacccgccactcttagcctttc acccggtgagcgcgcaaccctgtcttgcagagcctccca agacatctcaaaataccttaattggtatcaacagaagccc ggacaggctcctcgccttctgatctaccacaccagccggc tccattctggaatccctgccaggttcagcggtagcggatct |
| | PEACRPAAGGAVHTRGLDFA CDIYIWAPLAGTCGVLLLSLVIT LYCKRGRKKLLYIFKQPFMRP VQTTQEEDGCSCRFPEEEEG GCELRVKFSRSADAPAYKQG QNQLYNELNLGRREEYDVLD KRRGRDPEMGGKPRRKNPQ EGLYNELQKDKMAEAYSEIGM KGERRRGKGHDGLYQGLSTA TKDTYDALHMQALPPR (SEQ ID NO: 157) | gggaccgactacaccctcactatcagctcactgcagcca gaggacttcgctgtctatttctgtcagcaagggaacaccct gccctacacctttggacagggcaccaagctcgagattaa aggtggaggtggcagcggaggaggtgggtccggcggtg gaggaagccaggtccaactccaagaaagcggaccggg tcttgtgaagccatcagaaactctttcactgacttgtactgtg agcggagtgtctctccccgattacggggtgtcttggatcag acagccaccggggaagggtctggaatggattggagtgat ttggggctctgagactacttactactcttcatccctcaagtca cgcgtcaccatctcaaaggacaactctaagaatcaggtgt cactgaaactgtcatctgtgaccgcagccgacaccgccgt gtactattgcgctaagcattactattatggcgggagctacgc aatggattactggggacagggtactctggtcaccgtgtcca gcaccactaccccagcaccgaggccacccaccccggct cctaccatcgcctcccagcctctgtccctgcgtccggaggc atgtagacccgcagctggtggggccgtgcatacccgggg tcttgacttcgcctgcgatatctacatttgggcccctctggctg gtacttgcggggtcctgctgctttcactcgtgatcactctttact gtaagcgcggtcggaagaagctgctgtacatctttaagca acccttcatgaggcctgtgcagactactcaagaggagga cggctgttcatgccggttcccagaggaggaggaaggcgg ctgcgaactgcgcgtgaaattcagccgcagcgcagatgc tccagcctacaagcaggggcagaaccagctctacaacg aactcaatcttggtcggagagaggagtacgacgtgctgg acaagcggagaggacgggacccagaaatgggcggga agccgcgcagaaagaatccccaagagggcctgtacaa cgagctccaaaaggataagatggcagaagcctatagcg agattggtatgaaaggggaacgcagaagaggcaaagg ccacgacggactgtaccagggactcagcaccgccacca aggacacctatgacgctcttcacatgcaggccctgccgcc tcgg (SEQ ID NO: 158) |
| SP-Linker 1-ERmut2-Furin cleavage site 3-CD19 CAR2 | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLGTGAEDPR PSRKRREIVMTQSPATLSLSP GERATLSCRASQDISKYLNWY QQKPGQAPRLLIYHTSRLHSG IPARFSGSGSGTDYTLTISSLQ PEDFAVYFCQQGNTLPYTFG QGTKLEIKGGGGSGGGGSGG GGSQVQLQESGPGLVKPSET LSLTCTVSGVSLPDYGVSWIR QPPGKGLEWIGVIWGSETTYY SSSLKSRVTISKDNSKNQVSL KLSSVTAADTAVYYCAKHYYY GGSYAMDYWGQGTLVTVSST | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactcGgga cgggagctgaagatccacgacccagcagaaagcgacg ggaaattgtgatgacccagtcacccgccactcttagcctttc acccggtgagcgcgcaaccctgtcttgcagagcctccca agacatctcaaaataccttaattggtatcaacagaagccc ggacaggctcctcgccttctgatctaccacaccagccggc |
| | TTPAPRPPTPAPTIASQPLSLR PEACRPAAGGAVHTRGLDFA CDIYIWAPLAGTCGVLLLSLVIT LYCKRGRKKLLYIFKQPFMRP VQTTQEEDGCSCRFPEEEEG GCELRVKFSRSADAPAYQQG QNQLYNELNLGRREEYDVLD KRRGRDPEMGGKPRRKNPQ EGLYNELQKDKMAEAYSEIGM KGERRRGKGHDGLYQGLSTA TKDTYDALHMQALPPR (SEQ ID NO: 159) | tccattctggaatccctgccaggttcagcggtagcggatct gggaccgactacaccctcactatcagctcactgcagcca gaggacttcgctgtctatttctgtcagcaagggaacaccct gccctacacctttggacagggcaccaagctcgagattaa aggtggaggtggcagcggaggaggtgggtccggcggtg gaggaagccaggtccaactccaagaaagcggaccggg tcttgtgaagccatcagaaactctttcactgacttgtactgtg agcggagtgtctctccccgattacggggtgtcttggatcag acagccaccggggaagggtctggaatggattggagtgat ttggggctctgagactacttactactcttcatccctcaagtca cgcgtcaccatctcaaaggacaactctaagaatcaggtgt cactgaaactgtcatctgtgaccgcagccgacaccgccgt gtactattgcgctaagcattactattatggcgggagctacgc aatggattactggggacagggtactctggtcaccgtgtcca gcaccactaccccagcaccgaggccacccaccccggct cctaccatcgcctcccagcctctgtccctgcgtccggaggc atgtagacccgcagctggtggggccgtgcatacccgggg tcttgacttcgcctgcgatatctacatttgggcccctctggctg gtacttgcggggtcctgctgctttcactcgtgatcactctttact gtaagcgcggtcggaagaagctgctgtacatctttaagca acccttcatgaggcctgtgcagactactcaagaggagga cggctgttcatgccggttcccagaggaggaggaaggcgg ctgcgaactgcgcgtgaaattcagccgcagcgcagatgc tccagcctaccagcaggggcagaaccagctctacaacg aactcaatcttggtcggagagaggagtacgacgtgctgg acaagcggagaggacgggacccagaaatgggcggga agccgcgcagaaagaatccccaagagggcctgtacaa cgagctccaaaaggataagatggcagaagcctatagcg agattggtatgaaaggggaacgcagaagaggcaaagg ccacgacggactgtaccagggactcagcaccgccacca aggacacctatgacgctcttcacatgcaggccctgccgcc tcgg (SEQ ID NO: 160) |
| SP-Linker 1-ERmut1-Furin cleavage site 1-CD123 CAR | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLRTKRQVQL VQSGAEVKKPGASVKVSCKA SGYTFTGYYMHWVRQAPGQ GLEWMGWINPNSGGTNYAQ KFQGRVTLTRDTSISTVYMEL SRLRSDDTAVYYCARDMNILA TVPFDIWGQGTMVTVSSGGG GSGGGGSGGGGSDIQMTQS PSSLSASVGDRVTITCRASQSI SSYLNWYQQKPGKAPKLLIYA ASSLQSGVPSRFSGSGSGTD FTLTVNSLQPEDFATYYCQQG | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctggccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgtccaagaggatggaacacc tgtacagcatgaagtgcaagaacgtcgtgcctctgtccgat ctgctcctggaaatgctggacgcgcacagactccgtacta aaagacaagtgcaactcgtccaaagcggagcggaagtc aagaaacccggagcgagcgtgaaagtgtcctgcaaagc ctccggctacacctttacgggctactacatgcactgggtgc gccaggcaccaggacagggtcttgaatggatgggatgg |
| | DSVPLTFGGGTRLEIKTTTPAP RPPTPAPTIASQPLSLRPEAC RPAAGGAVHTRGLDFACDIYI WAPLAGTCGVLLLSLVITLYCK RGRKKLLYIFKQPFMRPVQTT QEEDGCSCRFPEEEEGGCEL RVKFSRSADAPAYQQGQNQL YNELNLGRREEYDVLDKRRG RDPEMGGKPRRKNPQEGLYN ELQKDKMAEAYSEIGMKGER RRGKGHDGLYQGLSTATKDT YDALHMQALPPR (SEQ ID NO: 161) | atcaaccctaattcgggcggaactaactacgcacagaag ttccaggggagagtgactctgactcgggatacctccatctc aactgtctacatggaactctcccgcttgcggtcagatgata cggcagtgtactactgcgcccgcgacatgaatatcctggct accgtgccgttcgacatctggggacaggggactatggtta ctgtctcatcgggcggtggaggttcaggaggaggcggctc gggaggcggaggttcggacattcagatgacccagtcccc atcctctctgtcggccagcgtcggagatagggtgaccatta cctgtcgggcctcgcaaagcatctcctcgtacctcaactgg tatcagcaaaagccgggaaaggcgcctaagctgctgatc tacgccgcttcgagcttgcaaagcggggtgccatccagat tctcgggatcaggctcaggaaccgacttcaccctgaccgt gaacagcctccagccggaggactttgccacttactactgc cagcagggagactccgtgccgcttactttcggggggggta cccgcctggagatcaagaccactaccccagcaccgagg ccacccaccccggctcctaccatcgcctcccagcctctgtc cctgcgtccggaggcatgtagacccgcagctggtggggc cgtgcatacccggggtcttgacttcgcctgcgatatctacatt tgggcccctctggctggtacttgcggggtcctgctgctttcac tcgtgatcactctttactgtaagcgcggtcggaagaagctg ctgtacatctttaagcaacccttcatgaggcctgtgcagact actcaagaggaggacggctgttcatgccggttcccagag gaggaggaaggcggctgcgaactgcgcgtgaaattcag ccgcagcgcagatgctccagcctaccagcaggggcaga accagctctacaacgaactcaatcttggtcggagagagg agtacgacgtgctggacaagcggagaggacgggaccc agaaatgggcgggaagccgcgcagaaagaatccccaa gagggcctgtacaacgagctccaaaaggataagatggc agaagcctatagcgagattggtatgaaaggggaacgca gaagaggcaaaggccacgacggactgtaccagggact cagcaccgccaccaaggacacctatgacgctcttcacat gcaggccctgccgcctcgg (SEQ ID NO: 162) |
| SP-Linker 1-**ERmut1-Furin cleavage site 2-CD123 CAR (construct 119)** | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRRSLGDVGQVQLVQSG AEVKKPGASVKVSCKASGYTF TGYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV | atggccctccctgtcaccgccctgctgcttccgctggctcttc tgctccacgccgctcggccccggtcgtcgttggcactttccc tgactgccgaccagatggtgtccgcccttctggacgccga gcctccaattctgtactcggagtacgatccgactcgcccgtt ctccgaagccagcatgatgggcctgttgactaacctggcg gaccgcgagttggtgcacatgattaactgggctaagcggg tgccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtcaagtgcaactcgtccaaagcg gagcggaagtcaagaaacccggagcgagcgtgaaagt gtcctgcaaagcctccggctacacctttacgggctactaca |
| | NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 163) | tgcactgggtgcgccaggcaccaggacagggtcttgaat ggatgggatggatcaaccctaattcgggcggaactaacta cgcacagaagttccaggggagagtgactctgactcggga tacctccatctcaactgtctacatggaactctcccgcttgcg gtcagatgatacggcagtgtactactgcgcccgcgacatg aatatcctggctaccgtgccgttcgacatctggggacagg ggactatggttactgtctcatcgggcggtggaggttcagga ggaggcggctcgggaggcggaggttcggacattcagat gacccagtccccatcctctctgtcggccagcgtcggagat agggtgaccattacctgtcgggcctcgcaaagcatctcctc gtacctcaactggtatcagcaaaagccgggaaaggcgc ctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 164) |
| SP-Linker 1-**ERmut1-Furin cleavage site 3-CD123 CAR (construct 122)** | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRRQVQLVQSGAEVKKP GASVKVSCKASGYTFTGYYM HWVRQAPGQGLEWMGWINP NSGGTNYAQKFQGRVTLTRD TSISTVYMELSRLRSDDTAVY YCARDMNILATVPFDIWGQGT MVTVSSGGGGSGGGGSGGG GSDIQMTQSPSSLSASVGDR VTITCRASQSISSYLNWYQQK | atggccctccctgtcaccgccctgctgcttccgctggctcttc tgctccacgccgctcggccccggtcgtcgttggcactttccc tgactgccgaccagatggtgtccgcccttctggacgccgag cctccaattctgtactcggagtacgatccgactcgcccgttc tccgaagccagcatgatgggcctgttgactaacctggcgga ccgcgagttggtgcacatgattaactgggctaagcgggtgc cgggcttcgtggacctggccctgcacgaccaagtgcacctc ctggaatgcgcctggatggaaatcctcatgatcggcctcgt gtggagatccatggagcatcccggaaagctcctgtttgcac ccaacctcctgcttgatcgcaaccagggaaaatgcgtgga agggggtgtcgagattttcgacatgctgctcgccacctcttc ccggttccggatgatgaatctgcagggagaagagttcgtgt gtctgaagtcaatcatcctgctgaactccggggtctatacct tcctgagctcgaccctcaagtcactggaggaaaaagacca catccatcgcgtgctcgataagatcaccgacacccttatcc atctcatggcgaaggctggactgaccctgcaacagcagca ccagaggctggcccagttgctgctgattctgagccacatcc ggcacatgtcgtccaagaggatggaacacctgtacagcat gaagtgcaagaacgtcgtgcctctgtccgatctgctcctgg |
| | PGKAPKLLIYAASSLQSGVPS RFSGSGSGTDFTLTVNSLQPE DFATYYCQQGDSVPLTFGGG TRLEIKTTTPAPRPPTPAPTI ASQPLSLRPEACRPAAGG AVHTRGLDFACDIYIWAPLA GTCGVLLLSLVITLYCKRGR KKLLYIFKQPFMRPVQTTQ EEDGCSCRFPEEEEGGCE LRVKFSRSADAPAYQQGQ NQLYNELNLGRREEYDVLD KRRGRDPEMGGKPRRKNP QEGLYNELQKDKMAEAYS EIGMKGERRRGKGHDGLY QGLSTATKDTYDALHMQAL PPR (SEQ ID NO: 165) | aaatgctggacgcgcacagactcggaaccggcgcggaag acccccggccctccaggaagcgaaggcaagtgcaactcgt ccaaagcggagcggaagtcaagaaacccggagcgagcgt gaaagtgtcctgcaaagcctccggctacacctttacgggct actacatgcactgggtgcgccaggcaccaggacagggtct tgaatggatgggatggatcaaccctaattcgggcggaact aactacgcacagaagttccaggggagagtgactctgactc gggatacctccatctcaactgtctacatggaactctcccgct tgcggtcagatgatacggcagtgtactactgcgcccgcgac atgaatatcctggctaccgtgccgttcgacatctggggaca ggggactatggttactgtctcatcgggcggtggaggttcag gaggaggcggctcgggaggcggaggttcggacattcagat gacccagtccccatcctctctgtcggccagcgtcggagata gggtgaccattacctgtcgggcctcgcaaagcatctcctcgt acctcaactggtatcagcaaaagccgggaaaggcgcctaa gctgctgatctacgccgcttcgagcttgcaaagcggggtgc catccagattctcgggatcaggctcaggaaccgacttcacc ctgaccgtgaacagcctccagccggaggactttgccactta ctactgccagcagggagactccgtgccgcttactttcgggg ggggtacccgcctggagatcaagaccactaccccagcacc gaggccacccaccccggctcctaccatcgcctcccagcctc tgtccctgcgtccggaggcatgtagacccgcagctggtggg gccgtgcatacccggggtcttgacttcgcctgcgatatctac atttgggcccctctggctggtacttgcggggtcctgctgcttt cactcgtgatcactctttactgtaagcgcggtcggaagaag ctgctgtacatctttaagcaacccttcatgaggcctgtgcag actactcaagaggaggacggctgttcatgccggttcccaga ggaggaggaaggcggctgcgaactgcgcgtgaaattcag ccgcagcgcagatgctccagcctaccagcaggggcagaac cagctctacaacgaactcaatcttggtcggagagaggagt acgacgtgctggacaagcggagaggacgggacccagaaa tgggcgggaagccgcgcagaaagaatccccaagagggcc tgtacaacgagctccaaaaggataagatggcagaagccta tagcgagattggtatgaaaggggaacgcagaagaggcaa aggccacgacggactgtaccagggactcagcaccgccacc aaggacacctatgacgctcttcacatgcaggccctgccgcc tcgg (SEQ ID NO: 166) |
| SP-Linker 1-ERmut2-Furin cleavage site 1-CD123 CAR | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga |
| | SDLLLEMLDAHRLRTKRQVQL VQSGAEVKKPGASVKVSCKA SGYTFTGYYMHWVRQAPGQ GLEWMGWINPNSGGTNYAQ KFQGRVTLTRDTSISTVYMEL SRLRSDDTAVYYCARDMNILA TVPFDIWGQGTMVTVSSGGG GSGGGGSGGGGSDIQMTQS PSSLSASVGDRVTITCRASQSI SSYLNWYQQKPGKAPKLLIYA ASSLQSGVPSRFSGSGSGTD FTLTVNSLQPEDFATYYCQQG DSVPLTFGGGTRLEIKTTTPAP RPPTPAPTIASQPLSLRPEAC RPAAGGAVHTRGLDFACDIYI WAPLAGTCGVLLLSLVITLYCK RGRKKLLYIFKQPFMRPVQTT QEEDGCSCRFPEEEEGGCEL RVKFSRSADAPAYQQGQNQL YNELNLGRREEYDVLDKRRG RDPEMGGKPRRKNPQEGLYN ELQKDKMAEAYSEIGMKGER RRGKGHDGLYQGLSTATKDT YDALHMQALPPR (SEQ ID NO: 167) | gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactccgtact aaaagacaagtgcaactcgtccaaagcggagcggaagt caagaaacccggagcgagcgtgaaagtgtcctgcaaag cctccggctacacctttacgggctactacatgcactgggtg cgccaggcaccaggacagggtcttgaatggatgggatgg atcaaccctaattcgggcggaactaactacgcacagaag ttccaggggagagtgactctgactcgggatacctccatctc aactgtctacatggaactctcccgcttgcggtcagatgata cggcagtgtactactgcgcccgcgacatgaatatcctggct accgtgccgttcgacatctggggacaggggactatggtta ctgtctcatcgggcggtggaggttcaggaggaggcggctc gggaggcggaggttcggacattcagatgacccagtcccc atcctctctgtcggccagcgtcggagatagggtgaccatta cctgtcgggcctcgcaaagcatctcctcgtacctcaactgg tatcagcaaaagccgggaaaggcgcctaagctgctgatc tacgccgcttcgagcttgcaaagcggggtgccatccagat tctcgggatcaggctcaggaaccgacttcaccctgaccgt gaacagcctccagccggaggactttgccacttactactgc cagcagggagactccgtgccgcttactttcggggggggta cccgcctggagatcaagaccactaccccagcaccgagg ccacccaccccggctcctaccatcgcctcccagcctctgtc cctgcgtccggaggcatgtagacccgcagctggtggggc cgtgcatacccggggtcttgacttcgcctgcgatatctacatt tgggcccctctggctggtacttgcggggtcctgctgctttcac tcgtgatcactctttactgtaagcgcggtcggaagaagctg ctgtacatctttaagcaacccttcatgaggcctgtgcagact actcaagaggaggacggctgttcatgccggttcccagag gaggaggaaggcggctgcgaactgcgcgtgaaattcag ccgcagcgcagatgctccagcctaccagcaggggcaga accagctctacaacgaactcaatcttggtcggagagagg agtacgacgtgctggacaagcggagaggacgggaccc agaaatgggcgggaagccgcgcagaaagaatccccaa gagggcctgtacaacgagctccaaaaggataagatggc agaagcctatagcgagattggtatgaaaggggaacgca gaagaggcaaaggccacgacggactgtaccagggact cagcaccgccaccaaggacacctatgacgctcttcacat gcaggccctgccgcctcgg (SEQ ID NO: 168) |
| SP-Linker 1-ERmut2-Furin cleavage site 2-CD123 CAR | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca |
| | MSNKRMEHLYSMKCKNVVPL SDLLLEMLDAHRLGTGAEDPR PSRKRRSLGDVGQVQLVQSG AEVKKPGASVKVSCKASGYTF TGYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 169) | cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactcggaa ccggcgcggaagacccccggccctccaggaagcgaag gtccctcggagacgtgggtcaagtgcaactcgtccaaag cggagcggaagtcaagaaacccggagcgagcgtgaaa gtgtcctgcaaagcctccggctacacctttacgggctacta catgcactgggtgcgccaggcaccaggacagggtcttga atggatgggatggatcaaccctaattcgggcggaactaac tacgcacagaagttccaggggagagtgactctgactcgg gatacctccatctcaactgtctacatggaactctcccgcttg cggtcagatgatacggcagtgtactactgcgcccgcgac atgaatatcctggctaccgtgccgttcgacatctggggaca ggggactatggttactgtctcatcgggcggtggaggttcag gaggaggcggctcgggaggcggaggttcggacattcag atgacccagtccccatcctctctgtcggccagcgtcggag atagggtgaccattacctgtcgggcctcgcaaagcatctcc tcgtacctcaactggtatcagcaaaagccgggaaaggcg cctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 170) |
| SP-Linker 1-ERmut2-Furin cleavage site 3-CD123 CAR | MALPVTALLLPLALLLHAARPR SSLALSLTADQMVSALLDAEP PILYSEYDPTRPFSEASMMGL LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg |
| | DHIHRVLDKITDTLIHLMAKAG LTLQQQHQRLAQLLLILSHIRH MSNKRMEHLYSMKCKNVVPL SDLLLEMLDAHRLGTGAEDPR PSRKRRQVQLVQSGAEVKKP GASVKVSCKASGYTFTGYYM HWVRQAPGQGLEWMGWINP NSGGTNYAQKFQGRVTLTRD TSISTVYMELSRLRSDDTAVY YCARDMNILATVPFDIWGQGT MVTVSSGGGGSGGGGSGGG GSDIQMTQSPSSLSASVGDR VTITCRASQSISSYLNWYQQK PGKAPKLLIYAASSLQSGVPS RFSGSGSGTDFTLTVNSLQPE DFATYYCQQGDSVPLTFGGG TRLEIKTTTPAPRPPTPAPTIA SQPLSLRPEACRPAAGGAVH TRGLDFACDIYIWAPLAGTCG VLLLSLVITLYCKRGRKKLLYIF KQPFMRPVQTTQEEDGCSCR FPEEEEGGCELRVKFSRSAD APAYQQGQNQLYNELNLGRR EEYDVLDKRRGRDPEMGGKP RRKNPQEGLYNELQKDKMAE AYSEIGMKGERRRGKGHDGL YQGLSTATKDTYDALHMQALP PR* (SEQ ID NO: 171) | tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactcggga cgggagctgaagatccacgacccagcagaaagcgacg gcaagtgcaactcgtccaaagcggagcggaagtcaaga aacccggagcgagcgtgaaagtgtcctgcaaagcctcc ggctacacctttacgggctactacatgcactgggtgcgcca ggcaccaggacagggtcttgaatggatgggatggatcaa ccctaattcgggcggaactaactacgcacagaagttcca ggggagagtgactctgactcgggatacctccatctcaact gtctacatggaactctcccgcttgcggtcagatgatacggc agtgtactactgcgcccgcgacatgaatatcctggctaccg tgccgttcgacatctggggacaggggactatggttactgtct catcgggcggtggaggttcaggaggaggcggctcggga ggcggaggttcggacattcagatgacccagtccccatcct ctctgtcggccagcgtcggagatagggtgaccattacctgt cgggcctcgcaaagcatctcctcgtacctcaactggtatca gcaaaagccgggaaaggcgcctaagctgctgatctacg ccgcttcgagcttgcaaagcggggtgccatccagattctcg ggatcaggctcaggaaccgacttcaccctgaccgtgaac agcctccagccggaggactttgccacttactactgccagc agggagactccgtgccgcttactttcggggggggtacccg cctggagatcaagaccactaccccagcaccgaggccac ccaccccggctcctaccatcgcctcccagcctctgtccctg cgtccggaggcatgtagacccgcagctggtggggccgtg catacccggggtcttgacttcgcctgcgatatctacatttgg gcccctctggctggtacttgcggggtcctgctgctttcactcg tgatcactctttactgtaagcgcggtcggaagaagctgctgt acatctttaagcaacccttcatgaggcctgtgcagactactc aagaggaggacggctgttcatgccggttcccagaggagg aggaaggcggctgcgaactgcgcgtgaaattcagccgc agcgcagatgctccagcctaccagcaggggcagaacca gctctacaacgaactcaatcttggtcggagagaggagtac gacgtgctggacaagcggagaggacgggacccagaaa tgggcgggaagccgcgcagaaagaatccccaagaggg cctgtacaacgagctccaaaaggataagatggcagaag cctatagcgagattggtatgaaaggggaacgcagaaga ggcaaaggccacgacggactgtaccagggactcagca ccgccaccaaggacacctatgacgctcttcacatgcagg ccctgccgcctcgg (SEQ ID NO: 172) |
| SP-Linker 1-ERmut1-Furin cleavage site 1-BCMA CAR | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctggccctgcacgaccaagtgc |
| | ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLRTKREVQL VESGGGLVQPGGSLRLSCAV SGFALSNHGMSWVRRAPGK GLEWVSGIVYSGSTYYAASVK GRFTISRDNSRNTLYLQMNSL RPEDTAIYYCSAHGGESDVW GQGTTVTVSSASGGGGSGG RASGGGGSDIQLTQSPSSLSA SVGDRVTITCRASQSISSYLN WYQQKPGKAPKLLIYAASSLQ SGVPSRFSGSGSGTDFTLTIS SLQPEDFATYYCQQSYSTPYT FGQGTKVEIKTTTPAPRPPTP APTIASQPLSLRPEACRPAAG GAVHTRGLDFACDIYIWAPLA GTCGVLLLSLVITLYCKRGRKK LLYIFKQPFMRPVQTTQEEDG CSCRFPEEEEGGCELRVKFS RSADAPAYQQGQNQLYNELN LGRREEYDVLDKRRGRDPEM GGKPRRKNPQEGLYNELQKD KMAEAYSEIGMKGERRRGKG HDGLYQGLSTATKDTYDALH MQALPPR (SEQ ID NO: 173) | acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgtccaagaggatggaacacc tgtacagcatgaagtgcaagaacgtcgtgcctctgtccgat ctgctcctggaaatgctggacgcgcacagactccgtacta aaagagaagtgcaattggtggaatcagggggaggacttg tgcagcctggaggatcgctgagactgtcatgtgccgtgtcc ggctttgccctgtccaaccacgggatgtcctgggtccgccg cgcgcctggaaagggcctcgaatgggtgtcgggtattgtgt acagcggtagcacctactatgccgcatccgtgaagggga gattcaccatcagccgggacaactccaggaacactctgta cctccaaatgaattcgctgaggccagaggacactgccatc tactactgctccgcgcatggcggagagtccgacgtctggg gacaggggaccaccgtgaccgtgtctagcgcgtccggcg gaggcggcagcgggggtcgggcatcagggggcggcgg atcggacatccagctcacccagtccccgagctcgctgtcc gcctccgtgggagatcgggtcaccatcacgtgccgcgcc agccagtcgatttcctcctacctgaactggtaccaacagaa gcccggaaaagccccgaagcttctcatctacgccgcctc gagcctgcagtcaggagtgccctcacggttctccggctcc ggttccggtactgatttcaccctgaccatttcctccctgcaac cggaggacttcgctacttactactgccagcagtcgtactcc accccctacactttcggacaaggcaccaaggtcgaaatc aagaccactaccccagcaccgaggccacccaccccgg ctcctaccatcgcctcccagcctctgtccctgcgtccggag gcatgtagacccgcagctggtggggccgtgcatacccgg ggtcttgacttcgcctgcgatatctacatttgggcccctctgg ctggtacttgcggggtcctgctgctttcactcgtgatcactcttt actgtaagcgcggtcggaagaagctgctgtacatctttaag caacccttcatgaggcctgtgcagactactcaagaggag gacggctgttcatgccggttcccagaggaggaggaaggc ggctgcgaactgcgcgtgaaattcagccgcagcgcagat gctccagcctaccagcaggggcagaaccagctctacaa cgaactcaatcttggtcggagagaggagtacgacgtgctg gacaagcggagaggacgggacccagaaatgggcggg aagccgcgcagaaagaatccccaagagggcctgtaca acgagctccaaaaggataagatggcagaagcctatagc gagattggtatgaaaggggaacgcagaagaggcaaag gccacgacggactgtaccagggactcagcaccgccacc aaggacacctatgacgctcttcacatgcaggccctgccgc ctcgg (SEQ ID NO: 174) |
| SP-Linker 1-**ERmut1-Furin cleavage site 2-BCMA CAR (construct 164)** | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL LLDRNQGKCVEGGVEIFDMLL | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctggccctgcacgaccaagtgc |
| | ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRRSLGDVGEVQLVESG GGLVQPGGSLRLSCAVSGFA LSNHGMSWVRRAPGKGLEW VSGIVYSGSTYYAASVKGRFTI SRDNSRNTLYLQMNSLRPED TAIYYCSAHGGESDVWGQGT TVTVSSASGGGGSGGRASGG GGSDIQLTQSPSSLSASVGDR VTITCRASQSISSYLNWYQQK PGKAPKLLIYAASSLQSGVPS RFSGSGSGTDFTLTISSLQPE DFATYYCQQSYSTPYTFGQG TKVEIKTTTPAPRPPTPAPTIA SQPLSLRPEACRPAAGGAVH TRGLDFACDIYIWAPLAGTCG VLLLSLVITLYCKRGRKKLLYIF KQPFMRPVQTTQEEDGCSCR FPEEEEGGCELRVKFSRSAD APAYQQGQNQLYNELNLGRR EEYDVLDKRRGRDPEMGGKP RRKNPQEGLYNELQKDKMAE AYSEIGMKGERRRGKGHDGL YQGLSTATKDTYDALHMQALP PR (SEQ ID NO: 175) | acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgtccaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcagg gggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 176) |
| SP-Linker 1-**ERmut1-Furin cleavage site 3-BCMA CAR** | MALPVTALLLPLALLLHAARPR SSLALSLTADQMVSALLDAEP PILYSEYDPTRPFSEASMMGL LTNLADRELVHMINWAKRVPG FVDLALHDQVHLLECAWMEIL MIGLVWRSMEHPGKLLFAPNL | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg ttctccgaagccagcatgatgggcctgttgactaacctggc ggaccgcgagttggtgcacatgattaactgggctaagcgg |
| | LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSSKRMEHLYSMKCKNVVPL SDLLLEMLDAHRLGTGAEDPR PSRKRREVQLVESGGGLVQP GGSLRLSCAVSGFALSNHGM SWVRRAPGKGLEWVSGIVYS GSTYYAASVKGRFTISRDNSR NTLYLQMNSLRPEDTAIYYCS AHGGESDVWGQGTTVTVSSA SGGGGSGGRASGGGGSDIQL TQSPSSLSASVGDRVTITCRA SQSISSYLNWYQQKPGKAPKL LIYAASSLQSGVPSRFSGSGS GTDFTLTISSLQPEDFATYYC QQSYSTPYTFGQGTKVEIKTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 177) | gtgccgggcttcgtggacctggccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgtccaagaggatggaacacc tgtacagcatgaagtgcaagaacgtcgtgcctctgtccgat ctgctcctggaaatgctggacgcgcacagactcGggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 178) |
| SP-Linker 1-ERmut2-Furin cleavage site 1-BCMA CAR | MALPVTALLLPLALLLHAARPR SSLALSLTADQMVSALLDAEP PILYSEYDPTRPFSEASMMGL LTNLADRELVHMINWAKRVPG | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg |
| | FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLRTKREVQL VESGGGLVQPGGSLRLSCAV SGFALSNHGMSWVRRAPGK GLEWVSGIVYSGSTYYAASVK GRFTISRDNSRNTLYLQMNSL RPEDTAIYYCSAHGGESDVW GQGTTVTVSSASGGGGSGG RASGGGGSDIQLTQSPSSLSA SVGDRVTITCRASQSISSYLN WYQQKPGKAPKLLIYAASSLQ SGVPSRFSGSGSGTDFTLTIS SLQPEDFATYYCQQSYSTPYT FGQGTKVEIKTTTPAPRPPTP APTIASQPLSLRPEACRPAAG GAVHTRGLDFACDIYIWAPLA GTCGVLLLSLVITLYCKRGRKK LLYIFKQPFMRPVQTTQEEDG CSCRFPEEEEGGCELRVKFS RSADAPAYQQGQNQLYNELN LGRREEYDVLDKRRGRDPEM GGKPRRKNPQEGLYNELQKD KMAEAYSEIGMKGERRRGKG HDGLYQGLSTATKDTYDALH MQALPPR (SEQ ID NO: 179) | ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactccgtact aaaagagaagtgcaattggtggaatcagggggaggactt gtgcagcctggaggatcgctgagactgtcatgtgccgtgtc cggctttgccctgtccaaccacgggatgtcctgggtccgcc gcgcgcctggaaagggcctcgaatgggtgtcgggtattgt gtacagcggtagcacctactatgccgcatccgtgaaggg gagattcaccatcagccgggacaactccaggaacactct gtacctccaaatgaattcgctgaggccagaggacactgc catctactactgctccgcgcatggcggagagtccgacgtct ggggacaggggaccaccgtgaccgtgtctagcgcgtcc ggcggaggcggcagcgggggtcgggcatcagggggcg gcggatcggacatccagctcacccagtccccgagctcgc tgtccgcctccgtgggagatcgggtcaccatcacgtgccg cgccagccagtcgatttcctcctacctgaactggtaccaac agaagcccggaaaagccccgaagcttctcatctacgccg cctcgagcctgcagtcaggagtgccctcacggttctccgg ctccggttccggtactgatttcaccctgaccatttcctccctgc aaccggaggacttcgctacttactactgccagcagtcgtac tccaccccctacactttcggacaaggcaccaaggtcgaa atcaagaccactaccccagcaccgaggccacccacccc ggctcctaccatcgcctcccagcctctgtccctgcgtccgg aggcatgtagacccgcagctggtggggccgtgcataccc ggggtcttgacttcgcctgcgatatctacatttgggcccctct ggctggtacttgcggggtcctgctgctttcactcgtgatcact ctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctaccagcaggggcagaaccagctcta caacgaactcaatcttggtcggagagaggagtacgacgt gctggacaagcggagaggacgggacccagaaatgggc gggaagccgcgcagaaagaatccccaagagggcctgt acaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 180) |
| SP-Linker 1-ERmut2-Furin cleavage site 2-BCMA CAR | MALPVTALLLPLALLLHAARPR SSLALSLTADQMVSALLDAEP PILYSEYDPTRPFSEASMMGL LTNLADRELVHMINWAKRVPG | atggccctccctgtcaccgccctgctgcttccgctggctcttc tgctccacgccgctcggccccggtcgTcgttggcactttcc ctgactgccgaccagatggtgtccgcccttctggacgccg agcctccaattctgtactcggagtacgatccgactcgcccg |
| | FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLGTGAEDPR PSRKRRSLGDVGEVQLVESG GGLVQPGGSLRLSCAVSGFA LSNHGMSWVRRAPGKGLEW VSGIVYSGSTYYAASVKGRFTI SRDNSRNTLYLQMNSLRPED TAIYYCSAHGGESDVWGQGT TVTVSSASGGGGSGGRASGG GGSDIQLTQSPSSLSASVGDR VTITCRASQSISSYLNWYQQK PGKAPKLLIYAASSLQSGVPS RFSGSGSGTDFTLTISSLQPE DFATYYCQQSYSTPYTFGQG TKVEIKTTTPAPRPPTPAPTIA SQPLSLRPEACRPAAGGAVH TRGLDFACDIYIWAPLAGTCG VLLLSLVITLYCKRGR KKLLYIFKQPFMRPVQTTQEE DGCSCRFPEEEEGGCELRVK FSRSADAPAYQQGQNQLYNE LNLGRREEYDVLDKRRGRDP EMGGKPRRKNPQEGLYNELQ KDKMAEAYSEIGMKGERRRG KGHDGLYQGLSTATKDTYDAL HMQALPPR (SEQ ID NO: 181) | ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactcggaa ccggcgcggaagacccccggccctccaggaagcgaag gtccctcggagacgtgggtgaagtgcaattggtggaatca gggggaggacttgtgcagcctggaggatcgctgagactg tcatgtgccgtgtccggctttgccctgtccaaccacgggatg tcctgggtccgccgcgcgcctggaaagggcctcgaatgg gtgtcgggtattgtgtacagcggtagcacctactatgccgc atccgtgaaggggagattcaccatcagccgggacaactc caggaacactctgtacctccaaatgaattcgctgaggcca gaggacactgccatctactactgctccgcgcatggcggag agtccgacgtctggggacaggggaccaccgtgaccgtgt ctagcgcgtccggcggaggcggcagcgggggtcgggc atcagggggcggcggatcggacatccagctcacccagtc cccgagctcgctgtccgcctccgtgggagatcgggtcacc atcacgtgccgcgccagccagtcgatttcctcctacctgaa ctggtaccaacagaagcccggaaaagccccgaagcttct catctacgccgcctcgagcctgcagtcaggagtgccctca cggttctccggctccggttccggtactgatttcaccctgacc atttcctccctgcaaccggaggacttcgctacttactactgc cagcagtcgtactccaccccctacactttcggacaaggca ccaaggtcgaaatcaagaccactaccccagcaccgagg ccacccaccccggctcctaccatcgcctcccagcctctgtc cctgcgtccggaggcatgtagacccgcagctggtggggc cgtgcatacccggggtcttgacttcgcctgcgatatctacatt tgggcccctctggctggtacttgcggggtcctgctgctttcac tcgtgatcactctttactgtaagcgcggtcggaagaagctg ctgtacatctttaagcaacccttcatgaggcctgtgcagact actcaagaggaggacggctgttcatgccggttcccagag gaggaggaaggcggctgcgaactgcgcgtgaaattcag ccgcagcgcagatgctccagcctaccagcaggggcaga accagctctacaacgaactcaatcttggtcggagagagg agtacgacgtgctggacaagcggagaggacgggaccc agaaatgggcgggaagccgcgcagaaagaatccccaa gagggcctgtacaacgagctccaaaaggataagatggc agaagcctatagcgagattggtatgaaaggggaacgca gaagaggcaaaggccacgacggactgtaccagggact cagcaccgccaccaaggacacctatgacgctcttcacat gcaggccctgccgcctcgg (SEQ ID NO: 182) |
| SP-Linker 1-**ERmut2-Furin cleavage site 3-** | MALPVTALLLPLALLLHAARPR SSLALSLTADQMVSALLDAEP PILYSEYDPTRPFSEASMMGL | atggccctccctgtcaccgccctgctgcttccgctggctcttc tgctccacgccgctcggccccggtcgTcgttggcactttcc ctgactgccgaccagatggtgtccgcccttctggacgccg |
| **BCMA CAR** | LTNLADRELVHMINWAKRVPG FVDLTLHDQVHLLECAWMEIL MIGLVWRSMEHPGKLLFAPNL LLDRNQGKCVEGGVEIFDMLL ATSSRFRMMNLQGEEFVCLK SIILLNSGVYTFLSSTLKSLEEK DHIHRVLDKITDTLIHLMAKAG LTLQQQHQRLAQLLLILSHIRH MSNKRMEHLYSMKCKNVVPL SDLLLEMLDAHRLGTGAEDPR PSRKRREVQLVESGGGLVQP GGSLRLSCAVSGFALSNHGM SWVRRAPGKGLEWVSGIVYS GSTYYAASVKGRFTISRDNSR NTLYLQMNSLRPEDTAIYYCS AHGGESDVWGQGTTVTVSSA SGGGGSGGRASGGGGSDIQL TQSPSSLSASVGDRVTITCRA SQSISSYLNWYQQKPGKAPKL LIYAASSLQSGVPSRFSGSGS GTDFTLTISSLQPEDFATYYC QQSYSTPYTFGQGTKVEIKTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 183) | agcctccaattctgtactcggagtacgatccgactcgcccg ttctccgaagccagcatgatgggcctgttgactaacctggc ggaccgcgagttggtgcacatgattaactgggctaagcgg gtgccgggcttcgtggacctgaccctgcacgaccaagtgc acctcctggaatgcgcctggatggaaatcctcatgatcgg cctcgtgtggagatccatggagcatcccggaaagctcctg tttgcacccaacctcctgcttgatcgcaaccagggaaaatg cgtggaagggggtgtcgagattttcgacatgctgctcgcca cctcttcccggttccggatgatgaatctgcagggagaaga gttcgtgtgtctgaagtcaatcatcctgctgaactccggggt ctataccttcctgagctcgaccctcaagtcactggaggaaa aagaccacatccatcgcgtgctcgataagatcaccgaca cccttatccatctcatggcgaaggctggactgaccctgcaa cagcagcaccagaggctggcccagttgctgctgattctga gccacatccggcacatgtcgaacaagaggatggaacac ctgtacagcatgaagtgcaagaacgtcgtgcctctgtccg atctgctcctggaaatgctggacgcgcacagactcggga cgggagctgaagatccacgacccagcagaaagcgacg ggaagtgcaattggtggaatcagggggaggacttgtgca gcctggaggatcgctgagactgtcatgtgccgtgtccggct ttgccctgtccaaccacgggatgtcctgggtccgccgcgc gcctggaaagggcctcgaatgggtgtcgggtattgtgtac agcggtagcacctactatgccgcatccgtgaaggggaga ttcaccatcagccgggacaactccaggaacactctgtacc tccaaatgaattcgctgaggccagaggacactgccatcta ctactgctccgcgcatggcggagagtccgacgtctgggg acaggggaccaccgtgaccgtgtctagcgcgtccggcgg aggcggcagcgggggtcgggcatcagggggcggcgga tcggacatccagctcacccagtccccgagctcgctgtccg cctccgtgggagatcgggtcaccatcacgtgccgcgcca gccagtcgatttcctcctacctgaactggtaccaacagaag cccggaaaagccccgaagcttctcatctacgccgcctcg agcctgcagtcaggagtgccctcacggttctccggctccg gttccggtactgatttcaccctgaccatttcctccctgcaacc ggaggacttcgctacttactactgccagcagtcgtactcca ccccctacactttcggacaaggcaccaaggtcgaaatca agaccactaccccagcaccgaggccacccaccccggct cctaccatcgcctcccagcctctgtccctgcgtccggaggc atgtagacccgcagctggtggggccgtgcatacccgggg tcttgacttcgcctgcgatatctacatttgggcccctctggctg gtacttgcggggtcctgctgctttcactcgtgatcactctttact gtaagcgcggtcggaagaagctgctgtacatctttaagca acccttcatgaggcctgtgcagactactcaagaggagga cggctgttcatgccggttcccagaggaggaggaaggcgg ctgcgaactgcgcgtgaaattcagccgcagcgcagatgc tccagcctaccagcaggggcagaaccagctctacaacg aactcaatcttggtcggagagaggagtacgacgtgctgg acaagcggagaggacgggacccagaaatgggcggga agccgcgcagaaagaatccccaagagggcctgtacaa cgagctccaaaaggataagatggcagaagcctatagcg agattggtatgaaaggggaacgcagaagaggcaaagg ccacgacggactgtaccagggactcagcaccgccacca aggacacctatgacgctcttcacatgcaggccctgccgcc tcgg (SEQ ID NO: 184) |

### Cell lines and cell transduction

Jurkat cells were obtained from the ATCC and maintained in media according to supplier's recommendations. Jurkat cell lines were transduced by spinoculation with lentiviral supernatant from 293T cells transfected with the various plasmid DNAs.

### Cancer Cells

NALM6, K562, Molm13 cell lines were obtained from the ATCC and maintained in media according to supplier's recommendations. To produce a bioluminescent model for T cell killing assays, all cells were transduced with a luciferase lentiviral construct and kept under antibiotic selection.

### Compound treatments

4-hydroxytamoxifen (4-OHT) was resuspended in ethanol. Bazedoxifene and raloxifene were resuspended in DMSO. In all instances, where concentrations not indicated, compounds were added at 1uM for 24 hours prior to analysis by flow cytometry.

### Flow cytometry

Cells were isolated from *in vitro* culture, washed once in PBS supplemented with 0.5% bovine serum albumin, and stained on ice using either biotinylated Protein L followed by incubated with a fluorochromeconjugated streptavidin reagent or an antibody recognizing the given target antigen. In all analyses, the population of interest was gated based on forward vs side scatter characteristics, followed by singlet gating, and live cells were gated. Flow cytometry was performed on a four laser Fortessa (Becton-Dickinson).

### Tumor killing assay

In brief, luciferase transduced target cell lines were co-cultured at the indicated ratios with effector CD19CAR, FurON-CD19CAR, CD123CAR, FurON-CD123 CAR T cells for 20 hours, in the presence or absence of bazedoxifene. Remaining luciferase activity was measured by adding Bright-Glo (Promega) on a luminescence reader (Envision). Percentage killing was calculated using negative controls.

### Cytokine secretion

Effector and luciferase transduced target cell lines were co-cultured at a 3:1 ratio in RPMI containing 10% FBS for 20 hours, in the presence or absence of bazedoxifene. Supernatant was analysed by 3-plex array according the manufacturer's instructions (Invitrogen).

### Proliferation assay

Effector and irradiated target cells were co-cultured for 4 days, in the presence or absence of bazedoxifene. Cells were then harvested, washed, and stained with an anti-CD3 antibody and/or protein L reagent to detect CAR surface expression. Cells were then washed, resuspended in FACS buffer and a fixed volume of Absolute Counting Beads are added to each sample. Samples were run on a Fortessa FACS analyzer and the same number of events in the Counting Beads gate was collected for all samples.

### EXAMPLE 14: EVALUATION OF DIFFERENT FURIN CLEAVAGE SITES WITHIN THE FURIN DEGRON DOMAIN IN THE CONTEXT OF CAR19 (ANTI-CD19 SCFV CAR)

Gene fragments, comprising a furin degron domain fused to an anti-CD19 scFV CAR and preceded by a T7 promoter, were subjected to in vitro transcription/ translation. The differences between gene fragments lay in the furin cleavage sites created, as annotated. The *in vitro* synthesized proteins were incubated with furin as indicated, at 37⁰C for 1hr. The samples were loaded onto a reducing SDS-PAGE gel and analyzed by immunoblotting using an anti-CD3ζ antibody.

FIGs. 12A-12B showed the degree of furin cleavage across the various tested furin cleavage sites, in the CAR19 context. The tested furin cleavage sites are: #105 - LQWLEQQVAKRRTKR (SEQ ID NO: 129); #106 - GTGAEDPRPSRKRRSLGDVG (SEQ ID NO: 125); #107 - GTGAEDPRPSRKRRSLGG (SEQ ID NO: 131); #108 - GTGAEDPRPSRKRRSLG (SEQ ID NO: 133); #102 - SLNLTESHNSRKKR (SEQ ID NO: 135); #103 - GTGAEDPRPSRKRR (SEQ ID NO: 127); #104 - CKINGYPKRGRKRR (SEQ ID NO: 137); #73 - RTKR (SEQ ID NO: 123). A gene fragment derived from the parental CAR19 was used as a control (Ctrl) and no cleavage was observed (FIGs. 12A-12B).

### EXAMPLE 15: COMPOUND INDUCIBLE CAR EXPRESSION IN PRIMARY HUMAN T CELLS

Primary human T cells were transduced with a furin degron domain fused to an anti-CD19 scFV CAR construct (construct #106). Untransduced T cells (UTD) and the parental CAR19 were used as controls. Cells were expanded for 10 days in the presence of anti-CD3/CD28 stimulation beads. 100U/mL IL-2 was added on day 7, bazedoxifene (BZA) was added on day 8. Prior to freezing down the cells, CAR expression was determined on day 10 by FACS using protein L staining. Cell number, size, and viability were also assessed.

The data demonstrated that the furin degron domain regulates CAR19 expression in a stabilizing compound-dependent manner in human primary T cells, with the MFI (mean fluorescence intensity) of the CAR being lower compared to the parental CAR construct (FIG. 13). Also, the furin degron domain had no impact on cell viability and cell proliferation (FIG. 13).

### EXAMPLE 16: COMPOUND-DEPENDENT FURON CAR-MEDIATED TUMOR KILLING

Primary human T cells were transduced with a furin degron domain fused to an anti-CD19 scFV CAR (construct #106). Untransduced T cells (UTD) were used as control. The parental CAR19 construct was included as a benchmark for full CAR functional activity. Cells are expanded for 10 days in the presence of anti-CD3/CD28 stimulation beads. 100U/mL IL-2 was added on day 7, bazedoxifene was added on day 8 (samples CD19CAR+ and FurON 106+) or omitted (samples CD19CAR and FurON 106). Cells were frozen on day 10. T cells were thawed and co-cultured with the luciferized target cell lines (CD19+ NALM6 cells or CD19- K562 cells) and bazedoxifene, for 20 hours. CART cells were normalized to the same percentage of CAR expressing cells. Percent tumor lysis was determined by analysis of remaining luciferase activity.

These data demonstrated that CAR19 with the furin degron domain kills CD19+ tumor cells in a stabilizing compound dose-dependent manner and is noninferior to the parental CAR construct (FIG. 14).

### EXAMPLE 17: COMPOUND- AND TUMOR-DEPENDENT CYTOKINE SECRETION BY THE FURON CAR

Primary human T cells were transduced with a furin degron domain fused to an anti-CD19 scFV CAR (construct 106). Untransduced T cells (UTD) were used as control. The parental CAR19 construct was included as a benchmark for full CAR functional activity. Cells were expanded for 10 days in the presence of anti-CD3/CD28 stimulation beads. 100U/mL IL-2 was added on day 7, bazedoxifene (BAZ) was added on day 8 (samples CD19CAR+BAZ and FurON 106+BAZ) or omitted (samples CD19CAR and FurON 106). Cells were frozen on day 10. T cells were thawed and co-cultured with luciferized NALM6, a CD19+ target cell line, for 20 hours, and bazedoxifene as indicated. CART cells were normalized to the same percentage of CAR expressing cells. Supernatants were harvested and evaluated for levels of IFNγ.

These data demonstrated that primary human T cells expressing ERαFurON CAR19 secrete IFNγ in the presence of CD19+ tumor cells, in a manner that is stabilizing compound dose-dependent and is noninferior to the parental CAR19 construct (FIG. 15).

### EXAMPLE 18: COMPOUND- AND TUMOR-DEPENDENT PROLIFERATION OF THE FURON CAR

Primary human T cells were transduced with a furin degron domain fused to an anti-CD19 scFV CAR (construct 106). Untransduced T cells (UTD) were used as control. The parental CAR19 construct was included as a benchmark for full CAR functional activity. Cells were expanded for 10 days in the presence of anti-CD3/CD28 stimulation beads. 100U/mL IL-2 was added on day 7, bazedoxifene (Baz) was added on day 8 (samples CD19CAR+BAZ and FurON 106+BAZ) or omitted (samples CD19CAR and FurON 106). Cells were frozen on day 10. T cells were thawed and co-cultured with the luciferized target cell lines (CD19+ NALM6 cells or CD19- K562 cells) and bazedoxifene. CART cells were normalized to the same percentage of CAR expressing cells. After 4 days, the samples were analyzed by FACS and 3000 events in the counting beads gate were collected for all samples.

These data demonstrated that primary human T cells expressing ERαFurON CAR19 proliferate in the presence of CD19+ tumor cells, in a manner that is stabilizing compound-dependent and is noninferior to the parental CAR19 construct (FIG. 16).

### EXAMPLE 19: COMPOUND INDUCIBLE CAR EXPRESSION IN JURKAT T CELLS

Jurkat T cells were transduced with one of two furin degron domains fused to an anti-CD19 scFV CAR constructs. The two constructs share the same furin cleavage sequence but differ in the number of mutations in the degradation domain (ERmut1 or ERmut 2 shown in Table 23). The construct with more mutations was FurONCART19 #106 (with ERmut1); and the construct with fewer was FurONCART19 #130 (with ERmut2). The transduced cells were incubated with 4-OH tamoxifen, bazedoxifene, or raloxifene, at the indicated concentrations, for 42 hours. Expression of the anti-CD19 scFV was determined by FACS, using protein L.

These data demonstrated that the tested furin degron domains can regulate CAR19 expression in a stabilizing compound-dependent manner, regardless of the number of mutations; and no CAR surface expression is detected in the absence of compound (FIG. 17).

### EXAMPLE 20: COMPOUND INDUCIBLE CAR EXPRESSION IN HUMAN PRIMARY T CELLS

Primary human T cells were transduced with a furin degron domain fused to an anti-CD19 scFV CAR construct (construct #130). Untransduced T cells (UTDs) and the parental CAR19 were used as controls. Cells were expanded for 10 days in the presence of anti-CD3/CD28 stimulation beads. 100U/mL IL-2 was added on day 7 or omitted, bazedoxifene (BZA) was added on day 8. Prior to freezing down the cells, CAR expression was determined on day 10 by FACS, using protein L staining.

These data demonstrated that the FurON domain - comprising a limited number of mutations in the degron domain - regulates CAR19 expression in a stabilizing compound- and IL-2-dependent manner in human primary T cells (FIG. 18). Also, no surface CAR expression is detected in the absence of the stabilizing compound bazedoxifene, when the FurON moiety is fused to CAR19 (FIG. 18).

### EXAMPLE 21: COMPOUND-DEPENDENT FURON CAR-MEDIATED TUMOR KILLING

Primary human T cells were transduced with a furin degron domain fused to an anti-CD19 scFV CAR construct (construct #130). Untransduced T cells (UTDs) were used as control. The parental CAR19 construct was included as a benchmark for full CAR functional activity. Cells were expanded for 10 days in the presence of anti-CD3/CD28 stimulation beads. 100U/mL IL-2 was added on day 7. Bazedoxifene (BZA) was added on day 8 to a fraction of the FurON 130 BZA sample, so CAR expression can be determined on day 10 by FACS, using protein L staining, prior to freezing down the cells. T cells were thawed and co-cultured with the luciferized target cell lines (CD19+ NALM6 cells or CD19- K562 cells) for 20 hourrs, in the presence or absence of bazedoxifene. Different ratios of CART ("effector") and target cells are used as indicated in the x-axis of FIGs. 19A-19B. CART cells were normalized to the same percentage of CAR expressing cells. Percent tumor lysis was determined by analysis of remaining luciferase activity.

These data demonstrated that CART cells expressing FurON CAR19 kill CD19+ tumor cells, comprising a limited number of mutations in the degron domain, in a stabilizing compound- and target-dependent manner and is noninferior to the parental CAR construct (FIGs. 19A-19B).

### EXAMPLE 22: COMPOUND- AND TUMOR-DEPENDENT CYTOKINE SECRETION BY THE FURON CAR

Primary human T cells were transduced with a furin degron domain fused to an anti-CD19 scFV CAR construct (construct #130). Untransduced T cells (UTDs) were used as control. The parental CAR19 construct was included as a benchmark for full CAR functional activity. Cells were expanded for 10 days in the presence of anti-CD3/CD28 stimulation beads. 100U/mL IL-2 was added on day 7. Bazedoxifene (BZA) was added on day 8 to a fraction of the FurON 130 BZA sample, so CAR expression can be determined on day 10 by FACS, using protein L staining, prior to freezing down the cells. Cells were frozen on day 10. T cells were thawed and co-cultured with the luciferized target cell lines (CD19+ NALM6 cells) for 20 hours, at a 5:1 effector:target ratio, in the presence or absence of bazedoxifene. CART cells were normalized to the same percentage of CAR expressing cells. Supernatants were harvested and evaluated for levels of IFNγ.

These data demonstrated that CART cells expressing FurON CAR19, comprising a limited number of mutations in the degron domain, secrete cytokines in the presence of CD19+ tumor cells, in a manner that is stabilizing compound-dependent and is noninferior to the parental CAR construct (FIG. 20).

### EXAMPLE 23: COMPOUND- AND TUMOR-DEPENDENT T CELL PROLIFERATION

Primary human T cells were transduced with a furin degron domain fused to an anti-CD19 scFV CAR (construct #130). Untransduced T cells (UTD) were used as control. The parental CAR19 construct was included as a benchmark for full CAR functional activity. Cells were expanded for 10 days in the presence of anti-CD3/CD28 stimulation beads. 100U/mL IL-2 was added on day 7. Bazedoxifene was added on day 8 to a fraction of the FurON 130 BZA sample, so CAR expression can be determined on day 10 by FACS, using protein L staining, prior to freezing down the cells. Cells were frozen on day 10. T cells were thawed and co-cultured with the indicated luciferized target cell lines for 4 days, in the presence or absence of bazedoxifene. CART cells were normalized to the same percentage of CAR expressing cells. After 4 days, the samples were analyzed by FACS and 3000 events in the counting beads gate are collected for all samples.

These data demonstrated that CD3+ T cells comprising FurON CAR19 proliferate in the presence of CD19+ tumor cells, in a manner that is stabilizing compound-dependent and is noninferior to the parental CAR construct (FIG. 21).

### EXAMPLE 24: COMPOUND INDUCIBLE CAR123 EXPRESSION IN PRIMARY HUMAN T CELLS

Primary human T cells were transduced with a furin degron domain fused to an anti-CD123 scFV CAR construct. Untransduced T cells (UTD) and the parental CAR123 were used as controls. Cells were expanded for 10 days in the presence of anti-CD3/CD28 stimulation beads. 100U/mL IL-2 was added on day 7, bazedoxifene (BZA) was added on day 8 (Furon123+BZA) or omitted (Furon123). Prior to freezing down the cells, CAR expression was determined on day 10 by FACS using protein L staining.

These data demonstrated that the FurON domain regulates CAR123 expression in primary human T cells, in a stabilizing compound-dependent manner (FIG. 22).

### EXAMPLE 25: COMPOUND-DEPENDENT CAR123-MEDIATED TUMOR KILLING

Primary human T cells were transduced with a furin degron domain fused to an anti-CD123 scFV CAR construct. Untransduced T cells (UTD) were used as control. The parental CAR123 construct was included as a benchmark for full CAR functional activity. Cells were expanded for 10 days in the presence of anti-CD3/CD28 stimulation beads. 100U/mL IL-2 was added on day 7, bazedoxifene (BZA) was added on day 8 (FurON123+ BZA) or omitted (FurON123). Prior to freezing down the cells, CAR expression was determined on day 10 by FACS, using protein L staining. T cells were thawed and co-cultured with the luciferized target cell lines for 20 hours, in the presence or absence of bazedoxifene. Molm13 is a CD123+ cell line; very low expression of CD123+ observed in K562cells. Different ratios of CART and target cells were used as indicated in the x-axis (E:T ratios) of FIG. 25. CART cells were normalized to the same percentage of CAR expressing cells. Percent tumor lysis was determined by analysis of remaining luciferase activity.

These data demonstrated that CART cells expressing FurON CAR123 kill CD123+ tumor cells, in a stabilizing compound- and target-dependent manner and is noninferior to the parental CAR construct (FIG. 23).

### EXAMPLE 26: COMPOUND- AND TUMOR-DEPENDENT IFN-γ CYTOKINE SECRETION BY THE FURON CAR123

Primary human T cells were transduced with a furin degron domain fused to an anti-CD123 scFV CAR. Untransduced T cells (UTD) were used as control. The parental CAR123 construct was included as a benchmark for full CAR functional activity. Cells were expanded for 10 days in the presence of anti-CD3/CD28 stimulation beads. 100U/mL IL-2 was added on day 7, bazedoxifene (BZA) was added on day 8 (FurON123+ BZA) or omitted (FurON123). Cells were frozen on day 10. T cells were thawed and co-cultured with the luciferized target cell lines for 20hrs at a 5:1 effector to target ratio, in the presence or absence of bazedoxifene. Molm13 is a CD123+ cell line; very low expression of CD123+ observed in K562cells. Supernatants were harvested and evaluated for levels of IFNγ.

These data demonstrated that CART cells expressing FurON CAR123 secrete cytokines in the presence of CD123+ tumor cells, in a manner that is stabilizing compound-dependent and is noninferior to the parental CAR construct (FIG. 24).

### EXAMPLE 27: FURON CART19 IS EFFECTIVE AGAINST CD19+ TUMOR CELLS IN AN ANIMAL MODEL ONLY WHEN BAZEDOXIFENE IS DOSED

### Cell line

NALM6 (RRID: CVCL_0092) is a human leukemia cell line that was derived from the peripheral blood of a 19-year-old man with acute lymphoblastic leukemia (ALL) in relapse in 1976. Cells were grown in RMPI medium containing 10% fetal bovine serum. This cell line grows in suspension in tissue culture flasks. This cell line persists and expands in mice when implanted intravenously. The NALM6 cells have been modified to express luciferase, so that that tumor cell growth can also be monitored by imaging the mice.

### Mice

6 week old NSG (NOD.Cg-*Prkdc^{scid}Il2rg^{tm1Wjl}*/SzJ) mice were received from the Jackson Laboratory. Animals were allowed to acclimate in the NIBR animal facility for at least 3 days prior to experimentation. Animals were handled in accordance with Novartis ACUC regulations and guidelines. Electronic transponders for animal identification were implanted on the left flank one day prior to tumor implantation.

### Tumor implantation

NALM6 cells in logarithmic growth phase were harvested and washed in 50ml falcon tubes at 1200rpm for 5 minutes, once in growth media and then two times in cold sterile PBS. The cells were resuspended in PBS at a concentration of 5×10⁶ per ml, placed on ice, and immediately injected in mice. Tumor cells were injected intravenously in 200ul through the caudal vein. The NALM6 model endogenously expresses CD19 and thus, can be used to test the in vivo efficacy of CD19-directed CAR T cells. This model grows well when implanted intravenously in mice and can be imaged for tumor burden measurements.

### Preparation of CART19 and FurON CART19 cells for the in vivo study

CART cells were generated by starting with blood from healthy apheresed donors whose naive T cells were obtained by negative selection for T cells, CD4+ and CD8+ lymphocytes. These cells were activated by the addition of CD3/CD28 beads (Dynabeads^{®} Human T-Expander CD3/CD28, Thermo Fisher Scientific) at a ratio of 1:3 (T cell to bead) in T cell medium (RPMI1640, 10% heat-inactivated fetal calf serum (FCS), 2 mM L-glutamine, 1x Penicillin/Streptomycin, 100 µM non-essential amino acids, 1 mM Sodium Pyruvate, 10 mM Hepes, and 55 µM 2-mercaptoethanol) at 37°C, 5% CO2. T cells were cultured at 4×10⁶ T cells (UTDs) or 5×10⁶ (CART19 and FurON CART 19) in 1 mL medium per well of a 24-well plate. After 24 hours, when T cells were blasting, 0.5 mL of non-concentrated, or smaller volumes of concentrated viral supernatant were added; T cells were transduced at a multiplicity of infection (MOI) of 5. T cells began to divide in a logarithmic growth pattern, which is monitored by measuring the cell counts per mL, and T cells are diluted in fresh medium every two days. On day 7, 100U/ml of IL2 (Peprotech, Rocky Hill, NJ) and 1uM bazedoxifene is added to the cultures. On day 9, 1uM bazedoxifene is added. On day 10 the cells are collected and the percentage of cells transduced (expressing the CD19-specific CAR on the cell surface) was determined by flow cytometry analysis on a FACS Fortessa (BD). The viral transduction shows similar transduction efficiency; the surface expression of the FurON CAR19 (mean fluorescence intensity, MFI) is lower than the observed for CART 19. All CAR T cells were produced in research grade (i.e., not clinical grade) manufacturing conditions.

### Treatment with CART19 or FurON CART19 cells

CD19+ NALM6 tumor cells expressing luciferase, were infused into NSG mice. Seven days later, the tumor burden was measured by whole body bioluminescence, the animals were randomized into treatment groups (5 mice per group), and infused with PBS (vehicle), CART19 (1×10⁶ or 5×10⁶ CAR+ T cells), FurON CART19 cells (5×10⁶ cells CAR+ T cells), or untransduced T cells (UTD), intravenously via the lateral tail vein. All animals received the same dose of total T cells (16.4×10⁶/ mouse). Some treatment groups were dosed with bazedoxifene (BZA), bazedoxifene plus interleukin 2 (BZA/IL2), or vehicle, as indicated by the line. BZA was dosed for 10 days (single dose 200mg/kg; p.o). IL2 was dosed for 5 days, rested for two, and dosed again for 3 days (single dose 18×10^6 IU/kg; i.p.). Tumor growth and animal health were monitored over time. The mean bioluminescence for all treatment groups is plotted in FIG. 27; the arrow indicates when administration of BZA and IL2 was discontinued.

### Results

As shown in FIG. 47, while the tumor progressed in the negative control mice and mice treated with FurON CART19 only, tumor growth was inhibited in mice infused with CART 19, or infused with FurON CART19 and treated with BZA alone or both BZA and IL2. Once dosing of bazedoxifene and IL2 is discontinued, only the constitutively active CART19 at the highest dose was able to control tumor growth. BZA was previously shown to have no effect on the growth of NALM6 tumor cells at the same dose and dose regimen (data unshown). Thus, FurON CART19 in the presence of bazedoxifene shows comparable tumor inhibitory activity as the positive control CART19.

### EXAMPLE 28: ADDITIONAL FURON CAR SEQUENCES

**Table 24. Sequences of additional FurON CAR constructs and components thereof**

| | **Amino acid sequence** | **DNA sequence** |
|---|---|---|
| Leader sequence/ Signal peptide (SP) | MALPVTALLLPLALLLHAARP (SEQ ID NO: 139) | atggccctccctgtcaccgccctgctgcttccgctggctcttc tgctccacgccgctcggccc (SEQ ID NO: 140) |
| SP-Linker 2-ERmut1-Furin cleavage site 2 - CD19 CAR2 | MALPVTALLLPLALLLHAARPS LALSLTADQMVSALLDAEPPIL YSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 990) | atggccctccctgtcaccgccctgctgcttccgctggctcttc tgctccacgccgctcggccctcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg ctcctggaaatgctggacgcgcacagactcggaaccggc gcggaagacccccggccctccaggaagcgaaggtccct cggagacgtgggtgaaattgtgatgacccagtcacccgc cactcttagcctttcacccggtgagcgcgcaaccctgtcttg cagagcctcccaagacatctcaaaataccttaattggtatc aacagaagcccggacaggctcctcgccttctgatctacca caccagccggctccattctggaatccctgccaggttcagc ggtagcggatctgggaccgactacaccctcactatcagct cactgcagccagaggacttcgctgtctatttctgtcagcaa gggaacaccctgccctacacctttggacagggcaccaag ctcgagattaaaggtggaggtggcagcggaggaggtgg gtccggcggtggaggaagccaggtccaactccaagaaa gcggaccgggtcttgtgaagccatcagaaactctttcactg acttgtactgtgagcggagtgtctctccccgattacggggtg tcttggatcagacagccaccggggaagggtctggaatgg attggagtgatttggggctctgagactacttactactcttcatc cctcaagtcacgcgtcaccatctcaaaggacaactctaag aatcaggtgtcactgaaactgtcatctgtgaccgcagccg acaccgccgtgtactattgcgctaagcattactattatggcg ggagctacgcaatggattactggggacagggtactctggt caccgtgtccagcaccactaccccagcaccgaggccac ccaccccggctcctaccatcgcctcccagcctctgtccctg cgtccggaggcatgtagacccgcagctggtggggccgtg |
| | | catacccggggtcttgacttcgcctgcgatatctacatttgg gcccctctggctggtacttgcggggtcctgctgctttcactcg tgatcactctttactgtaagcgcggtcggaagaagctgctgt acatctttaagcaacccttcatgaggcctgtgcagactactc aagaggaggacggctgttcatgccggttcccagaggagg aggaaggcggctgcgaactgcgcgtgaaattcagccgc agcgcagatgctccagcctaccagcaggggcagaacca gctctacaacgaactcaatcttggtcggagagaggagtac gacgtgctggacaagcggagaggacgggacccagaaa tgggcgggaagccgcgcagaaagaatccccaagaggg cctgtacaacgagctccaaaaggataagatggcagaag cctatagcgagattggtatgaaaggggaacgcagaaga ggcaaaggccacgacggactgtaccagggactcagca ccgccaccaaggacacctatgacgctcttcacatgcagg ccctgccgcctcgg (SEQ ID NO: 1050) |
| SP-Linker 2-ERmut2-Furin cleavage site 2 - CD19 CAR2 | MALPVTALLLPLALLLHAARPS LALSLTADQMVSALLDAEPPIL YSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 991) | atggccctccctgtcaccgccctgctgcttccgctggctcttc tgctccacgccgctcggccctcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta cagcatgaagtgcaagaacgtcgtgcctctgtccgatctg ctcctggaaatgctggacgcgcacagactcggaaccggc gcggaagacccccggccctccaggaagcgaaggtccct cggagacgtgggtgaaattgtgatgacccagtcacccgc cactcttagcctttcacccggtgagcgcgcaaccctgtcttg cagagcctcccaagacatctcaaaataccttaattggtatc aacagaagcccggacaggctcctcgccttctgatctacca caccagccggctccattctggaatccctgccaggttcagc ggtagcggatctgggaccgactacaccctcactatcagct cactgcagccagaggacttcgctgtctatttctgtcagcaa gggaacaccctgccctacacctttggacagggcaccaag ctcgagattaaaggtggaggtggcagcggaggaggtgg gtccggcggtggaggaagccaggtccaactccaagaaa gcggaccgggtcttgtgaagccatcagaaactctttcactg acttgtactgtgagcggagtgtctctccccgattacggggtg tcttggatcagacagccaccggggaagggtctggaatgg attggagtgatttggggctctgagactacttactactcttcatc cctcaagtcacgcgtcaccatctcaaaggacaactctaag aatcaggtgtcactgaaactgtcatctgtgaccgcagccg acaccgccgtgtactattgcgctaagcattactattatggcg ggagctacgcaatggattactggggacagggtactctggt caccgtgtccagcaccactaccccagcaccgaggccac ccaccccggctcctaccatcgcctcccagcctctgtccctg |
| | | cgtccggaggcatgtagacccgcagctggtggggccgtg catacccggggtcttgacttcgcctgcgatatctacatttgg gcccctctggctggtacttgcggggtcctgctgctttcactcg tgatcactctttactgtaagcgcggtcggaagaagctgctgt acatctttaagcaacccttcatgaggcctgtgcagactactc aagaggaggacggctgttcatgccggttcccagaggagg aggaaggcggctgcgaactgcgcgtgaaattcagccgc agcgcagatgctccagcctaccagcaggggcagaacca gctctacaacgaactcaatcttggtcggagagaggagtac gacgtgctggacaagcggagaggacgggacccagaaa tgggcgggaagccgcgcagaaagaatccccaagaggg cctgtacaacgagctccaaaaggataagatggcagaag cctatagcgagattggtatgaaaggggaacgcagaaga ggcaaaggccacgacggactgtaccagggactcagca ccgccaccaaggacacctatgacgctcttcacatgcagg ccctgccgcctcgg (SEQ ID NO: 1051) |
| SP-Linker 2 - ERmut1 - Furin cleavage site 3 - CD19 CAR2 | MALPVTALLLPLALLLHAARPS LALSLTADQMVSALLDAEPPIL YSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 992) | atggccctccctgtcaccgccctgctgcttccgctggctcttc tgctccacgccgctcggccctcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg ctcctggaaatgctggacgcgcacagactcgggacggg agctgaagatccacgacccagcagaaagcgacgggaa attgtgatgacccagtcacccgccactcttagcctttcaccc ggtgagcgcgcaaccctgtcttgcagagcctcccaagac atctcaaaataccttaattggtatcaacagaagcccggac aggctcctcgccttctgatctaccacaccagccggctccatt ctggaatccctgccaggttcagcggtagcggatctgggac cgactacaccctcactatcagctcactgcagccagagga cttcgctgtctatttctgtcagcaagggaacaccctgcccta cacctttggacagggcaccaagctcgagattaaaggtgg aggtggcagcggaggaggtgggtccggcggtggagga agccaggtccaactccaagaaagcggaccgggtcttgtg aagccatcagaaactctttcactgacttgtactgtgagcgg agtgtctctccccgattacggggtgtcttggatcagacagc caccggggaagggtctggaatggattggagtgatttgggg ctctgagactacttactactcttcatccctcaagtcacgcgtc accatctcaaaggacaactctaagaatcaggtgtcactga aactgtcatctgtgaccgcagccgacaccgccgtgtactat tgcgctaagcattactattatggcgggagctacgcaatgga ttactggggacagggtactctggtcaccgtgtccagcacc |
| | | actaccccagcaccgaggccacccaccccggctcctacc atcgcctcccagcctctgtccctgcgtccggaggcatgtag acccgcagctggtggggccgtgcatacccggggtcttgac ttcgcctgcgatatctacatttgggcccctctggctggtacttg cggggtcctgctgctttcactcgtgatcactctttactgtaagc gcggtcggaagaagctgctgtacatctttaagcaacccttc atgaggcctgtgcagactactcaagaggaggacggctgtt catgccggttcccagaggaggaggaaggcggctgcgaa ctgcgcgtgaaattcagccgcagcgcagatgctccagcct accagcaggggcagaaccagctctacaacgaactcaat cttggtcggagagaggagtacgacgtgctggacaagcg gagaggacgggacccagaaatgggcgggaagccgcg cagaaagaatccccaagagggcctgtacaacgagctcc aaaaggataagatggcagaagcctatagcgagattggta tgaaaggggaacgcagaagaggcaaaggccacgacg gactgtaccagggactcagcaccgccaccaaggacacc tatgacgctcttcacatgcaggccctgccgcctcgg (SEQ ID NO: 1052) |
| SP-Linker 2 - ERmut2 - Furin cleavage site 3 - CD19 CAR2 | MALPVTALLLPLALLLHAARPS LALSLTADQMVSALLDAEPPIL YSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 993) | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta cagcatgaagtgcaagaacgtcgtgcctctgtccgatctg ctcctggaaatgctggacgcgcacagactcGggacggg agctgaagatccacgacccagcagaaagcgacgggaa attgtgatgacccagtcacccgccactcttagcctttcaccc ggtgagcgcgcaaccctgtcttgcagagcctcccaagac atctcaaaataccttaattggtatcaacagaagcccggac aggctcctcgccttctgatctaccacaccagccggctccatt ctggaatccctgccaggttcagcggtagcggatctgggac cgactacaccctcactatcagctcactgcagccagagga cttcgctgtctatttctgtcagcaagggaacaccctgcccta cacctttggacagggcaccaagctcgagattaaaggtgg aggtggcagcggaggaggtgggtccggcggtggagga agccaggtccaactccaagaaagcggaccgggtcttgtg aagccatcagaaactctttcactgacttgtactgtgagcgg agtgtctctccccgattacggggtgtcttggatcagacagc caccggggaagggtctggaatggattggagtgatttgggg ctctgagactacttactactcttcatccctcaagtcacgcgtc accatctcaaaggacaactctaagaatcaggtgtcactga aactgtcatctgtgaccgcagccgacaccgccgtgtactat tgcgctaagcattactattatggcgggagctacgcaatgga |
| | | ttactggggacagggtactctggtcaccgtgtccagcacc actaccccagcaccgaggccacccaccccggctcctacc atcgcctcccagcctctgtccctgcgtccggaggcatgtag acccgcagctggtggggccgtgcatacccggggtcttgac ttcgcctgcgatatctacatttgggcccctctggctggtacttg cggggtcctgctgctttcactcgtgatcactctttactgtaagc gcggtcggaagaagctgctgtacatctttaagcaacccttc atgaggcctgtgcagactactcaagaggaggacggctgtt catgccggttcccagaggaggaggaaggcggctgcgaa ctgcgcgtgaaattcagccgcagcgcagatgctccagcct accagcaggggcagaaccagctctacaacgaactcaat cttggtcggagagaggagtacgacgtgctggacaagcg gagaggacgggacccagaaatgggcgggaagccgcg cagaaagaatccccaagagggcctgtacaacgagctcc aaaaggataagatggcagaagcctatagcgagattggta tgaaaggggaacgcagaagaggcaaaggccacgacg gactgtaccagggactcagcaccgccaccaaggacacc tatgacgctcttcacatgcaggccctgccgcctcgg (SEQ ID NO: 1053) |
| SP-Linker 2 - ERmut1 - Furin cleavage site 2 - CD123 CAR | MALPVTALLLPLALLLHAARPS LALSLTADQMVSALLDAEPPIL YSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 994) | atggccctccctgtcaccgccctgctgcttccgctggctcttc tgctccacgccgctcggccctcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg ctcctggaaatgctggacgcgcacagactcggaaccggc gcggaagacccccggccctccaggaagcgaaggtccct cggagacgtgggtcaagtgcaactcgtccaaagcggag cggaagtcaagaaacccggagcgagcgtgaaagtgtcc tgcaaagcctccggctacacctttacgggctactacatgca ctgggtgcgccaggcaccaggacagggtcttgaatggat gggatggatcaaccctaattcgggcggaactaactacgc acagaagttccaggggagagtgactctgactcgggatac ctccatctcaactgtctacatggaactctcccgcttgcggtc agatgatacggcagtgtactactgcgcccgcgacatgaat atcctggctaccgtgccgttcgacatctggggacagggga ctatggttactgtctcatcgggcggtggaggttcaggagga ggcggctcgggaggcggaggttcggacattcagatgacc cagtccccatcctctctgtcggccagcgtcggagatagggt gaccattacctgtcgggcctcgcaaagcatctcctcgtacc tcaactggtatcagcaaaagccgggaaaggcgcctaag ctgctgatctacgccgcttcgagcttgcaaagcggggtgcc atccagattctcgggatcaggctcaggaaccgacttcacc |
| | | ctgaccgtgaacagcctccagccggaggactttgccactt actactgccagcagggagactccgtgccgcttactttcggg gggggtacccgcctggagatcaagaccactaccccagc accgaggccacccaccccggctcctaccatcgcctccca gcctctgtccctgcgtccggaggcatgtagacccgcagct ggtggggccgtgcatacccggggtcttgacttcgcctgcg atatctacatttgggcccctctggctggtacttgcggggtcct gctgctttcactcgtgatcactctttactgtaagcgcggtcgg aagaagctgctgtacatctttaagcaacccttcatgaggcc tgtgcagactactcaagaggaggacggctgttcatgccgg ttcccagaggaggaggaaggcggctgcgaactgcgcgt gaaattcagccgcagcgcagatgctccagcctaccagca ggggcagaaccagctctacaacgaactcaatcttggtcg gagagaggagtacgacgtgctggacaagcggagagga cgggacccagaaatgggcgggaagccgcgcagaaag aatccccaagagggcctgtacaacgagctccaaaagga taagatggcagaagcctatagcgagattggtatgaaagg ggaacgcagaagaggcaaaggccacgacggactgtac cagggactcagcaccgccaccaaggacacctatgacgc tcttcacatgcaggccctgccgcctcgg (SEQ ID NO: 1054) |
| SP-Linker 2 - ERmut2 - Furin cleavage site 2 - CD123 CAR | MALPVTALLLPLALLLHAARPS LALSLTADQMVSALLDAEPPIL YSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta cagcatgaagtgcaagaacgtcgtgcctctgtccgatctg ctcctggaaatgctggacgcgcacagactcggaaccggc gcggaagacccccggccctccaggaagcgaaggtccct cggagacgtgggtcaagtgcaactcgtccaaagcggag cggaagtcaagaaacccggagcgagcgtgaaagtgtcc tgcaaagcctccggctacacctttacgggctactacatgca ctgggtgcgccaggcaccaggacagggtcttgaatggat gggatggatcaaccctaattcgggcggaactaactacgc acagaagttccaggggagagtgactctgactcgggatac ctccatctcaactgtctacatggaactctcccgcttgcggtc agatgatacggcagtgtactactgcgcccgcgacatgaat atcctggctaccgtgccgttcgacatctggggacagggga ctatggttactgtctcatcgggcggtggaggttcaggagga ggcggctcgggaggcggaggttcggacattcagatgacc cagtccccatcctctctgtcggccagcgtcggagatagggt gaccattacctgtcgggcctcgcaaagcatctcctcgtacc tcaactggtatcagcaaaagccgggaaaggcgcctaag |
| | GHDGLYQGLSTATKDTYDAL HMQALPPR (SEQ ID NO: 995) | ctgctgatctacgccgcttcgagcttgcaaagcggggtgcc atccagattctcgggatcaggctcaggaaccgacttcacc ctgaccgtgaacagcctccagccggaggactttgccactt actactgccagcagggagactccgtgccgcttactttcggg gggggtacccgcctggagatcaagaccactaccccagc accgaggccacccaccccggctcctaccatcgcctccca gcctctgtccctgcgtccggaggcatgtagacccgcagct ggtggggccgtgcatacccggggtcttgacttcgcctgcg atatctacatttgggcccctctggctggtacttgcggggtcct gctgctttcactcgtgatcactctttactgtaagcgcggtcgg aagaagctgctgtacatctttaagcaacccttcatgaggcc tgtgcagactactcaagaggaggacggctgttcatgccgg ttcccagaggaggaggaaggcggctgcgaactgcgcgt gaaattcagccgcagcgcagatgctccagcctaccagca ggggcagaaccagctctacaacgaactcaatcttggtcg gagagaggagtacgacgtgctggacaagcggagagga cgggacccagaaatgggcgggaagccgcgcagaaag aatccccaagagggcctgtacaacgagctccaaaagga taagatggcagaagcctatagcgagattggtatgaaagg ggaacgcagaagaggcaaaggccacgacggactgtac cagggactcagcaccgccaccaaggacacctatgacgc tcttcacatgcaggccctgccgcctcgg (SEQ ID NO: 1055) |
| SP-Linker 2 - ERmut1 - Furin cleavage site 3 - CD123 CAR | MALPVTALLLPLALLLHAARPS LALSLTADQMVSALLDAEPPIL YSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTR GLDFACDIYIWAPLAGTCG VLLLSLVITLYCKRGRKKLL YIFKQPFMRPVQTTQEEDG CSCRFPEEEEGGCELRVK FSRSADAPAYQQGQNQLY | atggccctccctgtcaccgccctgctgcttccgctggctcttc tgctccacgccgctcggccctcgttggcactttccctgactg ccgaccagatggtgtccgcccttctggacgccgagcctcca attctgtactcggagtacgatccgactcgcccgttctccgaa gccagcatgatgggcctgttgactaacctggcggaccgcg agttggtgcacatgattaactgggctaagcgggtgccgggc ttcgtggacctggccctgcacgaccaagtgcacctcctgga atgcgcctggatggaaatcctcatgatcggcctcgtgtgga gatccatggagcatcccggaaagctcctgtttgcacccaac ctcctgcttgatcgcaaccagggaaaatgcgtggaagggg gtgtcgagattttcgacatgctgctcgccacctcttcccggtt ccggatgatgaatctgcagggagaagagttcgtgtgtctga agtcaatcatcctgctgaactccggggtctataccttcctga gctcgaccctcaagtcactggaggaaaaagaccacatcca tcgcgtgctcgataagatcaccgacacccttatccatctcat ggcgaaggctggactgaccctgcaacagcagcaccagag gctggcccagttgctgctgattctgagccacatccggcacat gtcgtccaagaggatggaacacctgtacagcatgaagtgc aagaacgtcgtgcctctgtccgatctgctcctggaaatgctg gacgcgcacagactcggaaccggcgcggaagacccccgg ccctccaggaagcgaaggcaagtgcaactcgtccaaagcg gagcggaagtcaagaaacccggagcgagcgtgaaagtgt cctgcaaagcctccggctacacctttacgggctactacatg cactgggtgcgccaggcaccaggacagggtcttgaatgga tgggatggatcaaccctaattcgggcggaactaactacgca cagaagttccaggggagagtgactctgactcgggatacctc catctcaactgtctacatggaactctcccgcttgcggtcaga tgatacggcagtgtactactgcgcccgcgacatgaatatcc |
| | NELNLGRREEYDVLDKRR GRDPEMGGKPRRKNPQE GLYNELQKDKMAEAYSEIG MKGERRRGKGHDGLYQGL STATKDTYDALHMQALPPR (SEQ ID NO: 996) | tggctaccgtgccgttcgacatctggggacaggggactatg gttactgtctcatcgggcggtggaggttcaggaggaggcgg ctcgggaggcggaggttcggacattcagatgacccagtcc ccatcctctctgtcggccagcgtcggagatagggtgaccat tacctgtcgggcctcgcaaagcatctcctcgtacctcaactg gtatcagcaaaagccgggaaaggcgcctaagctgctgatc tacgccgcttcgagcttgcaaagcggggtgccatccagatt ctcgggatcaggctcaggaaccgacttcaccctgaccgtga acagcctccagccggaggactttgccacttactactgccag cagggagactccgtgccgcttactttcggggggggtacccg cctggagatcaagaccactaccccagcaccgaggccaccc accccggctcctaccatcgcctcccagcctctgtccctgcgt ccggaggcatgtagacccgcagctggtggggccgtgcata cccggggtcttgacttcgcctgcgatatctacatttgggccc ctctggctggtacttgcggggtcctgctgctttcactcgtgat cactctttactgtaagcgcggtcggaagaagctgctgtaca tctttaagcaacccttcatgaggcctgtgcagactactcaag aggaggacggctgttcatgccggttcccagaggaggagga aggcggctgcgaactgcgcgtgaaattcagccgcagcgca gatgctccagcctaccagcaggggcagaaccagctctaca acgaactcaatcttggtcggagagaggagtacgacgtgct ggacaagcggagaggacgggacccagaaatgggcggga agccgcgcagaaagaatccccaagagggcctgtacaacg agctccaaaaggataagatggcagaagcctatagcgagat tggtatgaaaggggaacgcagaagaggcaaaggccacga cggactgtaccagggactcagcaccgccaccaaggacacc tatgacgctcttcacatgcaggccctgccgcctcgg (SEQ ID NO: 1056) |
| SP-Linker 2 - ERmut2 - Furin cleavage site 3 - CD123 CAR | MALPVTALLLPLALLLHAARPS LALSLTADQMVSALLDAEPPIL YSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta cagcatgaagtgcaagaacgtcgtgcctctgtccgatctg ctcctggaaatgctggacgcgcacagactcgggacggg agctgaagatccacgacccagcagaaagcgacggcaa gtgcaactcgtccaaagcggagcggaagtcaagaaacc |
| | KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 997) | cggagcgagcgtgaaagtgtcctgcaaagcctccggcta cacctttacgggctactacatgcactgggtgcgccaggca ccaggacagggtcttgaatggatgggatggatcaacccta attcgggcggaactaactacgcacagaagttccagggga gagtgactctgactcgggatacctccatctcaactgtctaca tggaactctcccgcttgcggtcagatgatacggcagtgtac tactgcgcccgcgacatgaatatcctggctaccgtgccgtt cgacatctggggacaggggactatggttactgtctcatcgg gcggtggaggttcaggaggaggcggctcgggaggcgg aggttcggacattcagatgacccagtccccatcctctctgtc ggccagcgtcggagatagggtgaccattacctgtcgggc ctcgcaaagcatctcctcgtacctcaactggtatcagcaa aagccgggaaaggcgcctaagctgctgatctacgccgcttc gagcttgcaaagcggggtgccatccagattctcgggatca ggctcaggaaccgacttcaccctgaccgtgaacagcctc cagccggaggactttgccacttactactgccagcaggga gactccgtgccgcttactttcggggggggtacccgcctgga gatcaagaccactaccccagcaccgaggccacccaccc cggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctaccagcaggggcagaaccagctcta caacgaactcaatcttggtcggagagaggagtacgacgt gctggacaagcggagaggacgggacccagaaatgggc gggaagccgcgcagaaagaatccccaagagggcctgt acaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 1057) atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg ctcctggaaatgctggacgcgcacagactcggaaccggc gcggaagacccccggccctccaggaagcgaaggtccct |
| SP-Linker 2 - ERmut1 - Furin cleavage site 2-BCMA CAR | MALPVTALLLPLALLLHAARPS LALSLTADQMVSALLDAEPPIL YSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg ctcctggaaatgctggacgcgcacagactcggaaccggc gcggaagacccccggccctccaggaagcgaaggtccct |
| | TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 998) | cggagacgtgggtgaagtgcaattggtggaatcaggggg aggacttgtgcagcctggaggatcgctgagactgtcatgtg ccgtgtccggctttgccctgtccaaccacgggatgtcctgg gtccgccgcgcgcctggaaagggcctcgaatgggtgtcg ggtattgtgtacagcggtagcacctactatgccgcatccgt gaaggggagattcaccatcagccgggacaactccagga acactctgtacctccaaatgaattcgctgaggccagagga cactgccatctactactgctccgcgcatggcggagagtcc gacgtctggggacaggggaccaccgtgaccgtgtctagc gcgtccggcggaggcggcagcgggggtcgggcatcag ggggcggcggatcggacatccagctcacccagtccccg agctcgctgtccgcctccgtgggagatcgggtcaccatca cgtgccgcgccagccagtcgatttcctcctacctgaactgg taccaacagaagcccggaaaagccccgaagcttctcatc tacgccgcctcgagcctgcagtcaggagtgccctcacggt tctccggctccggttccggtactgatttcaccctgaccatttcc tccctgcaaccggaggacttcgctacttactactgccagca gtcgtactccaccccctacactttcggacaaggcaccaag gtcgaaatcaagaccactaccccagcaccgaggccacc caccccggctcctaccatcgcctcccagcctctgtccctgc gtccggaggcatgtagacccgcagctggtggggccgtgc atacccggggtcttgacttcgcctgcgatatctacatttggg cccctctggctggtacttgcggggtcctgctgctttcactcgt gatcactctttactgtaagcgcggtcggaagaagctgctgt acatctttaagcaacccttcatgaggcctgtgcagactactc aagaggaggacggctgttcatgccggttcccagaggagg aggaaggcggctgcgaactgcgcgtgaaattcagccgc agcgcagatgctccagcctaccagcaggggcagaacca gctctacaacgaactcaatcttggtcggagagaggagtac gacgtgctggacaagcggagaggacgggacccagaaa tgggcgggaagccgcgcagaaagaatccccaagaggg cctgtacaacgagctccaaaaggataagatggcagaag cctatagcgagattggtatgaaaggggaacgcagaaga ggcaaaggccacgacggactgtaccagggactcagca ccgccaccaaggacacctatgacgctcttcacatgcagg ccctgccgcctcgg (SEQ ID NO: 1058) atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta cagcatgaagtgcaagaacgtcgtgcctctgtccgatctg ctcctggaaatgctggacgcgcacagactcggaaccggc |
| SP-Linker 2 - ERmut2 - Furin cleavage site 2 - BCMA CAR | MALPVTALLLPLALLLHAARPS LALSLTADQMVSALLDAEPPIL YSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta cagcatgaagtgcaagaacgtcgtgcctctgtccgatctg ctcctggaaatgctggacgcgcacagactcggaaccggc |
| | SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 999) | gcggaagacccccggccctccaggaagcgaaggtccct cggagacgtgggtgaagtgcaattggtggaatcaggggg aggacttgtgcagcctggaggatcgctgagactgtcatgtg ccgtgtccggctttgccctgtccaaccacgggatgtcctgg gtccgccgcgcgcctggaaagggcctcgaatgggtgtcg ggtattgtgtacagcggtagcacctactatgccgcatccgt gaaggggagattcaccatcagccgggacaactccagga acactctgtacctccaaatgaattcgctgaggccagagga cactgccatctactactgctccgcgcatggcggagagtcc gacgtctggggacaggggaccaccgtgaccgtgtctagc gcgtccggcggaggcggcagcgggggtcgggcatcag ggggcggcggatcggacatccagctcacccagtccccg agctcgctgtccgcctccgtgggagatcgggtcaccatca cgtgccgcgccagccagtcgatttcctcctacctgaactgg taccaacagaagcccggaaaagccccgaagcttctcatc tacgccgcctcgagcctgcagtcaggagtgccctcacggt tctccggctccggttccggtactgatttcaccctgaccatttcc tccctgcaaccggaggacttcgctacttactactgccagca gtcgtactccaccccctacactttcggacaaggcaccaag gtcgaaatcaagaccactaccccagcaccgaggccacc caccccggctcctaccatcgcctcccagcctctgtccctgc gtccggaggcatgtagacccgcagctggtggggccgtgc atacccggggtcttgacttcgcctgcgatatctacatttggg cccctctggctggtacttgcggggtcctgctgctttcactcgt gatcactctttactgtaagcgcggtcggaagaagctgctgt acatctttaagcaacccttcatgaggcctgtgcagactactc aagaggaggacggctgttcatgccggttcccagaggagg aggaaggcggctgcgaactgcgcgtgaaattcagccgc agcgcagatgctccagcctaccagcaggggcagaacca gctctacaacgaactcaatcttggtcggagagaggagtac gacgtgctggacaagcggagaggacgggacccagaaa tgggcgggaagccgcgcagaaagaatccccaagaggg cctgtacaacgagctccaaaaggataagatggcagaag cctatagcgagattggtatgaaaggggaacgcagaaga ggcaaaggccacgacggactgtaccagggactcagca ccgccaccaaggacacctatgacgctcttcacatgcagg ccctgccgcctcgg (SEQ ID NO: 1059) atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg |
| SP-Linker 2 - ERmut1 - Furin cleavage site 3-BCMA CAR | MALPVTALLLPLALLLHAARPS LALSLTADQMVSALLDAEPPIL YSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctggccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgtccaagaggatggaacacctgta cagcatgaagtgcaagaacgtcgtgcctctgtccgatctg |
| | GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1000) | ctcctggaaatgctggacgcgcacagactcGggacggg agctgaagatccacgacccagcagaaagcgacgggaa gtgcaattggtggaatcagggggaggacttgtgcagcctg gaggatcgctgagactgtcatgtgccgtgtccggctttgcc ctgtccaaccacgggatgtcctgggtccgccgcgcgcctg gaaagggcctcgaatgggtgtcgggtattgtgtacagcgg tagcacctactatgccgcatccgtgaaggggagattcacc atcagccgggacaactccaggaacactctgtacctccaa atgaattcgctgaggccagaggacactgccatctactact gctccgcgcatggcggagagtccgacgtctggggacag gggaccaccgtgaccgtgtctagcgcgtccggcggaggc ggcagcgggggtcgggcatcagggggcggcggatcgg acatccagctcacccagtccccgagctcgctgtccgcctc cgtgggagatcgggtcaccatcacgtgccgcgccagcca gtcgatttcctcctacctgaactggtaccaacagaagcccg gaaaagccccgaagcttctcatctacgccgcctcgagcct gcagtcaggagtgccctcacggttctccggctccggttccg gtactgatttcaccctgaccatttcctccctgcaaccggagg acttcgctacttactactgccagcagtcgtactccaccccct acactttcggacaaggcaccaaggtcgaaatcaagacc actaccccagcaccgaggccacccaccccggctcctacc atcgcctcccagcctctgtccctgcgtccggaggcatgtag acccgcagctggtggggccgtgcatacccggggtcttgac ttcgcctgcgatatctacatttgggcccctctggctggtacttg cggggtcctgctgctttcactcgtgatcactctttactgtaagc gcggtcggaagaagctgctgtacatctttaagcaacccttc atgaggcctgtgcagactactcaagaggaggacggctgtt catgccggttcccagaggaggaggaaggcggctgcgaa ctgcgcgtgaaattcagccgcagcgcagatgctccagcct accagcaggggcagaaccagctctacaacgaactcaat cttggtcggagagaggagtacgacgtgctggacaagcg gagaggacgggacccagaaatgggcgggaagccgcg cagaaagaatccccaagagggcctgtacaacgagctcc aaaaggataagatggcagaagcctatagcgagattggta tgaaaggggaacgcagaagaggcaaaggccacgacg gactgtaccagggactcagcaccgccaccaaggacacc tatgacgctcttcacatgcaggccctgccgcctcgg (SEQ ID NO: 1060) |
| SP-Linker 2 - ERmut2 - Furin cleavage site 3 - BCMA CAR | MALPVTALLLPLALLLHAARPS LALSLTADQMVSALLDAEPPIL YSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA | atggccctccctgtcaccgccctgctgcttccgctggctcttc tgctccacgccgctcggcccTcgttggcactttccctgactg ccgaccagatggtgtccgcccttctggacgccgagcctcc aattctgtactcggagtacgatccgactcgcccgttctccga agccagcatgatgggcctgttgactaacctggcggaccg cgagttggtgcacatgattaactgggctaagcgggtgccg ggcttcgtggacctgaccctgcacgaccaagtgcacctcc tggaatgcgcctggatggaaatcctcatgatcggcctcgtg tggagatccatggagcatcccggaaagctcctgtttgcac ccaacctcctgcttgatcgcaaccagggaaaatgcgtgg aagggggtgtcgagattttcgacatgctgctcgccacctctt cccggttccggatgatgaatctgcagggagaagagttcgt gtgtctgaagtcaatcatcctgctgaactccggggtctatac cttcctgagctcgaccctcaagtcactggaggaaaaaga ccacatccatcgcgtgctcgataagatcaccgacaccctt atccatctcatggcgaaggctggactgaccctgcaacagc agcaccagaggctggcccagttgctgctgattctgagcca catccggcacatgtcgaacaagaggatggaacacctgta |
| | HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1001) | cagcatgaagtgcaagaacgtcgtgcctctgtccgatctg ctcctggaaatgctggacgcgcacagactcgggacggg agctgaagatccacgacccagcagaaagcgacgggaa gtgcaattggtggaatcagggggaggacttgtgcagcctg gaggatcgctgagactgtcatgtgccgtgtccggctttgcc ctgtccaaccacgggatgtcctgggtccgccgcgcgcctg gaaagggcctcgaatgggtgtcgggtattgtgtacagcgg tagcacctactatgccgcatccgtgaaggggagattcacc atcagccgggacaactccaggaacactctgtacctccaa atgaattcgctgaggccagaggacactgccatctactact gctccgcgcatggcggagagtccgacgtctggggacag gggaccaccgtgaccgtgtctagcgcgtccggcggaggc ggcagcgggggtcgggcatcagggggcggcggatcgg acatccagctcacccagtccccgagctcgctgtccgcctc cgtgggagatcgggtcaccatcacgtgccgcgccagcca gtcgatttcctcctacctgaactggtaccaacagaagcccg gaaaagccccgaagcttctcatctacgccgcctcgagcct gcagtcaggagtgccctcacggttctccggctccggttccg gtactgatttcaccctgaccatttcctccctgcaaccggagg acttcgctacttactactgccagcagtcgtactccaccccct acactttcggacaaggcaccaaggtcgaaatcaagacc actaccccagcaccgaggccacccaccccggctcctacc atcgcctcccagcctctgtccctgcgtccggaggcatgtag acccgcagctggtggggccgtgcatacccggggtcttgac ttcgcctgcgatatctacatttgggcccctctggctggtacttg cggggtcctgctgctttcactcgtgatcactctttactgtaagc gcggtcggaagaagctgctgtacatctttaagcaacccttc atgaggcctgtgcagactactcaagaggaggacggctgtt catgccggttcccagaggaggaggaaggcggctgcgaa ctgcgcgtgaaattcagccgcagcgcagatgctccagcct accagcaggggcagaaccagctctacaacgaactcaat cttggtcggagagaggagtacgacgtgctggacaagcg gagaggacgggacccagaaatgggcgggaagccgcg cagaaagaatccccaagagggcctgtacaacgagctcc aaaaggataagatggcagaagcctatagcgagattggta tgaaaggggaacgcagaagaggcaaaggccacgacg gactgtaccagggactcagcaccgccaccaaggacacc tatgacgctcttcacatgcaggccctgccgcctcgg (SEQ ID NO: 1061) |
| SP-Linker 3 - ERmut1 - Furin cleavage site 2-CD19 CAR2 | MALPVTALLLPLALLLHAARPD SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL | atggccctccctgtcaccgccctgctgcttccgctggctcttc tgctccacgccgctcggcccgattcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc |
| | TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1002) | cacatccggcacatgtcgtccaagaggatggaacacctg tacagcatgaagtgcaagaacgtcgtgcctctgtccgatct gctcctggaaatgctggacgcgcacagactcggaaccg gcgcggaagacccccggccctccaggaagcgaaggtc cctcggagacgtgggtgaaattgtgatgacccagtcaccc gccactcttagcctttcacccggtgagcgcgcaaccctgtc ttgcagagcctcccaagacatctcaaaataccttaattggt atcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1062) |
| SP-Linker 3 - ERmut2 - Furin cleavage site 2 - CD19 CAR2 | MALPVTALLLPLALLLHAARPD SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS | caggccctgccgcctggg (SEQ ID NO: 1002) atggccctccctgtcaccgccctgctgcttccgctggctcttc tgctccacgccgctcggcccgattcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctgaccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca |
| | RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1003) | gcagcaccagaggctggcccagttgctgctgattctgagc cacatccggcacatgtcgaacaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1063) |
| SP-Linker 3 - ERmut1 - Furin cleavage site 3 - CD19 CAR2 | MALPVTALLLPLALLLHAARPD SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREIVMTQSPATLSLSPG ERATLSCRASQDISKYLNWYQ | atggccctccctgtcaccgccctgctgcttccgctggctcttc tgctccacgccgctcggccccgattcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc |
| | QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1004) | cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcgggacgg gagctgaagatccacgacccagcagaaagcgacggga aattgtgatgacccagtcacccgccactcttagcctttcacc cggtgagcgcgcaaccctgtcttgcagagcctcccaaga catctcaaaataccttaattggtatcaacagaagcccgga caggctcctcgccttctgatctaccacaccagccggctcca ttctggaatccctgccaggttcagcggtagcggatctggga ccgactacaccctcactatcagctcactgcagccagagg acttcgctgtctatttctgtcagcaagggaacaccctgccct acacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1064) |
| SP-Linker 3 - ERmut2 - Furin cleavage site 3 - CD19 CAR2 | MALPVTALLLPLALLLHAARPD SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREIVMTQSPATLSLSPG | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa |
| | ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1005) | agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcGggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg acaggctcctcgccttctgatctaccacaccagccggctcc attctggaatccctgccaggttcagcggtagcggatctggg accgactacaccctcactatcagctcactgcagccagag gacttcgctgtctatttctgtcagcaagggaacaccctgccc tacacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1065) |
| SP-Linker 3 - ERmut1 - Furin cleavage site 2 - CD123 CAR | MALPVTALLLPLALLLHAARPD SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP | [?] (SEQ ID NO:[?]) atggccctccctgtcaccgccctgctgcttccgctggctcttc tgctccacgccgctcggccccgattcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat |
| | SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1006) | accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcggaaccg gcgcggaagacccccggccctccaggaagcgaaggtc cctcggagacgtgggtcaagtgcaactcgtccaaagcgg agcggaagtcaagaaacccggagcgagcgtgaaagtg tcctgcaaagcctccggctacacctttacgggctactacat gcactgggtgcgccaggcaccaggacagggtcttgaatg gatgggatggatcaaccctaattcgggcggaactaactac gcacagaagttccaggggagagtgactctgactcgggat acctccatctcaactgtctacatggaactctcccgcttgcgg tcagatgatacggcagtgtactactgcgcccgcgacatga atatcctggctaccgtgccgttcgacatctggggacaggg gactatggttactgtctcatcgggcggtggaggttcaggag gaggcggctcgggaggcggaggttcggacattcagatg acccagtccccatcctctctgtcggccagcgtcggagata gggtgaccattacctgtcgggcctcgcaaagcatctcctcg tacctcaactggtatcagcaaaagccgggaaaggcgcct aagctgctgatctacgccgcttcgagcttgcaaagcgggg tgccatccagattctcgggatcaggctcaggaaccgacttc accctgaccgtgaacagcctccagccggaggactttgcc acttactactgccagcagggagactccgtgccgcttactttc ggggggggtacccgcctggagatcaagaccactacccc agcaccgaggccacccaccccggctcctaccatcgcctc ccagcctctgtccctgcgtccggaggcatgtagacccgca gctggtggggccgtgcatacccggggtcttgacttcgcctg cgatatctacatttgggcccctctggctggtacttgcggggt cctgctgctttcactcgtgatcactctttactgtaagcgcggtc ggaagaagctgctgtacatctttaagcaacccttcatgagg cctgtgcagactactcaagaggaggacggctgttcatgcc ggttcccagaggaggaggaaggcggctgcgaactgcgc gtgaaattcagccgcagcgcagatgctccagcctaccag caggggcagaaccagctctacaacgaactcaatcttggt cggagagaggagtacgacgtgctggacaagcggagag gacgggacccagaaatgggcgggaagccgcgcagaa agaatccccaagagggcctgtacaacgagctccaaaag gataagatggcagaagcctatagcgagattggtatgaaa ggggaacgcagaagaggcaaaggccacgacggactgt accagggactcagcaccgccaccaaggacacctatgac gctcttcacatgcaggccctgccgcctcgg (SEQ ID NO: 1066) |
| SP-Linker 3 - ERmut2 - Furin cleavage site 2 - CD123 CAR | MALPVTALLLPLALLLHAARPD SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac |
| | SNKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1007) | ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtcaagtgcaactcgtccaaagcg gagcggaagtcaagaaacccggagcgagcgtgaaagt gtcctgcaaagcctccggctacacctttacgggctactaca tgcactgggtgcgccaggcaccaggacagggtcttgaat ggatgggatggatcaaccctaattcgggcggaactaacta cgcacagaagttccaggggagagtgactctgactcggga tacctccatctcaactgtctacatggaactctcccgcttgcg gtcagatgatacggcagtgtactactgcgcccgcgacatg aatatcctggctaccgtgccgttcgacatctggggacagg ggactatggttactgtctcatcgggcggtggaggttcagga ggaggcggctcgggaggcggaggttcggacattcagat gacccagtccccatcctctctgtcggccagcgtcggagat agggtgaccattacctgtcgggcctcgcaaagcatctcctc gtacctcaactggtatcagcaaaagccgggaaaggcgc ctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 1067) |
| SP-Linker 3 - ERmut1 - Furin cleavage site 3 - CD123 CAR | MALPVTALLLPLALLLHAARPD SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD | ID NO: 1007) atggccctccctgtcaccgccctgctgcttccgctggctcttc tgctccacgccgctcggcccgattcgttggcactttccctga ctgccgaccagatggtgtccgcccttctggacgccgagcct ccaattctgtactcggagtacgatccgactcgcccgttctcc gaagccagcatgatgggcctgttgactaacctggcggacc gcgagttggtgcacatgattaactgggctaagcgggtgccg ggcttcgtggacctggccctgcacgaccaagtgcacctcct |
| | HIHRVLDKITDTLIHLMAKAGLT LQQQHQRLAQLLLILSHIRHM SSKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTR GLDFACDIYIWAPLAGTCG VLLLSLVITLYCKRGRKKLL YIFKQPFMRPVQTTQEEDG CSCRFPEEEEGGCELRVK FSRSADAPAYQQGQNQLY NELNLGRREEYDVLDKRR GRDPEMGGKPRRKNPQE GLYNELQKDKMAEAYSEIG MKGERRRGKGHDGLYQGL STATKDTYDALHMQALPPR (SEQ ID NO: 1008) | ggaatgcgcctggatggaaatcctcatgatcggcctcgtgt ggagatccatggagcatcccggaaagctcctgtttgcaccc aacctcctgcttgatcgcaaccagggaaaatgcgtggaag ggggtgtcgagattttcgacatgctgctcgccacctcttccc ggttccggatgatgaatctgcagggagaagagttcgtgtgt ctgaagtcaatcatcctgctgaactccggggtctataccttc ctgagctcgaccctcaagtcactggaggaaaaagaccaca tccatcgcgtgctcgataagatcaccgacacccttatccatc tcatggcgaaggctggactgaccctgcaacagcagcacca gaggctggcccagttgctgctgattctgagccacatccggc acatgtcgtccaagaggatggaacacctgtacagcatgaa gtgcaagaacgtcgtgcctctgtccgatctgctcctggaaat gctggacgcgcacagactcggaaccggcgcggaagaccc ccggccctccaggaagcgaaggcaagtgcaactcgtccaa agcggagcggaagtcaagaaacccggagcgagcgtgaaa gtgtcctgcaaagcctccggctacacctttacgggctactac atgcactgggtgcgccaggcaccaggacagggtcttgaat ggatgggatggatcaaccctaattcgggcggaactaacta cgcacagaagttccaggggagagtgactctgactcgggat acctccatctcaactgtctacatggaactctcccgcttgcgg tcagatgatacggcagtgtactactgcgcccgcgacatgaa tatcctggctaccgtgccgttcgacatctggggacagggga ctatggttactgtctcatcgggcggtggaggttcaggagga ggcggctcgggaggcggaggttcggacattcagatgaccc agtccccatcctctctgtcggccagcgtcggagatagggtg accattacctgtcgggcctcgcaaagcatctcctcgtacctc aactggtatcagcaaaagccgggaaaggcgcctaagctgc tgatctacgccgcttcgagcttgcaaagcggggtgccatcc agattctcgggatcaggctcaggaaccgacttcaccctgac cgtgaacagcctccagccggaggactttgccacttactact gccagcagggagactccgtgccgcttactttcggggggggt acccgcctggagatcaagaccactaccccagcaccgaggc cacccaccccggctcctaccatcgcctcccagcctctgtccc tgcgtccggaggcatgtagacccgcagctggtggggccgt gcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagactac tcaagaggaggacggctgttcatgccggttcccagaggag gaggaaggcggctgcgaactgcgcgtgaaattcagccgca gcgcagatgctccagcctaccagcaggggcagaaccagct ctacaacgaactcaatcttggtcggagagaggagtacgac gtgctggacaagcggagaggacgggacccagaaatgggc gggaagccgcgcagaaagaatccccaagagggcctgtac aacgagctccaaaaggataagatggcagaagcctatagc gagattggtatgaaaggggaacgcagaagaggcaaaggc cacgacggactgtaccagggactcagcaccgccaccaagg acacctatgacgctcttcacatgcaggccctgccgcctcgg |
| | | (SEQ ID NO: 1068) |
| SP-Linker 3 - ERmut2 - Furin cleavage site 3 - CD123 CAR | MALPVTALLLPLALLLHAARPD SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1009) | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggc aagtgcaactcgtccaaagcggagcggaagtcaagaaa cccggagcgagcgtgaaagtgtcctgcaaagcctccggc tacacctttacgggctactacatgcactgggtgcgccaggc accaggacagggtcttgaatggatgggatggatcaaccct aattcgggcggaactaactacgcacagaagttccagggg agagtgactctgactcgggatacctccatctcaactgtctac atggaactctcccgcttgcggtcagatgatacggcagtgta ctactgcgcccgcgacatgaatatcctggctaccgtgccgt tcgacatctggggacaggggactatggttactgtctcatcg ggcggtggaggttcaggaggaggcggctcgggaggcg gaggttcggacattcagatgacccagtccccatcctctctgt cggccagcgtcggagatagggtgaccattacctgtcggg cctcgcaaagcatctcctcgtacctcaactggtatcagcaa aagccgggaaaggcgcctaagctgctgatctacgccgct tcgagcttgcaaagcggggtgccatccagattctcgggat caggctcaggaaccgacttcaccctgaccgtgaacagcc tccagccggaggactttgccacttactactgccagcaggg agactccgtgccgcttactttcggggggggtacccgcctgg agatcaagaccactaccccagcaccgaggccacccacc ccggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctaccagcaggggcagaaccagctcta caacgaactcaatcttggtcggagagaggagtacgacgt gctggacaagcggagaggacgggacccagaaatgggc gggaagccgcgcagaaagaatccccaagagggcctgt acaacgagctccaaaaggataagatggcagaagcctat agcgagattggtatgaaaggggaacgcagaagaggca aaggccacgacggactgtaccagggactcagcaccgcc |
| | | accaaggacacctatgacgctcttcacatgcaggccctgc cgcctcgg (SEQ ID NO: 1069) |
| SP-Linker 3 - ERmut1 - Furin cleavage site 2 - BCMA CAR | MALPVTALLLPLALLLHAARPD SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1010) | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag |
| | | aagaggcaaaggccacgacggactgtaccagggactc agcaccgccaccaaggacacctatgacgctcttcacatg caggccctgccgcctcgg (SEQ ID NO: 1070) |
| SP-Linker 3 - ERmut2 - Furin cleavage site 2 - BCMA CAR | MALPVTALLLPLALLLHAARPD SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1011) | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca |
| | | gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1071) |
| SP-Linker 3 - ERmut1 - Furin cleavage site 3 - BCMA CAR | MALPVTALLLPLALLLHAARPD SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1012) | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcGggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga |
| | | gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1072) |
| SP-Linker 3 - ERmut2 - Furin cleavage site 3 - BCMA CAR | MALPVTALLLPLALLLHAARPD SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1013) | atggccctccctgtcaccgccctgctgcttccgctggctcttc tgctccacgccgctcggcccgatTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc |
| | | cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1073) |
| SP-Linker 4 - ERmut1 - Furin cleavage site 2 - CD19 CAR2 | MALPVTALLLPLALLLHAARPN SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1014) | atggccctccctgtcaccgccctgctgcttccgctggctcttc tgctccacgccgctcggcccaactcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcggaaccg gcgcggaagacccccggccctccaggaagcgaaggtc cctcggagacgtgggtgaaattgtgatgacccagtcaccc gccactcttagcctttcacccggtgagcgcgcaaccctgtc ttgcagagcctcccaagacatctcaaaataccttaattggt atcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga |
| | | gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1074) |
| SP-Linker 4 - ERmut2 - Furin cleavage site 2 - CD19 CAR2 | MALPVTALLLPLALLLHAARPN SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1015) | atggccctccctgtcaccgccctgctgcttccgctggctcttc tgctccacgccgctcggcccaactcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctgaccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgaacaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa |
| | | ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1075) |
| SP-Linker 4 - ERmut1 - Furin cleavage site 3 - CD19 CAR2 | MALPVTALLLPLALLLHAARPN SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1016) | atggccctccctgtcaccgccctgctgcttccgctggctcttc tgctccacgccgctcggcccaactcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcgggacgg gagctgaagatccacgacccagcagaaagcgacggga aattgtgatgacccagtcacccgccactcttagcctttcacc cggtgagcgcgcaaccctgtcttgcagagcctcccaaga catctcaaaataccttaattggtatcaacagaagcccgga caggctcctcgccttctgatctaccacaccagccggctcca ttctggaatccctgccaggttcagcggtagcggatctggga ccgactacaccctcactatcagctcactgcagccagagg acttcgctgtctatttctgtcagcaagggaacaccctgccct acacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc |
| | | agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1076) |
| SP-Linker 4 - ERmut2 - Furin cleavage site 3 - CD19 CAR2 | MALPVTALLLPLALLLHAARPN SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1017) | atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcGggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg acaggctcctcgccttctgatctaccacaccagccggctcc attctggaatccctgccaggttcagcggtagcggatctggg accgactacaccctcactatcagctcactgcagccagag gacttcgctgtctatttctgtcagcaagggaacaccctgccc tacacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg |
| | | cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1077) |
| SP-Linker 4 - ERmut1 - Furin cleavage site 2 - CD123 CAR | MALPVTALLLPLALLLHAARPN SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1018) | atggccctccctgtcaccgccctgctgcttccgctggctcttc tgctccacgccgctcggcccaactcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcggaaccg gcgcggaagacccccggccctccaggaagcgaaggtc cctcggagacgtgggtcaagtgcaactcgtccaaagcgg agcggaagtcaagaaacccggagcgagcgtgaaagtg tcctgcaaagcctccggctacacctttacgggctactacat gcactgggtgcgccaggcaccaggacagggtcttgaatg gatgggatggatcaaccctaattcgggcggaactaactac gcacagaagttccaggggagagtgactctgactcgggat acctccatctcaactgtctacatggaactctcccgcttgcgg tcagatgatacggcagtgtactactgcgcccgcgacatga atatcctggctaccgtgccgttcgacatctggggacaggg gactatggttactgtctcatcgggcggtggaggttcaggag gaggcggctcgggaggcggaggttcggacattcagatg acccagtccccatcctctctgtcggccagcgtcggagata gggtgaccattacctgtcgggcctcgcaaagcatctcctcg tacctcaactggtatcagcaaaagccgggaaaggcgcct aagctgctgatctacgccgcttcgagcttgcaaagcgggg tgccatccagattctcgggatcaggctcaggaaccgacttc accctgaccgtgaacagcctccagccggaggactttgcc acttactactgccagcagggagactccgtgccgcttactttc ggggggggtacccgcctggagatcaagaccactacccc agcaccgaggccacccaccccggctcctaccatcgcctc ccagcctctgtccctgcgtccggaggcatgtagacccgca gctggtggggccgtgcatacccggggtcttgacttcgcctg cgatatctacatttgggcccctctggctggtacttgcggggt cctgctgctttcactcgtgatcactctttactgtaagcgcggtc ggaagaagctgctgtacatctttaagcaacccttcatgagg |
| | | cctgtgcagactactcaagaggaggacggctgttcatgcc ggttcccagaggaggaggaaggcggctgcgaactgcgc gtgaaattcagccgcagcgcagatgctccagcctaccag caggggcagaaccagctctacaacgaactcaatcttggt cggagagaggagtacgacgtgctggacaagcggagag gacgggacccagaaatgggcgggaagccgcgcagaa agaatccccaagagggcctgtacaacgagctccaaaag gataagatggcagaagcctatagcgagattggtatgaaa ggggaacgcagaagaggcaaaggccacgacggactgt accagggactcagcaccgccaccaaggacacctatgac gctcttcacatgcaggccctgccgcctcgg (SEQ ID NO: 1078) |
| SP-Linker 4 - ERmut2 - Furin cleavage site 2 - CD123 CAR | MALPVTALLLPLALLLHAARPN SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1019) | atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtcaagtgcaactcgtccaaagcg gagcggaagtcaagaaacccggagcgagcgtgaaagt gtcctgcaaagcctccggctacacctttacgggctactaca tgcactgggtgcgccaggcaccaggacagggtcttgaat ggatgggatggatcaaccctaattcgggcggaactaacta cgcacagaagttccaggggagagtgactctgactcggga tacctccatctcaactgtctacatggaactctcccgcttgcg gtcagatgatacggcagtgtactactgcgcccgcgacatg aatatcctggctaccgtgccgttcgacatctggggacagg ggactatggttactgtctcatcgggcggtggaggttcagga ggaggcggctcgggaggcggaggttcggacattcagat gacccagtccccatcctctctgtcggccagcgtcggagat agggtgaccattacctgtcgggcctcgcaaagcatctcctc gtacctcaactggtatcagcaaaagccgggaaaggcgc ctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg |
| | | ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 1079) |
| SP-Linker 4 - ERmut1 - Furin cleavage site 3 - CD123 CAR | MALPVTALLLPLALLLHAARPN SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTR GLDFACDIYIWAPLAGTCG VLLLSLVITLYCKRGRKKLL YIFKQPFMRPVQTTQEEDG CSCRFPEEEEGGCELRVK FSRSADAPAYQQGQNQLY NELNLGRREEYDVLDKRR GRDPEMGGKPRRKNPQE GLYNELQKDKMAEAYSEIG MKGERRRGKGHDGLYQGL STATKDTYDALHMQALPPR (SEQ ID NO: 1020) | atggccctccctgtcaccgccctgctgcttccgctggctcttc tgctccacgccgctcggcccaactcgttggcactttccctga ctgccgaccagatggtgtccgcccttctggacgccgagcct ccaattctgtactcggagtacgatccgactcgcccgttctcc gaagccagcatgatgggcctgttgactaacctggcggacc gcgagttggtgcacatgattaactgggctaagcgggtgccg ggcttcgtggacctggccctgcacgaccaagtgcacctcct ggaatgcgcctggatggaaatcctcatgatcggcctcgtgt ggagatccatggagcatcccggaaagctcctgtttgcaccc aacctcctgcttgatcgcaaccagggaaaatgcgtggaagg gggtgtcgagattttcgacatgctgctcgccacctcttccc ggttccggatgatgaatctgcagggagaagagttcgtgtgt ctgaagtcaatcatcctgctgaactccggggtctataccttc ctgagctcgaccctcaagtcactggaggaaaaagaccaca tccatcgcgtgctcgataagatcaccgacacccttatccatc tcatggcgaaggctggactgaccctgcaacagcagcacca gaggctggcccagttgctgctgattctgagccacatccggc acatgtcgtccaagaggatggaacacctgtacagcatgaa gtgcaagaacgtcgtgcctctgtccgatctgctcctggaaat gctggacgcgcacagactcggaaccggcgcggaagaccc ccggccctccaggaagcgaaggcaagtgcaactcgtccaa agcggagcggaagtcaagaaacccggagcgagcgtgaaa gtgtcctgcaaagcctccggctacacctttacgggctactac atgcactgggtgcgccaggcaccaggacagggtcttgaat ggatgggatggatcaaccctaattcgggcggaactaacta cgcacagaagttccaggggagagtgactctgactcgggat acctccatctcaactgtctacatggaactctcccgcttgcgg tcagatgatacggcagtgtactactgcgcccgcgacatgaa tatcctggctaccgtgccgttcgacatctggggacagggga ctatggttactgtctcatcgggcggtggaggttcaggagga ggcggctcgggaggcggaggttcggacattcagatgaccc agtccccatcctctctgtcggccagcgtcggagatagggtg accattacctgtcgggcctcgcaaagcatctcctcgtacctc aactggtatcagcaaaagccgggaaaggcgcctaagctgc tgatctacgccgcttcgagcttgcaaagcggggtgccatcc agattctcgggatcaggctcaggaaccgacttcaccctgac |
| | | cgtgaacagcctccagccggaggactttgccacttactact gccagcagggagactccgtgccgcttactttcggggggggt acccgcctggagatcaagaccactaccccagcaccgaggc cacccaccccggctcctaccatcgcctcccagcctctgtccc tgcgtccggaggcatgtagacccgcagctggtggggccgt gcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagactac tcaagaggaggacggctgttcatgccggttcccagaggag gaggaaggcggctgcgaactgcgcgtgaaattcagccgca gcgcagatgctccagcctaccagcaggggcagaaccagct ctacaacgaactcaatcttggtcggagagaggagtacgac gtgctggacaagcggagaggacgggacccagaaatgggc gggaagccgcgcagaaagaatccccaagagggcctgtac aacgagctccaaaaggataagatggcagaagcctatagc gagattggtatgaaaggggaacgcagaagaggcaaaggc cacgacggactgtaccagggactcagcaccgccaccaagg acacctatgacgctcttcacatgcaggccctgccgcctcgg (SEQ ID NO: 1080) |
| SP-Linker 4 - ERmut2 - Furin cleavage site 3 - CD123 CAR | MALPVTALLLPLALLLHAARPN SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK | atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggc aagtgcaactcgtccaaagcggagcggaagtcaagaaa cccggagcgagcgtgaaagtgtcctgcaaagcctccggc tacacctttacgggctactacatgcactgggtgcgccaggc accaggacagggtcttgaatggatgggatggatcaaccct aattcgggcggaactaactacgcacagaagttccagggg agagtgactctgactcgggatacctccatctcaactgtctac atggaactctcccgcttgcggtcagatgatacggcagtgta ctactgcgcccgcgacatgaatatcctggctaccgtgccgt tcgacatctggggacaggggactatggttactgtctcatcg ggcggtggaggttcaggaggaggcggctcgggaggcg gaggttcggacattcagatgacccagtccccatcctctctgt cggccagcgtcggagatagggtgaccattacctgtcggg |
| | NPQEGLYNELQKDKMAEAYS EIGMKGERRRGKGHDGLYQG LSTATKDTYDALHMQALPPR (SEQ ID NO: 1021) | cctcgcaaagcatctcctcgtacctcaactggtatcagcaa aagccgggaaaggcgcctaagctgctgatctacgccgct tcgagcttgcaaagcggggtgccatccagattctcgggat caggctcaggaaccgacttcaccctgaccgtgaacagcc tccagccggaggactttgccacttactactgccagcaggg agactccgtgccgcttactttcggggggggtacccgcctgg agatcaagaccactaccccagcaccgaggccacccacc ccggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctaccagcaggggcagaaccagctcta caacgaactcaatcttggtcggagagaggagtacgacgt gctggacaagcggagaggacgggacccagaaatgggc gggaagccgcgcagaaagaatccccaagagggcctgt acaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 1081) |
| SP-Linker 4 - ERmut1 - Furin cleavage site 2 - BCMA CAR | MALPVTALLLPLALLLHAARPN SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY | atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc |
| | DVLDKRRGRDPEMGGKPRRK NPQEGLYNELQKDKMAEAYS EIGMKGERRRGKGHDGLYQG LSTATKDTYDALHMQALPPR (SEQ ID NO: 1022) | ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1082) |
| SP-Linker 4 - ERmut2 - Furin cleavage site 2 - BCMA CAR | MALPVTALLLPLALLLHAARPN SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA | atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat |
| | YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1023) | cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1083) |
| SP-Linker 4 - ERmut1 - Furin cleavage site 3 - BCMA CAR | MALPVTALLLPLALLLHAARPN SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC | atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcGggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag |
| | ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1024) | gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1084) |
| SP-Linker 4 - ERmut2 - Furin cleavage site 3 - BCMA CAR | MALPVTALLLPLALLLHAARPN SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ | g (SEQ ID NO: 1004) atggccctccctgtcaccgccctgctgcttccgctggctcttc tgctccacgccgctcggcccaacTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca |
| | TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1025) | ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1085) |
| SP-Linker 5 - ERmut1 - Furin cleavage site 2-CD19 CAR2 | MALPVTALLLPLALLLHAARPE SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC | g (SEQ ID NO: 1003) atggccctccctgtcaccgccctgctgcttccgctggctcttc tgctccacgccgctcggccccgagtcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc |
| | GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1026) | aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1086) |
| SP-Linker 5 - ERmut2 - Furin cleavage site 2-CD19 CAR2 | MALPVTALLLPLALLLHAARPE SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV | [?] atggccctccctgtcaccgccctgctgcttccgctggctcttc tgctccacgccgctcggcccgagtcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca |
| | HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1027) | gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1087) |
| SP-Linker 5 - ERmut1 - Furin cleavage site 3-CD19 CAR2 | MALPVTALLLPLALLLHAARPE SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP | atggccctccctgtcaccgccctgctgcttccgctggctcttc tgctccacgccgctcggcccgagtcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcgggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg acaggctcctcgccttctgatctaccacaccagccggctcc attctggaatccctgccaggttcagcggtagcggatctggg |
| | EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1028) | accgactacaccctcactatcagctcactgcagccagag gacttcgctgtctatttctgtcagcaagggaacaccctgccc tacacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1088) |
| SP-Linker 5 - ERmut2 - Furin cleavage site 3-CD19 CAR2 | MALPVTALLLPLALLLHAARPE SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG | atggccctccctgtcaccgccctgctgcttccgctggctcttc tgctccacgccgctcggccccgagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcGggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg |
| | GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1029) | acaggctcctcgccttctgatctaccacaccagccggctcc attctggaatccctgccaggttcagcggtagcggatctggg accgactacaccctcactatcagctcactgcagccagag gacttcgctgtctatttctgtcagcaagggaacaccctgccc tacacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1089) |
| SP-Linker 5 - ERmut1 - Furin cleavage site 2 - CD123 CAR | MALPVTALLLPLALLLHAARPE SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ | [?] (SEQ ID NO: 1990) atggccctccctgtcaccgccctgctgcttccgctggctcttc tgctccacgccgctcggcccgagtcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtcaagtgcaactcgtccaaagcg gagcggaagtcaagaaacccggagcgagcgtgaaagt |
| | SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1030) | gtcctgcaaagcctccggctacacctttacgggctactaca tgcactgggtgcgccaggcaccaggacagggtcttgaat ggatgggatggatcaaccctaattcgggcggaactaacta cgcacagaagttccaggggagagtgactctgactcggga tacctccatctcaactgtctacatggaactctcccgcttgcg gtcagatgatacggcagtgtactactgcgcccgcgacatg aatatcctggctaccgtgccgttcgacatctggggacagg ggactatggttactgtctcatcgggcggtggaggttcagga ggaggcggctcgggaggcggaggttcggacattcagat gacccagtccccatcctctctgtcggccagcgtcggagat agggtgaccattacctgtcgggcctcgcaaagcatctcctc gtacctcaactggtatcagcaaaagccgggaaaggcgc ctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 1090) |
| SP-Linker 5 - ERmut2 - Furin cleavage site 2-CD123 CAR | MALPVTALLLPLALLLHAARPE SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA | atggccctccctgtcaccgccctgctgcttccgctggctcttc tgctccacgccgctcggcccgagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt |
| | SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1031) | ccctcggagacgtgggtcaagtgcaactcgtccaaagcg gagcggaagtcaagaaacccggagcgagcgtgaaagt gtcctgcaaagcctccggctacacctttacgggctactaca tgcactgggtgcgccaggcaccaggacagggtcttgaat ggatgggatggatcaaccctaattcgggcggaactaacta cgcacagaagttccaggggagagtgactctgactcggga tacctccatctcaactgtctacatggaactctcccgcttgcg gtcagatgatacggcagtgtactactgcgcccgcgacatg aatatcctggctaccgtgccgttcgacatctggggacagg ggactatggttactgtctcatcgggcggtggaggttcagga ggaggcggctcgggaggcggaggttcggacattcagat gacccagtccccatcctctctgtcggccagcgtcggagat agggtgaccattacctgtcgggcctcgcaaagcatctcctc gtacctcaactggtatcagcaaaagccgggaaaggcgc ctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 1091) |
| SP-Linker 5 - ERmut1 - Furin cleavage site 3 - CD123 CAR | MALPVTALLLPLALLLHAARPE SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM | atggccctccctgtcaccgccctgctgcttccgctggctcttc tgctccacgccgctcggcccgagtcgttggcactttccctga ctgccgaccagatggtgtccgcccttctggacgccgagcct ccaattctgtactcggagtacgatccgactcgcccgttctcc gaagccagcatgatgggcctgttgactaacctggcggacc gcgagttggtgcacatgattaactgggctaagcgggtgccg ggcttcgtggacctggccctgcacgaccaagtgcacctcct ggaatgcgcctggatggaaatcctcatgatcggcctcgtgt ggagatccatggagcatcccggaaagctcctgtttgcaccc aacctcctgcttgatcgcaaccagggaaaatgcgtggaag ggggtgtcgagattttcgacatgctgctcgccacctcttccc ggttccggatgatgaatctgcagggagaagagttcgtgtgt ctgaagtcaatcatcctgctgaactccggggtctataccttc ctgagctcgaccctcaagtcactggaggaaaaagaccaca tccatcgcgtgctcgataagatcaccgacacccttatccatc tcatggcgaaggctggactgaccctgcaacagcagcacca |
| | VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTR GLDFACDIYIWAPLAGTCG VLLLSLVITLYCKRGRKKLL YIFKQPFMRPVQTTQEEDG CSCRFPEEEEGGCELRVK FSRSADAPAYQQGQNQLY NELNLGRREEYDVLDKRR GRDPEMGGKPRRKNPQE GLYNELQKDKMAEAYSEIG MKGERRRGKGHDGLYQGL STATKDTYDALHMQALPPR (SEQ ID NO: 1032) | gaggctggcccagttgctgctgattctgagccacatccggc acatgtcgtccaagaggatggaacacctgtacagcatgaa gtgcaagaacgtcgtgcctctgtccgatctgctcctggaaat gctggacgcgcacagactcggaaccggcgcggaagaccc ccggccctccaggaagcgaaggcaagtgcaactcgtccaa agcggagcggaagtcaagaaacccggagcgagcgtgaaa gtgtcctgcaaagcctccggctacacctttacgggctactac atgcactgggtgcgccaggcaccaggacagggtcttgaat ggatgggatggatcaaccctaattcgggcggaactaacta cgcacagaagttccaggggagagtgactctgactcgggat acctccatctcaactgtctacatggaactctcccgcttgcgg tcagatgatacggcagtgtactactgcgcccgcgacatgaa tatcctggctaccgtgccgttcgacatctggggacagggga ctatggttactgtctcatcgggcggtggaggttcaggagga ggcggctcgggaggcggaggttcggacattcagatgaccc agtccccatcctctctgtcggccagcgtcggagatagggtg accattacctgtcgggcctcgcaaagcatctcctcgtacctc aactggtatcagcaaaagccgggaaaggcgcctaagctgc tgatctacgccgcttcgagcttgcaaagcggggtgccatcc agattctcgggatcaggctcaggaaccgacttcaccctgac cgtgaacagcctccagccggaggactttgccacttactact gccagcagggagactccgtgccgcttactttcggggggggt acccgcctggagatcaagaccactaccccagcaccgaggc cacccaccccggctcctaccatcgcctcccagcctctgtccc tgcgtccggaggcatgtagacccgcagctggtggggccgt gcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagactac tcaagaggaggacggctgttcatgccggttcccagaggag gaggaaggcggctgcgaactgcgcgtgaaattcagccgca gcgcagatgctccagcctaccagcaggggcagaaccagct ctacaacgaactcaatcttggtcggagagaggagtacgac gtgctggacaagcggagaggacgggacccagaaatgggc gggaagccgcgcagaaagaatccccaagagggcctgtac aacgagctccaaaaggataagatggcagaagcctatagc gagattggtatgaaaggggaacgcagaagaggcaaaggc cacgacggactgtaccagggactcagcaccgccaccaagg acacctatgacgctcttcacatgcaggccctgccgcctcgg (SEQ ID NO: 1092) |
| SP-Linker 5 - ERmut2 - Furin cleavage site 3-CD123 CAR | MALPVTALLLPLALLLHAARPE SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI | atggccctccctgtcaccgccctgctgcttccgctggctcttc tgctccacgccgctcggcccgagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc |
| | ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1033) | tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggc aagtgcaactcgtccaaagcggagcggaagtcaagaaa cccggagcgagcgtgaaagtgtcctgcaaagcctccggc tacacctttacgggctactacatgcactgggtgcgccaggc accaggacagggtcttgaatggatgggatggatcaaccct aattcgggcggaactaactacgcacagaagttccagggg agagtgactctgactcgggatacctccatctcaactgtctac atggaactctcccgcttgcggtcagatgatacggcagtgta ctactgcgcccgcgacatgaatatcctggctaccgtgccgt tcgacatctggggacaggggactatggttactgtctcatcg ggcggtggaggttcaggaggaggcggctcgggaggcg gaggttcggacattcagatgacccagtccccatcctctctgt cggccagcgtcggagatagggtgaccattacctgtcggg cctcgcaaagcatctcctcgtacctcaactggtatcagcaa aagccgggaaaggcgcctaagctgctgatctacgccgct tcgagcttgcaaagcggggtgccatccagattctcgggat caggctcaggaaccgacttcaccctgaccgtgaacagcc tccagccggaggactttgccacttactactgccagcaggg agactccgtgccgcttactttcggggggggtacccgcctgg agatcaagaccactaccccagcaccgaggccacccacc ccggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctaccagcaggggcagaaccagctcta caacgaactcaatcttggtcggagagaggagtacgacgt gctggacaagcggagaggacgggacccagaaatgggc gggaagccgcgcagaaagaatccccaagagggcctgt acaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 1093) |
| SP-Linker 5 - ERmut1 - Furin cleavage site 2 - BCMA CAR | MALPVTALLLPLALLLHAARPE SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA | atggccctccctgtcaccgccctgctgcttccgctggctcttc tgctccacgccgctcggcccgagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca |
| | TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1034) | cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1094) |
| SP-Linker 5 - ERmut2 - Furin cleavage site 2 - BCMA CAR | MALPVTALLLPLALLLHAARPE SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL | atggccctccctgtcaccgccctgctgcttccgctggctcttc tgctccacgccgctcggcccgagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt |
| | LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1035) | gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1095) |
| SP-Linker 5 - ERmut1 - Furin cleavage site 3 - BCMA CAR | MALPVTALLLPLALLLHAARPE SLALSLTADQMVSALLDAEPPI LYSEYDPTRPFSEASMMGLLT NLADRELVHMINWAKRVPGF VDLALHDQVHLLECAWMEILM | atggccctccctgtcaccgccctgctgcttccgctggctcttc tgctccacgccgctcggcccgagTcgttggcactttccctg actgccgaccagatggtgtccgcccttctggacgccgagc ctccaattctgtactcggagtacgatccgactcgcccgttct ccgaagccagcatgatgggcctgttgactaacctggcgg |
| | IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1036) | accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcGggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1096) |
| SP-Linker 5 - ERmut2 - Furin cleavage site 3 - BCMA CAR | MALPVTALLLPLALLLHAARPE SLALSLTADQMVSALLDAEPPI LYSEYDPTRPFSEASMMGLLT NLADRELVHMINWAKRVPGF | atggccctccctgtcaccgccctgctgcttccgctggctcttc tgctccacgccgctcggcccgagTcgttggcactttccctg actgccgaccagatggtgtccgcccttctggacgccgagc ctccaattctgtactcggagtacgatccgactcgcccgttct |
| | VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG ERRRGKGHDGLYQGLSTATK DTYDALHMQALPPR (SEQ ID NO: 1037) | ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1097) |
| SP-Linker 6 - ERmut1 - Furin | MALPVTALLLPLALLLHAARPQ SLALSLTADQMVSALLDAEPPI | atggccctccctgtcaccgccctgctgcttccgctggctcttc tgctccacgccgctcggccccagtcgttggcactttccctga |
| cleavage site 2 - CD19 CAR2 | LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1038) | ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcggaaccg gcgcggaagacccccggccctccaggaagcgaaggtc cctcggagacgtgggtgaaattgtgatgacccagtcaccc gccactcttagcctttcacccggtgagcgcgcaaccctgtc ttgcagagcctcccaagacatctcaaaataccttaattggt atcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1098) |
| SP-Linker 6 - | MALPVTALLLPLALLLHAARPQ | atggccctccctgtcaccgccctgctgcttccgctggctcttc |
| ERmut2 - Furin cleavage site 2 - CD19 CAR2 | SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEIVMTQSPAT LSLSPGERATLSCRASQDISK YLNWYQQKPGQAPRLLIYHTS RLHSGIPARFSGSGSGTDYTL TISSLQPEDFAVYFCQQGNTL PYTFGQGTKLEIKGGGGSGG GGSGGGGSQVQLQESGPGL VKPSETLSLTCTVSGVSLPDY GVSWIRQPPGKGLEWIGVIW GSETTYYSSSLKSRVTISKDN SKNQVSLKLSSVTAADTAVYY CAKHYYYGGSYAMDYWGQG TLVTVSSTTTPAPRPPTPAPTI ASQPLSLRPEACRPAAGGAV HTRGLDFACDIYIWAPLAGTC GVLLLSLVITLYCKRGRKKLLYI FKQPFMRPVQTTQEEDGCSC RFPEEEEGGCELRVKFSRSA DAPAYQQGQNQLYNELNLGR REEYDVLDKRRGRDPEMGGK PRRKNPQEGLYNELQKDKMA EAYSEIGMKGERRRGKGHDG LYQGLSTATKDTYDALHMQAL PPR (SEQ ID NO: 1039) | tgctccacgccgctcggccccagtcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctgaccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgaacaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaaattgtgatgacccagtcacc cgccactcttagcctttcacccggtgagcgcgcaaccctgt cttgcagagcctcccaagacatctcaaaataccttaattgg tatcaacagaagcccggacaggctcctcgccttctgatcta ccacaccagccggctccattctggaatccctgccaggttc agcggtagcggatctgggaccgactacaccctcactatca gctcactgcagccagaggacttcgctgtctatttctgtcagc aagggaacaccctgccctacacctttggacagggcacca agctcgagattaaaggtggaggtggcagcggaggaggt gggtccggcggtggaggaagccaggtccaactccaaga aagcggaccgggtcttgtgaagccatcagaaactctttca ctgacttgtactgtgagcggagtgtctctccccgattacggg gtgtcttggatcagacagccaccggggaagggtctggaat ggattggagtgatttggggctctgagactacttactactcttc atccctcaagtcacgcgtcaccatctcaaaggacaactct aagaatcaggtgtcactgaaactgtcatctgtgaccgcag ccgacaccgccgtgtactattgcgctaagcattactattatg gcgggagctacgcaatggattactggggacagggtactct ggtcaccgtgtccagcaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1099) |
| SP-Linker 6 - ERmut1 - Furin cleavage site 3 - CD19 CAR2 | MALPVTALLLPLALLLHAARPQ SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1040) | atggccctccctgtcaccgccctgctgcttccgctggctcttc tgctccacgccgctcggccccagtcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcgggacgg gagctgaagatccacgacccagcagaaagcgacggga aattgtgatgacccagtcacccgccactcttagcctttcacc cggtgagcgcgcaaccctgtcttgcagagcctcccaaga catctcaaaataccttaattggtatcaacagaagcccgga caggctcctcgccttctgatctaccacaccagccggctcca ttctggaatccctgccaggttcagcggtagcggatctggga ccgactacaccctcactatcagctcactgcagccagagg acttcgctgtctatttctgtcagcaagggaacaccctgccct acacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg |
| | | g (SEQ ID NO: 1100) |
| SP-Linker 6 - ERmut2 - Furin cleavage site 3 - CD19 CAR2 | MALPVTALLLPLALLLHAARPQ SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREIVMTQSPATLSLSPG ERATLSCRASQDISKYLNWYQ QKPGQAPRLLIYHTSRLHSGIP ARFSGSGSGTDYTLTISSLQP EDFAVYFCQQGNTLPYTFGQ GTKLEIKGGGGSGGGGSGGG GSQVQLQESGPGLVKPSETL SLTCTVSGVSLPDYGVSWIRQ PPGKGLEWIGVIWGSETTYYS SSLKSRVTISKDNSKNQVSLK LSSVTAADTAVYYCAKHYYYG GSYAMDYWGQGTLVTVSSTT TPAPRPPTPAPTIASQPLSLRP EACRPAAGGAVHTRGLDFAC DIYIWAPLAGTCGVLLLSLVITL YCKRGRKKLLYIFKQPFMRPV QTTQEEDGCSCRFPEEEEGG CELRVKFSRSADAPAYQQGQ NQLYNELNLGRREEYDVLDK RRGRDPEMGGKPRRKNPQE GLYNELQKDKMAEAYSEIGMK GERRRGKGHDGLYQGLSTAT KDTYDALHMQALPPR (SEQ ID NO: 1041) | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcGggacg ggagctgaagatccacgacccagcagaaagcgacggg aaattgtgatgacccagtcacccgccactcttagcctttcac ccggtgagcgcgcaaccctgtcttgcagagcctcccaag acatctcaaaataccttaattggtatcaacagaagcccgg acaggctcctcgccttctgatctaccacaccagccggctcc attctggaatccctgccaggttcagcggtagcggatctggg accgactacaccctcactatcagctcactgcagccagag gacttcgctgtctatttctgtcagcaagggaacaccctgccc tacacctttggacagggcaccaagctcgagattaaaggtg gaggtggcagcggaggaggtgggtccggcggtggagg aagccaggtccaactccaagaaagcggaccgggtcttgt gaagccatcagaaactctttcactgacttgtactgtgagcg gagtgtctctccccgattacggggtgtcttggatcagacag ccaccggggaagggtctggaatggattggagtgatttggg gctctgagactacttactactcttcatccctcaagtcacgcgt caccatctcaaaggacaactctaagaatcaggtgtcactg aaactgtcatctgtgaccgcagccgacaccgccgtgtact attgcgctaagcattactattatggcgggagctacgcaatg gattactggggacagggtactctggtcaccgtgtccagca ccactaccccagcaccgaggccacccaccccggctccta ccatcgcctcccagcctctgtccctgcgtccggaggcatgt agacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg |
| | | acacctatgacgctcttcacatgcaggccctgccgcctcg g (SEQ ID NO: 1101) |
| SP-Linker 6 - ERmut1 - Furin cleavage site 2 - CD123 CAR | MALPVTALLLPLALLLHAARPQ SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1042) | atggccctccctgtcaccgccctgctgcttccgctggctcttc tgctccacgccgctcggccccagtcgttggcactttccctga ctgccgaccagatggtgtccgcccttctggacgccgagcc tccaattctgtactcggagtacgatccgactcgcccgttctc cgaagccagcatgatgggcctgttgactaacctggcgga ccgcgagttggtgcacatgattaactgggctaagcgggtg ccgggcttcgtggacctggccctgcacgaccaagtgcac ctcctggaatgcgcctggatggaaatcctcatgatcggcct cgtgtggagatccatggagcatcccggaaagctcctgtttg cacccaacctcctgcttgatcgcaaccagggaaaatgcgt ggaagggggtgtcgagattttcgacatgctgctcgccacct cttcccggttccggatgatgaatctgcagggagaagagttc gtgtgtctgaagtcaatcatcctgctgaactccggggtctat accttcctgagctcgaccctcaagtcactggaggaaaaa gaccacatccatcgcgtgctcgataagatcaccgacacc cttatccatctcatggcgaaggctggactgaccctgcaaca gcagcaccagaggctggcccagttgctgctgattctgagc cacatccggcacatgtcgtccaagaggatggaacacctg tacagcatgaagtgcaagaacgtcgtgcctctgtccgatct gctcctggaaatgctggacgcgcacagactcggaaccg gcgcggaagacccccggccctccaggaagcgaaggtc cctcggagacgtgggtcaagtgcaactcgtccaaagcgg agcggaagtcaagaaacccggagcgagcgtgaaagtg tcctgcaaagcctccggctacacctttacgggctactacat gcactgggtgcgccaggcaccaggacagggtcttgaatg gatgggatggatcaaccctaattcgggcggaactaactac gcacagaagttccaggggagagtgactctgactcgggat acctccatctcaactgtctacatggaactctcccgcttgcgg tcagatgatacggcagtgtactactgcgcccgcgacatga atatcctggctaccgtgccgttcgacatctggggacaggg gactatggttactgtctcatcgggcggtggaggttcaggag gaggcggctcgggaggcggaggttcggacattcagatg acccagtccccatcctctctgtcggccagcgtcggagata gggtgaccattacctgtcgggcctcgcaaagcatctcctcg tacctcaactggtatcagcaaaagccgggaaaggcgcct aagctgctgatctacgccgcttcgagcttgcaaagcgggg tgccatccagattctcgggatcaggctcaggaaccgacttc accctgaccgtgaacagcctccagccggaggactttgcc acttactactgccagcagggagactccgtgccgcttactttc ggggggggtacccgcctggagatcaagaccactacccc agcaccgaggccacccaccccggctcctaccatcgcctc ccagcctctgtccctgcgtccggaggcatgtagacccgca gctggtggggccgtgcatacccggggtcttgacttcgcctg cgatatctacatttgggcccctctggctggtacttgcggggt cctgctgctttcactcgtgatcactctttactgtaagcgcggtc ggaagaagctgctgtacatctttaagcaacccttcatgagg cctgtgcagactactcaagaggaggacggctgttcatgcc ggttcccagaggaggaggaaggcggctgcgaactgcgc gtgaaattcagccgcagcgcagatgctccagcctaccag caggggcagaaccagctctacaacgaactcaatcttggt cggagagaggagtacgacgtgctggacaagcggagag gacgggacccagaaatgggcgggaagccgcgcagaa agaatccccaagagggcctgtacaacgagctccaaaag |
| | | gataagatggcagaagcctatagcgagattggtatgaaa ggggaacgcagaagaggcaaaggccacgacggactgt accagggactcagcaccgccaccaaggacacctatgac gctcttcacatgcaggccctgccgcctcgg (SEQ ID NO: 1102) |
| SP-Linker 6 - ERmut2 - Furin cleavage site 2 - CD123 CAR | MALPVTALLLPLALLLHAARPQ SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGQVQLVQSGA EVKKPGASVKVSCKASGYTFT GYYMHWVRQAPGQGLEWM GWINPNSGGTNYAQKFQGRV TLTRDTSISTVYMELSRLRSD DTAVYYCARDMNILATVPFDI WGQGTMVTVSSGGGGSGGG GSGGGGSDIQMTQSPSSLSA SVGDRVTITCRASQSISSYLN WYQQKPGKAPKLLIYAASSLQ SGVPSRFSGSGSGTDFTLTV NSLQPEDFATYYCQQGDSVP LTFGGGTRLEIKTTTPAPRPPT PAPTIASQPLSLRPEACRPAA GGAVHTRGLDFACDIYIWAPL AGTCGVLLLSLVITLYCKRGRK KLLYIFKQPFMRPVQTTQEED GCSCRFPEEEEGGCELRVKF SRSADAPAYQQGQNQLYNEL NLGRREEYDVLDKRRGRDPE MGGKPRRKNPQEGLYNELQK DKMAEAYSEIGMKGERRRGK GHDGLYQGLSTATKDTYDAL HMQALPPR (SEQ ID NO: 1043) | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtcaagtgcaactcgtccaaagcg gagcggaagtcaagaaacccggagcgagcgtgaaagt gtcctgcaaagcctccggctacacctttacgggctactaca tgcactgggtgcgccaggcaccaggacagggtcttgaat ggatgggatggatcaaccctaattcgggcggaactaacta cgcacagaagttccaggggagagtgactctgactcggga tacctccatctcaactgtctacatggaactctcccgcttgcg gtcagatgatacggcagtgtactactgcgcccgcgacatg aatatcctggctaccgtgccgttcgacatctggggacagg ggactatggttactgtctcatcgggcggtggaggttcagga ggaggcggctcgggaggcggaggttcggacattcagat gacccagtccccatcctctctgtcggccagcgtcggagat agggtgaccattacctgtcgggcctcgcaaagcatctcctc gtacctcaactggtatcagcaaaagccgggaaaggcgc ctaagctgctgatctacgccgcttcgagcttgcaaagcgg ggtgccatccagattctcgggatcaggctcaggaaccga cttcaccctgaccgtgaacagcctccagccggaggacttt gccacttactactgccagcagggagactccgtgccgctta ctttcggggggggtacccgcctggagatcaagaccacta ccccagcaccgaggccacccaccccggctcctaccatcg cctcccagcctctgtccctgcgtccggaggcatgtagaccc gcagctggtggggccgtgcatacccggggtcttgacttcg cctgcgatatctacatttgggcccctctggctggtacttgcgg ggtcctgctgctttcactcgtgatcactctttactgtaagcgcg gtcggaagaagctgctgtacatctttaagcaacccttcatg aggcctgtgcagactactcaagaggaggacggctgttcat gccggttcccagaggaggaggaaggcggctgcgaactg cgcgtgaaattcagccgcagcgcagatgctccagcctac cagcaggggcagaaccagctctacaacgaactcaatctt |
| | | ggtcggagagaggagtacgacgtgctggacaagcgga gaggacgggacccagaaatgggcgggaagccgcgca gaaagaatccccaagagggcctgtacaacgagctccaa aaggataagatggcagaagcctatagcgagattggtatg aaaggggaacgcagaagaggcaaaggccacgacgg actgtaccagggactcagcaccgccaccaaggacacct atgacgctcttcacatgcaggccctgccgcctcgg (SEQ ID NO: 1103) |
| SP-Linker 6 - ERmut1 - Furin cleavage site 3 - CD123 CAR | MALPVTALLLPLALLLHAARPQ SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTI ASQPLSLRPEACRPAAGG AVHTRGLDFACDIYIWAPLA GTCGVLLLSLVITLYCKRGR KKLLYIFKQPFMRPVQTTQ EEDGCSCRFPEEEEGGCE LRVKFSRSADAPAYQQGQ NQLYNELNLGRREEYDVLD KRRGRDPEMGGKPRRKNP QEGLYNELQKDKMAEAYS EIGMKGERRRGKGHDGLY QGLSTATKDTYDALHMQAL PPR (SEQ ID NO: 1044) | atggccctccctgtcaccgccctgctgcttccgctggctcttc tgctccacgccgctcggccccagtcgttggcactttccctga ctgccgaccagatggtgtccgcccttctggacgccgagcct ccaattctgtactcggagtacgatccgactcgcccgttctcc gaagccagcatgatgggcctgttgactaacctggcggacc gcgagttggtgcacatgattaactgggctaagcgggtgccg ggcttcgtggacctggccctgcacgaccaagtgcacctcct ggaatgcgcctggatggaaatcctcatgatcggcctcgtgt ggagatccatggagcatcccggaaagctcctgtttgcaccc aacctcctgcttgatcgcaaccagggaaaatgcgtggaagg gggtgtcgagattttcgacatgctgctcgccacctcttccc ggttccggatgatgaatctgcagggagaagagttcgtgtgt ctgaagtcaatcatcctgctgaactccggggtctataccttc ctgagctcgaccctcaagtcactggaggaaaaagaccaca tccatcgcgtgctcgataagatcaccgacacccttatccatc tcatggcgaaggctggactgaccctgcaacagcagcacca gaggctggcccagttgctgctgattctgagccacatccggc acatgtcgtccaagaggatggaacacctgtacagcatgaa gtgcaagaacgtcgtgcctctgtccgatctgctcctggaaat gctggacgcgcacagactcggaaccggcgcggaagaccc ccggccctccaggaagcgaaggcaagtgcaactcgtccaa agcggagcggaagtcaagaaacccggagcgagcgtgaaa gtgtcctgcaaagcctccggctacacctttacgggctactac atgcactgggtgcgccaggcaccaggacagggtcttgaat ggatgggatggatcaaccctaattcgggcggaactaacta cgcacagaagttccaggggagagtgactctgactcgggat acctccatctcaactgtctacatggaactctcccgcttgcgg tcagatgatacggcagtgtactactgcgcccgcgacatgaa tatcctggctaccgtgccgttcgacatctggggacagggga ctatggttactgtctcatcgggcggtggaggttcaggagga ggcggctcgggaggcggaggttcggacattcagatgaccc agtccccatcctctctgtcggccagcgtcggagatagggtg accattacctgtcgggcctcgcaaagcatctcctcgtacctc aactggtatcagcaaaagccgggaaaggcgcctaagctgc tgatctacgccgcttcgagcttgcaaagcggggtgccatcc agattctcgggatcaggctcaggaaccgacttcaccctgac cgtgaacagcctccagccggaggactttgccacttactact gccagcagggagactccgtgccgcttactttcggggggggt acccgcctggagatcaagaccactaccccagcaccgaggc cacccaccccggctcctaccatcgcctcccagcctctgtccc tgcgtccggaggcatgtagacccgcagctggtggggccgt |
| | | gcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagactac tcaagaggaggacggctgttcatgccggttcccagaggag gaggaaggcggctgcgaactgcgcgtgaaattcagccgca gcgcagatgctccagcctaccagcaggggcagaaccagct ctacaacgaactcaatcttggtcggagagaggagtacgac gtgctggacaagcggagaggacgggacccagaaatgggc gggaagccgcgcagaaagaatccccaagagggcctgtac aacgagctccaaaaggataagatggcagaagcctatagc gagattggtatgaaaggggaacgcagaagaggcaaaggc cacgacggactgtaccagggactcagcaccgccaccaagg acacctatgacgctcttcacatgcaggccctgccgcctcgg (SEQ ID NO: 1104) |
| SP-Linker 6 - ERmut2 - Furin cleavage site 3 - CD123 CAR | MALPVTALLLPLALLLHAARPQ SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRQVQLVQSGAEVKKPG ASVKVSCKASGYTFTGYYMH WVRQAPGQGLEWMGWINPN SGGTNYAQKFQGRVTLTRDT SISTVYMELSRLRSDDTAVYY CARDMNILATVPFDIWGQGTM VTVSSGGGGSGGGGSGGGG SDIQMTQSPSSLSASVGDRVT ITCRASQSISSYLNWYQQKPG KAPKLLIYAASSLQSGVPSRFS GSGSGTDFTLTVNSLQPEDFA TYYCQQGDSVPLTFGGGTRL EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR (SEQ ID NO: 1045) | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggc aagtgcaactcgtccaaagcggagcggaagtcaagaaa cccggagcgagcgtgaaagtgtcctgcaaagcctccggc tacacctttacgggctactacatgcactgggtgcgccaggc accaggacagggtcttgaatggatgggatggatcaaccct aattcgggcggaactaactacgcacagaagttccagggg agagtgactctgactcgggatacctccatctcaactgtctac atggaactctcccgcttgcggtcagatgatacggcagtgta ctactgcgcccgcgacatgaatatcctggctaccgtgccgt tcgacatctggggacaggggactatggttactgtctcatcg ggcggtggaggttcaggaggaggcggctcgggaggcg gaggttcggacattcagatgacccagtccccatcctctctgt cggccagcgtcggagatagggtgaccattacctgtcggg cctcgcaaagcatctcctcgtacctcaactggtatcagcaa aagccgggaaaggcgcctaagctgctgatctacgccgct tcgagcttgcaaagcggggtgccatccagattctcgggat caggctcaggaaccgacttcaccctgaccgtgaacagcc |
| | | tccagccggaggactttgccacttactactgccagcaggg agactccgtgccgcttactttcggggggggtacccgcctgg agatcaagaccactaccccagcaccgaggccacccacc ccggctcctaccatcgcctcccagcctctgtccctgcgtccg gaggcatgtagacccgcagctggtggggccgtgcatacc cggggtcttgacttcgcctgcgatatctacatttgggcccctc tggctggtacttgcggggtcctgctgctttcactcgtgatcac tctttactgtaagcgcggtcggaagaagctgctgtacatcttt aagcaacccttcatgaggcctgtgcagactactcaagag gaggacggctgttcatgccggttcccagaggaggaggaa ggcggctgcgaactgcgcgtgaaattcagccgcagcgc agatgctccagcctaccagcaggggcagaaccagctcta caacgaactcaatcttggtcggagagaggagtacgacgt gctggacaagcggagaggacgggacccagaaatgggc gggaagccgcgcagaaagaatccccaagagggcctgt acaacgagctccaaaaggataagatggcagaagcctat agcgagattggtatgaaaggggaacgcagaagaggca aaggccacgacggactgtaccagggactcagcaccgcc accaaggacacctatgacgctcttcacatgcaggccctgc cgcctcgg (SEQ ID NO: 1105) |
| SP-Linker 6 - ERmut1 - Furin cleavage site 2 - BCMA CAR | MALPVTALLLPLALLLHAARPQ SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG LSTATKDTYDALHMQALPPR | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc atctacgccgcctcgagcctgcagtcaggagtgccctcac |
| | (SEQ ID NO: 1046) | ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1106) |
| SP-Linker 6 - ERmut2 - Furin cleavage site 2-BCMA CAR | MALPVTALLLPLALLLHAARPQ SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRRSLGDVGEVQLVESGG GLVQPGGSLRLSCAVSGFALS NHGMSWVRRAPGKGLEWVS GIVYSGSTYYAASVKGRFTISR DNSRNTLYLQMNSLRPEDTAI YYCSAHGGESDVWGQGTTVT VSSASGGGGSGGRASGGGG SDIQLTQSPSSLSASVGDRVTI TCRASQSISSYLNWYQQKPG KAPKLLIYAASSLQSGVPSRFS GSGSGTDFTLTISSLQPEDFA TYYCQQSYSTPYTFGQGTKV EIKTTTPAPRPPTPAPTIASQP LSLRPEACRPAAGGAVHTRG LDFACDIYIWAPLAGTCGVLLL SLVITLYCKRGRKKLLYIFKQP FMRPVQTTQEEDGCSCRFPE EEEGGCELRVKFSRSADAPA YQQGQNQLYNELNLGRREEY DVLDKRRGRDPEMGGKPRRK NPQEGLYNELQKDKMAEAYS EIGMKGERRRGKGHDGLYQG | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcggaacc ggcgcggaagacccccggccctccaggaagcgaaggt ccctcggagacgtgggtgaagtgcaattggtggaatcag ggggaggacttgtgcagcctggaggatcgctgagactgtc atgtgccgtgtccggctttgccctgtccaaccacgggatgtc ctgggtccgccgcgcgcctggaaagggcctcgaatgggt gtcgggtattgtgtacagcggtagcacctactatgccgcat ccgtgaaggggagattcaccatcagccgggacaactcc aggaacactctgtacctccaaatgaattcgctgaggccag aggacactgccatctactactgctccgcgcatggcggaga gtccgacgtctggggacaggggaccaccgtgaccgtgtc tagcgcgtccggcggaggcggcagcgggggtcgggcat cagggggcggcggatcggacatccagctcacccagtcc ccgagctcgctgtccgcctccgtgggagatcgggtcacca tcacgtgccgcgccagccagtcgatttcctcctacctgaac tggtaccaacagaagcccggaaaagccccgaagcttctc |
| | LSTATKDTYDALHMQALPPR (SEQ ID NO: 1047) | atctacgccgcctcgagcctgcagtcaggagtgccctcac ggttctccggctccggttccggtactgatttcaccctgaccat ttcctccctgcaaccggaggacttcgctacttactactgcca gcagtcgtactccaccccctacactttcggacaaggcacc aaggtcgaaatcaagaccactaccccagcaccgaggcc acccaccccggctcctaccatcgcctcccagcctctgtccc tgcgtccggaggcatgtagacccgcagctggtggggccg tgcatacccggggtcttgacttcgcctgcgatatctacatttg ggcccctctggctggtacttgcggggtcctgctgctttcactc gtgatcactctttactgtaagcgcggtcggaagaagctgct gtacatctttaagcaacccttcatgaggcctgtgcagacta ctcaagaggaggacggctgttcatgccggttcccagagg aggaggaaggcggctgcgaactgcgcgtgaaattcagc cgcagcgcagatgctccagcctaccagcaggggcagaa ccagctctacaacgaactcaatcttggtcggagagagga gtacgacgtgctggacaagcggagaggacgggaccca gaaatgggcgggaagccgcgcagaaagaatccccaag agggcctgtacaacgagctccaaaaggataagatggca gaagcctatagcgagattggtatgaaaggggaacgcag aagaggcaaaggccacgacggactgtaccagggactc agcaccgccaccaaggacacctatgacgctcttcacatg caggccctgccgcctcgg (SEQ ID NO: 1107) |
| SP-Linker 6 - ERmut1 - Furin cleavage site 3 - BCMA CAR | MALPVTALLLPLALLLHAARPQ SLALSLTADQMVSALLDAEPPI LYSEYDPTRPFSEASMMGLLT NLADRELVHMINWAKRVPGF VDLALHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SSKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG LYNELQKDKMAEAYSEIGMKG | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctggccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgtccaagaggatggaacacct gtacagcatgaagtgcaagaacgtcgtgcctctgtccgat ctgctcctggaaatgctggacgcgcacagactcGggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc cagtcgatttcctcctacctgaactggtaccaacagaagcc |
| | ERRRGKGHDGLYQGLSTATK DTYDALHMQALPPR (SEQ ID NO: 1048) | cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1108) |
| SP-Linker 6 - ERmut2 - Furin cleavage site 3-BCMA CAR | MALPVTALLLPLALLLHAARPQ SLALSLTADQMVSALLDAEPPI LYSEYDPTRPFSEASMMGLLT NLADRELVHMINWAKRVPGF VDLTLHDQVHLLECAWMEILM IGLVWRSMEHPGKLLFAPNLL LDRNQGKCVEGGVEIFDMLLA TSSRFRMMNLQGEEFVCLKSI ILLNSGVYTFLSSTLKSLEEKD HIHRVLDKITDTLIHLMAKAGLT LQQQHQRLAQLLLILSHIRHM SNKRMEHLYSMKCKNVVPLS DLLLEMLDAHRLGTGAEDPRP SRKRREVQLVESGGGLVQPG GSLRLSCAVSGFALSNHGMS WVRRAPGKGLEWVSGIVYSG STYYAASVKGRFTISRDNSRN TLYLQMNSLRPEDTAIYYCSA HGGESDVWGQGTTVTVSSAS GGGGSGGRASGGGGSDIQLT QSPSSLSASVGDRVTITCRAS QSISSYLNWYQQKPGKAPKLL IYAASSLQSGVPSRFSGSGSG TDFTLTISSLQPEDFATYYCQ QSYSTPYTFGQGTKVEIKTTT PAPRPPTPAPTIASQPLSLRPE ACRPAAGGAVHTRGLDFACDI YIWAPLAGTCGVLLLSLVITLY CKRGRKKLLYIFKQPFMRPVQ TTQEEDGCSCRFPEEEEGGC ELRVKFSRSADAPAYQQGQN QLYNELNLGRREEYDVLDKR RGRDPEMGGKPRRKNPQEG | atggccctccctgtcaccgccctgctgcttccgctggctcttc tgctccacgccgctcggccccagTcgttggcactttccctg actgccgaccagatggtgtccgcccttctggacgccgagc ctccaattctgtactcggagtacgatccgactcgcccgttct ccgaagccagcatgatgggcctgttgactaacctggcgg accgcgagttggtgcacatgattaactgggctaagcgggt gccgggcttcgtggacctgaccctgcacgaccaagtgca cctcctggaatgcgcctggatggaaatcctcatgatcggcc tcgtgtggagatccatggagcatcccggaaagctcctgttt gcacccaacctcctgcttgatcgcaaccagggaaaatgc gtggaagggggtgtcgagattttcgacatgctgctcgccac ctcttcccggttccggatgatgaatctgcagggagaagagt tcgtgtgtctgaagtcaatcatcctgctgaactccggggtct ataccttcctgagctcgaccctcaagtcactggaggaaaa agaccacatccatcgcgtgctcgataagatcaccgacac ccttatccatctcatggcgaaggctggactgaccctgcaac agcagcaccagaggctggcccagttgctgctgattctgag ccacatccggcacatgtcgaacaagaggatggaacacc tgtacagcatgaagtgcaagaacgtcgtgcctctgtccgat ctgctcctggaaatgctggacgcgcacagactcgggacg ggagctgaagatccacgacccagcagaaagcgacggg aagtgcaattggtggaatcagggggaggacttgtgcagc ctggaggatcgctgagactgtcatgtgccgtgtccggctttg ccctgtccaaccacgggatgtcctgggtccgccgcgcgcc tggaaagggcctcgaatgggtgtcgggtattgtgtacagc ggtagcacctactatgccgcatccgtgaaggggagattca ccatcagccgggacaactccaggaacactctgtacctcc aaatgaattcgctgaggccagaggacactgccatctacta ctgctccgcgcatggcggagagtccgacgtctggggaca ggggaccaccgtgaccgtgtctagcgcgtccggcggag gcggcagcgggggtcgggcatcagggggcggcggatc ggacatccagctcacccagtccccgagctcgctgtccgcc tccgtgggagatcgggtcaccatcacgtgccgcgccagc |
| | LYNELQKDKMAEAYSEIGMKG ERRRGKGHDGLYQGLSTATK DTYDALHMQALPPR (SEQ ID NO: 1049) | cagtcgatttcctcctacctgaactggtaccaacagaagcc cggaaaagccccgaagcttctcatctacgccgcctcgag cctgcagtcaggagtgccctcacggttctccggctccggtt ccggtactgatttcaccctgaccatttcctccctgcaaccgg aggacttcgctacttactactgccagcagtcgtactccacc ccctacactttcggacaaggcaccaaggtcgaaatcaag accactaccccagcaccgaggccacccaccccggctcct accatcgcctcccagcctctgtccctgcgtccggaggcatg tagacccgcagctggtggggccgtgcatacccggggtctt gacttcgcctgcgatatctacatttgggcccctctggctggta cttgcggggtcctgctgctttcactcgtgatcactctttactgta agcgcggtcggaagaagctgctgtacatctttaagcaacc cttcatgaggcctgtgcagactactcaagaggaggacgg ctgttcatgccggttcccagaggaggaggaaggcggctg cgaactgcgcgtgaaattcagccgcagcgcagatgctcc agcctaccagcaggggcagaaccagctctacaacgaac tcaatcttggtcggagagaggagtacgacgtgctggaca agcggagaggacgggacccagaaatgggcgggaagc cgcgcagaaagaatccccaagagggcctgtacaacga gctccaaaaggataagatggcagaagcctatagcgaga ttggtatgaaaggggaacgcagaagaggcaaaggcca cgacggactgtaccagggactcagcaccgccaccaagg acacctatgacgctcttcacatgcaggccctgccgcctcg g (SEQ ID NO: 1109) |

### EXAMPLE 29: TEMPORALLY CONTROLLED TOTAL B CELL DEPLETION.

The materials and methods employed in these experiments are now described.

*Construction of CAR constructs including suicide genes.* Constructs using the FMC63 (anti-CD19) single-chain variable fragment (scFv) used the previously described vector (pRRLSIN.cPPT.gene-of interest-WPRE with a human EF1alpha promoter), which was modified for caspase-9 expression as follows:
1. For expression of the inducible caspase-9 3' of the FMC63-bbz CAR, the transmembrane domain and the cytoplasmic tail were removed by digestion with EcoRV and Sail and replaced with a geneblock (IDT), that encoded the same transmembrane domain and cytoplasmic tail followed by a 2A site and FKBP12F36V followed by a short GS-linker and the caspase-9 promolecule domain.
2. For expression of the inducible caspase-9 5' of the FMC63-bbz CAR, the signal peptide and the part of FMC63 ScFv were removed by digestion with Xbal and Tth111I and replaced with a geneblock (IDT), that encoded the same inducible caspase-9 domain as described in 1. followed by a 2A site and the sequence encoding the previously removed part of the original plasmid.
3. For expression of the reversible caspase-9 3' of the FMC63-bbz CAR, the transmembrane domain and the cytoplasmic tail were removed by digestion with EcoRV and Sail and replaced with a geneblock (geneart, Thermofisher), that encoded the same transmembrane domain and cytoplasmic tail followed by a 2A site and 2 repeats of FKBP12F36M followed by a short GS-linker (SGGGS, SEQ ID NO:3036) and the caspase-9 promolecule domain.
4. For expression of an anti-human CD20 ScFv, the FMC63 ScFv and the CD8 hinge were removed from the constructs described in 1-3 and replaced with a codon-optimized gene block (IDT) encoding the amino acid sequence of veltuzumab.

*Construction of CAR constructs including conditional aggregation domains.* For expression of the conditional aggregation domains 5' of the FMC63-bbz CAR, the plasmid was cut with BamHI between signal peptide and ScFv and a gene block encoding (geneart) 4 repeats of FKBP12F36M followed by a furin recognition sequence were inserted between signal peptide and ScFv.

*Lentiviral production.* VSV-G pseudotyped lentiviral particles were produced using a 4th generation packaging system. 293T cells were transfected at a confluency of 90% with a mixture of the pRRLSIN.cPPT.EF1α-gene-of -interest.WPRE, the envelope plasmid pMD2.G (Addgene #12252), the packaging plasmids pRSVRev (Addgene #12253) and pMDLgm/pRRE (Addgene #12251) in a complex with Lipofectamine 2000 (Life Technologies). Lentivirus containing supernatant was harvested after 24 and 48 hours, filtered through a 0.4 micron PES membrane, concentrated at 12,000xg for 12 hours at 4°C and stored at -80°C.

*Stimulation and expansion of primary human T cells.* Primary human T cells were cultured in RPMI1640, 10% FBS, 10 mM HEPES, 1% penicillin/streptomycin. Bulk T cells (CD4⁺ and CD8⁺) were stimulated with anti-CD3 and anti-CD28 beads (Dynabeads, Life Technologies) at a bead:cell ratio of 3:1. The culture medium was supplemented with 100 IU/mL interleukin 2. 24 hours after stimulation, 10⁶ T cells were transduced with lentiviral particles encoding various CAR constructs or control constructs or mock transduced. Expansion of the T cells was monitored for 8-12 days by measurement of cell volume and concentration (Coulter counter, Beckman Coulter). Cell surface expression of the CAR constructs was validated by flow cytometry (BD LSRII) with polyconal antibodies against the human or mouse heavy and light chain. CAR staining was performed for 25 minutes at room temperature. All staining procedures were performed under saturating conditions. For some experiments, transduced T cells were previously frozen in 90% FBS and 10% DMSO. Experiments were performed when cells were rested after stimulation (i.e. volume of <450fl).

*In vitro cytotoxicity and cytokine assays. In vitro* killing of Nalm-6 cells was tested with a ⁵¹Cr-release assay. 5×10⁵ target cells were loaded with 50 □Ci of Na₂⁵¹CrO₄ (Perkin Elmer) for 90-120 minutes, washed twice and resuspended in phenol red-free medium with 5% FBS. CAR, control CAR, or mock transduced T cells (8-10 days after initial activation) were co-incubated with loaded target cells for 4 hours at various effector:target (E:T) ratios, and chromium release into the supernatant was measured with a MicroBeta2 plate counter (Perkin Elmer). Spontaneous release by target cells (without effector cells) was analyzed in the same volume, and maximum release was assessed by lysing target cells with SDS at a final concentration of 5%. Percent specific lysis = [(Experimental Release - Spontaneous Release)/ (Maximum Release - Spontaneous Release)] *100. All experiments were performed with at least 3 replicates.

*Elimination of CD19 CAR T cells.* CD19 sCAR T cells were incubated in the presence of the indicated concentrations AP20187 for 16 hours at 37°C. Dead cells were detected with live/dead-violet dye and quantified by flow cytometry.

*CD19 sCAR T Cells Retain Biologic Efficacy In Vivo.* Mice were pretreated with IVIG (Privigen, CSL Behring) at a dose of 600 mg/kg i.v. for 2 days in order to block Fc□Rs on monocytes and neutrophils. NSG mice were then injected with 1×10⁶ CD19+ Nalm6 cells expressing click-beetle luciferase to allow for bioluminescence detection. Five days later, mice were treated either with 5×10⁶ CD19 CAR T cells that express an inducible caspase9 or with nontransduced control T cells. Peripheral blood was obtained by retro-orbital bleeding, and the presence of B cells and T-cell engraftment was determined by flow cytometry using BD TruCOUNT (BD Biosciences) tubes as described in the manufacturer's instructions. Bioluminescence was assessed on day 5, 6, 7, 10, 13 and 18 after B cell injection, which was combined with intraperitoneally administered IVIG (600 mg/kg). Mice were euthanized for organ harvest according to local IACUC guidelines, and bone marrow, spleen and blood samples were assessed by flow cytometry.

The results of the experiments are now described.

B cell depletion, if 100% complete, should be curative in pemphigus vulgaris (PV) and other antibody-mediated diseases, because the pathogenic B cells should be eliminated, and a brand new B cell repertoire must form. Given the multiple tolerance checkpoints that prevent autoimmunity, it is unlikely that B cell autoreactivity will recur.

The advantage of the CD19 CAR T cell approach is that it is a universal approach to the treatment of any B cell or antibody-mediated disease and has been validated in human clinical trials to be highly effective at CD19⁺ B cell depletion. Thus, the target antigen for the disease does not need to be known. This therapeutic approach could be applied to any disease in which B cells or the antibodies they produce cause autoimmunity or disease. In addition, the CD19 CAR could be used to eliminate alloantibodies to non-self proteins that prevent functional rescue of genetic diseases, such as HLA or beta2 microglobulin antibodies from organ transplant, blood group or Rh antibodies from transfusion or pregnancy, or Factor VIII, α-L-iduronidase, and many other protein replacement therapies in clinical use or development.

CD19 CAR T cells have shown efficacy for B cell depletion in human clinical trials without significant off-target cytotoxicity, although B cell depletion is permanent, resulting in lifelong immunosuppression. CD20 is also a clinical target for B cell depletion. Thus, the incorporation of a suicide gene into the anti-CD19 or anti-CD20 CAR design (sCAR), or selective activation of CAR-T function (via a reversible suicide casssette or conditional expression of the CAR), will allow temporal control of CAR-mediated B cell depletion as an important safety measure.

An example of a suicide gene in a sCAR includes fusion of human caspase 9 to a modified human FK-binding protein, which allows conditional dimerization upon exposure to a synthetic dimerizing drug such as AP1903. Upon dimerization caspase 9 becomes activated, leading to death of cells expressing the suicide gene. Thus, infusion of CD19 sCAR T cells would result in complete B cell depletion, which would cure B cell- or antibody-mediated disease. Subsequent activation of the suicide gene with AP1903, AP20187, or similar agent would then eliminate the sCAR T cells, allowing B cell repopulation to occur, thereby regenerating normal immune function. An example of a reverse suicide gene in a revCAR includes fusion of human caspase 9 to a modified human FK-binding protein, whose default state is to dimerize and cause death of cells expressing the suicide gene. Upon solubilization of dimerization domains, caspase 9 activity is inhibited and cell death is prevented, allowing CAR T cell function to occur. An example of a regulatory on-CAR system includes fusion of the CAR to modified human FK-binding protein domains, whose default state is to dimerize and cause aggregation and degradation of proteins in the secretory system of the cell. Solubilization of the dimerization domains allows egress and processing of the CAR in the secretory system of the cell, including furin cleavage of the CAR from the FKBP domains to allow functional cell surface expression of the CAR.

To evaluate these approaches for temporal control of CAR T cell function, several constructs were engineered; for one example, one with a suicide gene in front of the CAR and one with the suicide gene after the CAR construct (FIG. 27). For another example, a reverse suicide gene (either in front of or after the CAR construct) (FIG. 28). For yet another example, a regulatory onCAR (FIG. 26). Primary human T cells were shown to efficiently express the CD19 sCARs, CD19 revCAR, and CD20 CAR (FIGS. 29A-29F) and CD19 sCAR T cells demonstrated specific cytotoxicity against their intended targets (FIG. 30). Additionally, primary human T cells expressing the CD19 sCAR were specifically killed by activation of the suicide switch with AP20187 *in vitro,* whereas nontransduced T cells showed no cell death after AP20187 treatment (FIG. 31). Additionally, CD19 sCAR T cells remained effective at eliminating CD19⁺ B cells in an *in vivo* mouse model (FIG. 32) and demonstrated engraftment (FIG. 33).

As seen in the killing assay in FIG. 34, CD20sCAR, 20revCAR and CD20 onCAR show equivalent and specific killing of the CD20+ target cells. On-CAR was tested in presence of 500nM Shield-1.

As seen in FIG. 35, the absence of solubilizing FKBP ligand (e.g. shield-1) inhibits CAR function. At lower E:T ratio's the On-CAR function is strongly inhibited if no FKBP ligand is present.

As seen in FIG. 36, the CAR surface expression could be modulated with FKBP ligand Shield-1. Shield-1 resulted in a dose-dependent increase in CAR expression while the absence of Shield-1 resulted in -60% reduction of CD20 on-CAR expression.

As seen in FIG. 37, Shield-1 was titrated in CD19rev CAR system and lower doses of Shield-1 resulted in higher caspase-9 activation and increased apoptosis.

As seen in FIG. 38, the *in vivo* assessment of apoptosis efficiency in anti CD19 revCAR T cells indicated a sufficient in vivo apoptosis of revCAR T cells (FIG. 38). The *in vivo* efficacy of CD19 revCAR T cells was also evaluated (FIG. 39).

As seen in FIGS. 40, 41 and 42, the suicide gene activation resulted in peripheral depletion of the suicide CAR T cells (e.g. depletion of sCAR T cells from lymphoid organs in FIG. 42).

The timeline for T cell production of the present disclosure is outlined in a schematic in FIG. 43 and the timeline of the experimental design of the *in vivo* experiment of the present disclosure is outlined in a schematic in FIG. 44.

As shown in FIG. 45, 'Universal' sCAR T cells can deplete peripheral B cells in non-autologous BT mice and human B cells in a non-cancer humanized mouse model. Furthermore, FIG. 46 demonstrates that the universal sCAR T cells can be depleted with AP1903 treatment.

The relevant sequences of the some experimental results presented herein are as follows:
**CD19 sCAR:**
   Suicide gene followed by CD19 CAR (nt)
   Suicide gene followed by CD19 CAR (aa)
   CD19 CAR followed by suicide gene (nt)
   CD19 CAR followed by suicide gene (aa)
**CD20 CAR and sCAR:**
   CD20bbz CAR (nt and aa)
   CD20 CAR followed by suicide gene (nt and aa)
   suicide gene followed by CD20 CAR (nt and aa)
**CD19 revCAR:**
   CD19 CAR followed by reversible suicide cassette (nt and aa)
**CD19 onCAR:**
   CD19 on-CAR (nt and aa)
**CD20 revCAR:**
   CD20 CAR followed by reversible suicide cassette (nt and aa)
**CD20 onCAR:**
   CD20 on-CAR (nt and aa)

Suicide gene = inducible caspase9 (activates apoptosis upon dimerization with small molecule activators) 2A site = ribosome skipping site

CD19 CAR, comprising a CD8 transmembrane domain, CD137 and CD3-zeta (CD3-zeta is also known as CD247, collectively the CD137-CD3zeta is also known as bbz) cytoplasmic signaling domains

Inducible caspase9-2A-CD19bbz nucleotide sequence (SEQ ID NO: 3018)

ATGCTCGAGGGGGTTCAGGTGGAGACTATCAGCCCGGGCGACGGACGGACATTCCCAAAGCGCGGGCAGACG
TGTGTGGTGCATTACACCGGGATGCTTGAGGACGGAAAGAAAGTGGACTCTTCCCGAGACCGAAATAAACCAT
TCAAGTTCATGTTGGGCAAGCAGGAGGTTATCAGAGGGTGGGAGGAGGGCGTCGCTCAGATGAGTGTCGGAC
AGAGGGCCAAGCTCACGATCTCCCCTGATTACGCCTACGGGGCAACTGGTCACCCCGGAATCATCCCCCCTCACG
CAACCCTCGTGTTCGACGTCGAGCTGCTCAAACTGGAATCAGGCGGAGGCAGTGGCGCTAGCGGGTTTGGCGA
T

GTCGGTGCCCTTGAAAGCTTGAGAGGAAATGCCGATCTCGCTTACATCTTGAGCATGGAGCCCTGTGGGCACTG
TCTGATCATCAACAATGTTAACTTTTGCCGGGAGTCCGGCCTGCGCACACGCACAGGCTCCAACATTGACTGCGA
AAAACTTCGAAGGAGGTTTAGCTCTCTGCATTTCATGGTAGAGGTGAAGGGGGATCTGACCGCCAAGAAAATG
GTTCTCGCCCTTCTCGAGCTTGCGCAGCAGGACCATGGAGCGCTTGACTGTTGTGTCGTTGTGATACTGAGCCAT
GGCTGTCAGGCTTCCCATCTCCAGTTTCCAGGGGCCGTGTACGGAACCGATGGATGCCCTGTGTCAGTTGAAAA
GATCGTAAACATCTTTAACGGAACATCTTGCCCGAGCCTCGGCGGTAAACCGAAGCTTTTTTTTATCCAGGCCTG
CGGCGGTGAACAGAAAGATCATGGCTTCGAGGTTGCCAGTACCAGCCCTGAAGACGAATCCCCCGGGTCAAAT
CCTGAACCAGATGCGACCCCTTTCCAGGAAGGACTCCGCACTTTTGACCAGCTTGACGCCATTTCCTCCCTGCCAA
CACCTTCCGACATATTTGTAAGCTACTCCACCTTTCCAGGATTCGTGAGCTGGCGCGACCCAAAATCCGGCAGTT
GGTATGTTGAAACCCTGGACGATATTTTCGAACAATGGGCCCACAGTGAGGACCTGCAGTCCCTTCTTCTGCGCG
TAGCCAATGCCGTGTCAGTCAAAGGGATTTACAAGCAGATGCCAGGCTGCTTTAATTTCCTGCGCAAGAAACTG
TTTTTTAAGACCAGTGGCTCCGGCGCAACAAATTTCTCCTTGCTGAAACAGGCAGGCGACGTTGAGGAAAATCCC
GGCCCAATGGCCTTACCAGTGACCGCCTTGCTCCTGCCGCTGGCCTTGCTGCTCCACGCCGCCAGGCCGGACATCCA
GATGACACAGACTACATCCTCCCTGTCTGCCTCTCTGGGAGACAGAGTCACCATCAGTTGCAGGGCAAGTCAGGAC
ATTAGTAAATATTTAAATTGGTATCAGCAGAAACCAGATGGAACTGTTAAACTCCTGATCTACCATACATCAAGATT
ACACTCAGGAGTCCCATCAAGGTTCAGTGGCAGTGGGTCTGGAACAGATTATTCTCTCACCATTAGCAACCTGGAG
CAAGAAGATATTGCCACTTACTTTTGCCAACAGGGTAATACGCTTCCGTACACGTTCGGAGGGGGGACTAAGTTGG
AAATAACAGGTGGCGGTGGCTCGGGCGGTGGTGGGTCGGGTGGCGGCGGATCTGAGGTGAAACTGCAGGAGTC
AGGACCTGGCCTGGTGGCGCCCTCACAGAGCCTGTCCGTCACATGCACTGTCTCAGGGGTCTCATTACCCGACTAT
GGTGTAAGCTGGATTCGCCAGCCTCCACGAAAGGGTCTGGAGTGGCTGGGAGTAATATGGGGTAGTGAAACCAC
ATACTATAATTCAGCTCTCAAATCCAGACTGACCATCATCAAGGACAACTCCAAGAGCCAAGTTTTCTTAAAAATGA
ACAGTCTGCAAACTGATGACACAGCCATTTACTACTGTGCCAAACATTATTACTACGGTGGTAGCTATGCTATGGAC
TACTGGGGTCAAGGAACCTCAGTCACCGTCTCCTCAACCACGACGCCAGCGCCGCGACCACCAACACCGGCGCCCA
CCATCGCGTCGCAGCCCCTGTCCCTGCGCCCAGAGGCGTGCCGGCCAGCGGCGGGGGGCGCAGTGCACACGAGG
GGGCTGGACTTCGCCTGTGATATCTACATCTGGGCGCCCTTGGCCGGGACTTGTGGGGTCCTTCTCCTGTCACTGGT
TATCACCCTTTACTGCAAACGGGGCAGAAAGAAACTCCTGTATATATTCAAACAACCATTTATGAGACCAGTACAAA
CTACTCAAGAGGAAGATGGCTGTAGCTGCCGATTTCCAGAAGAAGAAGGAGGATGTGAACTGAGAGTGAAG
TTCAGCAGGAGCGCAGACGCCCCCGCGTACCAGCAGGGCCAGAACCAGCTCTATAACGAGCTCAATCTAGGACGA
AGAGAGGAGTACGATGTTTTGGACAAGAGACGTGGCCGGGACCCTGAGATGGGGGGAAAGCCGAGAAGGAAGA
ACCCTCAGGAAGGCCTGTACAATGAACTGCAGAAAGATAAGATGGCGGAGGCCTACAGTGAGATTGGGATGAAA
GGCGAGCGCCGGAGGGGCAAGGGGCACGATGGCCTTTACCAGGGTCTCAGTACAGCCACCAAGGACACCTACGA
CGCCCTTCACATGCAGGCCCTGCCCCCTCGCTAA

Inducible caspase9-2A-CD19bbz amino acid sequence (SEQ ID NO: 3019)

MLEGVQVETISPGDGRTFPKRGQTCVVHYTGMLEDGKKVDSSRDRNKPFKFMLGKQEVIRGWEEGVAQMSVGQ
RAKLTISPDYAYGATGHPGIIPPHATLVFDVELLKLESGGGSGASGFDVGALESLRGNADLAYILSMEPCGHCLIINN
VNFCRESGLRTRTGSNIDCEKLRRRFSSLHFMVEVKGDLTAKKMVLALLELAQQDHGALDCCVVVILSHGCQASHLQ
FPGAVYGTDGCPVSVEKIVNIFNGTSCPSLGGKPKLFFIQACGGEQKDHGFEVASTSPEDESPGSNPEPDATPFQEGL
RTFDQLDAISSLPTPSDIFVSYSTFPGFVSWRDPKSGSWYVETLDDIFEQWAHSEDLQSLLLRVANAVSVKGIYKQMP
GCFNFLRKKLFFKTSGSGATNFSLLKQAGDVEENPGPMALPVTALLLPLALLLHAARPDIQMTQTTSSLSASLGDRVTISC

RASQDISKYLNWYQQKPDGTVKLLIYHTSRLHSGVPSRFSGSGSGTDYSLTISNLEQEDIATYFCQQGNTLPYTFGGGTKL
EITGGGGSGGGGSGGGGSEVKLQESGPGLVAPSQSLSVTCTVSGVSLPDYGVSWIRQPPRKGLEWLGVIWGSETTYYN
SALKSRLTIIKDNSKSQVFLKMNSLQTDDTAIYYCAKHYYYGGSYAMDYWGQGTSVTVSSTTTPAPRPPTPAPTIASQPL
SLRPEACRPAAGGAVHTRGLDFACDIYIWAPLAGTCGVLLLSLVITLYCKRGRKKLLYIFKQPFMRPVQTTQEEDGCSCRF
PEEEEGGCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR

CD19bbz -2A-inducible caspase9 nucleotide sequence (SEQ ID NO: 3020)

ATGGCCTTACCAGTGACCGCCTTGCTCCTGCCGCTGGCCTTGCTGCTCCACGCCGCCAGGCCGGACATCCAGATGAC
ACAGACTACATCCTCCCTGTCTGCCTCTCTGGGAGACAGAGTCACCATCAGTTGCAGGGCAAGTCAGGACATTAGT
AAATATTTAAATTGGTATCAGCAGAAACCAGATGGAACTGTTAAACTCCTGATCTACCATACATCAAGATTACACTC
AGGAGTCCCATCAAGGTTCAGTGGCAGTGGGTCTGGAACAGATTATTCTCTCACCATTAGCAACCTGGAGCAAGAA
GATATTGCCACTTACTTTTGCCAACAGGGTAATACGCTTCCGTACACGTTCGGAGGGGGGACTAAGTTGGAAATAA
CAGGTGGCGGTGGCTCGGGCGGTGGTGGGTCGGGTGGCGGCGGATCTGAGGTGAAACTGCAGGAGTCAGGACC
TGGCCTGGTGGCGCCCTCACAGAGCCTGTCCGTCACATGCACTGTCTCAGGGGTCTCATTACCCGACTATGGTGTA
AGCTGGATTCGCCAGCCTCCACGAAAGGGTCTGGAGTGGCTGGGAGTAATATGGGGTAGTGAAACCACATACTAT
AATTCAGCTCTCAAATCCAGACTGACCATCATCAAGGACAACTCCAAGAGCCAAGTTTTCTTAAAAATGAACAGTCT
GCAAACTGATGACACAGCCATTTACTACTGTGCCAAACATTATTACTACGGTGGTAGCTATGCTATGGACTACTGG
GGTCAAGGAACCTCAGTCACCGTCTCCTCAACCACGACGCCAGCGCCGCGACCACCAACACCGGCGCCCACCATCG
CGTCGCAGCCCCTGTCCCTGCGCCCAGAGGCGTGCCGGCCAGCGGCGGGGGGCGCAGTGCACACGAGGGGGCTG
GACTTCGCCTGTGATATCTACATCTGGGCGCCCTTGGCCGGGACTTGTGGGGTCCTTCTCCTGTCACTGGTTATCAC
CCTTTACTGCAAACGGGGCAGAAAGAAACTCCTGTATATATTCAAACAACCATTTATGAGACCAGTACAAACTACTC
AAGAGGAAGATGGCTGTAGCTGCCGATTTCCAGAAGAAGAAGGAGGATGTGAACTGAGAGTGAAGTTCAGC
AGGAGCGCAGACGCCCCCGCGTACCAGCAGGGCCAGAACCAGCTCTATAACGAGCTCAATCTAGGACGAAGAGA
GGAGTACGATGTTTTGGACAAGAGACGTGGCCGGGACCCTGAGATGGGGGGAAAGCCGAGAAGGAAGAACCCT
CAGGAAGGCCTGTACAATGAACTGCAGAAAGATAAGATGGCGGAGGCCTACAGTGAGATTGGGATGAAAGGCG
AGCGCCGGAGGGGCAAGGGGCACGATGGCCTTTACCAGGGTCTCAGTACAGCCACCAAGGACACCTACGACGCCC
TTCACATGCAGGCCCTGCCCCCTCGCTCGGAAGCGACGGGGCTCCGGCGCAACAAATTTCTCCTTGCTGAAACAGG
CAGGCGACGTTGAGGAAAATCCCGGCCCAGGGGTTCAGGTGGAGACTATCAGCCCGGGCGACGGACGGACATTC
CCAAAGCGCGGGCAGACGTGTGTGGTGCATTACACCGGGATGCTTGAGGACGGAAAGAAAGTGGACTCTTCCC
GAGACCGAAATAAACCATTCAAGTTCATGTTGGGCAAGCAGGAGGTTATCAGAGGGTGGGAGGAGGGCGTCG
CTCAGATGAGTGTCGGACAGAGGGCCAAGCTCACGATCTCCCCTGATTACGCCTACGGGGCAACTGGTCACCCC
GGAATCATCCCCCCTCACGCAACCCTCGTGTTCGACGTCGAGCTGCTCAAACTGGAATCAGGCGGAGGCAGTGG
CGGGTTTGGCGATGTCGGTGCCCTTGAAAGCTTGAGAGGAAATGCCGATCTCGCTTACATCTTGAGCATGGAGC
CCTGTGGGCACTGTCTGATCATCAACAATGTTAACTTTTGCCGGGAGTCCGGCCTGCGCACACGCACAGGCTCCA
ACATTGACTGCGAAAAACTTCGAAGGAGGTTTAGCTCTCTGCATTTCATGGTAGAGGTGAAGGGGGATCTGACC
GCCAAGAAAATGGTTCTCGCCCTTCTCGAGCTTGCGCAGCAGGACCATGGAGCGCTTGACTGTTGTGTCGTTGTG
ATACTGAGCCATGGCTGTCAGGCTTCCCATCTCCAGTTTCCAGGGGCCGTGTACGGAACCGATGGATGCCCTGTG
TCAGTTGAAAAGATCGTAAACATCTTTAACGGAACATCTTGCCCGAGCCTCGGCGGTAAACCGAAGCTTTTTTTT
ATCCAGGCCTGCGGCGGTGAACAGAAAGATCATGGCTTCGAGGTTGCCAGTACCAGCCCTGAAGACGAATCCCC

CGGGTCAAATCCTGAACCAGATGCGACCCCTTTCCAGGAAGGACTCCGCACTTTTGACCAGCTTGACGCCATTTC
CTCCCTGCCAACACCTTCCGACATATTTGTAAGCTACTCCACCTTTCCAGGATTCGTGAGCTGGCGCGACCCAAAA
TCCGGCAGTTGGTATGTTGAAACCCTGGACGATATCTTCGAACAATGGGCCCACAGTGAGGACCTGCAGTCCCTT
CTTCTGCGCGTAGCCAATGCCGTGTCAGTCAAAGGGATTTACAAGCAGATGCCAGGCTGCTTTAATTTCCTGCGC
AAGAAACTGTTTTTTAAGACCAGTTGAGTCGACGGAGGAGGAG

CD19bbz -2A-inducible caspase9 amino acid sequence (SEQ ID NO: 3021)

MALPVTALLLPLALLLHAARPDIQMTQTTSSLSASLGDRVTISCRASQDISKYLNWYQQKPDGTVKLLIYHTSRLHSGVPS
RFSGSGSGTDYSLTISNLEQEDIATYFCQQGNTLPYTFGGGTKLEITGGGGSGGGGSGGGGSEVKLQESGPGLVAPSQSL
SVTCTVSGVSLPDYGVSWIRQPPRKGLEWLGVIWGSETTYYNSALKSRLTIIKDNSKSQVFLKMNSLQTDDTAIYYCAKH
YYYGGSYAMDYWGQGTSVTVSSTTTPAPRPPTPAPTIASQPLSLRPEACRPAAGGAVHTRGLDFACDIYIWAPLAGTCG
VLLLSLVITLYCKRGRKKLLYIFKQPFMRPVQTTQEEDGCSCRFPEEEEGGCELRVKFSRSADAPAYQQGQNQLYNELNL
GRREEYDVLDKRRGRDPEMGGKPRRKNPQEGLYNELQKDKMAEAYSEIGMKGERRRGKGHDGLYQGLSTATKDTYD
ALHMQALPPRRKRRGSGATNFSLLKQAGDVEENPGPGVQVETISPGDGRTFPKRGQTCVVHYTGMLEDGKKVDSSR
DRNKPFKFMLGKQEVIRGWEEGVAQMSVGQRAKLTISPDYAYGATGHPGIIPPHATLVFDVELLKLESGGGSGGFG
DVGALESLRGNADLAYILSMEPCGHCLIINNVNFCRESGLRTRTGSNIDCEKLRRRFSSLHFMVEVKGDLTAKKMVLA
LLELAQQDHGALDCCVVVILSHGCQASHLQFPGAVYGTDGCPVSVEKIVNIFNGTSCPSLGGKPKLFFIQACGGEQKD
HGFEVASTSPEDESPGSNPEPDATPFQEGLRTFDQLDAISSLPTPSDIFVSYSTFPGFVSWRDPKSGSWYVETLDDIFE
QWAHSEDLQSLLLRVANAVSVKGIYKQMPGCFNFLRKKLFFKTS

Signal peptide-Anti-human CD20-CD8 hinge-CD8transmembrane-CD137-CD247 nucleotide sequence
(SEQ ID NO:3022)

ATGGCCTTACCAGTGACCGCCTTGCTCCTGCCGCTGGCCTTGCTGCTCCACGCCGCCAGGCCGGGATCCGATATTC
AACTTACACAGTCCCCTAGTTCCTTGTCCGCCTCCGTCGGCGACAGAGTTACCATGACTTGTCGAGCGTCTTCTAGC
GTTTCCTACATTCATTGGTTCCAACAGAAGCCCGGAAAGGCACCAAAACCTTGGATCTACGCCACGTCCAATTTGG
CTAGTGGTGTTCCGGTGCGATTTTCCGGGTCAGGTAGCGGGACGGACTATACTTTTACCATTTCAAGCCTTCAGCC
TGAAGACATCGCGACGTACTATTGTCAGCAGTGGACCTCTAACCCTCCTACCTTTGGCGGTGGAACAAAGCTGGA
GATCAAACGAGGCGGGGGTGGCTCTGGGGGAGGAGGTTCCGGTGGGGGAGGCTCTCAAGTACAACTGCAACAA
AGTGGGGCTGAAGTGAAGAAGCCCGGTTCTTCAGTCAAAGTATCATGTAAGGCAAGTGGTTATACTTTTACGTCTT
ATAACATGCATTGGGTAAAGCAAGCCCCTGGTCAGGGCCTCGAGTGGATTGGTGCGATCTACCCTGGAATGGGTG
ATACGAGCTATAATCAGAAATTCAAGGGGAAAGCCACCTTGACTGCAGACGAATCTACGAACACGGCTTACATGG
AGCTTAGCTCACTCAGATCAGAGGATACAGCCTTTTACTACTGCGCTAGATCAACTTATTACGGAGGAGACTGGTA
TTTTGATGTATGGGGTCAGGGGACCACAGTCACTGTTAGCTCTGCTAGCACCACGACGCCAGCGCCGCGACCACC
AACACCGGCGCCCACCATCGCGTCGCAGCCCCTGTCCCTGCGCCCAGAGGCGTGCCGGCCAGCGGCGGGGGGCG
CAGTGCACACGAGGGGGCTGGACTTCGCCTGTGATTCCGGAATCTACATCTGGGCGCCCTTGGCCGGGACTTGTG
GGGTCCTTCTCCTGTCACTGGTTATCACCCTTTACTGCAAACGGGGCAGAAAGAAACTCCTGTATATATTCAAACAA
CCATTTATGAGACCAGTACAAACTACTCAAGAGGAAGATGGCTGTAGCTGCCGATTTCCAGAAGAAGAAGAAGGA
GGATGTGAACTGAGAGTGAAGTTCAGCAGGAGCGCAGACGCCCCCGCGTACCAGCAGGGCCAGAACCAGCTCTA

TAACGAGCTCAATCTAGGACGAAGAGAGGAGTACGATGTTTTGGACAAGAGACGTGGCCGGGACCCTGAGATGG
GGGGAAAGCCGAGAAGGAAGAACCCTCAGGAAGGCCTGTACAATGAACTGCAGAAAGATAAGATGGCGGAGGC
CTACAGTGAGATTGGGATGAAAGGCGAGCGCCGGAGGGGCAAGGGGCACGATGGCCTTTACCAGGGTCTCAGTA
CAGCCACCAAGGACACCTACGACGCCCTTCACATGCAGGCCCTGCCCCCTCGC

Signal peptide-Anti-human CD20-CD8 hinge-CD8transmembrane-CD137-CD247 amino acid sequence
(SEQ ID NO: 3023)

MALPVTALLLPLALLLHAARPGSDIQLTQSPSSLSASVGDRVTMTCRASSSVSYIHWFQQKPGKAPKPWIYATSNLASG
VPVRFSGSGSGTDYTFTISSLQPEDIATYYCQQWTSNPPTFGGGTKLEIKRGGGGSGGGGSGGGGSQVQLQQSGAEVK
KPGSSVKVSCKASGYTFTSYNMHWVKQAPGQGLEWIGAIYPGMGDTSYNQKFKGKATLTADESTNTAYMELSSLRSE
DTAFYYCARSTYYGGDWYFDVWGQGTTVTVSSASTTTPAPRPPTPAPTIASQPLSLRPEACRPAAGGAVHTRGLDFAC
DSGIYIWAPLAGTCGVLLLSLVITLYCKRGRKKLLYIFKQPFMRPVQTTQEEDGCSCRFPEEEEGGCELRVKFSRSADAPAY
QQGQNQLYNELNLGRREEYDVLDKRRGRDPEMGGKPRRKNPQEGLYNELQKDKMAEAYSEIGMKGERRRGKGHDG
LYQGLSTATKDTYDALHMQALPPRZ

Anti-human CD20bbz-2A-iCasp9 nucleotide sequence (SEQ ID NO: 3024)

ATGGCTCTGCCTGTGACAGCTCTGCTGCTGCCTCTGGCCCTGCTGCTGCATGCTGCCAGACCTGGCTCCGATATTCA
ACTTACACAGTCCCCTAGTTCCTTGTCCGCCTCCGTCGGCGACAGAGTTACCATGACTTGTCGAGCGTCTTCTAGCG
TTTCCTACATTCATTGGTTCCAACAGAAGCCCGGAAAGGCACCAAAACCTTGGATCTACGCCACGTCCAATTTGGCT
AGTGGTGTTCCGGTGCGATTTTCCGGGTCAGGTAGCGGGACGGACTATACTTTTACCATTTCAAGCCTTCAGCCTG
AAGACATCGCGACGTACTATTGTCAGCAGTGGACCTCTAACCCTCCTACCTTTGGCGGTGGAACAAAGCTGGAGAT
CAAACGAGGCGGGGGTGGCTCTGGGGGAGGAGGTTCCGGTGGGGGAGGCTCTCAAGTACAACTGCAACAAAGT
GGGGCTGAAGTGAAGAAGCCCGGTTCTTCAGTCAAAGTATCATGTAAGGCAAGTGGTTATACTTTTACGTCTTATA
ACATGCATTGGGTAAAGCAAGCCCCTGGTCAGGGCCTCGAGTGGATTGGTGCGATCTACCCTGGAATGGGTGATA
CGAGCTATAATCAGAAATTCAAGGGGAAAGCCACCTTGACTGCAGACGAATCTACGAACACGGCTTACATGGAGC
TTAGCTCACTCAGATCAGAGGATACAGCCTTTTACTACTGCGCTAGATCAACTTATTACGGAGGAGACTGGTATTTT
GATGTATGGGGTCAGGGGACCACAGTCACTGTTAGCTCTGCTAGCACCACGACGCCAGCGCCGCGACCACCAACA
CCGGCGCCCACCATCGCGTCGCAGCCCCTGTCCCTGCGCCCAGAGGCGTGCCGGCCAGCGGCGGGGGGCGCAGT
GCACACGAGGGGGCTGGACTTCGCCTGTGATTCCGGAATCTACATCTGGGCGCCCTTGGCCGGGACTTGTGGGGT
CCTTCTCCTGTCACTGGTTATCACCCTTTACTGCAAACGGGGCAGAAAGAAACTCCTGTATATATTCAAACAACCAT
TTATGAGACCAGTACAAACTACTCAAGAGGAAGATGGCTGTAGCTGCCGATTTCCAGAAGAAGAAGAAGGAGGA
TGTGAACTGAGAGTGAAGTTCAGCAGGAGCGCAGACGCCCCCGCGTACCAGCAGGGCCAGAACCAGCTCTATAA
CGAGCTCAATCTAGGACGAAGAGAGGAGTACGATGTTTTGGACAAGAGACGTGGCCGGGACCCTGAGATGGGG
GGAAAGCCGAGAAGGAAGAACCCTCAGGAAGGCCTGTACAATGAACTGCAGAAAGATAAGATGGCGGAGGCCT
ACAGTGAGATTGGGATGAAAGGCGAGCGCCGGAGGGGCAAGGGGCACGATGGCCTTTACCAGGGTCTCAGTAC
AGCCACCAAGGACACCTACGACGCCCTTCACATGCAGGCCCTGCCCCCTCGCGGCTCCGGCGCAACAAATTTCTCC
TTGCTGAAACAGGCAGGCGACGTTGAGGAAAATCCCGGCCCAGGGGTTCAGGTGGAGACTATCAGCCCGGGCG
ACGGACGGACATTCCCAAAGCGCGGGCAGACGTGTGTGGTGCATTACACCGGGATGCTTGAGGACGGAAAGA

AAGTGGACTCTTCCCGAGACCGAAATAAACCATTCAAGTTCATGTTGGGCAAGCAGGAGGTTATCAGAGGGTG
GGAGGAGGGCGTCGCTCAGATGAGTGTCGGACAGAGGGCCAAGCTCACGATCTCCCCTGATTACGCCTACGGG
GCAACTGGTCACCCCGGAATCATCCCCCCTCACGCAACCCTCGTGTTCGACGTCGAGCTGCTCAAACTGGAATCA
GGCGGAGGCAGTGGCGGGTTTGGCGATGTCGGTGCCCTTGAAAGCTTGAGAGGAAATGCCGATCTCGCTTACA
TCTTGAGCATGGAGCCCTGTGGGCACTGTCTGATCATCAACAATGTTAACTTTTGCCGGGAGTCCGGCCTGCGCA
CACGCACAGGCTCCAACATTGACTGCGAAAAACTTCGAAGGAGGTTTAGCTCTCTGCATTTCATGGTAGAGGTG
AAGGGGGATCTGACCGCCAAGAAAATGGTTCTCGCCCTTCTCGAGCTTGCGCAGCAGGACCATGGAGCGCTTGA
CTGTTGTGTCGTTGTGATACTGAGCCATGGCTGTCAGGCTTCCCATCTCCAGTTTCCAGGGGCCGTGTACGGAAC
CGATGGATGCCCTGTGTCAGTTGAAAAGATCGTAAACATCTTTAACGGAACATCTTGCCCGAGCCTCGGCGGTA
AACCGAAGCTTTTTTTTATCCAGGCCTGCGGCGGTGAACAGAAAGATCATGGCTTCGAGGTTGCCAGTACCAGC
CCTGAAGACGAATCCCCCGGGTCAAATCCTGAACCAGATGCGACCCCTTTCCAGGAAGGACTCCGCACTTTTGAC
CAGCTTGACGCCATTTCCTCCCTGCCAACACCTTCCGACATATTTGTAAGCTACTCCACCTTTCCAGGATTCGTGAG
CTGGCGCGACCCAAAATCCGGCAGTTGGTATGTTGAAACCCTGGACGATATCTTCGAACAATGGGCCCACAGTG
AGGACCTGCAGTCCCTTCTTCTGCGCGTAGCCAATGCCGTGTCAGTCAAAGGGATTTACAAGCAGATGCCAGGC
TGCTTTAATTTCCTGCGCAAGAAACTGTTTTTTAAGACCAGTTGA

Anti-human CD20bbz-2A-iCasp9 amino acid sequence (SEQ ID NO: 3025)

MALPVTALLLPLALLLHAARPGSDIQLTQSPSSLSASVGDRVTMTCRASSSVSYIHWFQQKPGKAPKPWIYATSNLASG
VPVRFSGSGSGTDYTFTISSLQPEDIATYYCQQWTSNPPTFGGGTKLEIKRGGGGSGGGGSGGGGSQVQLQQSGAEVK
KPGSSVKVSCKASGYTFTSYNMHWVKQAPGQGLEWIGAIYPGMGDTSYNQKFKGKATLTADESTNTAYMELSSLRSE
DTAFYYCARSTYYGGDWYFDVWGQGTTVTVSSASTTTPAPRPPTPAPTIASQPLSLRPEACRPAAGGAVHTRGLDFAC
DSGIYIWAPLAGTCGVLLLSLVITLYCKRGRKKLLYIFKQPFMRPVQTTQEEDGCSCRFPEEEEGGCELRVKFSRSADAPAY
QQGQNQLYNELNLGRREEYDVLDKRRGRDPEMGGKPRRKNPQEGLYNELQKDKMAEAYSEIGMKGERRRGKGHDG
LYQGLSTATKDTYDALHMQALPPRGSGATNFSLLKQAGDVEENPGPGVQVETISPGDGRTFPKRGQTCVVHYTGML
EDGKKVDSSRDRNKPFKFMLGKQEVIRGWEEGVAQMSVGQRAKLTISPDYAYGATGHPGIIPPHATLVFDVELLKLE
SGGGSGGFGDVGALESLRGNADLAYILSMEPCGHCLIINNVNFCRESGLRTRTGSNIDCEKLRRRFSSLHFMVEVKGD
LTAKKMVLALLELAQQDHGALDCCVVVILSHGCQASHLQFPGAVYGTDGCPVSVEKIVNIFNGTSCPSLGGKPKLFFI
QACGGEQKDHGFEVASTSPEDESPGSNPEPDATPFQEGLRTFDQLDAISSLPTPSDIFVSYSTFPGFVSWRDPKSGS
WYVETLDDIFEQWAHSEDLQSLLLRVANAVSVKGIYKQMPGCFNFLRKKLFFKTSZ

iCasp9-2A-anti-human CD20bbz nucleotide sequence (SEQ ID NO: 3026)

ATGCTCGAGGGGGTTCAGGTGGAGACTATCAGCCCGGGCGACGGACGGACATTCCCAAAGCGCGGGCAGACGT
GTGTGGTGCATTACACCGGGATGCTTGAGGACGGAAAGAAAGTGGACTCTTCCCGAGACCGAAATAAACCATT
CAAGTTCATGTTGGGCAAGCAGGAGGTTATCAGAGGGTGGGAGGAGGGCGTCGCTCAGATGAGTGTCGGACA
GAGGGCCAAGCTCACGATCTCCCCTGATTACGCCTACGGGGCAACTGGTCACCCCGGAATCATCCCCCCTCACGC
AACCCTCGTGTTCGACGTCGAGCTGCTCAAACTGGAATCAGGCGGAGGCAGTGGCGCTAGCGGGTTTGGCGAT
GTCGGTGCCCTTGAAAGCTTGAGAGGAAATGCCGATCTCGCTTACATCTTGAGCATGGAGCCCTGTGGGCACTG
TCTGATCATCAACAATGTTAACTTTTGCCGGGAGTCCGGCCTGCGCACACGCACAGGCTCCAACATTGACTGCGA

AAAACTTCGAAGGAGGTTTAGCTCTCTGCATTTCATGGTAGAGGTGAAGGGGGATCTGACCGCCAAGAAAATG
GTTCTCGCCCTTCTCGAGCTTGCGCAGCAGGACCATGGAGCGCTTGACTGTTGTGTCGTTGTGATACTGAGCCAT
GGCTGTCAGGCTTCCCATCTCCAGTTTCCAGGGGCCGTGTACGGAACCGATGGATGCCCTGTGTCAGTTGAAAA
GATCGTAAACATCTTTAACGGAACATCTTGCCCGAGCCTCGGCGGTAAACCGAAGCTTTTTTTTATCCAGGCCTG
CGGCGGTGAACAGAAAGATCATGGCTTCGAGGTTGCCAGTACCAGCCCTGAAGACGAATCCCCCGGGTCAAAT
CCTGAACCAGATGCGACCCCTTTCCAGGAAGGACTCCGCACTTTTGACCAGCTTGACGCCATTTCCTCCCTGCCAA
CACCTTCCGACATATTTGTAAGCTACTCCACCTTTCCAGGATTCGTGAGCTGGCGCGACCCAAAATCCGGCAGTT
GGTATGTTGAAACCCTGGACGATATTTTCGAACAATGGGCCCACAGTGAGGACCTGCAGTCCCTTCTTCTGCGCG
TAGCCAATGCCGTGTCAGTCAAAGGGATTTACAAGCAGATGCCAGGCTGCTTTAATTTCCTGCGCAAGAAACTG
TTTTTTAAGACCAGTGGCTCCGGCGCAACAAATTTCTCCTTGCTGAAACAGGCAGGCGACGTTGAGGAAAATCCC
GGCCCAATGGCCTTACCAGTGACCGCCTTGCTCCTGCCGCTGGCCTTGCTGCTCCACGCCGCCAGGCCGGGATCCG
ATATTCAACTTACACAGTCCCCTAGTTCCTTGTCCGCCTCCGTCGGCGACAGAGTTACCATGACTTGTCGAGCGTCT
TCTAGCGTTTCCTACATTCATTGGTTCCAACAGAAGCCCGGAAAGGCACCAAAACCTTGGATCTACGCCACGTCCA
ATTTGGCTAGTGGTGTTCCGGTGCGATTTTCCGGGTCAGGTAGCGGGACGGACTATACTTTTACCATTTCAAGCCT
TCAGCCTGAAGACATCGCGACGTACTATTGTCAGCAGTGGACCTCTAACCCTCCTACCTTTGGCGGTGGAACAAAG
CTGGAGATCAAACGAGGCGGGGGTGGCTCTGGGGGAGGAGGTTCCGGTGGGGGAGGCTCTCAAGTACAACTGC
AACAAAGTGGGGCTGAAGTGAAGAAGCCCGGTTCTTCAGTCAAAGTATCATGTAAGGCAAGTGGTTATACTTTTA
CGTCTTATAACATGCATTGGGTAAAGCAAGCCCCTGGTCAGGGCCTCGAGTGGATTGGTGCGATCTACCCTGGAA
TGGGTGATACGAGCTATAATCAGAAATTCAAGGGGAAAGCCACCTTGACTGCAGACGAATCTACGAACACGGCTT
ACATGGAGCTTAGCTCACTCAGATCAGAGGATACAGCCTTTTACTACTGCGCTAGATCAACTTATTACGGAGGAGA
CTGGTATTTTGATGTATGGGGTCAGGGGACCACAGTCACTGTTAGCTCTGCTAGCACCACGACGCCAGCGCCGCG
ACCACCAACACCGGCGCCCACCATCGCGTCGCAGCCCCTGTCCCTGCGCCCAGAGGCGTGCCGGCCAGCGGCGGG
GGGCGCAGTGCACACGAGGGGGCTGGACTTCGCCTGTGATTCCGGAATCTACATCTGGGCGCCCTTGGCCGGGA
CTTGTGGGGTCCTTCTCCTGTCACTGGTTATCACCCTTTACTGCAAACGGGGCAGAAAGAAACTCCTGTATATATTC
AAACAACCATTTATGAGACCAGTACAAACTACTCAAGAGGAAGATGGCTGTAGCTGCCGATTTCCAGAAGAAGAA
GAAGGAGGATGTGAACTGAGAGTGAAGTTCAGCAGGAGCGCAGACGCCCCCGCGTACCAGCAGGGCCAGAACC
AGCTCTATAACGAGCTCAATCTAGGACGAAGAGAGGAGTACGATGTTTTGGACAAGAGACGTGGCCGGGACCCT
GAGATGGGGGGAAAGCCGAGAAGGAAGAACCCTCAGGAAGGCCTGTACAATGAACTGCAGAAAGATAAGATGG
CGGAGGCCTACAGTGAGATTGGGATGAAAGGCGAGCGCCGGAGGGGCAAGGGGCACGATGGCCTTTACCAGGG
TCTCAGTACAGCCACCAAGGACACCTACGACGCCCTTCACATGCAGGCCCTGCCCCCTCGC

iCasp9-2A-anti-human CD20bbz amino acid sequence (SEQ ID NO: 3027)

MLEGVQVETISPGDGRTFPKRGQTCVVHYTGMLEDGKKVDSSRDRNKPFKFMLGKQEVIRGWEEGVAQMSVGQRA
KLTISPDYAYGATGHPGIIPPHATLVFDVELLKLESGGGSGASGFGDVGALESLRGNADLAYILSMEPCGHCLIINNVNFC
RESGLRTRTGSNIDCEKLRRRFSSLHFMVEVKGDLTAKKMVLALLELAQQDHGALDCCVVVILSHGCQASHLQFPGAVY
GTDGCPVSVEKIVNIFNGTSCPSLGGKPKLFFIQACGGEQKDHGFEVASTSPEDESPGSNPEPDATPFQEGLRTFDQLDA
ISSLPTPSDIFVSYSTFPGFVSWRDPKSGSWYVETLDDIFEQWAHSEDLQSLLLRVANAVSVKGIYKQMPGCFNFLRKKL
FFKTSGSGATNFSLLKQAGDVEENPGPMALPVTALLLPLALLLHAARPGSDIQLTQSPSSLSASVGDRVTMTCRASSSV
SYIHWFQQKPGKAPKPWIYATSNLASGVPVRFSGSGSGTDYTFTISSLQPEDIATYYCQQWTSNPPTFGGGTKLEIKR
GGGGSGGGGSGGGGSQVQLQQSGAEVKKPGSSVKVSCKASGYTFTSYNMHWVKQAPGQGLEWIGAIYPGMGD

TSYNQKFKGKATLTADESTNTAYMELSSLRSEDTAFYYCARSTYYGGDWYFDVWGQGTTVTVSSASTTTPAPRPPT
PAPTIASQPLSLRPEACRPAAGGAVHTRGLDFACDSGIYIWAPLAGTCGVLLLSLVITLYCKRGRKKLLYIFKQPFMRPV
QTTQEEDGCSCRFPEEEEGGCELRVKFSRSADAPAYQQGQNQLYNELNLGRREEYDVLDKRRGRDPEMGGKPRRK
NPQEGLYNELQKDKMAEAYSEIGMKGERRRGKGHDGLYQGLSTATKDTYDALHMQALPPRZ

FMC63-bbz-2A- revCasp9 nucleotide sequence (SEQ ID NO: 3028)

ATGGCCTTACCAGTGACCGCCTTGCTCCTGCCGCTGGCCTTGCTGCTCCACGCCGCCAGGCCGGGATCCGACATCC
AGATGACACAGACTACATCCTCCCTGTCTGCCTCTCTGGGAGACAGAGTCACCATCAGTTGCAGGGCAAGTCAGG
ACATTAGTAAATATTTAAATTGGTATCAGCAGAAACCAGATGGAACTGTTAAACTCCTGATCTACCATACATCAAG
ATTACACTCAGGAGTCCCATCAAGGTTCAGTGGCAGTGGGTCTGGAACAGATTATTCTCTCACCATTAGCAACCTG
GAGCAAGAAGATATTGCCACTTACTTTTGCCAACAGGGTAATACGCTTCCGTACACGTTCGGAGGGGGGACTAAG
TTGGAAATAACAGGTGGCGGTGGCTCGGGCGGTGGTGGGTCGGGTGGCGGCGGATCTGAGGTGAAACTGCAGG
AGTCAGGACCTGGCCTGGTGGCGCCCTCACAGAGCCTGTCCGTCACATGCACTGTCTCAGGGGTCTCATTACCCGA
CTATGGTGTAAGCTGGATTCGCCAGCCTCCACGAAAGGGTCTGGAGTGGCTGGGAGTAATATGGGGTAGTGAAA
CCACATACTATAATTCAGCTCTCAAATCCAGACTGACCATCATCAAGGACAACTCCAAGAGCCAAGTTTTCTTAAAA
ATGAACAGTCTGCAAACTGATGACACAGCCATTTACTACTGTGCCAAACATTATTACTACGGTGGTAGCTATGCTAT
GGACTACTGGGGTCAAGGAACCTCAGTCACCGTCTCCTCAGCTAGCACCACGACGCCAGCGCCGCGACCACCAAC
ACCGGCGCCCACCATCGCGTCGCAGCCCCTGTCCCTGCGCCCAGAGGCGTGCCGGCCAGCGGCGGGGGGCGCAG
TGCACACGAGGGGGCTGGACTTCGCCTGTGATTCCGGAATCTACATCTGGGCCCCTCTGGCCGGCACCTGTGGCG
TGCTGCTGCTGTCTCTCGTGATCACCCTGTACTGCAAGCGGGGCAGAAAGAAGCTGCTGTACATCTTCAAGCAGCC
CTTCATGCGGCCCGTGCAGACCACCCAGGAAGAGGACGGCTGCTCCTGCAGATTCCCCGAGGAAGAAGAAGGCG
GCTGCGAGCTGAGAGTGAAGTTCAGCAGAAGCGCCGACGCCCCTGCCTATCAGCAGGGCCAGAACCAGCTGTAC
AACGAGCTGAACCTGGGCAGACGGGAAGAGTACGACGTGCTGGACAAGCGGAGAGGCAGGGACCCTGAGATGG
GCGGCAAGCCCAGAAGAAAGAACCCCCAGGAAGGCCTGTATAACGAACTGCAGAAAGACAAGATGGCCGAGGC
CTACAGCGAGATCGGAATGAAGGGCGAGCGGAGAAGAGGCAAGGGCCACGATGGCCTGTACCAGGGCCTGAGC
ACCGCCACCAAGGACACCTATGACGCCCTGCACATGCAGGCCCTGCCTCCAAGAGGAAGCGGCGCCACCAATTTC
AGCCTGCTGAAACAGGCCGGCGACGTGGAAGAGAACCCTGGCCCTAGAGGCGTGCAGGTGGAAACCATCTCTCC
CGGCGACGGCAGAACCTTCCCTAAGAGGGGCCAGACCTGCGTGGTGCACTACACCGGCATGCTGGAAGATGGC
AAGAAGATGGACAGCTCCCGGGACCGGAACAAGCCCTTCAAGTTCATGCTGGGCAAGCAGGAAGTGATCCGGG
GCTGGGAAGAGGGCGTGGCACAGATGTCTGTGGGCCAGAGAGCCAAGCTGACCATCAGCCCCGATTACGCCTA
CGGCGCCACAGGCCACCCTGGCATCATTCCTCCACACGCCACACTGGTGTTCGATGTGGAACTGCTGAAGCTGGA
AACCCGGGGAGTGCAGGTGGAAACAATCAGCCCTGGCGACGGCCGGACCTTTCCAAAACGGGGACAGACATGT
GTGGTGCATTATACAGGGATGCTGGAAGATGGGAAAAAAATGGATAGCAGCCGCGACCGCAACAAACCTTTTA
AGTTTATGCTGGGGAAACAGGAAGTGATTAGAGGCTGGGAAGAGGGGGTGGCACAGATGAGCGTGGGACAG
CGGGCCAAACTGACAATCTCCCCCGACTATGCCTATGGGGCCACCGGACACCCCGGAATCATCCCACCTCATGCT
ACCCTGGTGTTTGACGTGGAACTGCTGAAACTGGAAACAAGCGGCGGAGGCAGCGGCGGCTTTGGAGATGTGG
GAGCCCTGGAAAGCCTGCGGGGCAATGCCGATCTGGCCTACATCCTGAGCATGGAACCCTGCGGCCACTGCCTG
ATTATCAACAACGTGAACTTCTGCAGAGAGAGCGGCCTGCGGACCAGAACCGGCAGCAACATCGACTGCGAGA
AGCTGCGGCGGAGATTCAGCAGCCTGCACTTCATGGTGGAAGTGAAGGGGGACCTGACCGCCAAGAAAATGGT
GCTGGCTCTGCTGGAACTGGCCCAGCAGGATCATGGCGCCCTGGACTGTTGCGTGGTCGTGATCCTGAGCCACG

GCTGCCAGGCCAGCCATCTGCAGTTTCCCGGCGCTGTGTATGGCACCGATGGCTGCCCTGTGTCCGTGGAAAAG
ATCGTGAATATCTTCAACGGCACCAGCTGCCCCAGCCTGGGCGGAAAGCCTAAGCTGTTCTTTATTCAAGCCTGT
GGGGGCGAGCAGAAGGACCACGGATTTGAGGTGGCCAGCACCTCCCCCGAGGATGAGAGCCCTGGCAGCAAC
CCTGAGCCTGACGCCACCCCATTCCAGGAAGGACTGCGGACCTTCGACCAGCTGGACGCCATCTCTAGCCTGCCC
ACCCCCAGCGACATCTTCGTGTCCTACAGCACCTTCCCTGGCTTTGTGTCCTGGCGGGACCCCAAGTCCGGCTCTT
GGTACGTGGAAACCCTGGACGACATCTTTGAGCAGTGGGCCCATAGCGAGGACCTGCAGAGCCTGCTGCTGAG
GGTGGCCAATGCCGTGTCCGTGAAGGGCATCTACAAGCAGATGCCCGGCTGCTTCAACTTCCTGCGGAAGAAGC
TGTTTTTCAAGACCAGC

FMC63-bbz-2A- revCasp9 amino acid sequence (SEQ ID NO:3029)

MALPVTALLLPLALLLHAARPGSDIQMTQTTSSLSASLGDRVTISCRASQDISKYLNWYQQKPDGTVKLLIYHTSRLHSGV
PSRFSGSGSGTDYSLTISNLEQEDIATYFCQQGNTLPYTFGGGTKLEITGGGGSGGGGSGGGGSEVKLQESGPGLVAPS
QSLSVTCTVSGVSLPDYGVSWIRQPPRKGLEWLGVIWGSETTYYNSALKSRLTIIKDNSKSQVFLKMNSLQTDDTAIYYC
AKHYYYGGSYAMDYWGQGTSVTVSSASTTTPAPRPPTPAPTIASQPLSLRPEACRPAAGGAVHTRGLDFACDSGIYIW
APLAGTCGVLLLSLVITLYCKRGRKKLLYIFKQPFMRPVQTTQEEDGCSCRFPEEEEGGCELRVKFSRSADAPAYQQGQN
QLYNELNLGRREEYDVLDKRRGRDPEMGGKPRRKNPQEGLYNELQKDKMAEAYSEIGMKGERRRGKGHDGLYQGLS
TATKDTYDALHMQALPPRGSGATNFSLLKQAGDVEENPGPRGVQVETISPGDGRTFPKRGQTCVVHYTGMLEDGKK
MDSSRDRNKPFKFMLGKQEVIRGWEEGVAQMSVGQRAKLTISPDYAYGATGHPGIIPPHATLVFDVELLKLETRGV
QVETISPGDGRTFPKRGQTCVVHYTGMLEDGKKMDSSRDRNKPFKFMLGKQEVIRGWEEGVAQMSVGQRAKLTI
SPDYAYGATGHPGIIPPHATLVFDVELLKLETSGGGSGGFGDVGALESLRGNADLAYILSMEPCGHCLIINNVNFCRES
GLRTRTGSNIDCEKLRRRFSSLHFMVEVKGDLTAKKMVLALLELAQQDHGALDCCVVVILSHGCQASHLQFPGAVYG
TDGCPVSVEKIVNIFNGTSCPSLGGKPKLFFIQACGGEQKDHGFEVASTSPEDESPGSNPEPDATPFQEGLRTFDQLD
AISSLPTPSDIFVSYSTFPGFVSWRDPKSGSWYVETLDDIFEQWAHSEDLQSLLLRVANAVSVKGIYKQMPGCFNFLR
KKLFFKTS

CD19bbz on-CAR nucleotide sequence, CD19 on-CAR (SEQ ID NO: 977, also provided previously herein)
Signal peptide-conditional aggregation domains (4 repeats)- Furin site-FMC63bbz

ATGGCCTTACCAGTGACCGCCTTGCTCCTGCCGCTGGCCTTGCTGCTCCACGCCGCCAGGCCGGGATCCCGGGGCG
TGCAGGTTGAGACAATTTCCCCAGGAGATGGGCGAACGTTCCCCAAGCGCGGACAGACATGCGTTGTGCACTACA
CAGGAATGTTGGAGGACGGAAAGAAAATGGACAGTTCAAGAGATCGGAACAAACCATTCAAATTCATGTTGGGA
AAACAGGAAGTGATACGGGGCTGGGAAGAGGGTGTAGCGCAAATGTCCGTTGGTCAACGAGCAAAACTCACGAT
AAGTCCCGATTATGCTTACGGCGCAACCGGTCACCCGGGCATCATACCGCCTCATGCGACTTTGGTCTTTGATGTG
GAGCTGTTGAAACTTGAAACTCGCGGAGTACAGGTTGAAACAATATCACCCGGGGACGGGCGGACTTTTCCGAAG
AGAGGTCAGACCTGCGTCGTCCATTATACCGGTATGCTGGAGGACGGAAAGAAAATGGACAGCTCACGGGACCG
AAATAAACCATTCAAATTTATGTTGGGGAAACAAGAGGTTATCAGGGGCTGGGAGGAGGGTGTGGCCCAGATGT
CTGTCGGTCAGCGCGCGAAACTCACAATCTCTCCGGATTATGCGTATGGGGCGACAGGGCATCCGGGAATTATCC
CTCCCCACGCTACCTTGGTTTTCGATGTTGAGCTTCTGAAGTTGGAGACCAGAGGAGTTCAAGTGGAGACAATATC
TCCTGGGGATGGACGGACGTTCCCCAAGCGCGGCCAGACCTGTGTAGTCCACTACACAGGGATGCTTGAAGACG

GAAAAAAGATGGATAGCAGTAGAGATCGCAACAAACCATTTAAGTTCATGCTGGGGAAGCAGGAAGTAATACGC
GGCTGGGAGGAAGGCGTGGCACAGATGAGTGTTGGTCAACGGGCCAAACTTACTATTTCTCCCGATTATGCGTAT
GGAGCCACCGGGCACCCTGGCATTATCCCACCCCATGCCACATTGGTTTTTGACGTTGAATTGCTTAAATTGGAGA
CCAGGGGAGTCCAAGTGGAAACAATATCACCGGGGGATGGTCGGACTTTTCCTAAAAGGGGCCAAACCTGTGTA
GTCCATTATACCGGAATGCTCGAAGACGGAAAGAAAATGGACTCTTCTAGAGACCGCAATAAGCCCTTCAAGTTCA
TGTTGGGTAAGCAAGAGGTGATCCGGGGCTGGGAAGAGGGGGTCGCTCAAATGTCCGTCGGTCAGCGAGCTAAA
CTGACTATTTCCCCAGACTACGCATATGGAGCGACTGGCCACCCCGGTATTATTCCTCCCCATGCGACTCTCGTGTT
CGACGTAGAACTCTTGAAATTGGAAACGTCAGCCCGGAACAGGCGGAAGAGAGGATCCGACATCCAGATGACAC
AGACTACATCCTCCCTGTCTGCCTCTCTGGGAGACAGAGTCACCATCAGTTGCAGGGCAAGTCAGGACATTAGTAA
ATATTTAAATTGGTATCAGCAGAAACCAGATGGAACTGTTAAACTCCTGATCTACCATACATCAAGATTACACTCAG
GAGTCCCATCAAGGTTCAGTGGCAGTGGGTCTGGAACAGATTATTCTCTCACCATTAGCAACCTGGAGCAAGAAG
ATATTGCCACTTACTTTTGCCAACAGGGTAATACGCTTCCGTACACGTTCGGAGGGGGGACTAAGTTGGAAATAAC
AGGTGGCGGTGGCTCGGGCGGTGGTGGGTCGGGTGGCGGCGGATCTGAGGTGAAACTGCAGGAGTCAGGACCT
GGCCTGGTGGCGCCCTCACAGAGCCTGTCCGTCACATGCACTGTCTCAGGGGTCTCATTACCCGACTATGGTGTAA
GCTGGATTCGCCAGCCTCCACGAAAGGGTCTGGAGTGGCTGGGAGTAATATGGGGTAGTGAAACCACATACTATA
ATTCAGCTCTCAAATCCAGACTGACCATCATCAAGGACAACTCCAAGAGCCAAGTTTTCTTAAAAATGAACAGTCT
GCAAACTGATGACACAGCCATTTACTACTGTGCCAAACATTATTACTACGGTGGTAGCTATGCTATGGACTACTGG
GGTCAAGGAACCTCAGTCACCGTCTCCTCAGCTAGCACCACGACGCCAGCGCCGCGACCACCAACACCGGCGCCC
ACCATCGCGTCGCAGCCCCTGTCCCTGCGCCCAGAGGCGTGCCGGCCAGCGGCGGGGGGCGCAGTGCACACGAG
GGGGCTGGACTTCGCCTGTGATTCCGGAATCTACATCTGGGCGCCCTTGGCCGGGACTTGTGGGGTCCTTCTCCTG
TCACTGGTTATCACCCTTTACTGCAAACGGGGCAGAAAGAAACTCCTGTATATATTCAAACAACCATTTATGAGACC
AGTACAAACTACTCAAGAGGAAGATGGCTGTAGCTGCCGATTTCCAGAAGAAGAAGAAGGAGGATGTGAACTGA
GAGTGAAGTTCAGCAGGAGCGCAGACGCCCCCGCGTACCAGCAGGGCCAGAACCAGCTCTATAACGAGCTCAAT
CTAGGACGAAGAGAGGAGTACGATGTTTTGGACAAGAGACGTGGCCGGGACCCTGAGATGGGGGGAAAGCCGA
GAAGGAAGAACCCTCAGGAAGGCCTGTACAATGAACTGCAGAAAGATAAGATGGCGGAGGCCTACAGTGAGATT
GGGATGAAAGGCGAGCGCCGGAGGGGCAAGGGGCACGATGGCCTTTACCAGGGTCTCAGTACAGCCACCAAGG
ACACCTACGACGCCCTTCACATGCAGGCCCTGCCCCCTCGCTAA

CD19bbz on-CAR amino acid sequence (SEQ ID NO: 978, also provided previously herein)
Signal peptide-conditional aggregation domains (4 repeats)-Furin site-FMC63bbz

MALPVTALLLPLALLLHAARPGSRGVQVETISPGDGRTFPKRGQTCVVHYTGMLEDGKKMDSSRDRNKPFKFMLGKQ
EVIRGWEEGVAQMSVGQRAKLTISPDYAYGATGHPGIIPPHATLVFDVELLKLETRGVQVETISPGDGRTFPKRGQTCV
VHYTGMLEDGKKMDSSRDRNKPFKFMLGKQEVIRGWEEGVAQMSVGQRAKLTISPDYAYGATGHPGIIPPHATLVFD
VELLKLETRGVQVETISPGDGRTFPKRGQTCVVHYTGMLEDGKKMDSSRDRNKPFKFMLGKQEVIRGWEEGVAQMS
VGQRAKLTISPDYAYGATGHPGIIPPHATLVFDVELLKLETRGVQVETISPGDGRTFPKRGQTCVVHYTGMLEDGKKMD
SSRDRNKPFKFMLGKQEVIRGWEEGVAQMSVGQRAKLTISPDYAYGATGHPGIIPPHATLVFDVELLKLETSARNRRKR
GSDIQMTQTTSSLSASLGDRVTISCRASQDISKYLNWYQQKPDGTVKLLIYHTSRLHSGVPSRFSGSGSGTDYSLTISNLE
QEDIATYFCQQGNTLPYTFGGGTKLEITGGGGSGGGGSGGGGSEVKLQESGPGLVAPSQSLSVTCTVSGVSLPDYGVS
WIRQPPRKGLEWLGVIWGSETTYYNSALKSRLTIIKDNSKSQVFLKMNSLQTDDTAIYYCAKHYYYGGSYAMDYWGQG
TSVTVSSASTTTPAPRPPTPAPTIASQPLSLRPEACRPAGGAVHTRGLDFACDSGIYIWAPLAGTCGVLLLSLVITLYCKR

GRKKLLYIFKQPFMRPVQTTQEEDGCSCRFPEEEEGGCELRVKFSRSADAPAYQQGQNQLYNELNLGRREEYDVLDKR
RGRDPEMGGKPRRKNPQEGLYNELQKDKMAEAYSEIGMKGERRRGKGHDGLYQGLSTATKDTYDALHMQALPPRZ

Signal peptide-Anti-human CD20-CD8 hinge-CD8transmembrane-CD137-CD247-2A- revCasp9 amino
acid sequence nucleotide sequence (SEQ ID NO: 3030)

ATGGCCTTACCAGTGACCGCCTTGCTCCTGCCGCTGGCCTTGCTGCTCCACGCCGCCAGGCCGGGATCCGATATTCA
ACTTACACAGTCCCCTAGTTCCTTGTCCGCCTCCGTCGGCGACAGAGTTACCATGACTTGTCGAGCGTCTTCTAGCG
TTTCCTACATTCATTGGTTCCAACAGAAGCCCGGAAAGGCACCAAAACCTTGGATCTACGCCACGTCCAATTTGGCT
AGTGGTGTTCCGGTGCGATTTTCCGGGTCAGGTAGCGGGACGGACTATACTTTTACCATTTCAAGCCTTCAGCCTG
AAGACATCGCGACGTACTATTGTCAGCAGTGGACCTCTAACCCTCCTACCTTTGGCGGTGGAACAAAGCTGGAGAT
CAAACGAGGCGGGGGTGGCTCTGGGGGAGGAGGTTCCGGTGGGGGAGGCTCTCAAGTACAACTGCAACAAAGT
GGGGCTGAAGTGAAGAAGCCCGGTTCTTCAGTCAAAGTATCATGTAAGGCAAGTGGTTATACTTTTACGTCTTATA
ACATGCATTGGGTAAAGCAAGCCCCTGGTCAGGGCCTCGAGTGGATTGGTGCGATCTACCCTGGAATGGGTGATA
CGAGCTATAATCAGAAATTCAAGGGGAAAGCCACCTTGACTGCAGACGAATCTACGAACACGGCTTACATGGAGC
TTAGCTCACTCAGATCAGAGGATACAGCCTTTTACTACTGCGCTAGATCAACTTATTACGGAGGAGACTGGTATTTT
GATGTATGGGGTCAGGGGACCACAGTCACTGTTAGCTCTGCTAGCACCACGACGCCAGCGCCGCGACCACCAAC
ACCGGCGCCCACCATCGCGTCGCAGCCCCTGTCCCTGCGCCCAGAGGCGTGCCGGCCAGCGGCGGGGGGCGCA
GTGCACACGAGGGGGCTGGACTTCGCCTGTGATTCCGGAATCTACATCTGGGCGCCCTTGGCCGGGACTTGTGG
GGTCCTTCTCCTGTCACTGGTTATCACCCTTTACTGCAAACGGGGCAGAAAGAAACTCCTGTATATATTCAAACAAC
CATTTATGAGACCAGTACAAACTACTCAAGAGGAAGATGGCTGTAGCTGCCGATTTCCAGAAGAAGAAGAAGGA
GGATGTGAACTGAGAGTGAAGTTCAGCAGGAGCGCAGACGCCCCCGCGTACCAGCAGGGCCAGAACCAGCTCTA
TAACGAGCTCAATCTAGGACGAAGAGAGGAGTACGATGTTTTGGACAAGAGACGTGGCCGGGACCCTGAGATGG
GGGGAAAGCCGAGAAGGAAGAACCCTCAGGAAGGCCTGTACAATGAACTGCAGAAAGATAAGATGGCGGAGGC
CTACAGTGAGATTGGGATGAAAGGCGAGCGCCGGAGGGGCAAGGGGCACGATGGCCTTTACCAGGGTCTCAGT
ACAGCCACCAAGGACACCTACGACGCCCTTCACATGCAGGCCCTGCCCCCTCGCGGAAGCGGCGCCACCAATTTCA
GCCTGCTGAAACAGGCCGGCGACGTGGAAGAGAACCCTGGCCCTAGAGGCGTGCAGGTGGAAACCATCTCTCCC
GGCGACGGCAGAACCTTCCCTAAGAGGGGCCAGACCTGCGTGGTGCACTACACCGGCATGCTGGAAGATGGCA
AGAAGATGGACAGCTCCCGGGACCGGAACAAGCCCTTCAAGTTCATGCTGGGCAAGCAGGAAGTGATCCGGGG
CTGGGAAGAGGGCGTGGCACAGATGTCTGTGGGCCAGAGAGCCAAGCTGACCATCAGCCCCGATTACGCCTAC
GGCGCCACAGGCCACCCTGGCATCATTCCTCCACACGCCACACTGGTGTTCGATGTGGAACTGCTGAAGCTGGAA
ACCCGGGGAGTGCAGGTGGAAACAATCAGCCCTGGCGACGGCCGGACCTTTCCAAAACGGGGACAGACATGTG
TGGTGCATTATACAGGGATGCTGGAAGATGGGAAAAAAATGGATAGCAGCCGCGACCGCAACAAACCTTTTAA
GTTTATGCTGGGGAAACAGGAAGTGATTAGAGGCTGGGAAGAGGGGGTGGCACAGATGAGCGTGGGACAGC
GGGCCAAACTGACAATCTCCCCCGACTATGCCTATGGGGCCACCGGACACCCCGGAATCATCCCACCTCATGCTAC
CCTGGTGTTTGACGTGGAACTGCTGAAACTGGAAACAAGCGGCGGAGGCAGCGGCGGCTTTGGAGATGTGGGA
GCCCTGGAAAGCCTGCGGGGCAATGCCGATCTGGCCTACATCCTGAGCATGGAACCCTGCGGCCACTGCCTGATT
ATCAACAACGTGAACTTCTGCAGAGAGAGCGGCCTGCGGACCAGAACCGGCAGCAACATCGACTGCGAGAAGC
TGCGGCGGAGATTCAGCAGCCTGCACTTCATGGTGGAAGTGAAGGGGGACCTGACCGCCAAGAAAATGGTGCT
GGCTCTGCTGGAACTGGCCCAGCAGGATCATGGCGCCCTGGACTGTTGCGTGGTCGTGATCCTGAGCCACGGCT
GCCAGGCCAGCCATCTGCAGTTTCCCGGCGCTGTGTATGGCACCGATGGCTGCCCTGTGTCCGTGGAAAAGATCG

TGAATATCTTCAACGGCACCAGCTGCCCCAGCCTGGGCGGAAAGCCTAAGCTGTTCTTTATTCAAGCCTGTGGGG
GCGAGCAGAAGGACCACGGATTTGAGGTGGCCAGCACCTCCCCCGAGGATGAGAGCCCTGGCAGCAACCCTGA
GCCTGACGCCACCCCATTCCAGGAAGGACTGCGGACCTTCGACCAGCTGGACGCCATCTCTAGCCTGCCCACCCCC
AGCGACATCTTCGTGTCCTACAGCACCTTCCCTGGCTTTGTGTCCTGGCGGGACCCCAAGTCCGGCTCTTGGTACG
TGGAAACCCTGGACGACATCTTTGAGCAGTGGGCCCATAGCGAGGACCTGCAGAGCCTGCTGCTGAGGGTGGCC
AATGCCGTGTCCGTGAAGGGCATCTACAAGCAGATGCCCGGCTGCTTCAACTTCCTGCGGAAGAAGCTGTTTTTC
AAGACCAGC

Signal peptide-Anti human CD20-**CD8 hinge**-CD8transmembrane-CD137-CD247-2A- ***revCasp9*** amino acid sequence (SEQ ID NO: 3031) 
Signal peptide-Anti-human CD20-CD8 hinge-CD8transmembrane-CD137-CD247-2A- revCasp9 amino
acid sequence (SEQ ID NO: 3031)

MALPVTALLLPLALLLHAARPGSDIQLTQSPSSLSASVGDRVTMTCRASSSVSYIHWFQQKPGKAPKPWIYATSNLASG
VPVRFSGSGSGTDYTFTISSLQPEDIATYYCQQWTSNPPTFGGGTKLEIKRGGGGSGGGGSGGGGSQVQLQQSGAEVK
KPGSSVKVSCKASGYTFTSYNMHWVKQAPGQGLEWIGAIYPGMGDTSYNQKFKGKATLTADESTNTAYMELSSLRSE
DTAFYYCARSTYYGGDWYFDVWGQGTTVTVSSASTTTPAPRPPTPAPTIASQPLSLRPEACRPAAGGAVHTRGLDFA
CDSGIYIWAPLAGTCGVLLLSLVITLYCKRGRKKLLYIFKQPFMRPVQTTQEEDGCSCRFPEEEEGGCELRVKFSRSADAP
AYQQGQNQLYNELNLGRREEYDVLDKRRGRDPEMGGKPRRKNPQEGLYNELQKDKMAEAYSEIGMKGERRRGKGH
DGLYQGLSTATKDTYDALHMQALPPRGSGATNFSLLKQAGDVEENPGPRGVQVETISPGDGRTFPKRGQTCVVHYT
GMLEDGKKMDSSRDRNKPFKFMLGKQEVIRGWEEGVAQMSVGQRAKLTISPDYAYGATGHPGIIPPHATLVFDVE
LLKLETRGVQVETISPGDGRTFPKRGQTCVVHYTGMLEDGKKMDSSRDRNKPFKFMLGKQEVIRGWEEGVAQMSV
GQRAKLTISPDYAYGATGHPGIIPPHATLVFDVELLKLETSGGGSGGFGDVGALESLRGNADLAYILSMEPCGHCLIIN
NVNFCRESGLRTRTGSNIDCEKLRRRFSSLHFMVEVKGDLTAKKMVLALLELAQQDHGALDCCVVVILSHGCQASHL
QFPGAVYGTDGCPVSVEKIVNIFNGTSCPSLGGKPKLFFIQACGGEQKDHGFEVASTSPEDESPGSNPEPDATPFQEG
LRTFDQLDAISSLPTPSDIFVSYSTFPGFVSWRDPKSGSWYVETLDDIFEQWAHSEDLQSLLLRVANAVSVKGIYKQM
PGCFNFLRKKLFFKTS
CD20bbz on-CAR nucleotide sequence, CD20 on-CAR (SEQ ID NO: 3032)

Signal peptide-conditional aggregation domains (4 repeats)- Furin site-anti-CD20bbz

ATGGCCTTACCAGTGACCGCCTTGCTCCTGCCGCTGGCCTTGCTGCTCCACGCCGCCAGGCCGGGATCCCGGGGCG
TGCAGGTTGAGACAATTTCCCCAGGAGATGGGCGAACGTTCCCCAAGCGCGGACAGACATGCGTTGTGCACTACA
CAGGAATGTTGGAGGACGGAAAGAAAATGGACAGTTCAAGAGATCGGAACAAACCATTCAAATTCATGTTGGGA
AAACAGGAAGTGATACGGGGCTGGGAAGAGGGTGTAGCGCAAATGTCCGTTGGTCAACGAGCAAAACTCACGAT
AAGTCCCGATTATGCTTACGGCGCAACCGGTCACCCGGGCATCATACCGCCTCATGCGACTTTGGTCTTTGATGTG
GAGCTGTTGAAACTTGAAACTCGCGGAGTACAGGTTGAAACAATATCACCCGGGGACGGGCGGACTTTTCCGAAG
AGAGGTCAGACCTGCGTCGTCCATTATACCGGTATGCTGGAGGACGGAAAGAAAATGGACAGCTCACGGGACCG
AAATAAACCATTCAAATTTATGTTGGGGAAACAAGAGGTTATCAGGGGCTGGGAGGAGGGTGTGGCCCAGATGT
CTGTCGGTCAGCGCGCGAAACTCACAATCTCTCCGGATTATGCGTATGGGGCGACAGGGCATCCGGGAATTATCC
CTCCCCACGCTACCTTGGTTTTCGATGTTGAGCTTCTGAAGTTGGAGACCAGAGGAGTTCAAGTGGAGACAATATC
TCCTGGGGATGGACGGACGTTCCCCAAGCGCGGCCAGACCTGTGTAGTCCACTACACAGGGATGCTTGAAGACG

GAAAAAAGATGGATAGCAGTAGAGATCGCAACAAACCATTTAAGTTCATGCTGGGGAAGCAGGAAGTAATACGC
GGCTGGGAGGAAGGCGTGGCACAGATGAGTGTTGGTCAACGGGCCAAACTTACTATTTCTCCCGATTATGCGTAT
GGAGCCACCGGGCACCCTGGCATTATCCCACCCCATGCCACATTGGTTTTTGACGTTGAATTGCTTAAATTGGAGA
CCAGGGGAGTCCAAGTGGAAACAATATCACCGGGGGATGGTCGGACTTTTCCTAAAAGGGGCCAAACCTGTGTA
GTCCATTATACCGGAATGCTCGAAGACGGAAAGAAAATGGACTCTTCTAGAGACCGCAATAAGCCCTTCAAGTTCA
TGTTGGGTAAGCAAGAGGTGATCCGGGGCTGGGAAGAGGGGGTCGCTCAAATGTCCGTCGGTCAGCGAGCTAAA
CTGACTATTTCCCCAGACTACGCATATGGAGCGACTGGCCACCCCGGTATTATTCCTCCCCATGCGACTCTCGTGTT
CGACGTAGAACTCTTGAAATTGGAAACGTCAGCCCGGAACAGGCGGAAGAGAGATATTCAACTTACACAGTCCCC
TAGTTCCTTGTCCGCCTCCGTCGGCGACAGAGTTACCATGACTTGTCGAGCGTCTTCTAGCGTTTCCTACATTCATT
GGTTCCAACAGAAGCCCGGAAAGGCACCAAAACCTTGGATCTACGCCACGTCCAATTTGGCTAGTGGTGTTCCGG
TGCGATTTTCCGGGTCAGGTAGCGGGACGGACTATACTTTTACCATTTCAAGCCTTCAGCCTGAAGACATCGCGAC
GTACTATTGTCAGCAGTGGACCTCTAACCCTCCTACCTTTGGCGGTGGAACAAAGCTGGAGATCAAACGAGGCGG
GGGTGGCTCTGGGGGAGGAGGTTCCGGTGGGGGAGGCTCTCAAGTACAACTGCAACAAAGTGGGGCTGAAGTG
AAGAAGCCCGGTTCTTCAGTCAAAGTATCATGTAAGGCAAGTGGTTATACTTTTACGTCTTATAACATGCATTGGG
TAAAGCAAGCCCCTGGTCAGGGCCTCGAGTGGATTGGTGCGATCTACCCTGGAATGGGTGATACGAGCTATAATC
AGAAATTCAAGGGGAAAGCCACCTTGACTGCAGACGAATCTACGAACACGGCTTACATGGAGCTTAGCTCACTCA
GATCAGAGGATACAGCCTTTTACTACTGCGCTAGATCAACTTATTACGGAGGAGACTGGTATTTTGATGTATGGGG
TCAGGGGACCACAGTCACTGTTAGCTCTGCTAGCACCACGACGCCAGCGCCGCGACCACCAACACCGGCGCCCAC
CATCGCGTCGCAGCCCCTGTCCCTGCGCCCAGAGGCGTGCCGGCCAGCGGCGGGGGGCGCAGTGCACACGAGGG
GGCTGGACTTCGCCTGTGATTCCGGAATCTACATCTGGGCGCCCTTGGCCGGGACTTGTGGGGTCCTTCTCCTGTC
ACTGGTTATCACCCTTTACTGCAAACGGGGCAGAAAGAAACTCCTGTATATATTCAAACAACCATTTATGAGACCA
GTACAAACTACTCAAGAGGAAGATGGCTGTAGCTGCCGATTTCCAGAAGAAGAAGAAGGAGGATGTGAACTGAG
AGTGAAGTTCAGCAGGAGCGCAGACGCCCCCGCGTACCAGCAGGGCCAGAACCAGCTCTATAACGAGCTCAATCT
AGGACGAAGAGAGGAGTACGATGTTTTGGACAAGAGACGTGGCCGGGACCCTGAGATGGGGGGAAAGCCGAGA
AGGAAGAACCCTCAGGAAGGCCTGTACAATGAACTGCAGAAAGATAAGATGGCGGAGGCCTACAGTGAGATTGG
GATGAAAGGCGAGCGCCGGAGGGGCAAGGGGCACGATGGCCTTTACCAGGGTCTCAGTACAGCCACCAAGGAC
ACCTACGACGCCCTTCACATGCAGGCCCTGCCCCCTCGC

CD20bbz on-CAR amino acid sequence (SEQ ID NO: 3033)
Signal peptide-conditional aggregation domains (4 repeats)-Furin site-anti-CD20bbz

MALPVTALLLPLALLLHAARPGSRGVQVETISPGDGRTFPKRGQTCVVHYTGMLEDGKKMDSSRDRNKPFKFMLGKQ
EVIRGWEEGVAQMSVGQRAKLTISPDYAYGATGHPGIIPPHATLVFDVELLKLETRGVQVETISPGDGRTFPKRGQTCV
VHYTGMLEDGKKMDSSRDRNKPFKFMLGKQEVIRGWEEGVAQMSVGQRAKLTISPDYAYGATGHPGIIPPHATLVFD
VELLKLETRGVQVETISPGDGRTFPKRGQTCVVHYTGMLEDGKKMDSSRDRNKPFKFMLGKQEVIRGWEEGVAQMS
VGQRAKLTISPDYAYGATGHPGIIPPHATLVFDVELLKLETRGVQVETISPGDGRTFPKRGQTCVVHYTGMLEDGKKMD
SSRDRNKPFKFMLGKQEVIRGWEEGVAQMSVGQRAKLTISPDYAYGATGHPGIIPPHATLVFDVELLKLETSARNRRKR
GSDIQLTQSPSSLSASVGDRVTMTCRASSSVSYIHWFQQKPGKAPKPWIYATSNLASGVPVRFSGSGSGTDYTFTISSLQ
PEDIATYYCQQWTSNPPTFGGGTKLEIKRGGGGSGGGGSGGGGSQVQLQQSGAEVKKPGSSVKVSCKASGYTFTSYN
MHWVKQAPGQGLEWIGAIYPGMGDTSYNQKFKGKATLTADESTNTAYMELSSLRSEDTAFYYCARSTYYGGDWYFD
VWGQGTTVTVSSASTTTPAPRPPTPAPTIASQPLSLRPEACRPAAGGAVHTRGLDFACDSGIYIWAPLAGTCGVLLLSLV
ITLYCKRGRKKLLYIFKQPFMRPVQTTQEEDGCSCRFPEEEEGGCELRVKFSRSADAPAYQQGQNQLYNELNLGRREEY

DVLDKRRGRDPEMGGKPRRKNPQEGLYNELQKDKMAEAYSEIGMKGERRRGKGHDGLYQGLSTATKDTYDALHMQ
ALPPR

## Claims

1. A fusion protein, comprising two protein domains separated by a heterologous protease cleavage site, wherein a first of said protein domains is a degradation domain, and a second of said protein domains is a chimeric antigen receptor (CAR), wherein the degradation domain has a first state associated with a first level of surface expression of the fusion protein and a second state associated with a second level of surface expression of the fusion protein, wherein the second level is increased, e.g., by at least 2, 3, 4, 5, 10, 20 or 30 fold over the first level in the presence of a stabilization compound, further wherein the degradation domain is chosen from an estrogen receptor (ER) domain, an FKB protein (FKBP) domain or a dihydrofolate reductase (DHFR) domain.

2. The fusion protein of claim 1, wherein:
(i) the degradation domain from an estrogen receptor (ER) comprises an amino acid sequence that is at least 90, 95, 97, 98, 99, or 100% identical to either of SEQ ID NOs: 58 or 121;
(ii) the degradation domain from an FKB protein (FKBP) comprises an amino acid sequence that is at least 90, 95, 97, 98, 99, or 100% identical to SEQ ID NO: 56; or
(iii) the degradation domain from a dihydrofolate reductase (DHFR) comprises an amino acid sequence that is at least 90, 95, 97, 98, 99, or 100% identical to SEQ ID NO: 57.

3. The fusion protein of claim 1 or 2, wherein the stabilization compound is:
(i) selected from the group consisting of Bazedoxifene and 4-hydroxy tamoxifen (4-OHT) when the fusion protein comprises a degradation domain derived from an estrogen receptor; or
(ii) Shield-1 when the fusion protein comprises a degradation domain derived from an FKB protein.

4. The fusion protein of any one of claims 1-3, wherein the cleavage site:
(i) is cleaved by a mammalian intracellular protease, optionally wherein said cleavage site is cleaved by a protease selected from the group consisting of furin, PCSK1, PCSK5, PCSK6, PCSK7, cathepsin B, Granzyme B, Factor XA, Enterokinase, genenase, sortase, precission protease, thrombin, TEV protease, and elastase 1, the furin cleavage site e.g. being selected from the group consisting of RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); and CKINGYPKRGRKRR (SEQ ID NO: 137); and/or
(ii) comprises a polypeptide having a cleavage motif selected from the group consisting of RX(K/R)R consensus motif, RXXX[KR]R consensus motif, RRX consensus motif, I-E-P-D-X consensus motif (SEQ ID NO: 35), Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A consensus motif, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41), and [AGSV]-x (SEQ ID NO: 42).

5. The fusion protein of any one of claims 1-3, wherein the cleavage site is cleaved by a mammalian extracellular protease, optionally wherein:
(i) said mammalian extracellular protease is selected from the group consisting of Factor XA, Enterokinase, genenase, sortase, precission protease, thrombin, and elastase 1; or
(ii) said cleavage site comprises a polypeptide having an amino acid sequence selected from the group consisting of Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A consensus motif, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41), and [AGSV]-x (SEQ ID NO: 42).

6. The fusion protein of any one of claims 1-5, wherein said CAR comprises an antigen binding domain (optionally an scFv), a transmembrane domain, and one or more intracellular signaling domains.

7. The fusion protein of claim 6, wherein said one or more intracellular signaling domains comprise one or more primary signaling domains and/or one or more costimulatory signaling domains, optionally wherein:
(i) said one or more primary signaling domains comprise a CD3-zeta stimulatory domain; and/or
(ii) one or more of said costimulatory signaling domains is an intracellular domain from a costimulatory protein selected from the group consisting of CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, NKG2D, SLAMF7, NKp80, NKp30, NKp44, NKp46, CD160, B7-H3, and a ligand that specifically binds with CD83.

8. The fusion protein of claim 6 or 7, wherein the antigen binding domain binds an antigen selected from the group consisting of CD19; CD123; CD22; CD30; CD171; CS-1; C-type lectin-like molecule-1, CD33; epidermal growth factor receptor variant III (EGFRvIII); ganglioside G2 (GD2); ganglioside GD3; TNF receptor family member B cell maturation (BCMA); Tn antigen ((Tn Ag) or (GaINAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2; Mesothelin; Interleukin 11 receptor alpha (IL-11Ra); prostate stem cell antigen (PSCA); Protease Serine 21; vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3; transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1, melanoma antigen recognized by T cells 1; Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like, Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLL1).

9. The fusion protein of claim 8, wherein the antigen binding domain:
(a) binds to CD19, wherein the fusion protein comprises (i) an antigen binding domain comprising an amino acid sequence selected from any one of SEQ ID NOs: 356-368 or 381, or (ii) a chimeric antigen receptor comprising an amino acid sequence selected from any one of SEQ ID NOs: 897, 902, 907, 912, 917, 922, 927, 932, 937, 942, 947, 952, 956;
(b) binds to CD123, wherein the fusion protein comprises (i) an antigen binding domain comprising an amino acid sequence selected from any one of SEQ ID NOs: 751, 756, 761, or 766, or (ii) a chimeric antigen receptor comprising an amino acid sequence selected from any one of SEQ ID NOs: 750, 755, 760, or 765;
(c) binds to BCMA, wherein the fusion protein comprises (i) an antigen binding domain comprising an amino acid sequence selected from any one of SEQ ID NOs: 382, 386, 390, 394, 398, 402, 406, 410, 414, 418, 422, 426, 430, 434, 438, 442, 446, 450, 454, 458, 462, 466, 470, 474, 478, 482, 486, 490, 494, 498, 502, 506, 510, 514, 518, 522, 528, 531, 534, or 537, or (ii) a chimeric antigen receptor comprising an amino acid sequence selected from any one of SEQ ID NOs: 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, or 859; or
(d) binds to CD20, wherein the fusion protein comprises (i) an antigen binding domain comprising an amino acid sequence located at positions 470-712 or 470-939 of SEQ ID NO: 3033, or (ii) a chimeric antigen receptor comprising an amino acid sequence of SEQ ID NO: 3033.

10. The fusion protein of any one of claims 1-9, wherein:
(i) the degradation domain is located:
a) N-terminal to said second protein domain; or
b) C-terminal to said second protein domain; and/or
(ii) the fusion protein further comprises a signal peptide, e.g., comprises a signal peptide N-terminal to said degradation domain, optionally wherein the fusion protein further comprises a linker positioned between the signal peptide and another domain of the fusion protein, e.g., positioned between the signal peptide and said degradation domain.

11. The fusion protein of any one of claims 1-10, comprising an amino acid sequence selected from the group consisting of SEQ ID NOs: 59, 61, 63, 65, 67, 69, 75, 103, 105, 107, 109, 111, 113, 115, 141, 143, 145, 147, 149, 151, 153, 155, 157, 159, 161, 163, 165, 167, 169, 171, 173, 175, 177, 179, 181, 183, and 990-1049 .

12. A nucleic acid encoding the fusion protein of any one of claims 1-11.

13. A vector comprising the nucleic acid of claim 12, optionally wherein:
(i) said vector is a viral vector, e.g. a lentiviral vector; and/or
(ii) said vector is present in a viral particle.

14. A cell, e.g., a host cell, comprising the fusion protein of any one of claims 1-11, the nucleic acid of claim 12, or the vector of claim 13, optionally wherein said cell, e.g. host cell:
(a) is a human effector cell, e.g., a human T cell or a human NK cell; and/or
(b) further comprises a protease capable of cleaving said heterologous protease cleavage site.

15. The cell of claim 14, wherein:
(a) in the absence of a stabilization compound, the fusion protein is degraded by cellular degradation pathways, e.g., at least 50%, 60%, 70%, 80%, 90% or greater of the fusion protein is degraded; and/or
(b) said cell further comprises a stabilization compound, optionally wherein:
(i) the degradation domain assumes a conformation more resistant to cellular degradation in the presence of the stabilization compound, relative to a conformation in the absence of the stabilization compound; and/or
(ii) the conformation of the fusion protein is more permissive to cleavage at the heterologous protease cleavage site in the presence of the stabilization compound, relative to a conformation in the absence of the stabilization compound; or the level of cell surface expression of the fusion protein is greater, e.g., 2, 3, 4, 5, 6, 7, 8, 9, 10, 20, or 30 fold greater, than the level of cell surface expression of the fusion protein in a cell not comprising the stabilization compound.

16. A method of conditionally expressing a CAR on the surface of a cell, said method comprising:
contacting the fusion protein of any of claims 1-11, or the cell of claims 14-15, with a stabilization compound, wherein:
(a) in the presence of said stabilization compound, the surface expression of said CAR is increased, e.g., 2, 3, 4, 5, 6, 7, 8, 9, 10, 20, or 30 fold greater, relative to the level of surface expression of said CAR in the absence of said stabilization compound; and
(b) in the absence of said stabilization compound, the surface expression of said CAR is substantially decreased, e.g., 2, 3, 4, 5, 6, 7, 8, 9, 10, 20, or 30 fold less, relative to the level of surface expression of said CAR in the presence of the stabilization compound;
further wherein said method is not a method for treatment of the human or animal body by therapy.

17. The method of claim 16, wherein the presence of said stabilization compound:
(i) is associated with, e.g., causes, a change in conformation of the degradation domain from a first folding state to second folding state, wherein the first folding state is more susceptible to degradation relative to the second folding state; and/or
(ii) exposes said protease cleavage site, e.g., to a greater extent, e.g., 2, 3, 4, 5, 6, 7, 8, 9, 10, 20, or 30 fold greater, relative to the exposure of the protease cleavage site in the absence of said stabilization compound.

18. The fusion protein of any of claims 1-11, the nucleic acid of claim 12, the vector of claim 13, or the cell of claims 14-15, for use in the treatment of a disease associated with expression of a tumor antigen in a subject, e.g. a cancer.

19. The fusion protein, nucleic acid, vector or cell for use of claim 18, wherein the cancer is:
(i) mesothelioma (e.g., malignant pleural mesothelioma), e.g., in a subject who has progressed on at least one prior standard therapy; lung cancer (e.g., non-small cell lung cancer, small cell lung cancer, squamous cell lung cancer, or large cell lung cancer); pancreatic cancer (e.g., pancreatic ductal adenocarcinoma, or metastatic pancreatic ductal adenocarcinoma (PDA), e.g., in a subject who has progressed on at least one prior standard therapy); esophageal adenocarcinoma, ovarian cancer (e.g., serous epithelial ovarian cancer, e.g., in a subject who has progressed after at least one prior regimen of standard therapy), breast cancer, colorectal cancer, bladder cancer or any combination thereof;
(ii) a hematological cancer, e.g., a hematological cancer chosen from a leukemia or lymphoma; or
(iii) chosen from: chronic lymphocytic leukemia (CLL), mantle cell lymphoma (MCL), multiple myeloma, acute lymphoid leukemia (ALL), Hodgkin lymphoma, B-cell acute lymphoid leukemia (BALL), T-cell acute lymphoid leukemia (TALL), small lymphocytic lymphoma (SLL), B cell prolymphocytic leukemia, blastic plasmacytoid dendritic cell neoplasm, Burkitt's lymphoma, diffuse large B cell lymphoma (DLBCL), DLBCL associated with chronic inflammation, chronic myeloid leukemia, myeloproliferative neoplasms, follicular lymphoma, pediatric follicular lymphoma, hairy cell leukemia, small cell- or a large cell-follicular lymphoma, malignant lymphoproliferative conditions, MALT lymphoma (extranodal marginal zone lymphoma of mucosa-associated lymphoid tissue), Marginal zone lymphoma, myelodysplasia, myelodysplastic syndrome, non-Hodgkin lymphoma, plasmablastic lymphoma, plasmacytoid dendritic cell neoplasm, Waldenstrom macroglobulinemia, splenic marginal zone lymphoma, splenic lymphoma/leukemia, splenic diffuse red pulp small B-cell lymphoma, hairy cell leukemia-variant, lymphoplasmacytic lymphoma, a heavy chain disease, plasma cell myeloma, solitary plasmocytoma of bone, extraosseous plasmocytoma, nodal marginal zone lymphoma, pediatric nodal marginal zone lymphoma, primary cutaneous follicle center lymphoma, lymphomatoid granulomatosis, primary mediastinal (thymic) large B-cell lymphoma, intravascular large B-cell lymphoma, ALK+ large B-cell lymphoma, large B-cell lymphoma arising in HHV8-associated multicentric Castleman disease, primary effusion lymphoma, B-cell lymphoma, acute myeloid leukemia (AML), or unclassifiable lymphoma.

20. The fusion protein of any of claims 1-11, the nucleic acid of claim 12, the vector of claim 13, or the cell of claims 14-15 for use as a medicament.

## Patentansprüche

1. Fusionsprotein, umfassend zwei Proteindomänen, die durch eine heterologe Protease-Spaltungsstelle getrennt sind, wobei eine erste der Proteindomänen eine Abbaudomäne ist und eine zweite der Proteindomänen ein chimärer Antigenrezeptor (CAR) ist, wobei die Abbaudomäne einen ersten Zustand, der mit einem ersten Niveau der Oberflächenexpression des Fusionsproteins assoziiert ist, und einen zweiten Zustand, der mit einem zweiten Niveau der Oberflächenexpression des Fusionsproteins assoziiert ist, aufweist, wobei in Anwesenheit einer Stabilisierungsverbindung das zweite Niveau z. B. um mindestens das 2, 3, 4, 5, 10, 20 oder 30 fache über das erste Niveau erhöht ist, wobei die Abbaudomäne ferner aus einer Östrogenrezeptor (ER)-Domäne, einer FKB-Protein (FKBP)-Domäne oder einer Dihydrofolatreduktase (DHFR)-Domäne ausgewählt ist.

2. Fusionsprotein nach Anspruch 1, wobei:
(i) die Abbaudomäne aus einem Östrogenrezeptor (ER) eine Aminosäuresequenz umfasst, die zu mindestens 90, 95, 97, 98, 99 oder 100% mit einer der SEQ ID NOs: 58 oder 121 identisch ist;
(ii) die Abbaudomäne aus einem FKB-Protein (FKBP) eine Aminosäuresequenz umfasst, die zu mindestens 90, 95, 97, 98, 99 oder 100% mit SEQ ID NO: 56 identisch ist; oder
(iii) die Abbaudomäne aus einer Dihydrofolatreduktase (DHFR) eine Aminosäuresequenz umfasst, die zu mindestens 90, 95, 97, 98, 99 oder 100% mit SEQ ID NO: 57 identisch ist.

3. Fusionsprotein nach Anspruch 1 oder 2, wobei die Stabilisierungsverbindung:
(i) ausgewählt ist aus der Gruppe bestehend aus Bazedoxifen und 4-Hydroxytamoxifen (4-OHT), wenn das Fusionsprotein eine von einem Östrogenrezeptor abgeleitete Abbaudomäne umfasst; oder
(ii) Shield-1 ist, wenn das Fusionsprotein eine von einem FKB-Protein abgeleitete Abbaudomäne umfasst.

4. Fusionsprotein nach einem der Ansprüche 1-3, wobei die Spaltungsstelle:
(i) durch eine intrazelluläre Säuger-Protease gespalten wird, wobei die Spaltungsstelle optional durch eine Protease gespalten wird, die ausgewählt ist aus der Gruppe bestehend aus Furin, PCSK1, PCSK5, PCSK6, PCSK7, Cathepsin B, Granzym B, Faktor XA, Enterokinase, Genenase, Sortase, Precission-Protease, Thrombin, TEV-Protease und Elastase 1, wobei die Furin-Spaltungsstelle z. B. ausgewählt ist aus der Gruppe bestehend aus RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); und CKINGYPKRGRKRR (SEQ ID NO: 137); und/oder
(ii) ein Polypeptid umfasst, das ein Spaltungsmotiv aufweist, ausgewählt aus der Gruppe bestehend aus RX(K/R)R-Konsensusmotiv, RXXX[KR]R-Konsensusmotiv, RRX-Konsensusmotiv, I-E-P-D-X-Konsensusmotiv (SEQ ID NO: 35), Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A-Konsensusmotiv, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41) und [AGSV]-x (SEQ ID NO: 42).

5. Fusionsprotein nach einem der Ansprüche 1-3, wobei die Spaltungsstelle durch eine extrazelluläre Säuger-Protease gespalten wird, wobei optional:
(i) die extrazelluläre Säuger-Protease ausgewählt ist aus der Gruppe bestehend aus Faktor XA, Enterokinase, Genenase, Sortase, Precission-Protease, Thrombin und Elastase 1; oder
(ii) die Spaltungsstelle ein Polypeptid umfasst, aufweisend eine Aminosäuresequenz, ausgewählt aus der Gruppe bestehend aus Glu/Asp-Gly-Arg, Asp-Asp-Asp-Asp-Lys (SEQ ID NO: 36), Pro-Gly-Ala-Ala-His-Tyr (SEQ ID NO: 37), LPXTG/A-Konsensusmotiv, Leu-Glu-Val-Phe-Gln-Gly-Pro (SEQ ID NO: 38), Leu-Val-Pro-Arg-Gly-Ser (SEQ ID NO: 40), E-N-L-Y-F-Q-G (SEQ ID NO: 41) und [AGSV]-x (SEQ ID NO: 42).

6. Fusionsprotein nach einem der Ansprüche 1-5, wobei der CAR eine Antigenbindungsdomäne (optional ein scFv), eine Transmembrandomäne und eine oder mehrere intrazelluläre Signalisierungsdomänen umfasst.

7. Fusionsprotein nach Anspruch 6, wobei die eine oder die mehreren intrazellulären Signalisierungsdomänen eine oder mehrere primäre Signalisierungsdomänen und/oder eine oder mehrere costimulatorische Signalisierungsdomänen umfassen, wobei optional:
(i) die eine oder die mehreren primären Signalisierungsdomänen eine CD3-zeta-stimulatorische Domäne umfassen; und/oder
(ii) es sich bei einer oder mehreren der costimulatorischen Signalisierungsdomänen um eine intrazelluläre Domäne aus einem costimulatorischen Protein handelt, ausgewählt aus der Gruppe bestehend aus CD27, CD28, 4-1BB (CD137), OX40, GITR, CD30, CD40, ICOS, BAFFR, HVEM, ICAM-1, Lymphozytenfunktions-assoziiertes Antigen-1 (LFA-1), CD2, CDS, CD7, CD287, LIGHT, NKG2C, NKG2D, SLAMF7, NKp80, NKp30, NKp44, NKp46, CD160, B7-H3 und einem Liganden, der spezifisch mit CD83 bindet.

8. Fusionsprotein nach Anspruch 6 oder 7, wobei die Antigenbindungsdomäne ein Antigen bindet, ausgewählt aus der Gruppe, bestehend aus CD19; CD123; CD22; CD30; CD171; CS-1; C-Typ-Lektin-ähnliches-Molekül-1, CD33; Epidermal-Wachstumsfaktor-Rezeptor Variante III (EGFRvIII); Gangliosid G2 (GD2); Gangliosid GD3; einem Mitglied der TNF-Rezeptorfamilie "B-Cell-Maturation" (BCMA); Tn-Antigen ((Tn Ag) oder (GalNAcα-Ser/Thr)); Prostata-spezifisches-Membranantigen (PSMA); Rezeptortyrosinkinase-ähnlicher Orphan-Rezeptor 1 (ROR1); Fmsähnliche Tyrosinkinase 3 (FLT3); Tumorassoziiertes Glykoprotein 72 (TAG72); CD38; CD44v6; Carcinoembryonales Antigen (CEA); Epithelzell-Adhäsionsmolekül (EPCAM); B7H3 (CD276); KIT (CD117); Interleukin-13-Rezeptor-Untereinheit alpha-2; Mesothelin; Interleukin-11-Rezeptor-alpha (IL-11Ra); Prostata-Stammzellen-Antigen (PSCA); Protease Serin 21; Vaskulär-Endothelialer-Wachstumsfaktor-Rezeptor 2 (VEGFR2); Lewis(Y)-Antigen; CD24; Blutplättchen-abgeleiteter-Wachstumsfaktor-Rezeptor beta (PDGFR-beta); Stadienspezifisches embryonales Antigen-4 (SSEA-4); CD20; Folatrezeptor alpha; Rezeptor-Tyrosin-Proteinkinase ERBB2 (Her2/neu); Mucin 1, zelloberflächenassoziiert (MUC1); Epidermal-Wachstumsfaktor-Rezeptor (EGFR); neurales Zelladhäsionsmolekül (NCAM); Prostase; Prostataspezifische saure Phosphatase (PAP); Elongationsfaktor 2, mutiert (ELF2M); Ephrin B2; Fibroblastenaktivierungsprotein alpha (FAP); Insulinähnlicher Wachstumsfaktor 1-Rezeptor (IGF-I-Rezeptor), Carboanhydrase IX (CAIX); Proteasom (Prosom, Macropain)-Untereinheit, Beta-Typ, 9 (LMP2); Glykoprotein 100 (gp100); Onkogen-Fusionsprotein, bestehend aus Breakpoint-Cluster-Region (BCR) und Abelson-Maus-Leukämievirus-Onkogen-Homolog 1 (Abl) (bcr-abl); Tyrosinase; Ephrin-Typ-A-Rezeptor 2 (EphA2); Fucosyl-GM1; Sialyl-Lewis-Adhäsionsmolekül (sLe); Gangliosid GM3; Transglutaminase 5 (TGS5); Melanom-assoziiertes Antigen hohen Molekulargewichts ("High Molecular Weight-Melanoma-Associated Antigen", HMWMAA); o-Acetyl-GD2-Gangliosid (OAcGD2); Folatrezeptor beta; Tumor-Endothelial-Marker 1 (TEM1/CD248); Tumor-Endothelial-Marker-7-related (TEM7R); Claudin 6 (CLDN6); Thyreoidea-stimulierendes-Hormon-Rezeptor (TSHR); G-Protein-gekoppelter Rezeptor Klasse C Gruppe 5, Mitglied D (GPRC5D); Chromosom X offener Leserahmen 61 (CXORF61); CD97; CD179a; anaplastische Lymphomkinase (ALK); Polysialinsäure; Placenta-specific 1 (PLAC1); Hexasaccharidanteil von GloboH-Glycoceramid (GloboH); Milchdrüsen-Differenzierungsantigen (NY-BR-1); Uroplakin 2 (UPK2); Hepatitis-A-Virus-zellulärer-Rezeptor 1 (HAVCR1); Beta-3-Adrenozeptor (ADRB3); Pannexin 3 (PANX3); G-Protein-gekoppelter Rezeptor 20 (GPR20); Lymphozyten-Antigen-6-Komplex, Locus K 9 (LY6K); Olfaktorischer Rezeptor 51E2 (OR51E2); TCR Gamma Alternate Reading Frame Protein (TARP); Wilms-Tumor-Protein (WT1); Tumor/Hoden-Antigen 1 (NY-ESO-1); Tumor/Hoden-Antigen 2 (LAGE-1a); Melanom-assoziiertes Antigen 1 (MAGE-A1); ETS-Translokationsvariante Gen 6, lokalisiert auf Chromosom 12p (ETV6-AML); Spermien-Protein 17 (SPA17); X-Antigenfamilie, Mitglied 1A (XAGE1); Angiopoietin-bindender Zelloberflächenrezeptor 2 (Tie 2); Melanom-Tumor-Hoden-Antigen-1 (MAD-CT-1); Melanom-Tumor-Hoden-Antigen-2 (MAD-CT-2); Fos-related Antigen 1; Tumorprotein p53 (p53); p53-Mutante; Prostein; Survivin; Telomerase; Prostatakarzinom-Tumorantigen-1, von T-Zellen erkanntes Melanom-Antigen 1; "Rat-Sarcom" (Ras)-Mutante; humane Telomerase-Reverse-Transkriptase (hTERT); Sarkom-Translokations-Breakpoints; Melanom-Apoptose-Inhibitor ("melanoma inhibitor of apoptosis", ML-IAP); ERG (Transmembranprotease, Serin 2 (TMPRSS2) ETS-Fusionsgen) ; N-Acetylglucosaminyl-Transferase V (NA17); Paired-Box-Protein Pax-3 (PAX3); Androgenrezeptor; Cyclin B1; v-myc aviäre Myelocytomatose virales Onkogen-Neuroblastom-abgeleitetes Homolog (MYCN); Ras-Homolog-Familienmitglied C (RhoC); Tyrosinase-verwandtes Protein 2 (TRP-2); Cytochrom P450 1B1 (CYP1B1); CCCTC-Bindungsfaktor (Zinkfingerprotein)-Like; "Squamous Cell Carcinoma Antigen Recognized By T Cells 3" (SART3); Paired-Box-Protein Pax-5 (PAX5); Proacrosin-bindendes Protein sp32 (OY-TES1); lymphozytenspezifische Proteintyrosinkinase (LCK); A-Kinase-Ankerprotein 4 (AKAP-4); Synovial-Sarcoma-X-Breakpoint 2 (SSX2); "Receptor for Advanced Glycation Endproducts" (RAGE-1); "Renal ubiquitous 1" (RU1); "Renal ubiquitous 2" (RU2); Legumain; Human-Papillomavirus-E6 (HPV E6); Human-Papillomavirus-E7 (HPV E7); intestinale Carboxylesterase; Hitzeschockprotein 70-2 mutiert (mut hsp70-2); CD79a; CD79b; CD72; Leukozyten-assoziierter Immunglobulin-ähnlicher Rezeptor 1 (LAIR1); Fc-Fragment des IgA-Rezeptors (FCAR oder CD89); Leukozyten-Immunglobulin-ähnlicher Rezeptor-Unterfamilie A Mitglied 2 (LILRA2); CD300-Molekülähnliches Familienmitglied f (CD300LF); C-Typ-Lektin-Domäne-Familie 12 Mitglied A (CLEC12A); Knochenmark-Stromazellen-Antigen 2 (BST2); "EGF-like module-containing mucin-like hormone receptor-like 2" (EMR2); Lymphozyten-Antigen 75 (LY75); Glypican-3 (GPC3); "Fc Receptor-like 5" (FCRL5); und "Immunglobulin lambda-like Polypeptide 1" (IGLL1).

9. Fusionsprotein nach Anspruch 8, wobei die Antigenbindungsdomäne:
(a) an CD19 bindet, wobei das Fusionsprotein (i) eine Antigenbindungsdomäne, umfassend eine Aminosäuresequenz, ausgewählt aus einer der SEQ ID NOs: 356-368 oder 381, oder (ii) einen chimären Antigenrezeptor, umfassend eine Aminosäuresequenz, ausgewählt aus einer der SEQ ID NOs: 897, 902, 907, 912, 917, 922, 927, 932, 937, 942, 947, 952, 956, umfasst;
(b) an CD123 bindet, wobei das Fusionsprotein (i) eine Antigenbindungsdomäne, umfassend eine Aminosäuresequenz, ausgewählt aus einer der SEQ ID NOs: 751, 756, 761 oder 766, oder (ii) einen chimären Antigenrezeptor, umfassend eine Aminosäuresequenz, ausgewählt aus einer der SEQ ID NOs: 750, 755, 760 oder 765, umfasst;
(c) an BCMA bindet, wobei das Fusionsprotein (i) eine Antigenbindungsdomäne, umfassend eine Aminosäuresequenz, ausgewählt aus einer der SEQ ID NOs: 382, 386, 390, 394, 398, 402, 406, 410, 414, 418, 422, 426, 430, 434, 438, 442, 446, 450, 454, 458, 462, 466, 470, 474, 478, 482, 486, 490, 494, 498, 502, 506, 510, 514, 518, 522, 528, 531, 534 oder 537, oder (ii) einen chimären Antigenrezeptor, umfassend eine Aminosäuresequenz, ausgewählt aus einer der SEQ ID NOs: 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, oder 859, umfasst; oder
(d) an CD20 bindet, wobei das Fusionsprotein (i) eine Antigenbindungsdomäne, umfassend eine Aminosäuresequenz, die an den Positionen 470-712 oder 470-939 der SEQ ID NO: 3033 lokalisiert ist, oder (ii) einen chimären Antigenrezeptor, umfassend eine Aminosäuresequenz von SEQ ID NO: 3033, umfasst.

10. Fusionsprotein nach einem der Ansprüche 1-9, wobei:
(i) die Abbaudomäne lokalisiert ist:
a) N-terminal zu der zweiten Proteindomäne; oder
b) C-terminal zu der zweiten Proteindomäne; und/oder
(ii) das Fusionsprotein ferner ein Signalpeptid umfasst, z. B. ein Signalpeptid N-terminal zu der Abbaudomäne umfasst, wobei das Fusionsprotein optional ferner einen Linker umfasst, der zwischen dem Signalpeptid und einer anderen Domäne des Fusionsproteins positioniert ist, z. B. zwischen dem Signalpeptid und der Abbaudomäne positioniert ist.

11. Fusionsprotein nach einem der Ansprüche 1-10, umfassend eine Aminosäuresequenz ausgewählt aus der Gruppe bestehend aus SEQ ID NOs: 59, 61, 63, 65, 67, 69, 75, 103, 105, 107, 109, 111, 113, 115, 141, 143, 145, 147, 149, 151, 153, 155, 157, 159, 161, 163, 165, 167, 169, 171, 173, 175, 177, 179, 181, 183 und 990-1049.

12. Nukleinsäure, die das Fusionsprotein nach einem der Ansprüche 1-11 codiert.

13. Vektor, umfassend die Nukleinsäure nach Anspruch 12, wobei optional:
(i) der Vektor ein viraler Vektor, z. B. ein lentiviraler Vektor, ist; und/oder
(ii) der Vektor in einem viralen Partikel vorhanden ist.

14. Zelle, z. B. eine Wirtszelle, umfassend das Fusionsprotein nach einem der Ansprüche 1-11, die Nukleinsäure nach Anspruch 12 oder den Vektor nach Anspruch 13, wobei die Zelle, z. B. Wirtszelle, optional:
(a) eine menschliche Effektorzelle, z. B. eine menschliche T-Zelle oder eine menschliche NK-Zelle ist; und/oder
(b) ferner eine Protease umfasst, die in der Lage ist, die heterologe Protease-Spaltungsstelle zu spalten.

15. Zelle nach Anspruch 14, wobei:
(a) in Abwesenheit einer Stabilisierungsverbindung das Fusionsprotein durch zelluläre Abbauwege abgebaut wird, wobei z. B. mindestens 50%, 60%, 70%, 80%, 90% oder mehr des Fusionsproteins abgebaut werden; und/oder
(b) die Zelle ferner eine Stabilisierungsverbindung umfasst, wobei optional:
(i) die Abbaudomäne in Anwesenheit der Stabilisierungsverbindung eine Konformation annimmt, die gegenüber zellulärem Abbau widerstandsfähiger ist, relativ zu einer Konformation in Abwesenheit der Stabilisierungsverbindung; und/oder
(ii) die Konformation des Fusionsproteins in Anwesenheit der Stabilisierungsverbindung permissiver für eine Spaltung an der heterologen Protease-Spaltungsstelle, relativ zu einer Konformation in Abwesenheit der Stabilisierungsverbindung, ist; oder das Niveau der Zelloberflächenexpression des Fusionsproteins größer, z. B. 2, 3, 4, 5, 6, 7, 8, 9, 10, 20 oder 30 fach größer, ist als das Niveau der Zelloberflächenexpression des Fusionsproteins in einer Zelle, die die Stabilisierungsverbindung nicht umfasst.

16. Verfahren zur konditionalen Expression eines CAR auf der Oberfläche einer Zelle, wobei das Verfahren Folgendes umfasst:
Inkontaktbringen des Fusionsproteins nach einem der Ansprüche 1-11 oder der Zelle nach den Ansprüchen 14-15 mit einer Stabilisierungsverbindung, wobei:
(a) in Anwesenheit der Stabilisierungsverbindung die Oberflächenexpression des CAR erhöht ist, z. B. 2, 3, 4, 5, 6, 7, 8, 9, 10, 20 oder 30 fach größer ist, relativ zu dem Niveau der Oberflächenexpression des CAR in Abwesenheit der Stabilisierungsverbindung; und
(b) in Abwesenheit der Stabilisierungsverbindung die Oberflächenexpression des CAR wesentlich verringert ist, z. B. 2, 3, 4, 5, 6, 7, 8, 9, 10, 20 oder 30 fach geringer ist, relativ zu dem Niveau der Oberflächenexpression des CAR in Anwesenheit der Stabilisierungsverbindung;
wobei das Verfahren ferner kein Verfahren zur therapeutischen Behandlung des menschlichen oder tierischen Körpers ist.

17. Verfahren nach Anspruch 16, wobei die Anwesenheit der Stabilisierungsverbindung:
(i) mit einer Änderung der Konformation der Abbaudomäne von einem ersten Faltungszustand zu einem zweiten Faltungszustand assoziiert ist, z. B. eine solche verursacht, wobei der erste Faltungszustand relativ zu dem zweiten Faltungszustand anfälliger für einen Abbau ist; und/oder
(ii) die Protease-Spaltungsstelle exponiert, z. B. in einem stärkeren Ausmaß, z. B. 2, 3, 4, 5, 6, 7, 8, 9, 10, 20 oder 30 fach stärker, relativ zu der Exponierung der Protease-Spaltungsstelle in Abwesenheit der Stabilisierungsverbindung.

18. Fusionsprotein nach einem der Ansprüche 1-11, Nukleinsäure nach Anspruch 12, Vektor nach Anspruch 13 oder Zelle nach den Ansprüchen 14-15 zur Verwendung bei der Behandlung einer Krankheit, die mit der Expression eines Tumorantigens assoziiert ist, in einem Subjekt, z. B. einer Krebserkrankung.

19. Fusionsprotein, Nukleinsäure, Vektor oder Zelle zur Verwendung nach Anspruch 18, wobei der Krebs:
(i) Mesotheliom (z. B. malignes Pleuramesotheliom), z. B. bei einem Subjekt, das unter mindestens einer vorangegangenen Standardtherapie einen progredienten Verlauf aufwies; Lungenkrebs (z. B. nicht-kleinzelliger Lungenkrebs, kleinzelliger Lungenkrebs, Plattenepithel-Lungenkrebs oder großzelliger Lungenkrebs); Bauchspeicheldrüsenkrebs (z. B. duktales Adenokarzinom des Pankreas oder metastasierendes duktales Adenokarzinom des Pankreas (PDA), z. B. bei einem Subjekt, das unter mindestens einer vorangegangenen Standardtherapie einen progredienten Verlauf aufwies); Ösophagus-Adenokarzinom, Eierstockkrebs (z. B. seröser epithelialer Eierstockkrebs, z. B. bei einem Subjekt, das nach mindestens einer vorangegangenen Standardtherapie einen progredienten Verlauf aufwies), Brustkrebs, kolorektaler Krebs, Blasenkrebs oder eine beliebige Kombination davon ist;
(ii) ein hämatologischer Krebs ist, z. B. ein hämatologischer Krebs, ausgewählt aus einer Leukämie oder einem Lymphom; oder
(iii) ausgewählt ist aus: chronischer lymphatischer Leukämie (CLL), Mantelzell-Lymphom (MCL), multiplem Myelom, akuter lymphatischer Leukämie (ALL), Hodgkin-Lymphom, akuter lymphatischer B-Zell-Leukämie (BALL), akuter lymphatischer T-Zell-Leukämie (TALL), kleinzelligem lymphozytischem Lymphom (SLL), prolymphozytischer B-Zell-Leukämie, blastischem plasmazytoidem dendritischem Zell-Neoplasma, Burkitt-Lymphom, diffusem großzelligen B-Zell-Lymphom (DLBCL), DLBCL in Verbindung mit chronischer Entzündung, chronischer myeloischer Leukämie, myeloproliferativen Neoplasmen, follikulärem Lymphom, pädiatrischem follikulärem Lymphom, Haarzell-Leukämie, kleinzelligem oder großzelligem follikulärem Lymphom, malignen lymphoproliferativen Erkrankungen, MALT-Lymphom (extranodales Marginalzonen-Lymphom des Schleimhaut-assoziierten lymphatischen Gewebes), Marginalzonen-Lymphom, Myelodysplasie, myelodysplastischem Syndrom, Non-Hodgkin-Lymphom, plasmablastischem Lymphom, plasmazytoidem dendritischem Zell-Neoplasma, Waldenström-Makroglobulinämie, Marginalzonen-Lymphom der Milz, Milz-Lymphom/Leukämie, kleinzelligem B-Zell-Lymphom der Milz mit diffuser roter Pulpa, Haarzell-Leukämie-Variante, lymphoplasmozytischem Lymphom, einer Schwerkettenkrankheit, Plasmazellmyelom, solitärem Plasmozytom des Knochens, extraossärem Plasmozytom, nodalem Marginalzonen-Lymphom, pädiatrischem nodalen Marginalzonen-Lymphom, primärem kutanen Follikelzentrums-Lymphom, lymphomatoider Granulomatose, primärem mediastinalem (thymischem) großzelligem B-Zell-Lymphom, intravaskulärem großzelligem B-Zell-Lymphom, ALK+ großzelligem B-Zell-Lymphom, großzelligem B-Zell-Lymphom entstehend bei HHV8-assoziierter multizentrischer Castleman-Krankheit, primärem Erguss-Lymphom, B-Zell-Lymphom, akuter myeloischer Leukämie (AML) oder nicht klassifizierbarem Lymphom.

20. Fusionsprotein nach einem der Ansprüche 1-11, Nukleinsäure nach Anspruch 12, Vektor nach Anspruch 13 oder Zelle nach den Ansprüchen 14-15 zur Verwendung als Medikament.

## Revendications

1. Protéine de fusion, comprenant deux domaines protéiques séparés par un site de clivage de protéase hétérologue, dans laquelle un premier desdits domaines protéiques est un domaine de dégradation, et un second desdits domaines protéiques est un récepteur d'antigène chimérique (CAR), dans laquelle le domaine de dégradation a un premier état associé à un premier niveau d'expression de surface de la protéine de fusion et un second état associé à un second niveau d'expression de surface de la protéine de fusion, dans laquelle le second niveau est augmenté, par ex., d'au moins 2, 3, 4, 5, 10, 20 ou 30 fois par rapport au premier niveau en présence d'un composé de stabilisation, dans laquelle en outre le domaine de dégradation est choisi parmi un domaine de récepteur d'oestrogène (ER), un domaine de protéine FKB (FKBP) ou un domaine de dihydrofolate réductase (DHFR).

2. Protéine de fusion selon la revendication 1, dans laquelle:
(i) le domaine de dégradation d'un récepteur d'œstrogènes (ER) comprend une séquence d'acides aminés qui est au moins 90, 95, 97, 98, 99 ou 100% identique à l'une ou l'autre des SEQ ID NO: 58 ou 121;
(ii) le domaine de dégradation provenant d'une protéine FKB (FKBP) comprend une séquence d'acides aminés qui est au moins 90, 95, 97, 98, 99 ou 100% identique à la SEQ ID NO: 56; ou
(iii)le domaine de dégradation d'une dihydrofolate réductase (DHFR) comprend une séquence d'acides aminés qui est au moins 90, 95, 97, 98, 99 ou 100% identique à la SEQ ID NO: 57.

3. Protéine de fusion selon la revendication 1 ou 2, dans laquelle le composé de stabilisation est:
(i)choisi dans le groupe constitué par le Bazédoxifène et le 4-hydroxy tamoxifène (4-OHT) lorsque la protéine de fusion comprend un domaine de dégradation dérivé d'un récepteur d'œstrogènes; ou
(ii) Shield-1 lorsque la protéine de fusion comprend un domaine de dégradation dérivé d'une protéine FKB.

4. Protéine de fusion selon l'une quelconque des revendications 1 à 3, dans laquelle le site de clivage:
(i) est clivé par une protéase intracellulaire de mammifère, éventuellement dans lequel ledit site de clivage est clivé par une protéase choisie dans le groupe constitué par la furine, PCSK1, PCSK5, PCSK6, PCSK7, la cathepsine B, le granzyme B, le facteur XA, l'entérokinase, la génénase, la sortase, la protéase de précission, la thrombine, la protéase TEV et l'élastase 1, le site de clivage de la furine *par ex.* étant choisi dans le groupe constitué par RTKR (SEQ ID NO: 123); GTGAEDPRPSRKRRSLGDVG (SEQ ID NO: 125); GTGAEDPRPSRKRR (SEQ ID NO: 127); LQWLEQQVAKRRTKR (SEQ ID NO: 129); GTGAEDPRPSRKRRSLGG (SEQ ID NO: 131); GTGAEDPRPSRKRRSLG (SEQ ID NO: 133); SLNLTESHNSRKKR (SEQ ID NO: 135); et CKINGYPKRGRKR (SEQ ID NO: 137); et/ou
(ii) comprend un polypeptide ayant un motif de clivage choisi dans le groupe constitué par le motif consensus RX(K/R)R, le motif consensus RXXX[KR]R, le motif consensus RRX, le motif consensus I-E-P-D X (SEQ ID NO: 35), Glu/Asp-Gly-Arg, Asp-Asp-Asp-Asp-Lys (SEQ ID NO: 36), Pro-Gly-Ala-Ala-His-Tyr (SEQ ID NO: 37), le motif de consensus LPXTG/A, Leu-Glu-Val-Phe Gln-Gly-Pro (SEQ ID NO: 38), Leu-Val-Pro-Arg-Gly-Ser (SEQ ID NO: 40), E-N-L-Y-F-Q-G (SEQ ID NO: 41), et [AGSV]-x (SEQ ID NO: 42).

5. Protéine de fusion selon l'une quelconque des revendications 1 à 3, dans laquelle le site de clivage est clivé par une protéase extracellulaire de mammifère, éventuellement dans laquelle:
(i)ladite protéase extracellulaire de mammifère est choisie dans le groupe constitué par le facteur XA, l'entérokinase, la génénase, la sortase, la protéase de précission, la thrombine et l'élastase 1; ou
(ii) ledit site de clivage comprend un polypeptide ayant une séquence d'acides aminés choisie dans le groupe constitué par Glu/Asp-Gly-Arg, Asp-Asp-Asp-Asp-Lys (SEQ ID NO: 36), Pro Gly-Ala-Ala-His-Tyr (SEQ ID NO: 37), le motif consensus LPXTG/A, Leu-Glu-Val-Phe-Gln-Gly Pro (SEQ ID NO: 38), Leu-Val-Pro-Arg-Gly-Ser (SEQ ID NO: 40), E-N-L-Y-F-Q-G (SEQ ID NO: 41), et [AGSV]-x (SEQ ID NO: 42).

6. Protéine de fusion selon l'une quelconque des revendications 1 à 5, dans laquelle ledit CAR comprend un domaine de liaison à l'antigène (éventuellement un scFv), un domaine transmembranaire, et un ou plusieurs domaines de signalisation intracellulaire.

7. Protéine de fusion selon la revendication 6, dans laquelle lesdits un ou plusieurs domaines de signalisation intracellulaire comprennent un ou plusieurs domaines de signalisation primaire et/ou un ou plusieurs domaines de signalisation costimulatrice, éventuellement dans laquelle:
(i) lesdits un ou plusieurs domaines de signalisation primaires comprennent un domaine de stimulation de CD3-zêta; et/ou
(ii) un ou plusieurs desdits domaines de signalisation costimulatrice est un domaine intracellulaire d'une protéine costimulatrice choisie dans le groupe constitué par CD27, CD28, 4-1BB (CD137), OX40, GITR, CD30, CD40, ICOS, BAFFR, HVEM, ICAM-1, antigène-1 associé à la fonction lymphocytaire (LFA-1), CD2, CDS, CD7, CD287, LIGHT, NKG2C, NKG2D, SLAMF7, NKp80, NKp30, NKp44, NKp46, CD160, B7-H3, et un ligand qui se lie spécifiquement à CD83.

8. Protéine de fusion selon la revendication 6 ou 7, dans laquelle le domaine de liaison à l'antigène se lie à un antigène choisi dans le groupe constitué par CD19; CD123; CD22; CD30; CD171; CS-1; molécule de type lectine-1 de type C, CD33; récepteur du facteur de croissance épidermique variant III (EGFRvIII); ganglioside G2 (GD2); ganglioside GD3; Membre de la famille des récepteurs du TNF, maturation des cellules B (BCMA); antigène Tn ((Tn Ag) ou (GalNAcα-Ser/Thr)); antigène de la membrane spécifique de la prostate (PSMA); récepteur orphelin 1 de type tyrosine kinase (ROR1); Fms-Like Tyrosine Kinase 3 (FLT3); glycoprotéine 72 associée aux tumeurs (TAG72); CD38; CD44v6; antigène carcinoembryonnaire (CEA); Molécule d'adhésion des cellules épithéliales (EPCAM); B7H3 (CD276); KIT (CD117); sous-unité alpha-2 du récepteur de l'interleukine-13; mésothéline; récepteur alpha de l'interleukine 11 (IL-11Ra); antigène des cellules souches de la prostate (PSCA); protéase sérine 21; récepteur 2 du facteur de croissance de l'endothélium vasculaire (VEGFR2); antigène Lewis(Y); CD24; Récepteur bêta du facteur de croissance dérivé des plaquettes (PDGFR-bêta); Antigène embryonnaire spécifique du stade 4 (SSEA-4); CD20; Récepteur alpha des folates; Récepteur tyrosine-protéine kinase ERBB2 (Her2/neu); Mucine 1, associée à la surface cellulaire (MUC1); Récepteur du facteur de croissance épidermique (EGFR); Molécule d'adhésion des cellules neurales (NCAM); Prostase; phosphatase acide prostatique (PAP); facteur d'élongation 2 muté (ELF2M); éphrine B2; protéine d'activation des fibroblastes alpha (FAP); récepteur du facteur de croissance 1 analogue à l'insuline (récepteur de l'IGF-1), anhydrase carbonique IX (CAIX); sous-unité de protéasome (prosome, macropaïne), type bêta, 9 (LMP2); glycoprotéine 100 (gp100); protéine de fusion oncogénique constituée de la région du breakpoint cluster (BCR) et de l'homologue 1 de l'oncogène viral de la leucémie murine d'Abelson (Abl) (bcr-abl); tyrosinase; récepteur 2 de l'éphrine de type A (EphA2); Fucosyl GM1; molécule d'adhésion de Lewis sialyle (sLe); ganglioside GM3; transglutaminase 5 (TGS5); antigène associé au mélanome de haut poids moléculaire (HMWMAA); o-acétyl-GD2 ganglioside (OAcGD2); récepteur de folate bêta; marqueur endothélial de tumeur 1 (TEM1/CD248); marqueur endothélial de tumeur 7 lié (TEM7R); claudine 6 (CLDN6); récepteur de l'hormone thyréostimulante (TSHR); récepteur couplé à la protéine G classe C groupe 5, membre D (GPRC5D); cadre de lecture ouvert 61 du chromosome X (CXORF61); CD97; CD179a; lymphome kinase anaplasique (ALK); acide polysialique; spécifique du placenta 1 (PLAC1); portion hexasaccharide du globoH glycocéramide (GloboH); antigène de différenciation de la glande mammaire (NY-BR-1); uroplakine 2 (UPK2); récepteur cellulaire 1 du virus de l'hépatite A (HAVCR1); adrénorécepteur bêta 3 (ADRB3); pannexine 3 (PANX3); récepteur 20 couplé à la protéine G (GPR20); complexe antigène lymphocyte 6, locus K 9 (LY6K); récepteur olfactif 51E2 (OR51E2); protéine du cadre de lecture alternatif du TCR Gamma (TARP); protéine de la tumeur de Wilms (WT1); antigène 1 du cancer/du testicule (NY ESO-1); antigène 2 du cancer/du testicule (LAGE-1a); antigène 1 associé au mélanome (MAGE-A1); gène 6 de la translocation-variante ETS, situé sur le chromosome 12p (ETV6-AML); protéine 17 du sperme (SPA17); famille des antigènes X, membre 1A (XAGE1); récepteur 2 de la surface cellulaire se liant à l'angiopoïétine (Tie 2); antigène testiculaire-1 du cancer du mélanome (MAD-CT-1); antigène testiculaire-2 du cancer du mélanome (MAD-CT-2); antigène 1 lié à Fos; protéine tumorale p53 (p53); mutant de p53; prostéine; survivine; télomérase; antigène tumoral du carcinome de la prostate-1, antigène du mélanome reconnu par les cellules T 1; mutant du sarcome du rat (Ras); transcriptase inverse de la télomérase humaine (hTERT); points de rupture de translocation du sarcome; inhibiteur d'apoptose du mélanome (ML-IAP); ERG (gène de fusion ETS de la protéase transmembranaire, sérine 2 (TMPRSS2)); N-acétyl-glucosaminyl-transférase V (NA17); protéine à boîte appariée Pax-3 (PAX3); récepteur des androgènes; cycline B1; homologue dérivé de neuroblastome oncogène viral de myélocytomatose aviaire v-myc (MYCN); Membre C de la famille de l'homologue Ras (RhoC); protéine 2 liée à la tyrosinase (TRP-2); Cytochrome P450 1B1 (CYP1B1); Facteur de liaison de type CCCTC (protéine à doigt de zinc), antigène du carcinome spinocellulaire reconnu par les cellules T 3 (SART3); protéine Pax-5 à boîte appariée (PAX5); protéine sp32 de liaison à la proacrosine (OY-TES1); protéine tyrosine kinase spécifique des lymphocytes (LCK); protéine d'ancrage A kinase 4 (AKAP-4); sarcome synovial, point de rupture X 2 (SSX2); récepteur des produits de glycation avancée (RAGE-1); ubiquitaire rénale 1 (RU1); ubiquitaire rénale 2 (RU2); légumine; virus du papillome humain E6 (HPV E6); virus du papillome humain E7 (HPV E7); carboxyl-estérase intestinale; protéine de choc thermique 70-2 mutée (mut hsp70-2); CD79a; CD79b; CD72; récepteur 1 de type immunoglobuline associée aux leucocytes (LAIR1); fragment Fc du récepteur IgA (FCAR ou CD89); récepteur de type immunoglobuline associée aux leucocytes, sous-famille A, membre 2 (LILRA2); membre f de la famille des molécules CD300 (CD300LF); élément A de la famille 12 du domaine de lectine de type C (CLEC12A); antigène des cellules stromales de la moelle osseuse 2 (BST2); récepteur hormonal 2 de type mucine contenant un module de type EGF (EMR2); antigène lymphocytaire 75 (LY75); Glypican-3 (GPC3); récepteur 5 de type Fc (FCRL5); et polypeptide 1 de type lambda d'immunoglobuline (IGLL1).

9. Protéine de fusion selon la revendication 8, dans laquelle le domaine de liaison à l'antigène:
(a) se lie à CD19, la protéine de fusion comprenant
(i) un domaine de liaison à l'antigène comprenant une séquence d'acides aminés choisie parmi l'une quelconque des SEQ ID NO: 356-368 ou 381, ou (ii) un récepteur d'antigène chimérique comprenant une séquence d'acides aminés choisie parmi l'une quelconque des SEQ ID NO: 897, 902, 907, 912, 917, 922, 927, 932, 937, 942, 947, 952, 956;
(b) se lie à CD123, la protéine de fusion comprenant
(i) un domaine de liaison à l'antigène comprenant une séquence d'acides aminés choisie parmi l'une quelconque des SEQ ID NO: 751, 756, 761 ou 766, ou (ii) un récepteur d'antigène chimérique comprenant une séquence d'acides aminés choisie parmi l'une quelconque des SEQ ID NO: 750, 755, 760 ou 765;
(c) se lie à BCMA, la protéine de fusion comprenant
(i) un domaine de liaison à l'antigène comprenant une séquence d'acides aminés choisie parmi l'une quelconque des SEQ ID NO: 382, 386, 390, 394, 398, 402, 406, 410, 414, 418, 422, 426, 430, 434, 438, 442, 446, 450, 454, 458, 462, 466, 470, 474, 478, 482, 486, 490, 494, 498, 502, 506, 510, 514, 518, 522, 528, 531, 534, ou 537, ou (ii) un récepteur d'antigène chimérique comprenant une séquence d'acides aminés choisie parmi l'une quelconque des SEQ ID NO: 789, 791, 793, 795, 797, 799, 801, 803, 805, 807, 809, 811, 813, 815, 817, 819, 821, 823, 825, 827, 829, 831, 833, 835, 837, 839, 841, 843, 845, 847, 849, 851, 853, 855, 857, ou 859; ou
(d) se lie à CD20, la protéine de fusion comprenant
(i) un domaine de liaison à l'antigène comprenant une séquence d'acides aminés située aux positions 470-712 ou 470-939 de la SEQ ID NO: 3033, ou (ii) un récepteur d'antigène chimérique comprenant une séquence d'acides aminés de la SEQ ID NO: 3033.

10. Protéine de fusion selon l'une quelconque des revendications 1 à 9, dans laquelle:
(i)le domaine de dégradation est situé:
a) N-terminal par rapport audit second domaine protéique; ou
b) C-terminal par rapport audit deuxième domaine protéique; et/ou
(ii) la protéine de fusion comprend en outre un peptide signal, par ex., comprend un peptide signal N-terminal par rapport audit domaine de dégradation, éventuellement dans laquelle la protéine de fusion comprend en outre un lieur positionné entre le peptide signal et un autre domaine de la protéine de fusion, par ex., positionné entre le peptide signal et ledit domaine de dégradation.

11. Protéine de fusion selon l'une quelconque des revendications 1 à 10, comprenant une séquence d'acides aminés choisie dans le groupe constitué par les SEQ ID NO: 59, 61, 63, 65, 67, 69, 75, 103, 105, 107, 109, 111, 113, 115, 141, 143, 145, 147, 149, 151, 153, 155, 157, 159, 161, 163, 165, 167, 169, 171, 173, 175, 177, 179, 181, 183 et 990 à 1049.

12. Acide nucléique codant pour la protéine de fusion selon l'une quelconque des revendications 1 à 11.

13. Vecteur comprenant l'acide nucléique selon la revendication 12, éventuellement dans lequel:
(i) ledit vecteur est un vecteur viral, par ex. un vecteur lentiviral; et/ou
(ii) ledit vecteur est présent dans une particule virale.

14. Cellule, par ex. cellule hôte, comprenant la protéine de fusion selon l'une quelconque des revendications 1 à 11, l'acide nucléique selon la revendication 12, ou le vecteur selon la revendication 13, éventuellement dans laquelle ladite cellule, par ex. la cellule hôte:
(a) est une cellule effectrice humaine, par ex., une cellule T humaine ou une cellule NK humaine; et/ou
(b) comprend en outre une protéase capable de cliver ledit site de clivage de la protéase hétérologue.

15. Cellule selon la revendication 14, dans laquelle:
(a) en l'absence d'un composé de stabilisation, la protéine de fusion est dégradée par les voies de dégradation cellulaire, par ex., au moins 50%, 60%, 70%, 80%, 90% ou plus de la protéine de fusion est dégradée; et/ou
(b) ladite cellule comprend en outre un composé de stabilisation, éventuellement dans laquelle:
(i) le domaine de dégradation adopte une conformation plus résistante à la dégradation cellulaire en présence du composé de stabilisation, par rapport à une conformation en l'absence du composé de stabilisation; et/ou
(ii) la conformation de la protéine de fusion est plus permissive au clivage au niveau du site de clivage de la protéase hétérologue en présence du composé de stabilisation, par rapport à une conformation en l'absence du composé de stabilisation; ou le niveau d'expression de la protéine de fusion à la surface des cellules est supérieur, par ex. 2, 3, 4, 5, 6, 7, 8, 9, 10, 20 ou 30 fois supérieur, au niveau d'expression de la protéine de fusion à la surface des cellules dans une cellule ne comprenant pas le composé de stabilisation.

16. Procédé d'expression de manière conditionnelle d'un CAR sur la surface d'une cellule, ledit procédé comprenant: la mise en contact de la protéine de fusion selon l'une quelconque des revendications 1 à 11, ou de la cellule des revendications 14 à 15, avec un composé de stabilisation, dans lequel:
(a) en la présence dudit composé de stabilisation, l'expression de surface dudit CAR est augmentée, par ex. 2, 3, 4, 5, 6, 7, 8, 9, 10, 20 ou 30 fois plus, par rapport au niveau d'expression de surface dudit CAR en l'absence dudit composé de stabilisation; et
(b) en l'absence dudit composé de stabilisation, l'expression de surface dudit CAR est sensiblement diminuée, par ex. 2, 3, 4, 5, 6, 7, 8, 9, 10, 20 ou 30 fois moins, par rapport au niveau d'expression de surface dudit CAR en présence du composé de stabilisation;
dans lequel, en outre, ladite méthode n'est pas une méthode de traitement du corps humain ou animal par thérapie.

17. Procédé selon la revendication 16, dans lequel la présence dudit composé de stabilisation:
(i) est associée à, par ex., provoque, un changement de conformation du domaine de dégradation d'un premier état de pliage à un second état de pliage, dans lequel le premier état de pliage est plus sensible à la dégradation par rapport au second état de pliage; et/ou
(ii) expose ledit site de clivage de protéase, par ex., dans une plus grande mesure, par ex., 2, 3, 4, 5, 6, 7, 8, 9, 10, 20 ou 30 fois plus grande, par rapport à l'exposition du site de clivage de protéase en l'absence dudit composé de stabilisation.

18. Protéine de fusion selon l'une quelconque des revendications 1 à 11, acide nucléique selon la revendication 12, vecteur selon la revendication 13, ou cellule des revendications 14 à 15, pour une utilisation dans le traitement d'une maladie associée à l'expression d'un antigène tumoral chez un sujet, par ex. un cancer.

19. Protéine de fusion, acide nucléique, vecteur ou cellule à utiliser selon la revendication 18, dans laquelle le cancer est:
(i) mésothéliome (par ex., mésothéliome pleural malin), par ex., chez un sujet dont l'état a progressé après au moins un traitement standard antérieur; cancer du poumon (par ex., cancer du poumon non à petites cellules, cancer du poumon à petites cellules, cancer du poumon à cellules squameuses ou cancer du poumon à grandes cellules); cancer du pancréas (par ex., adénocarcinome du canal pancréatique ou adénocarcinome du canal pancréatique métastatique (PDA), par ex., chez un sujet qui a progressé après au moins un traitement standard antérieur); adénocarcinome de l'œsophage, cancer de l'ovaire (par ex., cancer épithélial séreux de l'ovaire, par ex., chez un sujet qui a progressé après au moins un régime antérieur de traitement standard), cancer du sein, cancer colorectal, cancer de la vessie ou toute combinaison de ceux-ci;
(ii) un cancer hématologique, par ex., un cancer hématologique choisi parmi une leucémie ou un lymphome; ou
(iii) choisi parmi: leucémie lymphocytaire chronique (CLL), lymphome à cellules du manteau (MCL), myélome multiple, leucémie aiguë lymphoïde (ALL), lymphome de Hodgkin, leucémie aiguë lymphoïde à cellules B (BALL), leucémie aiguë lymphoïde à cellules T (TALL), lymphome lymphocytaire à petites cellules (SLL), leucémie prolymphocytaire à cellules B, néoplasme à cellules dendritiques plasmacytoïdes blastiques, lymphome de Burkitt, lymphome diffus à grandes cellules B (DLBCL), DLBCL associé à une inflammation chronique, leucémie myéloïde chronique, néoplasmes myéloprolifératifs, lymphome folliculaire, lymphome folliculaire pédiatrique, leucémie à cellules ciliées, lymphome folliculaire à petites ou grandes cellules, affections lymphoprolifératives malignes, lymphome du MALT (lymphome extranodal de la zone marginale du tissu lymphoïde associé aux muqueuses), lymphome de la zone marginale, myélodysplasie, syndrome myélodysplasique, lymphome non hodgkinien, lymphome plasmablastique, néoplasme des cellules dendritiques plasmacytoïdes, macroglobulinémie de Waldenstrom, lymphome de la zone marginale splénique, lymphome/leucémie splénique, lymphome à petites cellules B à pulpe rouge diffuse splénique, leucémie à cellules velues-variante, lymphome lymphoplasmocytaire, maladie des chaînes lourdes, myélome à plasmocytes, plasmocytome solitaire de l'os, plasmocytome extra-osseux, lymphome de la zone marginale nodale, lymphome pédiatrique de la zone marginale nodale, lymphome cutané primaire du centre des follicules, granulomatose lymphomatoïde, lymphome primaire médiastinal (thymique) à grandes cellules B, lymphome intravasculaire à grandes cellules B, lymphome ALK+ à grandes cellules B, lymphome à grandes cellules B survenant dans le cadre d'une maladie de Castleman multicentrique associée au HHV8, lymphome primaire d'épanchement, lymphome à cellules B, leucémie myéloïde aiguë (LMA) ou lymphome inclassable.

20. Protéine de fusion selon l'une quelconque des revendications 1 à 11, l'acide nucléique selon la revendication 12, le vecteur selon la revendication 13, ou la cellule des revendications 14 à 15 pour une utilisation comme médicament.
